# EUROPEAN PATENT SPECIFICATION

(11) **EP 2 106 396 B1**
(45) Date of publication and mention of the grant of the patent: **24.09.2014**
(21) Application number: 08700158.2
(22) Date of filing: 10.01.2008
(51) Int. Cl.: C07D 213/80, C07D 401/12, C07D 471/04, C07D 471/08, C07D 519/00, A61K 31/519, A61P 11/06

(54) **CHEMICAL COMPOUNDS 637: PYRIDOPYRIMIDINEDIONES AS PDE4 INHIBITORS**
CHEMISCHE VERBINDUNGEN 637: PYRIDOPYRIMIDINDIONE ALS PDE4-INHIBITOREN
COMPOSÉS CHIMIQUES 637: PYRIDOPYRIMIDINEDIONES COMME INHIBITEURS PDE4

(30) Priority: 11.01.2007 US 884454 P; 04.06.2007 US 941749 P; 26.07.2007 US 952047 P
(43) Date of publication of application: 07.10.2009
(73) Proprietor: Alcon Research, Ltd., Fort Worth, TX 76134-2099 (US)
(72) Inventor: BONNERT, Roger, Victor, Loughborough Leicestershire LE11 5RH (GB); BURKAMP, Frank, Loughborough Leicestershire LE11 5RH (GB); COX, Rhona, Jane, Loughborough Leicestershire LE11 5RH (GB); DE SOUSA, Simon, Loughborough Leicestershire LE11 5RH (GB); DICKINSON, Mark, Loughborough Leicestershire LE11 5RH (GB); HUNT, Simon, Fraser, Loughborough Leicestershire LE11 5RH (GB); MEGHANI, Premji, Loughborough Leicestershire LE11 5RH (GB); PIMM, Austen, Loughborough Leicestershire LE11 5RH (GB); SANGANEE, Hitesh, Jayantilal, Loughborough Leicestershire LE11 5RH (GB)
(74) Representative: Teipel, Stephan
(86) International application number: PCT/GB2008/000061
(87) International publication number: WO 2008/084223

(56) References cited:
- WO-A-93/19068
- WO-A-2005/086656
- WO-A-2007/004958
- WO-A-2007/108750
- DE-A1- 2 257 203
- US-A- 3 838 156
- LOWE, J. A. ET AL.: "Structure-Activity Relationship of Quinazolinedione Inhibitors of Calcium-Independent Phosphodiesterase" JOURNAL OF MEDICINAL CHEMISTRY, AMERICAN CHEMICAL SOCIETY. WASHINGTON, US, vol. 34, no. 2, 1991, pages 624-628, XP003003222 ISSN: 0022-2623
- DATABASE BEILSTEIN [Online] BEILSTEIN INSTITUTE FOR ORGANIC CHEMISTRY, FRANKFURT-MAIN, DE; XP002477479 Database accession no. 388408 (BRN) & J. MED. CHEM., vol. 33, no. 10, 1990, pages 2697-2706,
- MARTIN, T. J.: "PDE4 INHIBITORS - A REVIEW OF THE RECENT PATENT LITERATURE" IDRUGS, CURRENT DRUGS LTD, GB, vol. 4, no. 3, 2001, pages 312-338, XP008006266 ISSN: 1369-7056
- ODINGO, J. O.: "Inhibitors of PDE4: A review of recent patent literature" EXPERT OPINION ON THERAPEUTIC PATENTS, ASHLEY PUBLICATIONS, GB, vol. 15, no. 7, 2005, pages 773-787, XP002413778 ISSN: 1354-3776
- DATABASE BEILSTEIN [Online] BEILSTEIN INSTITUTE FOR ORGANIC CHEMISTRY, FRANKFURT-MAIN, DE; XP002489032 Database accession no. 415896, 479775, 410392 (BRNs) & FARMACO, EDIZIONE SCIENTIFICA, vol. 26, 1971, pages 844-845,
- DATABASE BEILSTEIN [Online] BEILSTEIN INSTITUTE FOR ORGANIC CHEMISTRY, FRANKFURT-MAIN, DE; XP002489033 Database accession no. 417267, 484686 (BRNs) & ACTA POLONIAE PHARMACEUTICA, vol. 24, 1967, pages 113-117,
- DATABASE BEILSTEIN [Online] BEILSTEIN INSTITUTE FOR ORGANIC CHEMISTRY, FRANKFURT-MAIN, DE; XP002489034 Database accession no. 6423185, 6424485, 6396315, 6400301 (BRNs) & J. MED. CHEM., vol. 31, no. 11, 1988, pages 2108-2121,
- DATABASE BEILSTEIN [Online] BEILSTEIN INSTITUTE FOR ORGANIC CHEMISTRY, FRANKFURT-MAIN, DE; XP002489035 Database accession no. 619160 (BRN) & J. MED. CHEM., vol. 10, 1967, pages 954-955,
- DATABASE BEILSTEIN [Online] BEILSTEIN INSTITUTE FOR ORGANIC CHEMISTRY, FRANKFURT-MAIN, DE; XP002489036 Database accession no. 210425 (BRN) & J. AM. CHEM. SOC., vol. 80, 1958, pages 421-425,
- DATABASE BEILSTEIN [Online] BEILSTEIN INSTITUTE FOR ORGANIC CHEMISTRY, FRANKFURT-MAIN, DE; XP002489037 Database accession no. 4403671, 4407611 (BRNs) & J. HETEROCYCL. CHEM., vol. 17, 1980, pages 235-240,
- DATABASE BEILSTEIN [Online] BEILSTEIN INSTITUTE FOR ORGANIC CHEMISTRY, FRANKFURT-MAIN, DE; XP002489038 Database accession no. 513419 (BRN) & HOPPE-SEYLER'S ZEITSCHR. PHYSIOL. CHEM., vol. 322, 1960, pages 164-172,
- DATABASE BEILSTEIN [Online] BEILSTEIN INSTITUTE FOR ORGANIC CHEMISTRY, FRANKFURT-MAIN, DE; XP002489039 Database accession no. 609165, 610600 (BRNs) & J. CHEM. SOC., 1961, page 4418,
- DATABASE BEILSTEIN [Online] BEILSTEIN INSTITUTE FOR ORGANIC CHEMISTRY, FRANKFURT-MAIN, DE; XP002489040 Database accession no. 674620, 806392 (BRNs) & J. MED. CHEM., vol. 19, 1976, pages 262-275,
- DATABASE BEILSTEIN [Online] BEILSTEIN INSTITUTE FOR ORGANIC CHEMISTRY, FRANKFURT-MAIN, DE; XP002489041 Database accession no. 10462500 (BRN) & J. ORG. CHEM., vol. 71, no. 10, 2006, pages 4021-4023,

## Description

The present invention concerns pyridopyrimidine derivatives having pharmaceutical activity, to processes for preparing such derivatives, to pharmaceutical compositions comprising such derivatives and to the use of such derivatives as active therapeutic agents.

Pharmaceutically active pyridopyrimidine derivatives are disclosed in EP-A-0260817, WO 98/02162, WO 93/19068, WO 00/45800 and WO 2007/101213.

Pharmaceutically active 1,4-dihydro-1,8-naphthyridines are disclosed in WO 2007/050576, WO 2004/105698, US 2004/0102472, WO 2004/048374, WO 2004/047836, WO 02/094823 and WO 99/07704.

Phosphodiesterases (PDEs) work by converting cAMP or cGMP to AMP and GMP, or the inactive nucleotide forms incapable of activating downstream signalling pathways. The inhibition of PDEs leads to the accumulation of cAMP or cGMP, and subsequent activation of downstream pathways. PDEs comprise a large family of second messengers with 11 families and over 50 isoforms. In addition splice variants have been described for each isoform. The PDEs can be cAMP-specific (PDE4, 7, 8, 10), cGMP specific (PDE5, 6, 9) or have dual specificity (PDE1, 2, 3, 11).

cAMP is generated from ATP at the inner leaflet of the plasma membrane through the action of GPCR-regulated adenylate cyclase. Once cAMP is generated, the only way to terminate the signal is through phosphodiesterase action, degrading cAMP into 5'-AMP. Increased concentrations of cAMP are translated into cellular responses mainly by activation of cAMP-dependent protein kinase (PKA). The specific activity of PKA is in part regulated by the sub-cellular localization of PKA, which limits the phosphorylation of PKA to substrates in its near vicinity. The downstream events caused by activation of PKA appear poorly elucidated and involve many components in the initiation of signalling cascades. PDE4s have been shown to have abundant roles in regulating cell desensitisation, adaptation, signal cross-talk, cAMP compartmentalization and feedback loops, and are major regulators of cAMP homeostasis.

The physiological role implicated for elevated cAMP levels include: 1) broad suppression the activity of many imunocompetent cells; 2) induction of airway smooth muscle relaxation; 3) suppression of smooth muscle mitogenesis; and, 4) has beneficial modulatory effects on the activity of pulmonary nerves.

PDE4 has been found to be the predominant cAMP metabolising isozyme family in immune and inflammatory cells and, along with the PDE3 family, a major contributor to cAMP metabolism in airway smooth muscle.

Over the last two decades significant attention has been devoted into the development of PDE4 selective inhibitors for the treatment of inflammatory and immune disorders including asthma, rhinitis, bronchitis, COPD, arthritis and psoriasis. A number of compounds (for example rolipram, tibenelast and denbufylline) have been reported to have impressive effects in animal models of inflammation, especially pulmonary inflammation.

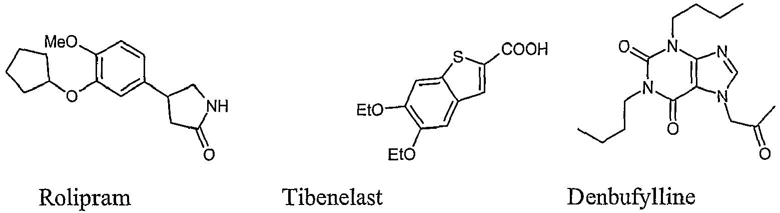

Unfortunately the clinical utility of these inhibitors has been limited by PDE4 related side-effects, including nausea, vomiting and gastric acid secretion. Recently a second generation of PDE4 inhibitors (for example cilomilast, roflumilast and AWD 12-281) has been described having significantly reduced risk of emetic side effects in animal models of emesis, thus providing the potential for an increased therapeutic ratio.

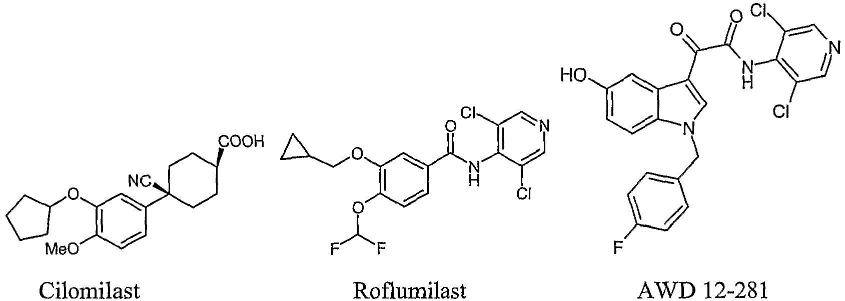

The present invention discloses novel pyridopyrimidine derivatives that are inhibitors of human PDE4 and are thereby useful in therapy.

The present invention provides a compound of formula (I): 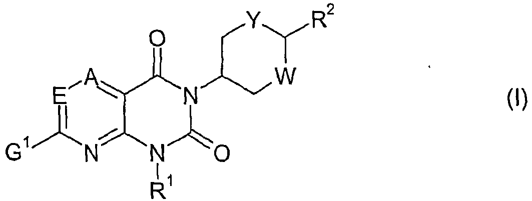 wherein:
A is N or CA¹;
E is N or CE¹;
W is (CH₂)ₙ;
Y is (CH₂)ₚ;
n and p are, independently 0 or 1;
R¹ is aryl or heteroaryl either of which is substituted by one or more of CO₂H, aryl, heteroaryl, (C₁₋₆ alkyl)NR³⁹R⁴⁰, C(O)NHaryl, C(O)N(C₁₋₆ alkyl)(aryl(C₁₋₆ alkyl)), C(O)NHheteroaryl, C(O)NHheterocyclyl, C(O)NH(CH₂)ᵥNH₂, C(O)NH(CH₂)ᵥNHCO₂(C₁₋₆ alkyl), C(O)NH((C₁₋₄ alkyl)aryl), C(O)N(C₁₋₄ alkyl)((C₁₋₄ alkyl)aryl), (C₁₋₆ alkyl)NHC(O)(C₁₋₆ alkoxy), heterocyclyl(C₁₋₄ alkoxy), CH=CH(aryl), C≡C(aryl), aryl(C₁₋₄ alkyl)), heteroaryl(C₁₋₄ alkyl), aryloxy, heteroaryloxy, arylthio, heteroarylthio,
CH=CH(heteroaryl) or C≡C(heteroaryl); and either of which may be additionally optionally substituted by halogen, cyano, hydroxy, C₁₋₄ alkyl, C₁₋₄ alkoxy, CF₃, OCF₃, C₁₋₄ alkylthio,
S(O)(C₁₋₄ alkyl), S(O)₂(C₁₋₄ alkyl) or CO₂(C₁₋₄ alkyl);
or R¹ is aryl(C₁₋₄ alkyl) or heteroaryl(C₁₋₄ alkyl) either of which is optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl), CO₂H, CO₂(C₁₋₄ alkyl), C(O)NHaryl, C(O)NH(CH₂)ᵥNH₂,
C(O)NH(CH₂)ᵥNHCO₂(C₁₋₆ alkyl), aryl, heteroaryl, CH=CH(aryl), C≡C(aryl),
CH=CH(heteroaryl) or C≡C(heteroaryl);
or R¹ is C₅₋₇ cycloalkyl optionally substituted by hydroxy, C₁₋₄ alkyl, CO₂H, CO₂(C₁₋₄ alkyl), aryl or heteroaryl;
or R¹ is C₁₋₁₀ alkyl;
or R¹ is C₁₋₆ alkyl substituted by NR⁴⁷R⁴⁸;
or R¹ is heterocyclyl optionally substituted by C₁₋₆ alkyl, aryl or heteroaryl;
   provided that R¹ is not: 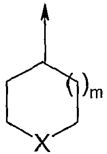 wherein:
   X is S, S(O) or S(O)₂; and m is 0 or 1;
   wherein the aryl or heteroaryl substituents of R¹ are optionally substituted by halogen, cyano, hydroxy, SH, C₁₋₄ alkyl, C₁₋₄ alkoxy, CF₃, OCF₃, C(O)H, C₁₋₆ alkylthio, S(O)(C₁₋₆ alkyl), S(O)₂(C₁₋₆ alkyl), CO₂H, CO₂(C₁₋₆ alkyl), NR⁴¹R⁴², C₁₋₆ alkyl (optionally substituted by halogen, OH, CO₂H, NR²⁹R³⁰, NHC(O)O(C₁₋₆ alkyl), OS(O)₂(C₁₋₆ alkyl) or heterocyclyl), C₁₋₆ alkoxy (optionally substituted by halogen, OH, CO₂H, NR³⁵R³⁶ or heterocyclyl), C₃₋₆ cycloalkyl (optionally substituted halogen, OH, CO₂H, NR³⁷R³⁸ or heterocyclyl) or heterocyclyl;
v is 1, 2, 3 or 4;
R² is NR⁵⁰C(O)R³ or NR⁴R⁵;
R³ is C₁₋₆ alkyl {optionally substituted by hydroxyl, C₁₋₆ alkoxy, NR⁷R⁸, heterocyclyl {optionally substituted by oxo, hydroxy, C₁₋₆ alkyl, CO₂(C₁₋₆ alkyl), aryl, heteroaryl, aryl(C₁₋₄ alkyl), heterocyclyl or C(O)(C₁₋₄ alkyl)phenyl}, aryl, heteroaryl, C₃₋₇ cycloalkyl, C₃₋₇ cycloalkyl(C₁₋₄ alkyl), CO₂H, CO₂(C₁₋₆ alkyl), aryl(C₁₋₄ alkoxy), aryl(C₁₋₄ alkylthio), S(O)₂(C₁₋₆ alkyl), NHC(O)heteroaryl or NHC(O)R⁶}, C₁₋₆ alkoxy, C₃₋₆ cycloalkyl {optionally substituted by hydroxyl, NR⁴³R⁴⁴ or C₁₋₆ alkyl}, heterocyclyl {optionally substituted by oxo, hydroxy, C₁₋₆ alkyl, amino, aryl, heteroaryl, aryl(C₁₋₄ alkyl), heteroaryl(C₁₋₄ alkyl), heterocyclyl or C(O)(C₁₋₄ alkyl)phenyl}, aryl(C₁₋₄ alkyl) {substituted by amino(C₁₋₄ alkyl)}, aryl or heteroaryl;
R⁴ is hydrogen, C₁₋₆ alkyl (optionally substituted by aryl or heteroaryl), aryl or heteroaryl; R⁵ is hydrogen, C₁₋₆ alkyl (optionally substituted by hydroxyl, C₁₋₆ alkoxy, aryl, aryloxy, phenyl(C₁₋₆ alkoxy), heteroaryl, C₃₋₁₀ cycloalkyl, CO₂H, CO₂(C₁₋₆ alkyl), NHC(O)O(C₁₋₆ alkyl) or NHC(O)R⁶), C₁₋₆ alkoxy, C₃₋₆ cycloalkyl (optionally substituted by hydroxy, C₁₋₆ alkyl, phenyl, phenyl(C₁₋₆ alkyl), heteroaryl or heteroaryl(C₁₋₆ alkyl)), heterocyclyl (optionally substituted by C₁₋₆ alkyl, C(O)NH₂ or phenyl(C₁₋₆ alkyl)), aryl or heteroaryl; R⁶ is C₁₋₆ alkyl or phenyl;
R⁷ and R⁸ are, independently, hydrogen, C₁₋₆ alkyl or phenyl(C₁₋₄ alkyl);
the foregoing phenyl, aryl and heteroaryl moieties of R³, R⁴, R⁵, R⁶, R⁷ and R⁸ are, independently, optionally substituted by: halogen, cyano, nitro, hydroxy, S(O)_{q}R⁹, OC(O)NR¹⁰R¹¹, NR¹²R¹³, NR¹⁴C(O)R¹⁵, NR¹⁶C(O)NR¹⁷R¹⁸, S(O)₂NR¹⁹R²⁰, NR²¹S(O)₂R²², C(O)NR²³R²⁴, C(O)R²⁵, CO₂R²⁶, NR²⁷CO₂R²⁸, OC(O)(C₁₋₆ alkyl), C₁₋₆ alkyl, C₁₋₆ hydroxyalkyl, C₁₋₆ haloalkyl, C₁₋₆ alkoxy(C₁₋₆)alkyl, amino(C₁₋₄ alkyl), di(C₁₋₆)alkylamino(C₁₋₆)alkyl, C₁₋₆ alkoxy, C₁₋₆ haloalkoxy, hydroxyl(C₁₋₆ alkoxy), heterocyclyl(C₁₋₆ alkoxy), C₁₋₆ alkoxy(C₁₋₆)alkoxy, amino(C₁₋₄ alkoxy), C₁₋₄ alkylamino(C₁₋₄ alkoxy) (itself optionally substituted by phenyl), di(C₁₋₄ alkyl)amino(C₁₋₄ alkoxy), C₁₋₆ alkylthio, C₂₋₆ alkenyl, C₂₋₆ alkynyl, C₃₋₁₀ cycloalkyl (itself optionally substituted by C₁₋₄ alkyl or oxo), methylenedioxy, difluoromethylenedioxy, heterocyclyl, heterocyclyl(C₁₋₄ alkyl), phenyl, phenyl(C₁₋₄)alkyl, phenoxy, phenylthio, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halogen, hydroxy, nitro, S(O)ᵣ(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃;
A¹, E¹ and G¹ are, independently, hydrogen, halogen, cyano, hydroxy, C₁₋₄ alkyl, C₁₋₄ alkoxy, CF₃ or OCF₃;
q and r are, independently, 0, 1 or 2;
unless otherwise stated heterocyclyl is optionally substituted by OH, C₁₋₆ alkyl, C₃₋₇ cycloalkyl, NR³¹R³², (C₁₋₆ alkyl)OH or (C₁₋₆ alkyl)NR³³R³⁴, NR⁴⁹CO₂(C₁₋₆ alkyl), CO₂(C₁₋₆ alkyl), C(O)(C₁₋₆ alkyl), C(O)heterocyclyl, heteroaryl, (C₁₋₆ alkyl)C(O)NR⁵³R⁵⁴, (C₁₋₆ alkyl)C(O)NR⁵⁵R⁵⁶, (C₁₋₆ alkyl)C(O)heterocyclyl or heterocyclyl;
R⁹, R¹⁰, R¹¹, R¹², R¹³, R¹⁴, R¹⁵ R¹⁶, R¹⁷, R¹⁸, R¹⁹, R²⁰, R²¹, R²², R²³, R²⁴, R²⁵, R²⁶, R²⁷ and R²⁸ are, independently, C₁₋₆ alkyl {optionally substituted by halogen, hydroxy or C₁₋₆ alkoxy}, CH₂(C₂₋₆ alkenyl), phenyl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃} or heteroaryl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃};
R¹⁰, R¹¹, R¹², R¹³, R¹⁴, R¹⁵, R¹⁶, R¹⁷, R¹⁸, R¹⁹, R²⁰, R²¹, R²³, R²⁴, R²⁵, R²⁶, R²⁷ and R²⁸ can also be hydrogen;
R⁵⁰ is hydrogen or C₁₋₆ alkyl (optionally substituted by NR⁵¹R⁵²);
R³⁰, R³², R³⁴, R³⁶, R³⁸, R⁴⁰, R⁴², R⁴⁴ or R⁴⁸ are, independently, hydrogen, C₁₋₆ alkyl (optionally substituted by hydroxy, C₁₋₆ alkoxy, C₁₋₆ alkylthio, C₃₋₇ cycloalkyl (optionally substituted by hydroxy) or NR⁴⁵R⁴⁶), C₃₋₇ cycloalkyl (optionally substituted by hydroxy(C₁₋₆ alkyl)) or heterocyclyl (optionally substituted by C₁₋₆ alkyl);
R²⁹, R³¹, R³³, R³⁵, R³⁷, R³⁹, R⁴¹, R⁴³, R⁴⁵, R⁴⁶, R⁴⁷, R⁴⁹, R⁵¹, R⁵², R⁵³, R⁵⁴, R⁵⁵ and R⁵⁶ are, independently, hydrogen or C₁₋₆ alkyl;
or a N-oxide thereof; or a pharmaceutically acceptable salt thereof.

Certain compounds of the present invention can exist in different isomeric forms (such as enantiomers, diastereomers, geometric isomers or tautomers). The present invention covers all such isomers and mixtures thereof in all proportions. Enantiomerically pure forms are particularly desired. When in solid crystalline form a compound of formula (I) can be in the form of a co-crystal with another chemical entity and the invention encompasses all such co-crystals.

A pharmaceutically acceptable salt of a compound of formula (I) includes a salt prepared from a pharmaceutically acceptable non-toxic base, such as an inorganic or organic base. A salt derived from an inorganic base is, for example, an aluminium, calcium, potassium, magnesium, sodium or zinc salt. A salt derived from an organic base is, for example, a salt of a primary, secondary or tertiary amine, such as arginine, betaine, benzathine, caffeine, choline, chloroprocaine, cycloprocaine, N',N'-dibenzylethylenediamine, diethanolamine, diethylamine, 2-diethyl-aminoethanol, 2-dimethylaminoethanol, ethanolamine, ethylendiamine, N-ethyl-morpholine, N-ethyl piperidine, glucamine, glucosamine, histidine, hydrabamine, isopropylamine, lysine, meglumine, morpholine, piperazine, piperidine, polyamine resins, procaine, purines, tertiary butylamine, theobromine, triethylamine, trimethylamine, tripropylamine, tromethamine or thanolamine.

A pharmaceutically acceptable salt of a compound of formula (I) also includes a quaternary ammonium salt, for example where an amine group in a compound of formula (I) reacts with a C₁₋₁₀ alkyl halide (for example a chloride, bromide or iodide) to form a quaternary ammonium salt.

A pharmaceutically acceptable salt also includes a salt of pharmaceutically acceptable organic acid, such as a carboxylic or sulphonic acid, for example: an acetate, adipate, alginate, ascorbate, aspartate, benzenesulphonate (besylate), benzoate, butyrate, camphorate, camphorsulphonate (such as [(1S,4R)-7,7-dimethyl-2-oxobicyclo[2.2.1]hept-1-yl]methanesulfonic acid salt), camsylate, citrate, p-chlorobenzenesulphonate, cyclopentate, 2,5-dichlorobesyalte, digluconate, edisylate (ethane-1,2-disulfonate or ethane-1-(sulfonic acid)-2-sulfonate), esylate, ethanesulphonate, fumarate, formate, 2-furoate, 3-furoate, gluconate, glucoheptanate, glutamate, glutarate, glycerophosphate, glycolate, heptanoate, hexanoate, hippurate, 2-hydroxyethane sulfonate, lactate, lactobionate, laurate, malate, maleate, malonate, mandelate, methanesulphonate, 2-naphthalenesulfonate, napadisylate (naphthalene-1,5-disulfonate or naphthalene-1-(sulfonic acid)-5-sulfonate), nicotinate, oleate, orotate, oxalate, pantothenate, pamoate, pamoic, pectinate, 3-phenylpropionate, pivalate, propionate, pivolate, pyruvate, saccharinate, salicylate, stearate, succinate, tartrate, p-toluenesulphonate, transcinnamic acid, trifluoroacetate, xinafoate, xinofolate, xylate (p-xylene-2-sulphonic acid), undecanoate, 2-mesitylenesulphonate, 2-naphthalenesulphonate, D-mandelate, L-mandelate, 2,5-dichlorobenzenesulphonate, cinnamate or benzoate; or a salt of an inorganic acid such as a hydrobromide, hydrochloride, hydroiodide, sulphate, bisulfate, phosphate, nitrate, hemisulfate, thiocyanate, persulfate, phosphate or sulphonate salt. In another aspect of the invention the stoichiometry of the salt is, for example, a hemi- salt, or a mono- or di-salt or tri-salt.

A pharmaceutically acceptable salt of a compound of formula (I) can be prepared *in situ* during the final isolation and purification of a compound, or by separately reacting the compound or N-oxide with a suitable organic or inorganic acid and isolating the salt thus formed.

In one aspect of the invention acid addition salts are, for example, a hydrochloride, dihydrochloride, hydrobromide, phosphate, sulfate, acetate, diacetate, fumarate, maleate, malonate, succinate, tartrate, citrate, methanesulfonate or p-toluenesulfonate, camphorsulfonate (such as [(1S,4R)-7,7-dimethyl-2-oxobicyclo[2.2.1]hept-1-yl]methanesulfonic acid salt). An alternative acid addition salt is a trifluoroacetate salt.

Alternatively, a suitable salt can be a quaternary ammonium salt formed by the reaction of a primary, secondary or tertiary amine group in a compound of formula (I) with, for example, a C₁₋₆ alkyl halide (such as methyl iodide or methyl bromide).

The compounds of the invention may exist as solvates (such as hydrates) and the present invention covers all such solvates.

Halogen includes fluorine, chlorine, bromine and iodine. Halogen is, for example, fluorine or chlorine.

Alkyl moieties are straight or branched chain and are, for example, methyl, ethyl, n-propyl, iso-propyl or tert-butyl. Haloalkyl is, for example C₂F₅, CF₃ or CHF₂. Alkoxy is, for example, methoxy or ethoxy; and haloalkoxy is, for example OCF₃ or OCHF₂.

Alkenyl is, for example, vinyl or prop-2-enyl. Alkynyl is, for example, propargyl.

Cycloalkyl is a mono- or bi-cyclic ring system which is saturated or unsaturated but not aromatic. It is, for example, cyclopropyl, cyclobutyl, cyclopentyl, cyclohexyl or bicyclo[3.1.1]heptenyl. C₃₋₇ Cycloalkyl(C₁₋₄ alkyl) is, for example, cyclopentylCH₂. Cycloalkyloxy is, for example, cyclopropyloxy, cyclopentyloxy or cyclohexyloxy. Cycloalkylalkoxy is, for example, (cyclopropyl)methoxy or 2-(cyclopropyl)ethoxy.

Heterocyclyl is a non-aromatic 5- or 6-membered ring optionally fused to one or more other non-aromatic rings and optionally fused to a benzene ring, comprising at least one heteroatom selected from the group comprising nitrogen, oxygen and sulphur. Heterocyclyl is, for example, azetidinyl, pyrrolidinyl, piperidinyl, piperazinyl, isoindolyl, morpholinyl, 3,8-diazabicyclo[3.2.1]octyl, 8-azabicyclo[2.2.2]octyl, 2-oxa-6-azabicyclo[5.4.0]undeca-7,9,11-trienyl, 7-oxa-10-azabicyclo[4.4.0]deca-1,3,5-trienyl, 6-thia-1,4-diazabicyclo[3.3.0]octa-4,7-dienyl, tetrahydropyranyl, azabicyclo[3.2.1]octyl, 1,2,3,4-tetrahydroquinolinyl, 1,4-diazepinyl, quinuclidinyl, 9-oxa-2,8-diazaspiro[4.4]non-7-enyl, 1,2-dihydroquinazolinyl, 2,4,10-triazabicyclo[4.4.0]deca-1,3,5,8-tetraenyl or 2-oxa-5-aza-bicyclo[4.4.0]deca-7,9,1 1-trienyl. Further examples of heterocyclyl include azepinyl, homopiperazinyl, thiomorpholinyl, 1,4-oxazepinyl or 1-azabicyclo[2.2.2]octyl. Still further examples of heterocyclyl include homomorpholinyl, 1,4'-bipiperidinyl, 4'-morpholine-4-piperidinyl, 4-pyrrolidin-1-ylpiperidinyl or homomorpholinyl.

Hydroxyalkyl is, for example, CH₂OH; C₁₋₆ alkoxy(C₁₋₆)alkyl is, for example CH₃CH₂; and, C₁₋₆ alkoxy(C₁₋₆)alkoxy is, for example, CH₃OCH₂O. Dialkylaminoalkyl is, for example (CH₃)₂NCH₂ or (CH₃)(CH₃CH₂)NCH₂. Amino(C₁₋₄ alkyl) is, for example, CH₂NH₂. Amino(C₁₋₄ alkoxy) is, for example, OCH₂NH₂. C₁₋₄ Alkylamino(C₁₋₄ alkoxy) is, for example, CH₃NHCH₂O.

Aryl is, for example, phenyl or naphthyl. In one aspect aryl is phenyl. Aryl(C₁₋₄ alkyl) is, for example, benzyl. Aryl(C₁₋₄ alkoxy) is, for example, phenylmethoxy. Aryl(C₁₋₄ alkylthio) is, for example, phenylCH₂S.

Heteroaryl is, for example, an aromatic 5- or 6-membered ring, optionally fused to one or more other rings (which may be carbocyclic or heterocyclic, and aromatic or non-aromatic), comprising at least one heteroatom selected from the group comprising nitrogen, oxygen and sulphur; or an N-oxide thereof, or an S-oxide or S-dioxide thereof. Heteroaryl is, for example, furyl, thienyl (also known as thiophenyl), pyrrolyl, thiazolyl, isothiazolyl, pyrazolyl, oxazolyl, isoxazolyl, imidazolyl, [1,2,3]-thiadiazolyl, [1,2,4]-triazolyl, [1,2,3]-triazolyl, pyridinyl, pyrimidinyl, pyrazinyl, pyridazinyl, indolyl, benzo[b]furyl (also known as benzfuryl), benz[b]thienyl (also known as benzthienyl or benzthiophenyl), indazolyl, benzimidazolyl, 1,2,3-benztriazolyl, benzoxazolyl, 1,3-benzthiazolyl, 1,2,3-benzothiadiazolyl, thieno[3,2-b]pyridin-6-yl, 1,2,3-benzoxadiazolyl, benzo[1,2,3]thiadiazolyl, 2,1,3-benzothiadiazolyl, benzofurazan (also known as 2,1,3-benzoxadiazolyl), quinoxalinyl, a pyrazolopyridine (for example lH-pyrazolo[3,4-b]pyridinyl or pyrazolo[1,5-a]pyridinyl), an imidazopyridine (for example imidazo[1,2-a]pyridinyl or imidazo[1,2-a]-5,6,7,8-tetrahydropyridinyl), a dihydropyrido[2,3-d]pyrimidine (for example 1,4-dihydropyrido[2,3-d]pyrimidinyl), quinolinyl, isoquinolinyl, a naphthyridinyl (for example [1,6]naphthyridinyl, [1,7]naphthyridinyl or [1,8]naphthyridinyl), 1,2,3-thiadiazolyl, 1H-pyrrolo[2,3-b]pyridinyl, thieno[2,3-b]pyridinyl, thieno[2,3-b]pyrazinyl, [1,2,4]triazolo[1,5-a]pyrimidinyl or 6,7-dihydro-5H-[1,3]thiazolo[3,2-a]pyrimidinyl; or an N-oxide thereof, or an S-oxide or S-dioxide thereof. A further example of heteroaryl is 4,5,6,7-tetrahydrobenzfuryl.

In one aspect of the invention heteroaryl is, for example, pyrrolyl, thiazolyl, pyrazolyl, imidazolyl, [1,2,4]-triazolyl, pyridinyl, pyrimidinyl, pyrazinyl, pyridazinyl, indolyl, indazolyl, benzimidazolyl, quinoxalinyl, a pyrazolopyridine (for example 1H-pyrazolo[3,4-b]pyridinyl or pyrazolo[1,5-a]pyridinyl), an imidazopyridine (for example imidazo[1,2-a]pyridinyl or imidazo[1,2-a]-5,6,7,8-tetrahydropyridinyl), a dihydropyrido[2,3-d]pyrimidine (for example 1,4-dihydropyrido[2,3-d]pyrimidinyl), quinolinyl, isoquinolinyl, a naphthyridinyl (for example [1,6]naphthyridinyl, [1,7]naphthyridinyl or [1,8]naphthyridinyl), 1H-pyrrolo[2,3-b]pyridinyl or [1,2,4]triazolo[1,5-a]pyrimidinyl; or an N-oxide thereof.

NHC(O)Heteroaryl is, for example, NHC(O)pyridinyl. Heteroaryl(C₁₋₄ alkyl) is, for example, pyridinylCH₂.

Optionally substituted is, for example, an unsubstituted moiety or a moiety carrying 1, 2 or 3 substituents.

In one particular aspect the present invention provides a compound of formula (I), wherein: A is N or CA¹; E is N or CE¹; W is (CH₂)ₙ; Y is (CH₂)ₚ; n and p are, independently 0 or 1; R¹ is aryl or heteroaryl either of which is substituted by one or more of CO₂H, aryl, heteroaryl, (C₁₋₆ alkyl)NR³⁹R⁴⁰, C(O)NHaryl, C(O)N(C₁₋₆ alkyl)(aryl(C₁₋₆ alkyl)), C(O)NHheteroaryl, C(O)NHheterocyclyl, C(O)NH(CH₂)ᵥNH₂, C(O)NH(CH₂)ᵥNHCO₂(C₁₋₆ alkyl), C(O)NH((C₁₋₄ alkyl)aryl), heterocyclyl(C₁₋₄ alkoxy), CH=CH(aryl), C≡C(aryl), aryl(C₁₋₄ alkyl), heteroaryl(C₁₋₄ alkyl), aryloxy, heteroaryloxy, arylthio, heteroarylthio, CH=CH(heteroaryl) or C≡C(heteroaryl); and either of which may be additionally optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl) or CO₂(C₁₋₄ alkyl); or R¹ is aryl(C₁₋₄ alkyl) or heteroaryl(C₁₋₄ alkyl) either of which is optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl), CO₂H, CO₂(C₁₋₄ alkyl), C(O)NHaryl, C(O)NH(CH₂)ᵥNH₂, C(O)NH(CH₂)ᵥNHCO₂(C₁₋₆ alkyl), aryl, heteroaryl, CH=CH(aryl), C≡C(aryl), CH=CH(heteroaryl) or C≡C(heteroaryl); or R¹ is C₅₋₇ cycloalkyl optionally substituted by hydroxy, C₁₋₄ alkyl, CO₂H, CO₂(C₁₋₄ alkyl), aryl or heteroaryl; or R¹ is C₁₋₆ alkyl, substituted by NR⁴⁷R⁴⁸; or R¹ is heterocyclyl optionally substituted by C₁₋₆ alkyl, aryl or heteroaryl;
provided that R¹ is not: 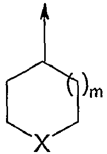 wherein: X is S, S(O) or S(O)₂; and m is 0 or 1;
wherein the aryl or heteroaryl substituents of R¹ are optionally substituted by halogen, cyano, hydroxy, SH, C₁₋₄ alkyl, C₁₋₄ alkoxy, CF₃, OCF₃, C(O)H, C₁₋₆ alkylthio, S(O)(C₁₋₆ alkyl), S(O)₂(C₁₋₆ alkyl), CO₂H, CO₂(C₁₋₆ alkyl), NR⁴¹R⁴², C₁₋₆ alkyl (optionally substituted by halogen, OH, CO₂H, NR²⁹R³⁰ or heterocyclyl), C₁₋₆ alkoxy (optionally substituted by halogen, OH, CO₂H, NR³⁵R³⁶ or heterocyclyl), C₃₋₆ cycloalkyl (optionally substituted halogen, OH, CO₂H, NR³⁷R³⁸ or heterocyclyl) or heterocyclyl; v is 1, 2, 3 or 4; R² is NR⁵⁰C(O)R³ or NR⁴R⁵; R³ is C₁₋₆ alkyl {optionally substituted by hydroxyl, C₁₋₆ alkoxy, NR⁷R⁸, heterocyclyl (optionally substituted by oxo, hydroxy, C₁₋₆ alkyl, aryl, heteroaryl, aryl(C₁₋₄ alkyl), heterocyclyl or C(O)(C₁₋₄ alkyl)phenyl), aryl, heteroaryl, C₃₋₇ cycloalkyl, C₃₋₇ cycloalkyl(C₁₋₄ alkyl), CO₂H, CO₂(C₁₋₆ alkyl), aryl(C₁₋₄ alkoxy), aryl(C₁₋₄ alkylthio), S(O)₂(C₁₋₆ alkyl), NHC(O)heteroaryl or NHC(O)R⁶}, C₁₋₆ alkoxy, C₃₋₆ cycloalkyl {optionally substituted by hydroxyl, NR⁴³R⁴⁴ or C₁₋₆ alkyl} heterocyclyl {optionally substituted by oxo, hydroxy, C₁₋₆ alkyl, amino, aryl, heteroaryl, aryl(C₁₋₄ alkyl), heteroaryl(C₁₋₄ alkyl), heterocyclyl or C(O)(C₁₋₄ alkyl)phenyl}, aryl(C₁₋₄ alkyl) {substituted by amino(C₁₋₄ alkyl)}, aryl or heteroaryl; R⁴ is hydrogen, C₁₋₆ alkyl (optionally substituted by aryl or heteroaryl), aryl or heteroaryl; R⁵ is hydrogen, C₁₋₆ alkyl (optionally substituted by hydroxyl, C₁₋₆ alkoxy, aryl, aryloxy, phenyl(C₁₋₆ alkoxy), heteroaryl, C₃₋₁₀ cycloalkyl, CO₂H, CO₂(C₁₋₆ alkyl), NHC(O)O(C₁₋₆ alkyl) or NHC(O)R⁶), C₁₋₆ alkoxy, C₃₋₆ cycloalkyl (optionally substituted by hydroxy, C₁₋₆ alkyl, phenyl, phenyl(C₁₋₆ alkyl), heteroaryl or heteroaryl(C₁₋₆ alkyl)), heterocyclyl (optionally substituted by C₁₋₆ alkyl, C(O)NH₂ or phenyl(C₁₋₆ alkyl)), aryl or heteroaryl; R⁶ is C₁₋₆ alkyl or phenyl; R⁷ and R⁸ are, independently, hydrogen, C₁₋₆ alkyl or phenyl(C₁₋₄ alkyl); the foregoing phenyl, aryl and heteroaryl moieties of R³, R⁴, R⁵, R⁶, R⁷ and R⁸ are, independently, optionally substituted by: halogen, cyano, nitro, hydroxy, S(O)_{q}R⁹, OC(O)NR¹⁰R¹¹, NR¹²R¹³, NR¹⁴C(O)R¹⁵, NR¹⁶C(O)NR¹⁷R¹⁸, S(O)₂NR¹⁹R²⁰, NR²¹S(O)₂R²², C(O)NR²³R²⁴, C(O)R²⁵, CO₂R²⁶, NR²⁷CO₂R²⁸, C₁₋₆ alkyl, C₁₋₆ hydroxyalkyl, C₁₋₆ haloalkyl, C₁₋₆ alkoxy(C₁₋₆)alkyl, amino(C₁₋₄ alkyl), di(C₁₋₆)alkylamino(C₁₋₆)alkyl, C₁₋₆ alkoxy, C₁₋₆ haloalkoxy, C₁₋₆ alkoxy(C₁₋₆)alkoxy, amino(C₁₋₄ alkoxy) or C₁₋₄ alkylamino(C₁₋₄ alkoxy) (itself optionally substituted by phenyl), C₁₋₆ alkylthio, C₂₋₆ alkenyl, C₂₋₆ alkynyl, C₃₋₁₀ cycloalkyl (itself optionally substituted by C₁₋₄ alkyl or oxo), methylenedioxy, difluoromethylenedioxy, heterocyclyl, heterocyclyl(C₁₋₄ alkyl), phenyl, phenyl(C₁₋₄)alkyl, phenoxy, phenylthio, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halogen, hydroxy, nitro, S(O)ᵣ(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃; A¹, E¹ and G¹ are, independently, hydrogen, halogen, cyano, hydroxy, C₁₋₄ alkyl, C₁₋₄ alkoxy, CF₃ or OCF₃; q and r are, independently, 0, 1 or 2; unless otherwise stated heterocyclyl is optionally substituted by OH, C₁₋₆ alkyl, NR³¹R³², (C₁₋₆ alkyl)OH or (C₁₋₆ alkyl)NR³³R³⁴, NR⁴⁹CO₂(C₁₋₆ alkyl), CO₂(C₁₋₆ alkyl), (C₁₋₆ alkyl)C(O)NR⁵³R⁵⁴ or heterocyclyl; R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷ and R²⁸ are, independently, C₁₋₆ alkyl {optionally substituted by halogen, hydroxy or C₁₋₆ alkoxy}, CH₂(C₂₋₆ alkenyl), phenyl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃} or heteroaryl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃}; R¹⁰, R¹¹,R¹², R¹³, R¹⁴, R¹⁵, R¹⁶, R¹⁷, R¹⁸, R¹⁹, R²⁰, R²¹, R²³, R²⁴, R²⁵, R²⁶, R²⁷ and R²⁸ can also be hydrogen; R⁵⁰ is hydrogen or C₁₋₆ alkyl (optionally substituted by NR⁵¹R⁵²); R³⁰, R³³, R³⁴, R³⁶, R³⁸ R⁴⁰, R⁴², R⁴⁴ or R⁴⁸ are, independently, hydrogen, C₁₋₆ alkyl (optionally substituted by C₁₋₆ alkoxy, C₁₋₆ alkylthio or NR⁴⁵R⁴⁶, (C₁₋₆ alkyl)OH or unsubstituted heterocyclyl; R²⁹, R³¹ R³³ R³⁵, R³⁷, R³⁹, R⁴¹, R⁴³, R⁴⁵, R⁴⁶, R⁴⁷, R⁴⁹, R⁵¹, R⁵², R⁵³ or R¹⁴ are, independently, hydrogen or C₁₋₆ alkyl; or a N-oxide thereof; or a pharmaceutically acceptable salt thereof.

In a further aspect the present invention provides a compound of formula (I) wherein: A is N or CA¹; E is N or CE¹; W is (CH₂)ₙ; Y is (CH₂)ₚ; n and p are, independently 0 or 1; R¹ is aryl or heteroaryl either of which is substituted by one or more of CO₂H, aryl, heteroaryl, (C₁₋₆ alkyl)NR³⁹R⁴⁰, C(O)NHaryl, C(O)NH(CH₂)ᵥNH₂, C(O)NH(CH₂)ᵥNHCO₂(C₁₋₆ alkyl), C(O)NH(CH₂)ᵥ(aryl(C₁₋₄ alkyl)), CH=CH(aryl), C.C(aryl), aryl(C₁₋₄ alkyl), heteroaryl(C₁₋₄ alkyl), CH=CH(aryl), C≡C(aryl), CH=CH(heteroaryl) or C≡C(heteroaryl); and either of which may be additionally optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl) or CO₂(C₁₋₄ alkyl); or R¹ is aryl(C₁₋₄ alkyl) or heteroaryl(C₁₋₄ alkyl) either of which is optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl), CO₂H, CO₂(C₁₋₄ alkyl), C(O)NHaryl, C(O)NH(CH₂)ᵥNH₂, C(O)NH(CH₂)ᵥNHCO₂(C₁₋₆ alkyl), aryl, heteroaryl, CH=CH(aryl), C≡C(aryl), CH=CH(heteroaryl) or C≡C(heteroaryl); R¹ is C₅₋₇ cycloalkyl optionally substituted by hydroxy, C₁₋₄ alkyl, CO₂H, CO₂(C₁₋₄ alkyl), aryl or heteroaryl; or R¹ is heterocyclyl optionally substituted by aryl or heteroaryl; provided that R¹ is not: 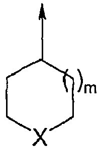 wherein: X is S, S(O) or S(O)₂; and m is 0 or 1; wherein the aryl or heteroaryl substituents of R¹ are optionally substituted by halogen, cyano, hydroxy, SH, C₁₋₄ alkyl, C₁₋₄ alkoxy, CF₃, OCF₃, C(O)H, C₁₋₆ alkylthio, S(O)(C₁₋₆ alkyl), S(O)₂(C₁₋₆ alkyl), CO₂H, CO₂(C₁₋₆ alkyl), NR⁴¹R⁴², C₁₋₆ alkyl (optionally substituted by halogen, OH, CO₂H, NR²⁹R³⁰ or heterocyclyl), C₁₋₆ alkoxy (optionally substituted by halogen, OH, CO₂H, NR³⁵R³⁶ or heterocyclyl) or C₃₋₆ cycloalkyl (optionally substituted halogen, OH, CO₂H, NR³⁷R³⁸ or heterocyclyl); v is 1, 2, 3 or 4; R² is NHC(O)R³ or NR⁴R⁵; R³ is C₁₋₆ alkyl {optionally substituted by hydroxyl, C₁₋₆ alkoxy, NR⁷R⁸, heterocyclyl (optionally substituted by oxo, hydroxy, C₁₋₆ alkyl, aryl, heteroaryl, aryl(C₁₋₄ alkyl), heterocyclyl or C(O)(C₁₋₄ alkyl)phenyl), aryl, heteroaryl, C₃₋₇ cycloalkyl, C₃₋₇ cycloalkyl(C₁₋₄ alkyl), CO₂H, CO₂(C₁₋₆ alkyl), aryl(C₁₋₄ alkoxy), aryl(C₁₋₄ alkylthio), S(O)₂(C₁₋₆ alkyl), NHC(O)heteroaryl or NHC(O)R⁶}, C₁₋₆ alkoxy), C₃₋₆ cycloalkyl {optionally substituted by hydroxyl or C₁₋₆ alkyl}, heterocyclyl {optionally substituted by oxo, hydroxy, C₁₋₆ alkyl, amino, aryl, heteroaryl, aryl(C₁₋₄ alkyl), heteroaryl(C₁₋₄ alkyl), heterocyclyl or C(O)(C₁₋₄ alkyl)phenyl}, aryl(C₁₋₄ alkyl) {substituted by amino(C₁₋₄ alkyl)}, aryl or heteroaryl; R⁴ is hydrogen, C₁₋₆ alkyl (optionally substituted by aryl or heteroaryl), aryl or heteroaryl; R⁵ is hydrogen, C₁₋₆ alkyl (optionally substituted by hydroxyl, C₁₋₆ alkoxy, aryl, aryloxy, phenyl(C₁₋₆ alkoxy), heteroaryl, C₃₋₁₀ cycloalkyl, CO₂H, CO₂(C₁₋₆ alkyl), NHC(O)O(C₁₋₆ alkyl) or NHC(O)R⁶), Cₗ-₆ alkoxy, C₃₋₆ cycloalkyl (optionally substituted by hydroxy, C₁₋₆ alkyl, phenyl, phenyl(C₁₋₆ alkyl), heteroaryl or heteroaryl(C₁₋₆ alkyl)), heterocyclyl (optionally substituted by C₁₋₆ alkyl, C(O)NH₂ or phenyl(C₁₋₆ alkyl)), aryl or heteroaryl; R⁶ is C₁₋₆ alkyl or phenyl; R⁷ and R⁸ are, independently, hydrogen, C₁₋₆ alkyl or phenyl(C₁₋₄ alkyl); the foregoing phenyl, aryl and heteroaryl moieties of R³, R⁴, R⁵, R⁶, R⁷ and R⁸ are, independently, optionally substituted by: halogen, cyano, nitro, hydroxy, S(O)_{q}R⁹, OC(O)NR¹⁰R¹¹, NR¹²R¹³, NR¹⁴C(O)R¹⁵, NR¹⁶C(O)NR¹⁷R¹⁸, S(O)₂NR¹⁹R²⁰, NR²¹S(O)₂R²², C(O)NR²³R²⁴, C(O)R²⁵, CO₂R²⁶, NR²⁷CO₂R²⁸, C₁₋₆ alkyl, C₁₋₆ hydroxyalkyl, C₁₋₆ haloalkyl, C₁₋₆ alkoxy(C₁₋₆)alkyl, amino(C₁₋₄ alkyl), di(C₁₋₆)alkylamino(C₁₋₆)alkyl, C₁₋₆ alkoxy, C₁₋₆ haloalkoxy, C₁₋₆ alkoxy(C₁-₆)alkoxy, amino(C₁₋₄ alkoxy) or C₁₋₄ alkylamino(C₁₋₄ alkoxy) (itself optionally substituted by phenyl), C₁₋₆ alkylthio, C₂₋₆ alkenyl, C₂₋₆ alkynyl, C₃₋₁₀ cycloalkyl (itself optionally substituted by C₁₋₄ alkyl or oxo), methylenedioxy, difluoromethylenedioxy, heterocyclyl, heterocyclyl(C₁₋₄ alkyl), phenyl, phenyl(C₁₋₄)alkyl, phenoxy, phenylthio, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halogen, hydroxy, nitro, S(O)ᵣ(C₁₋₄ alkyl), S(O)₂N-H₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃; A¹, E¹ and G¹ are, independently, hydrogen, halogen, cyano, hydroxy, C₁₋₄ alkyl, C₁₋₄ alkoxy, CF₃ or OCF₃; q and r are, independently, 0, 1 or 2; unless otherwise stated heterocyclyl is optionally substituted by OH, C₁₋₄ alkyl, NR³¹R³², (C₁₋₄ alkyl)OH or (C₁₋₄ alkyl)NR³³R³⁴; R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷ and R²⁸ are, independently, C₁₋₆ alkyl {optionally substituted by halogen, hydroxy or C₁₋₆ alkoxy}, CH₂(C₂₋₆ alkenyl), phenyl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃} or heteroaryl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃}; R¹⁰, R¹¹, R¹², R¹³, R¹⁴, R¹⁵, R¹⁶, R¹⁷, R¹⁸, R¹⁹, R²⁰, R²¹, R²³, R²⁴, R²⁵, R²⁶, R²⁷ and R²⁸ can also be hydrogen; R²⁹, R³¹, R³³, R³⁵, R³⁷, R³⁹ and R⁴¹ are, independently, hydrogen or C₁₋₆ alkyl; R³⁰, R³³, R³⁴, R³⁶, R³⁸, R⁴⁰ and R⁴² are, independently, hydrogen, C₁₋₆ alkyl, (C₁₋₆ alkyl)OH or unsubstituted heterocyclyl; or a N-oxide thereof; or a pharmaceutically acceptable salt thereof.

In a further aspect the present invention provides a compound of formula (I) wherein: A is N or CA¹; E is N or CE¹; W is (CH₂)ₙ; Y is (CH₂)ₚ; n and p are, independently 0 or 1; R¹ is aryl or heteroaryl either of which is substituted by CO₂H, C(O)NHphenyl, C(O)NH(CH₂)ᵥNH₂, C(O)NH(CH₂)ᵥNHCO₂(C₁₋₆ alkyl), C(O)NH(CH₂)ᵥ(phenyl(C₁₋₄ alkyl), CH=CH(aryl), C.C(aryl), phenyl(C₁₋₄ alkyl), heteroaryl(C₁₋₄ alkyl), CH=CH(phenyl), C≡C(phenyl), CH=CH(heteroaryl) or C≡C(heteroaryl); and either of which may be additionally optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl) or CO₂(C₁₋₄ alkyl); or R¹ is aryl(C₁₋₄ alkyl) or heteroaryl(C₁₋₄ alkyl) either of which is optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl), CO₂H, CO₂(C₁₋₄ alkyl), C(O)NHphenyl, C(O)NH(CH₂)ᵥNH₂, C(O)NH(CH₂)ᵥNHCO₂(C₁₋₆ alkyl), CH=CH(aryl), C≡C(aryl), CH=CH(heteroaryl) or C≡C(heteroaryl); or R¹ is C₅₋₇ cycloalkyl optionally substituted by hydroxy, C₁₋₄ alkyl, CO₂H or CO₂(C₁₋₄ alkyl); wherein the aryl or heteroaryl substituents of R¹ are optionally substituted by halogen, cyano, hydroxy, SH, C₁₋₄ alkyl, C₁₋₄ alkoxy, CF₃, OCF₃, C(O)H, C₁₋₆ alkylthio, S(O)(C₁₋₆ alkyl), S(O)₂(C₁₋₆ alkyl), CO₂H, CO₂(C₁₋₆ alkyl), NR⁴¹R⁴², C₁₋₆ alkyl (optionally substituted by halogen, OH, CO₂H, NR²⁹R³⁰ or heterocyclyl), C₁₋₆ alkoxy (optionally substituted by halogen, OH, CO₂H, NR³⁵R³⁶ or heterocyclyl) or C₃₋₆ cycloalkyl (optionally substituted halogen, OH, CO₂H, NR³⁷R³⁸ or heterocyclyl); v is 1, 2, 3 or 4; R² is NHC(O)R³ or NR⁴R⁵; R³ is C₁₋₆ alkyl {optionally substituted by hydroxyl, C₁₋₆ alkoxy, NR⁷R⁸, heterocyclyl (optionally substituted by oxo, hydroxy, C₁₋₆ alkyl, aryl, heteroaryl, aryl(C₁₋₄ alkyl), heterocyclyl or C(O)(C₁₋₄ alkyl)phenyl), aryl, heteroaryl, C₃₋₇ cycloalkyl, C₃₋₇ cycloalkyl(C₁₋₄ alkyl), CO₂H, CO₂(C₁₋₆ alkyl), aryl(C₁₋₄ alkoxy), aryl(C₁₋₄ alkylthio), S(O)₂(C₁₋₆ alkyl), NHC(O)heteroaryl or NHC(O)R⁶}, C₁₋₆ alkoxy, C₃₋₆ cycloalkyl {optionally substituted by hydroxyl or C₁₋₆ alkyl}, heterocyclyl {optionally substituted by oxo, hydroxy, C₁₋₆ alkyl, amino, aryl, heteroaryl, aryl(C₁₋₄ alkyl), heteroaryl(C₁₋₄ alkyl), heterocyclyl or C(O)(C₁₋₄ alkyl)phenyl}, aryl(C₁₋₄ alkyl) {substituted by amino(C₁₋₄ alkyl)}, aryl or heteroaryl; R⁴ is hydrogen, C₁₋₆ alkyl (optionally substituted by aryl or heteroaryl), aryl or heteroaryl; R⁵ is hydrogen, C₁₋₆ alkyl (optionally substituted by hydroxyl, C₁₋₆ alkoxy, aryl, aryloxy, phenyl(C₁₋₆ alkoxy), heteroaryl, C₃₋₁₀ cycloalkyl, CO₂H, CO₂(C₁₋₆ alkyl), NHC(O)O(C₁₋₆ alkyl) or NHC(O)R⁶), C₁₋₆ alkoxy, C₃₋₆ cycloalkyl (optionally substituted by hydroxy, C₁₋₆ alkyl, phenyl, phenyl(C₁₋₆ alkyl), heteroaryl or heteroaryl(C₁₋₆ alkyl)), heterocyclyl (optionally substituted by C₁₋₆ alkyl, C(O)NH₂ or phenyl(C₁₋₆ alkyl)), aryl or heteroaryl; R⁶ is C₁₋₆ alkyl or phenyl; R⁷ and R⁸ are, independently, hydrogen, C₁₋₆ alkyl or phenyl(C₁₋₄ alkyl); the foregoing phenyl, aryl and heteroaryl moieties of R³, R⁴, R⁵, R⁶, R⁷ and R⁸ are, independently, optionally substituted by: halogen, cyano, nitro, hydroxy, S(O)_{q}R⁹, OC(O)NR¹⁰R¹¹, NR¹²R¹³, NR¹⁴C(O)R¹⁵, NR¹⁶C(O)NR¹⁷R¹⁸, S(O)₂NR¹⁹R²⁰, NR²¹S(O)₂R²², C(O)NR²³R²⁴, C(O)R²⁵, CO₂R²⁶, NR²⁷CO₂R²⁸, C₁₋₆ alkyl, C₁₋₆ hydroxyalkyl, C₁₋₆ haloalkyl, C₁₋₆ alkoxy(C₁₋₆)alkyl, amino(C₁₋₄ alkyl), di(C₁₋₆)alkylamino(C₁₋₆)alkyl, C₁₋₆ alkoxy, C₁₋₆ haloalkoxy, C₁₋₆ alkoxy(C₁₋₆)alkoxy, amino(C₁₋₄ alkoxy) or C₁₋₄ alkylamino(C₁₋₄ alkoxy) (itself optionally substituted by phenyl), C₁₋₆ alkylthio, C₂₋₆ alkenyl, C₂₋₆ alkynyl, C₃₋₁₀ cycloalkyl (itself optionally substituted by C₁₋₄ alkyl or oxo), methylenedioxy, difluoromethylenedioxy, heterocyclyl, heterocyclyl(C₁₋₄ alkyl), phenyl, phenyl(C₁₋₄)alkyl, phenoxy, phenylthio, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halogen, hydroxy, nitro, S(O)ᵣ(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃; A¹, E¹ and G¹ are, independently, hydrogen, halogen, cyano, hydroxy, C₁₋₄ alkyl, C₁₋₄ alkoxy, CF₃ or OCF₃; q and r are, independently, 0, 1 or 2; unless otherwise stated heterocyclyl is optionally substituted by OH, C₁₋₄ alkyl, NR³¹R³², (C₁₋₄ alkyl)OH or (C₁₋₄ alkyl)NR³³R³⁴; R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷ and R²⁸ are, independently, C₁₋₆ alkyl {optionally substituted by halogen, hydroxy or C₁₋₆ alkoxy}, CH₂(C₂₋₆ alkenyl), phenyl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl) S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃} or heteroaryl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃}; R¹⁰, R¹¹,R¹², R¹³, R¹⁴, R¹⁵, R¹⁶, R¹⁷, R¹⁸, R¹⁹, R²⁰, R²¹, R²³, R²⁴, R²⁵, R²⁶, R²⁷ and R²⁸ can also be hydrogen; R²⁹, R³¹, R³³, R³⁵, R³⁷, R³⁹ and R⁴¹ are, independently, hydrogen or C₁₋₆ alkyl; R³⁰, R³³, R³⁴, R³⁶, R³⁸, R⁴⁰ and R⁴² are, independently, hydrogen, C₁₋₆ alkyl, (C₁₋₆ alkyl)OH or unsubstituted heterocyclyl; or a N-oxide thereof; or a pharmaceutically acceptable salt thereof.

In another aspect the present invention provides a compound of formula (I) wherein E is CE¹. For example E is CF.

In another aspect A¹, E¹ and G¹ are, independently, hydrogen or halogen (for example fluoro).

In a further aspect the present invention provides a compound of formula (I) wherein E is CE¹. E¹ is, for example, hydrogen or halogen (such as fluoro).

In another aspect the present invention provides a compound of formula (I) wherein G¹ is hydrogen or halogen (such as fluoro). For example G¹ is hydrogen.

In yet another aspect the present invention provides a compound of formula (I) wherein A is CH.

In a further aspect the present invention provides a compound of formula (I) wherein n and p are both 1.

In another aspect the present invention provides a compound of formula (I) wherein A is CA¹; E is CE¹; W and Y are both CH₂; and G¹, A¹ and E¹ are, independently, hydrogen or halogen (such as fluoro) (for example G¹ and A¹ are both hydrogen, and E¹ is hydrogen or fluoro (for example E¹ is fluoro)).

In a still further aspect the present invention provides a compound of formula (I) wherein R¹ is phenyl {substituted by phenyl, CO₂H, C(O)NH(CH₂)ᵥNH₂ (wherein v is 1, 2 or 3), C(O)NH(CH₂)ᵥ(phenyl(C₁₋₄ alkyl)) (wherein v is 1 or 2), C≡C-phenyl, C≡C-heteroaryl or phenyl(C₁₋₄ alkyl)}, C₅₋₇ cycloalkyl or phenyl(C₁₋₄ alkyl) (such as benzyl); wherein phenyl and heteroaryl rings are optionally substituted by halo (such as fluoro), C₁₋₄ alkyl, CF₃, C₁₋₄ alkoxy or OCF₃.

In a further aspect the present invention provides a compound of formula (I) wherein R¹ is phenyl (substituted by phenyl, C.C-heteroaryl, C(O)NH(CH₂)ᵥ(phenyl(C₁₋₄ alkyl)) (wherein v is 1 or 2) or phenyl(C₁₋₄ alkyl) (such as benzyl wherein phenyl and heteroaryl rings are optionally substituted by halo (such as fluoro), C₁₋₄ alkyl, CF₃, C₁₋₄ alkoxy or OCF₃.

In another aspect the present invention provides a compound of formula (I) wherein R¹ is phenyl substituted by phenyl {which is optionally substituted by halogen, hydroxy, CH(O), CO₂H, C₁₋₄ alkyl, C₁₋₄ alkyl(N(C₁₋₄ alkyl)₂), C₁₋₄ alkyl(NH₂), C₁₋₄ alkyl(NH(C₁₋₄ alkyl)), C₁₋₄ hydroxyalkyl, CF₃, C₁₋₄ alkylthio, C₁₋₄ alkyl(heterocyclyl) or C₁₋₄ alkylNHC(O)O(C₁₋₄ alkyl)} or heterocyclyl; and any heterocyclyl is optionally substituted by hydroxy or C₁₋₆ alkyl.

In yet another aspect the present invention provides a compound of formula (I) wherein R¹ is phenyl substituted by phenyl {which is optionally substituted by halogen, hydroxy, CH(O), CO₂H, C₁₋₄ alkyl, C₁₋₄ alkyl(N(C₁₋₄ alkyl)₂), C₁₋₄ alkyl(NH₂), C₁₋₄ alkyl(NH(C₁₋₄ alkyl)), C₁₋₄ hydroxyalkyl, CF₃, C₁₋₄ alkylthio, C₁₋₄ alkyl(heterocyclyl) or C₁₋₄ alkylNHC(O)O(C₁₋₄ alkyl)} or heterocyclyl.

In another aspect the present invention provides a compounf of formula (I) wherein R¹ is phenyl substituted by phenyl {which is optionally substituted by hydroxy, C₁₋₄ alkyl(NR⁶⁰R⁶¹) or C₁₋₄ alkyl(heterocyclyl)}; R⁶⁰ and R⁶¹ are, independently, hydrogen, C₁₋₄ hydroxyalkyl, C₁₋₄ alkoxy(C₁₋₄ alkyl), C₁₋₄ alkyl (itself optionally substituted by NH₂, NH(C₁₋₄ alkyl) or N(C₁₋₄ alkyl)₂); and heterocyclyl is optionally substituted by C₁₋₄ alkyl, C(O) (C₁₋₄ alkyl), C₁₋₄ hydroxyalkyl or C₃₋₇ cycloalkyl.

In a further aspect the present invention provides a compound of formula (I) wherein R¹ is phenyl substituted (for example in the 3-position) by phenyl {which is optionally substituted (for example substituted by 1 or 2) by C₁₋₄ alkyl(heterocyclyl) [such as CH₂(heterocyclyl)], C₁₋₄ alkoxy substituted by heterocyclyl [for example ethoxy substituted by heterocyclyl], hydroxy or (C₁₋₄ alkyl)NH(C₁₋₄ alkyl)OH [such as (CH₂)₃NH(CH₂)₃OH]); wherein heterocyclyl is optionally substituted by C₁₋₆ alkyl.

In another aspect the present invention provides a compound of formula (I) wherein R¹ is phenyl substituted (for example in the 3-position) by phenyl {which is optionally substituted by C₁₋₄ alkyl(heterocyclyl) [such as CH₂(heterocyclyl)], C₁₋₄ alkoxy substituted by heterocyclyl [for example ethoxy substituted by heterocyclyl], or (C₁₋₄ alkyl)NH(C₁₋₄ alkyl)OH [such as (CH₂)₃NH(CH₂)₃0H]}.

In a further aspect the present invention provides a compound of formula (I) wherein R¹ is phenyl substituted by phenyl (substituted by C₁₋₄ alkyl(heterocyclyl) [such as CH₂(heterocyclyl)] and optionally further substituted by hydroxy); the heterocyclyl being optionally substituted by C₁₋₆ alkyl.

In a still further aspect heterocyclyl is, for example, 1,4-oxazepinyl, 1,4-diazepanyl, pyrrolidinyl, morpholinyl, piperidinyl or piperazinyl, homopiperazinyl, homomorpholinyl, 1,4'-bipiperidinyl.

In another aspect heterocyclyl is, for example, pyrrolidinyl, morpholinyl, piperidinyl or piperazinyl.

In yet another aspect heterocyclyl is, for example, pyrrolidinyl, morpholinyl, piperidinyl or piperazinyl, homopiperazinyl, homomorpholinyl, 1,4'-bipiperidinyl.

In another aspect heterocyclyl is, for example, morpholinyl, piperidinyl or piperazinyl.

In yet another aspect heterocyclyl is, for example, morpholinyl, piperidinyl or piperazinyl homopiperazinyl, homomorpholinyl, 1,4'-bipiperidinyl.

In a further aspect heterocyclyl is, for example, pyrrolidinyl or piperazinyl

In a still further aspect heterocyclyl is, for example, pyrrolidinyl or piperazinyl, homopiperazinyl, homomorpholinyl, 1,4'-bipiperidinyl.

In another aspect the present invention provides a compound of formula (I) wherein R² is NHC(O)R³; and R³ is C₁₋₄ alkyl {substituted by NR⁷R⁸, heterocyclyl or heteroaryl}, C₃₋₇ cycloalkyl (optionally substituted by NR⁴³R⁴⁴) or heteroaryl; wherein R⁷, R⁸, R⁴³ and R⁴⁴ are as defined above (for example they are, independently, hydrogen or C₁₋₆ alkyl); heteroaryl being optionally substituted by hydroxy, halo, C₁₋₄ alkyl, CF₃, C₁₋₄ alkoxy, OCF₃, heterocyclyl (such as pyrrolidinyl) or amino(C₁₋₄ alkyl).

In a still further aspect the present invention provides a compound of formula (I) wherein R² is NH₂ or NHC(O)R³; and R³ is C₁₋₄ alkyl {substituted by NR⁷R⁸, heterocyclyl or heteroaryl} or heteroaryl; wherein R⁷ and R⁸ are as defined above; heteroaryl being optionally substituted by hydroxy, halo, C₁₋₄ alkyl, CF₃, C₁₋₄ alkoxy, OCF₃, heterocyclyl (such as pyrrolidinyl) or amino(C₁₋₄ alkyl).

In a still further aspect the present invention provides a compound of formula (I) wherein R² is NH₂ or NHC(O)R³; and R³ is C₁₋₄ alkyl {substituted by NR⁷R⁸, heterocyclyl or heteroaryl} or heteroaryl; wherein R⁷ and R⁸ are as defined above; heteroaryl being optionally substituted by halo, C₁₋₄ alkyl, CF₃, C₁₋₄ alkoxy, OCF₃, heterocyclyl (such as pyrrolidinyl) or amino(C₁₋₄ alkyl).

In another aspect the present invention provides a compound of formula (I) wherein R² is NHC(O)R³, wherein R³ is heteroaryl (for example imidazolyl, imidazo[1,2-a]pyridinyl, imidazo[1,2-a]-5,6,7,8-tetrahydropyridinyl, thiazoyl, pyridinyl, quinoxalinyl or quinolinyl), heterocyclyl (such as pyrrolidinyl), C₁₋₆ alkoxy or C₃₋₇ cycloalkyl (such as cyclopropyl); heteroaryl being optionally substituted by halo (such as fluoro), hydroxy, C₁₋₄ alkyl (such as methyl) or heterocyclyl; and, heterocyclyl being optionally substituted by C₁₋₄ alkyl.

In a further aspect the present invention provides a compound of formula (I) wherein R² is NHC(O)R³, wherein R³ is heteroaryl (for example imidazo[1,2-a]pyridinyl), heterocyclyl (such as pyrrolidinyl), C₁₋₆ alkoxy or C₃₋₇ cycloalkyl (such as cyclopropyl); heteroaryl being optionally substituted by halo (such as fluoro) or heterocyclyl; and, heterocyclyl being optionally substituted by C₁₋₄ alkyl.

In yet another aspect the present invention provides a compound of formula (I) wherein R² is NHC(O)R³, wherein R³ is heteroaryl (for example imidazo[1,2-a]pyridinyl), heterocyclyl (such as pyrrolidinyl) or C₃₋₇ cycloalkyl (such as cyclopropyl); heteroaryl being optionally substituted by halo (such as fluoro); and, heterocyclyl being optionally substituted by C₁₋₄ alkyl.

In a further aspect the present invention provides a compound of formula (I) wherein R² is NHC(O)R³, wherein R³ is heteroaryl (for example imidazo[1,2-a]pyridinyl, thiazoyl, pyridinyl, quinoxalinyl or quinolinyl), heterocyclyl (such as pyrrolidinyl) or C₃₋₇ cycloalkyl (such as cyclopropyl); heteroaryl being optionally substituted by halo (such as fluoro), hydroxy or C₁₋₄ alkyl; and, heterocyclyl being optionally substituted by C₁₋₄ alkyl.

In a further aspect the present invention provides a compound of formula (I) wherein R² is NHC(O)R³, wherein R³ is heteroaryl (for example imidazo[1,2-a]pyridinyl, imidazo[1,2-a]-5,6,7,8-tetrahydropyridinyl, thiazoyl, pyridinyl, quinoxalinyl or quinolinyl)) optionally substituted by halo (such as fluoro), hydroxy or C₁₋₄ alkyl.

In another aspect the present invention provides a compound of formula (I) wherein R² is NHCH₂R³; and R³ is C₁₋₄ alkyl {substituted by NR⁷R⁸, heterocyclyl or heteroaryl}, C₃₋₇ cycloalkyl (optionally substituted by NR⁴³R⁴⁴) or heteroaryl; wherein R⁷, R⁸, R⁴³ and R⁴⁴ are as defined above (for example they are, independently, hydrogen or C₁₋₆ alkyl); heteroaryl being optionally substituted by halo, C₁₋₄ alkyl, CF₃, C₁₋₄ alkoxy, OCF₃, heterocyclyl (such as pyrrolidinyl) or amino(C₁₋₄ alkyl).

In a still further aspect the present invention provides a compound of formula (I) wherein R² is NHCH₂R³; and R³ is C₁₋₄ alkyl {substituted by NR⁷R⁸, heterocyclyl or heteroaryl} or heteroaryl; wherein R⁷ and R⁸ are as defined above; heteroaryl being optionally substituted by halo, C₁₋₄ alkyl, CF₃, C₁₋₄ alkoxy, OCF₃, heterocyclyl (such as pyrrolidinyl) or amino(C₁₋₄ alkyl).

In another aspect the present invention provides a compound of formula (I) wherein R² is NHCH₂R³, wherein R³ is heteroaryl (for example imidazolyl, imidazo[1,2-a]pyridinyl or imidazo[1,2-a]-5,6,7,8-tetrahydropyridinyl), heterocyclyl (such as pyrrolidinyl), C₁₋₆ alkoxy or C₃₋₇ cycloalkyl (such as cyclopropyl); heteroaryl being optionally substituted by halo (such as fluoro), C₁₋₄ alkyl (such as methyl) or heterocyclyl; and, heterocyclyl being optionally substituted by C₁₋₄ alkyl.

In a further aspect the present invention provides a compound of formula (I) wherein R² is NHCH₂R³, wherein R³ is heteroaryl (for example imidazo[1,2-a]pyridinyl), heterocyclyl (such as pyrrolidinyl), C₁₋₆ alkoxy or C₃₋₇ cycloalkyl (such as cyclopropyl); heteroaryl being optionally substituted by halo (such as fluoro) or heterocyclyl; and, heterocyclyl being optionally substituted by C₁₋₄ alkyl.

In yet another aspect the present invention provides a compound of formula (I) wherein R² is NHCH₂R³, wherein R³ is heteroaryl (for example imidazo[1,2-a]pyridinyl), heterocyclyl (such as pyrrolidinyl) or C₃₋₇ cycloalkyl (such as cyclopropyl); heteroaryl being optionally substituted by halo (such as fluoro); and, heterocyclyl being optionally substituted by C₁₋₄ alkyl.

In a further aspect the present invention provides a compound of formula (I) wherein R² is NHCH₂R³, wherein R³ is heteroaryl (for example imidazo[1,2-a]pyridinyl or imidazo[1,2-a]-5,6,7,8-tetrahydropyridinyl) optionally substituted by halo (such as fluoro).

In a further aspect the present invention provides a compound of formula (I) having the stereochemistry shown below:

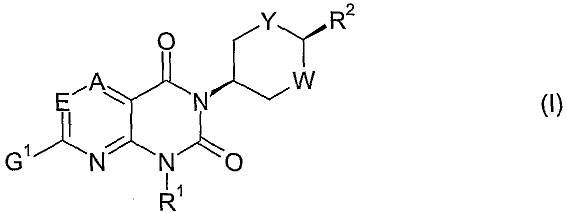

Compounds of the invention are described in the Examples. Each of the compounds of the Examples is a further aspect of the present invention. In another aspect the present invention provides each individual compound of an Example or a pharmaceutically acceptable salt thereof. Further, where the individual compound of an Example is a salt of a compound of formula (I) the invention further provides each parent compound of formula (I), or a different pharmaceutically acceptable salt thereof.

In another aspect the present invention provides a compound of formula (I) which is:
6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (Example 54);
6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl] cyclohexyl} imidazo[1,2-*a*]pyridine-2-carboxamide (Example 79);
6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (Example 96);
6-Fluoro-3-(*cis*-4-{[(6-fluoroimidazo[1,2-*a*]pyridin-2-yl)methyl]amino}cyclohexyl)-1-[4'-hydroxy-2'-(morpholin-4-ylmethyl)biphenyl-3-yl]pyrido[2,3-*d*]pyrimidine-2,4(1*H*,3*H*)-dione (Example 246); or,
*N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}-6-methylpyridine-2-carboxamide (Example 308);
or a pharmaceutically acceptable salts thereof.

The compounds of the present invention can be prepared as described below, by adapting methods known in the art, or by using or adapting the preparative methods described in the Examples.

In a further aspect the invention provides a process for the preparation of a compound of formula (I) wherein R² is NHC(O)R³, which comprises removing the Boc protecting group from a compound of formula (II): 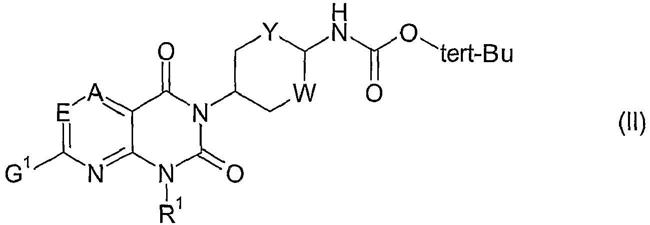 wherein R¹, G¹, E, A, Y and W are as defined in formula (I), (for example with an acid such as trifluoroacetic acid or hydrochloric acid) and reacting the product so formed with a compound of formula (IIIa, IIIb, or IIIc): 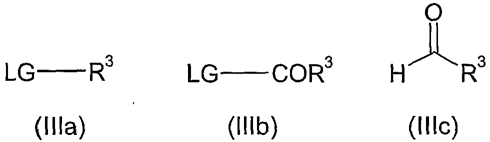 wherein R³ is as defined in formula (I), and LG is a leaving group. The process with formula (IIIa) (for which LG is, for example, halogen) is carried out at a suitable temperature, generally between 0 °C and the boiling point of the solvent, in a suitable solvent such as dichloromethane or N-methylpyrrolidinone. The process with formula (IIIb) is optionally carried out in the presence of a base and/or a coupling reagent such as HATU, HOAT, HOBT or DIEA. (When a coupling agent is used LG is OH, and when no coupling agent is used LG is, for example, halogen.) The reductive amination process with formula (IIIc) is carried out at a suitable temperature, generally between 0 °C and the boiling point of the solvent in a suitable solvent such as 1,2-dicholoroethane, for example using sodium triacetoxyborohydride and catalytic acetic acid.

A compound of formula (II) wherein R¹, G¹, E, A, Y and W are as defined in formula (I) and wherein R² is NHC(O)R³, can be prepared by condensing a compound of formula (IV): 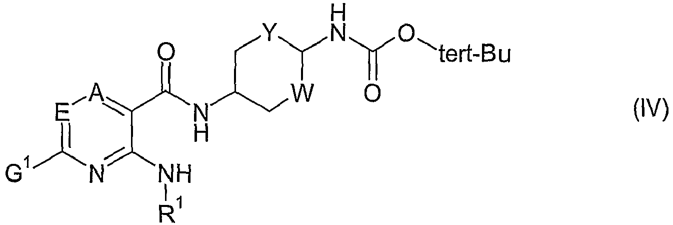 wherein R¹, G¹, E, A, Y and W are as defined in formula (I), with a suitable carbonylating agent such as carbonyl diimidazole or ethyl chloroformate in the presence of a suitable base such as sodium hydride. The process is carried out at a suitable temperature, generally between 0 °C and the boiling point of the solvent, in a suitable solvent such as tetrahydrofuran.

A compound of formula (IV) wherein R¹, G¹, E, A, Y and W are as defined in formula (I), can be prepared by reacting a compound of formula (V): 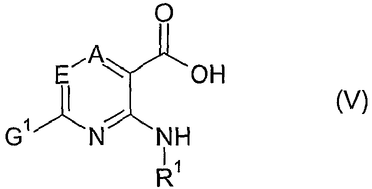 wherein R¹, G¹, E, and A are as defined in formula (I), with an amine of formula (VI) 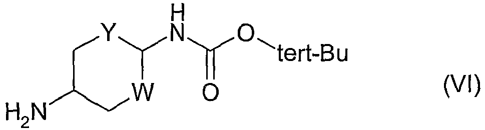 wherein Y and W are as defined in formula (I). The process is carried out at a suitable temperature, generally between 0 °C and the boiling point of the solvent, in a suitable solvent such as dichloromethane. The process is optionally carried out in the presence of a base and a coupling reagent such as HATU, HOAT, HOBT or DIEA.

A compound of formula (V) wherein R¹, G¹, E, and A are as defined in formula (I), can be prepared by reacting a compound of formula (VII): 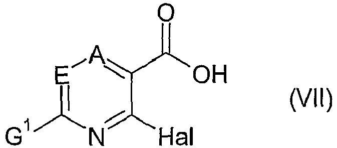 wherein G¹, E, and A are as defined in formula (I) and Hal represents a halogen atom, with an aniline (VIII):

R¹-NH₂ (VIII)

wherein R¹ is as defined in formula (I). The process is carried out at a suitable temperature, generally between 50 °C and the boiling point of the solvent, in a suitable solvent such as dimethylformamide. The process is optionally carried out in the presence of a base such as potassium carbonate.

The compounds of the invention can also be prepared using the preparative methods shown in the schemes below wherein R' is a substituent on a phenyl group of R¹:

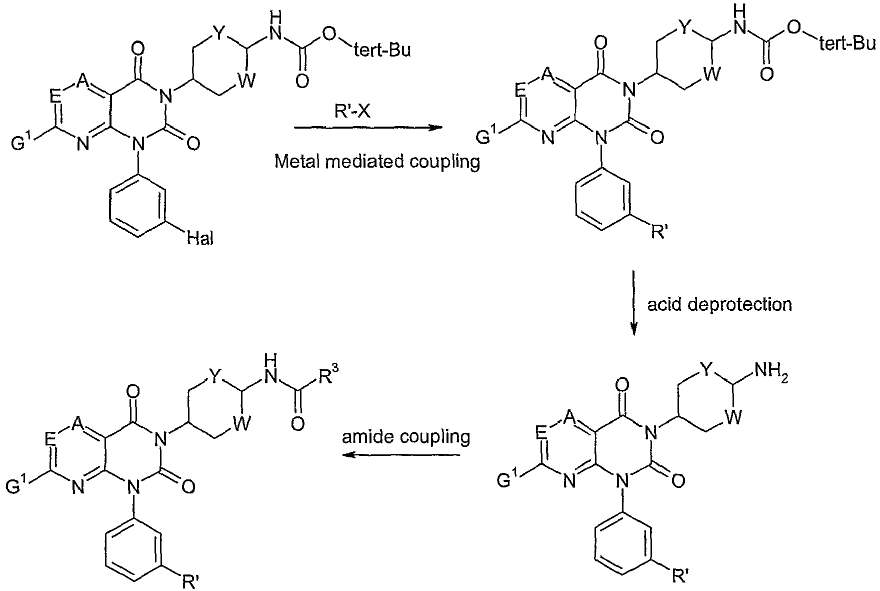

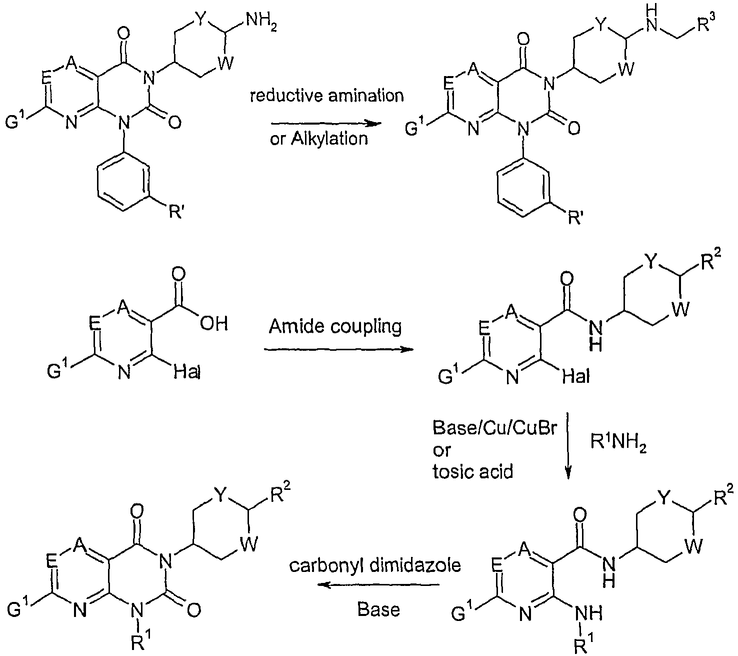

The preparations of various intermediates are described in the literature or can be prepared by routine adaptation of methods described in the literature.

In the above processes it may be desirable or necessary to protect an acid group or a hydroxy or other potentially reactive group. Suitable protecting groups and details of processes for adding and removing such groups may be found in "Protective Groups in Organic Synthesis", 3rd Edition (1999) by Greene and Wuts.

In another aspect the present invention provides processes for the preparation of compounds of formula (I).

Intermediates useful in the preparation of compounds of the invention are for example an intermediate of formula (II): 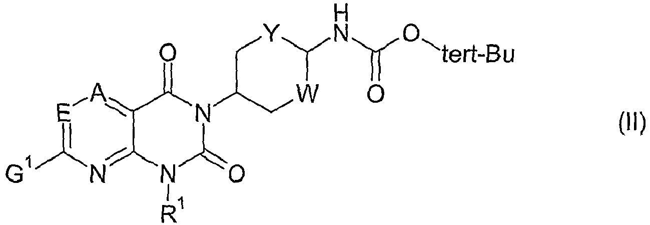 or formula (IV): 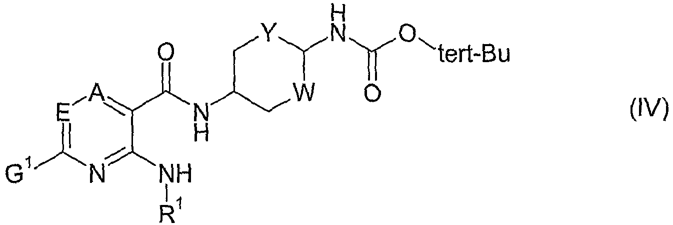 or formula (V): 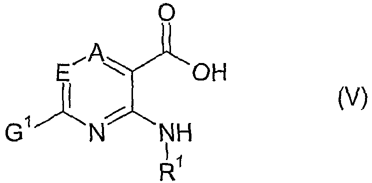 or 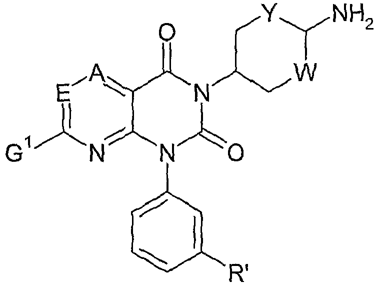 or 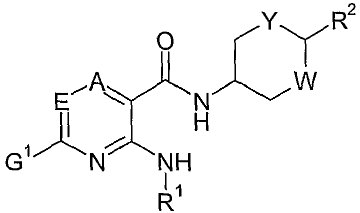 wherein R¹, R², G¹, E, A, Y and W are as defined in formula (I), and R' is a substituent on a phenyl group of R¹.

The compounds of formula (I) have activity as pharmaceuticals, in particular as modulators of PDE 4 receptor activity, and may be used in the treatment of inflammatory diseases, asthma or COPD.

Examples of disease states that can be treated with a compound of the invention are:
1. respiratory tract: obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) or adenovirus; or eosinophilic esophagitis;
2. bone and joints: arthritides associated with or including osteoarthritis/osteoarthrosis, both primary and secondary to, for example, congenital hip dysplasia; cervical and lumbar spondylitis, and low back and neck pain; osteoporosis; rheumatoid arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease; primary and secondary Sjogren's syndrome; systemic sclerosis and limited scleroderma; systemic lupus erythematosus, mixed connective tissue disease, and undifferentiated connective tissue disease; inflammatory myopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arteritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndrome, and Familial Hibernian Fever, Kikuchi disease; drug-induced arthalgias, tendonititides, and myopathies;
3. pain and connective tissue remodelling of musculoskeletal disorders due to injury [for example sports injury] or disease: arthritides (for example rheumatoid arthritis, osteoarthritis, gout or crystal arthropathy), other joint disease (such as intervertebral disc degeneration or temporomandibular joint degeneration), bone remodelling disease (such as osteoporosis, Paget's disease or osteonecrosis), polychondritits, scleroderma, mixed connective tissue disorder, spondyloarthropathies or periodontal disease (such as periodontitis);
4. skin: psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis;cutaneous lymphomas, non-melanoma skin cancer and other dysplastic lesions; drug-induced disorders including fixed drug eruptions;
5. eyes: blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral, fungal, and bacterial;
6. gastrointestinal tract: glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema);
7. abdominal: hepatitis, including autoimmune, alcoholic and viral; fibrosis and cirrhosis of the liver; cholecystitis; pancreatitis, both acute and chronic;
8. genitourinary: nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);
9. allograft rejection: acute and chronic following, for example, transplantation of kidney, heart, liver, lung, bone marrow, skin or cornea or following blood transfusion; or chronic graft versus host disease;
10. CNS: Alzheimer's disease and other dementing disorders including CJD and nvCJD; amyloidosis; multiple sclerosis and other demyelinating syndromes; cerebral atherosclerosis and vasculitis; temporal arteritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes;
11. other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;
12. other disorders with an inflammatory or immunological component; including acquired immune deficiency syndrome (AIDS), leprosy, Sezary syndrome, and paraneoplastic syndromes;
13. cardiovascular: atherosclerosis, affecting the coronary and peripheral circulation; pericarditis; myocarditis , inflammatory and auto-immune cardiomyopathies including myocardial sarcoid; ischaemic reperfusion injuries; endocarditis, valvulitis, and aortitis including infective (for example syphilitic); vasculitides; disorders of the proximal and peripheral veins including phlebitis and thrombosis, including deep vein thrombosis and complications of varicose veins;
14. oncology: treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumour recurrences, and paraneoplastic syndromes; or,
15. gastrointestinal tract: Coeliac disease, proctitis, eosinopilic gastro-enteritis, mastocytosis, Crohn's disease, ulcerative colitis, microscopic colitis, indeterminant colitis, irritable bowel disorder, irritable bowel syndrome, non-inflammatory diarrhea, food-related allergies which have effects remote from the gut, e.g., migraine, rhinitis and eczema.

According to a further feature of the present invention there is provided a method for treating a PDE 4 mediated disease state in a mammal, such as man, suffering from, or at risk of, said disease state, which comprises administering to a mammal in need of such treatment a therapeutically effective amount of a compound of the formula (I) or a pharmaceutically acceptable salt thereof.

The invention also provides a compound of the formula (I), or a pharmaceutically acceptable salt thereof, for use in therapy.

In another aspect the invention provides the use of a compound of formula (I), or a pharmaceutically acceptable salt thereof, in the manufacture of a medicament for use in therapy (for example modulating PDE 4 enzymatic activity).

The invention further provides the use of a compound of formula (I), or a pharmaceutically acceptable salt thereof, in the manufacture of a medicament for use in the treatment of:
1. respiratory tract: obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) or adenovirus; or eosinophilic esophagitis;
2. bone and joints: arthritides associated with or including osteoarthritis/osteoarthrosis, both primary and secondary to, for example, congenital hip dysplasia; cervical and lumbar spondylitis, and low back and neck pain; osteoporosis; rheumatoid arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease; primary and secondary Sjogren's syndrome; systemic sclerosis and limited scleroderma; systemic lupus erythematosus, mixed connective tissue disease, and undifferentiated connective tissue disease; inflammatory myopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arteritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndrome, and Familial Hibernian Fever, Kikuchi disease; drug-induced arthalgias, tendonititides, and myopathies;
3. pain and connective tissue remodelling of musculoskeletal disorders due to injury [for example sports injury] or disease: arthritides (for example rheumatoid arthritis, osteoarthritis, gout or crystal arthropathy), other joint disease (such as intervertebral disc degeneration or temporomandibular joint degeneration), bone remodelling disease (such as osteoporosis, Paget's disease or osteonecrosis), polychondritits, scleroderma, mixed connective tissue disorder, spondyloarthropathies or periodontal disease (such as periodontitis);
4. skin: psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis;cutaneous lymphomas, non-melanoma skin cancer and other dysplastic lesions; drug-induced disorders including fixed drug eruptions;
5. eyes: blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral, fungal, and bacterial;
6. gastrointestinal tract: glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema);
7. abdominal: hepatitis, including autoimmune, alcoholic and viral; fibrosis and cirrhosis of the liver; cholecystitis; pancreatitis, both acute and chronic;
8. genitourinary: nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);
9. allograft rejection: acute and chronic following, for example, transplantation of kidney, heart, liver, lung, bone marrow, skin or cornea or following blood transfusion; or chronic graft versus host disease;
10. CNS: Alzheimer's disease and other dementing disorders including CJD and nvCJD; amyloidosis; multiple sclerosis and other demyelinating syndromes; cerebral atherosclerosis and vasculitis; temporal arteritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes;
11. other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;
12. other disorders with an inflammatory or immunological component; including acquired immune deficiency syndrome (AIDS), leprosy, Sezary syndrome, and paraneoplastic syndromes;
13. cardiovascular: atherosclerosis, affecting the coronary and peripheral circulation; pericarditis; myocarditis , inflammatory and auto-immune cardiomyopathies including myocardial sarcoid; ischaemic reperfusion injuries; endocarditis, valvulitis, and aortitis including infective (for example syphilitic); vasculitides; disorders of the proximal and peripheral veins including phlebitis and thrombosis, including deep vein thrombosis and complications of varicose veins;
14. oncology: treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumour recurrences, and paraneoplastic syndromes; or,
15. gastrointestinal tract: Coeliac disease, proctitis, eosinopilic gastro-enteritis, mastocytosis, Crohn's disease, ulcerative colitis, microscopic colitis, indeterminant colitis, irritable bowel disorder, irritable bowel syndrome, non-inflammatory diarrhea, food-related allergies which have effects remote from the gut, e.g., migraine, rhinitis and eczema;
in a mammal (for example man).

In a further aspect the invention provides a compound of formula (I), or a pharmaceutically acceptable salt thereof, for use in the treatment of asthma {such as bronchial, allergic, intrinsic, extrinsic or dust asthma, particularly chronic or inveterate asthma (for example late asthma or airways hyper-responsiveness)}; or COPD.

In a still further aspect a compound of formula (I), or a pharmaceutically acceptable salt thereof, is useful in the treatment of COPD.

The present invention also provides the use of a compound of formula (I), or a pharmaceutically acceptable salt thereof, in the manufacture of a medicament for use in the treatment of asthma {such as bronchial, allergic, intrinsic, extrinsic or dust asthma, particularly chronic or inveterate asthma (for example late asthma or airways hyper-responsiveness)}; or COPD.

In order to use a compound of the invention, or a pharmaceutically acceptable salt thereof, for the therapeutic treatment of a mammal, such as man, said ingredient is normally formulated in accordance with standard pharmaceutical practice as a pharmaceutical composition. Therefore in another aspect the present invention provides a pharmaceutical composition which comprises a compound of the formula (I), or a pharmaceutically acceptable salt thereof (active ingredient), and a pharmaceutically acceptable adjuvant, diluent or carrier.

In a further aspect the present invention provides a process for the preparation of said composition which comprises mixing active ingredient with a pharmaceutically acceptable adjuvant, diluent or carrier. Depending on the mode of administration, the pharmaceutical composition will, for example, comprise from 0.05 to 99 %w (per cent by weight), such as from 0.05 to 80 %w, for example from 0.10 to 70 %w, such as from 0.10 to 50 %w, of active ingredient, all percentages by weight being based on total composition.

The pharmaceutical compositions of this invention may be administered in standard manner for the disease condition that it is desired to treat, for example by topical (such as to the lung and/or airways or to the skin), inhalation, oral, rectal or parenteral administration. For these purposes the compounds of this invention may be formulated by means known in the art. A suitable pharmaceutical composition of this invention is one suitable for oral administration in unit dosage form, for example a tablet or capsule which contains between 0.1 mg and 1 g of active ingredient.

Each patient may receive, for example, a dose of 0.001 mgkg⁻¹ to 100 mgkg⁻¹, for example in the range of 0.1 mgkg⁻¹ to 20 mgkg⁻¹, of the active ingredient administered, for example, 1 to 4 times per day.

The invention further relates to a combination therapy wherein a compound of the invention, or a pharmaceutically acceptable salt thereof, or a pharmaceutical composition or formulation comprising a compound of the invention, is administered concurrently or sequentially or as a combined preparation with another therapeutic agent or agents, for the treatment of one or more of the conditions listed.

In particular, for the treatment of the inflammatory diseases such as (but not restricted to) rheumatoid arthritis, osteoarthritis, asthma, allergic rhinitis, chronic obstructive pulmonary disease (COPD), psoriasis, and inflammatory bowel disease, the compounds of the invention may be combined with agents listed below.

Non-steroidal anti-inflammatory agents (hereinafter NSAIDs) including non-selective cyclo-oxygenase COX-1 / COX-2 inhibitors whether applied topically or systemically (such as piroxicam, diclofenac, propionic acids such as naproxen, flurbiprofen, fenoprofen, ketoprofen and ibuprofen, fenamates such as mefenamic acid, indomethacin, sulindac, azapropazone, pyrazolones such as phenylbutazone, salicylates such as aspirin); selective COX-2 inhibitors (such as meloxicam, celecoxib, rofecoxib, valdecoxib, lumarocoxib, parecoxib and etoricoxib); cyclo-oxygenase inhibiting nitric oxide donors (CINODs); glucocorticosteroids (whether administered by topical, oral, intramuscular, intravenous, or intra-articular routes); methotrexate; leflunomide; hydroxychloroquine; d-penicillamine; auranofin or other parenteral or oral gold preparations; analgesics; diacerein; intra-articular therapies such as hyaluronic acid derivatives; and nutritional supplements such as glucosamine.

The present invention still further relates to the combination of a compound of the invention, or a pharmaceutically acceptable salt thereof, together with a cytokine or agonist or antagonist of cytokine function, (including agents which act on cytokine signalling pathways such as modulators of the SOCS system) including alpha-, beta-, and gamma-interferons; insulin-like growth factor type I (IGF-1); interleukins (IL) including IL1 to 17, and interleukin antagonists or inhibitors such as anakinra; tumour necrosis factor alpha (TNF-α) inhibitors such as anti-TNF monoclonal antibodies (for example infliximab; adalimumab, and CDP-870) and TNF receptor antagonists including immunoglobulin molecules (such as etanercept) and low-molecular-weight agents such as pentoxyfylline.

In addition the invention relates to a combination of a compound of the invention, or a pharmaceutically acceptable salt thereof, with a monoclonal antibody targeting B-Lymphocytes (such as CD20 (rituximab), MRA-aIL16R and T-Lymphocytes, CTLA4-Ig, HuMax Il-15).

The present invention still further relates to the combination of a compound of the invention, or a pharmaceutically acceptable salt thereof, with a modulator of chemokine receptor function such as an antagonist of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family.

The present invention further relates to the combination of a compound of the invention, or a pharmaceutically acceptable salt thereof, with an inhibitor of matrix metalloprotease (MMPs), i.e., the stromelysins, the collagenases, and the gelatinases, as well as aggrecanase; for example collagenase-1 (MMP-1), collagenase-2 (MMP-8), collagenase-3 (MMP-13), stromelysin-1 (MMP-3), stromelysin-2 (MMP-10), and stromelysin-3 (MMP-11) and MMP-9 and MMP-12, including agents such as doxycycline.

The present invention still further relates to the combination of a compound of the invention, or a pharmaceutically acceptable salt thereof, and a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; a N-(5-substituted)-thiophene-2-alkylsulfonamide; 2,6-di-tert-butylphenolhydrazones; a methoxytetrahydropyrans such as Zeneca ZD-2138; the compound SB-210661; a pyridinyl-substituted 2-cyanonaphthalene compound such as L-739,010; a 2-cyanoquinoline compound such as L-746,530; or an indole or quinoline compound such as MK-591, MK-886, and BAY x 1005.

The present invention further relates to the combination of a compound of the invention, or a pharmaceutically acceptable salt thereof, and a receptor antagonist for leukotrienes (LT) B4, LTC4, LTD4, and LTE4. selected from the group consisting of the phenothiazin-3-yls such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention, or a pharmaceutically acceptable salt thereof, and a phosphodiesterase (PDE) inhibitor such as a methylxanthanine including theophylline and aminophylline; a selective PDE isoenzyme inhibitor including a PDE4 inhibitor an inhibitor of the isoform PDE4D, or an inhibitor of PDE5.

The present invention further relates to the combination of a compound of the invention, or a pharmaceutically acceptable salt thereof, and a histamine type 1 receptor antagonist such as cetirizine, loratadine, desloratadine, fexofenadine, acrivastine, terfenadine, astemizole, azelastine, levocabastine, chlorpheniramine, promethazine, cyclizine, or mizolastine; applied orally, topically or parenterally.

The present invention still further relates to the combination of a compound of the invention, or a pharmaceutically acceptable salt thereof, and a proton pump inhibitor (such as omeprazole) or a gastroprotective histamine type 2 receptor antagonist.

The present invention further relates to the combination of a compound of the invention, or a pharmaceutically acceptable salt thereof, and an antagonist of the histamine type 4 receptor.

The present invention still further relates to the combination of a compound of the invention, or a pharmaceutically acceptable salt thereof, and an alpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride or ethylnorepinephrine hydrochloride.

The present invention further relates to the combination of a compound of the invention, or a pharmaceutically acceptable salt thereof, and an anticholinergic agent including muscarinic receptor (Ml, M2, and M3) antagonist such as atropine, hyoscine, glycopyrrrolate, ipratropium bromide, tiotropium bromide, oxitropium bromide, pirenzepine or telenzepine.

The present invention still further relates to the combination of a compound of the invention, or a pharmaceutically acceptable salt thereof, and a beta-adrenoceptor agonist (including beta receptor subtypes 1-4) such as isoprenaline, salbutamol, formoterol, salmeterol, terbutaline, orciprenaline, bitolterol mesylate, indacaterol, or pirbuterol, or a chiral enantiomer thereof.

The present invention further relates to the combination of a compound of the invention, or a pharmaceutically acceptable salt thereof, and a chromone, such as sodium cromoglycate or nedocromil sodium.

The present invention still further relates to the combination of a compound of the invention, or a pharmaceutically acceptable salt thereof, with a glucocorticoid, such as flunisolide, triamcinolone acetonide, beclomethasone dipropionate, budesonide, fluticasone propionate, ciclesonide or mometasone furoate.

The present invention further relates to the combination of a compound of the invention, or a pharmaceutically acceptable salt thereof, with an agent that modulates a nuclear hormone receptor such as PPARs.

The present invention still further relates to the combination of a compound of the invention, or a pharmaceutically acceptable salt thereof, together with an immunoglobulin (Ig) or Ig preparation or an antagonist or antibody modulating Ig function such as anti-IgE (for example omalizumab).

The present invention further relates to the combination of a compound of the invention, or a pharmaceutically acceptable salt thereof, and another systemic or topically-applied anti-inflammatory agent, such as thalidomide or a derivative thereof, a retinoid, dithranol or calcipotriol.

The present invention still further relates to the combination of a compound of the invention, or a pharmaceutically acceptable salt thereof, and combinations of aminosalicylates and sulfapyridine such as sulfasalazine, mesalazine, balsalazide, and olsalazine; and immunomodulatory agents such as the thiopurines, and corticosteroids such as budesonide.

The present invention further relates to the combination of a compound of the invention, or a pharmaceutically acceptable salt thereof, together with an antibacterial agent such as a penicillin derivative, a tetracycline, a macrolide, a beta-lactam, a fluoroquinolone, metronidazole, an inhaled aminoglycoside; an antiviral agent including acyclovir, famciclovir, valaciclovir, ganciclovir, cidofovir, amantadine, rimantadine, ribavirin, zanamavir and oseltamavir; a protease inhibitor such as indinavir, nelfinavir, ritonavir, and saquinavir; a nucleoside reverse transcriptase inhibitor such as didanosine, lamivudine, stavudine, zalcitabine or zidovudine; or a non-nucleoside reverse transcriptase inhibitor such as nevirapine or efavirenz.

The present invention still further relates to the combination of a compound of the invention, or a pharmaceutically acceptable salt thereof, and a cardiovascular agent such as a calcium channel blocker, a beta-adrenoceptor blocker, an angiotensin-converting enzyme (ACE) inhibitor, an angiotensin-2 receptor antagonist; a lipid lowering agent such as a statin or a fibrate; a modulator of blood cell morphology such as pentoxyfylline; thrombolytic, or an anticoagulant such as a platelet aggregation inhibitor.

The present invention further relates to the combination of a compound of the invention, or a pharmaceutically acceptable salt thereof, and a CNS agent such as an antidepressant (such as sertraline), an anti-Parkinsonian drug (such as deprenyl, L-dopa, ropinirole, pramipexole, a MAOB inhibitor such as selegine and rasagiline, a comP inhibitor such as tasmar, an A-2 inhibitor, a dopamine reuptake inhibitor, an NMDA antagonist, a nicotine agonist, a dopamine agonist or an inhibitor of neuronal nitric oxide synthase), or an anti-Alzheimer's drug such as donepezil, rivastigmine, tacrine, a COX-2 inhibitor, propentofylline or metrifonate.

The present invention still further relates to the combination of a compound of the invention, or a pharmaceutically acceptable salt thereof, and an agent for the treatment of acute or chronic pain, such as a centrally or peripherally-acting analgesic (for example an opioid or derivative thereof), carbamazepine, phenytoin, sodium valproate, amitryptiline or other anti-depressant agents, paracetamol, or a non-steroidal anti-inflammatory agent.

The present invention further relates to the combination of a compound of the invention, or a pharmaceutically acceptable salt thereof, together with a parenterally or topically-applied (including inhaled) local anaesthetic agent such as lignocaine or a derivative thereof.

A compound of the present invention, or a pharmaceutically acceptable salt thereof, can also be used in combination with an anti-osteoporosis agent including a hormonal agent such as raloxifene, or a biphosphonate such as alendronate.

The present invention still further relates to the combination of a compound of the invention, or a pharmaceutically acceptable salt thereof, together with a: (i) tryptase inhibitor; (ii) platelet activating factor (PAF) antagonist; (iii) interleukin converting enzyme (ICE) inhibitor; (iv) IMPDH inhibitor; (v) adhesion molecule inhibitors including VLA-4 antagonist; (vi) cathepsin; (vii) kinase inhibitor such as an inhibitor of tyrosine kinase (such as Btk, Itk, Jak3 or MAP, for example Gefitinib or Imatinib mesylate), a serine / threonine kinase (such as an inhibitor of a MAP kinase such as p38, JNK, protein kinase A, B or C, or IKK), or a kinase involved in cell cycle regulation (such as a cylin dependent kinase); (viii) glucose-6 phosphate dehydrogenase inhibitor; (ix) kinin-B.subl. - or B.sub2. -receptor antagonist; (x) anti-gout agent, for example colchicine; (xi) xanthine oxidase inhibitor, for example allopurinol; (xii) uricosuric agent, for example probenecid, sulfinpyrazone or benzbromarone; (xiii) growth hormone secretagogue; (xiv) transforming growth factor (TGFβ); (xv) platelet-derived growth factor (PDGF); (xvi) fibroblast growth factor for example basic fibroblast growth factor (bFGF); (xvii) granulocyte macrophage colony stimulating factor (GM-CSF); (xviii) capsaicin cream; (xix) tachykinin NK.sub1. or NK.sub3. receptor antagonist such as NKP-608C, SB-233412 (talnetant) or D-4418; (xx) elastase inhibitor such as UT-77 or ZD-0892; (xxi) TNF-alpha converting enzyme inhibitor (TACE); (xxii) induced nitric oxide synthase (iNOS) inhibitor; (xxiii) chemoattractant receptor-homologous molecule expressed on TH2 cells, (such as a CRTH2 antagonist); (xxiv) inhibitor of p38; (xxv) agent modulating the function of Toll-like receptors (TLR), (xxvi) agent modulating the activity of purinergic receptors such as P2X7; (xxvii) inhibitor of transcription factor activation such as NFkB, API, or STATS; or (xxviii) a non-steroidal glucocorticoid receptor (GR-receptor) agonist.

In a further embodiment the present invention provides a pharmaceutical product comprising, in combination, a first active ingredient which is a compound of formula (I), or a pharmaceutically acceptable salt thereof, as hereinbefore described, and at least one further active ingredient selected from:-
- a β2. adrenoceptor agonist,
- a modulator of chemokine receptor function,
- an inhibitor of kinase function,
- a protease inhibitor,
- a steroidal glucocorticoid receptor agonist,
- an anticholinergic agent, and a
- a non-steroidal glucocorticoid receptor agonist.

The pharmaceutical product according to this embodiment may, for example, be a pharmaceutical composition comprising the first and further active ingredients in admixture. Alternatively, the pharmaceutical product may, for example, comprise the first and further active ingredients in separate pharmaceutical preparations suitable for simultaneous, sequential or separate administration to a patient in need thereof.
The pharmaceutical product of this embodiment is of particular use in treating respiratory diseases such as asthma, COPD or rhinitis.

Examples of a β₂-adrenoceptor agonist that may be used in the pharmaceutical product according to this embodiment include metaproterenol, isoproterenol, isoprenaline, albuterol, salbutamol (e.g. as sulphate), formoterol (e.g. as fumarate), salmeterol (e.g. as xinafoate), terbutaline, orciprenaline, bitolterol (e.g. as mesylate), pirbuterol or indacaterol. The β₂-adrenoceptor agonist of this embodiment may be a long-acting β₂-agonists, for example salmeterol (e.g. as xinafoate), formoterol (e.g. as fumarate), bambuterol (e.g. as hydrochloride), carmoterol (TA 2005, chemically identified as 2(1H)-Quinolone, 8-hydroxy-5-[1-hydroxy-2-[[2-(4-methoxy-phenyl)-1-methylethyl]-amino]ethyl]-monohydrochloride, [R-(R*,R*)] also identified by Chemical Abstract Service Registry Number 137888-11-0 and disclosed in U.S. Patent No 4,579,854), indacaterol (CAS no 312753-06-3; QAB-149), formanilide derivatives e.g. 3-(4-{[6-({(2R)-2-[3-(formylamino)-4-hydroxyphenyl]-2-hydroxyethyl}amino)hexyl]oxy}-butyl)-benzenesulfonamide as disclosed in WO 2002/76933, benzenesulfonamide derivatives e.g. 3-(4-{[6-({(2R)-2-hydroxy-2-[4-hydroxy-3-(hydroxy-methyl)phenyl]ethyl)amino)-hexyl]oxy]butyl)benzenesulfonamide as disclosed in WO 2002/88167, aryl aniline receptor agonists as disclosed in WO 2003/042164 and WO 2005/025555, indole derivatives as disclosed in WO 2004/032921 and US 2005/222144, and compounds GSK 159797, GSK 159802, GSK 597901, GSK 642444 and GSK 678007.

Examples of a modulator of chemokine receptor function that may be used in the pharmaceutical product according to this embodiment include a CCR1 receptor antagonist.

Examples of an inhibitor of kinase function that may be used in the pharmaceutical product according to this embodiment include a p38 kinase inhibitor and an IKK inhibitor.

Examples of a protease inhibitor that may be used in the pharmaceutical product according to this embodiment include an inhibitor of neutrophil elastase or an inhibitor of MMP12.

Examples of a steroidal glucocorticoid receptor agonist that may be used in the pharmaceutical product according to this embodiment include budesonide, fluticasone (e.g. as propionate ester), mometasone (e.g. as furoate ester), beclomethasone (e.g. as 17-propionate or 17,21-dipropionate esters), ciclesonide, loteprednol (as e.g. etabonate), etiprednol (as e.g. dicloacetate), triamcinolone (e.g. as acetonide), flunisolide, zoticasone, flumoxonide, rofleponide, butixocort (e.g. as propionate ester), prednisolone, prednisone, tipredane, steroid esters e.g. 6α,9α-difluoro-17α-[(2-furanylcarbonyl)oxy]-11β-hydroxy-16α-methyl-3-oxo-androsta-1,4-diene-17β-carbothioic acid S-fluoromethyl ester, 6α,9α-difluoro-11β-hydroxy-16α-methyl-3-oxo-17α-propionyloxy-androsta-1,4-diene-17β-carbothioic acid S-(2-oxo-tetrahydro-furan-3S-yl) ester and 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester, steroid esters according to DE 4129535, steroids according to WO 2002/00679, WO 2005/041980, or steroids GSK 870086, GSK 685698 and GSK 799943.

Examples of an anticholinergic agent that may be used in the pharmaceutical product according to this embodiment include for example a muscarinic receptor antagonist (for example a M1, M2 or M3 antagonist, such as a M3 antagonist) for example ipratropium (e.g. as bromide), tiotropium (e.g. as bromide), oxitropium (e.g. as bromide), tolterodine, pirenzepine, telenzepine, glycopyrronium bromide (such as R,R-glycopyrronium bromide or a mixture of R,S- and S,R-glycopyrronium bromide); mepensolate (e.g. as bromide), a quinuclidine derivative such as 3(R)-(2-hydroxy-2,2-dithien-2-ylacetoxy)-1-(3-phenoxypropyl)-1-azonia-bicyclo[2.2.2]octane bromide as disclosed in US 2003/0055080, quinuclidine derivatives as disclosed in WO 2003/087096 and WO 2005/115467 and DE 10050995; or GSK 656398 or GSK 961081.

Examples of a modulator of a non-steroidal glucocorticoid receptor agonist that may be used in the pharmaceutical product according to this embodiment include those described in WO2006/046916.

A compound of the invention, or a pharmaceutically acceptable salt thereof, can also be used in combination with an existing therapeutic agent for the treatment of cancer, for example suitable agents include:
(i) an antiproliferative/antineoplastic drug or a combination thereof, as used in medical oncology, such as an alkylating agent (for example cis-platin, carboplatin, cyclophosphamide, nitrogen mustard, melphalan, chlorambucil, busulphan or a nitrosourea); an antimetabolite (for example an antifolate such as a fluoropyrimidine like 5-fluorouracil or tegafur, raltitrexed, methotrexate, cytosine arabinoside, hydroxyurea, gemcitabine or paclitaxel); an antitumour antibiotic (for example an anthracycline such as adriamycin, bleomycin, doxorubicin, daunomycin, epirubicin, idarubicin, mitomycin-C, dactinomycin or mithramycin); an antimitotic agent (for example a vinca alkaloid such as vincristine, vinblastine, vindesine or vinorelbine, or a taxoid such as taxol or taxotere); or a topoisomerase inhibitor (for example an epipodophyllotoxin such as etoposide, teniposide, amsacrine, topotecan or a camptothecin);
(ii) a cytostatic agent such as an antioestrogen (for example tamoxifen, toremifene, raloxifene, droloxifene or iodoxyfene), an oestrogen receptor down regulator (for example fulvestrant), an antiandrogen (for example bicalutamide, flutamide, nilutamide or cyproterone acetate), a LHRH antagonist or LHRH agonist (for example goserelin, leuprorelin or buserelin), a progestogen (for example megestrol acetate), an aromatase inhibitor (for example as anastrozole, letrozole, vorazole or exemestane) or an inhibitor of 5α-reductase such as finasteride;
(iii) an agent which inhibits cancer cell invasion (for example a metalloproteinase inhibitor like marimastat or an inhibitor of urokinase plasminogen activator receptor function);
(iv) an inhibitor of growth factor function, for example: a growth factor antibody (for example the anti-erb b2 antibody trastuzumab, or the anti-erb b1 antibody cetuximab [C225]), a farnesyl transferase inhibitor, a tyrosine kinase inhibitor or a serine/threonine kinase inhibitor, an inhibitor of the epidermal growth factor family (for example an EGFR family tyrosine kinase inhibitor such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) or 6-acrylamido-N-(3-chloro-4-fluorophenyl)-7-(3-morpholinopropoxy)quinazolin-4-amine (CI 1033)), an inhibitor of the platelet-derived growth factor family, or an inhibitor of the hepatocyte growth factor family;
(v) an antiangiogenic agent such as one which inhibits the effects of vascular endothelial growth factor (for example the anti-vascular endothelial cell growth factor antibody bevacizumab, a compound disclosed in WO 97/22596, WO 97/30035, WO 97/32856 or WO 98/13354), or a compound that works by another mechanism (for example linomide, an inhibitor of integrin αvβ3 function or an angiostatin);
(vi) a vascular damaging agent such as combretastatin A4, or a compound disclosed in WO 99/02166, WO 00/40529, WO 00/41669, WO 01/92224, WO 02/04434 or WO 02/08213;
(vii) an agent used in antisense therapy, for example one directed to one of the targets listed above, such as ISIS 2503, an anti-ras antisense;
(viii) an agent used in a gene therapy approach, for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; or,
(ix) an agent used in an immunotherapeutic approach, for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The invention will now be illustrated by the following non-limiting Examples in which, unless stated otherwise:
(i) when given, ¹H NMR data is quoted and is in the form of delta values for major diagnostic protons, given in parts per million (ppm) relative to tetramethylsilane (TMS) as an internal standard, determined at 300MHz or 400MHz using perdeuterio DMSO-D6 (CD₃SOCD₃) or CDCl₃ as the solvent unless otherwise stated;
(ii) mass spectra (MS) were run with an electron energy of 70 electron volts in the chemical ionisation (CI) mode using a direct exposure probe. Where indicated ionisation was effected by electrospray ionisation (ES), or atmospheric pressure chemical ionisation (APCI), or multimode ionisation, a combination of ES ionisation and APCI. Where values for m/z are given, generally only ions which indicate the parent mass are reported, and the mass ions quoted are the positive or negative mass ions: [M]⁺, [M+H]⁺ or [M-H]⁻;
(iii) The title and sub-title compounds of the examples and methods were named using the index name program from Advanced Chemistry Development Inc, version 8.00 or were named using the IUPAC name program from Openeye and stereochemical descriptors added by hand. (See www.eyesopen.com/products/applications/ogham.html)
(iv) Unless stated otherwise, reverse phase HPLC was conducted using a Symmetry^{™}, NovaPak^{™} or Xterra^{™}, Sunfire , x-bridge reverse phase silica column, all available from Waters Corp.
(v) the following abbreviations are used:

| | |
|---|---|
| DMF | *N,N-*Dimethylformamide |
| DME | 1,2-Dimethoxyethane |
| NMP | 1-*N*-Methyl-2-pyrrolidinone |
| HOAT | 1-Hydroxy-7-azabenzotriazole |
| DIEA | *N*,*N*-Diisopropylethylamine |
| HATU | *O*-(7-Benzotriazol-1-yl)-*N*,*N*,*N*',*N*'-tetramethyluronium hexafluorophosphate |
| THF | Tetrahydrofuran |
| DCM | Dichloromethane |
| BOC | *tert*-butoxycarbonyl |
| HPLC | High pressure liquid chromatography |
| d | Day(s) |
| h | Hour(s) |
| min | Minute(s) |
| DMSO | Dimethylsulfoxide |
| EtOAc | Ethyl acetate |

(vi) Chem elut cartridges are available from Varian Inc (http://www.varianinc.com/) or Kinesis (http://www.kinesis.co.uk/)
(vii) Gemini columns are available from Phenomenex (http://www.phenomenex.com)

The starting materials for the Examples below are either commercially available or readily prepared by standard methods from known starting materials (The compounds are named using the IUPAC name program from Advanced Chemistry Development Inc, version 8.00)

### Example 1

### N-[cis-4-(1-biphenyl-3-yl-6-fluoro-2,4-dioxo-1,4-dihydropyrido[2,3-d]pyrimidin-3(2H)-yl)cyclohexyl]-6-fluoroimidazo[1,2-a]pyridine-2-carboxamide

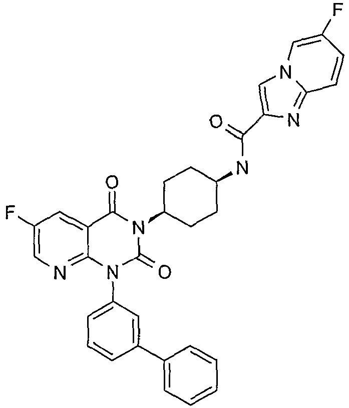

### Step (a) 2-(biphenyl-3-ylamino)-5-fluoronicotinic acid

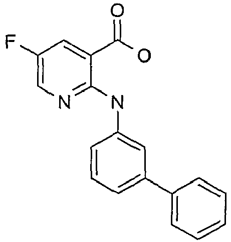

2-Chloro-5-fluoronicotinic acid (1.83 g, 10.45 mmol) and potassium carbonate (1.74 g, 12.6 mmol) were added to dry DMF (20 ml). Copper (41 mg), copper(I)bromide (75 mg) and 3-aminobiphenyl (3.0 g, 17.8 mmol) were added and the reaction mixture was stirred at 150 °C for 12 hours. 1M HCl was added and the product that precipitated was collected by filtration, washed with 1M HCl then water and dried to afford the sub-title compound (1.98 g, 62%).
APCI (-ve): 307 (M-H)

### Step (b) tert-butyl [cis-4-(1-biphenyl-3-yl-6-fluoro-2,4-dioxo-1,4-dihydropyrido[2,3-d]pyrimidin-3 (2H)-yl)cyclohexyl] carbamate

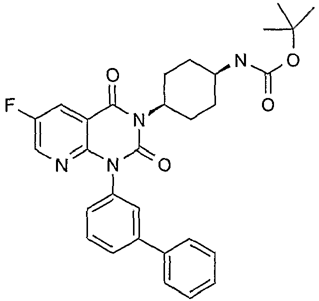

2-(Biphenyl-3-ylamino)-5-fluoronicotinic acid (1.6 g, 5.2 mmol) was dissolved in dry DMF (20 ml) and DIEA (4 ml, 23 mmol) was added, followed by HATU (1.97 g, 5.2 mmol), and the mixture allowed to stir at room temperature for 10 min. tert-Butyl (cis-4-aminocyclohexyl)carbamate (945 mg, 4.39 mmol) was added and the mixture allowed to stir at room temperature overnight. The mixturewas quenched by pouring into water and the product that precipitated was collected by filtration, then purified by flash chromatography on silica using ethyl acetate:isohexane (1:2) as the eluent to give the intermediate (2.0g). This intermediate (1.8 g, 3.6 mmol) was dissolved in dry NMP (10 ml) and carbonyldiimidazole (1.83 g, 11.3 mmol) was added, followed by 60% sodium hydride in mineral oil (450 mg, 11.25 mmol) portion-wise over 5 min. The mixture was then heated to 70 °C for 15 min. Mixture allowed to cool to room temperature then poured carefully into water and the solid that precipitated was collected by filtration, then purified using flash chromatography on silica using ethyl acetate isohexane (1:3) as eluent to afford the sub-title compound (0.98 g, 36%).
¹H NMR (300 MHz, DMSO-*d₆*) δ 8.59 (d, 1H), 8.29 (m, 1H), 7.56 (m, 9H), 6.50 (s, 1H), 4.77 (t, 1H), 3.56 (s, 1H), 2.58 (t, 2H), 1.91 (d, 2H), 1.42 (m, 13H)

### Step (c) 3-(cis-4-aminocyclohexyl)-1-biphenyl-3-yl-6-fluoropyrido[2,3-d]pyrimidine-2,4(1H,3H)-dione

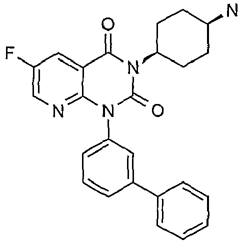

4.0M hydrogen chloride in dioxane (20 ml) was added to [4-(1-Biphenyl-3-yl-6-fluoro-2,4-dioxo-1,4-dihydro-2H-pyrido[2,3-d]pyrimidin-3-yl)-cyclohexyl]-carbamic acid tert-butyl ester (830mg, 1.56 mmol) and the mxiture allowed to stir at room temperature for 2h. The mixture was evaporated to dryness and the residue triturated with acetonitrile to give the sub-title compound (607 mg, 90%).
¹H NMR (400 MHz, DMSO-*d₆*) δ 8.59 (t, 1H), 8.30 (dt, 1H), 7.57 (m, 9H), 4.75 (t, 1H), 3.06 (s, 1H), 2.72 (q, 2H), 1.53 (m, 8H)
APCI (Multimode) m/z: 431 [M+H]

Step (d) N-[cis-4-(1-biphenyl-3-yl-6-fluoro-2,4-dioxo-1,4-dihydropyrido[2,3-d]pyrimidin-3(2H)-yl)cyclohexyl]-6-fluoroimidazo[1,2-a]pyridine-2-carboxamide 6-fluoro-imidazole[1,2a]pyridine-2-carboxylic acid (80 mg, 0.43 mmol) was dissolved in dry DMF (5 ml) and DIEA (0.2 ml, 1.15 mmol) was added, followed by HATU (167 mg, 0.43 mmol) and the mixture stirred for 10 min. 3-(cis-4-aminocyclohexyl)-l-biphenyl-3-yl-6-fluoropyrido[2,3-d]pyrimidine-2,4(1H,3H)-dione (185 mg, 0.43 mmol) was added and the mixture stirred at room temperature overnight. The mixture was poured into water and the solid that precipitated was collected by filtration, then purified by reverse phase HPLC (25-95% acetonitrile in aqueous ammonia) to afford the title compound (117 mg, 46%). ¹H NMR (300 MHz, DMSO-*d₆*) δ 8.79 (s, 1H), 8.60 (s, 1H), 8.33 (m, 2H), 7.58 (m, 10H), 4.88 (t, 1H), 4.17 (s, 1H), 2.63 (m, 2H), 1.67 (m, 4H), 2.01 (d, 2H)
APCI (Multimode) m/z: 593 [M+H]

### Example 2

### N-[cis-4-(1-biphenyl-3-yl-6-fluoro-2,4-dioxo-1,4-dihydropyrido[2,3-d]pyrimidin-3(2H)-yl)cyclohexyl]-6-(1-methylpyrrolidin-2-yl)imidazo[1,2-a]pyridine-2-carboxamide.

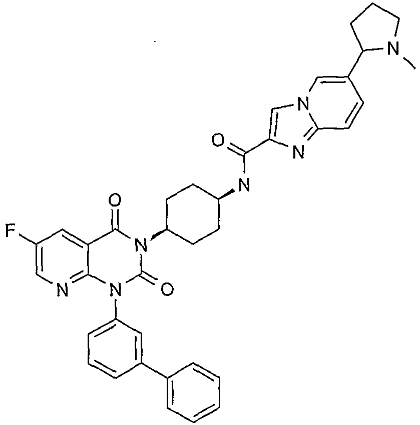

### Step (a) 6-(1-methylpyrrolidin-2-yl)imidazo[1,2-a]pyridine-2-carboxylic acid.

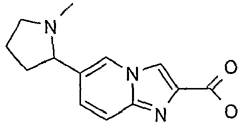

A mixture of 5-(1-Methyl-pyrrolidin-2-yl)-pyridin-2-ylamine (600 mg, 3 mmol), and ethylbromo pyruvate (1.17 g, 6.0 mmol) in ethanol (20 ml) was heated at 70 °C for 2h. The mixture was evaporated to dryness and the residue triturated with acetonitrile (10 ml). The crude ester was collected by filtration and then dissolved in a mixture of water/dioxan (1:1) (20 ml). To this solution was added lithium hydroxide monohydrate (0.3 g, 7.14 mmol) and the mixture stirred at room temperature overnight. The mixture was evaporated to dryness and the residue taken up into water (20 ml) and the pH was adjusted to 4-5 by the addition of acetic acid. The solid was then collected by filtration and dried to afford the sub-title compound (310 mg, 42%).
¹H NMR (400 MHz, DMSO-d₆) δ 8.46 (s, 1H), 8.35 (s, 1H), 7.55 (d, 1H), 7.29 (dd, 1H), 3.12 (m, 2H), 2.50 (m, 3H), 2.27 (q, 1H), 2.17 (m, 1H), 1.75 (m, 3H)

Step (b) N-[cis-4-(1-biphenyl-3-yl-6-fluoro-2,4-dioxo-1,4-dihydropyrido[2,3-d]pyrimidin-3(2H)-yl)cyclohexyl]-6-(1-methylpyrrolidin-2-yl)imidazo[1,2-a]pyridine-2-carboxamide 6-(1-methylpyrrolidin-2-yl)imidazo[1,2-a]pyridine-2-carboxylic acid. (105 mg, 0.43 mmol) was dissolved in dry DMF (5 ml) and DIEA (0.2 ml, 1.15 mmol) was added, followed by HATU (167 mg, 0.43 mmol) and the mixture stirred for 10 min. 3-(cis-4-aminocyclohexyl)-1-biphenyl-3-yl-6-fluoropyrido[2,3-d]pyrimidine-2,4(1H,3H)-dione (185 mg, 0.43 mmol) was added and the mixture stirred at room temperature overnight. The mixture was poured into water and the solid that precipitated was collected by filtration, then purified by reverse phase HPLC (25-95% acetonitrile in aqueous ammonia) to afford the title compound (49 mg, 17.3%).
¹H NMR (300 MHz, DMSO-*d₆*) δ 8.60 (dd, 1H), 8.49 (s, 1H), 8.32 (m, 2H), 7.54 (m, 13H), 7.54 (m, 1H), 4.89 (m, 1H), 4.18 (s, 1H), 3.13 (dt, 2H), 2.65 (m, 2H), 2.27 (m, 1H), 2.14 (s, 3H), 1.82 (m, 8H)
APCI (Multimode) m/z: 658 [M+H]

### Example 3

### N-[cis-4-(1-biphenyl-3-yl-6-fluoro-2,4-dioxo-1,4-dihydropyrido[2,3-d]pyrimidin-3(2H)-yl)cyclohexyl]imidazo[1,2-a]pyridine-2-carboxamide.

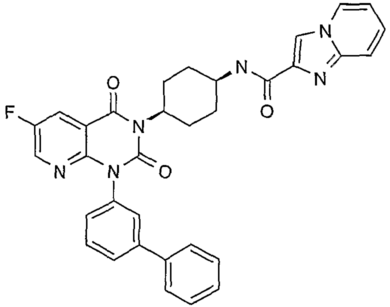

### Step (a) tert-butyl {cis-4-[(imidazo[1,2-a]pyridin-2-ylcarbonyl)amino] cyclohexyl} carbamate

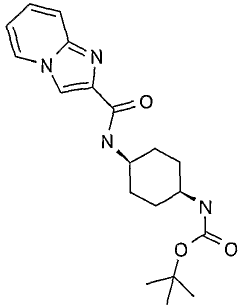

Imidazo[1,2a]pyridine-2-carboxyli acid (5 g, 30.8 mmol) was dissolved in NMP (200 ml) and DIEA (11.96 g, 16.43 ml) was added, followed by O-(7-Azabezotriazol-1-yl)-N,N,N',N'-tetramethyluronium hexafluorophosphate (14.07 g, 37 mmol). The reaction was stirred for 5 min. (4-Amino-cyclohexyl)-carbamic acid tert-butyl ester (6.61 g, 30.8 mmol) was then added and the solution stirred at room temperature ovenight. Water was added and the product extracted with ethyl acetate. The organic phase was dried (anhydrous magnesium sulphate), filtered and concentrated in vacuo to afford the sub-title compound (8.5 g, contains some NMP). This was used without further purification.
APCI (+ve) m/z: 359 [M+H]

### Step (b) N-(cis-4-aminocyclohexyl)imidazo[1,2-a]pyridine-2-carboxamide

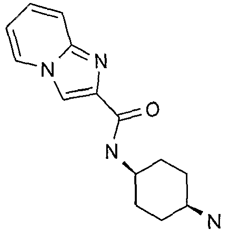

To tert-butyl {cis-4-[(imidazo[1,2-a]pyridin-2-ylcarbonyl)amino]cyclohexyl}carbamate was added HCl (4M in 1,4-Dioxane) (30 ml) and the reaction stirred at room temperature overnight. Solvents were evaporated. This was then purified by using Flash SCX using Methanol as eluent followed by MeOH/10% aq NH₃ as eluent to furnish the pure sub-title compound (3.2g, 40%) as a gum.
¹H NMR (400 MHz, DMSO-d₆) δ 8.59 (dt, 1H), 8.39 (d, 1H), 7.75 (d, 1H), 7.61 (dd, 1H), 7.35 (ddd, 1H), 6.99 (td, 1H), 3.74 - 3.96 (m, 3H), 2.98 (s, 1H), 1.52 - 1.87 (m, 8H) APCI (+ve) m/z: 259 [M+H]

### Step (c) N-[cis-4-({[2-(biphenyl-3-ylamino)-5-fluoropyridin-3-yl]carbonyl}amino)cyclohexyl]imidazo[1,2-a]pyridine-2-carboxamide

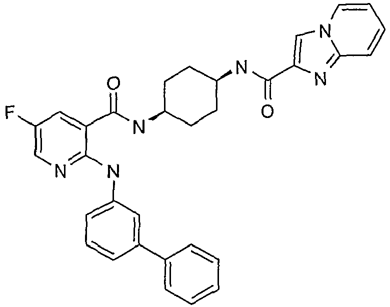

To 2-(biphenyl-3-ylamino)-5-fluoronicotinic acid (0.298g, 0.97 mmol) was added DMF (2 ml), N-(cis-4-aminocyclohexyl)imidazo[1,2-a]pyridine-2-carboxamide (0.2498 g, 0.97 mmol), DIPEA (0.345 g, 0.441 ml) followed by O-(7-Azabezotriazol-1-yl)-N,N,N',N'-tetramethyluronium hexafluorophosphate (0.404 g, 1.06 mmol). The reaction was stirred at room temperature for 3 hours. Water was added and the product extracted with Ethyl acetate, and DCM. The organic phase was dried with anhydrous sodium sulphate, filtered and concentrated in vacuo to give the crude compound. This was then purified by reverse phase HPLC (35-45% acetonitrile in aqueous ammonia). This gave the sub-title compound (0.126 g, 24%).
¹H NMR (400 MHz, CDCl₃) δ 8.25 (d, 1H), 8.16 - 8.14 (m, 2H), 7.82 (t, 1H), 7.70 - 7.68 (m, 1H), 7.63 - 7.57 (m, 3H), 7.50 - 7.32 (m, 7H), 7.28 - 7.24 (m, 2H), 6.87 (t, 1H), 6.13 (d, 1H), 4.20 (d, 2H), 1.78 - 1.99 (m, 8H)
APCI (Multimode) m/z: 549 [M+H]

### Step (d) N-[cis-4-(1-biphenyl-3-yl-6-fluoro-2,4-dioxo-1,4-dihydropyrido[2,3-d]pyrimidin-3(2H)-yl)cyclohexyl]imidazo[1,2-a]pyridine-2-carboxamide

To a solution of N-[cis-4-({[2-(biphenyl-3-ylamino)-5-fluoropyridin-3-yl]carbonyl}amino)cyclohexyl]imidazo[1,2-a]pyridine-2-carboxamide (0.05 g) and 1,1'-carbonyldimidazole (0.0517 g) in NMP (2 ml) was added sodium hydride (in mineral oil) (0.01093 g). This mixture was left to stir for 12h at room temperature. Water was added and the product extracted with ethyl acetate. The organic phase was dried with anhydrous sodium sulphate, filtered and concentrated in vacuo to give the crude compound. This was then purified by reverse phase HPLC (25-75% acetonitrile in aqueous ammonia). This gave the title compound (0.020 g, 38%).
¹H NMR (400 MHz, CDCl₃) δ 8.37 (s, 1H), 8.25 - 8.22 (m, 1H), 8.11 (d, 2H), 7.79 - 7.30 (m, 11H), 7.22 - 7.18 (m, 1H), 6.82 (dd, 1H), 5.10 - 5.02 (m, 1H), 4.45 - 4.43 (m, 1H), 2.75 - 2.87 (m, 2H), 2.14 (d, 2H), 1.72 - 1.85 (m, 4H)
APCI (Multimode) m/z: 575 [M+H]

### Example 4

### N-{cis-4-[1-[3-(anilinocarbonyl)phenyl]-6-fluoro-2,4-dioxo-1,4-dihydropyrido[2,3-d]pyrimidin-3(2H)-yl]cyclohexyl}imidazo[1,2-a]pyridine-2-carboxamide.

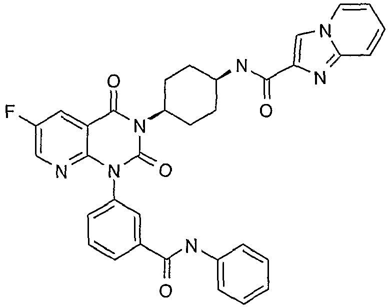

### Step (a) methyl 3-[3-(cis-4-aminocyclohexyl)-6-fluoro-2,4-dioxo-3,4-dihydropyrido[2,3-d]pyrimidin-1(2H)-yl]benzoate

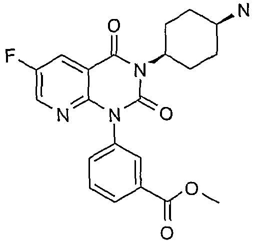

4.0M Hydrogen chloride in 1,4-dioxane (20 ml, 80 mmol) was added to methyl 3-[3-{cis-4-[(tert-butoxycarbonyl)amino]cyclohexyl}-6-fluoro-2,4-dioxo-3,4-dihydropyrido[2,3-d]pyrimidin-1(2H)-yl]benzoate (2 g, 3.9 mmol) and the mixture was stirred at room temperature for 1h. The reaction mixture was poured slowly onto saturated aq. sodium carbonate which was then extracted with DCM (x3). The organic extracts were combined, dried (anhydrous sodium sulphate), filtered and evaporated to afford the sub-title compound (1.8 g, 100%).
¹H NMR (400 MHz, DMSO-d₆) δ 8.55 (d, 1H), 8.29 (dd, 1H), 8.08 - 7.98 (m, 2H), 7.71 - 7.65 (m, 2H), 4.73 (qt, 1H), 3.87 (s, 3H), 3.06 (d, 2H), 2.69 (qd, 2H), 1.67 - 1.60 (m, 2H), 1.59 - 1.48 (m, 2H), 1.47 - 1.39 (m, 2H), 1.39 - 1.31 (m, 2H)

### Step (b) methyl 3-[6-fluoro-3-{cis-4-[(imidazo[1,2-a]pyridin-2-ylcarbonyl)amino]cyclohexyl}-2,4-dioxo-3,4-dihydropyrido[2,3-d]pyrimidin-1(2H)-yl]benzoate

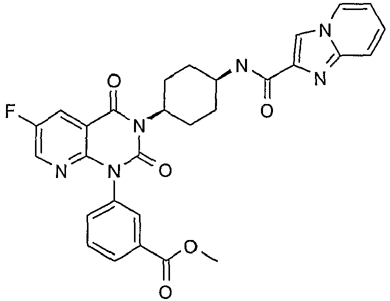

Imidazo[1,2-a]pyridine-2-carboxylic acid (660 mg, 4.07 mmol) was suspended in NMP (30 ml) and DIEA (1.31 g, 1.77 ml, 10.17 mmol) was added, followed by HATU (1.55 g, 4.07 mmol) and the mixture was stirred at room temperature for 10 min. 3-[3-(4-Amino-cyclohexyl)-6-fluoro-2,4-dioxo-3,4-dihydro-2H-pyrido[2,3-d]pyrimidin-1-yl]-benzoic acid methyl ester (1.4 g, 3.39 mmol) in DMF (10 ml) was added and the reaction mixture was stirred at room temperature for 48 hours. The reaction mixture was poured onto water and the solid that precipitated was collected by filtration and dried to afford the sub-title compound (1.35 g, 72%).
¹H NMR (400 MHz, DMSO-*d₆*) δ 8.58 (d, 1H), 8.56 (d, 1H), 8.38 (s, 1H), 8.33 (dd, 1H), 8.06 - 8.03 (m, 1H), 8.03 - 8.02 (m, 1H), 7.71 - 7.68 (m, 3H), 7.65 (d, 1H), 7.34 (ddd, 1H), 6.98 (td, 1H), 4.85 (t, 1H), 4.22 - 4.11 (m, 1H), 3.87 (s, 3H), 2.59 (d, 2H), 2.00 (d, 2H), 1.78 - 1.60 (m, 4H)

### Step (c) 3-[6-fluoro-3-{cis-4-[(imidazo[1,2-a]pyridin-2-ylcarbonyl)amino]cyclohexyl}-2,4-dioxo-3,4-dihydropyrido[2,3-d]pyrimidin-1(2H)-yl]benzoic acid

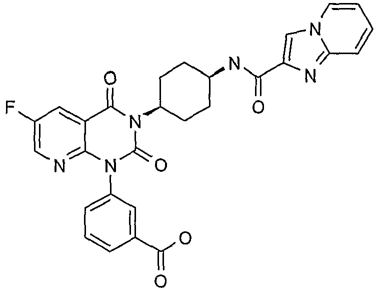

3-(6-Fluoro-3-{4-[(imidazo[1,2-a]pyridine-2-carbonyl)-amino]-cyclohexyl}-2,4-dioxo-3,4-dihydro-2H-pyrido[2,3-d]pyrimidin-1-yl)-benzoic acid methyl ester (1.75 g, 3.14 mmol) was dissolved in 1,4-dioxane (100 ml) and a solution of lithium hydroxide (150 mg, 6.29 mmol) in water (20 ml) was added and the reaction mixture was stirred at room temperature overnight. The mixture was acidified with glacial acetic acid and then concentrated in vacuo. The residue was purified by reverse phase HPLC (25-95% acetonitrile in aqueous trifluoroacetic acid) to afford the sub-title compound (440 mg, 26%).
¹H NMR (400 MHz, DMSO-*d₆*) δ 8.76 (s, 1H), 8.62 (s, 1H), 8.57 (d, 1H), 8.32 (dd, 1H), 8.10 (d, 1H), 8.04 - 8.01 (m, 1H), 7.99 - 7.97 (m, 1H), 7.74 (d, 1H), 7.65 (q, 2H), 7.59 (t, 1H), 7.19 (t, 1H), 4.86 (t, 1H), 4.18 - 4.12 (m, 1H), 2.70 - 2.59 (m, 2H), 2.11 - 1.99 (m, 2H), 1.78 - 1.58 (m, 4H)

### Step (d) N-{cis-4-[1-[3-(anilinocarbonyl)phenyl]-6-fluoro-2,4-dioxo-1,4-dihydropyrido[2,3-d]pyrimidin-3(2H)-yl]cyclohexyl} imidazo[1,2-a]pyridine-2-carboxamide

3-(6-Fluoro-3-{4-[(imidazo[1,2-a]pyridine-2-carbonyl)-amino]-cyclohexyl}-2,4-dioxo-3,4-dihydro-2H-pyrido[2,3-d]pyrimidin-1-yl)-benzoic acid (220 mg, 0.41 mmol) was dissolved in DMF (3 ml) and DIEA (157 mg, 212 ul, 1.22 mmol) was added, followed by HATU (185 mg, 0.49 mmol) and the reaction mixture was stirred at room temperature for 10 mins. Aniline (41.6 mg, 41 ul, 0.45 mmol) was added and the reaction mixture was left to stir at room temperature overnight. The solvent was removed and the residue partitioned between ethyl acetate and water. The organic extracts were combined, dried (anhydrous magnesium sulphate), filtered and evaporated and the residue was purified by reverse phase HPLC (25-95% acetonitrile in aqueous ammonia) to afford the title compound (120 mg, 48%).
¹H NMR (400 MHz, DMSO-*d₆*) δ 10.34 (s, 1H), 8.58 (d, 2H), 8.39 (s, 1H), 8.34 (dd, 1H), 8.08 (d, 1H), 7.98 (s, 1H), 7.77 (d, 2H), 7.73 - 7.61 (m, 4H), 7.39 - 7.30 (m, 3H), 7.11 (t, 1H), 6.98 (t, 1H), 4.87 (d, 1H), 4.18 (s, 1H), 2.70 - 2.56 (m, 2H), 2.01 (d, 2H), 1.78 - 1.59 (m, 4H)
APCI (Multimode) m/z: 618 [M+H]

### Example 5

### N-{cis-4-[1-(3-{[(2-aminoethyl)amino]carbonyl}phenyl)-6-fluoro-2,4-dioxo-1,4-dihydropyrido[2,3-d]pyrimidin-3(2H)-yl]cyclohexyl}imidazo[1,2-a]pyridine-2-carboxamide.

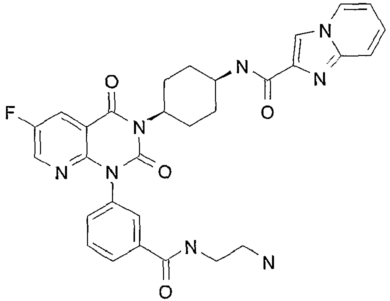

### Step (a) tert-butyl [2-({3-[6-fluoro-3-{cis-4-[(imidazo[1,2-a]pyridin-2-ylcarbonyl)amino]cyclohexyl}-2,4-dioxo-3,4-dihydropyrido[2,3-d]pyrimidin-1(2H)-yl]benzoyl}amino)ethyl]carbamate

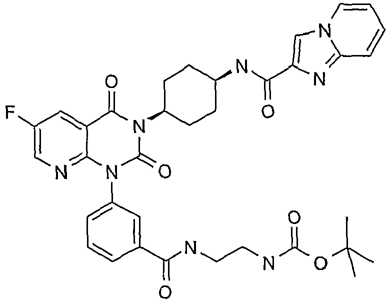

3-[6-fluoro-3-{cis-4-[(imidazo[1,2-a]pyridin-2-ylcarbonyl)amino]cyclohexyl}-2,4-dioxo-3,4-dihydropyrido[2,3-d]pyrimidin-1(2H)-yl]benzoic acid (220 mg, 0.41 mmol) was dissolved in DMF (3 ml) and DIEA (157 mg, 212 ul, 1.22 mmol) was added, followed by HATU (201 mg, 0.53 mmol) and the reaction mixture was stirred at room temperature for 10 min. (2-Amino-ethyl)-carbamic acid tert-butyl ester (72 mg, 71 ul, 0.45 mmol) was added and the reaction mixture was left to stir at room temperature overnight. The solvent was removed and the residue partitioned between ethyl acetate and water. The organic extracts were combined, dried (anhydrous magnesium sulphate), filtered and evaporated. the residue was purified by reverse phase HPLC (25-95% acetonitrile in aqueous ammonia) to afford the sub-title compound (30 mg, 10%).
¹H NMR (400 MHz, DMSO-*d₆*) δ 8.60 8.55 (m, 2H), 8.52 (t, 1H), 8.38 (s, 1H), 8.33 (dd, 1H), 7.93 (d, 1H), 7.83 (s, 1H), 7.70 (d, 1H), 7.67 - 7.63 (m, 1H), 7.60 (d, 1H), 7.56 - 7.53 (m, 1H), 7.36 - 7.30 (m, 1H), 6.98 (td, 1H), 6.90 (t, 1H), 4.86 (s, 1H), 4.17 (s, 1H), 3.29 - 3.19 (m, 2H), 3.11 (t, 2H), 2.64 - 2.54 (m, 2H), 2.00 (d, 2H), 1.70 (dd, 4H), 1.35 (s, 9H) APCI (Multimode) m/z: 685 [M+H]

### Step (b) N-{cis-4-[1-(3-{[(2-aminoethyl)amino]carbonyl}phenyl)-6-fluoro-2,4-dioxo-1,4-dihydropyrido[2,3-d]pyrimidin-3(2H)-yl]cyclohexyl}imidazo[1,2-a]pyridine-2-carboxamide.

tert-butyl[2-({3-[6-fluoro-3-{cis-4-[(imidazo[1,2-a]pyridin-2-ylcarbonyl)amino]cyclohe xyl}-2,4-dioxo-3,4-dihydropyrido[2,3-d]pyrimidin-1(2H)-yl]benzoyl}amino)ethyl] carbamate (150 mg, 0.22 mmol) was dissolved in 1,4-dioxane (2 ml) and 4.0M hydrogen chloride in 1,4-dioxane (5 ml, 20 mmol) was added, and the reaction mixture was stirred at room temperature for 2h. The solvent was evaporated and the residue was purified by reverse phase HPLC (25-95% acetonitrile in aqueous ammonia) to afford the title compound (35 mg, 50%).
¹H NMR (400 MHz, DMSO-*d₆*) δ 8.61 - 8.55 (m, 2H), 8.46 (t, 1H), 8.38 (s, 1H), 8.33 (dd, 1H), 7.95 (d, 1H), 7.85 (t, 1H), 7.70 (d, 1H), 7.67 - 7.57 (m, 2H), 7.56 - 7.52 (m, 1H), 7.37 - 7.31 (m, 1H), 6.98 (ddd, 1H), 4.86 (s, 1H), 4.17 (s, 1H), 3.16 - 3.03 (m, 2H), 2.67 (t, 2H), 2.60 (d, 2H), 2.00 (d, 2H), 1.70 (dd, 4H)
APCI (Multimode) m/z: 585 [M+H]

### Example 6

*N-*{*cis*-4-[6-fluoro-2,4-dioxo-1-[3-(pyridin-3-ylethynyl)phenyl]-1,4-dihydropyrido[2,3-*d*]pyrimidin-3(2*H*)-yl]cyclohexyl}imidazo[1,2-*a*]pyridine-2-carboxamide.

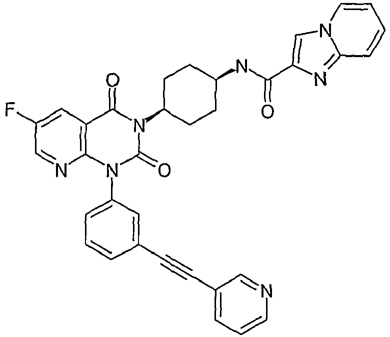

### Step (a) 5-Fluoro-2-(3-iodophenylamino)pyridine-3-carboxylic acid

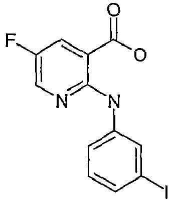

2-Chloro-5-fluoronicotinic acid (4.68g, 26.7mmol), potassium carbonate (4.43g, 32.1mmol), 3-Iodoaniline (8.8g, 40.2mmol), copper(I)bromide (192mg, 1.34mmol) and copper (102mg, 1.6mmol)) were weighed into a round bottomed flask and N-methylpyrrolidinone (40ml) was added. After degassing the mixture it was heated under nitrogen at 150 °C for 4 hours. The black reaction mixture was poured onto water and the mixture was stirred overnight. The obtained solid was filtered, washed with water and dried on the sinter to yield a dark brown solid (this is the sub-title compound) which was used without further purification in the subsequent step.
APCI-MS m/z: 358 [MH⁺].

### Step (b) 3-(4-tert-Butyloxycarbonylaminocyclohexyl)-6-fluoro-1-[3-iodophenyl]pyrido[2,3-d]pyrimidine-2,4(1H,3H)-dione

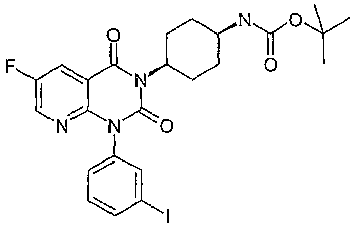

5-Fluoro-2-(3-iodophenylamino)pyridine-3-carboxylic acid (9.3g, 26mmol), cis-4-amino-1-tert-butoxycarbonylamino-cyclohexane (5.6g, 26.1 mmol), HATU (11.86g, 31.2mmol) and HOAT (4.25g, 31.2mmol) were dissolved in N-methylpyrrolidinone (30ml) at ambient temperature. N-ethyl-diisopropylamine (13.3ml, 77.7mmol) was added slowly during 1 minute, whilst an exothermic reaction occurred. After stirring for 15 hours the mixture was poured onto water and extracted with dichloromethane. The organic phase was washed with 3N hydrochloric acid, water, saturated aqueous sodium carbonate, water and brine, dried over sodium sulfate and adsorbed onto silica gel. Flash chromatography using a mixture of petrol ether and ethyl acetate yielded 3-(4-(tert-butoxycarbonylamino)cyclohexyl-1-aminocarbonyl)-5-fluoro-2-(3-iodophenylamino)pyridine (2.15g, 3.9mmol) as a red-brown solid, which was dissolved in a mixture of N-methylpyrrolidinone (3ml) and tetrahydrofuran (2ml). Carbonyldiimidazole (1.9g, 11.7mmol) was added followed by sodium hydride (60%) in one portion. The reaction was stirred at ambient temperature for 2 hours, poured onto water and extracted with ethyl acetate. The organic phase was washed with water, dried over sodium sulfate, filtered and adsorbed onto silica gel. Flash chromatography using a mixture of petrol ether and ethyl acetate yielded the sub-title compound (1.05g) as a yellow solid.
¹H NMR (DMSO-*d₆*): δ 8.58 (1H, d); 8.28 (1H, dd); 7.79 - 7.85 (2H, m); 7.43 (1H, ddd); 7.33 (1H, ddd); 6.52 (1H, brs); 4.72 (1H, bt); 3.55 (1H, bs); 2.50 - 2.64 (2H, brm); 1.84-1.96 (2H, brd); 1.37 -1.56 (4H, brm and 9H, s).
APCI-MS m/z: 555 [MH⁺].

### Step (c) 3-(4-tert-Butyloxycarbonylaminocyclohexyl)-6-fluoro-1-[3-(pyridin-3-ylethynyl)phenyl]pyrido[2,3-d]pyrimidine-2,4(1H,3H)-dione.

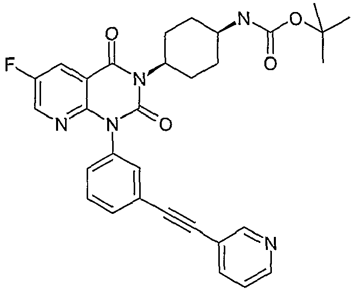

3-(4-tert-Butyloxycarbonylaminocyclohexyl)-6-fluoro-1-[3-iodophenyl]pyrido[2,3-d]pyrimidine-2,4(1H,3H)-dione (1.0g, 1.72mmol) and 3-pyridylacetylene (0.235g, 2.28mmol) were dissolved in tetrahydrofurane (5ml). Triethylamine (0.95ml, 6.83mmol) was added followed by addition of a mixture of bis(triphenylphosphino)palladium(II)dichloride (0.12g, 0.17mmol) and copper(I)iodide (16mg, 0.084mmol). The mixture was stirred at ambient temperature for 1h, poured onto a mixture of water and dichloromethane. The organic phase was separated and washed with water, dried over potassium carbonate, filtered and adsorbed onto silica gel. Flash-chromatography using a mixture of petrol ether and ethyl acetate yielded the sub-title compound as a yellow solid (0.75g, 79%).
¹H NMR (DMSO-d₆): δ 8.77 (1H, s); 8.58 - 8.62 (2H, m); 8.29 (1H, dd); 7.98 (1H, d); 7.64 - 7.71 (2H, m); 7.60 (1H, t); 7.44 - 7.51 (2H, m); 6.52 (1H, brs); 4.75 (1H, bt); 3.56 (1H, bs); 2.52 - 2.67 (2H, brm); 1.86 -1.95 (2H, brd); 1.42 -1.56 (4H, brm); 1.39 (9H, s). APCI-MS m/z: 555 [MH⁺].

### Step (d) 3-(4-aminocyclohexyl)-6-fluoro-1-[3-(pyridin-3-ylethynyl)phenyl]pyrido[2,3-d]pyrimidine-2,4(1H,3H)-dione.

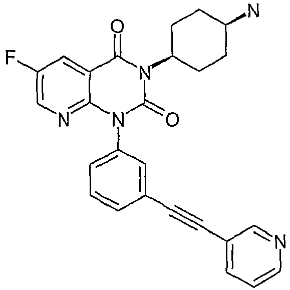

3-(4-tert-Butyloxycarbonylaminocyclohexyl)-6-fluoro-1-[3-(pyridin-3-ylethynyl)phenyl]pyrido[2,3-d]pyrimidine-2,4(1H,3H)-dione (700mg, 1.26mmol) was dissolved in dioxane (5ml) and hydrogen chloride (1.2 ml of 4N solution in dioxane, 4.8mmol) was added and the solution was heated at 50°C for 24 hours. After cooling to ambient temperature, diethyl ether was added to initiate crystallisation. The solid was filtered quickly, washed with diethylether and dried in the air to obtain the target compound as the dihydrochloride salt which is very hygroscopic and used immediately in the subsequent step.
Alternatively, the obtained salt was partitioned between chloroform and saturated aqueous sodium carbonate and the organic phase is washed with water, dried over potassium carbonate, filtered and concentrated under reduced pressure to yield the target compound as a beige solid.
¹H NMR (DMSO-*d₆*): δ 8.76 (1H, s); 8.57 - 8.62 (2H, m); 8.30 (1H, dd); 7.99 (1H, ddd); 7.57 - 7.71 (3H, m); 7.45 - 7.51 (2H, m); 4.73 (1H, bt); 3.06 (1H, bs); 2.63 - 2.78 (2H, brm); 1.60 -1.69 (2H, brd); 1.48 -1.59 (2H, brm); 1.37 -1.47 (2H, brm).
APCI-MS m/z: 455 [MH⁺].

### Step (e) N-{4-[6-fluoro-2,4-dioxo-1-[3-(pyridin-3-ylethynyl)phenyl]-1,4-dihydropyrido[2,3-d]pyrimidin-3(2H)-yl]cyclohexyl}imidazo[1,2-a]pyridine-2-carboxamide

To a mixture of imidazo[1,2-a]pyridine-2-carboxylic acid (110mg, 0.68mmol), HATU (250mg, 0.66mmol) and 3-(4-aminocyclohexyl)-6-fluoro-1-[3-(pyridin-3ylethynyl)phenyl]-pyrido[2,3-d]pyrimidine-2,4(1H,3H)-dione dihydrochloride (230mg, 0.44mmol) in Chloroform was added ethyl-di-isopropylamine (0.38ml, 2.2mmol) over a period of 20 seconds. Stirring was continued at ambient temperature for 15 hours. Water was added to the clear solution and the mixture was concentrated under reduced pressure using a rotary evaporator. The obtained solid was filtered and dissolved in acetonitrile. Purification on preparative HPLC resulted in the title compounds as an off white solid (45mg, 17%).
¹H NMR (DMSO-*d₆*): δ 8.75 (1H, s); 8.55 - 8.62 (3H, m); 8.38 (1H, s); 8.34 (1H, dd); 7.98 (1H, ddd); 7.57 - 7.74 (4H, m); 7.44 - 7.52 (2H, m); 7.33 (1H, brdd); 6.98 (1H, brdd); 4.87 (1H, bt); 4.18 (1H, bs); 2.54 - 2.69 (2H, brm); 2.00 (2H, brd); 1.59 -1.79 (4H, m).
APCI-MS m/z: 599 [MH⁺].

### Example 7

### N-[cis-4-(1-cyclopentyl-6-fluoro-2,4-dioxo-1,4-dihydropyrido[2,3-d]pyrimidin-3(2H)-yl)cyclohexyl]imidazo[1,2-a]pyridine-2-carboxamide.

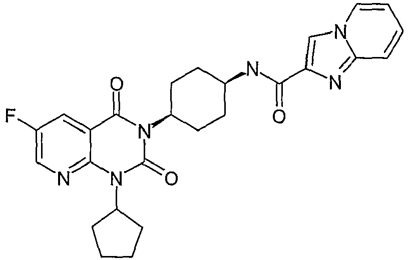

### Step (a) 5-Fluoro-2-(cyclopentylamino)pyridine-3-carboxylic acid

2-Chloro-5-fluoronicotinic acid (10.45g, 60mmol), potassium carbonate (9.9g, 71.6mmol), cyclopentylamine (9ml, 90.9mmol), copper(I)bromide (430mg, 3mmol) and copper (230mg, 3.6mmol) were weighed into a round bottomed flask and N-methylpyrrolidinone (40ml) was added. After degassing the mixture it was heated under nitrogen at 150°C for 3 days. The black reaction mixture was poured onto water and the pH was adjusted to 5 using 3N hydrochloric acid. The mixture was stirred overnight, the obtained solid was filtered, washed with water and dried on the sinter to yield the sub-title compound as a grey solid (4.58g) which was used without further purification in the subsequent step. APCI-MS m/z: 225 [MH⁺].

### Step (b) 3-(4-tert-Butyloxycarbonylaminocyclohexyl)-6-fluoro-1-[cyclopentyl]pyrido[2,3-d]pyrimidine-2,4(1H,3H)-dione

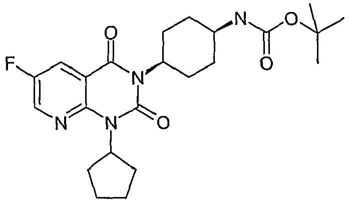

5-Fluoro-2-(cyclopentylamino)pyridine-3-carboxylic acid (3.15g, 14.1mmol), cis-4-amino-1-tert-butoxycarbonylamino-cyclohexane (3.16g, 14.7mmol), HATU (6.43g, 16.9mmol) and HOAT (2.3g, 16.9mmol) were dissolved in N-methylpyrrolidinone (50ml) at ambient temperature. N-ethyl-diisopropylamine (7.3ml, 42.6mmol) was added slowly during 1 minute, whilst an exothermic reaction occurred. After stirring for 3 hours the mixture was poured onto water and the mixture was stirred for an additional hour at ambient temperature. The obtained solid was filtered, washed with water and dried on the sinter to yield 3-(4-(tert-butoxycarbonylamino)cyclohexyl-1-aminocarbonyl)-5-fluoro-2-(cyclopentylamino)pyridine (5.52g, 13.1mmol) as an olive green solid, which was taken on as such. 3-(4-(tert-butoxycarbonylamino)cyclo-hexyl-1-aminocarbonyl)-5-fluoro-2-(cyclopentylamino)pyridine (4.31g, 10.3mmol) was dissolved in a mixture of N-methylpyrrolidinone (12ml) and tetrahydrofuran (8ml). Carbonyldiimidazole (4.99g, 30.8mmol) was added followed by sodium hydride (60%, 1.24g, 31mmol) in one portion. The reaction was stirred at ambient temperature for 4 days, poured onto water and the obtained solid was filtered, washed with water, dried on the sinter, dissolved in Dichloromethane and adsorbed onto silica gel. Flash chromatography using a mixture of petrol ether and ethyl acetate yielded the sub-title compound (3.01g, 65%) as a colourless solid.
¹H NMR (300MHz, DMSO-*d*₆): δ 8.76 (1H, d); 8.20 (1H, dd); 8.03 (2H, bs); 6.56 (1H, bs); 5.79 (1H, p); 4.73 (1H, bt); 3.55 (1H, bs); 2.49 - 2.67 (2H, bm); 2.05 - 2.20 (2H, bd); 1.73 - 2.01 (6H, m); 1.34 -1.67 (16H, m).
APCI-MS m/z: 447 [MH⁺].

### Step (c) 3-(4-Aminocyclohexyl)-1-cyclopentyl-6-fluoro-pyrido[2,3-d]pyrimidine-2,4(1H,3H)-dione

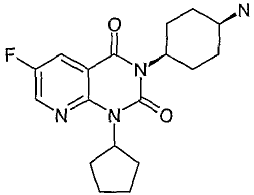

3-(4-tert-Butyloxycarbonylaminocyclohexyl)-6-fluoro-1-[cyclopentyl]pyrido[2,3-d]pyrimidine-2,4(1H,3H)-dione (2530mg, 5.67mmol) was dissolved in dioxane (15ml) and hydrogen chloride (5.5 ml of 4N solution in dioxane, 22mmol)) was added and the solution was heated at 55°C for 15 hours. After cooling to ambient temperature, diethyl ether was added to initiate crystallisation. The solid was filtered quickly, washed with diethylether and dried in the air to obtain the target compound (1.8g; 83%) as the hydrochloride salt and it was used immediately in the subsequent step.
Alternatively, the obtained salt was partitioned between chloroform and saturated aqueous sodium carbonate and the organic phase is washed with water, dried over potassium carbonate, filtered and concentrated under reduced pressure to yield the sub-title compound as an off-white solid.
¹H NMR (300MHz, DMSO-d₆): δ 8.78 (1H, d); 8.21 (1H, dd); 8.03 (2H, bs); 5.80 (1H, p); 4.74 (1H, bt); 3.33 - 3.45 (1H, bs); 2.47 - 2.64 (2H, bm); 2.05 - 2.22 (2H, bd); 1.51 - 2.01 (12H, m).
APCI-MS m/z: 347 [MH⁺].

### Step (d) N-[4-(1-cyclopentyl-6-fluoro-2,4-dioxo-1,4-dihydropyrido[2,3-d]pyrimidin-3(2H)-yl)cyclohexyl]imidazo[1,2-a]pyridine-2-carboxamide

To a mixture of imidazo[1,2-a]pyridine-2-carboxylic acid (89mg, 0.55mmol), HATU (220mg, 0.55mmol), HOAT (75mg, 0.55mmol) and 3-(4-aminocyclohexyl)-1-cyclopentyl-6-fluoro-pyrido[2,3-d]pyrimidine-2,4(1H,3H)-dione hydrochloride (176mg, 0.46mmol) in N-methylpyrrolidinone (10ml) was added ethyl-di-isopropylamine (0.31ml), 1.81mmol) over a period of 20 seconds. Stirring was continued at ambient temperature for 3 days and the reaction mixture was poured onto water. The obtained solid was filtered, dried on the sinter, dissolved in chloroform and adsorbed onto silica gel. Flash chromatography using a mixture of petrol ether and ethyl acetate yielded the title compound (55mg, 24%) as a colourless solid.
¹H NMR (300MHz, DMSO-*d*₆): δ 8.77 (1H, d); 8.60 (1H, ddd); 8.40 (1H, s); 8.25 (1H, dd); 7.78 (1H, d); 7.68 (1H, d); 7.38 (1H, dd); 7.00 (1H, dd); 5.80 (1H, p); 4.84 (1H, bt); 2.89 (1H, bs); 2.50 - 2.70 (2H, bm); 2.04 - 2.22 (2H, bd); 1.41- 2.04 (12H, m). APCI-MS m/z: 491 [MH⁺].

### Example 8

### methyl trans-4-[3-{cis-4-[(cyclopropylcarbonyl)amino]cyclohexyl}-6-fluoro-2,4-dioxo-3,4-dihydropyrido[2,3-d]pyrimidin-1(2H)-yl]cyclohexanecarboxylate.

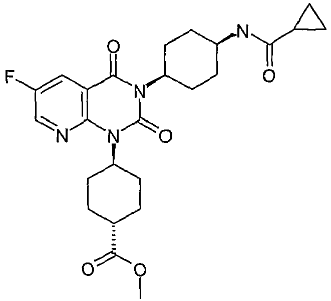

### Step (a) Methyl trans-4-aminocyclohexanecarboxylate

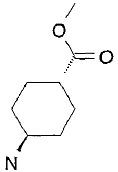

To a solution of trans-4-aminocyclohexane carboxylic acid hydrochloride (2 g, 11.13 mmol) in methanol (30 ml) sulphuric acid (0.7 ml) was added. The mixture was refluxed over night at 70 °C. The solvent was evaporated, water was added and the mixture was made basic with ammonia. The aqueous solution was repeatedly extracted with totally 500 ml of CH₂Cl₂. The organic extracts were dried with Mg₂SO₄, filtered and evaporated to give the sub-title compound with some impurity (1.045 g, 59 %).
APCI-MS m/z: 158 [MH⁺].

### Step (b) 5-Fluoro-2-{[trans-4-(methoxycarbonyl)cyclohexyl]amino}nicotinic acid

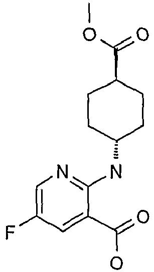

To a solution of 2-chloro-5-fluoronicotinic acid (640 mg, 3.65 mmol) in DMF (9 ml) methyl trans-4-aminocyclohexanecarboxylate (860 mg, 5.47 mmol), copper (70 mg, 1.1 mmol), Cu(I)Br (105 mg, 0.73 mmol) and potassium carbonate (605 mg, 4.38 mmol) were added. The mixture was heated at 150°C for 1.5 h. A solution of HCl was added and the mixture was extracted with EtOAc. The organic phase was extracted with a solution of potassium carbonate. The aqueous layer was made acidic with HCl and extracted with EtOAc. The organic phase was dried with Mg₂CO₃, filtered and evaporated the give the sub-title compound (732 mg, 63%).
APCI-MS m/z: 297 [MH⁺].

### Step (c) Methyl trans-4-({3-[({cis-4-[(tert-butoxycarbonyl)amino]cyclohexyl}amino)carbonyl]-5-fluoropyridin-2-yl} amino)cyclohexanecarboxylate

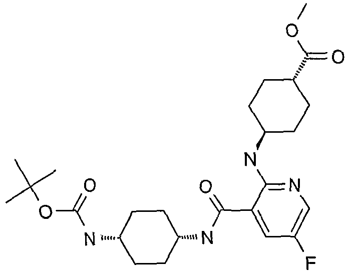

5-Fluoro-2-{[trans-4-(methoxycarbonyl)cyclohexyl]amino}nicotinic acid (732 mg, 2.47 mmol), tert-butyl cis-4-aminocyclohexylcarbamate (583 mg, 2.72 mmol), HATU (1125 mg, 2.96 mmol), HOAT (403 mg, 2.96 mmol) and DIEA (1.27 ml, 7.41 mmol) in NMP were stirred for 1 h. EtOAc was added and the mixture was washed with a solution of NaHCO₃, dried with Mg₂CO₃, filtered and evaporated. The remaining oil was purified by flash chromatography on silica [acetone:heptane (2:3)] to give the sub-title compound (796 mg, 65%).
APCI-MS m/z: 493 [MH⁺].

### Step (d) Methyl trans-4-[3-{cis-4-[(tert-butoxycarbonyl)amino]cyclohexyl}-6-fluoro-2,4-dioxo-3,4-dihydropyrido[2,3-d]pyrimidin-1(2H)-yl]cyclohexanecarboxylate

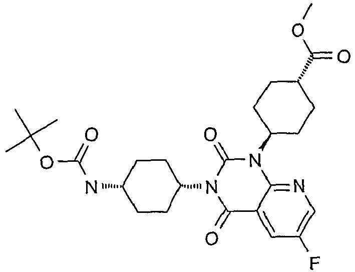

To a solution of methyl trans-4-({3-[({cis-4-[(tert-butoxycarbonyl)amino]cyclohexyl}amino)carbonyl]-5-fluoropyridin-2-yl}amino)cyclohexanecarboxylate (400 mg, 0.81 mmol) in argon flushed THF carbonyl dimidazole (395 mg, 2.44 mmol) and NaH (50 % in oil, 117 mg, 2.44 mmol) were added. The mixture was refluxed at 70 °C over night. Aqueous NaHCO₃ solution was added and the mixture was extracted with EtOAc. The crude residue was purified on by flash chromatography on silica [acetone:heptane (1:4)] to give the sub-title compound (180 mg, 43%).
APCI-MS m/z: 419 [MH⁺].

Step (e) Methyl trans-4-[3-{cis-4-[(cyclopropylcarbonyl)amino]cyclohexyl}-6-fluoro-2,4-dioxo-3,4-dihydropyrido[2,3-d]pyrimidin-1(2H)-yl]cyclohexanecarboxylate Methyl trans-4-[3-{cis-4-[(tert-butoxycarbonyl)amino]cyclohexyl}-6-fluoro-2,4-dioxo-3,4-dihydropyrido[2,3-d]pyrimidin-1(2H)-yl]cyclohexanecarboxylate (50 mg, 0.096 mmol) was stirred in 4 M HCl in dioxane for 3 h to give the free amine. The solvent was evaporated and the crude material was used directly in the next step were cyclopropyl carboxylic acid (8.5 µl, 0.106 mmol), HATU (44 mg, 0.116 mmol), HOAT (16 mg, 0.116 mmol), DIEA (49 µl, 0.289 mmol) and NMP (1 ml) were added. The mixture was stirred for 1h and the title compound was obtained by preparative HPLC (26 mg, 56%). ¹H NMR (400 MHz, DMSO-*d₆*) δ 8.77 (1H, d); 8.22 (1H, dd); 7.93 (1H, d); 5.22 (1H, s); 4.73 (1H, t); 3.78 (1H, s); 3.61 (3H, s); 2.68 - 2.55 (2H, m); 2.38 - 2.29 (1H, m); 2.10-2.02 (2H, m); 1.93 - 1.86 (2H, m); 1.84 - 1.70 (3H, m); 1.58 - 1.39 (6H, m); 0.69 - 0.62 (4H, m).
APCI-MS m/z: 487 [MH⁺].

### Example 9

### N-{cis-4-[6-fluoro-1-(4-fluorobenzyl)-2,4-dioxo-1,4-dihydropyrido[2,3-d]pyrimidin-3 (2H)-yl]cyclohexyl imidazo [1,2-a]pyridine-2-carboxamide.

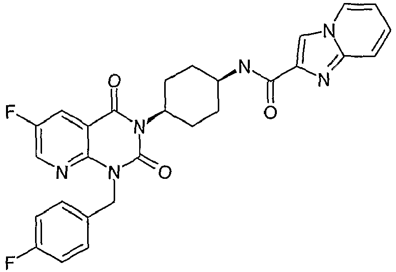

### Step (a) 5-Fluoro-2-((4-fluorobenzyl)amino)pyridine-3-carboxylic acid

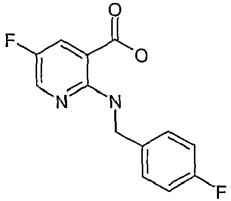

2-Chloro-5-fluoronicotinic acid (7.57g, 43.2mmol), potassium carbonate (7.2g, 52.1mmol)), 4-fluorobenzylamine (7.4g, 59.1mmol), copper(I)bromide (310mg, 2.16mmol) and copper (165mg, 2.6mmol) were weighed into a round bottomed flask and N-methylpyrrolidinone (30ml) was added. After degassing the mixture it was heated under nitrogen at 150 °C for 2 hours. The black reaction mixture was poured onto water and the mixture was stirred overnight. The obtained solid was filtered, washed with water and dried on the sinter to yield a light green solid (the sub-title compound) which was used without further purification in the subsequent step.
APCI-MS m/z: 265 [MH⁺].

### Step (b) 3-(4-tert-Butyloxycarbonylaminocyclohexyl)-6-fluoro-1-(4-fluorobenzyl)-pyrido[2,3-d]pyrimidine-2,4(1H,3H)-dione

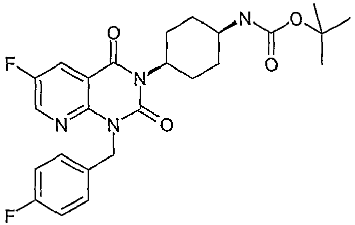

5-Fluoro-2-(4-fluorobenzylamino)pyridine-3-carboxylic acid (3.18g, 12mmol), cis-4-amino-1-tert-butoxycarbonylamino-cyclohexane (2.72g, 12.7mmol), HATU (5.02g, 13.2mmol) and HOAT (1.8g, 13.3mmol) were dissolved in N-methylpyrrolidinone (30ml) at ambient temperature. N-ethyl-diisopropylamine (6.2ml, 36.2mmol) was added slowly during 1 minute, whilst an exothermic reaction occurred. After stirring for 15 hours the mixture was poured onto water and the pH was adjusted to pH 6 using 3N hydrochloric acid. The mixture was stirred for 3 hours and the obtained solid was filtered, washed with water and dried on the sinter to yield 3-(4-(tert-butoxycarbonylamino)cyclohexyl-1-aminocarbonyl)-5-fluoro-2-(4-fluorobenzylamino)pyridine as a brown solid, which was dissolved in a mixture of N-methylpyrrolidinone (30ml) and tetrahydrofuran (20ml). Carbonyldiimidazole (5.84g, 36mmol) was added followed by sodium hydride (60%, 1.44g, 36mmol) in one portion. The reaction was stirred at ambient temperature for 1 hour, poured onto water and the pH was adjusted to pH = 7 using 3N hydrochloric acid. The mixture was stirred overnight and the obtained solid was filtered, washed with water and dried on the sinter. The solid was dissolved in Chloroform and adsorbed onto silica gel. Flash chromatography using a mixture of petrol ether and ethyl acetate yielded the sub-title compounds (3.6g, 62%) as a colourless solid.
¹H NMR (400MHz, DMSO-*d₆*): δ 8.75 (1H, d); 8.24 (1H, dd); 7.39 (2H, m); 7.11 (2H, t); 6.57 (1H, bs); 5.38 (2H, s); 4.75 (1H, m); 3.55 (1H, bs); 2.52 - 2.65 (2H, bm); 1.85 -1.94 (2H, bm); 1.36 - 1.56 (13H, m).
APCI-MS m/z: 487 [MH⁺].

### Step (c) 3-(4-Aminocyclohexyl)-6-fluoro-1-(4-fluorobenzyl)pyrido[2,3-d]pyrimidine-2,4(1H,3H)-dione

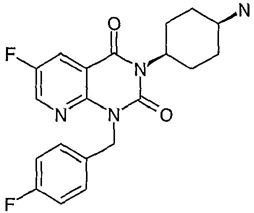

3-(4-tert-Butyloxycarbonylaminocyclohexyl)-6-fluoro-1-(4-fluorobenzyl)-pyrido[2,3-d]pyrimidine-2,4(1H,3H)-dione (3.52g, 7.2mmol)) was dissolved in dioxane (10ml) and hydrogen chloride (8 ml of 4N solution in dioxane, 32mmol) was added and the solution was heated at 50 °C for 3 hours. After cooling to ambient temperature, diethyl ether was added to initiate crystallisation. The obtained salt was decanted off and partitioned between chloroform and saturated aqueous sodium carbonate and the organic phase is washed with water, dried over potassium carbonate, filtered and concentrated under reduced pressure to yield the sub-title compound as a colourless solid (2.45g, 88%).
¹H NMR (300MHz, DMSO-*d₆*): δ 8.75 (1H, d); 8.25 (1H, dd); 7.34 - 7.44 (2H, m); 7.07-7.15 (2H, m); 5.38 (2H, s); 4.73 (1H, m); 3.06 (1H, bs); 2.62 - 2.78 (2H, bm); 1.23 -1.70 (6H, bm).
APCI-MS m/z: 387 [MH⁺].

### Step (d) N-{4-[6-fluoro-1-(4-fluorobenzyl)-2,4-dioxo-1,4-dihydropyrido[2,3-d]pyrimidin-3(2H)-yl]cyclohexyl}imidazo[1,2-a]pyridine-2-carboxamide

To a mixture of imidazo[1,2-a]pyridine-2-carboxylic acid (91mg, 0.56mmol), HATU (mg, 0.55mmol) and 3-(4-aminocyclohexyl)-6-fluoro-1-[4-fluorobenzyl]-pyrido[2,3-d]pyrimidine-2,4(1H,3H)-dione (180mg, 0.46mmol) in Chloroform (5 ml) was added ethyl-di-isopropylamine (0.32ml, 1.87mmol) over a period of 20 seconds. Stirring was continued at ambient temperature for 15 hours. Water was added to the clear solution and the mixture was concentrated under reduced pressure using a rotary evaporator. The obtained solid was filtered, washed with water and dried on the sinter to result in the title compound as an off white solid (120mg, 49%).
¹H NMR (300MHz, DMSO-*d₆*): δ 8.75 (1H, d); 8.59 (1H, ddd); 8.40 (1H, s); 8.29 (1H, dd); 7.75 (1H, d); 7.69 (1H, d); 7.34 - 7.43 (2H, m); 7.08 - 7.17 (2H, m); 6.99 (1H, ddd); 5.39 (2H, s); 4.86 (1H, bt); 4.17 (1H, bs); 2.45 - 2.69 (2H, bm); 1.94 - 2.04 (2H, bm); 1.54 -1.80 (4H, m).
APCI-MS m/z: 531 [MH⁺].

### Example 12

### N-{cis-4-[1-{4'-[(dimethylamino)methyl]biphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide.

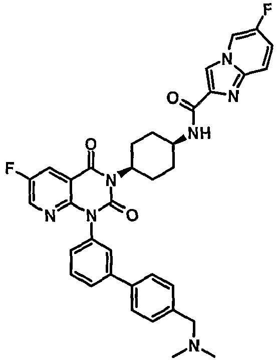

### Step (a) 5-fluoro-2-[(3-iodophenyl)amino]nicotinic acid

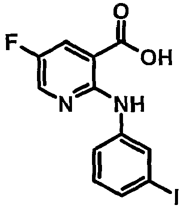

2-Chloro-5-fluoronicotinic acid (27 g, 153.8 mmol) was added to dry DMF (500 ml). Copper (977 mg), copper (I)bromide (2.20 g), potassium carbonate (25.51 g, 184.6 mmol) and 3-Iodo-phenylamine (27.7 ml, 230.7 mmol) were added and the reaction mixture was stirred at 110 °C for 2.5 hours. 1M HCl was added and the product extracted with ethyl acetate. The organic phase was washed with 1M HCl, dried (anhydrous magnesium sulphate), filtered and concentrated in vacuo to afford the sub-title compound as a mixture containing 2-Chloro-5-fluoronicotinic acid (21.64 g). This was used without further purification.
APCI (Multimode) m/z: 357 [M-H]

### Step (b) 6-fluoro-N-{cis-4-[({5-fluoro-2-[(3-iodophenyl)amino]pyridin-3-yl}carbonyl)amino]cyclohexyl}imidazo[1,2-a]pyridine-2-carboxamide

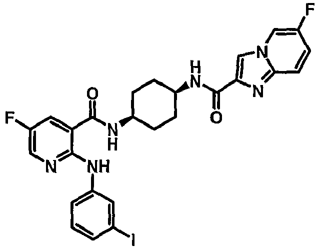

To 5-fluoro-2-[(3-iodophenyl)amino]nicotinic acid (8.4 g, 23.4 mmol) and N-(cis-4-aminocyclohexyl)-6-fluoroimidazo[1,2-a]pyridine-2-carboxamide (7.34 g, 23.4 mmol) was added acetonitrile (85 ml) and triethylamine (19.6 ml, 140.8 mmol). 2,4,6-Tripropyl-1,3,5,2,4,6-trioxatriphosorinan-2,4,6-trioxide (17.9 ml, 28.2 mmol) (50% wt solution in butyl acetate) was added dropwise over 15 min. and the reaction mixture was stirred at room temperature for 5 min. The solid was then collected by filtration and washed with water. Acetonitrile was added and the suspension heated, allowed to cool and the solid collected by filtration and dried to afford the sub-title compound (6.9 g).
¹H NMR (400 MHz, DMSO-*d₆*) δ10.70 (1H, s), 8.80 (1H, qd), 8.53 (1H, d), 8.41 (1H, d), 8.37 (1H, s), 8.23 - 8.19 (1H, m), 7.81 (1H, d), 7.67 (1H, t), 7.53 - 7.43 (2H, m), 7.32 - 7.29 (1H, m), 7.08 (1H, t), 4.05 - 3.96 (1H, m), 3.42 (1H, s), 1.95 - 1.87 (2H, m), 1.81-1.64 (6H, m)
APCI (Multimode) m/z: 617 [M+H]

### Step (c) 6-fluoro-N-{cis-4-[6-fluoro-1-(3-iodophenyl)-2,4-dioxo-1,4-dihydropyrido[2,3-d]pyrimidin-3(2H)-yl]cyclohexyl}imidazo[1,2-a]pyridine-2-carboxamide

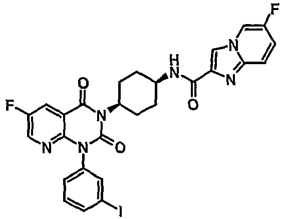

To a suspension of 6-fluoro-N-{cis-4-[({5-fluoro-2-[(3-iodophenyl)amino]pyridin-3-yl}carbonyl)amino]cyclohexyl}imidazo[1,2-a]pyridine-2-carboxamide (6.9 g, 11.2 mmol) in NMP (35 ml) was added 1,1'-carbonyldimidazole (6.35 g, 39.2 mmol). The solution was stirred at room temperature for 10 min. Sodium hydride (60% wt in mineral oil) (806 mg, 33.6 mmol) was added and the mixture was stirred at 40 °C for 1.5 h. The mixture was poured onto ice water and the product extracted with ethyl acetate. The organic phase was washed with water, dried with anhydrous magnesium sulphate, filtered and concentrated in vacuo. The residue was triturated in diethyl ether to afford the sub-title compound (4.50g). ¹H NMR (400 MHz, DMSO-*d₆*) δ 8.80 (1H, qd), 8.59 (1H, d), 8.37 (1H, d), 8.32 (1H, dd), 7.86 - 7.80 (2H, m), 7.77 (1H, dd), 7.68 (1H, d), 7.49 - 7.41 (2H, m), 7.33 (1H, t), 4.84 (1H, t), 4.18 (1H, s), 2.63 - 2.53 (2H, m), 2.04 - 1.96 (2H, m), 1.76 - 1.60 (4H, m)
APCI (Multimode) m/z: 642 [M+H]

Alternatively, the compound of Step (c) [6-fluoro-N-{cis-4-[6-fluoro-1-(3-iodophenyl)-2,4-dioxo-1,4-dihydropyrido[2,3-d]pyrimidin-3(2H)-yl]cyclohexyl}imidazo[1,2-a]pyridine-2-carboxamide] can be prepared as shown in Route A below.

### Step (d) N-{cis-4-[1-{4-[(dimethylamino)methyl]biphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

Acetonitrile (2 ml) was added to palladium(II) acetate (3 mg) and 2-(dicyclohexylphosphino) -2',6'-dimethoxy-1,1'-biphenyl (10 mg) and the mixture was stirred at room temperature for 10 min. Potassium carbonate (97 mg, 0.70 mmol) in water (1.6 ml), dimethyl-[4-(4,4,5,5-tetramethyl-[1,3,2]dioxaborolan-2-yl)-benzyl]-amine (104 mg, 0.35 mmol) and 6-fluoro-imidazo[1,2-a]pyridine-2-carboxylic acid 6-fluoro-N-{cis-4-[6-fluoro-1-(3-iodophenyl)-2,4-dioxo-1,4-dihydropyrido[2,3-d]pyrimidin-3(2H)-yl]cyclohexyl}imidazo[1,2-a]pyridine-2-carboxamide (150 mg, 0.23 mmol) were added and the reaction mixture was heated to reflux for 2 hours, and allowed to cool overnight. The mixture was loaded onto a Varian Bond Chemelut cartridge (available from Kinesis), the product eluted with DCM and concentrated in vacuo. The residue was purified by reverse phase HPLC (50-70% acetonitrile in aqueous ammonia) and lyophilised to afford the title compound (70 mg)
¹H NMR (400 MHz, DMSO-*d₆*) δ 8.79 (1H, dd), 8.59 (1H, d), 8.36 (1H, s), 8.33 (1H, dd), 7.79 - 7.71 (3H, m), 7.68 (1H, d), 7.65 - 7.57 (3H, m), 7.47 - 7.41 (1H, m), 7.39 - 7.35 (3H, m), 4.92 - 4.82 (1H, m), 4.17 (1H, s), 3.41 (2H, s), 2.69 - 2.55 (2H, m), 2.15 (6H, s), 2.00 (2H, d), 1.77 - 1.63 (4H, m)
APCI (Multimode) m/z: 650 [M+H]

The following compounds (Table 3) were prepared in a similar manner as solids from the appropriate boronic acid or ester and 6-fluoro-N-{cis-4-[6-fluoro-1-(3-iodophenyl)-2,4-dioxo-1,4-dihydropyrido[2,3-d]pyrimidin-3(2H)-yl]cyclohexyl}imidazo [1,2-a]pyridine-2-carboxamide using the method described above in Example 12 Step (d).

**Table 3**

| **Example Number** | **CHEMISTRY** | **Names** | **NMR** | **M+H** |
|---|---|---|---|---|
| 13 | 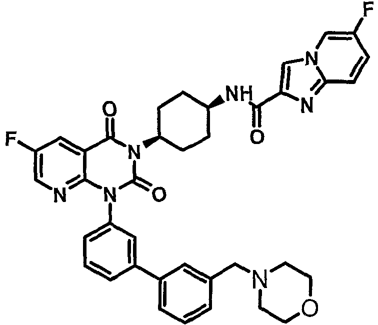 | 6-fluoro-*N*-{*cis*-4-[6-fluoro-1-[3'-(morpholin-4-ylmethyl)biphenyl-3-yl]-2,4-dioxo-1,4-dihydropyrido[2,3-d]pyrimidin-3(2*H*)-yl]cyclohexyl}imidazo[1,2-*a*]pyridine-2-carboxamide. | 1H NMR (400 MHz DMSO-d6) d 8.79 (1H, dd), 8.59 (1H, d), 8.36 (1H, s), 8.33 (1H, dd), 7.79 - 7.71 (3H, m), 7.67 (1H, d), 7.64 - 7.56 (3H, m), 7.46 - 7.37 (3H, m), 7.33 (1H, d), 4.87 (1H, t), 4.20 (1H, s), 3.55 (4H, t), 3.51 (2H, s), 2.65 - 2.56 (2H, m), 2.38 - 2.31 (4H, m), 2.00 (2H, d), 1.77 - 1.62 (4H, m) | 692 |
| 14 | 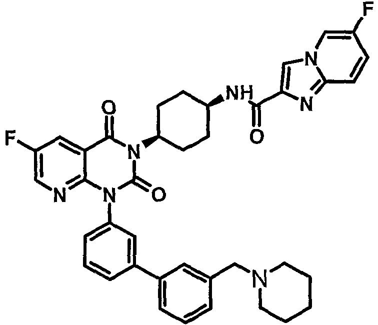 | 6-fluoro-*N*-{*cis*-4-[6-fluoro-2,4-dioxo-1-[3'-(pipexidin-1-ylmethyl)biphenyl-3-yl]-1,4-dihydropyrido[2,3-*d*]pyrimidin-3(2*H*)-yl]cyclohexyl)imidazo[1,2-*a*]pyridine-2-carboxamide. | 1H NMR (400 MHz DMSO-d6) d 8.80 - 8.76 (1H, m), 8.58 (1H, d), 8.37 (1H, s), 8.32 (1H, d), 7.78 - 7.68 (4H, m), 7.62 (1H, t), 7.55 (1H, d), 7.46 - 7.37 (4H, m), 7.30 (1H, d), 4.87 (1H, t), 4.20 (1H, s), 3.46 (2H, s), 2.61 (2H, q), 2.35 - 2.28 (4H, m), 2.00 (2H, d), 1.76 - 1.63 (4H, m), 1.46 (4H, d), 1.39 - 1.34 (2H, m) | 690 |
| 15 | 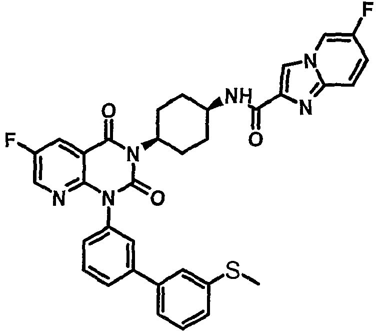 | 6-fluoro-*N*-{*cis*-4-[6-fluoro-1-[3'-(methylthio)biphenyl-3-yl]-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 8.79 (1H, dd), 8.59 (1H, d), 8.36 (1H, s), 8.33 (1H, dd), 7.80 (1H, d), 7.78 - 7.72 (2H, m), 7.68 (1H, d), 7.61 (1H, t), 7.51 (1H, s), 7.47 - 7.38 (4H, m), 7.28 (1H, dt), 4.94 - 4.80 (1H, m), 4.17 (1H, s), 2.68 - 2.54 (2H, m), 2.53 (3H, s), 2.04 - 1.96 (2H, m), 1.77 - 1.62 (4H, m) | 639 |
| 16 | 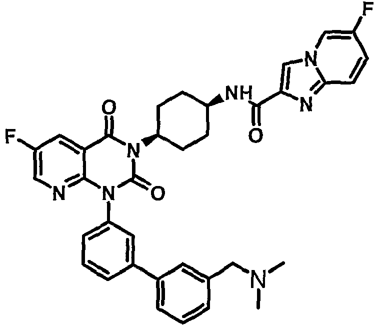 | *N*-{*cis*-*4*-[1-{*3*'-[(dimethylamino)methyl]biphenyl-3-yl}-6-fluoro-2,4-dioxo-1,4-dihydropyrido[2,3-d]pyrimidin-3(2*H*)-yl]cyclohexyl}-6-fluoroimidazo[1,2-*a*]pyridine-2-carboxamide. | 1H NMR (400 MHz CDCl3) d 8.37 (1H, d), 8.23 (1H, dd), 8.10 (1H, s), 8.05 (1H, t), 7.77 (1H, d), 7.74 (1H, d), 7.64 (1H, t), 7.59 - 7.50 (4H, m), 7.39 (1H, t), 7.29 (2H, d), 7.15 (1H, ddd), 5.06 (1H, t), 4.43 (1H, s), 3.47 (2H, s), 2.80 (2H, dd), 2.25 (6H, s), 2.13 (2H, d), 1.87 - 1.70 (4H, m) | 650 |
| 17 | 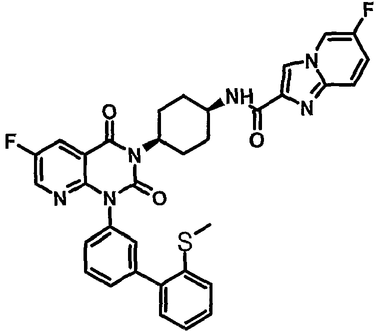 | 6-fluoro-*N*-{*cis*-4-[6-fluoro-1-[2'-(methylthio)biphenyl-3-yl]-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 8.82 8.77 (1H, m), 8.61 (1H, d), 8.37 (1H, d), 8.31 (1H, dd), 7.78 - 7.72 (1H, m), 7.68 (1H, d), 7.58 (1H, t), 7.50 (1H, dt), 7.47 - 7.44 (1H, m), 7.42 (2H, q), 7.39 - 7.36 (2H, m), 7.23 (2H, dd), 4.86 (1H, t), 4.19 (1H, s), 2.65 - 2.54 (2H, m), 2.38 (3H, s), 2.03 - 1.96 (2H, m), 1.76 - 1.61 (4H, m) | 639 |
| 18 | 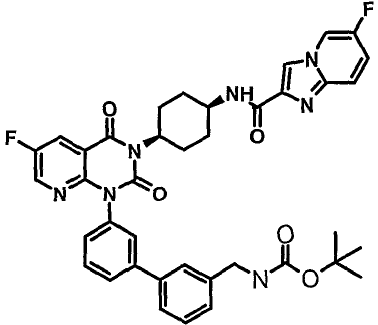 | *tert*-butyl ({3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-3-yl}methyl)carbamate. | 1H NMR (400 MHz DMSO-d6) d 8.79 (1H, s), 8.60 (1H, d), 8.38 - 8.29 (2H, m), 7.79 - 7.72 (2H, m), 7.66 (2H, d), 7.61 (1H, d), 7.55 (2H, d), 7.48 - 7.37 (4H, m), 7.25 (1H, d), 4.87 (1H, t), 4.23 - 4.09 (3H, m), 2.66 - 2.54 (2H, m), 2.06 - 1.97 (2H, m), 1.76 - 1.61 (4H, m), 1.34 (9H, s) | 722 |
| 19 | 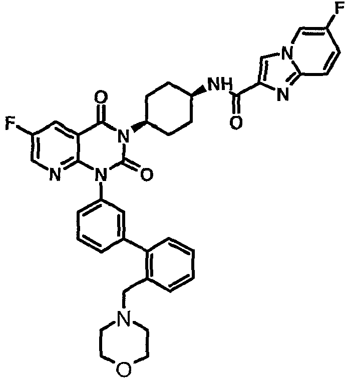 | 6-fluoro-*N*-{*cis*-4-[6-fluoro-1-[2'-(morpholin-4-ylmethyl)biphenyl-3-yl]-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 8.79 (1H, ddd), 8.60 (1H, d), 8.37 (1H, d), 8.33 (1H, dd), 7.77 - 7.71 (1H, m), 7.67 (1H, d), 7.64 - 7.62 (1H, m), 7.58 (1H, t), 7.51 - 7.42 (3H, m), 7.41 - 7.31 (4H, m), 4.93 - 4.81 (1H, m), 4.20 (1H, s), 3.42 - 3.37 (4H, m), 3.35 (2H, s), 2.64 - 2.55 (2H, m), 2.25 (4H, s), 2.03 - 1.94 (2H, m), 1.77 - 1.57 (4H, m) | 692 |
| 20 | 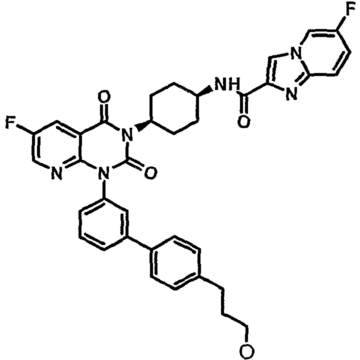 | 6-fluoro-*N*-{*cis*-4-[6-fluoro-1-[4'-(3-hydroxypropyl)biphenyl-3-yl]-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 8.78 (1H, ddd), 8.59 (1H, d), 8.36 (1H, s), 8.33 (1H, dd), 7.74 (2H, ddd), 7.71 - 7.66 (2H, m), 7.62 - 7.56 (3H, m), 7.45 (1H, dd), 7.35 (1H, dd), 7.30 (2H, d), 4.87 (1H, t), 4.47 (1H, t), 4.20 (1H, s), 3.43 (2H, q), 2.68 - 2.58 (4H, m), 2.00 (2H, d), 1.77 - 1.63 (6H, m) | 651 |
| 21 | 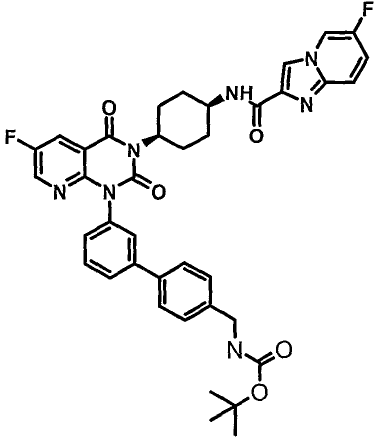 | *tert*-butyl ({3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}methyl)carbamate. | 1H NMR (400 MHz DMSO-d6) d 8.79 (1H, dd), 8.59 (1H, d), 8.36 (1H, s), 8.33 (1H, dd), 7.77 - 7.71 (3H, m), 7.68 (1H, d), 7.61 (3H, q), 7.47 - 7.40 (2H, m), 7.37 (1H, dtd), 7.33 (2H, d), 4.86 (1H, t), 4.16 (3H, d), 2.68 - 2.55 (2H, m), 2.00 (2H, d), 1.78 - 1.62 (4H, m), 1.39 (9H, s) | 722 |
| 22 | 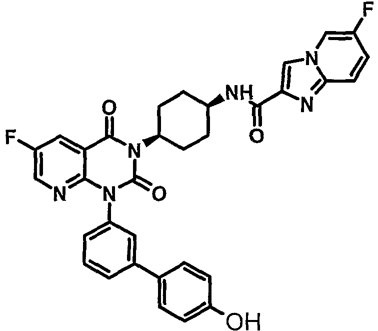 | 6-fluoro-*N*-{*cis*-4-[6-fluoro-1-(4'-hydroxybiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 8.76 (1H, ddd), 8.56 (1H, d), 8.34 (1H, s), 8.29 (1H, dd), 7.72 (1H, dd), 7.65 (2H, d), 7.59 (1H, t), 7.52 (1H, t), 7.47 (2H, d), 7.41 (1H, ddd), 7.27 - 7.23 (1H, m), 6.82 (2H, dd), 4.84 (1H, t), 4.17 (1H, s), 2.58 (2H, d), 1.97 (2H, d), 1.74 - 1.59 (4H, m) | 609 |
| 23 | 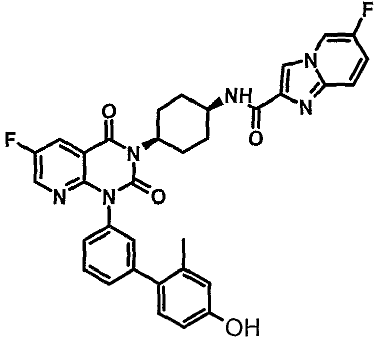 | 6-fluoro-*N*-{*cis*-4-[6-fluoro-1-(4'-hydroxy-2'-methylbiphenyl-3-yl)-2,4-dioxo-1,4-dihydropyrido[2,3-*d*]pyrimidin-3(2*H*)-yl]cyclohexyl]imidazo[1,2-*a*]pyridine-2-carboxamide. | 1H NMR (400 MHz DMSO-d6) d 8.82 - 8.77 (1H, m), 8.60 (1H, d), 8.37 (1H, s), 8.31 (1H, dd), 7.75 (1H, dd), 7.68 (1H, d), 7.56 - 7.43 (2H, m), 7.38 (1H, d), 7.31 (1H, d), 7.05 (1H, d), 6.70 - 6.63 (2H, m), 4.84 (1H, t), 4.21 (1H, s), 2.63 - 2.56 (2H, m), 2.22 (3H, s), 2.02 - 1.93 (2H, m), 1.78 - 1.59 (4H, m) | 623 |
| 24 | 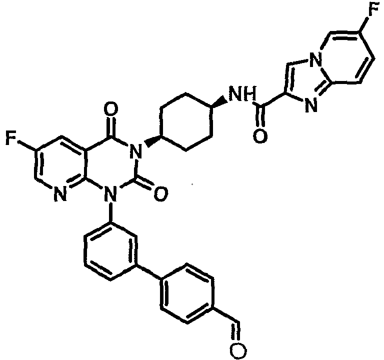 | 6-fluoro-*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 10.06 (1H, s), 8.79 (1H, dd), 8.60 (1H, d), 8.36 (1H, s), 8.34 (1H, dd), 8.03 - 7.99 (2H, m), 7.95 - 7.85 (4H, m), 7.74 (1H, dd), 7.71 - 7.63 (2H, m), 7.50 - 7.40 (2H, m), 4.87 (1H, t), 4.17 (1H, s), 2.68 - 2.55 (2H, m), 2.07 - 1.94 (2H, m), 1.78 - 1.59 (4H, m) | 621 |
| 25 | 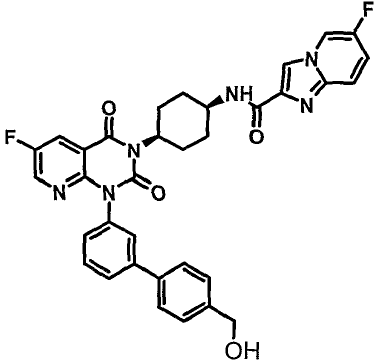 | 6-fluoro-*N*-{*cis-*4-[6-fluoro-1-[4'-(hydroxymethyl)biphenyl-3-yl]-2,4-dioxo-1,4-dihydropyrido[2,3-d]pyrimidin-3(2*H*)-yl]cyclohexyl}imidazo[1,2-*a*]pyridine-2-carboxamide. | 1H NMR (400 MHz DMSO-d6) d 8.78 (1H, ddd), 8.59 (1H, d), 8.36 (1H, s), 8.33 (1H, dd), 7.79 - 7.71 (3H, m), 7.69 - 7.57 (4H, m), 7.47 - 7.39 (3H, m), 7.39 - 7.35 (1H, m), 5.22 (1H, t), 4.92 - 4.82 (1H, m), 4.54 (2H, d), 4.20 (1H, s), 2.68 - 2.54 (2H, m), 2.01 (2H, d), 1.77 - 1.60 (4H, m) | 623 |
| 26 | 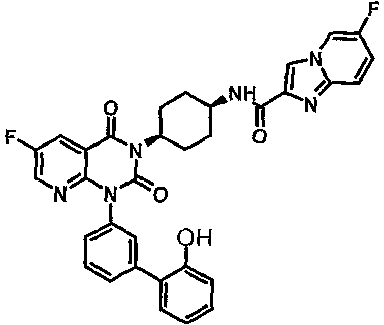 | 6-fluoro-*N*-{*cis*-4-[6-fluoro-1-(2'-hydroxybiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 9.63 (1H, s), 8.79 (1H, dd), 8.60 (1H, d), 8.36 (1H, s), 8.31 (1H, dd), 7.79 - 7.72 (1H, m), 7.70 - 7.64 (2H, m), 7.57 - 7.50 (2H, m), 7.48 - 7.41 (1H, m), 7.28 (2H, dd), 7.16 (1H, d), 6.95 (1H, d), 6.88 (1H, t), 4.91 - 4.77 (1H, m), 4.21 (1H, s), 2.62-2.54 (2H, m), 2.03 - 1.94 (2H, m), 1.77 - 1.61 (4H, m) | 609 |
| 27 | 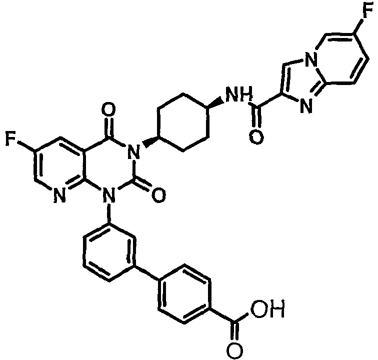 | 3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-d]pyrimidin-1(2*H*)-yl]biphenyl-4-carboxylic acid | 1H NMR (400 MHz DMSO-d6) d 8.81 - 8.78 (1H, m), 8.59 (1H, d), 8.37 (1H, s), 8.33 (1H, dd), 8.03 (2H, d), 7.83 (3H, dd), 7.77 - 7.62 (4H, m), 7.48 - 7.41 (2H, m), 4.91 - 4.84 (1H, m), 4.22 (1H, s), 2.63 - 2.57 (2H, m), 2.06 - 1.96 (2H, m), 1.76 - 1.61 (4H, m) | 637 |
| 28 | 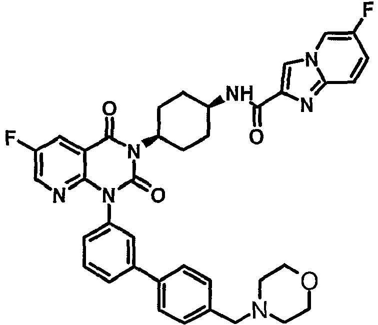 | 6-fluoro-*N*-{*cis*-4-[6-fluoro-1-[4'-(morpholin-4-ylmethyl)biphenyl-3-yl]-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 8.79 (1H, dd), 8.59 (1H, d), 8.36 (1H, s), 8.33 (1H, dd), 7.79 - 7.70 (3H, m), 7.67 (1H, d), 7.64 - 7.58 (3H, m), 7.46 - 7.36 (4H, m), 4.87 (1H, t), 4.22 (1H, s), 3.59 - 3.54 (4H, m), 3.48 (2H, s), 2.63 - 2.57 (2H, m), 2.38 - 2.34 (4H, m), 2.02 - 1.96 (2H, m), 1.76 - 1.62 (4H, m) | 692 |
| 29 | 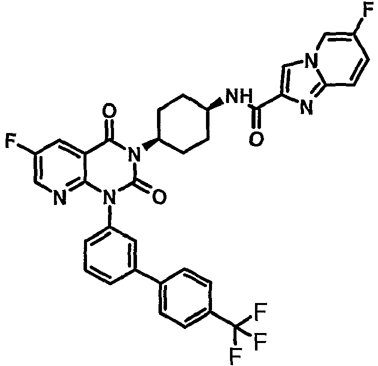 | 6-fluoro-*N*-{*cis*-4-[6-fluoro-2,4-dioxo-1-[4'-(trifluoromethyl)biphenyl-3-yl]-1,4-dihydropyrido[2,3-*d*]pyrimidin-3(2*H*)-yl]cyclohexyl}imidazo[1,2-a]pyridine-2-carboxamide. | 1H NMR (400 MHz DMSO-d6) d 8.79 (1H, dd), 8.60 (1H, d), 8.37 - 8.32 (2H, m), 7.93 - 7.87 (3H, m), 7.86 - 7.82 (3H, m), 7.77 - 7.64 (3H, m), 7.50 - 7.43 (2H, m), 4.87 (1H, t), 4.16 (1H, s), 2.68 - 2.54 (2H, m), 2.08 - 1.94 (2H, m), 1.80 - 1.58 (4H, m) | 661 |
| 30 | 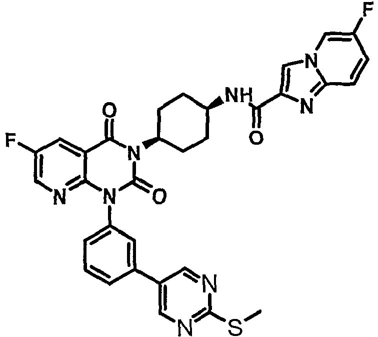 | *6*-fluoro-*N*-{*cis*-4-[6-fluoro-1-{3-[2-(methylthio)pyrimidin-5-yl]phenyl}-2,4-dioxo-1,4-dihydropyrido[2,3-*d*]pyrimidin-3(2*H*)-yl]cyclohexyl}imidazo[1,2-*a*]pyridine-2-carboxamide. | 1H NMR (400 MHz DMSO-d6) d 8.98 (2H, s), 8.79 (1H, dd), 8.60 (1H, d), 8.37 - 8.33 (2H, m), 7.92 - 7.86 (2H, m), 7.74 (1H, dd), 7.71 - 7.64 (2H, m), 7.50-7.41 (2H, m), 4.88 (1H, t), 4.23 (1H, s), 2.64 - 2.58 (2H, m), 2.56 (3H, s), 2.01 (2H, d), 1.78 - 1.62 (4H, m) | 641 |

### Example 31

### N-{cis-4-[1-[3'-(aminomethyl)biphenyl-3-yl]-6-fluoro-2,4-dioxo-1,4-dihydropyrido[2,3-d]pyrimidin-3(2H)-yl]cyclohexyl}-6-fluoroimidazo[1,2-a]pyridine-2-carboxamide. Hydrochloride

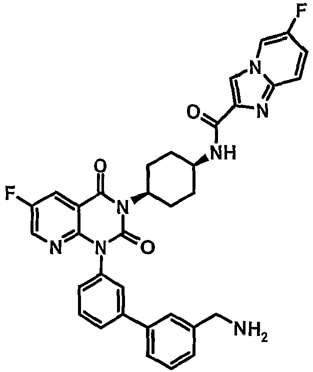

To a solution of tert-butyl ({3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-3-yl}methyl)carbamate (150 mg, 0.21 mmol) (example 18) in 1,4-dioxane (2 ml) was added hydrogen chloride 4 M in 1,4-dioxame (1 ml) and the reaction mixture was stirred at room temperature for 30 min. The resulting solid was filtered off and washed with ether, dried in the oven under vacuum to afford the title compound as a white solid (130 mg).
¹H NMR (400 MHz, DMSO-*d₆*) δ 8.90 (1H, d), 8.58 (1H, d), 8.49 (1H, s), 8.36 (1H, s), 8.32 (1H, dd), 7.88 (1H, d); 7.83 (1H, s), 7.81 - 7.74 (2H, m), 7.72 (1H, t), 7.67 (1H, dt), 7.63 (1H, t), 7.55 (1H, td), 7.52 - 7.44 (1H, m), 7.41 - 7.38 (1H, m), 4.85 (1H, t), 4.12 (1H, s), 4.06 (2H, q), 2.67 - 2.54 (2H, m), 2.02 (2H, d), 1.76 - 1.58 (4H, m)
APCI (Multimode) m/z: 622 [M+H]

### Example 32

### N-{cis-4-[1-[4'-(aminomethyl)biphenyl-3-yl]-6-fluoro-2,4-dioxo-1,4-dihydropyrido[2,3-d]pyrimidin-3(2H)-yl]cyclohexyl}-6-fluoroimidazo[1,2-a]pyridine-2-carboxamide. Hydrochloride.

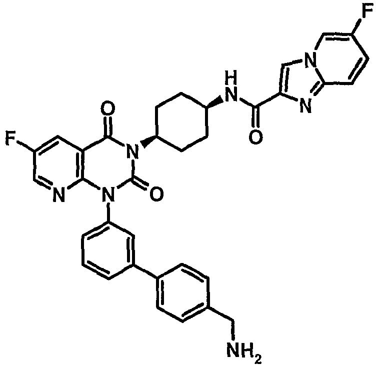

To a solution of tert-butyl ({3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl}methyl)carbamate (50 mg, 0.069 mmol) (example 21) in 1,4-dioxane (1 ml) was added hydrogen chloride 4 M in 1,4-dioxane (1 ml) and the reaction mixture was stirred at room temperature for 1 h. The resulting solid was filtered off and washed with ether, dried in the oven under vacuum to give the title compound as a white solid (42 mg). ¹H NMR (400 MHz, DMSO-*d₆*) δ 8.92 (1H, s), 8.59 (1H, d), 8.51 (1H, s), 8.42 - 8.36 (2H, m), 8.33 (1H, dd), 7.91 (1H, d), 7.82 - 7.72 (4H, m), 7.63 (2H, t), 7.58 (2H, d), 4.87 (1H, t), 4.18 - 4.02 (5H, m), 2.71 - 2.58 (2H, m), 2.08 - 2.00 (2H, m), 1.77 - 1.61 (4H, m)
APCI (Multimode) m/z: 622 [M+H]

### Example 33

### N-{cis-4-[1-{2'-[(dimethylamino)methyl]biphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide.

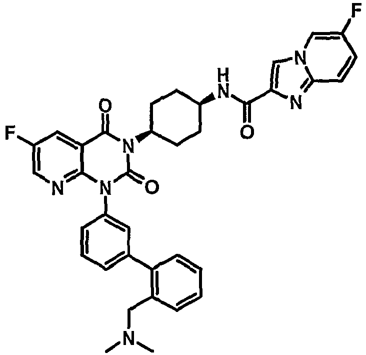

### Step (a) tert-butyl {cis-4-[1-{2'-[(dimethylamino)methyl]biphenyl-3-yl}-6-fluoro-2,4-dioxo-1,4-dihydropyrido[2,3-d]pyrimidin-3(2H)-yl]cyclohexyl}carbamate

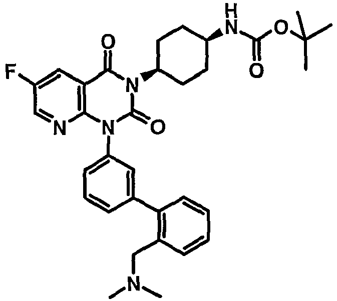

Acetonitrile (3 ml) was added to a mixture of palladium(II) acetate (4 mg) and 2-(dicyclohexylphosphino)-2',6'-dimethoxy-1,1'-biphenyl (14 mg) and the mixture was stirred at room temperature for 10 min. Potassium carbonate (143 mg) in water (2 ml), 2-(N,N-dimethylaminomethyl)phenylboronic acid (90 mg) and tert-butyl {cis-4-[6-fluoro-1-(3-iodophenyl)-2,4-dioxo-1,4-dihydropyrido[2,3-d]pyrimidin-3(2H)-yl]cyclohexyl}carbamate (200 mg) were added and the reaction mixture was heated to reflux for 2 h. and allowed to cool overnight. The mixture was loaded onto a Varian Bond Chemelut cartridge (available from Kinesis), the product eluted with DCM and concentrated in vacuo. The residue was purified by flash column chromatography eluting the product with 0.1% triethylamine in ethyl acetate to afford the sub-title compound as a pale brown solid (73 mg).

¹H NMR (300 MHz, DMSO-d₆) δ 8.59 (1H, d), 8.28 (1H, dd), 7.61 - 7.44 (3H, m), 7.39 - 7.28 (4H, m), 6.48 (1H, s), 4.77 (1H, t), 3.58 (1H, s), 3.33 (2H, s), 2.62 - 2.55 (2H, m), 2.11 (6H, s), 1.94 - 1.84 (2H, m), 1.56 - 1.41 (4H, m), 1.44 (9H, s)
APCI (Multimode) m/z: 588 [M+H]

### Step (b) 3-(cis-4-aminocyclohexyl)-1-{2'-[(dimethylamino)methyl]biphenyl-3-yl}-6-fluoropyrido[2,3-d]pyrimidine-2,4(1H,3H)-dione Hydrochloride

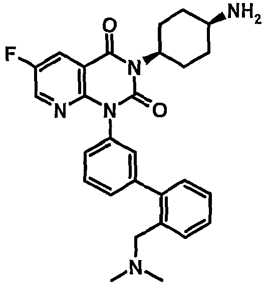

To a solution of tert-butyl {cis-4-[1-{2'-[(dimethylamino)methyl]biphenyl-3-yl}-6-fluoro-2,4-dioxo-l,4-dihydropyrido[2,3-d]pyrimidin-3(2H)-yl]cyclohexyl}carbamate (70 mg) in 1,4-dioxane (0.5 ml) was added hydrogen chloride 4M in 1,4-dioxane (0.5 ml) and the reaction mixture was stirred at room temperature overnight. The solvents were removed under vacuum to afford the sub-title compound (70 mg).
APCI (Multimode) m/z: 525 [M+H]

Step (c) To a mixture of 6-fluoro-imidazo[1,2-a]pyridine-2-carboxylic acid (30mg, 0.15 mmol), Hunigs base (64 mg, 0.5 mmol) in dry DMF (5 ml) was added HATU (57 mg, 0.15mmol)). The mixture was allowed to stir for 10 mins at room temperature. To this mixture was added 3-(cis-4-aminocyclohexyl)-1-{2'-[(dimethylamino)methyl]biphenyl-3-yl}-6-fluoropyrido[2,3-d]pyrimidine-2,4(1H,3H)-dione Hydrochloride (68 mg, 0.13 mmol) and the reaction mixture stirred overnight. The mixture was poured into water and the solid collected and purified by HPLC (acetonitrile and 0.1 % ammonia in water) and lyophilised to afford the title compound (30 mg).
¹H NMR (300 MHz, DMSO-*d₆*) δ 8.80 (1H, dd), 8.60 (1H, d), 8.37 (1H, s), 8.34 - 8.31 (1H, m), 7.77 - 7.21 (11H, m), 4.87 (1H, t), 4.16 (1H, s), 3.31 (2H, d), 2.63 (1H, m), 2.00 (9H, m), 1.69 (4H, m)
APCI (Multimode) m/z: 651 [M+H]

### Example 34

### N-{cis-4-[1-(3-benzylphenyl)-6-fluoro-2,4-dioxo-1,4-dihydropyrido[2,3-d]pyrimidin-3(2H)-yl]cyclohexyl}imidazo[1,2-a]pyridine-2-carboxamide.

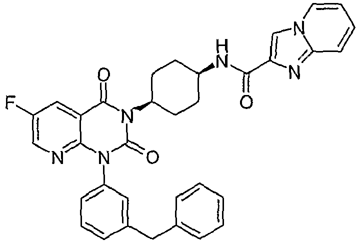

### Step (a) 2-[(3-benzylphenyl)amino]-5-fluoronicotinic acid

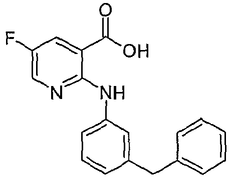

2-Chloro-5-fluoronicotinic acid (3.65 g, 21 mmol), potassium carbonate (3.6 g, 26 mmol), 3-benzylaniline (5.4 g, 30mmol), copper(I)bromide (155 mg, 1.2 mmol) and copper (80 mg, 1.3 mmol) were weighed into a round bottomed flask and N-methylpyrrolidinone (15 mL) was added. After degassing the mixture it was heated under nitrogen at 150 °C for 2 h. The black reaction mixture was poured onto water and the mixture was stirred overnight. The obtained solid was filtered, washed with water and dried in air to yield the sub-title compound as a pale brown solid (1.3 g, 18%) which was used without further purification in the subsequent step.
APCI (Multimode) m/z: 323 [M+H].

### Step (b) N-{cis-4-[({2-[(3-benzylphenyl)amino]-5-fluoropyridin-3-yl}carbonyl)amino]cyclohexyl}imidazo[1,2-a]pyridine-2-carboxamide

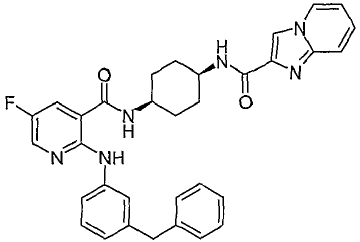

To 2-[(3-benzylphenyl)amino]-5-fluoronicotinic acid (1.22 g, 3.4 mmol) and N-(cis-4-aminocyclohexyl)-imidazo[1,2-a]pyridine-2-carboxamide (1.07 g, 3.4 mmol) was added acetonitrile (20 mL) and triethylamine (2 mL, 14 mmol). 2,4,6-Tripropyl-1,3,5,2,4,6-trioxatriphosorinan-2,4,6-trioxide (2.5 mL, 4 mmol) (50% wt solution in butyl acetate) was added dropwise over 15 min. and the reaction mixture was stirred at room temperature for 5 min. The solid was then collected by filtration and washed with water. Acetonitrile was added and the suspension heated, allowed to cool and the solid collected by filtration and dried to afford the sub-title compound (0.9 g, 47%).
APCI (Multimode) m/z: 563 [M+H].

### Step (c) N-{cis-4-[1-(3-benzylphenyl)-6-fluoro-2,4-dioxo-1,4-dihydropyrido[2,3-d]pyrimidin-3(2H)-yl]cyclohexyl}imidazo[1,2-a]pyridine-2-carboxamide

To a solution of N-{cis-4-[({2-[(3-benzylphenyl)amino]-5-fluoropyridin-3-yl}carbonyl)amino]cyclohexyl}imidazo[1,2-a]pyridine-2-carboxamide (0.116 g, 0.21 mmoles) in N,N-dimethylformamide (5 mL) at room temperature was added 1,1'-carbonyldiimidazole (0.1672 g, 1.03 mmoles) and 60% w/w sodium hydride in mineral oil (0.041 g, 1.03 mmoles). The reaction was heated to 70 °C for 30 min., before cooling to room temperature and stirring for a further 16 h. The reaction was quenched by addition to water (50 mL) which was then extracted with ethyl acetate (6 x 50 mL). The organic phases were combined, dried with anhydrous sodium sulphate and concentrated to give the crude product as a pale brown solid. The residue was purified by reverse phase HPLC to the title compound as a colourless solid (0.051 g, 42%).
¹H NMR (400 MHz, DMSO-*d6*) δ 8.60 - 8.55 (2H, m), 8.39 (1H, s), 8.31 - 8.28 (1H, m), 7.43 (2H, t), 7.37 - 7.16 (10H, m), 6.98 (1H, m), 6.98 (1H, t), 4.88 - 4.79 (1H, m), 4.20 - 4.13 (1H, m), 4.00 (2H, s), 2.68 - 2.52 (2H, m), 2.04 - 1.95 (2H, m), 1.76 - 1.60 (4H, m).
APCI (Multimode) m/z: 589 [M+H]

### Example 35

### N-{cis-4-[1-(1-benzyl-1H-indazol-5-yl)-6-fluoro-2,4-dioxo-1,4-dihydropyrido[2,3-d]pyrimidin-3(2H)-yl]cyclohexyl}imidazo[1,2-a]pyridine-2-carboxamide.

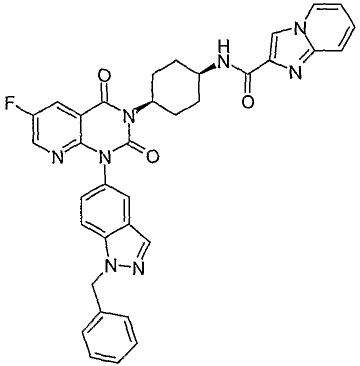

### Step (a) 2-[(1-benzyl-1H-indazol-5-yl)amino]-5-fluoronicotinic acid

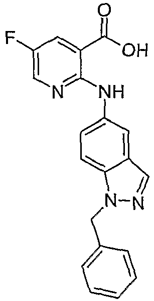

1-Benzyl-1H-indazol-5-ylamine (1.018 g, 4.56 mmoles), potassium carbonate (0.378 g, 2.74 mmoles), copper (0.015 g, 0.23 mmoles) and copper(I) bromide (0.033 g, 0.23 mmoles) were added to a stirred solution of 2-chloro-5-fluoronicotinic acid (0.4 g, 2.28 mmoles) in N,N-dimethylformamide (5 mL). The solution was heated to 120 °C for a period of 1 hr then the reaction was cooled and the DMF evaporated. The resultant solid plug was dissolved in 1M HCl (50 mL) and extracted three times into ethyl acetate (50 mL) which was then combined and dried with anhydrous sodium sulphate, filtered and concentrated *in vacuo* to give the crude product as a red-brown solid. The crude was redissolved in ethyl acetate (50 mL), washed three times with 1M HCl (50 mL) then once with saturated aqueous sodium hydrogencarbonate solution (50 mL). The organic layer was again dried and concentrated to give the sub-title compound as a brown film (99 mg, 12%).
APCI (Multimode) m/z: 362 [M+H].

### Step (b) N-{cis-4-[({2-[(1-benzyl-1H-indazol-5-yl)amino]-5-fluoropyridin-3-yl}carbonyl)amino]cyclohexyl}imidazo[1,2-a]pyridine-2-carboxamide

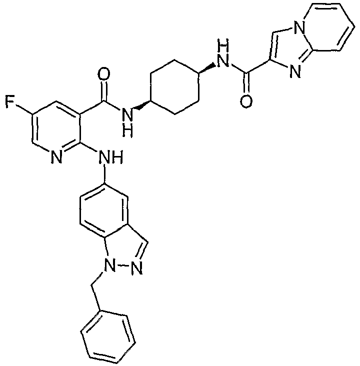

To 2-[(1-benzyl-1H-indazol-5-yl)amino]-5-fluoronicotinic acid (2.05 g, 3.4 mmol) and N-(cis-4-aminocyclohexyl)imidazo[1,2-a]pyridine-2-carboxamide (1.07 g, 3.4 mmol) was added acetonitrile (20 mL) and triethylamine (2 mL, 14 mmol). 2,4,6-Tripropyl-1,3,5,2,4,6-trioxatriphosorinan-2,4,6-trioxide (2.5 mL, 4 mmol) (50% wt solution in butyl acetate) was added dropwise over 15 min. and the reaction mixture was stirred at room temperature for 5 min. The resultant solid was then collected by filtration and washed with water. Acetonitrile was added and the suspension heated, allowed to cool and the solid collected by filtration and dried to afford the sub-title compound (199 mg 10%).
APCI (Multimode) m/z: 603 [M+H].

### Step (c) N-{cis-4-[1-(1-benzyl-1H-indazol-5-yl)-6-fluoro-2,4-dioxo-1,4-dihydropyrido[2,3-d]pyrimidin-3(2H)-yl]cyclohexyl}imidazo[1,2-a]pyridine-2-carboxamide

To a solution of N-{cis-4-[({2-[(1-benzyl-1H-indazol-5-yl)amino]-5-fluoropyridin-3-yl}carbonyl)amino]cyclohexyl}imidazo[1,2-a]pyridine-2-carboxamide (0.122 g, 0.2 mmoles) in N,N-dimethylformamide (3 mL) at room temperature was added 1,1'-carbonyldiimidazole (0.1641 g, 1.01 mmoles) and 60% w/w sodium hydride in mineral oil (0.0405 g, 1.01 mmoles). The reaction was then heated to 70 °C for 30 min. in a nitrogen flushed sample vial before cooling to room temperature and stirring for a further 48 h. The reaction was quenched by addition to brine (50 mL) and extracted with ethyl acetate (6 x 50 mL). The organic phase was washed once with water then dried with anhydrous sodium sulphate and concentrated to give the crude product as an orange oil. The residue was purified by reverse phase HPLC giving the title compound as a colourless solid (0.065 g, 51%).
¹H NMR (400MHz, DMSO-d6) 8.70 (m, 1H), 8.58 - 8.50 (m, 2H), 8.33 - 8.28 (m, 1H), 8.19 (m, 1H), 7.99 (s, 1H), 7.83 - 7.77 (m, 2H), 7.69 (d, 1H), 7.52 (t, 1H), 7.37 - 7.26 (m, 6H), 7.17 - 7.11 (m, 1H), 5.71 (s, 2H), 4.87 (t, 1H), 4.14 (s, 1H), 2.70 - 2.57 (m, 2H), 2.04 (m, 2H), 1.77 - 1.60 (m, 4H).
APCI (Multimode) m/z: 629 [M+H]

### Example 36

### N-{cis-4-[1-(1,5-dimethylhexyl)-6-fluoro-2,4-dioxo-1,4-dihydropyrido[2,3-d]pyrimidin-3 (2H)-yl]cyclohexyl}-6-fluoroimidazo[1,2-a]pyridine-2-carboxamide.

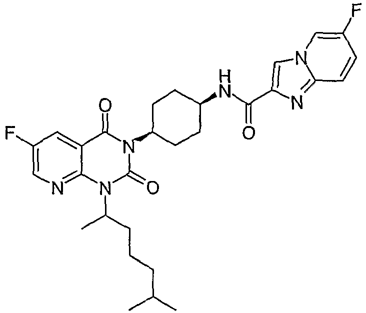

### Step (a) 2-[(1,5-dimethylhexyl)amino]-5-fluoronicotinic acid

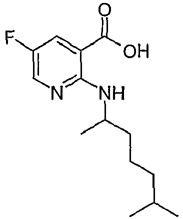

2-amino-6-methylheptane (0.589 g, 4.56 mmoles), potassium carbonate (0.378 g, 2.74 mmoles), copper (0.015 g, 0.23 mmoles) and copper(I) bromide (0.033 g, 0.23 mmoles) were added to a stirred solution of 2-chloro-5-fluoronicotinic acid (0.4 g, 2.28 mmoles) in N,N-dimethylformamide (5 mL). The solution was heated to 120 °C for a period of 2 h. The DMF was evaporated prior to aqueous workup. Resultant solid plug dissolved in 1M HCl (50 mL) and extracted three times into ethyl acetate (50 mL) which was combined and dried with anhydrous sodium sulphate, filtered and concentrated *in vacuo* to give the sub-title compound as a pale orange solid (509 mg, 81%).
APCI (Multimode) m/z: 269 [M+H].

### Step (b) N-{cis-4-[{2-[(1,5-dimethylhexyl)amino]-5-fluoropyridin-3-yl}carbonyl)amino]cyclohexyl}-6-fluoroimidazo[1,2-a]pyridine-2-carboxamide

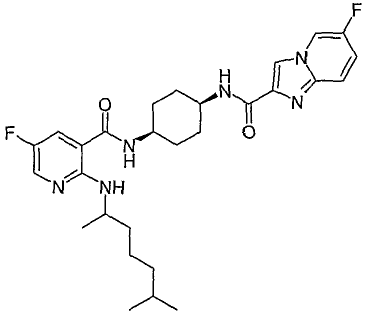

To a solution of 2-[(1,5-dimethylhexyl)amino]-5-fluoronicotinic acid (0.153 g, 0.57 mmoles), triethylamine (0.346 g, 3.42 mmoles) and 6-Fluoro-imidazo[1,2-a]pyridine-2-carboxylic acid (4-amino-cyclohexyl)-amide (0.1961 g, mono HCl adduct, 0.63 mmoles) in acetonitrile (0.5 mL) was added 2,4,6-tripropyl-1,3,5,2,4,6-trioxatriphosorinan-2,4,6-trioxide (0.2176 g, 0.436 mL, 50% wt solution in butyl acetate, 0.22 mmoles) dropwise. The reaction was stirred for 10 min. at room then quenched by the addition of water (50 mL) and the aqueous layer washed with ethyl acetate (3 x 50 mL). Crude obtained as a yellow oil which was purified by flash column chromatography eluting with 1:1 ethyl acetate:petroleum ether - 5% methanol/ethyl acetate to give the sub-title compound as a yellow solid (115 mg, 38%).
APCI (Multimode) m/z: 528 [M+H].

### Step (c) N-{cis-4-[1-(1,5-dimethylhexyl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

To a solution of N-{cis-4-[({2-[(1,5-dimethylhexyl)amino]-5-fluoropyridin-3-yl}carbonyl)amino]cyclohexyl}-6-fluoroimidazo[1,2-a]pyridine-2-carboxamide (0.106 g, 0.2 mmoles) in N,N-dimethylformamide (3 mL) at room temperature was added 1,1'-carbonyldiimidazole (0.1632 g, 1 mmoles) and 60% w/w sodium hydride in mineral oil (0.040 g, 1 mmoles). The reaction was then heated to 70 °C for 30 min. in a nitrogen flushed sample vial before cooling to room temperature and stirring for a further 48 h. The reaction was quenched by addition to brine (50 mL) and extracted with ethyl acetate (3 x 50 mL). Organic layers were then washed once more with brine (50 mL), dried with anhydrous sodium sulphate, filtered and concentrated *in vacuo* to give the crude as an orange oil. The residue was purified by reverse phase HPLC, giving the title compound as a colourless solid (0.042 g, 38%).
¹H NMR (400 MHz, DMSO-*d6*) 8.96 - 8.76 (m, 1H), 8.73 - 8.69 (m, 1H), 8.40 - 8.36 (1H, m), 8.22 - 8.15 (1H, m), 7.77 - 7.68 (2H, m), 7.48 - 7.41 (1H, m), 5.56 - 5.40 (1H, m), 4.92 - 4.80 (1H, m), 4.24 - 4.16 (1H, m), 2.69 - 2.58 (2H, m), 2.21 - 2.11 (1H, m), 2.05 - 1.97 (2H, m), 1.87 - 1.68 (2H, m), 1.62 - 1.54 (2H, m), 1.51 - 1.40 (5H, m), 1.27 - 1.07 (4H, m), 0.82 - 0.72 (6H, m)
APCI (Multimode) m/z: 553 [M+H]

### Example 37

### N-{cis-4-[1-(4-benzylphenyl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide.

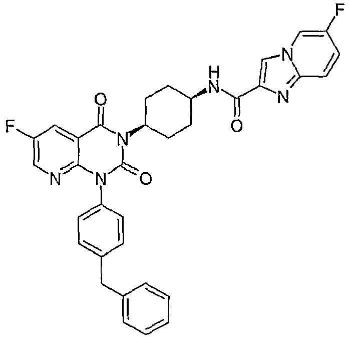

### Step (a) 2-[(4-benzylphenyl)amino]-5-fluoronicotinic acid

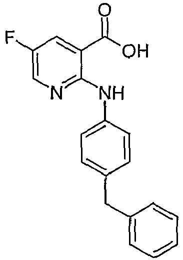

4-aminodiphenylmethane (0.835 g, 4.56 mmoles), potassium carbonate (0.378 g, 2.74 mmoles), copper (0.015 g, 0.23 mmoles) and copper(I) bromide (0.033 g, 0.23 mmoles) were added to a stirred solution of 2-chloro-5-fluoronicotinic acid (0.4 g, 2.28 mmoles) in N,N-dimethylformamide (5 mL). The solution was heated to 120 °C for a period of 2 h then the DMF was evaporated prior to aqueous workup. The resultant solid plug was dissolved in 1M HCl (50 mL) and extracted three times into ethyl acetate (50 mL) which was then combined and dried with anhydrous sodium sulphate, filtered and concentrated *in vacuo* to give the crude product as a brown solid. The crude was redissolved in ethyl acetate (50 mL), washed three times with 1M HCl (50 mL) then once with saturated aqueous sodium hydrogencarbonate solution (50 mL). The organic layer was again dried and concentrated to give the sub-title compound as a brown solid (603 mg, 82%).
APCI (Multimode) m/z: 323 [M+H]

### Step (b) N-{cis-4-[({2-[(4-benzylphenyl)amino]-5-fluoropyridin-3-yl} carbonyl)amino]cyclohexyl}-6-fluoroimidazo[1,2-a]pyridine-2-carboxamide

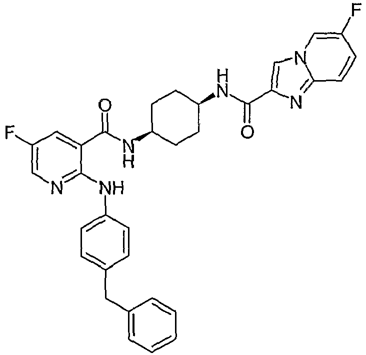

To a suspension of 2-[(4-benzylphenyl)amino]-5-fluoronicotinic acid (0.184 g, 0.57 mmoles) triethylamine (0.346 g, 3.42 mmoles) and 6-Fluoro-imidazo[1,2-a]pyridine-2-carboxylic acid (4-amino-cyclohexyl)-amide (0.1961 g, mono HCl adduct, 0.63 mmoles) in acetonitrile (1 mL) was added 2,4,6-tripropyl-1,3,5,2,4,6-trioxatriphosorinan-2,4,6-trioxide (0.2176 g, 0.436 mL, 50% wt solution in butyl acetate, 0.68 mmoles) dropwise. The reaction was stirred for 30 min. at room temperature then the acetonitrile was removed *in vacuo,* and the residue partitioned between ethyl acetate and water (50 mL of each). The aqueous layer was washed with further ethyl acetate (2 x 50 mL) then the organic phases were combined and dried with anhydrous sodium sulphate to give the sub-title compound as an orange oil (222 mg, 67%).
APCI (Multimode) m/z: 581 [M+H]

### Step (c) N-{cis-4-[1-(4-benzylphenyl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

To a solution of N-{cis-4-[({2-[(4-benzylphenyl)amino]-5-fluoropyridin-3-yl}carbonyl)amino]cyclohexyl}-6-fluoroimidazo[1,2-a]pyridine-2-carboxamide (0.21 g, 0.36 mmoles) in N,N-dimethylformamide (5 mL) at room temperature was added 1,1'-carbonyldiimidazole (0.2935 g, 1.81 mmoles) and sodium hydride in mineral oil (60% w/w, 0.0724 g, 1.81 mmoles). The reaction was then heated to 70 °C for 30 min. in a nitrogen flushed sample vial, before cooling to room temperature and stirring for a further 16 h. The reaction was quenched by addition to brine (50 mL) and extracted with ethyl acetate (3 x 50 mL). The combined organic layers were washed with water (50 mL), dried with anhydrous sodium sulphate, filtered and concentrated *in vacuo* to give very little material, and none of the desired product. LC analysis indicated that the product had remained with the aqueous layer, in the form of a suspension. This was filtered and the light brown filtrate washed with copious deionised water. This material was purified by reverse phase HPLC to yield the title compound as a pale yellow solid (57 mg, 27%).

¹H NMR (400 MHz, DMSO-*d6*) 8.86 (1H, m), 8.55 (1H, d), 8.44 (1H, s), 8.29 (1H, m), 7.80 - 7.74 (2H, m), 7.54 (1H, m), 7.39 - 7.20 (10H, m), 4.84 (1H, t), 4.18 (1H, s), 4.01 (2H, s), 2.65 - 2.52 (2H, m), 2.04 - 1.96 (2H, m), 1.76 - 1.57 (4H, m).
APCI (Multimode) m/z: 607 [M+H]

### Example 38

### 6-fluoro-N-{cis-4-[6-fluoro-2,4-dioxo-1-(2-phenylethyl)-1,4-dihydropyrido[2,3-d]pyrimidin-3(2H-yl]cyclohexyl)imidazo[1,2-a]pyridine-2-carboxamide.

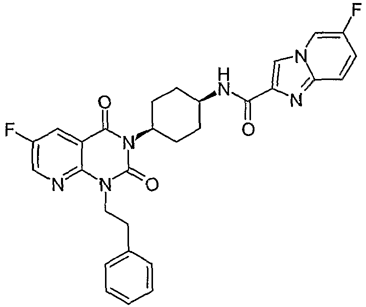

### Step (a) 5-fluoro-2-[(2-phenylethyl)amino]nicotinic acid

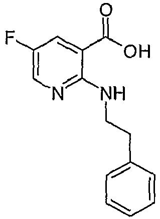

2-Phenylethylamine (0.551 g, 4.56 mmoles), potassium carbonate (0.378 g, 2.74 mmoles), copper (0.015 g, 0.23 mmoles) and copper(I) bromide (0.033 g, 0.23 mmoles) were added to a stirred solution of 2-chloro-5-fluoronicotinic acid (0.4 g, 2.28 mmoles) in N,N-dimethylformamide (5 mL). The solution was heated to 120 °C for a period of 1 hr. The reaction was quenched by addition to 1M HCl (50 mL) and extracted three times into ethyl acetate (50 mL) which was then combined and dried with anhydrous sodium sulphate, filtered and concentrated *in vacuo* to give the crude product as a yellow solid. Reaction was re-worked up by dissolving in ethyl acetate (50 mL) and washing sequentially with 1M HCl (3 x 50 mL) and saturated sodium hydrogencarbonate solution. The organic phase was dried with anhydrous sodium sulphate and concentrated to give the sub-title compound as a pale yellow oil (236 mg, 40%).
APCI (Multimode) m/z: 523 [M+H]

### Step (b) 6-fluoro-N-{cis-4-[({5-fluoro-2-[(2-phenylethyl)amino]pyridin-3-yl}carbonyl)amino]cyclohexyl}imidazo[1,2-a]pyridine-2-carboxamide

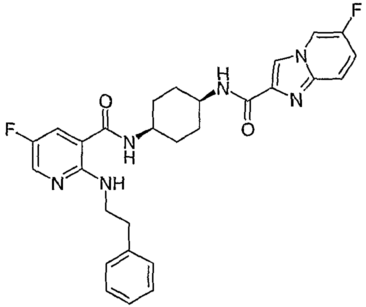

To a suspension of 5-fluoro-2-[(2-phenylethyl)amino]nicotinic acid (0.184 g, 0.57 mmoles) triethylamine (0.429 g, 0.591 mL, 3.42 mmoles) and 6-Fluoro-imidazo[1,2-a]pyridine-2-carboxylic acid (4-amino-cyclohexyl)-amide (0.2432 g, mono HCl adduct, 0.62 mmoles) in acetonitrile (1 mL) was added 2,4,6-tripropyl-1,3,5,2,4,6-trioxatriphosorinan-2,4,6-trioxide (0.270 g, 0.54 mL, 50% wt solution in butyl acetate, 0.684 mmoles) dropwise. Reaction stirred for 60 min. at room temperature before the acetonitrile was removed *in vacuo* and the residue partitioned between ethyl acetate and water (50 mL of each). The aqueous layer was washed with further ethyl acetate (2 x 50 mL) then the organic phases were combined and dried with anhydrous sodium sulphate to give the sub-title compound as a yellow oil (253 mg, 69%), which was used in the next step without further purification.

### Step (c) 6-fluoro-N-{cis-4-[6-fluoro-2,4-dioxo-1-(2-phenylethyl)-1,4-dihydropyrido[2,3-d]pyrimidin-3(2H)-yl]cyclohexyl}imidazo[1,2-a]pyridine-2-carboxamide

To a solution of 6-fluoro-*N*-{*cis*-4-[({5-fluoro-2-[(2-phenylethyl)amino]pyridin-3-yl}carbonyl)amino]cyclohexyl}imidazo[1,2-*a*]pyridine-2-carboxamide (0.25 g, 0.48 mmoles) in N,N-dimethylformamide (3 mL) at room temperature was added 1,1'-carbonyldiimidazole (0.163 g, 2.41 mmoles) and sodium hydride in mineral oil (60% w/w, 0.040 g, 2.41 mmoles). The reaction was then heated to 70 °C for 30 min., before cooling to room temperature and stirring for a further 48 h. The reaction was quenched by addition to brine (50 mL) and extracted with ethyl acetate (3 x 50 mL). Organic layers were then washed twice more with brine (50 mL) dried with anhydrous sodium sulphate, filtered and concentrated *in vacuo* to give the crude as a yellow oil. This material was purified by reverse phase HPLC to yield the title compound as a colourless solid (72 mg, 27%).
¹H NMR (DMSO-*d6*) 8.91 - 8.86 (1H, m), 8.81 (1H, d), 8.46 (1H, s), 8.29 - 8.24 (1H, m), 7.88 - 7.79 (2H, m), 7.60 - 7.53 (1H, m), 7.33 - 7.19 (5H, m), 4.84 (1H, t), 4.47 - 4.39 (2H, m), 4.20 (1H, s), 2.94 (2H, t), 2.66 - 2.54 (2H, m), 2.05 - 1.98 (2H, m), 1.77 - 1.51 (4H, m). APCI (Multimode) m/z: 545 [M+H]

### Example 39

### N-{cis-4-[1-(1-benzyl-1H-indazol-5-yl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide.

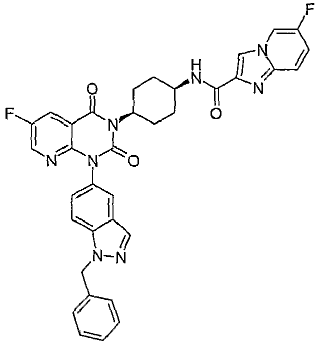

### Step (a) N-{cis-4-[({2-[(1-benzyl-1H-indazol-5-yl)amino]-5-fluoropyridin-3-yl}carbonyl)amino]cyclohexyl}-6-fluoroimidazo[1,2-a]pyridine-2-carboxamide

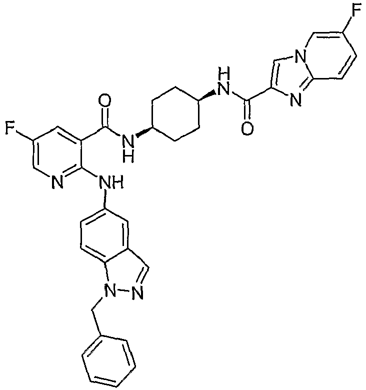

To a suspension of 2-[(1-benzyl-1H-indazol-5-yl)amino]-5-fluoronicotinic acid (0.104 g, 0.29 mmoles), triethylamine (0.174 g, 0.24 mL, 1.72 mmoles) and 6-Fluoro-imidazo[1,2-a]pyridine-2-carboxylic acid (4-amino-cyclohexyl)-amide (0.099 g, mono HCl adduct, 0.32 mmoles) in acetonitrile (1 mL) was added 2,4,6-tripropyl-1,3,5,2,4,6-trioxatriphosorinan-2,4,6-trioxide (0.110 g, 0.219 mL, 50% wt solution in butyl acetate, 0.34 mmoles) dropwise. The reaction was stirred for 60 min. at room temperature then the acetonitrile was removed *in vacuo* and the residue partitioned between ethyl acetate and water (50 mL of each). The aqueous layer was washed with further ethyl acetate (2 x 50 mL) then the organic phase was combined and dried with anhydrous sodium sulphate to give the sub-title compound as a brown foam (175 mg, 98%).
APCI (Multimode) m/z: 621 [M+H].

### Step (b) N-{cis-4-[1-(1-benzyl-1H-indazol-5-yl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

To a solution of *N*-{*cis*-4-[({2-[(1-benzyl-1*H*-indazol-5-yl)amino]-5-fluoropyridin-3-yl}carbonyl)amino]cyclohexyl}-6-fluoroimidazo[1,2-*a*]pyridine-2-carboxamide (0.162 g, 0.26 mmoles) in N,N-dimethylformamide (3 mL) at room temperature was added 1,1'-carbonyldiimidazole (0.163 g, 1.31 mmoles) and sodium hydride in mineral oil (60% w/w, 0.040 g, 1.31 mmoles). The reaction was then heated to 70 °C for 30 min., before cooling to room temperature and stirring for a further 48 h. The reaction was quenched by addition to brine (50 mL) and extracted with ethyl acetate (3 x 50 mL). Organic layers were then washed twice more with brine (50 mL) dried with anhydrous sodium sulphate, filtered and concentrated *in vacuo* to give the crude as a red oil. This material was purified by reverse phase HPLC to yield the title compound as a brown solid (31 mg, 18%).
¹H NMR (400 MHz, DMSO-*d6*) 8.85 - 8.82 (1H, m), 8.53 - 8.50 (1H, m), 8.43 (1H, s), 8.33 - 8.29 (1H, m), 8.17 (1H, d), 7.83 - 7.71 (4H, m), 7.53 - 7.46 (1H, m), 7.37 - 7.25 (6H, m), 5.71 (2H, s), 4.92 - 4.82 (1H, m), 4.19 - 4.12 (1H, m), 2.65 - 2.54 (2H, m), 2.05 - 1.97 (2H, m), 1.77 - 1.60 (4H, m).
APCI (Multimode) m/z: 647 [M+H]

### Example 40

### 6-fluoro-N-{cis-4-[6-fluoro-1-(1-methylpiperidin-4-yl)-2,4-dioxo-1,4-dihydropyrido[2,3-d]pyrimidin-3(2H)-yl]cyclohexyl}imidazo[1,2-a]pyridine-2-carboxamide.

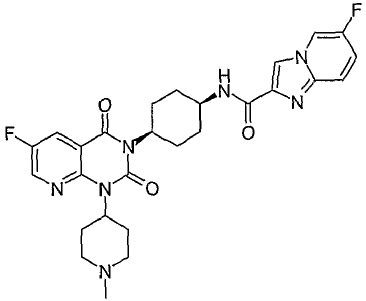

### Step (a) 5-fluoro-2-[(1-methylpiperidin-4-yl)amino]nicotinic acid

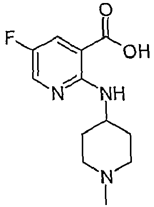

4-amino-N-methylpiperidine (0.2602 g, 2.28 mmoles), potassium carbonate (0.378 g, 2.74 mmoles), copper (0.015 g, 0.23 mmoles) and copper(I) bromide (0.033 g, 0.23 mmoles) were added to a stirred solution of 2-chloro-5-fluoronicotinic acid (0.4 g, 4.56 mmoles) in 1-methyl-2-pyrrolidinone (5 mL). The solution was heated to 120 °C for a period of 3 h then allowed to cool and stir for 48 h, before being applied directly to an SCX column. The column was washed with 200 mL methanol then eluted with 100 mL of 3.5N ammonia/methanol to give the crude product as a yellow oil. This material was dissolved in methanol (25 mL), loaded onto PE-AX resin (4 mL), washed with methanol and then eluted using 10% acetic acid in methanol (50 mL). The acidic layer was concentrated to give the sub-title compound as a colourless solid (100 mg, 17%).
APCI (Multimode) m/z: 254 [M+H].

### Step (b) tert-butyl {cis-4-[({5-fluoro-2-[(1-methylpiperidin-4-yl)amino]pyridin-3-yl}carbonyl)amino]cyclohexyl} carbamate

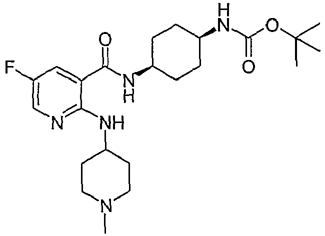

To a suspension of 5-fluoro-2-[(1-methylpiperidin-4-yl)amino]nicotinic acid (0.1 g, 0.40 mmoles), triethylamine (0.120 g, 0.165 mL, 1.19 mmoles) and (4-Amino-cyclohexyl)-carbamic acid tert-butyl ester (0.102 g, 0.47 mmoles) in acetonitrile (0.5 mL) was added 2,4,6-tripropyl-1,3,5,2,4,6-trioxatriphosorinan-2,4,6-trioxide (0.151 g, 0.3019 mL, 50% solution in butyl acetate, 0.47 mmoles) dropwise. The reaction was stirred for 60 min. at room then a further three equivalents of triethylamine added and the reaction stirred at room temperature overnight. The reaction was quenched by addition to water (50 mL) and extracted with ethyl acetate (3 x 50 mL) which was then dried and concentrated *in vacuo.* The residue was purified by flash column chromatography eluting with 5% methanol in DCM to give the title compound as a yellow oil (53 mg, 30%).
APCI (Multimode) m/z: 450 [M+H].

### Step (c) tert-butyl {cis-4-[6-fluoro-1-(1-methylpiperidin-4-yl)-2,4-dioxo-1,4-dihydropyrido[2,3-d]pyrimidin-3(2H)-yl]cyclohexyl}carbamate

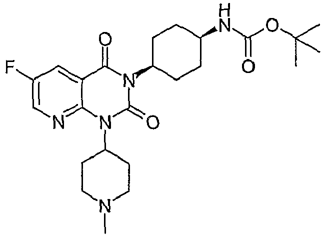

To a solution of *tert*-butyl {*cis*-4-[({5-fluoro-2-[(1-methylpiperidin-4-yl)amino]pyridin-3-yl}carbonyl)amino]cyclohexyl)carbamate (0.058 g, 0.13 mmoles) in N,N-dimethylformamide (1 mL) at room temperature was added 1,1'-carbonyldiimidazole (0.105 g, 0.65 mmoles) and sodium hydride in mineral oil (60% w/w, 0.026 g, 0.65 mmoles). The reaction was then heated to 70 °C for 30 min., before cooling to room temperature and stirring for a further 48 h. The reaction was quenched by addition to brine (50 mL) and extracted with ethyl acetate (3 x 50 mL). Organic layers were then washed twice more with brine (50 mL) dried with anhydrous sodium sulphate, filtered and concentrated *in vacuo* to give the crude as a yellow oil. The residue was purified by flash column chromatography eluting with 5% methanol in DCM to give the sub-title compound as a yellow oil (45 mg, 77%).
APCI (Multimode) m/z: 476 [M+H].

### Step (c) 6-fluoro-N-{cis-4-[6-fluoro-1-(1-methylpiperidin-4-yl)-2,4-dioxo-1,4-dihydropyrido[2,3-d]pyrimidin-3(2H)-yl]cyclohexyl}imidazo[1,2-a]pyridine-2-carboxamide

*tert*-butyl {*cis*-4-[6-fluoro-1-(1-methylpiperidin-4-yl)-2,4-dioxo-1,4-dihydropyrido[2,3-d]pyrimidin-3(2*H*)-yl]cyclohexyl)carbamate (0.045 g, 0.09 mmoles) was suspended in 4M HCl/Dioxane (0.5 mL, 2 mmoles) for a period of 30 min. then the solution was concentrated *in vacuo,* dissolved in N,N-dimethylformamide (0.5 mL) and added dropwise to a pre-stirred (over 10 min.) mixture of 6-Fluoro-imidazo[1,2-a]pyridine-2-carboxylic acid (0.017 g, 0.095 mmoles), N,N-diisopropylethylamine (0.034 g, 0.38 mmoles) and O-(7-Azabezotriazol-1-yl)-N,N,N',N'-tetramethyluronium hexafluorophosphate (0.036 g, 0.095 mmoles) in dry N,N-dimethylformamide (1 mL). The solution was allowed to stir overnight then worked up between ethyl acetate and water (3 x 50 mL, 50 mL). The organic layers were dried (magnesium sulphate) and concentrated *in vacuo* and the residue was purified by reverse phase HPLC to furnish the title compound as a colourless solid (6 mg, 12%).
¹H NMR (400 MHz, DMSO-*d6*) 8.86 - 8.74 (2H, m), 8.39 (1H, s), 8.25 (1H, m), 8.28 - 8.21 (1H, m), 7.88 - 7.80 (1H, m), 7.75 (1H, d), 7.53 - 7.45 (1H, m), 5.24 - 5.13 (1H, m), 4.90 - 4.78 (1H, m), 2.92 - 2.70 (4H, m), 2.69 - 2.53 (2H, m), 2.03 - 1.93 (4H, m), 1.71 (2H, t), 1.57 (4H, d), 2.20 (3H, s).
APCI (Multimode) m/z: 538 [M+H].

### Example 41

### N-{cis-4-[1-[3-(dimethylamino)propyl]-6-fluoro-2,4-dioxo-1,4-dihydropyrido[2,3-d]pyrimidin-3(2H)-yl]cyclohexyl}-6-fluoroimidazo[1,2-a]pyridine-2-carboxamide.

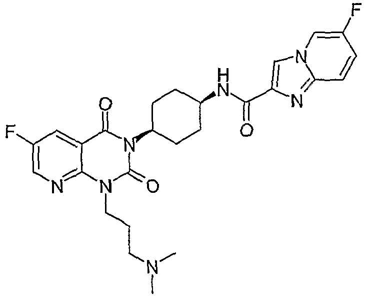

### Step (a) 2-{[3-(dimethylamino)propyl]amino}-5-fluoronicotinic acid

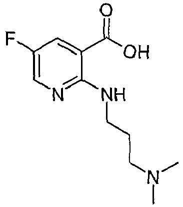

3-dimethylaminopropylamine (0.233 g, 2.28 mmoles), potassium carbonate (0.378 g, 2.74 mmoles), copper (0.015 g, 0.23 mmoles) and copper(I) bromide (0.033 g, 0.23 mmoles) were added to a stirred solution of 2-chloro-5-fluoronicotinic acid (0.4 g, 4.56 mmoles) in N,N-dimethylformamide (5 mL). The solution was heated to 120 °C for a period of 2 h, then cooled and partitioned between ethyl acetate (50 mL) and water (50 mL). The organic layer was dried, concentrated, dissolved in methanol (50 mL) and applied to a 20 g SCX column washing with further methanol (100 mL) and eluting with 3.5 N ammonia in methanol to give the sub-title compound as a brown oil after concentration (423 mg, 77%). APCI (Multimode) m/z: 240 [M+H].

### Step (b) tert-butyl (cis-4-{[(2-{[3-(dimethylamino)propyl]amino}-5-fluoropyridin-3-yl)carbonyl]amino}cyclohexyl)carbamate

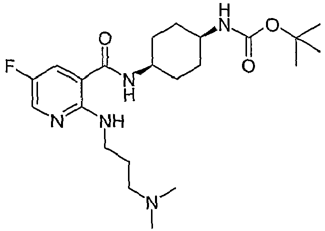

To a suspension of 2-{[3-(dimethylamino)propyl]amino}-5-fluoronicotinic acid (0.423 g, 1.75 mmoles), triethylamine (1.47 mL, 10.52 mmol) and (4-Amino-cyclohexyl)-carbamic acid tert-butyl ester (0.451 g, 2.51 mmoles) in acetonitrile (2 mL) was added 2,4,6-tripropyl-1,3,5,2,4,6-trioxatriphosorinan-2,4,6-trioxide (0.669 g, 1.34 mL, 50% wt solution in butyl acetate, 2.51 mmoles) dropwise. The reaction was stirred for 60 min. at room temperature then a further equivalent of 2,4,6-tripropyl-1,3,5,2,4,6-trioxatriphosorinan-2,4,6-trioxide (0.669 g, 1.34 mL, 50% wt solution in butyl acetate, 2.51 mmoles) was added and the mixture stirred overnight. The reaction was then partitioned between ethyl acetate (3 x 50 mL) and water (50 mL) to give the crude product as a red oil. The residue was purified by flash column chromatography eluting with 5% - 10% methanol in DCM to give the sub-title compound as an orange oil (320 mg, 41%).
APCI (Multimode) m/z: 438 [M+H].

### Step (c) tert-butyl {cis-4-[1-[3-(dimethylamino)propyl]-6-fluoro-2,4-dioxo-1,4-dihydropyrido[2,3-d]pyrimidin-3(2H)-yl]cyclohexyl}carbamate

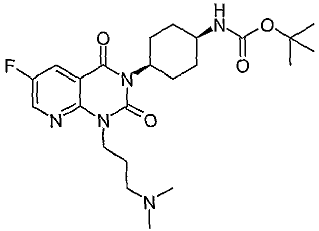

To a solution of *tert*-butyl (*cis*-4-{[(2-{[3-(dimethylamino)propyl]amino}-5-fluoropyridin-3-yl)carbonyl]amino}cyclohexyl)carbamate (0.229 g, 0.523 mmoles) in N,N-dimethylformamide (3 mL) at room temperature was added 1,1'-carbonyldiimidazole (0.401 g, 2.62 mmoles) and sodium hydride in mineral oil (60% w/w, 0.099 g, 2.62 mmoles). The reaction was then heated to 70 °C for 30 min., before cooling to room temperature and stirring for a further 48 h. The reaction was quenched by addition to brine (50 mL) and extracted with ethyl acetate (3 x 50 mL). Organic layers were then washed twice more with brine (50 mL) dried with anhydrous sodium sulphate, filtered and concentrated *in vacuo* to give the crude as a yellow oil. The residue was purified by flash column chromatography eluting with 5% methanol/DCM to give the sub-title compound as a yellow oil (156 mg, 64%).
APCI (Multimode) m/z: 464 [M+H].

### Step (d) N-{cis-4-[1-[3-(dimethylamino)propyl]-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

{4-[1-(3-Dimethylamino-propyl)-6-fluoro-2,4-dioxo-1,4-dihydro-2H-pyrido[2,3-d]pyrimidin-3-yl]-cyclohexyl}-carbamic acid tert-butyl ester (0.082 g, 0.177 mmoles) was suspended in 4M HCl/Dioxane (1 mL, 2 mmoles) for a period of 30 min. then concentrated *in vacuo,* dissolved in N,N-dimethylformamide (1 mL) and added dropwise to a pre-stirred (over 10 min.) mixture of 6-Fluoro-imidazo[1,2-a]pyridine-2-carboxylic acid (0.032 g, 0.177 mmoles), N,N-diisopropylethylamine (0.069 g, 0.71 mmoles) and 0-(7-Azabezotriazol-1-yl)-N,N,N',N'-tetramethyluronium hexafluorophosphate (0.067 g, 0.71 mmoles) in dry N,N-dimethylformamide (1 mL). The resultant solution was allowed to stir overnight then worked up between ethyl acetate (3 x 50 mL ) and water (50 mL). The residue was purified by reverse phase HPLC giving the title compound as a colourless solid (20 mg, 22%).
¹H NMR (400 MHz, DMSO-*d6*) 8.83 - 8.78 (2H, m), 8.39 (1H, s), 8.28 - 8.22 (1H, m), 7.82 - 7.70 (2H, m), 7.53 - 7.46 (1H, m), 4.85 (1H, t), 4.28 - 4.13 (3H, m), 2.69 - 2.52 (2H, m), 2.30 (2H, t), 2.12 (6H, s), 2.03 - 1.96 (2H, m), 1.82 - 1.66 (4H, m), 1.62 - 1.53 (2H, m). APCI (Multimode) m/z: 526 [M+H].

### Example 42

### N-{cis-4-[1-{3-[(1-azabicyclo[2.2.2]oct-3-ylamino)carbonyl]phenyl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide.

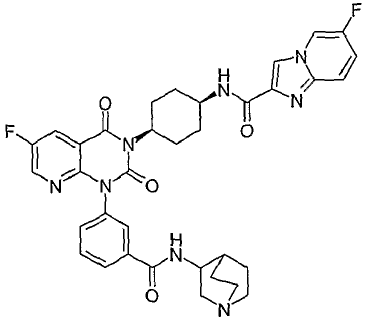

### Step (a) 3-amino-N-1-azabicyclo[2.2.2]oct-3-ylbenzamide acetate salt

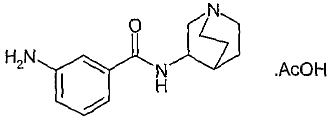

(±)-3-Aminoquinculidine dihydrochloride (10 g, 0.067 moles) was dissolved in water (20 mL) and cooled to 0°C and sodium hydroxide (8 g, 0.22 mmoles) was added slowly. A solution of 3-nitrobenzoyl chloride (11 g, 0.07 moles) in dry acetonitrile (20 mL) was then added in 1 mL aliquots over a period over 10 min. The mixture was left to stir for 20 min. and then diluted with water (50 mL) and extracted into chloroform (3 x 25 mL). The organic layer was then washed with sodium carbonate solution (pH 10.5) and concentrated to give the crude material, which was then triturated in ether (100 mL) to give *N*-1-azabicyclo[2.2.2]oct-3-yl-3-nitrobenzamide as a colourless crystalline solid (11.2 g, 74%). This was used in the next step without further purification.
3-amino-*N*-1-azabicyclo[2.2.2]oct-3-ylbenzamide acetate salt (11 g, 0.45 moles) was dissolved in ethanol (150 mL) and hydrogenated at room temperature and 5 bar hydrogen pressure. After 8 h glacial acetic acid was added to the suspension that had formed and the mixture re-hydrogenated at 3 bar hydrogen pressure for 18 h. The mixture was filtered and the filtrate concentrated in vacuo to give an oil as crude product. This was then triturated with 2:1 isohexane/ether, then with ether and then with isohexane alone for 18 h. The sub-title compound was isolated as a colourless solid (11.3 g, 99%).

¹H NMR (250 MHz, DMSO-*d6*) 8.09 (1H, d), 7.10 - 6.94 (3H, m), 6.67 (1H, ddd), 5.39 - 5.03 (2H, m), 3.93 (1H. d), 3.93 (1H, d), 3.15 - 3.04 (1H, m), 2.96 - 2.84 (1H, m), 1.95 - 1.73 (5H, m), 1.72 - 1.50 (3H, m), 1.43 - 1.21 (1H, m).

### Step (b) 2-({3-[(1-azabicyclo[2.2.2]oct-3-ylamino)carbonyl]phenyl}amino)-5-fluoronicotinic acid

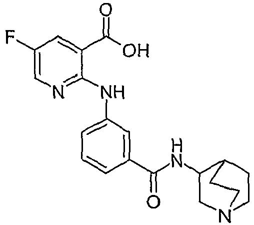

This compound was prepared from 2-chloro-5-fluoronicotinic acid (0.4 g) and 3-amino-*N-*1-azabicyclo[2.2.2]oct-3-ylbenzamide in a similar manner to that described in Example 41 step (a) to give the sub-title compound as an off-white solid (0.15 g, 17%).
APCI (Multimode) m/z: 385 [M+H].

### Step (c) tert-butyl [cis-4-({[2-({3-[(1-azabicyclo[2.2.2]oct-3-ylamino)carbonyl]phenyl}amino)-5-fluoropyridin-3-yl]carbonyl}amino)cyclohexyl]carbamate

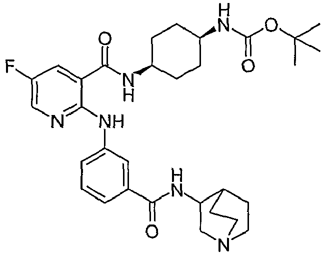

This compound was prepared from 2-({3-[(1-azabicyclo[2.2.2]oct-3-ylamino)carbonyl]phenyl}amino)-5-fluoronicotinic acid
(0.15 g) in a similar manner to that described in Example 41 step (b) to give the sub-title compound as a yellow oil (0.167 g, 73%).
APCI (Multimode) m/z: 581 [M+H].

### Step (d) tert-butyl {cis-4-[1-{3-[(1-azabicyclo[2.2.2]oct-3-ylamino)carbonyl]phenyl}-6-fluoro-2,4-dioxo-1,4-dihydropyrido[2,3-d]pyrimidin-3(2H)-yl]cyclohexyl}carbamate

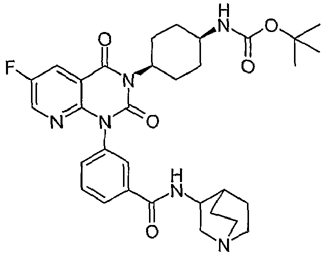

This compound was prepared from *tert*-butyl [*cis*-4-({[2-({3-[(1-azabicyclo[2.2.2]oct-3-ylamino)carbonyl]phenyl}amino)-5-fluoropyridin-3-yl]carbonyl}amino)cyclohexyl]carbamate (0.167 g) in a similar manner to that described in Example 41 step (c) to give the sub-title compound as a yellow oil (0.134 g, 78%).
APCI (Multimode) m/z: 607 [M+H].

### Step (e) N-{cis-4-[1-{3-[(1-azabicyclo[2.2.2]oct-3-ylamino)carbonyl]phenyl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

*tert*-butyl {*cis*-4-[1-{3-[(1-azabicyclo[2.2.2]oct-3-ylamino)carbonyl]phenyl}-6-fluoro-2,4-dioxo-1,4-dihydropyrido[2,3-d]pyrimidin-3(2*H*)-yl]cyclohexyl}carbamate (0.086 g, 0.14 mmoles) was suspended in 4M HCl/Dioxane (1 mL, 2 mmoles) for a period of 30 min. then concentrated *in vacuo,* dissolved in N,N-dimethylformamide (1 mL) and added dropwise to a pre-stirred (over 10 min.) mixture of 6-Fluoro-imidazo[1,2-a]pyridine-2-carboxylic acid (0.026 g, 0.14 mmoles), N,N-diisopropylethylamine (0.098 mL, 0.57 mmoles) and O-(7-Azabezotriazol-1-yl)-N,N,N',N'-tetramethyluronium hexafluorophosphate (0.054 g, 0.14 mmoles) in dry N,N-dimethylformamide (1 mL). The resultant solution was allowed to stir overnight then worked up between ethyl acetate (3 x 50 mL) and water (50 mL). The residue was purified by reverse phase HPLC giving the title compound as a colourless solid (10 mg, 11%).
¹H NMR (400 MHz, DMSO-*d6*) 8.84 - 8.76 (3H, m), 8.39 (1H, s), 8.25 (1H, dd), 7.83 - 7.70 (3H, m), 7.55 - 7.45 (1H, m), 4.85 (1H, t), 4.29 - 4.13 (4H, m), 2.69 - 2.52 (2H, m), 2.35 - 2.27 (4H, m), 2.03 - 1.96 (4H, m), 1.82 - 1.65 (7H, m), 1.62 - 1.53 (3H, m).
APCI (Multimode) m/z: 526 [M+H].

### Example 43

### 6-fluoro-N-{cis-4-[6-fluoro-1-[3-(2-morpholin-4-ylethoxy)phenyl]-2,4-dioxo-1,4-dihydropyrido[2,3-d]pyrimidin-3(2H)-yl]cyclohexyl}imidazo[1,2-a]pyridine-2-carboxamide.

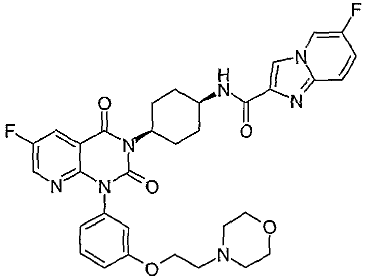

### Step (a) 5-fluoro-2-{[3-(2-morpholin-4-ylethoxy)phenyl]amino}nicotinic acid

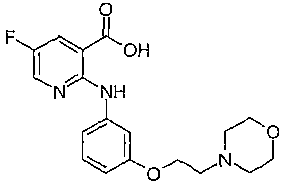

This compound was prepared from 2-chloro-5-fluoronicotinic acid (0.4 g) and 3-(2-morpholin-4-ylethoxy)aniline (Available from Maybridge) in a similar manner to that described in Example 41 step (a) to give the sub-title compound as a pale yellow solid (0.131 g, 13%).
APCI (Multimode) m/z: 362 [M+H].

### Step (b) tert-butyl (cis-4-{[(5-fluoro-2-{[3-(2-morpholin-4-ylethoxy)phenyl]amino}pyridin-3-yl)carbonyl]amino}cyclohexyl)carbamate

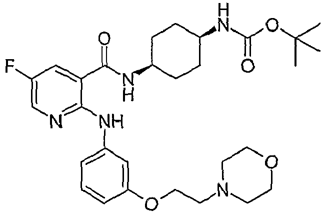

This compound was prepared from 5-fluoro-2-{[3-(2-morpholin-4-ylethoxy)phenyl]amino}nicotinic acid (0.131 g) in a similar manner to that described in Example 41 step (b) giving the sub-title compound as a yellow oil (0.098 mg, 48%). APCI (Multimode) m/z: 458 [-Boc, M+H].

### Step (c) N-(cis-4-aminocyclohexyl)-5-fluoro-2-{[3-(2-morpholin-4-ylethoxy)phenyl]amino}nicotinamide

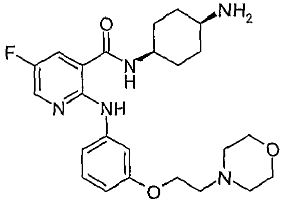

To a solution of *tert*-butyl (*cis*-4-{[(5-fluoro-2-{[3-(2-morpholin-4-ylethoxy)phenyl]amino}pyridin-3-yl)carbonyl]amino}cyclohexyl)carbamate (0.098 g, 0.176 mmoles) in N,N-dimethylformamide (1 mL) at room temperature was added 1,1'-carbonyldiimidazole (0.142 g, 0.88 mmoles) and sodium hydride in mineral oil (60% w/w, 0.035 g, 0.88 mmoles). The reaction was then heated to 70 °C for 30 min., before cooling to room temperature and stirring for a further 48 h. LCMS analysis indicated the major peak was consistent with Boc deprotected starting material. The reaction was quenched by addition to brine (50 mL) and extracted with ethyl acetate (3 x 50 mL). Organic layers were then washed twice more with brine (50 mL) dried with anhydrous sodium sulphate, filtered and concentrated *in vacuo* to give the sub-title compound (0.07 g, 94%) as a yellow oil.
APCI (Multimode) m/z: 458 [M+H].

### Step (d) 6-fluoro-N-(cis-4-{[(5-fluoro-2-{[3-(2-morpholin-4-ylethoxy)phenyl]amino}pyridin-3-yl)carbonyl]amino}cyclohexyl)imidazo[1,2-a]pyridine-2-carboxamide

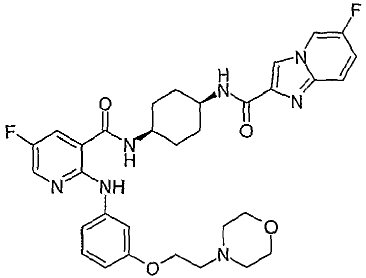

To a mixture of 6-Fluoro-imidazo[1,2-a]pyridine-2-carboxylic acid (0.028 g, 0.15 mmoles) and N,N-diisopropylethylamine (0.079 g, 0.107 mL, 0.61 mmoles) in dry N,N-dimethylformamide (1 mL) was added O-(7-Azabezotriazol-1-yl)-N,N,N',N'-tetramethyluronium hexafluorophosphate (0.058 g, 0.15 mmoles). The mixture was allowed to stir for 10 mins at room temperature. To this mixture was added *N*-(*cis*-4-aminocyclohexyl)-5-fluoro-2- {[3 -(2-morpholin-4-ylethoxy)phenyl]amino } nicotinamide (0.07 g, 0.15 mmoles) dissolved in dry N,N-dimethylformamide (1 mL) and the mixture stirred overnight. The reaction was quenched by addition to water (50 mL) then extracted using ethyl acetate (3 x 50 mL). The organic layers were dried and concentrated to give the sub-title compound as a yellow oil (0.009 g, 47%).
APCI (Multimode) m/z: 584 [M+H].

### Step (e) 6-fluoro-N-{cis-4-[6-fluoro-1-[3-(2-morpholin-4-ylethoxy)phenyl]-2,4-dioxo-1,4-dihydropyrido[2,3-d]pyrimidin-3(2H)-yl]cyclohexyl}imidazo[1,2-a]pyridine-2-carboxamide

To a solution of 6-fluoro-*N*-(*cis*-4-{[(5-fluoro-2-{[3-(2-morpholin-4-ylethoxy)phenyl]amino}pyridin-3-yl)carbonyl]amino}cyclohexyl)imidazo[1,2-*a*]pyridine-2-carboxamide (0.093 g, 0.15 mmoles) in N,N-dimethylformamide (2 mL) at room temperature was added 1,1'-carbonyldiimidazole (0.123 g, 0.75 mmoles) and sodium hydride in mineral oil (60% w/w, 0.03 g, 0.75 mmoles). The reaction was heated to 70 °C for 30 min., before cooling to room temperature and stirring for a further 48 h. The reaction was quenched by addition to brine (50 mL) and extracted with ethyl acetate (3 x 50 mL). Organic layers were then washed twice more with brine (50 mL) dried with anhydrous sodium sulphate, filtered and concentrated *in vacuo* to give the crude as a yellow oil. The residue was purified by reverse phase HPLC to yield the title compound as a white solid (12 mg, 12%).
¹H NMR (400 MHz, CD₃OD) 8.65 - 8.13 (4H, m), 7.71 - 7.23 (4H, m), 7.14 - 6.78 (4H, m), 5.06 - 4.75 (3H, m), 4.27 - 4.08 (4H, m), 2.84 - 2.69 (4H, m), 2.61 - 2.49 (4H, m), 2.17 - 2.06 (2H, m), 1.87 - 1.64 (4H, m).
APCI (Multimode) m/z: 646 [M+H].

### Example 44

### 6-fluoro-N-{cis-4-[6-fluoro-2,4-dioxo-1-[3-(phenylthio)phenyl]-1,4-dihydropyrido[2,3-d]pyrimidin-3(2H)-yl]cyclohexyl}imidazo[1,2-a]pyridine-2-carboxamide.

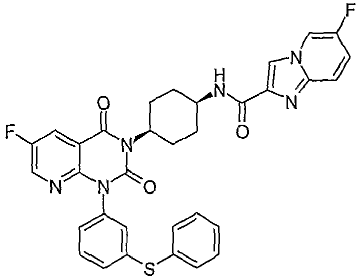

To a stirred solution of 6-fluoro-*N*-{*cis*-4-[6-fluoro-1-(3-iodophenyl)-2,4-dioxo-1,4-dihydropyrido[2,3-*d*]pyrimidin-3(2*H*)-yl]cyclohexyl}imidazo[1,2-*a*]pyridine-2-carboxamide (0.05 g, 0.078 mmoles), copper(I) iodide (0.0015 g, 0.008 mmoles) and potassium carbonate (0.022 g, 0.16 mmoles) under an atmosphere of nitrogen was added ethylene glycol (0.010 g, 0.009 mL, 0.16 mmoles), isopropanol (0.5 mL) and thiophenol (0.009 g, 0.008 mL, 0.078 mmoles). The reaction was heated to 80 °C for 16 h then cooled and worked up between ethyl acetate and brine (50 mL of each). The organic layer was further washed with 1N sodium carbonate solution, then dried with sodium sulphate and concentrated to give the crude product as a yellow oil which was purified by reverse phase HPLC to give the title compound as a colourless solid (0.036 g, 62%).
¹H NMR (400 MHz, DMSO-*d6*) 8.86 - 8.83 (1H, m), 8.60 (1H, d), 8.43 (1H, s), 8.32 - 8.27 (1H, m), 7.80 - 7.74 (2H, m), 7.55 - 7.48 (2H, m), 7.43 - 7.37 (4H, m), 7.36 - 7.29 (3H, m), 4.90 - 4.79 (1H, m), 4.20 - 4.10 (1H, m), 2.66 - 2.52 (2H, m), 2.05 - 1.95 (3H, m), 1.76 - 1.58 (4H, m).
APCI (Multimode) m/z: 625 [M+H].

### Example 45

### tert-butyl {cis-4-[6-fluoro-1-[2'-(morpholin-4-ylmethyl)biphenyl-3-yl]-2,4-dioxo-1,4-dihydropyrido[2,3-d]pyrimidin-3(2H)-yl]cyclohexyl}carbamate.

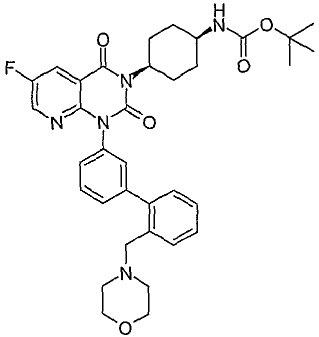

Palladium(II) acetate (0.0058 g, 0.026 mmoles) and 2-(Dicyclohexylphosphino)-2',6'-Dimethoxy-1,1'-biphenyl (0.021 g, 0.052 mmoles) were mixed in acetonitrile (2 mL) for 10 min. before the addition of water (1 mL) and potassium carbonate (0.107 g, 0.78 mmoles). 4-[2-(4,4,5,5-Tetramethyl-[1,3,2]dioxaborolan-2-yl)-benzyl]-morpholine (0.078 g, 0.26 mmoles) was then added to the mixture followed finally by *tert*-butyl {*cis*-4-[6-fluoro-1-(3-iodophenyl)-2,4-dioxo-1,4-dihydropyrido[2,3-d]pyrimidin-3(2*H*)-yl]cyclohexyl}carbamate (0.15 g, 0.26 mmoles) and the reaction heated to 80 °C for 5 h. The reaction was cooled and filtered through a Chemelut cartridge then purified reverse phase HPLC (Xterra column, 60-40 aqueous ammonia in acetonitrile) to give the title compound as a colourless solid (44 mg, 2%).
¹H NMR (400 MHz, DMSO-*d6*) 8.59 (1H, d), 8.29 (1H, m), 7.67 - 7.54 (2H, m), 7.51 - 7.29 (6H, m), 6.47 (1H, s), 4.81 - 4.69 (1H, m), 3.58 - 3.52 (1H, m), 3.45 - 3.37 (4H, m), 3.35 (2H, s), 2.65 - 2.51 (2H, m), 2.34 - 2.20 (4H, m), 1.94 - 1.85 (2H, m), 1.56 - 1.34 (13H, m).
APCI (Multimode) m/z: 630 [M+H].

### Example 46

### 3-(cis-4-aminocyclohexyl)-6-fluoro-1-[2'-(morpholin-4-ylmethyl)biphenyl-3-yl]pyrido[2,3-d]pyrimidine-2,4(1H,3H)-dione.

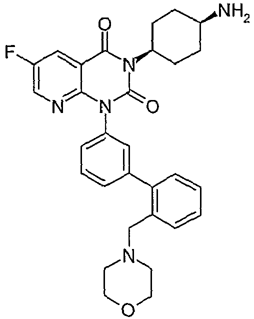

*tert*-butyl {*cis*-4-[6-fluoro-1-[2'-(morpholin-4-ylmethyl)biphenyl-3-yl]-2,4-dioxo-1,4-dihydropyrido[2,3-*d*]pyrimidin-3(2*H*)-yl]cyclohexyl}carbamate (0.65 g, 1.22 mmoles) was dissolved in 0.25mL dioxane then hydrogen chloride, 4.0M in 1,4-dioxane (5 mL, 10 mmoles) was added. The solution was allowed to stir overnight, then the solvent/HCl removed *in vacuo* to furnish the title compound as an orange solid (550 mg, 96%).
¹H NMR (400 MHz, DMSO-*d6*) 8.57 - 8.50 (1H, m), 8.27 - 8.20 (1H, m), 7.78 - 7.30 (12H, m), 4.86 - 4.76 (1H, m), 3.78 - 3.64 (1H, m), 3.56 - 3.46 (4H, m), 2.66 - 2.43 (6H, m), 2.00 - 1.88 (2H, m), 1.86 - 1.73 (2H, m), 1.70 - 1.60 (2H, m).
APCI (Multimode) m/z: 630 [M+H].

### Example 47

### 3-(cis-4-aminocyclohexyl)-6-fluoro-1-(4'-methylbiphenyl-3-yl)pyrido[2,3-d]pyrimidine-2,4(1H,3H)-dione.

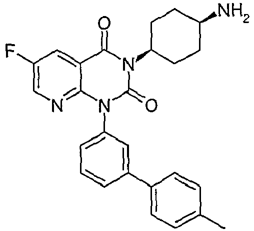

### Step (a) tert-butyl {cis-4-[6-fluoro-2,4-dioxo-1-[3-(4,4,5,5-tetramethyl-1,3,2-dioxaborolan-2-yl)phenyl]-1,4-dihydropyrido[2,3-d]pyrimidin-3(2H)-yl]cyclohexyl} carbamate

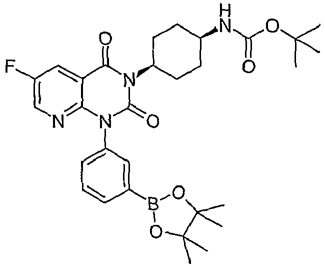

1,1'-bis(diphenylphosphino)ferrocene (0.010 g, 0.017 mmoles) and (1,1'-bis(diphenylphosphino)ferrocene)palladium(II) chloride, (0.013 g, 0.034 mmoles) were dissolved in dimethyl sulphoxide (0.5 mL) and allowed to stir for 10 min. before the addition of potassium acetate (0.102 g, 1.04 mmoles), diboron pinacol ester (0.096 g, 0.38 mmoles) and *tert*-butyl {4-[6-fluoro-1-(3-iodophenyl)-2,4-dioxo-1,4-dihydropyrido[2,3-*d*]pyrimidin-3(2*H*)-yl]cyclohexyl}carbamate (0.2 g, 0.35 mmoles) before flushing with nitrogen. dimethyl sulphoxide (0.5 mL) was then added and the solution warmed to 80 °C overnight then the solution was cooled and partitioned between water (50 mL) and ethyl acetate (50 mL). The organic phase was dried with anhydrous sodium sulphate, filtered and concentrated *in vacuo* to give the crude material as a brown oil, which was purified by reverse phase HPLC (Xterra column, 60 - 40 aqueous ammonia in acetonitrile) to give the sub-title compound as a colourless solid (60 mg, 30%).
APCI (Multimode) m/z: 581 [M+H].

### Step (b) tert-butyl {cis-4-[6-fluoro-1-(4'-methylbiphenyl-3-yl)-2,4-dioxo-1,4-dihydropyrido[2,3-d]pyrimidin-3(2H)-yl]cyclohexyl}carbamate

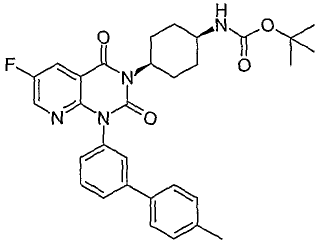

palladium(II) acetate (0.002 g, 0.01 mmoles) and 2-(Dicyclohexylphosphino)-2',6'-Dirnethoxy-1,1'-biphenyl (0.009 g, 0.02 mmoles) were mixed together in acetonitrile (0.5 mL) for 10 min. To the resultant solution was added *tert*-butyl {*cis*-4-[6-fluoro-2,4-dioxo-1-[3-(4,4,5,5-tetramethyl-1,3,2-dioxaborolan-2-yl)phenyl]-1,4-dihydropyrido[2,3-*d*]pyrimidin-3(2*H*)-yl]cyclohexyl}carbamate (0.06 g, 0.1 mmoles) and potassium carbonate (0.043 g, 0.3 mmoles) in water (0.5 mL). 4-Bromotoluene (0.021 g, 0.12 mmoles) was then added and the reaction heated to 80 °C for 1 hr before cooling and partitioning between ethyl acetate (50 mL) and water (50 mL). The organic layer was dried (sodium sulphate) and concentrated and the residue purified by reverse phase HPLC (Xbridge column, 40:30 aqueous ammonia in acetonitrile) to give the sub-title compound as a colourless solid (18 mg, 34%).
APCI (Multimode) m/z: 545 [M+H].

### Step (c) 3-(cis-4-aminocyclohexyl)-6-fluoro-1-(4'-methylbiphenyl-3-yl)pyrido[2,3-d]pyrimidine-2,4(1H,3H)-dione

*tert*-butyl {*cis*-4-[6-fluoro-1-(4'-methylbiphenyl-3-yl)-2,4-dioxo-1,4-dihydropyrido[2,3-d]pyrimidin-3(2*H*)-yl]cyclohexyl}carbamate (0.015 g, 0.028 mmoles) was suspended in hydrogen chloride, 4.0m in 1,4-dioxane (0.5 mL, 2 mmoles) and allowed to stand at room temperature for 30 min. The solvent was then removed *in vacuo* to give the title compound as a colourless solid (7 mg, 81 %).
¹H NMR (400 MHz, CD₃OD) 8.39 (1H, d), 8.27 - 8.23 (1H, m), 7.74 - 7.69 (1H, m), 7.61 - 7.48 (4H, m), 7.28 - 7.21 (4H, m), 5.05 - 4.94 (1H, m), 3.57 - 3.52 (1H, m), 2.67 - 2.54 (2H, m), 2.37 (3H, s), 2.09 - 1.85 (4H, m), 1.82 - 1.74 (2H, m).
APCI (Multimode) m/z: 630 [M+H].

### Example 48

### N-{cis-4-[6-fluoro-1-[2'-(morpholin-4-ylmethyl)biphenyl-3-yl]-2,4-dioxo-1,4-dihydropyrido[2,3-d]pyrimidin-3(2H)-yl]cyclohexyl}-1-hydroxycyclopropanecarboxamide.

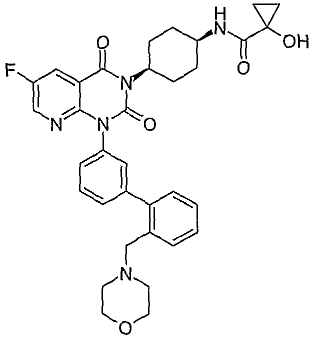

O-(7-Azabezotriazol-1-yl)-N,N,N',N'-tetramethyluronium hexafluorophosphate (0.079 g, 0.21 mmoles) was added to a solution of 1-hydroxy-1-cyclopropanecarboxylic acid (0.021 g, 0.21 mmoles) and N,N-diisopropylethylamine (0.073 g, 0.099 mL, 0.57 mmoles) in N,N-dimethylformamide (1 mL) and allowed to stir for 10 min. before the addition of 3-(*cis*-4-aminocyclohexyl)-6-fluoro-1-[2'-(morpholin-4-ylmethyl)biphenyl-3-yl]pyrido[2,3-*d*]pyrimidine-2,4(1*H*,3*H*)-dione (0.1 g, 0.19 mmoles). After 1 hr stirring a further portion of 1-hydroxy-1-cyclopropanecarboxylic acid (10 mg, 0.1 mmoles) activated with O-(7-Azabezotriazol-1-yl)-N,N,N',N'-tetramethyluronium hexafluorophosphate (40 mg, 0.1 mmoles) and diisopropylethylamine (50 µL, 0.1 mmoles) in DMF (0.2 mL) was added to the reaction and allowed to stir for a further hour. The reaction was worked up between ethyl acetate (50 mL) and water (50 mL) and the aqueous layer further extracted with ethyl acetate (50 mL). The organic phase was dried with anhydrous sodium sulphate, filtered and concentrated *in vacuo* to give the crude as a yellow foam. The residue was purified by reverse phase HPLC (ammonia/Xterra 70-50 aqueous) to give the title compound as a colourless solid (34 mg, 25%).
¹H NMR (400 MHz, DMSO-*d6*) 8.65 - 8.55 (m, 1H), 8.35 - 8.29 (m, 1H), 7.67 - 7.55 (m, 2H), 7.52 - 7.30 (m, 6H), 6.50 - 6.35 (m, 1H), 4.89 - 4.79 (m, 1H), 4.05 - 3.96 (m, 1H), 3.46 - 3.38 (m, 4H), 2.62 - 2.46 (m, 2H), 3.35 (s, 2H), 2.34 - 2.20 (m, 4H), 1.84 - 1.74 (m, 2H), 1.69 - 1.55 (m, 4H), 1.06 - 0.99 (m, 2H), 0.88 - 0.80 (m, 2H).
APCI (Multimode) m/z: 614 [M+H].

### Example 49

### 3-(cis-4-aminocyclohexyl)-1-{4'-[(dimethylamino)methyl]biphenyl-3-yl}-6-fluoropyrido[2,3-d]pyrimidine-2,4(1H,3H)-dione.

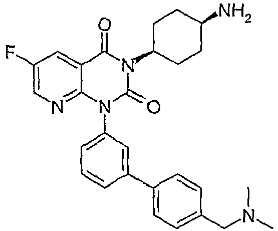

### Step (a) tert-butyl {cis-4-[1-{4'-[(dimethylamino)methyl]biphenyl-3-yl}-6-fluoro-2,4-dioxo-1,4-dihydropyrido[2,3-d]pyrimidin-3(2H)-yl]cyclohexyl}carbamate

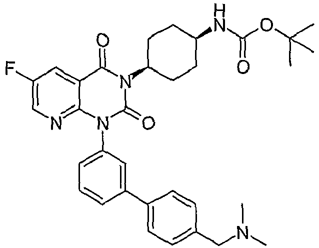

Acetonitrile (7.5 mL) was added to a mixture of palladium(II) acetate (0.017 g, 0.078 mmoles) and 2-(Dicyclohexylphosphino)-2',6'-Dimethoxy-1,1'-biphenyl (0.064 g, 0.16 mmoles) and the mixture was stirred at room temperature for 10 min. potassium carbonate (0.643 g, 4.65 mmoles) dissolved in water (5 mL) was added, followed by dimethyl-[4-(4,4,5,5-tetramethyl-[1,3,2]dioxaborolan-2-yl)-benzyl]-amine (540 mg, 1.85 mmoles) and finally *tert*-butyl {*cis*-4-[6-fluoro-1-(3-iodophenyl)-2,4-dioxo-1,4-dihydropyrido[2,3-*d*]pyrimidin-3(2*H*)-yl]cyclohexyl}carbamate (1.5 g, 3.1 mmoles). The reaction mixture was heated to 80 °C overnight. Then cooled and the solution passed through a Chemelut column eluting with DCM (100 mL) then 10%methanol/DCM. The organic solvents were concentrated to give the sub-title compound as a pale yellow foam (729 mg, 69%).
¹H NMR (400 MHz, DMSO-d6) 8.58 (1H, d), 8.29 (1H, d), 7.79 - 7.69 (2H, m), 7.65 - 7.59 (3H, m), 7.40 - 7.33 (3H, m), 6.55 - 6.44 (1H, m), 4.81 - 4.71 (1H, m), 3.59 - 3.51 (1H, m), 3.43 - 3.41 (2H, m), 3.29 - 3.28 (6H, m), 2.65 - 2.52 (2H, m), 1.94 - 1.87 (2H, m), 1.55 - 1.44 (4H, m), 1.40 - 1.35 (9H, m).
APCI (Multimode) m/z: 588 [M+H].

### Step (b) 3-(cis-4-aminocyclohexyl)-1-{4'-[(dimethylamino)methyl]biphenyl-3-yl}-6-fluoropyrido[2,3-d]pyrimidine-2,4(1H,3H)-dione

*tert*-butyl {*cis*-4-[1-{4'-[(dimethylamino)methyl]biphenyl-3-yl}-6-fluoro-2,4-dioxo-1,4-dihydropyrido[2,3-*d*]pyrimidin-3(2*H*)-yl]cyclohexyl}carbamate (0.534 g, 0.91 mmoles) was dissolved in a small amount of dioxane then hydrogen chloride, 4.0m in 1,4-dioxane (2.1 g, 2 mL, 5.9 mmole) was added. The solution was allowed to stir at room temperature overnight then the solvents removed *in vacuo.* The residue was stirred in ether (25 mL) for 1 hr then collected by filtration and washed with further ether to give the title compound as a colourless solid (377 mg, 74%).
¹H NMR (40 MHz, DMSO-*d6*) 11.10 (1H, s), 8.67 - 8.53 (1H, m), 8.36 - 8.25 (1H, m), 8.19 - 7.98 (3H, m), 7.86 - 7.57 (7H, m), 7.47 - 7.34 (1H, m), 4.87 - 4.72 (1H, m), 4.38 - 4.22 (2H, m), 3.61 - 3.51 (1H, m), 2.74 - 2.64 (6H, m), 2.59 - 2.49 (2H, m), 2.04 - 1.93 (2H, m), 1.82 - 1.57 (4H, m).
APCI (Multimode) m/z: 488 [M+H].

### Example 50

### 6-fluoro-N-{cis-4-[6-fluoro-1-[4'-(2-morpholin-4-ylethyl)biphenyl-3-yl]-2,4-dioxo-1,4-dihydropyrido[2,3-d]pyrimidin-3(2H)-yl]cyclohexyl}imidazo[1,2-a]pyridine-2-carboxamide.

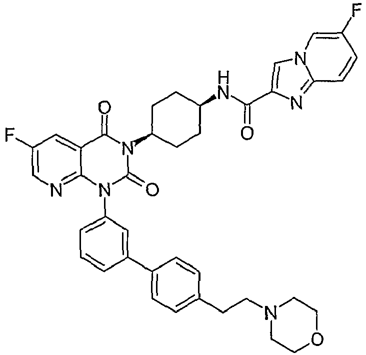

Acetonitrile (1 mL) was added to a mixture of palladium(II) acetate (0.004 g, 0.016 mmoles) and 2-(Dicyclohexylphosphino)-2',6'-Dimethoxy-1,1'-biphenyl (0.013 g, 0.031 mmoles) and the mixture was stirred at room temperature for 10 min. Potassium carbonate (0.065 g, 0.47 mmoles) dissolved in water (0.75 mL) was added, followed by 4-{2-[4-(4,4,5,5-Tetramethyl-[1,3,2]dioxaborolan-2-yl)-phenyl]-ethyl}-morpholine (82 mg, 0.23 mmoles) and *tert*-butyl {*cis*-4-[6-fluoro-1-(3-iodophenyl)-2,4-dioxo-1,4-dihydropyrido[2,3-*d*]pyrimidin-3(2*H*)-yl]cyclohexyl}carbamate (0.1 g, 0.16 mmoles). The reaction mixture was heated to 80 °C overnight, then cooled and passed through a Chemelut column to give the crude product which was purified by reverse phase HPLC to give the title compound as a colourless solid (45 mg, 41%).
¹H NMR (400 MHz, DMSO-*d6*) 8.78 (1H, m), 8.59 (1H, d), 8.39 - 8.30 (2H, m), 7.77 - 7.56 (6H, m), 7.47 - 7.30 (4H, m), 4.93 - 4.82 (1H, m), 4.20 - 4.06 (2H, m), 3.60 - 3.54 (4H, m), 2.81 - 2.73 (2H, m), 2.68 - 2.48 (4H, m), 2.45 - 2.39 (4H, m), 2.04 - 1.96 (2H, m), 1.78 - 1.61 (4H, m).
APCI (Multimode) m/z: 706 [M+H].

### Example 51

### tert-butyl 4-({3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl}methyl)piperazine-1-carboxylate

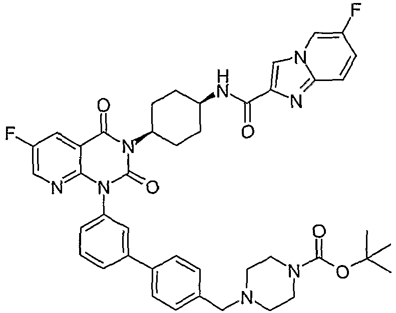

A solution of 6-fluoro-*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.069 g, 0.11 mmoles), tert-butyl 1-piperazinecarboxylate (0.031 g, 0.17 mmoles) and acetic acid (2 drops) in 1,2-dichloroethane (2 mL) was stirred at room temperature for 30 mins. Sodium triacetoxyborohydride (0.035 g, 0.17 mmoles) was added and the reaction mixture was stirred for 1 hr. The solution was then diluted with DCM (50 mL) and washed with water (50 mL). The organic phase was dried with anhydrous sodium sulphate, filtered and concentrated *in vacuo* to give the crude product as a colourless oil, which was purified by reverse phase HPLC to give the title compound as a colourless solid (16 mg, 18%).
¹H NMR (400 MHz, DMSO-*d6*) 8.82 - 8.76 (1H, m), 8.59 (1H, d), 8.38 - 8.30 (2H, m), 7.79 - 7.57 (7H, m), 7.47 - 7.35 (4H, m), 4.92 - 4.83 (1H, m), 4.20 - 4.13 (1H, m), 3.53 - 3.49 (2H, m), 3.33 - 3.28 (4H, m), 2.69 - 2.52 (2H, m), 2.36 - 2.30 (4H, m), 2.06 - 1.95 (2H, m), 1.78 - 1.60 (4H, m), 1.41 - 1.34 (9H, m).
APCI (Multimode) m/z: 791 [M+H].

### Example 52

### 3-(cis-4-aminocyclohexyl)-6-fluoro-1-(2'-methylbiphenyl-3-yl)pyrido[2,3-d]pyrimidine-2,4(1H,3H)-dione

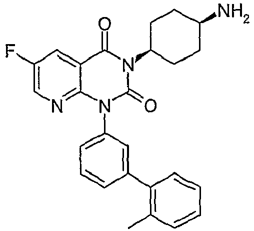

### Step (a) tert-butyl {cis-4-[6-fluoro-1-(2'-methylbiphenyl-3-yl)-2,4-dioxo-1,4-dihydropyrido[2,3-d]pyrimidin-3(2H)-yl]cyclohexyl}carbamate

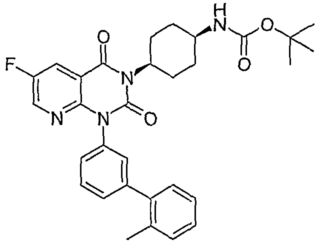

This compound was prepared from 2-bromotoluene (0.035 g) and *tert*-butyl {*cis*-4-[6-fluoro-2,4-dioxo-1-[3-(4,4,5,5-tetramethyl-1,3,2-dioxaborolan-2-yl)phenyl]-1,4-dihydropyrido[2,3-*d*]pyrimidin-3(2*H*)-yl]cyclohexyl}carbamate in a similar manner to that described in Example 47 step (b) to give the sub-title compound as a pale yellow solid (0.022 g, 23%).
APCI (Multimode) m/z: 444 [M+H - Boc].

### Step (b) 3-(cis-4-aminocyclohexyl)-6-fluoro-1-(2'-methylbiphenyl-3-yl)pyrido[2,3-d]pyrimidine-2,4(1H,3H)-dione

This compound was prepared from *tert*-butyl {*cis*-4-[6-fluoro-1-(2'-methylbiphenyl-3-yl)-2,4-dioxo-1,4-dihydropyrido[2,3-*d*]pyrimidin-3(2*H*)-yl]cyclohexyl}carbamate in a similar manner to that described in Example 47 step (c) to give the title compound as a colourless solid (0.011 g, 73%).
¹H NMR (400 MHz, DMSO-*d6*) 8.63 (1H, s), 8.33 - 8.25 (1H, m), 7.76 - 7.50 (4H, m), 7.46 - 7.18 (7H, m), 4.84 - 4.71 (1H, m), 3.42 - 3.35 (1H, m), 2.63 - 2.49 (2H, m), 2.28 - 2.24 (3H, m), 1.95 - 1.86 (2H, m), 1.84 - 1.69 (2H, m), 1.68 - 1.59 (2H, m).
APCI (Multimode) m/z: 445 [M+H].

### Example 53

### N-[cis-4-(1-biphenyl-3-yl-6-fluoro-2,4-dioxo-1,4-dihydropyrido[2,3-d]pyrimidin-3(2H)-yl)cyclohexyl]-1-(dimethylamino)cyclopropanecarboxamide

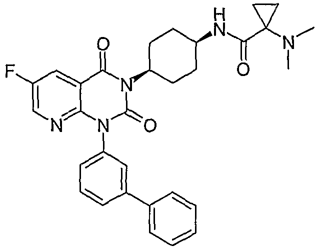

A mixture of 3-(*cis*-4-aminocyclohexyl)-1-biphenyl-3-yl-6-fluoropyrido[2,3-*d*]pyrimidine-2,4(1*H*,3*H*)-dione (0.04 g, 0.078 mmoles), formaldehyde (0.025 g, 0.025 mL, 0.333 mmoles) in 1,2-dichloroethane (2 mL) was allowed to stir for 30 min. before the addition of sodium triacetoxyborohydride (0.036 g, mmoles). The mixture was stirred for 16 h at room temperature. A further portion of formaldehyde (0.025 g, mmoles) was added, allowed to stir 10 min., then more sodium triacetoxyborohydride (0.036 g, 0.17 mmoles) was also added. This suspension was allowed to stir for 32 h then diluted with DCM (50 mL) and washed with water. The organic layer was dried and concentrated *in vacuo* and the residue purified by reverse phase HPLC (Xterra, 60-40 0.2% aqueous ammonia in acetonitrile) to give the sub-title compound as a colourless solid (9 mg, 21 %).
¹H NMR (400 MHz, DMSO-*d6*) 8.61 - 8.58 (1H, m), 8.34 - 8.28 (1H, m), 8.11 - 8.06 (1H, m), 7.80 - 7.58 (4H, m), 7.52 - 7.44 (2H, m), 7.42 - 7.35 (2H, m), 4.89 - 4.78 (1H, m), 3.98 - 3.91 (1H, m), 2.65 - 2.50 (2H, m), 1.82 (2H, d), 1.68 - 1.55 (4H, m), 0.98 - 0.90 (4H, m). APCI (Multimode) m/z: 542 [M+H].

### Example 54

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide

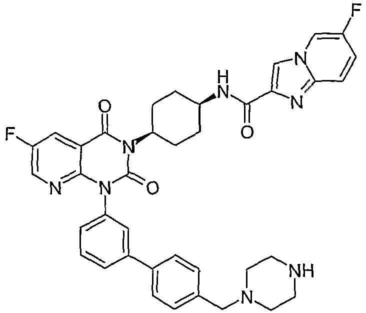

A solution of hydrogen chloride in dioxane (4.0M, 1.334 mL, 5.34 mmol) was added dropwise to a stirred solution of *tert*-butyl 4-({3'-[6-fluoro-3-(*cis*-4-{[(6-fluoroimidazo [1,2-*a*]pyridin-2-yl)carbonyl amino}cyclohexyl)-2,4-dioxo-3,4-dihydropyrido[2,3-*d*]pyrimidin-1(2*H*)-yl]biphenyl-4-yl}methyl)piperazine-1-carboxylate (256 mg, 0.32 mmol) in 1,4-Dioxane (1.432 mL) at 25 °C. The resulting solution was stirred at 25 °C for 2 h. The solvent was evaporated to dryness to give the title compound as a colourless solid (234 mg, 95 %).
¹H NMR (400 MHz, DMSO-*d6*) 8.96 (1H, s), 8.59 (2H, d), 8.55 (2H, s), 8.33 (1H, dd), 7.98 (1H, s), 7.84 - 7.73 (8H, m), 7.66 - 7.59 (2H, m), 7.44 - 7.39 (1H, m), 4.88 (2H, s), 4.76 - 4.48 (11H, m), 4.45 - 4.40 (4H, m), 4.17 - 4.11 (1H, m), 3.51 - 3.42 (8H, m), 2.69 - 2.58 (2H, m), 2.09 - 2.01 (2H, m), 1.77 - 1.61 (6H, m).
APCI (Multimode) m/z: 691 [M+H].

### Example 55

### 6-fluoro-N-{cis-4-[6-fluoro-2,4-dioxo-1-(3-pyridin-2-ylphenyl)-1,4-dihydropyrido[2,3-d]pyrimidin-3(2H)-yl]cyclohexyl}imidazo[1,2-a]pyridine-2-carboxamide

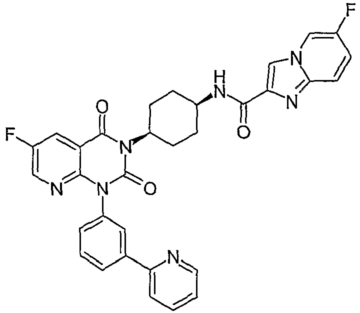

### Step (a) tert-butyl {cis-4-[6-fluoro-2,4-dioxo-1-(3-pyridin-2-ylphenyl)-1,4-dihydropyrido [2,3-d]pyrimidin-3(2H)-yl]cyclohexyl}carbamate

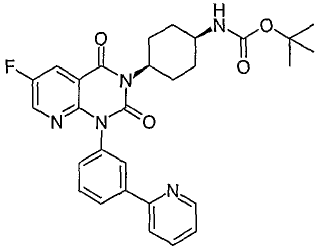

This compound was prepared from 2-bromopyridine (0.037 mL) and *tert*-butyl {*cis*-4-[6-fluoro-2,4-dioxo-1-[3-(4,4,5, 5-tetramethyl-1,3,2-dioxaborolan-2-yl)phenyl]-1,4-dihydropyrido[2,3-d]pyrimidin-3(2*H*)-yl]cyclohexyl}carbamate in a similar manner to that described in Example 47 step (b) to give the sub-title compound as a colourless solid (0.060 g, 44%).
APCI (Multimode) m/z: 532 [M+H].

### Step (b) 3-(cis-4-aminocyclohexyl)-6-fluoro-1-(3-pyridin-2-ylphenyl)pyrido[2,3-d]pyrimidine-2,4(1H,3H)-dione

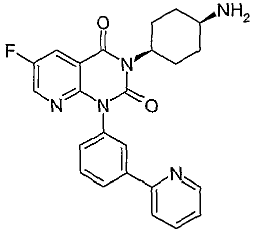

This compound was prepared from *tert*-butyl {*cis*-4-[6-fluoro-2,4-dioxo-l-(3-pyridin-2-ylphenyl)-1,4-dihydropyrido[2,3-*d*]pyrimidin-3(2*H*)-yl]cyclohexyl}carbamate (0.06 g) in a similar manner to that described in Example 47 step (c) to give the sub-title compound as a colourless solid (0.046 g, 94%).
APCI (Multimode) m/z: 432 [M+H].

### Step (c) 6-fluoro-N-{cis-4-[6-fluoro-2,4-dioxo-1-(3-pyridin-2-ylphenyl)-1,4-dihydropyrido [2,3-d]pyrimidin-3(2H)-yl]cyclohexyl}imidazo[1,2-a]pyridine-2-carboxamide

3-(cis-4-aminocyclohexyl)-6-fluoro-1-(3-pyridin-2-ylphenyl)pyrido[2,3-*d*]pyrimidine-2,4(1*H*,3*H*)-dione (46 mg, 0.11 mmol) was added to a stirred solution of O-(7-Azabenzotriazol-1-yl)-N,N,N',N'-tetramethyluronium hexafluorophosphate (45 mg, 0.12 mmol), 6-fluoroimidazo[1,2-a]pyridine-2-carboxylic acid (25 mg, 0.12 mmol) and N-ethyldiisopropylamine (57 µL, 0.32 mmol) in N,N-dimethylformamide (751 µL) at 25 °C. The resulting solution was stirred at 25 °C for 2 h. The reaction mixture was then diluted with ethyl acetate (50 mL), and washed with water (150 mL). The aqueous layer was back extracted with ethyl acetate (1 x 50 mL), and the combined organic layer was dried over sodium sulphate, filtered and evaporated to afford the crude product.
The crude product was purified by preparative LCMS on a Waters X-Terra column using a 60-40% gradient of aqueous 0.2% ammonia in acetonitrile as eluent. The fractions containing the desired compound were evaporated to dryness to afford the title compound (24 mg, 59 %) as a colourless solid.
¹H NMR (400 MHz, DMSO-*d6*) 8.84 - 8.79 (1H, m), 8.68 - 8.64 (1H, m), 8.59 - 8.55 (1H, m), 8.40 (1H, s), 8.35 - 8.30 (1H, m), 8.19 - 8.13 (1H, m), 8.01 - 7.97 (1H, m), 7.94 - 7.89 (1H, m), 7.77 - 7.71 (2H, m), 7.65 (1H, t), 7.50 - 7.42 (2H, m), 7.41 - 7.36 (1H, m), 4.93 - 4.83 (1H, m), 4.20 - 4.13 (1H, m), 2.68 - 2.54 (2H, m), 2.05 - 1.97 (2H, m), 1.77 - 1.62 (4H, m).
APCI (Multimode) m/z: 594 [M+H].

### Example 56

### 6-fluoro-N-{cis-4-[6-fluoro-1-(4'-{[(3R)-3-hydroxypyrrolidin-1-yl]methyl}biphenyl-3-yl)-2,4-dioxo-1,4-dihydropyrido[2,3-d]pyrimidin-3(2H)-yl]cyclohexyl}imidazo[1,2-a]pyridine-2-carboxamide

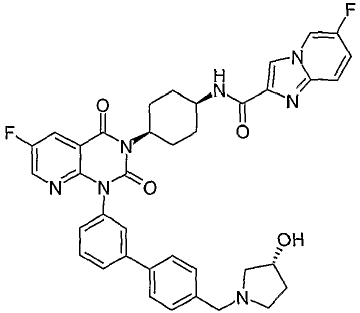

6-fluoro-*N*-{*cis*-4-[6-fluoro-l-(4'-formylbiphenyl-3-yl)-2,4-dioxo-1,4-dihydropyrido[2,3-d]pyrimidin-3(2*H*)-yl]cyclohexyl}imidazo[1,2-*a*]pyridine-2-carboxamide (312 mg, 0.50 mmol) and (R)-(+)-3-hydroxypyrrolidine (0.042 mL, 0.50 mmol) were dissolved in 1,2-dichloroethane (5 mL) and the solution allowed to stir for 10 min. Sodium triacetoxyborohydride (160 mg, 0.75 mmol) was then added over a period of 10 min. under air. The resulting suspension was stirred at 25 °C for 16 h. The reaction mixture was diluted with dichloromethane (50 mL), and washed with water (50 mL). The organic was dried over sodium sulfate, filtered and evaporated to afford crude product. The crude product was purified by preparative LCMS on a Waters X-Terra column using a 60-40% gradient of aqueous 0.2% ammonia in acetonitrile as eluent. The fractions containing the desired compound were evaporated to dryness to afford the title compound (141 mg, 41 %) as a colourless solid.
¹H NMR (400 MHz, DMSO-*d6*) 8.78 (1H, dd), 8.59 (1H, d), 8.38 - 8.30 (2H, m), 7.79 - 7.57 (6H, m), 7.47 - 7.35 (4H, m), 4.92 - 4.83 (1H, m), 4.66 (1H, d), 4.23 - 4.13 (2H, m), 3.58 (2H, d), 3.31 - 3.27 (1H, m), 2.71 - 2.51 (3H, m), 2.45 - 2.38 (1H, m), 2.34 - 2.29 (1H, m), 2.04 - 1.95 (3H, m), 1.78 - 1.61 (4H, m), 1.58 - 1.49 (1H, m).
APCI (Multimode) m/z: 692 [M+H].

### Example 57

### N-{cis-4-[1-(4'-{[tert-butyl(methyl)amino]methyl}biphenyl-3-yl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

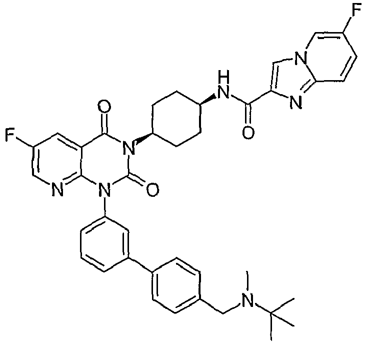

6-fluoro-*N*-{*cis*-4-[6-fluoro-1-(4'-formylbiphenyl-3-yl)-2,4-dioxo-1,4-dihydropyrido[2,3-d]pyrimidin-3(2*H*)-yl]cyclohexyl}imidazo[1,2-*a*]pyridine-2-carboxamide (300 mg, 0.48 mmol) and *N*-methyl-tert-butylamine (0.055 mL, 0.46 mmol) were dissolved in 1,2-dichloroethane (4.6 mL) and the solution allowed to stir for 10 min. Sodium triacetoxyborohydride (145 mg, 0.69 mmol) was then added over a period of 10 min. under air. The resulting suspension was stirred at 25 °C for 16 h. A further equivalent of N-methyl-tert-butylamine (0.055 mL, 0.46 mmol) was added as well as trimethyl orthoformate (0.200 mL, 1.83 mmol). The reaction was allowed to stir for a further hour, then more sodium triacetoxyborohydride (145 mg, 0.69 mmol) was added and the reaction stirred for 16 h. The crude material was dissolved in methanol (5 mL) and loaded on to an 20g SCX cartridge. The impurities were washed through with methanol (100 mL) and discarded. The product was eluted with 3.5N methanolic ammonia (100 mL) and evaporated *in vacuo* to give a yellow residue. The crude product was purified by preparative HPLC on a Waters X-Terra column using a 60-40% gradient of aqueous 0.2% ammonia in acetonitrile as eluent. The fractions containing the desired compound were evaporated to dryness to afford the title compound (47 mg, 15 %) as a colourless solid.
¹H NMR (400 MHz, DMSO-*d6*) 8.79 (1H, t), 8.60 (1H, t), 8.40 - 8.29 (2H, m), 7.82 - 7.57 (6H, m), 7.50 - 7.32 (4H, m), 4.97 - 4.83 (1H, m), 4.22 - 4.11 (1H, m), 3.57 - 3.48 (2H, m), 2.69 - 2.49 (2H, m), 2.07 - 1.95 (5H, m), 1.79 - 1.61 (4H, m), 1.12 (9H, s).
APCI (Multimode) m/z: 692 [M+H].

### Example 58

### 6-fluoro-N-{cis-4-[6-fluoro-1-[4'-(2-hydroxyethoxy)biphenyl-3-yl]-2,4-dioxo-1,4-dihydropyrido [2,3-d]pyrimidin-3(2H)-yl]cyclohexyl}imidazo[1,2-a]pyridine-2-carboxamide

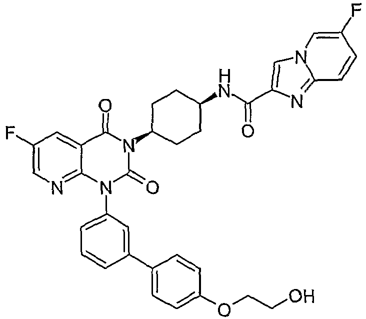

### Step (a) tert-butyl {cis-4-[6-fluoro-1-[4'-(2-hydroxyethoxy)biphenyl-3-yl]-2,4-dioxo-1,4-dihydropyrido[2,3-d]pyrimidin-3(2H)-yl]cyclohexyl}carbamate

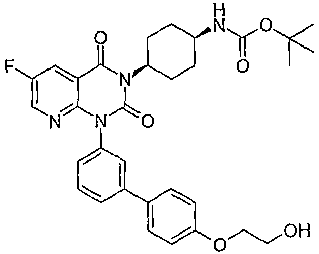

This compound was prepared from 2-(4-bromophenoxy)ethanol (0.112 g) and *tert-butyl* {*cis*-4-[6-fluoro-2,4-dioxo-1-[3-(4,4,5,5-tetramethyl-1,3,2-dioxaborolan-2-yl)phenyl]-1,4-dihydropyrido[2,3-*d*]pyrimidin-3(2*H*)-yl]cyclohexyl}carbamate in a similar manner to that described in Example 47 step (b) to give the sub-title compound as a colourless solid (0.088 g, 43%).
APCI (Multimode) m/z: 491 [M+H -Boc].

### Step (b) 3-(cis-4-aminocyclohexyl)-6-fluoro-1-[4'-(2-hydroxyethoxy)biphenyl-3-yl]pyrido[2,3-d]pyrimidine-2,4(1H,3H)-dione

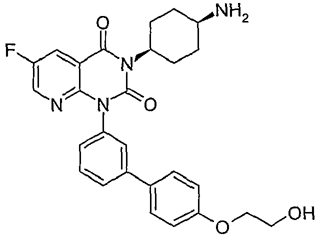

This compound was prepared from *tert*-butyl {*cis*-4-[6-fluoro-1-[4'-(2-hydroxyethoxy)biphenyl-3-yl]-2,4-dioxo-1,4-dihydropyrido[2,3-*d*]pyrimidin-3(2*H*)-yl]cyclohexyl}carbamate (0.088 g) in a similar manner to that described in Example 47 step (c) to give the sub-title compound as a colourless solid (0.064 g, 88%).
APCI (Multimode) m/z: 491 [M+H].

### Step (c) 6-fluoro-N-{cis,-4-[6-fluoro-1-[4'-(2-hydroxyethoxy)biphenyl-3-yl]-2,4-dioxo-1,4-dihydropyrido[2,3-d]pyrimidin-3(2H)-yl]cyclohexyl}imidazo[1,2-a]pyridine-2-carboxamide

3-(*cis*-4-aminocyclohexyl)-6-fluoro-1-[4'-(2-hydroxyethoxy)biphenyl-3-yl]pyrido[2,3-*d*]pyrimidine-2,4(1*H*,3*H*)-dione (46 mg, 0.11 mmol) was added to a stirred solution of O-(7-Azabenzotriazol-1-yl)-N,N,N',N'-tetramethyluronium hexafluorophosphate (45 mg, 0.12 mmol), 6-fluoroimidazo[1,2-a]pyridine-2-carboxylic acid (25 mg, 0.12 mmol) and N-ethyldiisopropylamine (57 µL, 0.32 mmol) in N,N-dimethylformamide (751 µL) at 25 °C. The resulting solution was stirred at 25 °C for 2 h. The reaction mixture was then diluted with ethyl acetate (50 mL), and washed with water (150 mL). The aqueous layer was back extracted with ethyl acetate (1 x 50 mL), and the combined organic layer was dried over sodium sulphate, filtered and evaporated to afford the crude product. The crude product was purified by preparative LCMS on a Waters X-Terra column using a 60-40% gradient of aqueous 0.2% ammonia in acetonitrile as eluent. The fractions containing the desired compound were evaporated to dryness to afford the title compound (24 mg, 59 %) as a colourless solid.
¹H NMR (400 MHz, DMSO-*d6*) 8.79 (1H, dd), 8.59 (1H, d), 8.36 (1H, s), 8.32 (1H, dd), 7.77 - 7.66 (4H, m), 7.63 - 7.54 (3H, m), 7.47 - 7.41 (1H, m), 7.31 (1H, d), 7.03 (2H, d), 4.93 - 4.83 (2H, m), 4.20 - 4.13 (1H, m), 4.03 (2H, t), 3.73 (2H, q), 2.68 - 2.53 (2H, m), 2.04 - 1.96 (2H, m), 1.78 - 1.62 (4H, m).
APCI (Multimode) m/z: 653 [M+H].

### Example 59

### 6-fluoro-N-{cis-4-[6-fluoro-1-[4'-({[(1R)-2-hydroxy-1-methylethyl]amino}methyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

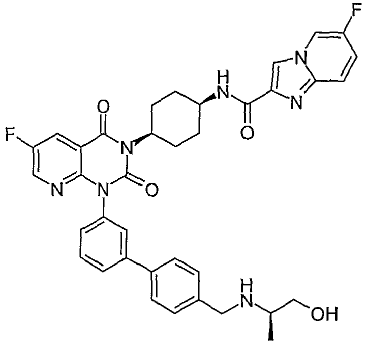

6-fluoro-*N*-{*cis*-4-[6-fluoro-1-(4'-formylbiphenyl-3-yl)-2,4-dioxo-1,4-dihydropyrido[2,3-d]pyrimidin-3(2*H*)-yl]cyclohexyl}imidazo[1,2-*a*]pyridine-2-carboxamide (300 mg, 0.48 mmol) and (R)-(-)-2-amino-1-propanol (0.056 mL, 0.73 mmol) were dissolved in 1,2-Dichloroethane (4.8 mL) and the solution allowed to stir for 10 min. Sodium triacetoxyborohydride (154 mg, 0.73 mmol) was then added over a period of 10 min. under air. The resulting suspension was stirred at 25 °C for 16 h. The reaction mixture was diluted with dichloromethane (50 mL), and washed with water (50 mL). The organic was dried over sodium sulfate, filtered and evaporated to afford crude product. The crude product was purified by preparative LCMS on a Waters X-Terra column using a 95-50% gradient of aqueous 0.2% trifluoroacetic acid in acetonitrile as eluent. The fractions containing the desired compound were evaporated to dryness to afford the title compound (57 mg, 17 %) as a colourless solid.
¹H NMR (400 MHz, DMSO-*d6*) 8.81 (2H, dd), 8.74 - 8.65 (1H, m), 8.59 (1H, d), 8.39 - 8.33 (2H, m), 7.84 - 7.60 (8H, m), 7.48 - 7.41 (2H, m), 4.92 - 4.83 (1H, m), 4.25 - 4.14 (2H, m), 3.70 - 3.64 (1H, m), 3.56 - 3.50 (1H, m), 3.23 - 3.16 (1H, m), 2.68 - 2.56 (2H, m), 2.05 - 1.97 (2H, m), 1.78 - 1.63 (4H, m), 1.24 (3H, d).
APCI (Multimode) m/z: 680 [M+H].

### Example 60

### 3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-2-carboxylic acid

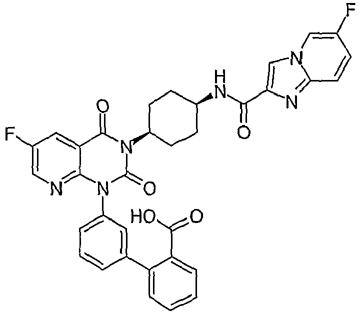

### Step (a) 6-fluoro-N-{cis-4-[6-fluoro-1-(2'-formylbiphenyl-3-yl)-2,4-dioxo-1,4-dihydropyrido[2,3-d]pyrimidin-3(2H)-yl]cyclohexyl}imidazo[1,2-a]pyridine-2-carboxamide

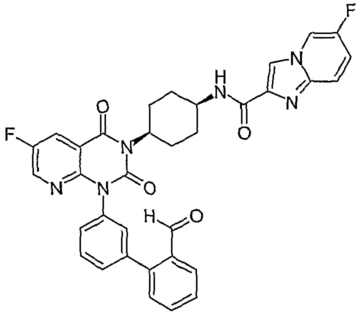

Palladium(II) acetate (0.035 g, 0.16 mmol) and 2-dicyclohexylphosphino-2',6'-dimethoxybiphenyl (0.128 g, 0.31 mmol) were stirred together in acetonitrile (30 mL) for 10 min. before the addition of a solution of potassium carbonate (1.291 g, 9.34 mmol) in water (15 mL). 2-Formylphenylboronic acid (0.467 g, 3.11 mmol) and 6-fluoro-*N*-{*cis-*4-[6-fluoro-1-(3-iodophenyl)-2,4-dioxo-1,4-dihydropyrido[2,3-d]pyrimidin-3(2*H*)-yl]cyclohexyl}imidazo[1,2-*a*]pyridine-2-carboxamide (2 g, 3.11 mmol) were then added sequentially. The resulting suspension was stirred at 80 °C for 16 h, then isolated by filtration and washed with 100 mL acetonitrile to afford the title compound (1.36 g, 70 %) as a tan solid.
APCI (Multimode) m/z: 621 [M+H].

### Step (b) 3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-2-carboxylic acid

Sodium chlorite (70.0 mg, 0.77 mmol) dissolved in water (0.5 mL) was added to a stirred solution of 6-fluoro-*N*-{*cis*-4-[6-fluoro-1-(2'-formylbiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (200 mg, 0.32 mmol) and sodium phosphate monobasic (97 mg, 0.81 mmol) in a mixture of DMSO (2 mL), and water (0.2 mL) over a period of 10 min. under air. The resulting suspension was stirred at 25 °C for 3 days. The reaction mixture was diluted with ethyl acetate (50 mL), and washed with water (50 mL) and saturated brine (50 mL). The organic was dried over magnesium sulfate, filtered and evaporated to afford crude product. The crude product was purified by preparative HPLC on a Waters X-Bridge column using a 75-5% gradient of aqueous 0.1% trifluoroacetic acid in acetonitrile as eluent. The fractions containing the desired compound were evaporated to dryness to afford the title compound (81 mg, 40%) as a colourless solid.

### Example 61

### 6-fluoro-N-{cis-4-[6-fluoro-1-(4'-{[4-(methylamino)piperidin-1-yl]methyl}biphenyl-3-yl)-2,4-dioxo-1,4-dihydropyrido[2,3-d]pyrimidin-3(2H)-yl]cyclohexyl}imidazo[1,2-a]pyridine-2-carboxamide

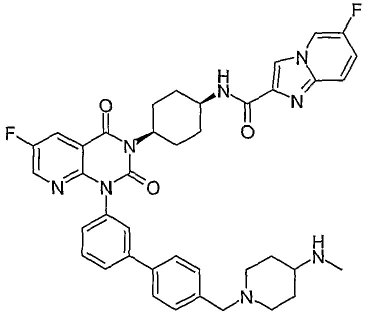

### Step (a) tert-butyl [1-({3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl}methyl)piperidin-4-yl]methylcarbamate

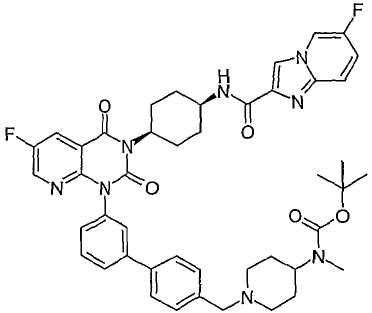

6-fluoro-*N*-{*cis*-4-[6-fluoro-1-(4'-formylbiphenyl-3-yl)-2,4-dioxo-1,4-dihydropyrido[2,3-d]pyrimidin-3(2*H*)-yl]cyclohexyl}imidazo[1,2-a]pyridine-2-carboxamide (300 mg, 0.48 mmol) and tert-butyl methyl(piperidin-4-yl)carbamate (155 mg, 0.73 mmol) were dissolved in 1,2-dichloroethane (4.576 mL) and the solution allowed to stir for 10 min. Sodium triacetoxyborohydride (145 mg, 0.69 mmol) was then added over a period of 10 min. under air. The resulting suspension was stirred at 25 °C for 16 h. The chlorinated solvent was removed *in vacuo* and the crude material was dissolved in methanol (5 mL) and loaded on to an 20g SCX cartridge. The impurities were washed through with methanol (100 mL) and discarded. The product was eluted with 3.5N methanolic ammonia (100 mL) and evaporated *in vacuo* to give the sub-title compound as a yellow residue (396 mg, 100%).
APCI (Multimode) m/z: 719 [M+H - Boc].

### Step (b) 6-fluoro-N-{cis-4-[6-fluoro-1-(4'-{[4-(methylamino)piperidin-1-yl]methyl}biphenyl-3-yl)-2,4-dioxo-1,4-dihydropyrido[2,3-d]pyrimidin-3(2H)-yl]cyclohexyl}imidazo[1,2-a]pyridine-2-carboxamide

A solution of Hydrogen chloride (4M/Dioxane, 2.418 mL, 9.67 mmol) was added dropwise to a stirred solution of amino} cyclohexyl)-2,4-dioxo-3,4-dihydropyrido[2,3-*d*]pyrimidin-1(2*H*)-yl]biphenyl-4-yl}methyl)piperidin-4-yl]methylcarbamate (396 mg, 0.48 mmol) in 1,4-Dioxane (2 mL) at 25 °C. The resulting solution was stirred at 25 °C for 4 days. The solvent was evaporated to dryness and then passed through an SCX column washing with methanol (100 mL) and eluting with 3.5N ammoniacal methanol (50 mL) to give the crude product as a yellow oil. The crude product was purified by preparative HPLC on a Waters X-Terra column using a 95-50% gradient of aqueous 0.2% trifluoroacetic acid in acetonitrile as eluent. The fractions containing the desired compound were evaporated to dryness to afford the title compound (70 mg, 20%) as a colourless solid.
¹H NMR (400 MHz, DMSO-*d6)* 8.96 (2H, s), 8.83 (1H, dd), 8.59 (1H, d), 8.42 (1H, s), 8.34 (1H, dd), 7.86 - 7.72 (5H, m), 7.66 (1H, t), 7.60 (2H, d), 7.51 - 7.42 (2H, m), 4.89 (1H, t), 4.35 (2H, s), 4.17 (1H, s), 3.55 - 3.48 (2H, m), 3.28 - 3.17 (1H, m), 3.08 - 2.97 (2H, m), 2.69 - 2.54 (4H, m), 2.27 - 2.17 (2H, m), 2.06 - 1.98 (2H, m), 1.81 - 1.62 (4H, m), 3.70 - 3.62 (4H, m).
APCI (Multimode) m/z: 719 [M+H].

### Example 62

### N-{cis-4-[6-fluoro-1-[2'-(morpholin-4-ylmethyl)biphenyl-3-yl]-2,4-dioxo-1,4-dihydropyrido [2,3-d]pyrimidin-3(2H)-yl]cyclohexyl}acetamide

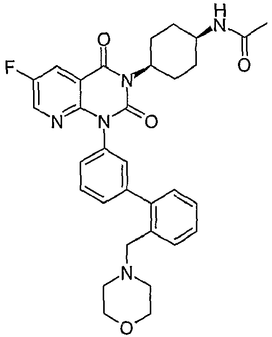

Acetic anhydride (0.060 mL, 0.64 mmol) was added to a mixture of 3-(*cis*-4-aminocyclohexyl)-6-fluoro-1-[2'-(morpholin-4-ylmethyl)biphenyl-3-yl]pyrido[2,3-*d*]pyrimidine-2,4(1*H*,3*H*)-dione (169 mg, 0.32 mmol) and *N*-Ethyldiisopropylamine (0.111 mL, 0.64 mmol) in methanol (3.776 mL) at 25 °C over a period of 1 minute under nitrogen. The resulting solution was stirred at 25 °C for 30 min. The reaction mixture was evaporated to dryness and redissolved in ethyl acetate (50 mL), then washed with 1M hydrochloric acid (50 mL), saturated sodium aqueous hydrogen carbonate (50 mL), and water (50 mL). The organic layer was dried over magnesium sulfate, filtered and evaporated to afford the title compound (135 mg, 74 %) as a colourless solid.
¹H NMR (400 MHz, DMSO-d6) 7.60 (1H, d), 7.44 (1H, dd), 7.00 (1H, d), 6.77 (1H, t), 6.69 - 6.61 (3H, m), 6.55 - 6.49 (4H, m), 4.19 - 4.10 (1H, m), 3.20 - 3.14 (1H, m), 2.74 - 2.64 (6H, m), 1.92 - 1.79 (2H, m), 1.57 - 1.48 (4H, m), 1.20 - 1.14 (5H, m), 0.91 - 0.77 (4H, m).
APCI (Multimode) m/z: 572 [M+H].

### Example 63

### N-{cis-4-[6-fluoro-1-[2'-(morpholin-4-ylmethyl)biphenyl-3-yl]-2,4-dioxo-1,4-dihydropyrido[2,3-d]pyrimidin-3(2H)-yl]cyclohexyl}-2-hydroxybenzamide

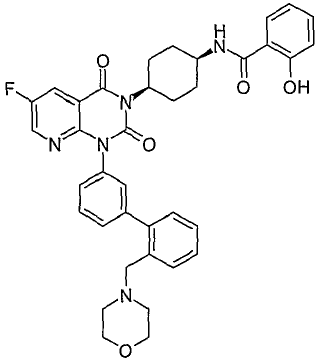

3-(*cis*-4-aminocyclohexyl)-6-fluoro-1-[2'-(morpholin-4-ylmethyl)biphenyl-3-yl]pyrido[2,3-*d*]pyrimidine-2,4(1*H*,3*H*)-dione (182 mg, 0.34 mmol) was added to a pre-stirred solution (10 min.) of Salicylic acid (52 mg, 0.38 mmol), O-(7-Azabenzotriazol-1-yl)-N,N,N',N'-tetramethyluronium hexafluorophosphate (144 mg, 0.38 mmol) and N,N-diisopropylethylamine (0.180 mL, 1.03 mmol) in DMF (1.7 mL) at 25 °C. The resulting solution was stirred at 25 °C for 2 h. The reaction mixture was diluted with ethyl acetate (50 mL), and washed with water (150 mL). The aqueous layer was back extracted with ethyl acetate (1 x 50 mL), and the combined organic layer was dried over sodium sulphate, filtered and evaporated to afford the crude product. The crude product was purified by preparative LCMS on a Waters X-Terra column using a 75-5% gradient of aqueous 0.2% ammonia in acetonitrile as eluent. The fractions containing the desired compound were evaporated to dryness to afford the title compound (27 mg, 12 %) as a colourless solid.
¹H NMR (400 MHz, DMSO-*d6*) 8.62 - 8.55 (2H, m), 8.33 (1H, dd), 7.96 (1H, dd), 7.74 - 7.34 (7H, m), 6.98 - 6.86 (3H, m), 4.90 - 4.80 (1H, m), 4.39 - 4.28 (2H, m), 4.18 - 4.10 (1H, m), 3.82 - 3.59 (4H, m), 3.22 - 3.05 (4H, m), 2.79 - 2.57 (2H, m), 2.09 - 1.94 (2H, m), 1.78 - 1.56 (4H, m).
APCI (Multimode) m/z: 650 [M+H].

### Example 64

### 6-fluoro-N-{cis-4-[6-fluoro-1-[4'-({[2-(methylamino)ethyl]amino}methyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

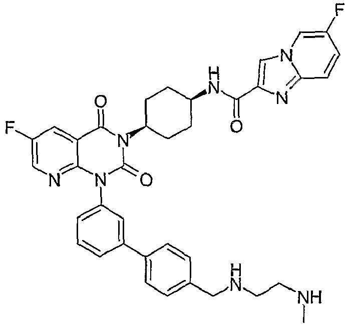

### Step (a) tert-butyl {2-[({3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl}methyl)amino]ethyl}methylcarbamate

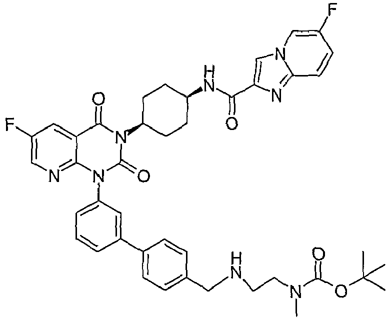

6-fluoro-*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (200 mg, 0.32 mmol) and Boc-N-methylethylenediamine (0.053 mL, 0.29 mmol) were dissolved in 1,2-Dichloroethane (3.5 mL) and the solution allowed to stir for 10 min. Sodium triacetoxyborohydride (102 mg, 0.48 mmol) was then added over a period of 10 min. under air. The resulting suspension was stirred at 25 °C for 16 h. The reaction mixture was diluted with dichloromethane (50 mL), and washed with water (50 mL). The organic was dried over sodium sulfate, filtered and evaporated to afford the sub-title compound (251 mg, 100%) which was taken to the next stage of the reaction without further purification.
APCI (Multimode) m/z: 679 [M+H - Boc].

### Step (b) 6-fluoro-N{cis-4-[6-fluoro-1-[4'-({[2-(methylamino)ethyl]amino}methyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

Hydrogen chloride (4M/Dioxane) (2.04 mL, 8.14 mmol) was added dropwise to a stirred solution of *tert*-butyl {2-[({3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}methyl)amino]ethyl}methylcarbamate (251 mg, 0.32 mmol) in 1,4-Dioxane (3 mL) at 25 °C under air. The resulting solution was stirred at 25 °C for 16 h. The crude material was dissolved in methanol (50 mL), acidified with acetic acid (0.5 mL) and loaded on to an 10g SCX cartridge. The impurities were washed through with methanol (200 mL) and discarded. The product was eluted with 1N methanolic ammonia (100 mL) and evaporated *in vacuo* to give the crude product as a yellow oil. This was then purified by preparative LCMS on a Waters X-Terra column using a 75-5% gradient of aqueous 0.1% trifluoroacetic acid in acetonitrile as eluent. The fractions containing the desired compound were evaporated to dryness to afford the title compound (114 mg, 42 %) as a colourless solid.
¹H NMR (400 MHz, DMSO-*d₆*) 9.16 - 9.00 (2H, m), 8.82 - 8.78 (1H, m), 8.69 - 8.56 (3H, m), 8.39 - 8.32 (2H, m), 7.85 - 7.71 (4H, m), 7.70 - 7.56 (4H, m), 7.49 - 7.41 (2H, m), 4.87 (1H, s), 4.27 (2H, s), 4.17 (1H, s), 3.32 - 3.19 (4H, m), 2.70 - 2.52 (5H, m), 2.06 - 1.97 (2H, m), 1.78 - 1.62 (4H, m).
APCI (Multimode) m/z: 679 [M+H].

### Example 65

### N-[cis-4-(1-biphenyl-3-yl-6-fluoro-2,4-dioxo-1,4-dihydropyrido[2,3-d]pyrimidin-3(2H)-yl)cyclohexyl]acetamide

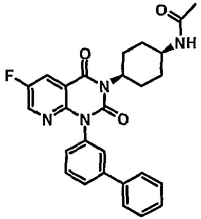

To a solution of 3-(*cis*-4-aminocyclohexyl)-1-biphenyl-3-yl-6-fluoropyrido[2,3-*d*]pyrimidine-2,4(1*H*,3*H*)-dione (0.2 g, 0.46mmol) in dichloromethane (10 mL) was added DIEA (0.5 mL) followed by acetyl chloride (0.04mL, 0.46 mmol) and the mixture stirred overnight. The mixture was evaporated to dryness and the residue triturated with water (10 mL) to give a buff solid, which was collected and purified silica chromatography ethyl acetate:dichloromethane (2:8) as the eluent to give the title compound as a colourless solid (96 mg, 43%)
1H NMR (400 MHz, DMSO-*d₆*) δ 8.59 (1H, d), 8.30 (1H, dd), 7.68 (6H, m), 7.48 (2H, t), 7.39 (2H, t), 4.77 (1H, t), 3.78 (1H, s), 2.62 (2H, m), 1.86 (5H, m), 1.53 (4H, m)

APCI (Multimode) m/z: 473.2 [M+H]

### Example 66

### 1-biphenyl-3-yl-3-[cis-4-(dimethylamino)cyclohexyl]-6-fluoropyrido[2,3-d]pyrimidine-2,4(1H,3H)-dione

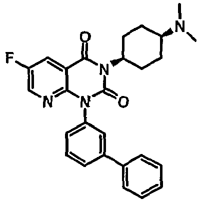

To a solution of 3-(*cis*-4-aminocyclohexyl)-1-biphenyl-3-yl-6-fluoropyrido[2,3-*d*]pyrimidine-2,4(1*H*,3*H*)-dione (0.15 g, 0.34 mmol) in dichloroethane (10 mL) was added 38-40% aqueous formaldehyde solution (0.1 mL) followed by sodium triacetoxyborohydride (0.144 mg, 0.68 mmol). The mixture was stirred for 2 h, evaporated to dryness and the residue purified by reverse phase HPLC (25-95% acetonitrile in aqueous ammonia) to afford the title compound (80 mg, 50%).

¹H NMR (400 MHz, DMSO-*d₆*) δ 8.58 (1H, d), 8.29 (1H, dd), 7.77 (1H, m), 7.71 (1H, m), 7.67 (2H, dd), 7.61 (1H, t), 7.48 (2H, m), 7.38 (2H, m), 4.82 (1H, m), 2.69 (2H, dd), 2.15 (6H, s), 2.04 (2H, d), 1.95 (1H, s), 1.39 (4H, m) 8.59 (1H, d), 8.30 (1H, m), 7.68 (6H, m), 7.48 (2H, t), 7.39 (2H, t), 4.77 (1H, t), 3.78 (1H, s), 2.62 (2H, m), 1.86 (5H, m), 1.53 (4H, m)
APCI (Multimode) m/z: 459.2 [M+H]

### Example 70

### 1-amino-N-{cis-4-[6-fluoro-1-[2'-(morpholin-4-ylmethyl)biphenyl-3-yl]-2,4-dioxo-1,4-dihydropyrido[2,3-d]pyrimidin-3(2H)-yl]cyclohexyl}cyclopentanecarboxamide trifluroacetate salt

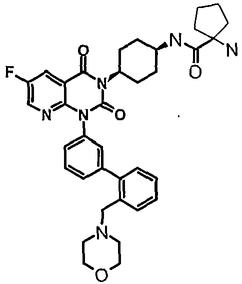

1-(Boc-amino)cyclopentanecarboxylic acid (0.082 g, 0.36 mmol), DIEA (0.063 mL, 0.36 mmol) and HATU (0.136 g, 0.36 mmol) in DMF (10 mL) was stirred for 10 mins at room temperature. To this solution was added 3-(*cis*-4-aminocyclohexyl)-6-fluoro-1-[2'-(morpholin-4-ylmethyl)biphenyl-3-yl]pyrido[2,3-*d*]pyrimidine-2,4(1*H*,3*H*)-dione (0.190 g, 0.36 mmol) and the mixture stirred overnight. The mixture was poured onto water (100 mL) and the crude intermediate collected by filtration and dried in vacuo. This solid (0.210 g) was dissolved 4.0M hydrogen chloride in dioxane (10 mL) and allowed to stand for 1 hr.
The mixture was evaporated to dryness and purified by reverse phase HPLC (5-50% acetonitrile in aqueous trifluoroacetic acid) to afford the title compound (148 mg, 47%). ¹H NMR (400 MHz, DMSO-*d₆*) δ 8.59 (1H, d), 8.33 (1H, dd), 8.11 (3H, s), 7.77 - 7.38 (9H, m), 4.78 (1H, d), 4.44 - 2.55 (16H, m), 2.27 (1H, t), 2.02 (2H, d), 1.83 (5H, s), 1.66 - 1.47 (3H, m)
APCI (Multimode) m/z: 641.3 [M+H]

### Example 71

### 6-fluoro-N-{cis-4-[6-fluoro-1-{4'-hydroxy-2'-[(4-methylpiperazin-1-yl)methyl]biphenyl-3-yl} -2,4-dioxo-1,4-dihydropyrido[2,3-d]pyrimidin-3(2H)-yl]cyclohexyl}imidazo[1,2-a]pyridine-2-carboxamide trifluroacetate salt

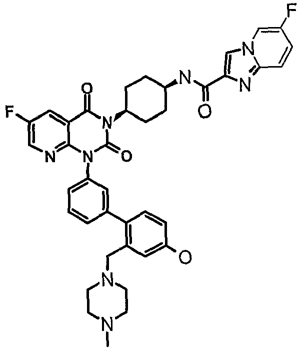

### Step (a) 5-hydroxy-2-(4,4,5,5-tetramethyl-1,3,2-dioxaborolan-2-yl)benzaldehyde

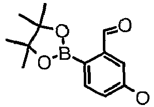

1,1'-bis(diphenylphosphino)ferrocenedichloro-palladium(ii) dichloromethane complex (1.009 g, 1.24 mmol) and 1,1'-bis(diphenylphosphino)ferrocene (0.685 g, 1.24 mmol) were stirred in DMSO (40 mL) at rt. for 10 min. 2-bromo-5-hydroxybenzaldehyde (5.00 g, 24.87 mmol), potassium acetate (7.28 g, 74.18 mmol) and diboron pinacol ester (8.16 g, 32.14 mmol) were added under nitrogen. The reaction was stirred for 16 h at 70°C, cooled and poured onto water (300 mL) . The resulting mixture was extracted into diethyl ether and washed with brine and dried over sodium sulfate to give the title compound (6.7g, 109%) as a purple gum. Used in the next step without purification or analysis

### Step (b) 6-fluoro-N-{cis-4-[6-fluoro-1-(2'-formyl-4'-hydroxybiphenyl-3-yl)-2,4-dioxo-1,4-dihydropyrido[2,3-d]pyrimidin-3(2H)-yl]cyclohexy}imidazo[1,2-a]pyridine-2-carboxamide

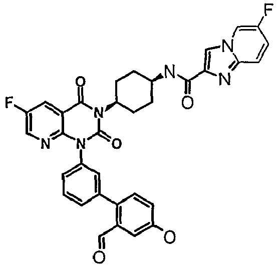

To a suspension of palladium acetate (8 mg, 0.04 mmol) in acetonitrile (2 mL) was added 2-dicyclohexylphosphino-2',6'-dimethoxy-1,1'-biphenyl (0.032 g, 0.08 mmol), and mixture stirred at room temperature, over a period of 10 minute under nitrogen. To the resulting solution was added potassium carbonate (0.323 g, 2.34 mmol) dissolved in water (1.6 mL), followed by 5-hydroxy-2-(4,4,5,5-tetramethyl-1,3,2-dioxaborolan-2-yl)benzaldehyde (0.290 g, 1.17 mmol) and 6-fluoro-*N*-{*cis*-4-[6-fluoro-1-(3-iodophenyl)-2,4-dioxo-1,4-dihydropyrido[2,3-*d*]pyrimidin-3(2*H*)-yl]cyclohexyl}imidazo[1,2-*a*]pyridine-2-carboxamide (0.500 g, 0.78 mmol) and the resultant mixture heated at 70 °C, for a further 2 h. The reaction mixture was diluted with ethyl acetate (15 mL), and washed with saturated brine (10 mL). The organic layer was dried over sodium sulfate. and purified silica chromatography ethyl acetate: dichloromethane (1:9) as eluent to give the title compound as a tan coloured solid (195 mg, 40%)
¹H NMR (400 MHz, DMSO-*d₆*) δ 10.07 (1H, s), 9.91 (1H, s), 8.79 (1H, dd), 8.60 (1H, d), 8.37 (2H, s), 8.32 (Hz, 2H, dd), 7.75 (2H, dd), 7.54 - 7.39 (4H, m), 7.29 (1H, d), 7.17 (1H, dd), 4.85 (1H, t), 4.16 (1H, s), 2.71 - 2.54 (2H, m), 2.04 - 1.95 (2H, m), 1.75 - 1.59 (4H, m) APCI (Multimode) m/z: 637.1 [M+H]

### Step (c) 6-fluoro-N-{cis-4-[6-fluoro-1-{4'-hydroxy-2'-[(4-methylpiperazin-1-yl)methyl]biphenyl-3-yl}-2,4-dioxo-1,4-dihydropyrido[2,3-d]pyrimidin-3(2H)-yl]cyclohexyl}imidazo[1,2-a]pyridine-2-carboxamide trifluroacetate salt.

1-methylpiperazine (34.0 mg, 0.34 mmol), and sodium triacetoxyborohydride (77 mg, 0.36 mmol) was added to 6-fluoro-*N*-{*cis*-4-[6-fluoro-1-(2'-formyl-4'-hydroxybiphenyl-3-yl)-2,4-dioxo-1,4-dihydropyrido[2,3-d]pyrimidin-3(2*H*)-yl]cyclohexyl}imidazo[1,2-*a*]pyridine-2-carboxamide (180 mg, 0.28 mmol) in dichloroethane (10 mL). The resulting suspension was stirred overnight. The mixture was evaporated to dryness and purified by reverse phase HPLC (5-50% acetonitrile in aqueous trifluoroacetic acid) to afford the title compound (119 mg, 44%).

¹H NMR (400 MHz, DMSO-*d₆*) δ 8.87 - 8.79 (1H, m), 8.60 (1H, d), 8.41 (1H, s), 8.34 (1H, dd), 7.81 - 7.70 (2H, m), 7.59 - 7.27 (5H, m), 7.14 (1H, d), 6.89 - 6.77 (2H, m), 4.85 (1H, s), 4.17 (1H, s), 3.60 - 3.16 (4H, m), 2.97 - 2.75 (6H, m), 2.72 (4H, s), 2.68 - 2.09 (2H, m), 2.09 - 1.93 (2H, m), 1.80 - 1.57 (4H, m)
APCI (Multimode) m/z: 721.3 [M+H]

### Example 72

### 6-fluoro-N-{cis-4-[6-fluoro-1-[4'-hydroxy-2'-(piperazin-1-ylmethyl)biphenyl-3-yl]-2,4-dioxo-1,4-dihydropyrido[2,3-d]pyrimidin-3(2H)-yl]cyclohexyl}imidazo[1,2-a]pyridine-2-carboxamide trifluoroacetate salt

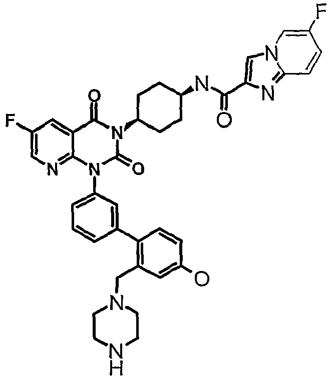

tert-butyl piperazine-1-carboxylate (63.2 mg, 0.34 mmol), and sodium triacetoxyborohydride (77 mg, 0.36 mmol) was added to 6-fluoro-*N*-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (180 mg, 0.28 mmol) in dichloroethane (10 mL). The resulting suspension was stirred overnight. The mixture was evaporated to dryness and the residue dissolved in dioxane (10 mL) followed by 4.0M hydrogen chloride in dioxane (10 mL), and the mixture stirred for 1 hr. The mixture was evaporated to dryness and purified by reverse phase HPLC (5-50% acetonitrile in aqueous trifluoroacetic acid) to afford the title compound (106 mg, 40%).

¹H NMR (400 MHz, DMSO-*d₆*) δ 8.83 (1H, dd), 8.60 (1H, d), 8.53 (2H, s), 8.40 (1H, s), 8.34 (2H, dd), 7.80 - 7.69 (2H, m), 7.59 - 7.31 (6H, m), 7.16 (1H, d), 6.88 (1H, s), 6.82 (1H, dd), 4.87 (3H, m), 4.17 (2H, s), 3.52 (1H, s), 2.99 (3H, s), 2.69 - 2.52 (2H, m), 2.02 (2H, d), 1.79 - 1.57 (4H, m)
APCI (Multimode) m/z: 707.3 [M+H]

### Example 73

### 6-fluoro-N-{cis-4-[6-fluoro-1-(4'-hydroxy-2'-{[4-(methylamino)piperidin-1-yl]methyl}biphenyl-3-yl)-2,4-dioxo-1,4-dihydropyrido[2,3-d]pyrimidin-3(2H)-yl]cyclohexyl}imidazo[1,2-a]pyridine-2-carboxamide trifluoroacetate salt

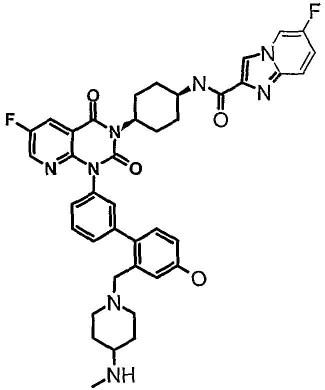

4-N-Boc-4-N-Methyl-aminopiperidine (78 mg, 0.36 mmol), and sodium triacetoxyborohydride (77 mg, 0.36 mmol) was added to 6-fluoro-*N*-{*cis*-4-[6-fluoro-1-(2'-formyl-4'-hydroxybiphenyl-3-yl)-2,4-dioxo-1,4-dihydropyrido[2,3-d]pyrimidin-3(2*H*)-yl]cyclohexyl}imidazo[1,2-*a*]pyridine-2-carboxamide (180 mg, 0.28 mmol) in dichloroethane (10 mL). The resulting suspension was stirred overnight. The mixture was evaporated to dryness and the residue dissolved in dioxane (10 mL) followed by 4.0M hydrogen chloride in dioxane (10 mL), and the mixture stirred for 1 hr. The mixture was evaporated to dryness and purified by reverse phase HPLC (5-50% acetonitrile in aqueous trifluoroacetic acid) to afford the title compound (57 mg, 21%).
¹H NMR (400 MHz, DMSO-*d₆*) δ 10.03 - 9.73 (2H, m), 8.89 (2H, s), 8.82 (1H, dd), 8.61 (1H, d), 8.42 - 8.32 (2H, m), 7.77 (1H, dd), 7.70 (1H, d), 7.62 (1H, t), 7.51 - 7.45 (1H, m), 7.41 (2H, dd), 7.29 - 7.21 (1H, m), 7.13 - 7.05 (1H, m), 7.00 - 6.93 (1H, m), 4.85 (3H, s), 4.35 - 4.11 (6H, m), 3.34 (1H, s), 3.12 (1H, s), 2.69 - 2.48 (2H, m), 2.02 (4H, d), 1.69 (6H, dd)
APCI (Multimode) m/z: 735.3 [M+H]

### Example 74

### 1-biphenyl-3-yl-6-fluoro-3-(cis-4-{[(6-fluoroimidazo[1,2-a]pyridin-2-yl)methyl]amino}cyclohexyl)pyrido[2,3-d]pyrimidine-2,4(1H,3H)-dione

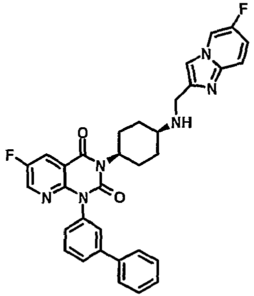

### Step (a) 3-(cis-4-aminocyclohexyl)-1-biphenyl-3-yl-6-fluoropyrido[2,3-d]pyrimidine-2,4(1H,3H)-dione hydrochloride

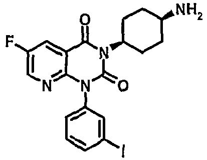

A suspension of *tert*-butyl {*cis*-4-[6-fluoro-1-(3-iodophenyl)-2,4-dioxo-1,4-dihydropyrido[2,3-*d*]pyrimidin-3(2*H*)-yl]cyclohexyl]carbamate (10 g, 17.3 mmol) and HCl (43 mL, 173 mmol) (4M in dioxane) in diethyl ether (150 mL) was stirred for 20h. Ether (200 mL) added and the solid was filtered washing with ether to give the sub-title compound (9.0 g).
APCI (Multimode) m/z: 431 [M+H]

### Step (b) (6-fluoroimidazo[1,2-a]pyridin-2-yl)methanol

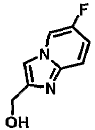

6-fluoroimidazo[1,2-*a*]pyridine-2-carboxylic acid (4 g, 22.21 mmol) was suspended in THF (10 mL) and borane tetrahydrofuran complex (111 mL, 111 mmol) was added. The mixture was heated at reflux for 3h then cooled to ambient temperature, methanol (30 mL) was added dropwise and heated at reflux for 18h. The reaction was cooled and concentrated *in vacuo.* The resulting solid was dissolved in methanol and passed down an SCX column, washing with methanol and then eluting the product with ammonia in methanol (7M) to leave, after evaporation, the sub-title compound as a solid (3.5 g, 95%).
APCI (Multimode) m/z: 167 [M+H]

### Step (c) 2-(chloromethyl)-6-fluoroimidazo[1,2-a]pyridine

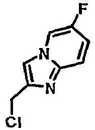

(6-fluoroimidazo[1,2-*a*]pyridin-2-yl)methanol (4.2 g, 25.3 mmol) was heated at 60 °C in thionyl chloride (46.0 mL, 630 mmol) for 2h. The resulting solution was cooled and concentrated to leave a solid, which was triturated with diethyl ether and *dried in vacuo* to give the sub-title compound as a solid (3.45 g, 74%).
¹H NMR (300 MHz, DMSO-*d₆*) δ 9.25 (1H, t), 8.54 (1H, s), 8.06 (1H, dd), 7.93 (1H, ddd), 4.99 (2H, s)
GC 184

### Step (d) 6-fluoro-3-(cis-4-{[(6-fluoroimidazo[1,2-a]pyridin-2-yl)methyl]aminol}cyclohexyl)-1-(3-iodophenyl)pyrido[2,3-d]pyrimidine-2,4(1H,3H)-dione

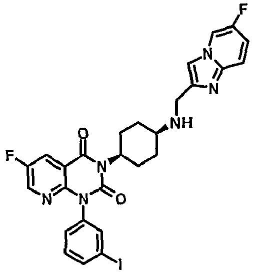

To a suspension of 2-(chloromethyl)-6-fluoroimidazo[1,2-*a*]pyridine (0.55 g, 2.98 mmol), 3-(*cis*-4-aminocyclohexyl)-6-fluoro-1-(3-iodophenyl)pyrido[2,3-*d*]pyrimidine-2,4(1*H*,3*H*)-dione hydrochloride (1.54 g, 2.98 mmol) and Hunig's Base (2.08 mL, 11.9 mmol) in acetonitrile (5 mL) was heated at 80 °C for 48h. The reaction was cooled, filtered and concentrated *in vacuo.* Ethyl acetate was added and the precipitate was filtered washing with ethyl acetate and dried in vacuo to leave a beige solid. The filtrate was purified by flash silica chromatography, elution gradient 100% ethyl acetate to 5% methanol in ethyl acetate. Pure fractions were evaporated to dryness to afford a further amount of the sub-title compound as a white solid (560 mg, 30%).
ES+ (M+H) 629

### Step (e) 1-biphenyl-3-yl-6-fluoro-3-(cis-4-{[(6-fluoroimidazo[1,2-a]pyridin-2-yl)methyl]amino}cyclohexyl)pyrido[2,3-d]pyrimidine-2,4(1H,3H)-dione

Palladium (II) acetate (3.6 mg, 0.02 mmol) and 2-dicyclohexylphosphino-2',6'-dimethoxy-1,1'-biphenyl (13 mg, 0.03 mmol) were stirred together in acetonitrile (5 mL) for 10 min. before the addition of a solution of potassium carbonate (132 mg, 0.95 mmol) in Water (2 mL). 6-fluoro-3-(*cis*-4-{[(6-fluoroimidazo[1,2-*a*]pyridin-2-yl)methyl]amino}cyclohexyl)-1-(3-iodophenyl)pyrido[2,3-*d*]pyrimidine-2,4(1*H*,3*H*)-dione (200 mg, 0.32 mmol) and phenylbornic acid (39 mg, 0.32 mmol) were then added sequentially. The resulting solution was stirred at 80°C for 16h then cooled, concentrated and the crude product was purified by preparative HPLC on a Waters X-Terra column using a 75-5% gradient of aqueous 0.2% ammonia in acetonitrile as eluent. The crude product was purified by preparative HPLC on a Symmetry column using a 95-5% gradient of aqueous 0.1% trifluoroacetic acid in acetonitrile as eluent. The fractions containing the desired compound were evaporated to dryness to afford the title compound as a white solid (21 mg, 11%).
¹H NMR (300 MHz, DMSO-*d₆*) δ 8.94 - 8.81 (3H, m), 8.61 (1H, d), 8.31 (1H, dd), 8.09 (1H, s), 7.81 - 7.71 (2H, m), 7.69 - 7.59 (4H, m), 7.53 - 7.43 (2H, m), 7.43 - 7.34 (2H, m), 4.88 - 4.77 (1H, m), 4.35 (2H, s), 3.42 - 3.32 (2H, m), 2.18 - 2.07 (2H, m), 1.84 - 1.60 (6H, m)
APCI (Multimode) m/z: 579 [M+H]

### Example 75

### 6-fluoro-3-(cis-4-{[(6-fluoroimidazo[1,2-a]pyridin-2-yl)methyl]amino}cyclohexyl)-1-[4'-(piperazin-1-ylmethyl)biphenyl-3-yl]pyrido[2,3-d]pyrimidine-2,4(1H,3H)-dione

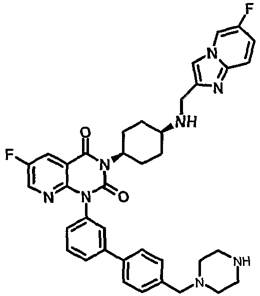

### Step (a) tert-butyl {cis-4-[6-fluoro-1-(4'-formylbiphenyl-3-yl)-2,4-dioxo-1,4-dihydropyrido[2,3-d]pyrimidin-3(2H)-yl]cyclohexyl}[(6-fluoroimidazo[1,2-a]pyridin-2-yl)methyl]carbamate

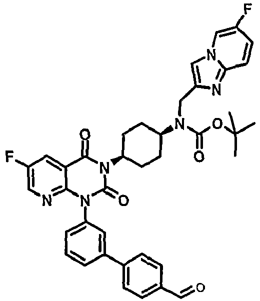

Palladium (II) acetate (3.6 mg, 0.02 mmol) and 2-dicyclohexylphosphino-2',6'-dimethoxy-1,1'-biphenyl (13 mg, 0.03 mmol) were stirred together in acetonitrile (5 mL) for 10 min before the addition of a solution of potassium carbonate (132 mg, 0.95 mmol) in Water (2 mL). 6-fluoro-3-(*cis*-4-{[(6-fluoroimidazo[1,2-*a*]pyridin-2-yl)methyl]amino}cyclohexyl)-1-(3-iodophenyl)pyrido[2,3-*d*]pyrimidine-2,4(1*H*,3*H*)-dione (200 mg, 0.32 mmol) and 4-formylbenzeneboronic acid (48 mg, 0.32 mmol) were then added sequentially. The resulting solution was stirred at 80°C for 16h then cooled, concentrated and the crude product was dissolved in dichloromethane (10 mL) and filtered. The filtrate was treated with di-t-butyl dicarbonate (0.15 mL, 0.64 mmol) and stirred for 20h at ambient temperature. The crude product was purified by flash silica chromatography, elution gradient 70 to 100% ethyl acetate in isohexane. Pure fractions were evaporated to dryness to afford the sub-title compound as a brown solid (75 mg, 33%).

APCI (Multimode) m/z: 707 [M+H]

### Step (b) 6-fluoro-3-(cis-4-{[(6-fluoroimidazo[1,2-a]pyridin-2-yl)methyl]amino}cyclohexyl)-1-[4'-(piperazin-1-ylmethyl)biphenyl-3-yl]pyrido[2,3-d]pyrimidine-2,4(1H,3H-dione

*tert*-butyl {*cis*-4-[6-fluoro-1-(4'-formylbiphenyl-3-yl)-2,4-dioxo-1,4-dihydropyrido[2,3-d]pyrimidin-3(2*H*)-yl]cyclohexyl}[(6-fluoroimidazo[1,2-*a*]pyridin-2-yl)methyl]carbamate (150 mg, 0.21 mmol) and t-butyl 1-piperazine carboxylate (79 mg, 0.42 mmol) were stirred in DCM (15 mL) for 1h and sodium triacetoxyborohydride (45 mg, 0.21 mmol) was added. The mixture was stirred for 20h. Methanol was added and the solution was concentrated *in vacuo.* The crude product was purified by preparative HPLC on a Waters X-Terra column using a 75-5% gradient of aqueous 0.2% ammonia in acetonitrile as eluent. The fractions containing the desired compound were evaporated to dryness, dissolved in DCM (10 mL) and trifluoroacetic acid (5 mL) and stirred for 18h. The solution was concentrated *in vacuo* and the residue was triturated with diethyl ether to leave the title compound as a white solid (70 mg, 49%).
¹H NMR (400 MHz, DMSO-*d₆*) δ 8.89 (1H, s), 8.94 - 8.76 (2H, m), 8.60 (1H, d), 8.31 (1H, dd), 8.09 (1H, s), 7.79 (1H, d), 7.74 - 7.60 (5H, m), 7.50 (2H, d), 7.42 (2H, d), 4.85 - 4.76 (1H, m), 4.35 (2H, s), 3.97 - 3.88 (1H, m), 3.42 - 3.33 (1H, m), 3.21 (3H, s), 2.97 - 2.80 (2H, m), 2.17 - 2.07 (2H, m), 1.82 - 1.70 (2H, m), 1.69 - 1.61 (2H, m), 2.68 - 2.56 (2H, m) APCI (Multimode) m/z: 677 [M+H]

### Example 76

### 6-fluoro-3-(cis-4-{[(6-fluoroimidazo[1,2-a]pyridin-2-yl)methyl]amino}cyclohexyl)-1-[4'-(morpholin-4-ylmethyl)biphenyl-3-yl]pyrido[2,3-d]pyrimidine-2,4(1H,3H)-dione ditrifluoroacetic acid

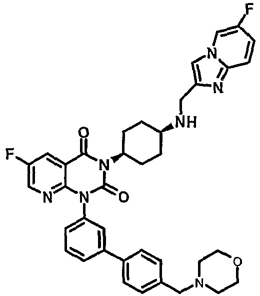

Palladium (II) acetate (3.6 mg, 0.02 mmol) and 2-dicyclohexylphosphino-2',6'-dimethoxy-1,1'-biphenyl (13 mg, 0.03 mmol) were stirred together in acetonitrile (5 mL) for 10 min before the addition of a solution of potassium carbonate (132 mg, 0.95 mmol) in Water (2 mL). 6-fluoro-3-(*cis*-4-{[(6-fluoroimidazo[1,2-*a*]pyridin-2-yl)methyl]amino}cyclohexyl)-1-(3-iodophenyl)pyrido[2,3-*d*]pyrimidine-2,4(1*H*,3*H*)-dione (200 mg, 0.32 mmol) and 4-(4-morpholinomethyl)-phenylboronic acid pinacol (96 mg, 0.32 mmol) were then added sequentially. The resulting solution was stirred at 80°C for 48h. The crude product was purified by preparative HPLC on a Waters X-Terra column using a 75-5% gradient of aqueous 0.1% trifluoroacetic acid in acetonitrile as eluent. The crude product was purified by preparative HPLC on a Symmetry Sunfire column using a 95-5% gradient of aqueous 0.1 % trifluoroacetic acid in acetonitrile as eluent. The fractions containing the desired compound were evaporated to dryness to afford the title compound as a white solid (12 mg, 6%).
¹H NMR (300 MHz, DMSO-*d₆*) δ 8.96 - 8.82 (3H, m), 8.61 (1H, d), 8.32 (1H, s), 8.09 (1H, s, dd), 7.85 - 7.73 (4H, m), 7.69 - 7.57 (4H, m), 7.47 - 7.36 (2H, m), 4.86 - 4.75 (1H, m), 4.38 (4H, s), 4.35 (4H, s), 3.70 - 3.59 (3H, m), 3.41 - 3.05 (6H, m), 2.31 - 2.07 (2H, m), 1.83 - 1.60 (6H, m)
APCI (Multimode) m/z: 677 [M+H]

### Example 77

### 6-fluoro-N-{cis-4-[6-fluoro-1-{4'-[2-(methylamino)ethoxy]biphenyl-3-yl}-2,4-dioxo-1,4-dihydropyrido[2,3-d]pyrimidin-3(2H)-yl]cyclohexyl}imidazo[1,2-a]pyridine-2-carboxamide

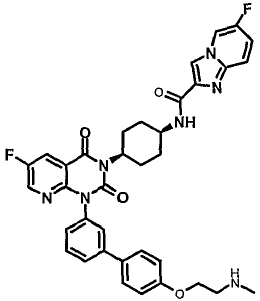

### Step (a) N-{cis-4-[1-[4'-(2-chloroethoxy)biphenyl-3-yl]-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

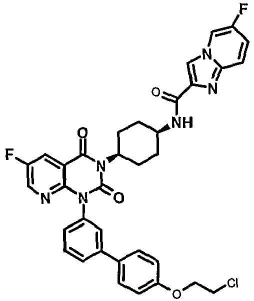

To a solution of 6-fluoro-*N*-{*cis*-4-[6-fluoro-1-(4-hydroxybiphenyl-3-yl)-2,4-dioxo-1,4-dihydropyrido[2,3-*d*]pyrimidin-3(2*H*-yl]cyclohexyl}imidazo[1,2-*a*]pyridine-2-carboxamide (1 g, 0.82 mmol), triphenylphosphine (0.43 g, 1.64 mmol) and 2-chloroethanol (0.11 mL, 1.64 mmol) in THF (50 mL) was added diisopropyl azodicarboxylate (0.33 g, 1.64 mmol) dropwise over 2 min giving a gentle exotherm. The resulting yellow solution was stirred for 20h and a further triphenylphosphine (0.431 g, 1.64 mmol) and 2-chloroethanol (0.110 mL, 1.64 mmol) were added followed by diisopropyl azodicarboxylate (0.332 g, 1.64 mmol) and the solution was stirred for 48h. The cream solid was filtered off washing with ether and dried in vacuo to give the sub-title compound (405 mg, 74%).
APCI (Multimode) m/z: 671 [M+H]

### Step (b) 6-fluoro-N-{cis-4-[6-fluoro-1-{4'-[2-(methylamino)ethoxy]biphenyl-3-yl}-2,4-dioxo-1,4-dihydropyrido[2,3-d]pyrimidin-3(2H)-yl]cyclohexyl}imidazo[1,2-a]pyridine-2-carboxamide

*N*-{*cis*-4-[1-[4'-(2-chloroethoxy)biphenyl-3-yl]-6-fluoro-2,4-dioxo-1,4-dihydropyrido[2,3-d]pyrimidin-3(2*H*)-yl]cyclohexyl}-6-fluoroimidazo[1,2-*a*]pyridine-2-carboxamide (0.2 g, 0.30 mmol), potassium iodide (2.5 mg, 0.01 mmol) and methylamine (1.0 g, 12.9 mmol) *in* acetonitrile (5 mL) were heated at 70 °C for 7h. The reaction was cooled, concentrated in *vacuo* and passed down an SCX column, washing with methanol and eluting with ammonia (7M in MeOH) to leave a solid, which was purified by preparative HPLC on a Waters X-Terra column using a 75-5% gradient of aqueous 0.2% ammonia in acetonitrile as eluent. The fractions containing the desired compound were evaporated to dryness to afford the title compound as a white solid (65 mg, 33%).
¹H NMR (300 MHz, DMSO-*d₆*) δ 8.59 (1H, d), 8.36 (1H, s), 8.33 (1H, dd), 7.78 - 7.71 (2H, m), 7.70 - 7.61 (3H, m), 7.58 (1H, t), 7.48 - 7.40 (1H, m), 7.33 (1H, d), 7.07 (2H, d), 4.93 - 4.82 (1H, m), 4.22 - 4.13 (1H, m), 4.19 (2H, t), 3.18 (2H, t), 2.55 (3H, s), 2.31 - 2.23 (2H, m), 2.05 - 1.95 (2H, m), 1.79 - 1.60 (4H, m)
APCI (Multimode) m/z: 666 [M+H]

### Example 78

### N-{cis-4-[1-{4'-[2-(dimethylamino)ethoxy]biphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

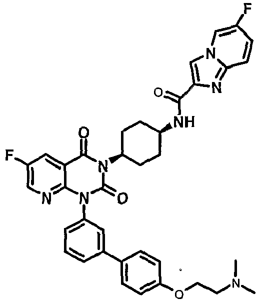

A solution of 6-fluoro-*N*-{*cis*-4-[6-fluoro-1-(4'-hydroxybiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.2 g, 0.33 mmol), cesium carbonate (0.214 g, 0.66 mmol) , potassium iodide (5.46 mg, 0.03 mmol) and 2-dimethylaminoethyl chloride hydrochloride (47 mg, 0.33 mmol) in NMP (4 mL) was heated to 80 °C for 24h. The reaction mixture was cooled, concentrated and the crude product was purified by preparative HPLC on a Waters X-Terra column using a 75-5% gradient of aqueous 0.2% ammonia in acetonitrile as eluent followed by a 75-5% gradient of aqueous 0.2% TFA in acetonitrile as eluent. The fractions containing the desired compound were evaporated to dryness to afford the title compound as a white solid (45 mg, 20%).
¹H NMR (300 MHz, DMSO-*d₆*) δ 9.62 (1H, s), 8.81 (1H, s), 8.59 (1H, d), 8.38 (1H, s), 8.34 (1H, dd), 7.78 - 7.62 (5H, m), 7.59 (1H, t), 7.46 (1H, dd), 7.34 (1H, d), 7.11 (2H, d), 4.93 - 4.80 (1H, m), 4.36 (2H, s ), 4.17 (1H, s), 3.53 (2H, s), 2.88 (6H, d), 2.84 - 2.75 (6H, m), 2.69 - 2.57 (2H, m), 2.53 - 2.39 (2H, m), 2.07 - 1.93 (2H, m), 1.79 - 1.58 (4H, m)
APCI (Multimode) m/z: 680 [M+H]

### Example 79

### 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide

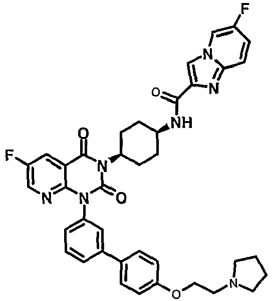

### Step (a) 1-{2-[4-(4,4,5,5-tetramethyl-1,3,2-dioxaborolan-2-yl)phenoxy]ethyl}pyrrolidine

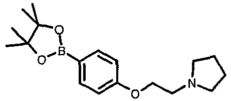

1,1'-bis(diphenylphosphino)ferrocenedichloro palladium(II) DCM complex (0.135 g, 0.19 mmol) and 1,1'-bis(diphenylphosphino)ferrocene (0.103 g, 0.19 mmol) in DMSO (5 mL) was stirred at ambient temperature for 15 mins. Potassium acetate (1.09 g, 11.1 mmol), bis(pinacolato)diboron (1.22 g, 4.81 mmol) and 1-(2-(4-bromophenoxy)ethyl)pyrrolidine (1 g, 3.70 mmol) were added and the reaction was heated at 80 °C for 20h. The reaction was cooled, water added and extracted with diethyl ether. The ether phases were dried (Na₂SO₄) and concentrated to give the sub-title compound as a brown oil (560 mg, 48%).
¹H NMR (300 MHz, CDCl₃) δ 7.75 (2H, d), 6.91 (2H, d), 4.19 (2H, t), 3.00 (2H, t), 2.78 - 2.70 (2H, m), 2.68 - 2.50 (2H, m), 1.89 - 1.81 (4H, m), 1.28 (12H, s).

### Step (b) 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide

Palladium(II) acetate (3.5 mg, 0.02 mmol) and 2-dicyclohexylphosphino-2',6'-dimethoxybiphenyl (13 mg, 0.03 mmol) were stirred together in acetonitrile (15 mL) for 10 min before the addition of a solution of potassium carbonate (0.13 g, 0.93 mmol) in Water (10 mL). 6-fluoro-*N*-{*cis*-4-[6-fluoro-1-(3-iodophenyl)-2,4-dioxo-1,4-dihydropyrido[2,3-d]pyrimidin-3(2*H*)-yl]cyclohexyl}imidazo[1,2-*a*]pyridine-2-carboxamide (0.2 g, 0.31 mmol) and 1-(2-(4-(4,4,5,5-tetramethyl-1,3,2-dioxaborolan-2-yl)phenoxy)ethyl)pyrrolidine (99 mg, 0.31 mmol) were then added sequentially. The resulting solution was stirred at 80°C for 16h. The reaction mixture was cooled, concentrated and the crude product was purified by preparative HPLC on a Waters X-Terra column using a 75-5% gradient of aqueous 0.2% ammonia in acetonitrile as eluent followed by a 75-5% gradient of aqueous 0.2% TFA in acetonitrile as eluent. The fractions containing the desired compound were evaporated to dryness to afford the title compound as a white solid (21 mg, 9%).
¹H NMR (300 MHz, DMSO-*d₆*) δ 9.94 - 9.70 (1H, m), 8.81 (1H, s), 8.59 (1H, d), 8.39 (1H, s), 8.34 (1H, dd), 7.79 - 7.62 (5H, m), 7.59 (1H, t), 7.46 (1H, dd), 7.34 (1H, d), 7.11 (2H, d), 4.88 (1H, t), 4.34 (2H, t), 4.20 - 4.12 (1H, m), 3.68 - 3.53 (4H, m), 3.21 - 3.06 (2H, m), 2.74 - 2.36 (2H, m), 2.08 - 1.94 (4H, m), 1.92 - 1.84 (2H, m), 1.80 - 1.58 (4H, m)
APCI (Multimode) m/z: 706 [M+H]

### Example 80

### 6-fluoro-N-{cis-4-[6-fluoro-1-(4'-fluoro-2'-hydroxybiphenyl-3-yl)-2,4-dioxo-1,4-dihydropyrido[2,3-d]pyrimidin-3(2H-yl]cyclohexyl}imidazo[1,2-a]pyridine-2-carboxamide

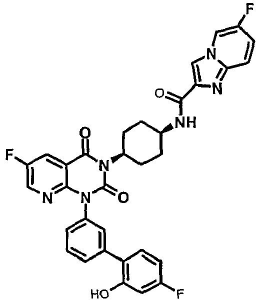

### Made according to Example 79 step (b)

¹H NMR (300 MHz, CDCl₃) δ 8.38 (1H, d), 8.25 (1H, dd), 8.11 (1H, s), 8.08 - 8.04 (1H, m), 7.73 - 7.64 (2H, m), 7.62 - 7.55 (2H, m), 7.45 (1H, t), 7.36 (1H, dd), 7.17 (1H, ddd), 6.75 - 6.66 (2H, m), 6.57 - 6.48 (1H, m), 5.08 (1H, t), 4.49 - 4.40 (1H, m), 2.88 - 2.65 (2H, m), 2.16 - 2.04 (2H, m), 1.93 - 1.66 (4H, m)
APCI (Multimode) m/z: 627 [M+H]

### Example 81

### 6-fluoro-N-{cis-4-[6-fluoro-1-[4'-(3-morpholin-4-ylpropoxy)biphenyl-3-yl]-2,4-dioxo-1,4-dihydropyrido[2,3-d]pyrimidin-3(2H)-yl]cyclohexyl}imidazo[1,2-a]pyridine-2-carboxamide

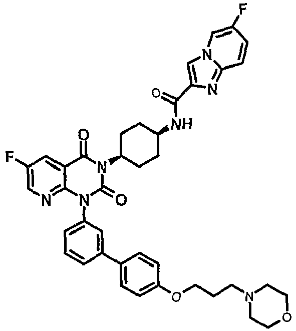

### Made according to Example 79_step (b)

¹H NMR (300 MHz, DMSO-*d₆*) δ 8.82 - 8.74 (1H, m), 8.59 (1H, d), 8.37 (1H, s), 8.32 (1H, dd), 7.78 - 7.64 (4H, m), 7.64 - 7.52 (2H, m), 7.44 (1H, t), 7.31 (1H, d), 7.02 (2H, d), 4.94 - 4.81 (1H, m), 4.21 - 4.12 (1H, m), 4.05 (2H, t), 3.57 (4H, t), 2.64 - 2.24 (8H, m), 2.00 (2H,
d), 1.88 (2H, t), 1.80 - 1.59 (4H, m)
APCI (Multimode) m/z: 736 [M+H]

### Example 82 and Example 83

### N-{cis-4-[1-{2'-[2-(dimethylamino)ethoxy]-4'-fluorobiphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide and N-cis-4-[1-{2'-[2-(dimethylamino)ethoxy]-4'-fluorobiphenyl-3-yl}-6-fluoro-2,4-dioxo-1,4-dihydropyrido[2,3-d]pyrimidin-3(2H)-yl]cyclohexyl}-N-[2-(dimethylamino)ethyl]-6-fluoroimidazo[1,2-a]pyridine-2-carboxamide

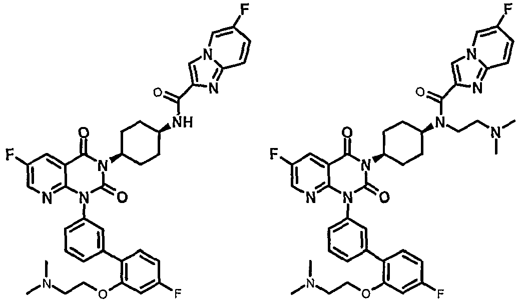

A solution of 6-fluoro-*N*-{*cis*-4-[6-fluoro-1-(4'-fluoro-2'-hydroxybiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.2 g, 0.32 mmol), cesium carbonate (0.208 g, 0.64 mmol) , potassium iodide (5.30 mg, 0.03 mmol) and 2-dimethylaminoethyl chloride hydrochloride (46 mg, 0.32 mmol) in NMP (3 mL) was heated to 80 °C for 24h. The reaction mixture was cooled, concentrated and the crude product was purified by preparative HPLC on a Waters X-Terra column using a 75-5% gradient of aqueous 0.2% ammonia in acetonitrile as eluent. The fractions containing the desired compound were evaporated to dryness to afford the title compounds:
*N*-{*cis*-4-[1-{2'-[2-(dimethylamino)ethoxy]-4'-fluorobiphenyl-3-yl]-6-fluoro-2,4-dioxo-1,4-dihydropyrido[2,3-*d*]pyrimidin-3(2*H*)-yl]cyclohexyl}-6-fluoroimidazo[1,2-*a*]pyridine-2-carboxamide (25 mg, 11%)
¹H NMR (300 MHz, DMSO-*d₆*) δ 8.79 (1H, s), 8.61 (1H, d), 8.37 (1H, s), 8.33 (1H, dd), 7.75 (1H, dd), 7.69 - 7.51 (4H, m), 7.49 - 7.30 (3H, m), 7.06 (1H, d), 6.91 - 6.83 (1H, m), 4.95 - 4.78 (1H, m), 4.16 (1H, s), 4.13 - 4.08 (2H, m), 3.38 - 3.22 (2H, m), 2.74 - 2.58 (2H, m), 2.13 (6H, s), 2.04 - 1.95 (2H, m), 1.79 - 1.58 (4H, m)
APCI (Multimode) m/z: 698 [M+H]

### N-{cis-4-[1-{2'-[2-(dimethylamino)ethoxy]-4'-fluorobiphenyl-3-yl]-6-fluoro-2,4-dioxo-1,4-dihydropyrido[2,3-d]pyrimidin-3(2H)-yl]cyclohexyl}-N-[2-(dimethylamino)ethyl]-6-fluoroimidazo[1,2-a]pyridine-2-carboxamide (29 mg, 12%)

¹H NMR (300 MHz, DMSO-*d₆*) δ 8.84 - 8.78 (1H, m), 8.60 (1H, d), 8.39 (1H, s), 8.36 (1H, dd), 7.77 (1H, dd), 7.70 (1H, d), 7.60 - 7.53 (2H, m), 7.52 - 7.34 (4H, m), 7.15 - 7.07 (1H, m), 7.01 - 6.93 (1H, m), 4.91 - 4.76 (1H, m), 4.60 - 4.40 (2H, m), 4.23 - 4.11 (1H, m), 3.81 (2H, s), 3.65 - 3.42 (4H, m), 3.05 (6H, s), 2.76 - 2.58 (2H, m), 2.11 - 1.93 (2H, m), 1.79 - 1.61 (4H, m)
APCI (Multimode) m/z: 769 [M+H]

### Example 84

### N-{cis-4-[1-{2'-[3-(dimethylamino)propoxy]-4'-fluorobiphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

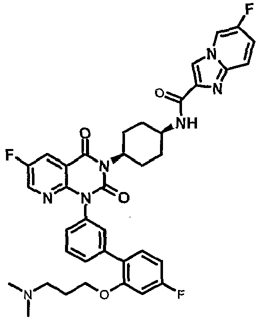

### Made according to Example 82

¹H NMR (300 MHz, DMSO-*d₆*) δ 8.81 (1H, s), 8.61 (1H, s), 8.38 (1H, s), 8.37 - 8.30 (1H, m), 7.81 - 7.72 (1H, m), 7.71 - 7.64 (1H, m), 7.63 - 7.55 (1H, m), 7.54 - 7.43 (2H, m), 7.40 - 7.28 (2H, m), 7.06 (1H, d), 6.95 - 6.85 (1H, m), 4.92 - 4.76 (1H, m), 4.22 - 4.08 (1H, m), 4.09 (2H, s), 3.06 (2H, s), 2.69 (6H, s), 2.53 - 2.28 (2H, m), 2.09 - 1.95 (4H, m), 1.78 - 1.56 (4H, m)
APCI (Multimode) m/z: 712 [M+H]

### Example 85

### 6-fluoro-N-{cis-4-[6-fluoro-1-[4'-fluoro-2'-(2-piperazin-1-ylethoxy)biphenyl-3-yl]-2,4-dioxo-1,4-dihydropyrido[2,3-d]pyrimidin-3(2H)-yl]cyclohexyl}imidazo[1,2-a]pyridine-2-carboxamide

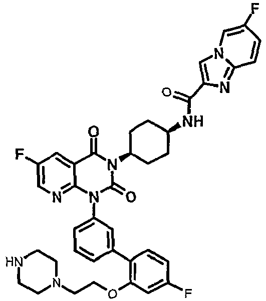

### Made according to Example 82

¹H NMR (300MHz, DMSO-*d₆*) δ 8.84 (1H, s), 8.79 - 8.68 (1H, m), 8.61 (1H, d), 8.39 (1H, s), 8.36 - 8.31 (1H, m), 7.77 (1H, dd), 7.70 (1H, d), 7.64 - 7.44 (5H, m), 7.41 - 7.32 (2H, m), 7.09 (1H, d), 6.94 - 6.86 (1H, m), 4.92 - 4.78 (1H, m), 4.66 - 4.22 (3H, m), 4.20 (4H, s), 3.09 (4H, s), 2.98 (1H, s), 2.81 (2H, s), 2.64 - 2.54 (2H, m), 2.06 - 1.96 (2H, m), 1.78 - 1.58 (4H, m)
APCI (Multimode) m/z: 739 [M+H]

### Example 86

### 1-biphenyl-3-yl-6-fluoro-3-{cis-4-[(2-hydroxyethyl)amino]cyclohexyl}pyrido[2,3-d]pyrimidine-2,4(1H,3H)-dione

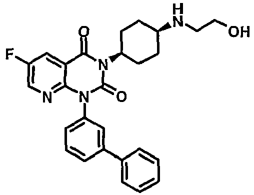

A solution of 3-(*cis*-4-aminocyclohexyl)-1-biphenyl-3-yl-6-fluoropyrido[2,3-*d*]pyrimidine-2,4(1*H*,3*H*)-dione (77 mg, 0.18 mmol) and [[(1,1-dimethylethyl)dimethylsilyl]oxy]acetaldehyde (34.3 mg, 0.20 mmol) in DCM (3 mL) was added sodium triacetoxyborohydride (41.7 mg, 0.20 mmol) and the reaction was stirred at 80 °C for 22 h. The mixture was quenched with methanol and dilute HCl (1 mL) stirred for 2 h and the crude product was purified by preparative HPLC on a Phenomenex Gemini column using a 75-5% gradient of aqueous 0.2% ammonia in acetonitrile as eluent. The fractions containing the desired compound were evaporated to dryness to afford the title compound (42 mg, 50 %) as a white solid.
¹H NMR (300MHz, CDCl₃) δ 8.41 (1H, d), 8.21 (1H, dd), 7.74 (1H, d), 7.67 - 7.58 (3H, m), 7.56 - 7.51 (1H, m), 7.46 (2H, t), 7.41 - 7.34 (1H, m), 7.30 (1H, d), 5.29 - 5.22 (1H, m), 5.08 - 4.93 (1H, m), 3.98 - 3.89 (2H, m), 3.40 (1H, s), 3.17 - 3.08 (2H, m), 2.81 - 2.63 (2H, m), 2.48 (2H, d), 1.91 - 1.73 (4H, m)
APCI (Multimode) m/z: 475 [M+H]

### Example 87

### {3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl}-N,N,N-trimethylmethanaminium iodide

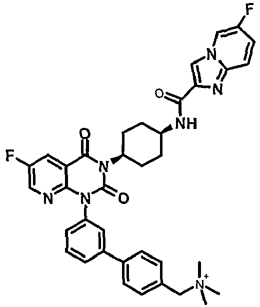

A solution of *N*-{*cis*-4-[1-{4'-[(dimethylamino)methyl]biphenyl-3-yl}-6-fluoro-2,4-dioxo-1,4-dihydropyrido[2,3-d]pyrimidin-3(2*H*)-yl]cyclohexyl}-6-fluoroimidazo[1,2-*a*]pyridine-2-carboxamide (75 mg, 0.12 mmol) and iodomethane (0.014 mL, 0.23 mmol) were dissolved in acetonitrile (3 mL) and the solution was stirred at 80 °C for 24h. The reaction was concentrated and was purified by preparative HPLC on a Phenomenex Gemini colum using a 75-5% gradient of aqueous 0.2% ammonia in acetonitrile as eluent. The fractions containing the desired compound were evaporated to dryness to afford the title compound as a white solid (26 mg, 32%).
¹H NMR (300 MHz, CDCl₃) δ 8.37 (1H, d), 8.24 (1H, dd), 8.10 (2H, s), 8.08 - 8.03 (2H, m), 7.75 - 7.66 (5H, m), 7.58 - 7.51 (2H, m), 7.39 - 7.33 (1H, m), 7.19 - 7.11 (1H, m), 5.11 - 5.00 (1H, m), 4.97 (2H, s), 4.41 (1H, s), 3.35 (9H, s), 2.86 - 2.69 (2H, m), 2.13 (2H, d), 1.90 - 1.52 (4H, m)
APCI (Multimode) m/z: 664 [M+H]

### Example 88

### N-{cis-A-[1-{2'-[(dimethylamino)methyl]biphenyl-3-yl}-6-fluoro-2,4-dioxo-1,4-dihydropyrido[2,3-d]pyrimidin-3(2H)-yl]cyclohexyl}-1-methyl-D-prolinamide

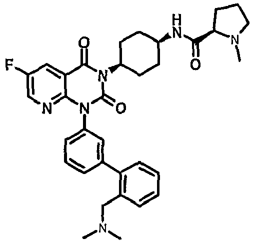

### Step (a) N-{cis-4-[1-{2'-[(3-iodophenyl}-6-fluoro-2,4-dioxo-1,4-dihydropyrido[2,3-d]pyrimidin-3(2H)-yl]cyclohexyl}-1-methyl-D-prolinamide

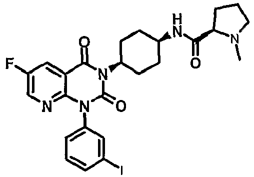

HATU (0.243 g, 0.64 mmol) was added to 1-methyl-pyrrolidine-2-carboxylic acid (82 mg, 0.64 mmol) and Hunig's Base (0.41 mL, 2.3 mmol) in NMP (4 mL). Stirred for 10 mins and 3-(cis-4-aminocyclohexyl)-6-fluoro-1-(3-iodophenyl)pyrido[2,3-d]pyrimidine-2,4(1*H*,3*H*)-dione hydrochloride (0.3 g, 0.58 mmol) was added. Stirred at r.t. for 4h. The reaction was poured onto water and the solid was filtered off, washed with water and dried *in vacuo* to leave the sub-title compound as a tan solid (0.2 g, 58%).
APCI (Multimode) m/z: 592 [M+H]

### Step (b) N-{cis-4-[1-{2'-[(dimethylamino)methyl]biphenyl-3-yl}-6-fluoro-2,4-dioxo-1,4-dihydropyrido[2,3-d]pyrimidin-3(2H)-yl]cyclohexyl}-1-methyl-D-prolinamide

Palladium(II) acetate (3 mg, 0.015 mmol) and 2-dicyclohexylphosphino-2',6'-dimethoxybiphenyl (10 mg, 0.03 mmol) were stirred together in acetonitrile (5 mL) for 10 min before the addition of a solution of potassium carbonate (105 mg, 0.76 mmol) in water (3 mL), *N-*{*cis*-4-[6-fluoro-1-(3-iodophenyl)-2,4-dioxo-1,4-dihydropyrido[2,3-*d*]pyrimidin-3(2*H*)-yl]cyclohexyl}-1-methyl-D-prolinamide (150 mg, 0.25 mmol) and 2-(N,N-dimethylaminomethyl)phenylboronic acid (45 mg, 0.25 mmol) were then added sequentially. The resulting solution was stirred at 80°C for 16h. The reaction mixture was cooled, concentrated and the crude product was purified by preparative HPLC on a Waters X-Terra column using a 75-5% gradient of aqueous 0.2% ammonia in acetonitrile as eluent followed by a 75-5% gradient of aqueous 0.2% TFA in acetonitrile as eluent. The fractions containing the desired compound were evaporated to dryness to afford the title compound as a white solid (2 mg, 2%).
¹H NMR (300MHz, DMSO-*d₆*) δ 8.69 (1H, d), 8.59 (1H, s), 8.30 (1H, dd), 7.89 (1H, d), 7.65 (1H, d), 7.58 - 7.50 (1H, m), 7.50 - 6.98 (4H, m), 4.86 - 4.70 (1H, m), 4.28 (2H, s), 4.17 - 4.05 (1H, m), 3.96 - 3.83 (1H, m), 3.57 (3H, s), 2.82 (6H, d), 2.65 - 2.53 (2H, m), 2.37 - 2.26 (2H, m), 2.14 - 1.81 (6H, m), 1.72 - 1.49 (4H, m)
APCI (Multimode) m/z: 599 [M+H]

### Example 89

### N-{cis-4-[1-{4'-[(dimethylamino)methyl]biphenyl-3-yl}-6-fluoro-2,4-dioxo-1,4-dihydropyrido[2,3-d]pyrimidin-3(2H)-yl]cyclohexyl}acetamide

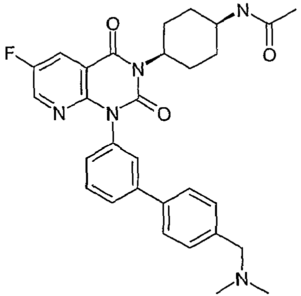

### Step (a) tert-butyl {cis-4-[1-{4'-[(dimethylamino)methyl]biphenyl-3-yl}-6-fluoro-2,4-dioxo-1,4-dihydropyrido[2,3-d]pyrimidin-3(2H)-yl]cyclohexyl}carbamate

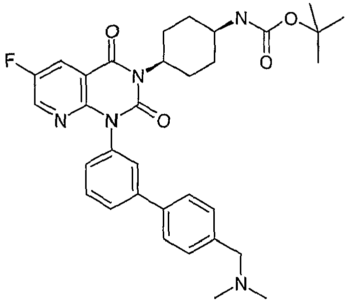

Acetonitrile (10 mL) was added to a mixture of palladium (II) acetate (0.025 g, 0.11 mmol) and 2-dicyclohexylphosphino-2',6'-dimethoxybiphenyl (0.092 g, 0.22 mmol) and the mixture was stirred at room temperature for 10 min. Potassium carbonate (0.929 g, 6.72 mmol) in water (3 mL) was added followed by *N,N*-dimethyl-1-[4-(4,4,5,5-tetramethyl-1,3,2-dioxaborolan-2-yl)phenyl]methanamine (1.00 g, 3.36 mmol) and *tert*-butyl {*cis-*4-[6-fluoro-1-(3-iodophenyl)-2,4-dioxo-1,4-dihydropyrido[2,3-*d*]pyrimidin-3(2H)-yl]cyclohexyl}carbamate (1.3 g, 2.24 mmol) and the reaction mixture was refluxed overnight at 80°C. The reaction mixture was loaded directly onto a Varian Bond Chemelut cartridge (available from Kinesis), the product eluted with DCM and concentrated in vacuo. The crude product was purified by flash silica chromatography, eluting with gradient 1 to 5 % methanol in dichloromethane with 0.1 % 5N methanolic ammonia. Pure fractions were evaporated to dryness to afford the sub-title compound as a pale yellow solid (1.32 g, 102 % - contains some solvents).
¹H NMR (400 MHz, DMSO-*d₆*) δ 8.58 (1H, d), 8.29 (1H, dd), 7.77 (1H, d), 7.71 (1H, d), 7.64 - 7.57 (3H, m), 7.41 - 7.34 (3H, m), 6.54 (1H, s), 4.75 (1H, t), 3.91 (2H, s), 3.59 (1H, s), 2.65 - 2.55 (2H, m), 2.17 (6H, s), 1.94 - 1.86 (2H, m), 1.54 - 1.44 (4H, m), 1.38 (9H, s) APCI (Multimode) m/z: 588 [M+H]

### Step (b) 3-(cis-4-aminocyclohexyl)-1-{4'-[(dimethylamino)methyl]biphenyl-3-yl}-6-fluoropyrido[2,3-d]pyrimidine-2,4(1H,3H)-dione dihydrochloride

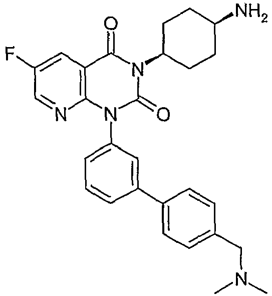

To a solution of *tert*-butyl {*cis*-4-[1-{4'-[(dimethylamino)methyl]biphenyl-3-yl}-6-fluoro-2,4-dioxo-1,4-dihydropyrido[2,3-d]pyrimidin-3(2*H*)-yl]cyclohexyl}carbamate (1.32 g, 2.25 mmol) in 1,4-Dioxane (6 mL) was added hydrogen chloride solution in 1,4-dioxane (4M) (4 mL, 16.00 mmol) and the reaction mixture was stirred at room temperature for 3h. Hydrogen chloride solution in 1,4-dioxane (4M) (2 mL, 8.00 mmol) was added and the reaction mixture stirred at room temperature for a further 2h. Hydrogen chloride solution in 1,4-dioxane (4M) (4 mL, 16.00 mmol) was added and the reaction mixture stirred at room temperature overnight. Diethyl ether (25 mL) was added and the mixture stirred for 1h. The solid was collected by filtration and washed with ether to give the sub-title compound as a white solid (1.17 g, 93 %).
¹H NMR (400 MHz, DMSO-*d₆*) δ 10.96 (1H, s), 8.61 (1H, d), 8.31 (1H, dd), 8.03 - 7.97 (3H, m), 7.83 (1H, d), 7.79 - 7.77 (2H, m), 7.75 (1H, s), 7.68 (1H, d), 7.64 (2H, t), 7.43 - 7.40 (1H, m), 4.83 - 4.72 (1H, m), 4.31 (2H, d), 3.45 - 3.37 (1H, m), 2.76 (6H, s), 2.62 - 2.54 (2H, m), 2.00 - 1.91 (2H, m), 1.84 - 1.71 (2H, m), 1.70 - 1.60 (2H, m)
APCI (Multimode) m/z: 488 [M+H]

### Step (c) N-{cis-4-[1-{4'-[(dimethylamino)methyl]biphenyl-3-yl}-6-fluoro-2,4-dioxo-1,4-dihydropyrido[2,3-d]pyrimidin-3(2H)-yl]cyclohexyl}acetamide

To a solution of 3-(*cis*-4-aminocyclohexyl)-1-{4'-[(dimethylamino)methyl]biphenyl-3-yl}-6-fluoropyrido[2,3-*d*]pyrimidine-2,4(1*H*,3*H*)-dione dihydrochloride (0.2 g, 0.36 mmol) in dichloromethane (10 mL) was added N,N-diisopropylethylamine (0.5 mL, 2.86 mmol) followed by acetyl chloride (0.025 mL, 0.36 mmol) and the reaction mixture was stirred at room temperature overnight. The solvent was evaporated and the residue triturated with water (10 mL) and stirred overnight to give a solid which was collected by filtration and purified by flash silica chromatography, elution gradient 20 % methanol in dichloromethane. Pure fractions were evaporated to dryness to afford the title compound as a white solid (0.045 g, 24 %).
¹H NMR (400 MHz, DMSO-*d₆*) δ 8.51 (d, 1H), 8.23 (dd, 1H), 7.73 (d, 1H), 7.70 - 7.65 (m, 2H), 7.60 - 7.54 (m, 3H), 7.36 - 7.28 (m, 3H), 4.78 - 4.67 (m, 1H), 3.77 - 3.72 (m, 1H), 3.39 (s, 2H), 2.59 - 2.52 (m, 2H), 2.12 (s, 6H), 1.86 - 1.83 (m, 2H), 1.82 (s, 3H), 1.53 - 1.45 (m, 4H)
APCI (Multimode) m/z: 530 [M+H]

### Example 90

### N-{cis-4-[1-(3-{[benzyl(methyl)amino]carbonyl}phenyl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

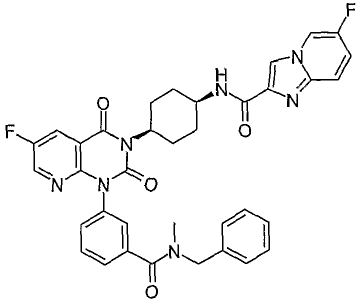

### Step (a) 3-[3-{cis-4-[(tert-butoxycarbonyl)amino]cyclohexyl}-6-fluoro-2,4-dioxo-3,4-dihydropyrido[2,3-d]pyrimidin-1(2H)-yl]benzoic acid

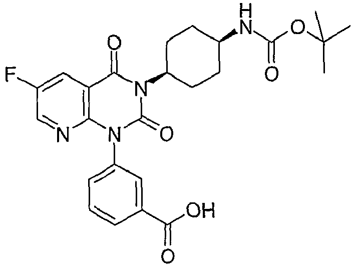

To a solution of methyl 3-[3-{*cis*-4-[(*tert*-butoxycarbonyl)amino]cyclohexyl}-6-fluoro-2,4-dioxo-3,4-dihydropyrido[2,3-*d*]pyrimidin-1(2*H*)-yl]benzoate (0.7 g, 1.37 mmol) in 1,4-dioxane (10 mL) was added in total lithium hydroxide (0.046 g, 1.92 mmol) in water (10.5 mL) over 3 days. The reaction mixture was quenched with acetic acid and the solvents evaporated. Water was added to the residue and the solid filtered, and dried in the oven to give the sub-title compound as a pale yellow solid. (0.322 g, 47 %)
¹H NMR (400 MHz, DMSO-*d₆*) δ 8.56 (1H, d), 8.28 (1H, dd), 8.02 (1H, dt), 7.96 (1H, d), 7.65 - 7.60 (2H, m), 6.56 - 6.43 (1H, m), 4.81 - 4.67 (1H, m), 2.63 - 2.54 (2H, m), 1.93 - 1.85 (2H, m), 1.54 - 1.43 (4H, m), 1.41 (9H, s)
APCI (Multimode) m/z: 460 [M+H]

### Step (b) tert-butyl {cis-4-[1-(3-{[benzyl(methyl)amino]carbonyl}phenyl)-6-fluoro-2,4-dioxo-1,4-dihydropyrido[2,3-d]pyrimidin-3(2H)-yl]cyclohexyl}carbamate

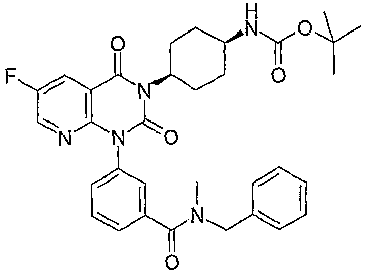

N,N-diisopropylethylamine (0.1167 g, 0.1572 mL, 0.90 mmoles) and HATU (0.1373 g, 0.14 mmol) were added to a solution of 3-[3-{*cis*-4-[(*tert-*butoxycarbonyl)amino]cyclohexyl}-6-fluoro-2,4-dioxo-3,4-dihydropyrido[2,3-*d*]pyrimidin-1(2*H*)-yl]benzoic acid (0.15 g, 0.30 mmoles) in DMF (3 mL). The mixture was stirred at room temperature for 10 min. N-benzylmethylamine (0.0401 g, 0.0427 mL, 0.33 mmoles) was added and the reaction mixture was stirred at room temperature overnight. The solvent was removed and the residue partitioned between ethyl acetate and water, the organics were combined, dried with anhydrous magnesium sulphate, filtered and concentrated in vacuo. The residue was purified by flash column chromatography eluting with 30 % ethyl acetate: 70 % isohexane increasing to 50 % ethyl acetate: 50 % isohexane to elute product to give the sub-title compound. (0.07 g, 39 %).
¹H NMR (400 MHz, DMSO-*d₆*) δ 8.59 - 8.42 (1H, m), 8.27 (1H, dd), 7.62 - 7.46 (3H, m), 7.38 - 7.26 (5H, m), 7.20 (1H, d), 6.59 (1H, s), 4.70 (2H, d), 3.55 (1H, s), 2.88 (3H, d), 2.64 - 2.55 (2H, m), 1.89 (2H, d), 1.54 - 1.44 (4H, m), 1.39 (9H, s)
APCI (Multimode) m/z: 602 [M+H]

### Step (c) 3-[3-(cis-4-aminocyclohexyl)-6-fluoro-2,4-dioxo-3,4-dihydropyrido[2,3-d]pyrimidin-1(2H)-yl]-N-benzyl-N-methylbenzamide hydrochloride

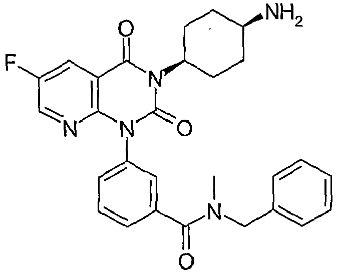

To a solution of *tert*-butyl {*cis*-4-[1-(3-{[benzyl(methyl)amino]carbonyl}phenyl)-6-fluoro-2,4-dioxo-1,4-dihydropyrido[2,3-*d*]pyrimidin-3(2*H*)-yl]cyclohexyl}carbamate (0.07 g, 0.116 mmoles) in 1,4-dioxane (1 mL) was added hydrogen chloride, 4.0M in 1,4-dioxane (1 mL, 4 mmoles) and the reaction mixture was stirred overnight at room temperature. The reaction mixture was concentrated in vacuo to give the sub-title compound as the hydrochloride salt as an off white foam. (0.06 g, 96 %)
APCI (Multimode) m/z: 539 [M+H]

### Step (d) N-{cis-4-[1-(3-{[benzyl(methyl)amino]carbonyl}phenyl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

To a solution of 6-fluoro-imidazo[1,2-a]pyridine-2-carboxylic acid (0.027 g, 0.123 mmoles) in DMF (2 mL) was added N,N-diisopropylethylamine (0.072 g, 0.097 mL, 0.558 mmoles) and HATU (0.051 g, 0.134 mmoles) and the mixture was stirred at room temperature for 10 min. This was followed by the addition of 3-[3-(4-amino-cyclohexyl)-6-fluoro-2,4-dioxo-3,4-dihydro-2H-pyrido[2,3-d]pyrimidin-1-yl]-N-benzyl-N-methyl-benzamide (0.06 g, 0.112 mmoles) and the reaction mixture was left to stir at room temperature overnight. The solvent was partially removed and water was added to the residue. The resulting solid was filtered. Water (0.5 mL) was added and the suspension heated with a heat gun and allowed to cool to room temperature. The solid was filtered, washed with cold water and dried in the oven to give the title compound as a white solid. (0.050 g, 67 %).
¹H NMR (400 MHz, DMSO-*d₆*) δ 8.79 (1H, dd), 8.57 (1H, s), 8.38 (1H, s), 8.31 (2H, dd), 7.76 - 7.65 (3H, m), 7.62 - 7.40 (7H, m), 7.38 - 7.28 (5H, m), 7.21 - 7.15 (2H, m), 4.84 (1H, t), 4.71 (1H, s), 4.22 (0H, s), 2.95 (0H, s), 2.00 (3H, d), 1.81 - 1.50 (6H, m), 2.65 - 2.54 (76H, m)
APCI (Multimode) m/z: 664 [M+H]

### Example 91

### 3-[3-(cis-4-aminocyclohexyl)-6-fluoro-2,4-dioxo-3,4-dihydropyrido[2,3-d]pyrimidin-1(2H)-yl]benzoic acid

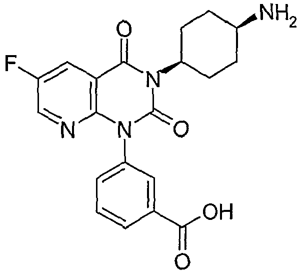

To a solution of 3-[3-{*cis*-4-[(*tert*-butoxycarbonyl)amino]cyclohexyl}-6-fluoro-2,4-dioxo-3,4-dihydropyrido[2,3-*d*]pyrimidin-1(2*H*)-yl]benzoic acid (0.07 g, 0.14 mmoles) in 1,4-dioxane (0.5 mL) was added hydrogen chloride, 4.0 M in 1,4-dioxane (0.5 mL, 3.2 mmoles) and the reaction mixture was stirred at room temperature overnight. Further hydrogen chloride, 4.0 M in 1,4-dioxane (0.3 mL, 1.92 mmoles) was added and the reaction mixture was stirred for another 4h at room temperature. The solid was filtered and washed with diethyl ether. The residue was purified by reverse phase HPLC (25-95 % acetonitrile in aqueous ammonia) and lyophilised to give the title compound as a white solid (0.02 g, 36 %).
¹H NMR (400 MHz, DMSO-d₆) δ 13.20 (1H, s), 8.58 (1H, d), 8.30 (1H, dd), 8.05 - 8.00 (1H, m), 7.98 - 7.97 (1H, m), 7.82 (2H, s), 7.67 - 7.64 (2H, m), 4.79 (1H, t), 3.46 (1H, s), 2.61 - 2.51 (2H, m), 1.95 - 1.77 (4H, m), 1.65 (2H, d).
APCI (Multimode) m/z: 399 [M+H]

### Example 92

### N-{cis-4-[1-{3-[(benzylamino)carbonyl]phenyl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

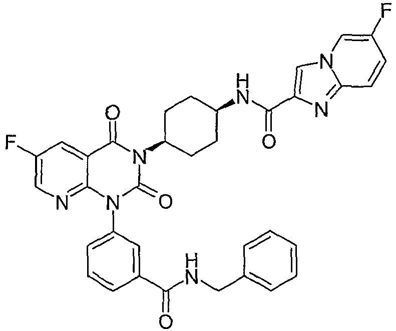

### Step (a) methyl 3-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]benzoate

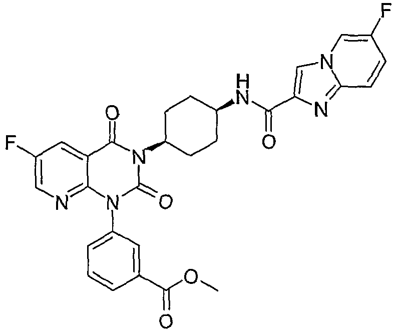

To a solution of 6-fluoro-imidazo[1,2-a]pyridine-2-carboxylic acid (0.76 g, 4.22 mmoles) in DMF (20 mL) was added N,N-diisopropylethylamine (1.636 g, 2.205 mL, 12.66 mmoles) and HATU (1.925 g, 5.06 mmoles) and the reaction mixture was stirred at room temperature for 10min. To this was added methyl 3-[3-(cis-4-aminocyclohexyl)-6-fluoro-2,4-dioxo-3,4-dihydropyrido[2,3-d]pyrimidin-1(2H)-yl]benzoate (1.74 g, 4.22 mmoles) in DMF (10 mL) and the reaction mixture was stirred at room temperature for 2h. The solvents were evaporated and the residue was partitioned between water and ethyl acetate. The combined organic phase was dried with anhydrous magnesium sulfate, filtered and concentrated in vacuo to give the sub-title compound as a pale brown solid (2.43 g, 100 %).
¹H NMR (400 MHz, DMSO-*d₆*) δ 8.82 - 8.75 (1H, m), 8.56 (1H, d), 8.37 (1H, s), 8.33 (1H, dd), 8.07 - 8.01 (2H, m), 7.79 - 7.67 (1H, m), 7.52 (2H, dd), 7.45 (2H, dd), 4.86 (1H, t), 4.16 (1H, s), 3.88 (3H, s), 1.72 - 1.62 (4H, m), 1.28 - 1.24 (4H, m).
APCI (Multimode) m/z: 575 [M+H]

### Step (b) 3-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]benzoic acid

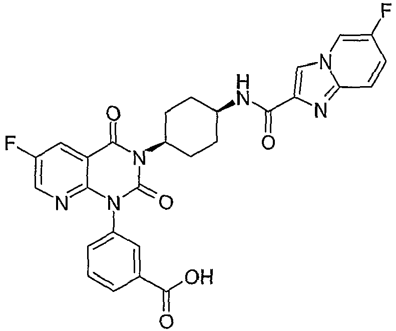

To a solution of methyl 3-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]benzoate (2.4 g, 4.18 mmoles) in 1,4-dioxane (100 mL) was added a solution of lithium hydroxide (0.0901 g, 3.76 mmoles) in water (50 mL) and the reaction mixture was stirred at room temperature for 1 h. The mixture was acidified with acetic acid and then concentrated in vacuo. To the residue was added 4M HCl in dioxane (300mL) and the reaction mixture was heated at 70 °C for 12h, allowed to cool and stand over the weekend. The solvents were removed by evaporation and the residue was purified by reverse phase HPLC (25-95 % acetonitrile in aqueous ammonia) to give the sub-title compound (0.280 g, 12 %).
¹H NMR (400 MHz, DMSO-d₆) δ 8.87 (1H, dd), 8.57 (1H, d), 8.45 (1H, s), 8.32 (1H, dd), 8.05 - 8.01 (1H, m), 7.98 (1H, t), 7.83 - 7.75 (2H, m), 7.65 (2H, td), 7.56 - 7.50 (1H, m), 4.94 - 4.77 (1H, m), 4.21 - 4.10 (1H, m), 2.70 - 2.55 (2H, m), 2.02 (2H, d), 1.77 - 1.59 (4H, m).
APCI (Multimode) m/z: 559 [M+H]

### Step (c) N-{cis-4-[1-{3-[(benzylamino)carbonyl]phenyl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

N,N-diisopropylethylamine (0.1729 g, 0.2331 mL, 1.338 mmoles) and HATU (0.2035 g, 0.535 mmoles) were added to a solution of 3-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]benzoic acid (0.25 g, 0.446 mmoles) in DMF (3 mL). The mixture was stirred at room temperature for 10mm. Benzylamine (0.053 g, 0.054 mL, 0.491 mmoles) was added and the reaction mixture was stirred overnight at room temperature. The solvent was removed and the residue partitioned between ethyl acetate and water, the combined organic phases were dried with anhydrous magnesium sulphate, filtered and concentrated in vacuo. The residue was purified by reverse phase HPLC (25-95 % acetonitrile in aqueous ammonia), and lyophilised to give the title compound as a white solid. (0.174 g, 60 %).
¹H NMR (400 MHz, DMSO-*d₆*) δ 9.12 (1H, t), 8.79 (1H, dd), 8.56 (1H, d), 8.37 (1H, s), 8.32 (1H, dd), 8.00 (1H, d), 7.90 (1H, d), 7.75 (1H, dd), 7.69 - 7.61 (2H, m), 7.56 (1H, d), 7.45 (1H, ddd), 7.32 (4H, d), 7.26 - 7.21 (1H, m), 4.90 - 4.82 (1H, m), 4.49 (2H, d), 4.19 - 4.13 (1H, m), 2.65 - 2.54 (2H, m), 2.00 (2H, d), 1.79 - 1.60 (4H, m)
APCI (Multimode) m/z: 650 [M+H]

### Example 93

### N-[cis-4-(1-biphenyl-3-yl-6-fluoro-2,4-dioxo-1,4-dihydropyrido[2,3-d]pyrimidin-3(2H)-yl)cyclohexyl]-4-(dimethylamino)butanamide

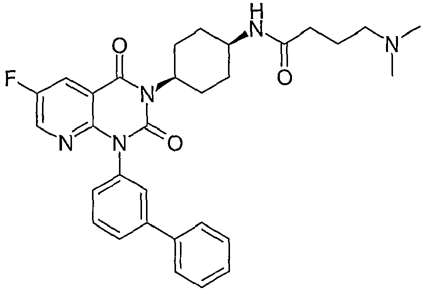

To a solution of 4-dimethylamino-butyric acid (0.0337 g, 0.257 mmoles) in DMF (2 mL) was added N,N-diisopropylethylamine (0.1107 g, 0.1492 mL, 0.857 mmoles) and HATU (0.098 g, 0.257 mmoles). The reaction mixture was stirred at room temperature for 10 min. 3-(*cis*-4-Aminocyclohexyl)-1-biphenyl-3-yl-6-fluoropyrido[2,3-*d*]pyrimidine-2,4(1*H*,3*H*)-dione (0.1 g, 0.214 mmoles) was added and the reaction mixture was stirred at room temperature overnight. In a separate vessel, N,N-diisopropylethylamine (0.055 g, 0.075 mL, 0.426 mmoles) and HATU (0.049 g, 0.129 mmoles) was added to a solution of 4-dimethylamino-butyric acid (0.013 g, 0.099 mmoles) in DMF (1 mL) and the mixture stirred at room temperature for 10 min. This solution was then added to the original reaction and the mixture was stirred at room temperature for a further 1h. The reaction mixture was concentrated in vacuo and the residue stirred in the water. The solid was removed by filtration and purified by flash column chromatography eluting with 5 % 7N methanolic ammonia in 95 % DCM to give the title compound as a white solid (0.055 g, 47 %).
¹H NMR (400 MHz, DMSO-*d₆*) δ 8.59 (1H, d), 8.29 (1H, dd), 7.79 - 7.76 (1H, m), 7.73 (1H, t), 7.70 - 7.65 (3H, m), 7.61 (1H, t), 7.48 (2H, t), 7.41 - 7.36 (2H, m), 4.79 (1H, t), 3.79 (1H, s), 2.67 - 2.55 (4H, m), 2.45 (6H, s), 2.20 (2H, t), 1.91 (2H, d), 1.73 (2H, quintet), 1.58 - 1.47 (4H, m).
APCI (Multimode) m/z: 544 [M+H]

### Example 94

### N-{cis-4-[1-{4'-[(dimethylamino)methyl]-2'-hydroxybiphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

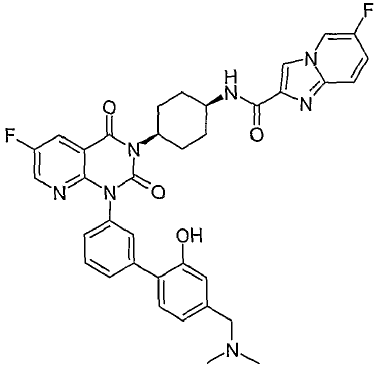

### Step (a) 6-fluoro-N-{cis-4-[6-fluoro-2,4-dioxo-1-[3-(4,4,5,5-tetramethyl-1,3,2-dioxaborolan-2-yl)phenyl]-1,4-dihydropyrido[2,3-d]pyrimidin-3(2H)-yl]cyclohexyl}imidazo[1,2-a]pyridine-2-carboxamide

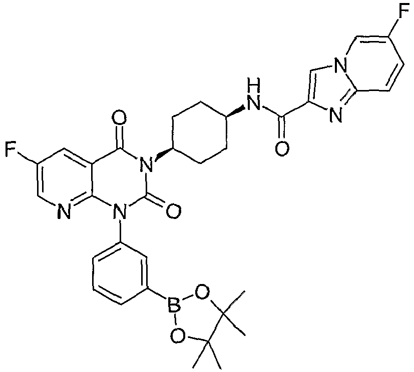

1,1'-Bis(diphenylphosphino)ferrocenedichloro palladium(II) dichloromethane complex (0.285 g, 0.39 mmol) and 1,1'-bis(diphenylphosphino)ferrocene (0.216 g, 0.39 mmol) were stirred at room temperature in dimethylsulfoxide (25 mL) for 10 min. 6-Fluoro-*N*-{*cis*-4-[6-fluoro-1-(3-iodophenyl)-2,4-dioxo-1,4-dihydropyrido[2,3-*d*]pyrimidin-3(2*H*)-yl]cyclohexyllimidazo[1,2-*a*]pyridine-2-carboxamide (5 g, 7.78 mmol), bis(pinacolato)diboron (2.57 g, 10.12 mmol) and potassium acetate (2.292 g, 23.35 mmol) were added and the reaction was heated at 80 °C overnight. After cooling, water was added and the mixture stirred for 3h before the solid was filtered. The residue was purified by flash silica chromatography, elution gradient 100 % ethyl acetate to afford the sub-title compound as a brown gum (4.21 g, 84 %).
¹H NMR (400 MHz, DMSO-d₆) δ 8.79 (ddd, 1H), 8.55 (d, 1H), 8.37 (s, 1H), 8.30 (dd, 1H), 7.78 - 7.73 (m, 2H), 7.69 - 7.65 (m, 2H), 7.57 - 7.49 (m, 2H), 7.44 (ddd, 1H), 4.85 (t, 1H), 4.22 (s, 1H), 2.64 - 2.55 (m, 2H), 2.03 - 2.00 (m, 2H), 1.77 - 1.63 (m, 4H), 1.33 (s, 12H) APCI (Multimode) m/z: 643 [M+H]

### Step (b) 6-fluoro-N-{cis-4-[6-fluoro-1-(4'-formyl-2'-hydroxybiphenyl-3-yl)-2,4-dioxo-1,4-dihydropyrido[2,3-d]pyrimidin-3(2H)-yl]cyclohexyl}imidazo[1,2-a]pyridine-2-carboxamide

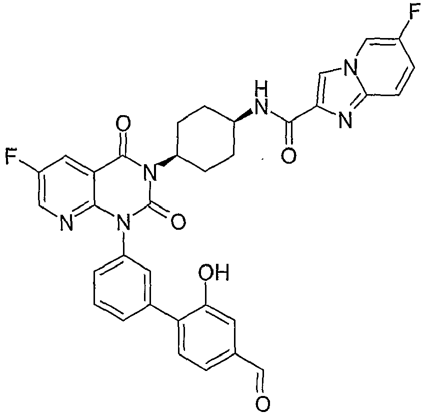

Acetonitrile (30 mL) was added to a mixture of palladium (II) acetate (0.105 g, 0.47 mmol) and 2-dicyclohexylphosphino-2',6'-dimethoxybiphenyl (0.383 g, 0.93 mmol) and the mixture was stirred at room temperature for 10 min. To this was added sequentially potassium carbonate (1.936 g, 14.01 mmol) in water (15 mL), 6-fluoro-*N*-{*cis*-4-[6-fluoro-2,4-dioxo-1-[3-(4,4,5,5-tetramethyl-1,3,2-dioxaborolan-2-yl)phenyl]-1,4-dihydropyrido[2,3-d]pyrimidin-3(2*H*)-yl]cyclohexyl}imidazo[1,2-*a*]pyridine-2-carboxamide (3 g, 4.67 mmol) and 3-hydroxy-4-iodobenzaldehyde (1.737 g, 7.00 mmol). The reaction mixture was heated to 80 °C overnight. The reaction mixture was directly added to onto a Varian Bond Chemelut cartridge (available from Kinesis), the product eluted with DCM and concentrated in vacuo. The residue was purified by flash silica chromatography, eluting with 30 % ethyl acetate in 70 % dichloromethane to afford as a yellow solid (0.282 g, 10 %).
¹H NMR (400 MHz, DMSO-*d₆*) δ 10.50 (s, 1H), 10.03 (s, 1H), 8.57 (d, 1H), 8.32 (dd, 1H), 7.96 (dd, 1H), 7.85 (dd, 2H), 7.63 (d, 1H), 7.54 - 7.45 (m, 6H), 7.41 - 7.36 (m, 2H), 4.86 (t, 1H), 4.11 - 4.06 (m, 1H), 2.63 - 2.57 (m, 2H), 1.97 - 1.88 (m, 2H), 1.74 - 1.59 (m, 4H) APCI (Multimode) m/z: 637 [M+H]

### Step (c) N-{cis-4-[1-{4'-[(dimethylamino)methyl]-2'-hydroxybiphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

To a solution of 6-fluoro-*N*-{*cis*-4-[6-fluoro-1-(4'-formyl-2'-hydroxybiphenyl-3-yl)-2,4-dioxo-1,4-dihydropyrido[2,3-*d*]pyrimidin-3(2*H*)-yl]cyclohexyl}imidazo[1,2-a]pyridine-2-carboxamide (0.28 g, 0.44 mmol) in 1,2-dichloroethane (10 mL) was added dimethylamine solution in tert-butylmethylether (2M) (0.660 mL, 1.32 mmol) and acetic acid (0.101 mL, 1.76 mmol). The reaction mixture was stirred for 10 min before addition of sodium triacetoxyborohydride (0.140 g, 0.66 mmol). Further dimethylamine solution in tert-butylmethylether (2M) (0.660 mL, 1.32 mmol) was added and the reaction mixture was stirred at room temperature overnight. The solvents were evaporated and the residue was dissolved in methanol (5 mL), and loaded onto a 10g SCX cartridge. The impurities were washed through with methanol (70 mL) and discarded. The product was eluted with 1N methanolic ammonia (100 mL) and evaporated in vacuo. The crude product was purified by flash silica chromatography, elution gradient 2 to 5 % methanol in dichloromethane. The compound was further purified by reverse phase HPLC (95-50 % gradient of aqueous 0.1 % trifluoroacetic acid in acetonitrile) to afford the title compound as a white solid (0.034 g, 10 %).
¹H NMR (400 MHz, DMSO-*d₆*) δ 10.21 (s, 1H), 9.65 (s, 1H), 8.58 (d, 1H), 8.31 (dd, 1H), 7.91 (dd, 1H), 7.84 (dd, 2H), 7.55 - 7.46 (m, 5H), 7.39 - 7.37 (m, 2H), 7.08 (s, 1H), 7.05 (d, 1H), 4.85 (t, 1H), 4.30 (s, 2H), 4.11 (s, 1H), 2.81 (d, 6H), 2.62 - 2.54 (m, 2H), 2.03 - 1.90 (m, 2H), 1.73 - 1.63 (m, 4H).
APCI (Multimode) m/z: 666 [M+H]

### Example 95

### 6-fluoro-N-{cis-4-[6-fluoro-1-{4'-[3-(isopropylamino)propyl]biphenyl-3-yl}-2,4-dioxo-1,4-dihydropyrido[2,3-d]pyrimidin-3(2H)-yl]cyclohexyl}imidazo[1,2-a]pyridine-2-carboxamide

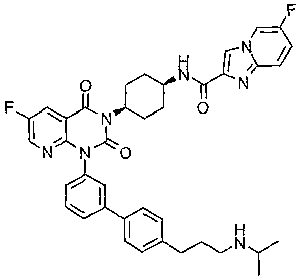

### Step (a) 3-{3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl}propyl methanesulfonate

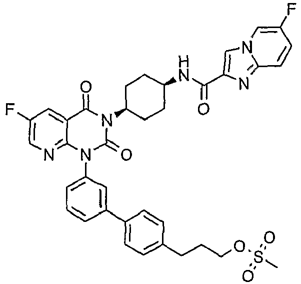

To a solution of 6-fluoro-*N*-{*cis*-4-[6-fluoro-1-[4'-(3-hydroxypropyl)biphenyl-3-yl]-2,4-dioxo-1,4-dihydropyrido[2,3-*d*]pyrimidin-3(2*H*)-yl]cyclohexyl}imidazo[1,2-*a*]pyridine-2-carboxamide (0.5 g, 0.77 mmol) and triethylamine (0.389 g, 3.84 mmol) in dichloromethane (10 mL) was added methanesulfonyl chloride (0.066 mL, 0.85 mmol) in dichloromethane (10 mL). The reaction mixture was stirred at room temperature for 2 h. Poured into water and extracted with dichloromethane. Organic portions washed with brine and dried over magnesium sulphate, filtered and evaporated to give the sub-title compound (0.550 g, 98 %) as an off white solid.
¹H NMR (300 MHz, CDCl₃) δ 8.38 (d, 1H), 8.23 (dd, 1H), 8.10 (s, 1H), 8.06 (t, 1H), 7.75 - 7.71 (m, 2H), 7.64 (t, 1H), 7.60 - 7.53 (m, 4H), 7.32 - 7.24 (m, 2H), 7.15 (ddd, 1H), 5.06 (s, 1H), 4.42 (s, 1H), 4.25 (t, 2H), 3.00 (s, 3H), 2.83 - 2.76 (m, 4H), 2.70 - 2.60 (m, 2H), 2.16 - 2.08 (m, 4H), 1.86 - 1.71 (m, 2H)

### Step (b)) 6-fluoro-N-{cis-4-[6-fluoro-1-{4'-[3-(isopropylamino)propyl]biphenyl-3-yl}-2,4-dioxo-1,4-dihydropyrido[2,3-d]pyrimidin-3(2H)-yl]cyclohexyl}imidazo[1,2-a]pyridine-2-carboxamide

A solution of 3-{3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}propyl methanesulfonate (0.2 g, 0.27 mmol), iso-propylamine (1 mL, 11.74 mmol) and potassium carbonate (0.379 g, 2.74 mmol) in Acetonitrile (10 mL) was heated at 70 °C for 5 h. The reaction mixture was cooled, poured into water (50mL) and extracted with ethyl acetate. The organic portions were combined, dried (magnesium sulphate) filtered and evaporated. The crude product was purified by preparative HPLC on an Waters X-Bridge column using a 50-30% gradient of aqueous 0.2% ammonia in acetonitrile as eluent. The fractions containing the desired compound were evaporated to dryness to afford the title compound (9 mg, 5 %) as a white solid.

¹H NMR (300 MHz, CDCl₃) δ 9.18 (2H, s), 8.35 (1H, d), 8.22 (1H, dd), 8.12 (1H, s), 8.05 (1H, t), 7.76 - 7.55 (4H, m), 7.51 - 7.45 (3H, m), 7.30 - 7.25 (1H, m), 7.21 - 7.13 (3H, m), 5.06 (1H, t), 4.41 (1H, s), 3.28 - 3.16 (1H, m), 2.93 - 2.70 (4H, m), 2.68 - 2.59 (2H, m), 2.17 - 2.09 (2H, m), 2.06 - 1.98 (2H, m), 1.87 - 1.69 (4H, m), 1.27 (6H, d)
APCI (Multimode) m/z: 692.3 [M+H]

### Example 96

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide

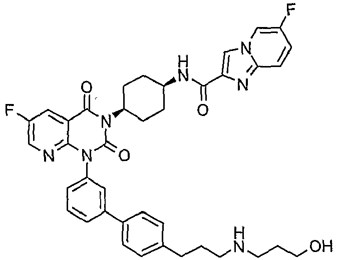

A solution of 3-{3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}propyl methanesulfonate (0.2 g, 0.27 mmol), 3-Amino-1-propanol (1 mL, 0.27 mmol) and potassium carbonate (0.379 g, 2.74 mmol) in Acetonitrile (10 mL) was heated at 70 °C for 5 h. The reaction mixture was cooled, poured into water (50mL) and extracted with ethyl acetate. The organic portions were combined, dried (magnesium sulphate) filtered and evaporated. The crude product was purified by preparative HPLC on a Waters X-Bridge column using a 60-40% gradient of aqueous 0.2% ammonia in acetonitrile as eluent. The fractions containing the desired compound were evaporated to dryness to afford the title compound (35 mg, 36%) as a white solid.
¹H NMR (300 MHz, CDCl₃) δ 8.37 (d, 1H), 8.23 (dd, 1H), 8.10 (d, 1H), 8.07 - 8.04 (m, 1H), 7.76 - 7.71 (m, 2H), 7.66 - 7.51 (m, 5H), 7.30 - 7.22 (m, 3H), 7.14 (ddd, 1H), 5.06 (s, 1H), 4.43 (s, 1H), 3.81 (t, 2H), 2.88 (t, 2H), 2.79 (d, 2H), 2.72 - 2.63 (m, 4H), 2.17 - 2.08 (m, 2H), 1.88 - 1.65 (m, 6H)
APCI (Multimode) m/z: 708.3 [M+H]

### Example 97

### 6-fluoro-N-{cis-4-[6-fluoro-2,4-dioxo-1-[4'-(3-piperazin-1-ylpropyl)biphenyl-3-yl]-1,4-dihydropyrido[2,3-d]pyrimidin-3(2H)-yl]cyclohexyl}imidazo[1,2-a]pyridine-2-carboxamide

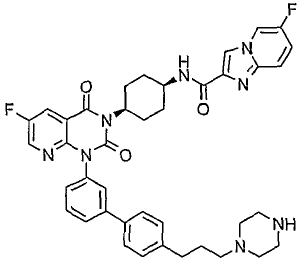

3-{3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}propyl methanesulfonate (0.15 g, 0.21 mmol), potassium carbonate (0.29 g, 2.10 mmol) and N-BOC-Piperazine (0.2 g, 1.07 mmol) were heated together at 70 °C for 12 h in Acetonitrile (10 mL). The reaction mixture was cooled, poured into water (50mL) and extracted with ethyl acetate. The organic portions were combined, dried (magnesium sulphate) filtered and evaporated. The residue was treated with hydrochloric acid (10mL, 40.00 mmol) in dioxane and stirred for 16 h. The solvent was evaporated. The crude product was purified by preparative HPLC on a Sunfire column using a 95-50% gradient of aqueous 0.1% trifluoroacetic acid in acetonitrile as eluent. The fractions containing the desired compound were evaporated to dryness to afford the title compound (0.050 g, 34 %) as a white solid.
¹H NMR (400 MHz, DMSO-D6) δ 9.20 (s, 1H), 8.78 (dd, 1H), 8.55 (d, 1H), 8.37 (s, 1H), 8.30 (dd, 1H), 7.75 - 7.67 (m, 4H), 7.62 - 7.56 (m, 3H), 7.46 - 7.40 (m, 1H), 7.35 - 7.29 (m, 2H), 4.84 (t, 1H), 4.16 (s, 1H), 3.63 - 3.31 (m, 8H), 3.11 (t, 2H), 2.67 - 2.55 (m, 4H), 2.01 - 1.91 (m, 4H), 1.73 - 1.60 (m, 4H)
APCI (Multimode) m/z: 719 [M+H]

### Example 98

### 6-fluoro-N-{cis-4-[6-fluoro-1-{4'-[2-(isopropylamino)ethyl]biphenyl-3-yl}-2,4-dioxo-1,4-dihydropyrido[2,3-d]pyrimidin-3(2H)-yl]cyclohexyl}imidazo[1,2-a]pyridine-2-carboxamide

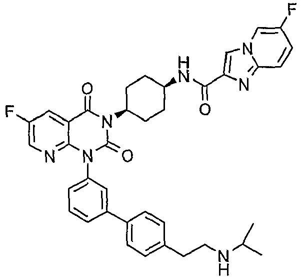

### Step (a) 6-fluoro-N-{cis-4-[6-fluoro-1-[4'-(2-hydroxyethyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

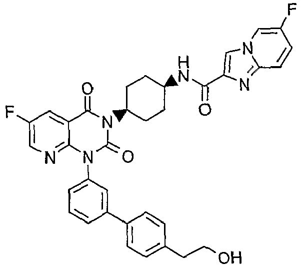

### Made in a similar manner to Example 79 step (b)

ES+ (M+H) 621

### Step (b) 2-{3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl} ethyl methanesulfonate

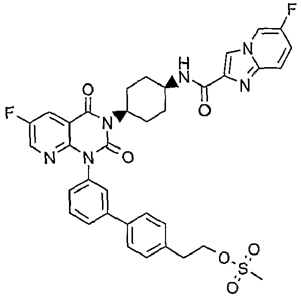

### Made in a similar manner to Example 95 step (a)

ES+ (M+H) 715

### Step (c) 6-fluoro-N-{cis-4-[6-fluoro-1-{4'-[2-(isopropylamino)ethyl]biphenyl-3-yl}-2,4-dioxo-1,4-dihydropyrido[2,3-d]pyrimidin-3(2H)-yl]cyclohexyl}imidazo[1,2-a]pyridine-2-carboxamide

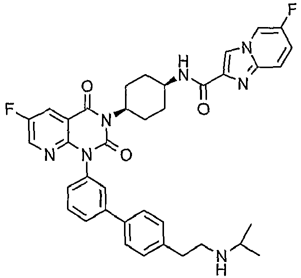

A solution of 2-{3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}ethyl methanesulfonate (0.15 g, 0.21 mmol) and iso-propylamine (1 mL, 11.74 mmol) and Potassium carbonate (0.29 g, 2.10 mmol) in Acetonitrile (10 mL) was heated at 70 °C for 5 h. The reaction mixture was cooled, poured into water (50mL) and extracted with ethyl acetate. The organic portions were combined, dried (magnesium sulphate) filtered and evaporated. The crude product was purified by preparative HPLC on a Waters X-Bridge column using a 50-30% gradient of aqueous 0.2% ammonia in acetonitrile as eluent. The fractions containing the desired compound were evaporated to dryness to afford the title compound (0.026 g, 18 %) as a white solid.
¹H NMR (300 MHz, CDCl₃) δ 9.47 (s, 2H), 8.36 (d, 1H), 8.23 (dd, 1H), 8.13 (s, 1H), 8.06 (t, 1H), 7.76 - 7.48 (m, 7H), 7.31 - 7.24 (m, 2H), 7.18 - 7.10 (m, 1H), 5.06 (t, 1H), 4.41 (s, 1H), 3.38 - 3.28 (m, 1H), 3.18 - 3.03 (m, 4H), 2.84 - 2.72 (m, 2H), 2.12 (d, 2H), 1.87 - 1.69 (m, 4H), 1.38 (d, 6H)
APCI (Multimode) m/z: 678 [M+H]

### Example 99

### N-{cis-4-[1-{4'-[2-(dimethylamino)ethyl]biphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

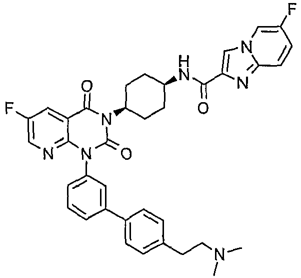

A solution of 2-{3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}ethyl methanesulfonate (0.15 g, 0.21 mmol) and dimethylamine (in MTBE) 2 .07M (10 mL, 20.70 mmol) and Potassium carbonate (0.29 g, 2.10 mmol) in Acetonitrile (5 mL) was heated at 70 °C for 3 h. The reaction mixture was cooled, poured into water (50mL) and extracted with ethyl acetate. The organic portions were combined, dried (magnesium sulphate) filtered and evaporated. The crude product was purified by preparative HPLC on an Xterra column using a 65-45% gradient of aqueous 0.1%ammonia in acetonitrile as eluent. The fractions containing the desired compound were evaporated to dryness to afford the title compound (0.014 g, 10 %) as a white solid.
¹H NMR (300 MHz, CDCl₃) δ 8.37 (d, 1H), 8.24 (dd, 1H), 8.11 (s, 1H), 8.07 (t, 1H), 7.72 (d, 2H), 7.67 - 7.51 (m, 5H), 7.33 - 7.26 (m, 2H), 7.16 (dd, 1H), 5.06 (t, 1H), 4.44 (s, 1H), 3.29 - 3.22 (m, 2H), 3.15 - 3.09 (m, 2H), 2.87 (s, 6H), 2.83 - 2.76 (m, 2H), 2.13 (d, 2H), 1.88 - 1.70 (m, 4H)
APCI (Multimode) m/z: 664.3 [M+H]

### Example 100

### 6-fluoro-N-{cis-4-[6-fluoro-1-{4'-[2-(4-methylpiperazin-1-yl)ethyl]biphenyl-3-yl}-2,4-dioxo-1,4-dihydropyrido[2,3-d]pyrimidin-3(2H)-yl]cyclohexyl}imidazo[1,2-a]pyridine-2-carboxamide

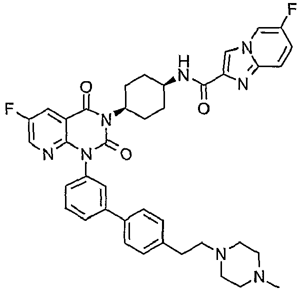

A solution of 2-{3'-[6-fluoro-3-(*cis*-4-{[(6-fluoroimidazo[1,2-a]pyridin-2-yl)carbonyl]amino}cyclohexyl)-2,4-dioxo-3,4-dihydropyrido[2,3-*d*]pyrimidin-1(2*H*)-yl]biphenyl-4-yl}ethyl methanesulfonate (0.15 g, 0.21 mmol) and 1-methylpiperazine (1 mL, 9.02 mmol) and Potassium carbonate (0.29 g, 2.10 mmol) in Acetonitrile (10 mL) was heated at 70 °C for 2 h. The reaction mixture was cooled, poured into water (50mL) and extracted with ethyl acetate. The organic portions were combined, dried (magnesium sulphate) filtered and evaporated. The crude product was purified by preparative HPLC on a Sunfire column using a 95-60% gradient of aqueous 0.1% trifluoroacetic acid in acetonitrile as eluent. The fractions containing the desired compound were evaporated to dryness to afford the tile compound (0.022 g, 15 %) as a white solid.
¹H NMR (300 MHz, CDCl₃) δ 8.37 (d, 1H), 8.24 (dd, 1H), 8.13 (s, 1H), 8.08 (s, 1H), 7.77 - 7.57 (m, 5H), 7.51 (s, 1H), 7.33 - 7.17 (m, 3H), 5.11 - 5.01 (m, 1H), 4.41 (s, 1H), 3.57 - 2.72 (m, 15H), 2.18 - 2.10 (m, 2H), 1.87 - 1.70 (m, 4H)
APCI (Multimode) m/z: 719.3 [M+H]

### Example 101

### N-{cis-4-[1-(4'-{2-[tert-butyl(methyl)amino]ethyl}biphenyl-3-yl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

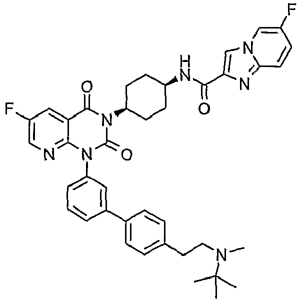

A solution of 2-{3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl} ethyl methanesulfonate (0.15 g, 0.21 mmol) and n-methyl-tert-butylamine (1 mL, 8.34 mmol) and Potassium carbonate (0.29 g, 2.10 mmol) in Acetonitrile (10 mL) was heated at 70 °C for 5h. The reaction mixture was cooled, poured into water (50mL) and extracted with ethyl acetate. The organic portions were combined, dried (magnesium sulphate) filtered and evaporated. The crude product was purified by preparative HPLC on a Sunfire column using a 95-50% gradient of aqueous 0.1% trifluoroacetic acid in acetonitrile as eluent. The fractions containing the desired compound were evaporated to dryness to afford the title compound (0.043 g, 29%) as a white solid.
¹H NMR (399.824 MHz, CDCl₃) δ 8.37 (d, 1H), 8.23 (dd, 1H), 8.14 (s, 1H), 8.08 (d, 1H), 7.77 - 7.69 (m, 2H), 7.64 (t, 1H), 7.58 (d, 2H), 7.50 (t, 1H), 7.31 (d, 2H), 7.25 - 7.20 (m, 1H), 5.10 - 5.01 (m, 1H), 4.40 (s, 1H), 3.59 - 3.52 (m, 1H), 3.34 - 3.26 (m, 1H), 3.06 (td, 1H), 2.87 - 2.75 (m, 6H), 2.15 - 2.12 (m, 2H), 1.85 - 1.70 (m, 4H), 1.46 (s, 9H)
APCI (Multimode) m/z: 706.3 [M+H]

### Example 102

### 6-fluoro-N-{cis-4-[6-fluoro-2,4-dioxo-1-[4'-(2-piperazin-1-ylethyl)biphenyl-3-yl]-1,4-dihydropyrido[2,3-d]pyrimidin-3(2H)-yl]cyclohexyl}imidazo[1,2-a]pyridine-2-carboxamide

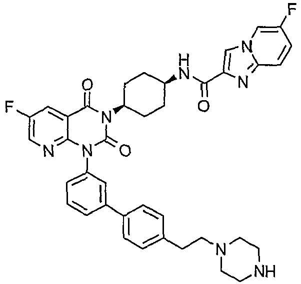

2-{3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}ethyl methanesulfonate (0.15 g, 0.21 mmol), Potassium carbonate (0.29 g, 2.10 mmol) and N-BOC-Piperazine (0.2 g, 1.07 mmol) were heated together at 70 °C for 12 h in Acetonitrile (10 mL). The reaction mixture was cooled, poured into water (50 mL) and extracted with ethyl acetate. The organic portions were combined, dried (magnesium sulphate) filtered and evaporated. The residue was treated with hydrochloric acid (10mL, 40.00 mmol) in dioxane and stirred for 16 h. The solvent was evaporated. The crude product was purified by preparative HPLC on a Sunfire column using a 95-50% gradient of aqueous 0.1% trifluoroacetic acid in acetonitrile as eluent. The fractions containing the desired compound were evaporated to dryness to afford the title compound (0.015 g, 10%) as a white solid.
¹H NMR (400 MHz, DMSO-D6) δ 8.82 (dd, 1H), 8.59 (d, 1H), 8.40 (s, 1H), 8.34 (dd, 2H), 7.79 - 7.59 (m, 8H), 7.49 - 7.43 (m, 4H), 7.41 - 7.37 (m, 4H), 4.88 (m, 1H), 4.16 (s, 1H), 4.09 - 3.83 (m, 2H), 3.44 - 3.32 (m, 8H), 3.04 - 2.99 (m, 2H), 2.68 - 2.56 (m, 4H), 2.02 (d, 2H), 1.77 - 1.63 (m, 4H)
APCI (Multimode) m/z: 705 [M+H]

### Example 103

### 6-fluoro-N-{cis-4-[6-fluoro-1-[4'-hydroxy-2'-(morpholin-4-ylmethyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

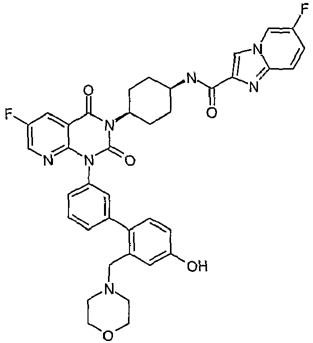

### Step (a) [4-hydroxy-2-(morpholin-4-ylmethyl)phenyl]boronic acid

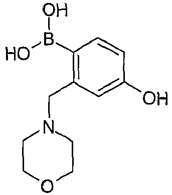

To a solution of 4-bromo-3-(morpholinomethyl)phenol (0.5 g, 1.84 mmol) in THF (10 mL) was added tert-Butyllithium (3.67 mL, 5.51 mmol) and the reaction mixture stirred at -78 °C for 10mins then warmed to 0 °C for 15mins. The reaction was then cooled to -78° and Triisopropyl borate (1.272 mL, 5.51 mmol) was added. The reaction was warmed to room temperature and stirred for 1h. Saturated NH₄Cl was added and the reaction stirred at room temperature for 2h. The reaction mixture was diluted with ethyl acetate (200 mL), The product was extracted with ethyl acetate. The combined organic extracts were washed with saturated brine (50 mL). The organic was dried over magnesium sulfate, filtered and evaporated to afford the sub-title compound (0.442 g, 97%). This was used without further purification
ES (-) m/z: 236 [M-H]

### Step (b) 6-fluoro-N-{cis-4-[6-fluoro-1-[4'-hydroxy-2'-(morpholin-4-ylmethyl)biphenyl-3-yl]-2,4-dioxo-1,4-dihydropyrido[2,3-d]pyrimidin-3(2H)-yl]cyclohexyllimidazo[1,2-a]pyridine-2-carboxamide

Palladium(II) acetate (6.99 mg, 0.03 mmol) and 2-Dicyclohexylphosphino-2',6'-dimethoxybiphenyl (0.026 g, 0.06 mmol) were stirred together in Acetonitrile (1.5 mL) for 10 min. before the addition of a solution of Potassium carbonate (0.129 g, 0.93 mmol) in Water (1.5 mL). 4-hydroxy-2-(morpholinomethyl)phenylboronic acid (0.074 g, 0.31 mmol) and 6-fluoro-*N*-{*cis*-4-[6-fluoro-1-(3-iodophenyl)-2,4-dioxo-1,4-dihydropyrido[2,3-*d*]pyrimidin-3(2*H*)-yl]cyclohexyl}imidazo[1,2-*a*]pyridine-2-carboxamide (0.2 g, 0.31 mmol) were then added sequentially. The resulting suspension was stirred at 80 °C for 16 h. The crude reaction mixture was passed through a Chemelut cartridge eluting with DCM, and obtained as a brown oil after concentration in vacuo. The crude product was purified by preparative HPLC on a Waters X-Bridge column using a 95-50% gradient of aqueous 0.1% trifluoroacetic acid in acetonitrile as eluent. The fractions containing the desired compound were evaporated to dryness to afford the title compound (39 mg, 18%) as a white solid.
¹H NMR (399.824 MHz, CDCl₃) δ 8.39 (d, 1H), 8.24 (dd, 1H), 8.17 (s, 1H), 8.13 (t, 1H), 7.82 (dd, 1H), 7.72 - 7.71 (m, 1H), 7.65 (t, 2H), 7.48 (d, 1H), 7.37 - 7.28 (m, 3H), 7.18 (t, 1H), 7.00 (dd, 1H), 5.05 - 4.98 (m, 1H), 4.38 (s, 1H), 4.29 (s, 2H), 3.79 - 3.73 (m, 4H), 2.81 - 2.73 (m, 2H), 2.37 (s, 4H), 2.19 - 2.16 (m, 2H), 1.85 - 1.78 (m, 2H), 1.72 - 1.69 (m, 2H)
APCI (Multimode) m/z: 708 [M+H]

### Example 104

### N-{cis-4-[1-{2'-[(dimethylamino)methyl]-4'-hydroxybiphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

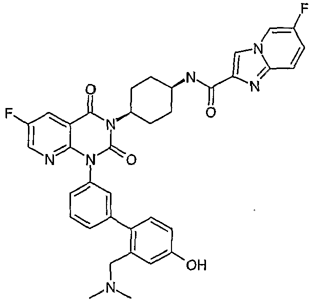

### Step (a) {2-[(dimethylamino)methyl]-4-hydroxyphenyl}boronic acid

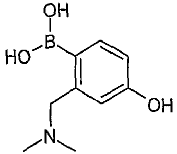

To a solution of 4-bromo-3-((dimethylamino)methyl)phenol (2.05 g, 8.91 mmol) in THF (40 mL) was added tert-Butyllithium (17.82 mL, 26.73 mmol) and the reaction mixture stirred at -78 °C for 10mins then warmed to 0 °C for 15 min. The reaction was then cooled to -78° and Triisopropyl borate (6.17 mL, 26.73 mmol) was added. The reaction was warmed to room temperature and stirred for 1hr. Saturated NH₄Cl was added and the reaction stirred at room temperature for 2h. The reaction mixture was diluted with ethyl acetate (200 mL), The product was extracted with ethyl acetate. The combined organic extracts were washed with saturated brine (200 mL). The organic was dried over magnesium sulfate, filtered and evaporated to afford the sub-title compound 2-((dimethylamino)methyl)-4-hydroxyphenylboronic acid (1.40 g, 81%) as a gum. This was used without further purification
ES (-) m/z: 294 [M-H]

### Step (b) N-{cis-4-[1-{2'-[(dimethylamino)methyl]-4'-hydroxybiphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

Palladium(II) acetate (11 mg, 0.05 mmol) and 2-Dicyclohexylphosphino-2',6'-dimethoxybiphenyl (0.038 g, 0.09 mmol) were stirred together in Acetonitrile (3 mL) for 10 min. before the addition of a solution of Potassium carbonate (0.258 g, 1.87 mmol) in Water (3 mL). 2-((dimethylamino)methyl)-4-hydroxyphenylboronic acid (0.182 g, 0.93 mmol) and 6-fluoro-*N*-{*cis*-4-[6-fluoro-1-(3-iodophenyl)-2,4-dioxo-1,4-dihydropyrido[2,3-*d*]pyrimidin-3(2*H*)-yl]cyclohexyl}imidazo[1,2-*a*]pyridine-2-carboxamide (0.3 g, 0.47 mmol) were then added sequentially. The resulting suspension was stirred at 80 °C for 16 h. The crude reaction mixture was passed through a Chemelut cartridge eluting with DCM, and obtained as a brown oil after concentration in vacuo. The crude product was purified by preparative HPLC on a Waters X-Bridge column using a 95-50% gradient of aqueous 0.1% trifluoroacetic acid in acetonitrile as eluent. The fractions containing the desired compound were evaporated to dryness to afford a still impure product as a white solid. This compound was further purified by preparative HPLC on a Phenomenex Gemini column using a 95-5% gradient of aqueous 0.2% ammonia in acetonitrile as eluent. The fractions containing the desired compound were evaporated to dryness to afford the title compound (31 mg, 10%) as a white solid.
¹H NMR (399.824 MHz, CDCl₃) δ 8.37 - 8.36 (m, 1H), 8.22 - 8.20 (m, 1H), 8.13 (s, 1H), 8.06 (s, 1H), 7.77 - 7.75 (m, 1H), 7.60 - 7.56 (m, 2H), 7.46 (d, 1H), 7.33 (s, 1H), 7.28 - 7.26 (m, 1H), 7.18 - 7.13 (m, 2H), 6.99 (d, 1H), 6.77 (dd,, 1H), 5.04 (t, 1H), 4.42 (s, 1H), 3.37 (s, 2H), 2.79 (dd, 2H), 2.12 (s, 8H), 1.83 - 1.69 (m, 4H)
APCI (Multimode) m/z: 666 [M+H]

### Example 105

### tert-butyl 4-(2- {[cis-4-(1-biphenyl-3-yl-6-fluoro-2,4-dioxo-1,4-dihydropyrido[2,3-d]pyrimidin-3(2H)-yl)cyclohexyl]amino}-2-oxoethyl)piperazine-1-carboxylate

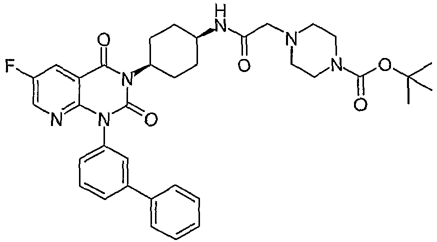

To a solution of 4-Carboxymethyl-piperazine-l-carboxylic acid tert-butyl ester (0.125 g, 0.51 mmole) in DMF (3 mL) was added DIPEA (0.2404 g, 0.331 mL, 1.86 mmole) followed by O-(7-Azabezotriazol-1-yl)-N,N,N',N'-tetramethyluronium hexafluorophosphate (0.1945 g, 0.51 mmole). The reaction was stirred for 10mins. 3-(*cis-*4-aminocyclohexyl)-1-biphenyl-3-yl-6-fluoropyrido[2,3-*d*]pyrimidine-2,4(1*H*,3*H*)-dione (0.2 g, 0.47 mmole) was then added and the reaction stirred for 12h. Water was added and the product filtered to afford the title compound (0.176 g, 71 %).
¹H NMR (399.824 MHz, CDCl₃) δ 8.38 (1H, d), 8.17 (1H, dd), 7.84 (1H, d), 7.73 (1H, dt), 7.64 - 7.60 (3H, m), 7.49 (1H, t), 7.45 - 7.42 (2H, m), 7.38 - 7.34 (1H, m), 7.27 - 7.25 (1H, m), 5.03 (1H, s), 4.28 (1H, d), 3.45 (4H, s), 3.02 (2H, s), 2.64 - 2.60 (2H, m), 2.49 (4H, s), 1.91 (2H, d), 1.72 - 1.65 (4H, m), 1.47 (9H, s)
APCI (Multimode) m/z: 657 [M+H]

### Example 106

### N-[cis-4-(1-biphenyl-3-yl-6-fluoro-2,4-dioxo-1,4-dihydropyrido[2,3-d]pyrimidin-3(2H)-yl)cyclohexyl]-2-piperazin-1-ylacetamide

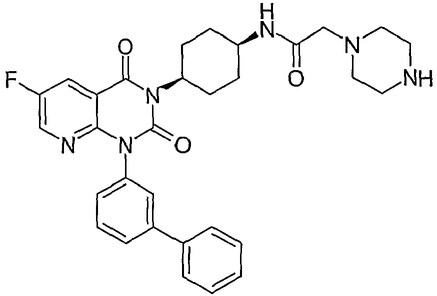

To a solution of *tert*-butyl 4-(2-{[*cis*-4-(1-biphenyl-3-yl-6-fluoro-2,4-dioxo-1,4-dihydropyrido[2,3-*d*]pyrimidin-3(2*H*)-yl)cyclohexyl]amino}-2-oxoethyl)piperazine-1-carboxylate (0.176 g, 0.27 mmole) in Dioxane (2mL) was added HCl (4M in 1,4-DIOXANE) (3 mL, 12 mmole) and the reaction stirred for 10h. Solvents were evaporated. The crude product was purified by preparative HPLC on a Xterra column using a 75-5% gradient of aqueous ammonia (0.1%) in acetonitrile as eluent to give the title compound (0.071 g, 44%).
APCI (Multimode) m/z: 557 [M+H]

### Example 107

### 1-biphenyl-3-yl-6-fluoro-3-[(3R)-pyrrolidin-3-yl]pyrido[2,3-d]pyrimidine-2,4(1H,3H)-dione

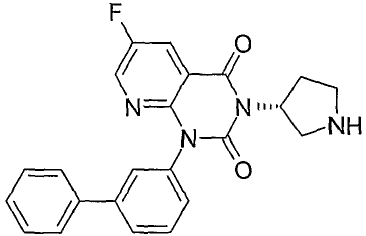

To a solution of 2-(biphenyl-3-ylamino)-5-fluoronicotinic acid (0.065 g, 0.21 mmole) in DMF (1.5mL) was added 1,1'-Carbonyldimidazole (0.051 g, 0.32 mmoles) the reaction left to stir for 10mins. *tert*-butyl (3R)-3-aminopyrrolidine-1-carboxylate (0.0432 g, 0.23 mmoles) was added and the reaction stirred at 35 °C for 1hr. 1,1'-Carbonyldimidazole (0.1196 g, 0.74 mmoles) was added followed by sodium hydride (60% in mineral oil) (0.015 g, 0.63 mmoles) and the reaction stirred for 30mins at 50 °C. Water was added and the product filtered. The residue was purified by flash column chromatography eluting with 10% ethyl acetate/hexane. 4M HCl in dioxane was added (10mL) and stirred for 24h. Solvents evaporated. The residue was purified by flash column chromatography eluting with 5%MEOH/DCM (with 0.2% aq NH₃), This gave the title compound (0.006 g, 7%) as a solid
¹H NMR (400 MHz, at 90°C in DMSO-D6) δ 2.26 - 2.17 (2H, m), 3.24 - 3.17 (2H, m), 3.41 - 3.34 (2H, m), 5.61 (1H, s), 7.37 (2H, t), 7.46 (2H, t), 7.68 - 7.58 (5H, m), 7.74 (1H, d), 8.26 - 8.23 (1H, m), 8.54 (1H, s)
APCI (Multimode) m/z: 403 [M+H]

### Example 108

### N-{cis-4-[6-fluoro-1-[4'-(morpholin-4-ylmethyl)biphenyl-3-yl]-2,4-dioxo-1,4-dihydropyrido[2,3-d]pyrimidin-3(2H)-yl]cyclohexyl}-1,5-dimethyl-1H-pyrazole-3-carboxamide

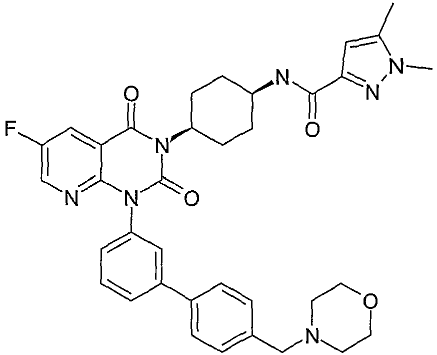

### Step (a) N-{cis-4-[6-fluoro-1-(3-iodophenyl)-2,4-dioxo-1,4-dihydropyrido[2,3-d]pyrimidin-3(2H)-yl]cyclohexyl}-1,5-dimethyl-1H-pyrazole-3-carboxamide

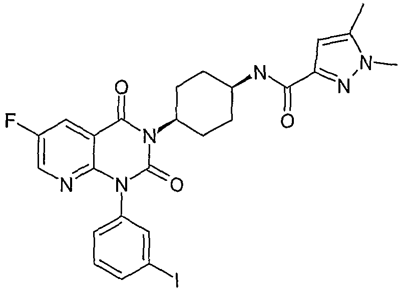

*N*-{*cis*-4-[6-fluoro-1-(3-iodophenyl)-2,4-dioxo-1,4-dihydropyrido[2,3-*d*]pyrimidin-3(2*H*)-yl]cyclohexyl}-1,5-dimethyl-1*H*-pyrazole-3-carboxamide was prepared from 3-(*cis*-4-aminocyclohexyl)-1-biphenyl-3-yl-6-fluoropyrido[2,3-*d*]pyrimidine-2,4(1*H*,3*H*)-dione (6.85 g, 14.27 mmol) and 1,5-dimethyl-1H-pyrazole-3-carboxylic acid (2 g, 14.27 mmol) using a similar method as example 38 step (b) to give the sub-title compound as a white solid (4.6 g).
¹H NMR (400 MHz, DMSO) δ 8.59 (d, 1H), 8.31 (dd, 1H), 7.85 - 7.81 (m, 2H), 7.44 (dt, 1H), 7.33 (t, 1H), 7.21 (d, 1H), 6.42 (d, 1H), 4.82 (t, 1H), 4.08 (s, 1H), 3.77 (s, 3H), 2.58 - 2.51 (m, 2H), 2.27 (s, 3H), 1.98 - 1.91 (m, 2H), 1.71 - 1.55 (m, 4H).

### Step (b) N-{cis-4-[6-fluoro-1-(4'-formylbiphenyl-3-yl)-2,4-dioxo-1,4-dihydropyrido[2,3-d]pyrimidin-3(2H)-yl]cyclohexyl}-1,5-dimethyl-1H-pyrazole-3-carboxamide

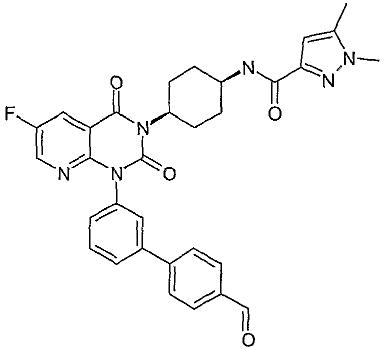

*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}-1,5-dimethyl-1*H*-pyrazole-3-carboxamide was prepared from the product from step (a) (4.5 g, 7.47 mmol) and 4-formylphenylboronic acid (1.680 g, 11.21 mmol) using a similar method as example 49 step (a) to give the sub-title compound as a cream solid (3.75 g).
¹H NMR (400 MHz, DMSO) δ 10.06 (s, 1H), 8.59 (d, 1H), 8.33 (dd, 1H), 8.01 (dd, 2H), 7.94 - 7.86 (m, 4H), 7.67 (t, 1H), 7.47 (dtd, 1H), 7.21 (d, 1H), 6.41 (d, 1H), 4.85 (s, 1H), 4.08 (s, 1H), 3.74 (s, 3H), 2.60 - 2.54 (m, 2H), 2.25 (s, 3H), 1.98 - 1.93 (m, 2H), 1.72 - 1.60 (m, 4H).

### Step (c) N-{cis-4-[6-fluoro-1-[4'-(morpholin-4-ylmethyl)biphenyl-3-yl]-2,4-dioxo-1,4-dihydropyrido[2,3-d]pyrimidin-3(2H)-yl]cyclohexyl}-1,5-dimethyl-1H-pyrazole-3-carboxamide

*N*-{*cis*-4-[6-fluoro-1-[4'-(morpholin-4-ylmethyl)biphenyl-3-yl]-2,4-dioxo-1,4-dihydropyrido[2,3-*d*]pyrimidin-3(2*H*)-yl]cyclohexyl}-1,5-dimethyl-1*H*-pyrazole-3-carboxamide was prepared from the product of step (b) (400mg, 0.69 mmol) using a similar method as example 51 to give the title compound as a white solid (as the di-trifluoroacetate salt) (142 mg).
¹H NMR (400 MHz, DMSO) δ 10.27 (s, 1H), 8.59 (d, 1H), 8.34 (dd, 1H), 7.85 - 7.77 (m, 4H), 7.67 - 7.59 (m, 3H), 7.43 (ddd, 1H), 7.20 (d, 1H), 6.42 (s, 1H), 4.85 (t, 1H), 4.39 (s, 2H), 4.08 (s, 1H), 3.98 - 3.92 (m, 2H), 3.75 (s, 3H), 3.70 - 3.59 (m, 2H), 3.33 - 3.23 (m, 2H), 3.19 - 3.08 (m, 2H), 2.63 - 2.53 (m, 2H), 2.25 (s, 3H), 1.96 (d, 2H), 1.72 - 1.59 (m, 4H).
[M+H]⁺=652 (MultiMode+)

### Example 109

### N-{cis-4-[6-fluoro-1-[4'-(morpholin-4-ylmethyl)biphenyl-3-yl]-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide

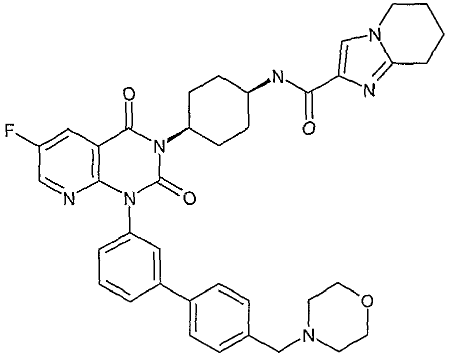

### Step (a) N-{cis-4-[6-fluoro-1-(3-iodophenyl)-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide

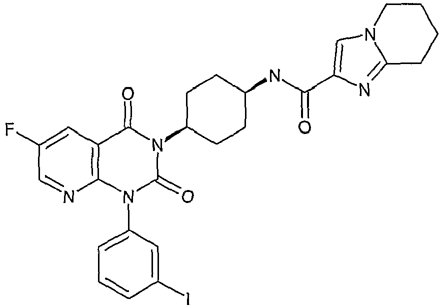

*N*-{*cis*-4-[6-fluoro-1-(3-iodophenyl)-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide was prepared from 3-(*cis*-4-aminocyclohexyl)-1-biphenyl-3-yl-6-fluoropyrido[2,3-*d*]pyrimidine-2,4(1*H*,3*H*)-dione (6.53 g, 13.60 mmol) 5,6,7,8-tetrahydroimidazo[1,2-*a*]pyridine-2-carboxylic acid hydochloride (2.87 g, 14.16 mmol) using a similar method as example 38 step (b) to give the sub-title compound as a white solid (2.598 g).
¹H NMR (400 MHz, DMSO) δ 8.59 (d, 1H), 8.31 (dd, 1H), 7.84 - 7.82 (m, 2H), 7.49 (s, 1H), 7.45 - 7.42 (m, 1H), 7.33 (t, 1H), 7.29 (d, 1H), 4.81 (t, 1H), 4.10 - 4.07 (m, 1H), 3.96 (t, 2H), 2.75 (t, 2H), 2.57 - 2.52 (m, 2H), 1.92 - 1.83 (m, 6H), 1.69 - 1.59 (m, 4H).

### Step (b) N-{cis-4-[6-fluoro-1-(4'-formylbiphenyl-3-yl)-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide

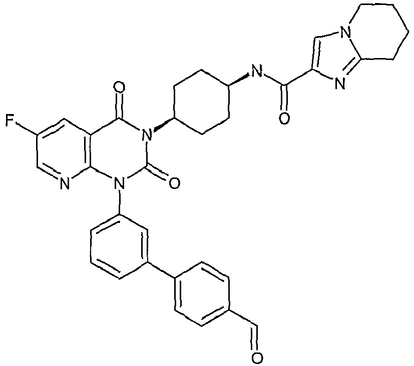

*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide was prepared from the product from step (a) (3.70 g, 5.89 mmol) and 4-formylphenylboronic acid (1.324 g, 8.83 mmol) using a similar method as example 49 step (a) to give the sub-title compound as a cream solid (2.28 g).
¹H NMR (400 MHz, DMSO) δ 10.06 (s, 1H), 8.59 (d, 1H), 8.33 (dd, 1H), 8.01 (d, 2H), 7.91 - 7.86 (m, 4H), 7.67 (t, 1H), 7.48 - 7.46 (m, 2H), 7.29 (d, 1H), 4.84 (t, 1H), 4.11 - 4.07 (m, 1H), 3.95 (t, 2H), 2.72 (t, 2H), 2.60 - 2.52 (m, 2H), 1.93 - 1.79 (m, 6H), 1.71 - 1.61 (m, 4H).

### Step (c) N-cis-4-[6-fluoro-1-[4'-(morpholin-4-ylmethyl)biphenyl-3-yl]-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide

*N*-{*cis*-4-[6-fluoro-1-[4'-(morpholin-4-ylmethyl)biphenyl-3-yl]-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide was prepared from the product of step (b) (306mg, 0.50 mmol) using a similar method as example 51 to give the title compound as a white solid (as the di-trifluoroacetate salt) (334 mg).
¹H NMR (400 MHz, DMSO) δ 8.59 (d, 1H), 8.31 (dd, 1H), 8.16 - 8.12 (m, 2H), 7.84 - 7.77 (m, 4H), 7.65 (t, 1H), 7.60 (d, 2H), 7.42 (d, 1H), 4.84 (t, 1H), 4.39 (s, 2H), 4.11 - 4.09 (m, 2H), 4.03 - 4.00 (m, 1H), 3.27 - 3.14 (m, 8H), 2.92 - 2.89 (m, 2H), 2.69 - 2.60 (m, 2H), 2.07 (d, 2H), 1.97 - 1.86 (m, 4H), 1.71 - 1.57 (m, 4H).
[M+H]⁺=678 (MultiMode+)

### Example 110

### 1-(dimethylamino)-N-{cis-4-[1-(4'-{[[2-(dimethylamino)ethyl](methyl)amino]methyl}biphenyl-3-yl)-6-fluoro-2,4-dioxo-1,4-dihydropyrido[2,3-d]pyrimidin-3(2H)-yl]cyclohexyl}cyclopropanecarboxamide

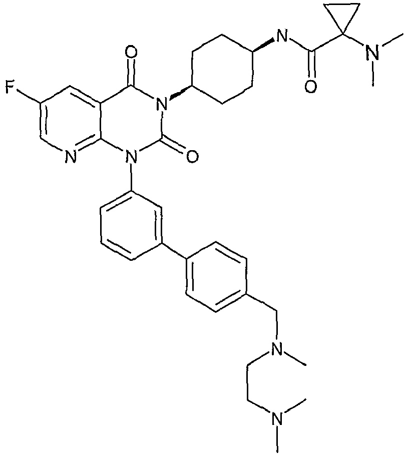

### Step (a) 1-(dimethylamino)-N-{cis-4-[6-fluoro-1-(3-iodophenyl)-2,4-dioxo-1,4-dihydropyrido[2,3-d]pyrimidin-3(2H)-yl]cyclohexyl}cyclopropanecarboxamide

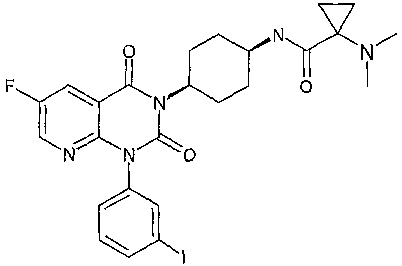

1-(dimethylamino)-*N*-{*cis*-4-[6-fluoro-1-(3-iodophenyl)-2,4-dioxo-1,4-dihydropyrido[2,3-*d*]pyrimidin-3(2*H*)-yl]cyclohexyl}cyclopropanecarboxamide was prepared from 3-(*cis*-4-ami4nocyclohexyl)-1-biphenyl-3-yl-6-fluoropyrido[2,3-*d*]pyrimidine-2,4(1*H*,3*H*)-dione (7.83 g, 16.30 mmol) and 1-(dimethylamino)cyclopropanecarboxylic acid (2.00 g, 15.49 mmol) using a similar method as example 38 step (b) to give the sub-title compound as a foam (7.5 g).
¹H NMR (400 MHz, CDCl₃) δ 8.36 (d, 1H), 8.19 (dd, 1H), 7.86 - 7.81 (m, 1H), 7.68 - 7.65 (m, 1H), 7.31 - 7.27 (m, 2H), 5.02 - 4.92 (m, 1H), 4.20 - 4.14 (m, 1H), 2.73 - 2.60 (m, 2H), 2.26 (s, 6H), 2.00 - 1.82 (m, 3H), 1.73 - 1.58 (m, 3H), 1.18 (dd, 2H), 0.96 (dd, 2H).

### Step (b) 1-(dimethylamino)-N-{cis-4-[6-fluoro-1-(4'-formylbiphenyl-3-yl)-2,4-dioxo-1,4-dihydropyrido[2,3-d]pyrimidin-3(2H)-yl]cyclohexyl}cyclopropanecarboxamide

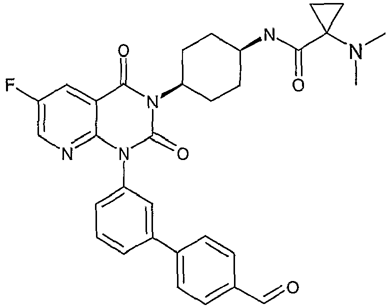

1-(dimethylamino)-*N*-{*cis-*4-[6-fluoro-1-(4'-formylbiphenyl-3-yl)-2,4-dioxo-1,4-dihydropyrido[2,3-*d*]pyrimidin-3(2*H*)-yl]cyclohexyl}cyclopropanecarboxamide was prepared from the product from step (a) (2.66 g, 4.5 mmol) which was dissolved in ethanol (7.2 mL) and toluene (12 mL), and 4-formylbenzeneboronic acid (0.675 g, 4.50 mmol) and 2M aq Na₂CO₃ (6 mL) added. Tetrakis (triphenylphosphine)Palladium(O) (60mg) was added and the mixture heated at 85 °C overnight. The reaction mixture was concentrated, dissolved in ethyl acetate, washed with water and purified on SCX. The product containing fractions were concentrated and stirred in acetone (30 mL) and 2M aq HCl (1 mL) for 3 days. Concentration and azeotroping afforded the sub-title compound as a foam (2.1 g).
¹H NMR (400 MHz, DMSO) δ 10.06 (s, 1H), 8.60 (d, 1H), 8.32 (dd, 1H), 8.02 (d, 2H), 7.92 (d, 2H), 7.88 (d, 2H), 7.68 (t, 1H), 7.47 (d, 1H), 4.88 - 4.77 (m, 1H), 3.96 - 3.53 (m, 7H), 3.88 - 3.84 (m, 2H), 1.99 - 1.90 (m, 2H), 1.64 - 1.49 (m, 4H), 1.40 - 1.22 (m, 4H) Resonances obscured by water and DMSO peaks.

Step (c) 1-(dimethylamino)-*N*-{*cis*-4-[1-(4'-{[[2-(dimethylamino)ethyl](methyl)amino]methyl}biphenyl-3-yl)-6-fluoro-2,4-dioxo-1,4-dihydropyrido[2,3-*d*]pyrimidin-3(2*H*)-yl]cyclohexyl}cyclopropanecarboxamide 1-(dimethylamino)-*N*-{*cis*-4-[1-( 4'-{[[2-(dimethylamino)ethyl](methyl)amino]methyl}biphenyl-3-yl)-6-fluoro-2,4-dioxo-1,4-dihydropyrido[2,3-*d*]pyrimidin-3(2*H*)-yl]cyclohexyl}cyclopropanecarboxamide was prepared from the product of step (b) (300mg, 0.53 mmol) using a similar method as example 51 to give the title compound as a white solid (as the di-trifluoroacetate salt) (43 mg).
1H NMR (400 MHz, DMSO) δ 8.59 (s, 1H), 8.41 - 8.27 (m, 1H), 7.91 - 7.68 (m, 4H), 7.69 - 7.59 (m, 1H), 7.59 - 7.50 (m, 2H), 7.47 - 7.37 (m, 1H), 4.92 - 4.72 (m, 1H), 4.25 - 3.93 (m, 1H), 3.93 - 3.79 (m, 1H), 2.81 (s, 6H), 2.09 (s, 3H), 1.99 - 1.85 (m, 2H), 1.67 - 1.46 (m, 4H), 1.43 - 1.13 (m, 4H). Other resonances obscured by DMSO peaks.
[M+H]⁺=656 (MultiMode+)

### Example 111

### N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}-5-methylimidazo[1,2-a]pyridine-2-carboxamide

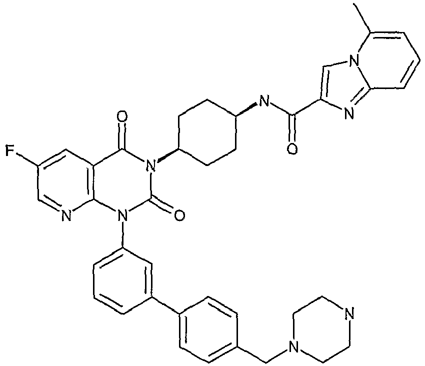

### Step (a) N-{cis-4-[6-fluoro-1-(3-iodophenyl)-2,4-dioxo-1,4-dihydropyrido[2,3-d]pyrimidin-3(2H)-yl]cyclohexyl}-5-methylimidazo[1,2-a]pyridine-2-carboxamide

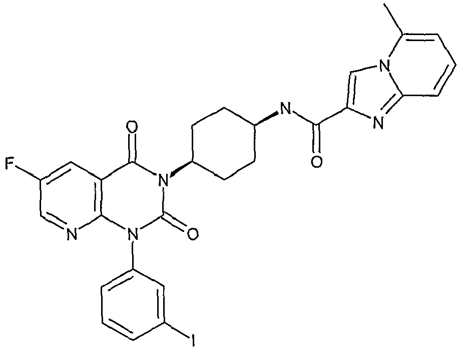

*N*-{*cis*-4-[6-fluoro-1-(3-iodophenyl)-2,4-dioxo-1,4-dihydropyrido[2,3-*d*]pyrimidin-3(2*H*)-yl]cyclohexyl}-5-methylimidazo[1,2-*a*]pyridine-2-carboxamide was prepared from 3-(*cis*-4-aminocyclohexyl)-1-biphenyl-3-yl-6-fluoropyrido[2,3-*d*]pyrimidine-2,4(1*H*,3*H*)-dione (0.820 g, 1.71 mmol) and 5-methylimidazo[1,2-a]pyridine-2-carboxylic acid (0.342 g, 1.94 mmol) using a similar method as example 38 step (b) to give the sub-title compound as a cream-coloured solid (0.823 g).
¹H NMR (400 MHz, DMSO) δ 8.59 (d, 1H), 8.32 (dd, 1H), 8.27 (s, 1H), 7.84 - 7.82 (m, 2H), 7.74 (d, 1H), 7.58 (d, 1H), 7.45 - 7.43 (m, 1H), 7.35 - 7.31 (m, 2H), 6.86 (d, 1H), 4.85 (t, 1H), 4.21 - 4.17 (m, 1H), 2.67 - 2.55 (m, 5H), 2.01 (d, 2H), 1.76 - 1.63 (m, 4H).

### Step (b) N-{cis-4-[6-fluoro-1-(4'-formylbiphenyl-3-yl)-2,4-dioxo-1,4-dihydropyrido[2,3-d]pyrimidin-3(2H)-yl]cyclohexyl}-5-methylimidazo[1,2-a]pyridine-2-carboxamide

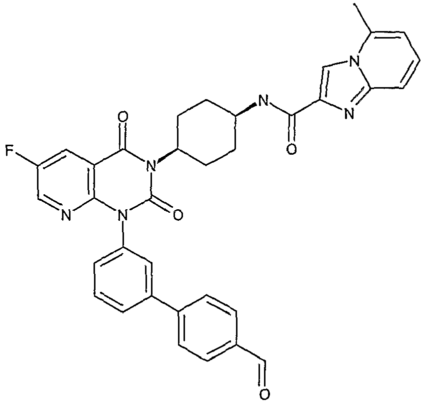

*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}-5-methylimidazo[1,2-*a*]pyridine-2-carboxamide was prepared from the product from step (a) (0.823 g, 1.29 mmol) and 4-formylphenylboronic acid (0.290 g, 1.93 mmol) using a similar method as example 49 step (a) to give the sub-title compound as a cream solid (0.611 g).
¹H NMR (400 MHz, DMSO) δ 10.06 (s, 1H), 8.60 (d, 1H), 8.34 (dd, 1H), 8.26 (s, 1H), 8.02 - 8.00 (m, 2H), 7.94 - 7.91 (m, 2H), 7.91 - 7.87 (m, 2H), 7.74 (d, 1H), 7.67 (t, 1H), 7.55 (d, 1H), 7.49 - 7.47 (m, 1H), 7.33 - 7.29 (m, 1H), 6.85 (d, 1H), 4.89 (t, 1H), 4.21 - 4.17 (m, 1H), 2.68 - 2.58 (m, 5H), 2.02 (d, 2H), 1.77 - 1.66 (m, 4H).

### Step (c) N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}-5-methylimidazo[1,2-a]pyridine-2-carboxamide

The product of step (b) (300 mg, 0.49 mmol) was reacted with tert-butyl piperazine-1-carboxylate (181 mg, 0.97 mmol) using a similar method as example 51 to give an crude oil. This was deprotected using a similar method as example 54 to give the title compound as a white solid (as the di-trifluoroacetate salt) (0.253 g).
¹H NMR (400 MHz, DMSO) δ 8.81 - 8.69 (m, 1H), 8.59 (d, 1H), 8.47 - 8.43 (m, 1H), 8.34 (dd, 1H), 7.95 - 7.89 (m, 1H), 7.80 - 7.79 (m, 1H), 7.74 - 7.71 (m, 3H), 7.65 - 7.58 (m, 2H), 7.52 - 7.40 (m, 3H), 6.99 (d, 1H), 4.88 (t, 1H), 4.19 - 4.15 (m, 1H), 4.01 - 2.60 (m, 15H), 2.05 (d, 2H), 1.77 - 1.65 (m, 4H).
[M+H]⁺=687 (MultiMode+)

The following compounds (Table 4) were prepared in a similar manner as solids from the appropriate aldehyde and amine using the method described above in Example 51.

**Table 4**

| **Example Number** | **Chemistry** | **Name** | **NMR** | **M+H** |
|---|---|---|---|---|
| 112 | 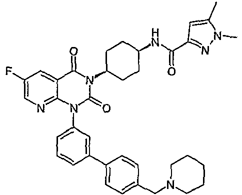 | *N*-{*cis-*4-[6-fluoro-2,4-dioxo-1-[4'-(piperidin-1-ylmethyl)biphenyl-3-yl]-1,4-dihydropyrido[2,3-*d*]pyrimidin-3(2*H*)-yl]cyclohexyl}-1,5-dimethyl-1*H*-pyrazole-3-carboxamide. | ¹H NMR (400 MHz, DMSO) δ 9.52 (s, 1H), 8.59 (d, 1H), 8.34 (dd, 1H), 7.85 - 7.77 (m, 4H), 7.67 - 7.58 (m, 3H), 7.42 (dd, 1H), 7.20 (d, 1H), 6.42 (s, 1H), 4.85 (t, 1H), 4.33 (d, 2H), 4.08 (s, 1H), 3.75 (s, 3H), 2.94 - 2.84 (m, 2H), 2.62 - 2.52 (m, 2H), 2.25 (s, 3H), 1.96 (d, 2H), 1.82 (d, 2H), 1.71 - 1.59 (m, 8H), 1.41 -1.33 (m, 2H). | 650 |
| 113 | 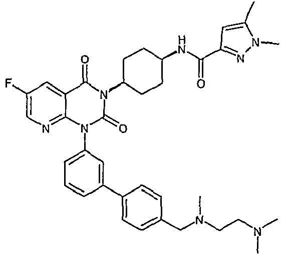 | *N*-{*cis*-4-[1-(4'-{[[2-(dimethylamino)ethyl](methyl)amino ]methyl}biphenyl-3-yl)-6-fluoro-2,4-dioxo-1,4-dihydropyrido[2,3-*d*]pyrimidin-3(2*H*)-yl]cyclohexyl}-1,5-dimethyl-1*H*-pyrazole-3-carboxamide. | ¹H NMR (90°C, 400 MHz, DMSO) δ 8.51 (d, 1H), 8.24 (dd, 1H), 7.76 (d, 1H), 7.70 - 7.66 (m, 3H), 7.60 (t, 1H), 7.47 (d, 2H), 7.37 (d, 1H), 7.11 (d, 1H), 6.39 (s, 1H), 4.92 - 4.82 (m, 1H), 4.12 (s, 2H), 3.80 (s, 1H), 3.73 (s, 3H), 3.26 (t, 2H), 2.88 (t, 2H), 2.77 (d, 6H), 2.63 - 2.52 (m, 2H), 2.34 (s, 3H), 2.25 (s, 3H), 1.96 (d, 2H), 1.73 - 1.61 (m, 4H). | 667 |
| 114 | 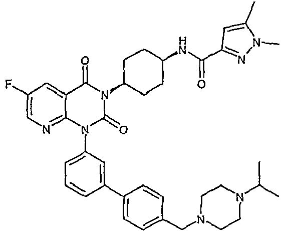 | *N*-{cis-4-[6-fluoro-1-{4'-[(4-isopropylpiperazin-1-yl)methyl]biphenyl-3-yl}-2,4-dioxo-1,4-dihydropyrido[2,3-*d*]pyrimidin-3(2*H*)-yl]cyclohexyl}-1,5-dimethyl-1*H*-pyrazole-3-carboxamide. | ¹H NMR (400 MHz, DMSO) δ 8.59 (d, 1H), 8.33 (dd, 1H), 7.78 (d, 1H), 7.73 (d, 1H), 7.69 (d, 2H), 7.62 (t, 1H), 7.46 (d, 2H), 7.39 (d, 1H), 7.20 (d, 1H), 6.42 (s, 1H), 4.84 (t, 1H), 4.08 (s, 1H), 3.75 (s, 3H), 3.65 - 3.37 (m, 7H), 3.08 - 3.03 (m, 4H), 2.58 - 2.52 (m, 2H), 2.25 (s, 3H), 1.96 (d, 2H), 1.71 - 1.59 (m, 4H), 1.23 (d, 6H). | 693 |
| 115 | 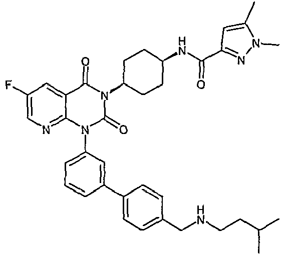 | *N*-{*cis*-4-[6-fluoro-1-(4'-{[(3-methylbutyl)amino]methyl}biphenyl -3-yl)-2,4-dioxo-1,4-dihydropyrido[2,3-*d*]pyrimidin-3(2*H*)-yl]cyclohexyl}-1,5-dimethyl-1*H*-pyrazole-3-carboxamide. | ¹H NMR (400 MHz, DMSO) δ 8.74 (s, 2H), 8.59 (d, 1H), 8.34 (dd, 1H), 7.82 (d, 1H), 7.79 - 7.76 (m, 3H), 7.64 (t, 1H), 7.59 (d, 2H), 7.42 (d, 1H), 7.20 (d, 1H), 6.42 (s, 1H), 4.87 - 4.81 (m, 1H), 4.20 (s, 2H), 4.08 (s, 1H), 3.75 (s, 3H), 2.96 (s, 2H), 2.60 - 2.52 (m, 2H), 2.26 (s, 3H), 1.96 (d, 2H), 1.24 (s, 1H), 1.71 - 1.59 (m, 4H), 1.55 - 1.48 (m, 2H), 0.89 (d, 6H). | 652 |
| 116 | 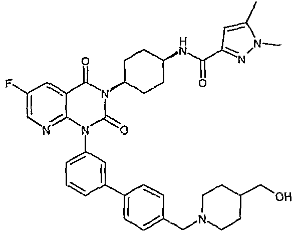 | *N*-{*cis*-4-[6-fluoro-1-(4'-{[4-(hydroxymethyl)piperidin-1-yl]methyl}biphenyl-3-yl)-2,4-dioxo-1,4-dihydropyrido[2,3-*d*]pyrimidin-3(2*H*)-yl]cyclohexyl}-1,5-dimethyl-1*H*-pyrazole-3-carboxamide. | ¹H NMR (400 MHz, DMSO) δ 9.40 (s, 1H), 8.59 (d, 1H), 8.34 (dd, 1H), 7.85 - 7.77 (m, 4H), 7.67 - 7.57 (m, 3H), 7.43 (d, 1H), 7.20 (d, 1H), 6.42 (s, 1H), 4.85 (t, 1H), 4.64 (s, 1H), 4.32 (d, 2H), 4.08 (s, 1H), 3.75 (s, 3H), 3.42 - 3.35 (m, 2H), 3.28 - 3.22 (m, 2H), 2.99 - 2.89 (m, 2H), 2.59 - 2.52 (m, 2H), 2.25 (s, 3H), 1.96 (d, 2H), 1.84 (d, 2H), 1.71 - 1.58 (m, 5H), 1.43 - 1.32 (m, 2H). | 680 |
| 117 | 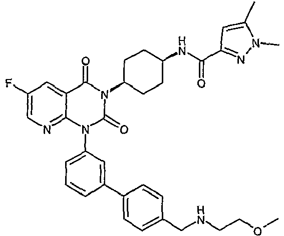 | *N*-{*cis*-4-[6-fluoro-1-(4'-{[(2-methoxyethyl)amino]methyl}biphen yl-3-yl)-2,4-dioxo-1,4-dihydropyrido[2,3-*d*]pyrimidin-3(2*H*)-yl]cyclohexyl}-1,5-dimethyl-1*H*-pyrazole-3-carboxamide. | ¹H NMR (400 MHz, DMSO) δ 8.99 (s, 2H), 8.59 (d, 1H), 8.33 (dd, 1H), 7.82 (d, 1H), 7.78 - 7.75 (m, 3H), 7.66 - 7.58 (m, 3H), 7.42 (dd, 1H), 7.20 (d, 1H), 6.42 (s, 1H), 4.85 (t, 1H), 4.21 (s, 2H), 4.08 (s, 1H), 3.75 (s, 3H), 3.59 (t, 2H), 3.31 (s, 3H), 3.13 - 3.10 (m, 2H), 2.62 - 2.53 (m, 2H), 2.25 (s, 3H), 1.96 (d, 2H), 1.71 - 1.59 (m, 4H). | 640 |
| 118 | 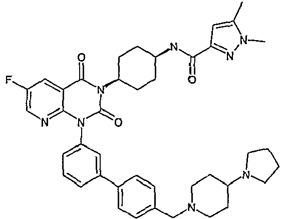 | *N*-{*cis*-4-[6-fluoro-2,4-dioxo-1-{4'-[(4-pyrrolidin-1-ylpiperidin-1-yl)methyl]biphenyl-3-yl}-1,4-dihydropyrido[2,3-*d*]pyrimidin-3(2*H*)-yl]cyclohexyl}-1,5-dimethyl-1*H*-pyrazole-3-carboxamide. | ¹H NMR (400 MHz, DMSO) δ 10.45 - 10.23 (m, 2H), 8.59 (d, 1H), 8.34 (dd, 1H), 7.85 - 7.77 (m, 4H), 7.65 (t, 1H), 7.60 (d, 2H), 7.43 (d, 1H), 7.20 (d, 1H), 6.42 (s, 1H), 4.85 (t, 1H), 4.36 (s, 2H), 4.08 (s, 1H), 3.78 - 3.50 (m, 4H), 3.75 (s, 3H), 3.31 (s, 1H), 3.09-2.93 (m, 4H), 2.62 - 2.52 (m, 2H), 2.31 - 2.27 (m, 2H), 2.25 (s, 3H), 2.03 - 1.81 (m, 8H), 1.71-1.59(m,4H). | 719 |
| 119 | 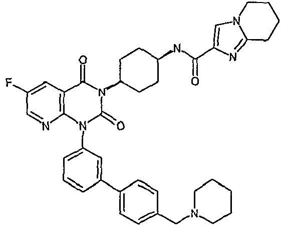 | *N*-{*cis*-4-[6-fluoro-2,4-dioxo-1-[4'-(piperidin-1-ylmethyl)biphenyl-3-yl]-1,4-dihydropyrido[2,3-*d*]pyrimidin-3(2*H*)-yl]cyclohexyl)-5,6,7,8-tetrahydroimidazo[1,2-*a*]pyridine-2-carboxamide | ¹H NMR (300 MHz, DMSO) δ 9.72 (br s, 1H), 8.60 - 8.59 (m, 1H), 8.34 - 8.29 (m, 1H), 8.19 - 8.16 (m, 2H), 7.85 - 7.78 (m, 4H), 7.68 - 7.59 (m, 3H), 7.42 (d, 1H), 4.85 (t, 1H), 4.33 (s, 2H), 4.12 - 4.09 (m, 2H), 4.04 - 3.99 (m, 1H), 3.35 (d, 2H), 2.94 - 2.85 (m, 4H), 2.72 - 2.59 (m, 2H), 2.09 (d, 2H), 1.97 - 1.81 (m, 6H), 1.71 -1.58 (m, 6H), 1.43 - 1.35 (m, 2H). | 676 |
| 120 | 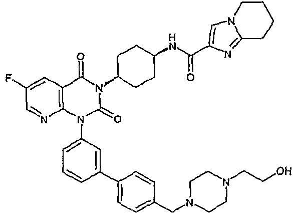 | *N*-{*cis*-4-[6-fluoro-1-(4'-{[4-(2-hydroxyethyl)piperazin-1-yl]methyl}biphenyl-3-yl)-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 8.58 (d, 1H), 8.30 (dd, 1H), 8.23 - 8.21 (m, 2H), 7.82 (d, 1H), 7.76 - 7.73 (m, 3H), 7.64 (t, 1H), 7.56 (d, 2H), 7.40 (d, 1H), 4.85 (t, 1H), 4.17-3.99 (m, 5H), 3.74 (t, 2H), 3.50 - 3.40 (m, 4H), 3.25 - 3.16 (m, 6H), 2.93 (t, 2H), 2.71 - 2.61 (m, 2H), 2.12 - 2.08 (m, 2H), 1.98 - 1.87 (m, 4H), 1.71 - 1.58 (m, 4H). | 721 |
| 121 | 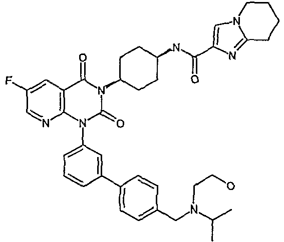 | *N*-{*cis*-4-[6-fluoro-1-(4'-{[(2-hydroxyethyl)(isopropyl)amino]meth yl}biphenyl-3-yl)-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (300 MHz, DMSO) δ 9.21 (br s, 1H), 8.60 - 8.59 (m, 1H), 8.34 - 8.29 (m, 1H), 8.17 - 8.13 (m, 2H), 7.87 - 7.78 (m, 4H), 7.71 - 7.63 (m, 3H), 7.42 (d, 1H), 4.85 (t, 1H), 4.39 (s, 2H), 4.13 - 4.08 (m, 2H), 4.04 - 3.99 (m, 1H), 3.30 - 2.89 (m, 7H), 2.72 - 2.59 (m, 2H), 2.08 (d, 2H), 1.99 - 1.85 (m, 4H), 1.72 - 1.58 (m, 4H), 1.38 - 1.29 (m, 6H). | 694 |
| 122 | 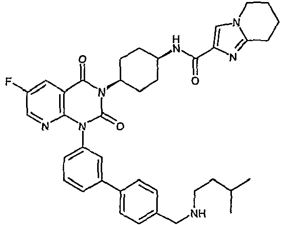 | *N*-{*cis*-4-[6-fluoro-1-(4'-{[(3-methylbutyl)amino]methyl}biphenyl -3-yl)-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 8.78 (br s, 2H), 8.59 (d, 1H), 8.31 (dd, 1H), 8.08 - 7.99 (m, 2H), 7.83 - 7.76 (m, 4H), 7.66 - 7.58 (m, 3H), 7.41 (d, 1H), 4.84 (t, 1H), 4.20 (t, 2H), 4.10 - 4.01 (m, 3H), 2.98 - 2.93 (m, 2H), 2.90 - 2.86 (m, 2H), 2.68 - 2.61 (m, 2H), 2.06 - 2.03 (m, 2H), 1.96 - 1.85 (m, 4H), 1.72 - 1.49 (m, 7H), 0.89 (d, J = 6.4 Hz, 6H). | 678 |
| 123 | 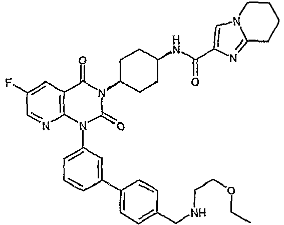 | *N*-{cis-4-[1-(4'-{[(2-ethoxyethyl)amino]methyl}biphenyl-3-yl)-6-fluoro-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 9.08 - 8.91 (m, 2H), 8.59 (d, 1H), 8.31 (dd, 1H), 8.17-8.10 (m, 2H), 7.83 - 7.75 (m, 4H), 7.65 - 7.59 (m, 3H), 7.40 (d, 1H), 4.84 (t, 1H), 4.24 - 4.20 (m, 2H), 4.12 - 4.08 (m, 2H), 4.03 - 4.00 (m, 1H), 3.63 (t, 2H), 3.49 (q, 2H), 3.13 - 3.09 (m, 2H), 2.92 - 2.88 (m, 2H), 2.70 - 2.60 (m, 2H), 2.07 (d, 2H), 1.96 - 1.86 (m, 4H), 1.71 - 1.58 (m, 4H), 1.15 (t, 3H). | 680 |
| 124 | 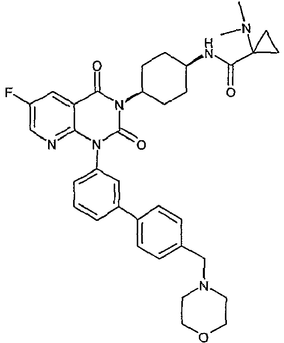 | 1-(dimethylamino)-*N*-{*cis*-4-[6-fluoro-1-[4'-(morpholin-4-ylmethyl)biphenyl-3-yl]-2,4-dioxo-1,4-dihydropyrido[2,3-*d*]pyrimidin-3(2*H*)-yl]cyclohexyl}cyclopropanecarboxa mide. | ¹H NMR (400 MHz, DMSO) δ 8.59 (d, 1H), 8.32 (dd, 1H), 7.87 - 7.74 (m, 4H), 7.65 (t, 1H), 7.60 (d, 2H), 7.42 (d, 1H), 4.81 (t, 1H), 4.39 (s, 2H), 4.08 - 3.91 (m, 2H), 3.92 - 3.81 (m, 2H), 2.03 - 1.85 (m, 2H), 1.67 - 1.46 (m, 4H), 1.48 - 1.16 (m, 3H). Other resonances under the DMSO peaks. | 641 |
| 125 | 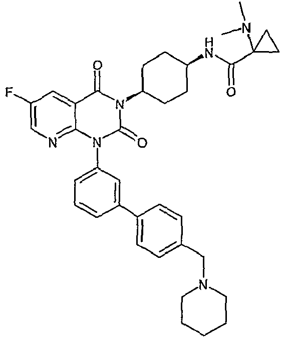 | 1-(dimethylamino)-N-{*cis*-4-[6-fluoro-2,4-dioxo-1-[4'-(piperidin-1-ylmethyl)biphenyl-3-yl]-1,4-dihydropyrido[2,3-*d*]pyrimidin-3(2*H*)-yl]cyclohexyl}cyclopropanecarboxa mide. | ¹H NMR (400 MHz, DMSO) δ 8.59 (d, 1H), 8.32 (dd, 1H), 7.87 - 7.74 (m, 4H), 7.64 (t, 1H), 7.60 (d, 2H), 7.42 (d, 1H), 4.89 - 4.75 (m, 1H), 4.33 (s, 2H), 3.92 - 3.84 (m, 1H), 2.98 - 2.83 (m, 2H), 2.00 - 1.88 (m, 2H), 1.88 - 1.76 (m, 2H), 1.75 - 1.48 (m, 8H), 1.46 - 1.16 (m, 5H). other resonances obscured by the DMSO peaks | 639 |
| 126 | 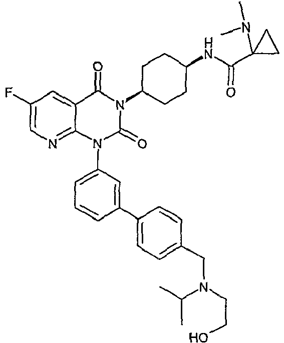 | 1-(dimethylamino)-*N*-{*cis*-4-[6-fluoro-1-(4'-{[(2-hydroxyethyl)(isopropyl)amino]meth yl}biphenyl-3-yl)-2,4-dioxo-1,4-dihydropyrido[2,3-*d*]pyrimidin-3(2*H*)-yl]cyclohexyl}cyclopropanecarboxa mide. | ¹H NMR (400z, DMSO) δ 9.13 (s, 1H), 8.59 (d, 1H), 8.32 (dd, 1H), 7.84 (d, 1H), 7.82-7.76 (m, 2H), 7.71 - 7.65 (m, 2H), 7.64 (d, 1H), 7.42 (d, 1H), 4.87 - 4.76 (m, 1H), 4.43 - 4.32 (m, 2H), 3.09 - 2.98 (m, H), 2.02 - 1.90 (m, 2H), 1.66 - 1.49 (m, 4H), 1.36 (d, 3H), 1.31 (d, 3H). Other resonances obscured by DMSO and water peaks | 657 |
| 127 | 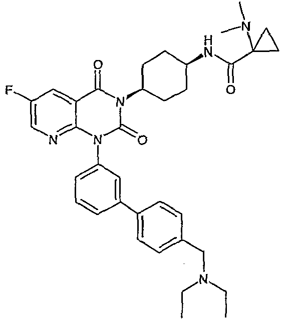 | *N*-{*cis*-4-[1-{4'-[(diethylamino)methyl]biphenyl-3-yl}-6-fluoro-2,4-dioxo-1,4-dihydropyrido[2,3-*d*]pyrimidin-3(2*H*)-yl]cyclohexyl}-1-(dimethylamino)cyclopropanecarbox amide. | ¹H NMR (400 MHz, DMSO) δ 9.64 - 9.41 (m, 1H), 8.60 (s, 1H), 8.36 - 8.28 (m, 1H), 7.89 - 7.72 (m, 4H), 7.69 - 7.58 (m, 3H), 7.46 - 7.37 (m, 1H), 4.90 - 4.74 (m, 1H), 4.37 (s, 2H), 3.88 (s, 1H), 3.17 - 3.03 (m, 4H), 2.70 - 2.54 (m, 3H), 2.01 - 1.89 (m, 2H), 1.65 - 1.50 (m, 4H), 1.40 - 1.29 (m, 1H), 1.29 - 1.16 (m, 5H), 1.15 - 1.02 (m, 8H). Other signals obscured by DMSO | 627 |
| 128 | 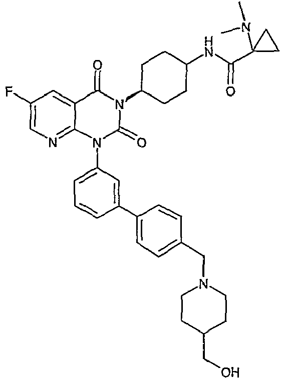 | 1-(dimethylamino)-*N*-{4-[6-fluoro-1-(4'-{[4-(hydroxymethyl)piperidin-1-yl]methyl}biphenyl-3-yl)-2,4-dioxo-1,4-dihydropyrido[2,3-*d*]pyrimidin-3(2*H*)-yl]cyclohexyl}cyclopropanecarboxa mide. | ¹H NMR (400 MHz, DMSO) δ 8.62 - 8.56 (m, 1H), 8.30 (d, 1H), 8.08 (d, 1H), 7.77 (d, 1H), 7.71 (s, 1H), 7.62 (t, 3H), 7.38 (t, 3H), 4.89 - 4.78 (m, 1H), 4.43 - 4.35 (m, 1H), 3.98 - 3.89 (m, 1H), 3.58 - 3.45 (m, 6H), 2.91 (q, 2H), 2.87 - 2.78 (m, 2H), 1.99 - 1.88 (m, 2H), 1.86 - 1.77 (m, 2H), 1.77 - 1.73 (m, 1H), 1.67 - 1.56 (m, 4H), 1.28 - 1.21 (m, 4H), 1.20 - 1.11 (m, 5H), 0.94 (s, 4H). | 669 |
| 129 | 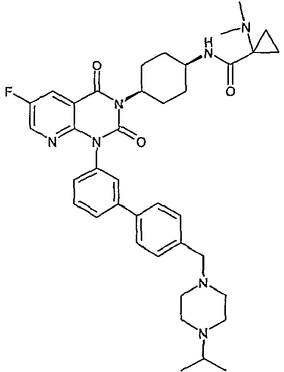 | 1-(dimethylamino)-*N-*{*cis*-4-[6-fluoro-1-{4'-[(4-isopropylpiperazin-1-yl)methyl]biphenyl-3-yl}-2,4-dioxo-1,4-dihydropyrido[2,3-*d*]pyrimidin-3(2*H*)-yl]cyclohexyl}cyclopropanecarboxa mide. | ¹H NMR (400 MHz, DMSO) δ 8.59 (d, 1H), 8.31 (dd, 1H), 7.80 (d, 1H), 7.75 - 7.68 (m, 3H), 7.63 (t, 1H), 7.54 - 7.47 (m, 2H), 7.39 (d, 1H), 4.81 (t, 1H), 4.03 - 3.91 (m, 1H), 3.87 - 3.80 (m, 1H), 3.55 - 3.40 (m, 2H), 3.33 - 3.03 (m, 3H), 2.86 - 2.70 (m, 7H), 1.99 (d, 2H), 1.62 - 1.38 (m, 8H), 1.24 (d, 6H). Other resonances obscured by DMSO and water peaks | 682 |
| 130 | 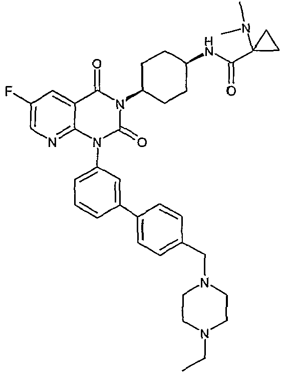 | 1-(dimethylamino)-*N*-{*cis*-4-[1-{4'-[(4-ethylpiperazin-1-yl)methyl]biphenyl-3-yl}-6-fluoro-2,4-dioxo-1,4-dihydropyrido[2,3-*d*]pyrimidin-3(2*H*)-yl]cyclohexyl}cyclopropanecarboxa mide. | ¹H NMR (400 MHz, DMSO) δ 8.59 (d, 1H), 8.31 (dd, 1H), 7.79 (d, 1H), 7.74 - 7.67 (m, 3H), 7.62 (t, 1H), 7.47 (d, 2H), 7.38 (d, 1H), 4.86 - 4.75 (m, 1H), 3.89 - 3.76 (m, 2H), 2.03 - 1.92 (m, 2H), 1.63 - 1.35 (m, 6H), 1.19 (t, 3H). Other resonances obscured by water and DMSO peaks | 668 |
| 131 | 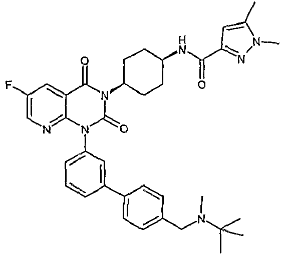 | *N*-{*cis*-4-[1-(4'-{[*tert-*butyl(methyl)amino]methyl}bipheny 1-3-yl)-6-fluoro-2,4-dioxo-1,4-dihydropyrido[2,3-*d*]pyrimidin-3(2*H*)-yl]cyclohexyl}-1,5-dimethyl-1*H*-pyrazole-3-carboxamide. | ¹H NMR (400 MHz, DMSO) δ 9.04 (s, 1H), 8.59 (d, 1H), 8.34 (dd, 1H), 7.86 - 7.78 (m, 4H), 7.67 - 7.61 (m, 3H), 7.43 (d, 1H), 7.20 (d, 1H), 6.42 (s, 1H), 4.89 - 4.80 (m, 1H), 4.66 (d, 1H), 4.08 (s, 1H), 3.99 - 3.93 (m, 1H), 3.75 (s, 3H), 2.61 - 2.54 (m, 5H), 2.25 (s, 3H), 1.96 (d, 2H), 1.71 - 1.59 (m, 4H), 1.45 (s, 9H). | 652 |
| 132 | 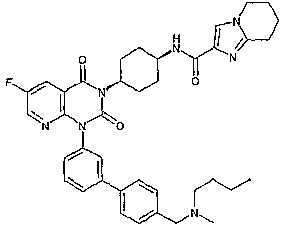 | *N*-{*cis*-4-[1-(4'-{[butyl(methyl)amino]methyl}biphe nyl-3-yl)-6-fluoro-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 8.59 (d, 1H), 8.31 (dd, 1H), 8.17 - 8.11 (m, 2H), 7.84 (d, 1H), 7.80 - 7.79 (m, 3H), 7.66 - 7.60 (m, 3H), 7.41 (d, 1H), 4.84 (t, 1H), 4.45 - 4.41 (m, 1H), 4.29 - 4.24 (m, 1H), 4.12 - 4.08 (m, 2H), 4.03 - 3.99 (m, 1H), 3.15 - 2.88 (m, 4H), 2.70 - 2.60 (m, 5H), 2.07 (d, 2H), 1.96 - 1.85 (m, 4H), 1.71 - 1.58 (m, 6H), 1.31 (sextet, 2H), 0.90 (t, 3H). | 678 |
| 133 | 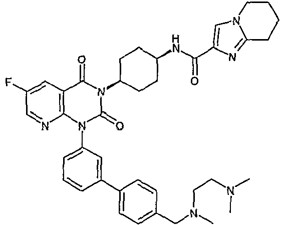 | N-{*cis*-*4*-[1-(4'-{[[2-(dimethylamino)ethyl](methyl)amino ]methyllbiphenyl-3-yl)-6-fluoro-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 8.59 (d, 1H), 8.31 (dd, 1H), 8.16 - 8.09 (m, 2H), 7.82 (d, J = 7.9 Hz, 1H), 7.78 - 7.74 (m, 3H), 7.64 (t, 1H), 7.60 - 7.55 (m, 2H), 7.40 (d, 1H), 4.84 (t, 1H), 4.12 - 4.08 (m, 2H), 4.03 - 4.00 (m, 1H), 3.84 - 3.00 (m, 9H), 2.92 - 2.88 (m, 2H), 2.84 - 2.81 (m, 4H), 2.68 - 2.58 (m, 4H), 2.07 (d, 2H), 1.97 - 1.85 (m, 4H), 1.71 - 1.58 (m, 4H). | 693 |
| 134 | 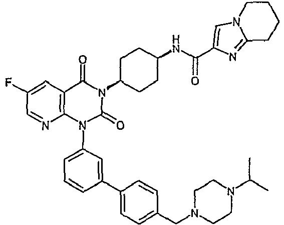 | *N*-{*cis*-4-[6-fluoro-1-{4'-[(4-isopropylpiperazin-1-yl)methyl]biphenyl-3-yl}-2,4-dioxo-1,4-dihydropyrido[2,3-*d*]pyrimidin-3(2*H*)-yl]cyc1ohexyl}-5,6,7,8-tetrahydroimidazo[1,2-*a*]pyridine-2-carboxamide. | ¹H NMR (400 MHz, DMSO) d 8.59 (d, 1H), 8.31 (dd, 1H), 8.16 - 8.07 (m, 2H), 7.79 (d, 1H), 7.72 - 7.67 (m, 3H), 7.62 (t, 1H), 7.49 - 7.43 (m, 2H), 7.38 (d, J = 7.9 Hz, 1H), 4.84 (t, 1H), 4.11 - 4.07 (m, 2H), 4.03 - 3.99 (m, 1H), 3.80 - 3.02 (m, 11H), 2.92 - 2.88 (m, 2H), 2.70 - 2.59 (m, 2H), 2.06 (d, 2H), 1.96 - 1.85 (m, 4H), 1.71 - 1.57 (m, 4H), 1.23 (d, 6H). | 719 |
| 135 | 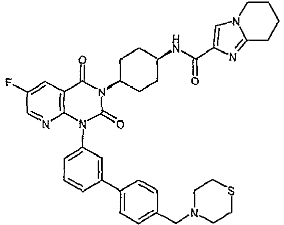 | *N*-{*cis*-4-[6-fluoro-2,4-dioxo-1-[4'-(thiomorpholin-4-ylmethyl)biphenyl-3-yl]-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 8.59 (d, 1H), 8.31 (dd, 1H), 8.20 - 8.14 (m, 2H), 7.84 (d, 1H), 7.80 - 7.78 (m, 3H), 7.64 (t, 1H), 7.61 (d, 2H), 7.42 (d, 1H), 4.85 (t, 1H), 4.41 (s, 2H), 4.12 - 4.08 (m, 2H), 4.03 - 3.99 (m, 1H), 3.56 - 3.47 (m, 8H), 2.94 - 2.88 (m, 2H), 2.70 - 2.60 (m, 2H), 2.08 (d, 2H), 1.98 - 1.85 (m, 4H), 1.71 - 1.57 (m, 4H). | 694 |
| 136 | 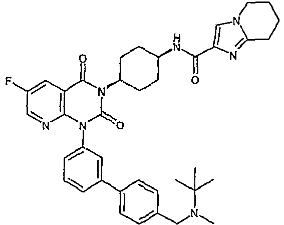 | *N*-{*cis*-4-[1-(4'{[*tert-*butyl(methyl)amino]methyl}bipheny 1-3-yl)-6-fluoro-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 8.59 (d, 1H), 8.32 (dd, 1H), 8.13 - 8.05 (m, 2H), 7.85 - 7.79 (m, 4H), 7.67 - 7.62 (m, 3H), 7.42 (d, 1H), 4.84 (t, 1H), 4.65 (d, 1H), 4.11 - 4.07 (m, 2H), 4.03 - 4.00 (m, 1H), 3.96 (dd, 1H), 2.91 - 2.87 (m, 2H), 2.68 - 2.61 (m, 2H), 2.58 - 2.57 (m, 3H), 2.09 - 2.03 (m, 2H), 1.96 - 1.85 (m, 4H), 1.71 - 1.56 (m, 4H), 1.46 (s, 9H). | 678 |
| 137 | 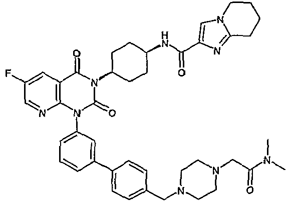 | *N*-{*cis*-4-[1-[4'-({4-[2-(dimethylamino)-2-oxoethyl]piperazin-1-yl}methyl)biphenyl-3-yl]-6-fluoro-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 8.58 (d, 1H), 8.31 (dd, 1H), 8.19 - 8.13 (m, 2H), 7.80 (d, 2H), 7.75 - 7.70 (m, 2H), 7.63 (t, 1H), 7.53 - 7.48 (m, 2H), 7.39 (d, 1H), 4.84 (t, 1H), 4.12 - 3.99 (m, 3H), 3.94 - 3.01 (m, 12H), 2.94 - 2.87 (m, 2H), 2.93 (s, 3H), 2.88 (s, 3H), 2.70 - 2.59 (m, 2H), 2.07 (d, 2H), 1.97 - 1.85 (m, 4H), 1.71 - 1.57 (m, 4H), | 762 |
| 138 | 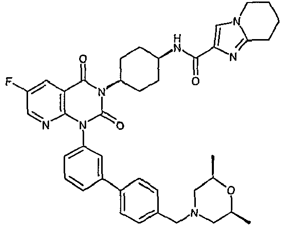 | *N*-{*cis*-4-[1-(4'-{[(2*R*,6*S*)-2,6-dimethylmorpholin-4-yl]methyl}biphenyl-3-yl)-6-fluoro-2,4-dioxo-1,4-dihydropyrido[2,3-*d*]pyrimidin-3(2*H*)-yl]cyclohexyl}-5,6,7,8-tetrahydroimidazo[1,2-*a*]pyridine-2-carboxamide. | ¹H NMR (400 MHz, DMSO) δ 8.59 (d, 1H), 8.32 (dd, 1H), 8.08 - 7.98 (m, 2H), 7.85 - 7.77 (m, 4H), 7.65 (t, 1H), 7.60 (d, 2H), 7.42 (d, 1H), 4.84 (t, 1H), 4.35 (s, 2H), 4.10 - 4.01 (m, 3H), 3.83 - 3.76 (m, 2H), 3.32 - 3.24 (m, 2H), 2.89 - 2.85 (m, 2H), 2.73 - 2.61 (m, 4H), 2.04 (d, 2H), 1.96 - 1.84 (m, 4H), 1.71 - 1.58 (m, 4H), 1.13 (d, 6H). | 706 |

The starting materials for the Examples below are either commercially available or readily prepared using standard methods by those skilled in the art, from known starting materials or are described in earlier examples above.

Powder X-ray diffraction (XRPD) was recorded with a PANalytical CubiX PRO (wavelength of X-rays 1.5418 Å Cu source, Voltage 45kV, filament emission 40 mA). Samples were scanned from 2-40° 2q using a 0.02° step width and a 100 second time count using an X'celerator detector (active length 2.54° 2q)

### Example 139

### 6-fluoro-N-{cis-4-[6-fluoro-2,4-dioxo-1-[4'-(piperazin-1-ylmethyl)biphenyl-3-yl]-1,4-dihydropyrido [2,3-d]pyrimidin-3(2H)-yl]cyclohexyl}imidazo[1,2-a]pyridine-2-carboxamide as a mono methanesulfonic acid salt

To a solution of 6-fluoro-*N*-{*cis*-4-[6-fluoro-2,4-dioxo-1-[4'-(piperazin-1-ylmethyl)biphenyl-3-yl]-1,4-dihydropyrido [2,3-*d*]pyrimidin-3(2*H*)-yllcyclohexyl}imidazo[1,2-*a*]pyridine-2-carboxamide (0.341 g, 0.49 mmol) in EtOH (10 mL) warmed to 50 °C was added methanesulfonic acid (0.032 mL, 0.49 mmol). The mixture was allowed to cool to room temperature overnight. The solid, which precipitated, was isolated by filtration to give a white solid. This was then dried overnight at 40 °C under vacuum to give the title compound (214 mg)

Elemental analysis: Found: C, 55.47; H, 5.58; N, 13.31.
C₃₈H₃₆F₂N₈O₃.1.35CH₃SO₃H.1.8H₂O requires C, 55.47; H, 5.32; N, 13.14%

### Example 140

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a dimethanesulfonic acid salt

To a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.8 g, 1.16 mmol) in EtOH (25 mL) warmed to 50 °C was added methanesulfonic acid (0.075 mL, 1.16 mmol). The mixture was allowed to cool to room temperature over 1 hour. The solid, which precipitated, was isolated by filtration, dried over the weekend under vacuum at 40 °C to give the title compound (470 mg)

Elemental analysis: Found: C, 52.62; H, 5.37; N, 12.54.
C₃₈H₃₆F₂N₈O₃.1.99CH₃SO₃H.1.51H₂O requires C, 52.83; H, 5.21; N, 12.32%

### Example 141

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a tri-methanesulfonic acid salt

To a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.8 g, 1.16 mmol) in ethanol (25 mL) warmed to 50 °C was added Methanesulfonic acid (0.150 mL, 2.32 mmol) The mixture was allowed to cool to room temperature over 1 hour but no solid had precipitated. The mixture was stirred over the 48 hours and a solid had precipitated after this time. This was isolated by filtration and dried overnight at 40 °C under vacuum to give the title compound (620 mg).

Elemental analysis: Found: C, 49.29; H, 5.21; N, 11.46.
C₃₈H₃₆F₂N₈O₃.2.95CH₄O₃S.1.1H₂O requires C, 49.48; H, 5.07; N, 11.27%

### Example 142

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3 (2H)-yl] cyclohexyl}imidazo[1,2-a]pyridine-2-carboxamide as a benzoic acid salt

To a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.8 g, 1.16 mmol) in EtOH (25 mL) warmed to 50 °C was added benzoic acid (0.141 g, 1.16 mmol). The solution was allowed to stir at room temperature overnight but no solid had precipitated. The ethanol was the evaporated under vacuum to give the title compound (940 mg)

### Example 143

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a mono 1,2-Ethanedisulfonic acid salt

To a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.72 mmol) in EtOH (10 mL) warmed to 50 °C was added 1,2-ethanedisulfonic acid (0.138 g, 0.72 mmol). The mixture was allowed to cool to room temperature over 1 hour and then stirred at room temperature for 48 hours The solid which precipitated was isolated by filtration and dried under vacuum at 40 °C to give the title compound (480 mg)

Elemental analysis: Found: C, 53.56; H, 5.29; N, 11.96.
C₃₈H₃₆F₂N₈O₃.0.9C₂H₆O₆S₂.1.7H₂O.0.8Ethanol requires C, 53.5; H, 5.38; N, 12.06%

### Example 144

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a hemi-1,2-Ethanedisulfonic acid salt

To a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.72 mmol) in EtOH (10 mL) warmed to 50 °C was added 1,2-ethanedisulfonic acid (0.069 g, 0.36 mmol). The mixture was allowed to cool to room temperature over 1 hour and then stirred at room temperature for 48 hours. The solid, which precipitated, was isolated by filtration and dried under vacuum at 40 °C to give the title compound (440 mg)

Elemental analysis: Found: C, 58.11; H, 5.58; N, 14.2.
C₃₈H₃₆F₂N₈O₃.0.45C₂H₆O₆S₂.1.7H₂O requires C, 57.9; H, 5.26; N, 13.89%

### Example 145

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3-(2H)-yl]cyclohexyl}imidazo[1,2-a]pyridine-2-carboxamide as a 1-hydroxy-2-naphthoic acid salt

A solution of 1-hydroxy-2-naphthoic acid (0.027 g, 0.14 mmol) dissolved in 1,2-dimethoxyethane (2 mL) was added to a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.1 g, 0.14 mmol) in 1,2-dimethoxyethane (2 mL) and the solution allowed to stir for 16 hours. The resulting precipitate was isolated by filtration and dried to give the title compound (0.089 g) as crystalline form A.

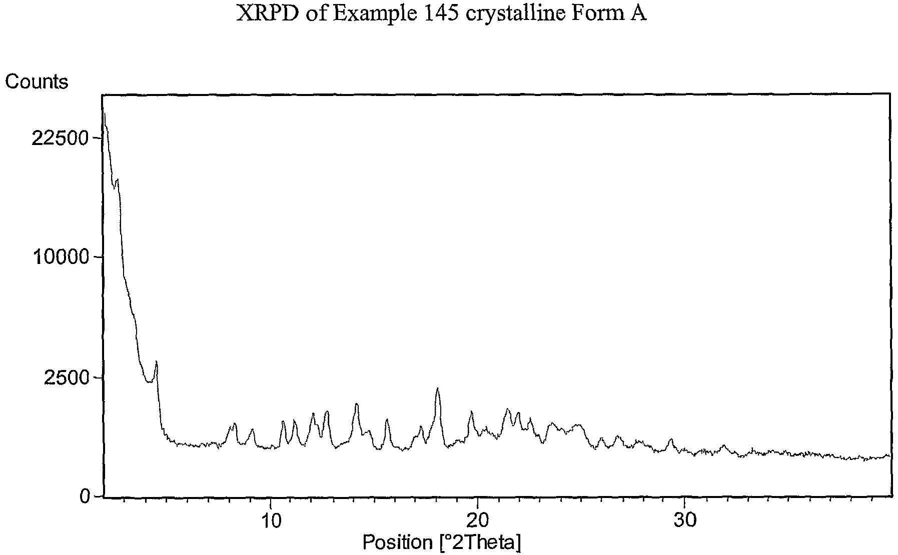

### Example 146

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a DL-mandelic acid salt

A solution of DL-mandelic acid (0.033 g, 0.22 mmol) dissolved in 1,2-dimethoxyethane (3 mL) was added to a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.15 g, 0.22 mmol) in 1,2-dimethoxyethane (3 mL) and the solution allowed to stir for 72 hours. The resulting precipitate was isolated by filtration and dried to give the title compound as a colourless solid (96 mg).

### Example 147

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}iinidazo[1,2-a]pyridine-2-carboxamide as a L-tartaric acid salt

A solution of L-tartaric acid (0.043 g, 0.29 mmol) dissolved in methanol (2 mL) was added to a solution of 6-fluoro-*N-*{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.20 g, 0.29 mmol) in methanol (2 mL). The resultant suspension was heated to boiling then allowed to cool and stir for 16 hours. The resulting precipitate was isolated by filtration and dried to give the title compound as a colourless solid (154 mg).

### Example 148

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a para-toleunesulfonic acid salt

A solution of para-toleunesulfonic acid (0.05 g, 0.29 mmol) dissolved in 1,2-dimethoxyethane (2 mL) was added to a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.20 g, 0.29 mmol) in 1,2-dimethoxyethane (2 mL). The resultant suspension was heated to boiling then allowed to cool and stir for 16 hours. The resulting precipitate was isolated by filtration and dried to give the title compound as a colourless solid (167 mg).

### Example 149

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a nicotinic acid salt

A solution of nicotinic acid (0.036 g, 0.29 mmol) dissolved in dichloromethane (2 mL) and methanol (0.1 mL) was added to a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl)imidazo[1,2-*a*]pyridine-2-carboxamide (0.20 g, 0.29 mmol) in dichloromethane (2 mL). The resultant suspension was allowed to stir for 16 hours then concentrated to give the title compound as a colourless solid (171 mg).

### Example 150

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as an adipic acid salt

A solution of adipic acid (0.042 g, 0.29 mmol) dissolved in 1,2-dimethoxyethane (2 mL) was added to a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.20 g, 0.29 mmol) in 1,2-dimethoxyethane (2 mL). The resultant suspension was allowed to stir for 48 hours then isolated by filtration to give the title compound as a colourless solid (143 mg).

### Example 151

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a tri-hydrochloric acid salt

Concentrated hydrochloric acid (2.2 mL) were added to a solution of 6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (1.6 g, 2.32 mmol) was dissolved in ethanol (20 mL). The resultant suspension was diluted with further ethanol (750 mL) and recrystallised in this solvent before being allowed to slurry 48 hours. The solid precipitate was then collected by filtration, washed with ethanol (200 mL), and dried in vacuo to give the title compound as a colourless solid (1.45 g).
¹H NMR (400 MHz, DMSO-d6) δ 9.89 - 9.59 (m, 2H), 8.84 (dd, 1H), 8.60 (d, 1H), 8.42 (s, 1H), 8.34 (dd, 1H), 7.86 - 7.59 (m, 9H), 7.54 - 7.38 (m, 2H), 4.88 (t, 1H), 4.51 - 4.27 (m, 2H), 4.16 (s, 1H), 3.97 - 3.34 (m, 8H), 2.69 - 2.55 (m, 2H), 2.02 (d, 2H), 1.79 - 1.60 (m, 4H).

Chloride Ion analysis: Found: Cl, 12.83% C₃₈H₃₆F₂N₈O₃.3HCl requires Cl,13.20%

### Example 152

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a sulfuric acid salt.

6-fluoro-*N*-{*cis*-4-[6-fluoro-2,4-dioxo-1-[4'-(piperazin-1-ylmethyl)biphenyl-3-yl]-1,4-dihydropyrido[2,3-d]pyrimidin-3(2*H*)-yl]cyclohexyl}imidazo[1,2-*a*]pyridine-2-carboxamide (0.5 g, 0.72 mmol) was dissolved with heating in ethanol (7 mL) and the solution allowed to cool to room temperature. Excess sulfuric acid (50%) (15 drops) was added and the mixture was stirred at room temperature overnight. The precipitate was removed by filtration and dried to give the title compound as a white solid (0.35 g).

### Example 153

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a mono benzenesulfonic acid salt.

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.3 g, 0.43 mmol) was dissolved with heating in ethanol (3 mL) and allowed to cool to room temperature. Benzenesulfonic acid (0.069 g, 0.43 mmol) was added and the mixture was stirred at room temperature for 2 days. The solid was collected by filtration and dried to give the title compound as a white solid (0.26 g).
¹H NMR (400 MHz, DMSO-d6) δ 8.79 (1H, dd), 8.59 (1H, d), 8.49 (1H, s), 8.37 (1H, s), 8.34 (1H, dd), 7.79 - 7.71 (3H, m), 7.68 - 7.63 (3H, m), 7.62 - 7.58 (3H, m), 7.47 - 7.37 (4H, m), 7.34 - 7.28 (2H, m), 4.87 (1H, t), 4.20 (1H, s), 3.59 (2H, s), 3.30 - 3.29 (2H, s) 3.10 - 3.07 (4H, m), 2.59 - 2.54 (4H, m), 2.04 - 1.96 (2H, m), 1.76 - 1.62 (4H, m)

### Example 154

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 2,5-dichlorobezenesulfonic acid salt.

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.72 mmol) was dissolved with heating in ethanol (3 mL) and allowed to cool to room temperature. 2,5-Dichlorobenzenesulfonic acid (0.164 g, 0.72 mmol) was added and the mixture was stirred at room temperature for 48 hours. The solid was collected by filtration and dried to give the title compound as a white solid (0.289 g).

### Example 155

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a mono malonic acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.3 g, 0.43 mmol) was dissolved with heating in ethanol (3 mL) and allowed to cool to room temperature. Malonic acid (0.045 g, 0.43 mmol) was added and the mixture was stirred at room temperature for 14 days. The solid was collected by filtration and dried to give the title compound as a white solid (0.192 g).

¹H NMR (400 MHz, DMSO-d6) δ 8.81 - 8.77 (1H, m), 8.59 (1H, d), 8.37 (1H, s), 8.34 (1H, dd), 7.79 - 7.70 (3H, m), 7.68 - 7.58 (4H, m), 7.47 - 7.36 (4H, m), 4.87 (1H, t), 4.20 (1H, s), 3.60 (2H, s), 3.07 (4H, d), 2.71 (2H, s), 2.63 - 2.54 (4H, m), 2.49 - 2.45 (4H, m), 2.03 - 1.96 (2H, m), 1.77 - 1.61 (4H, m)

### Example 156

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a tri-2,5-dichlorobenzenesulfonic acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.72 mmol) was dissolved with heating in ethanol (20 mL). Whilst still warm, a solution of 2,5-dichlorobenzenesulfonic acid (0.562 g, 2.48 mmol) in ethanol (5 mL) was added and the mixture was stirred at room temperature overnight. The solid was collected by filtration and dried to give the title compound as a white solid.

¹H NMR (400 MHz, DMSO-d6) δ 8.93 (1H, s), 8.78 (1H, s,), 8.59 (1H, d), 8.53 (1H, s), 8.34 (1H, dd), 7.96 - 7.92 (1H, m), 7.84 (2H, dd), 7.82 - 7.76 (5H, m), 7.68 - 7.58 (5H, m), 7.45 - 7.3 (6H, m), 4.88 (1H, t), 4.18 (1H, s), 4.60-3.93 (12H, m), 2.68 - 2.58 (2H, m), 2.08 - 2.01 (2H, m), 1.77 - 1.61 (4H, m)

### Example 157

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a di-2,5-Dichlorobenzenesulfonic acid

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.72 mmol) was dissolved with heating in ethanol (20 mL). Whilst still warm, a solution of 2,5-dichlorobenzenesulfonic acid (0.562 g, 2.48 mmol) in ethanol (5 mL) was added and the mixture was stirred at room temperature overnight. The solid was collected by filtration and dried. Ethanol (50 mL) was added and the suspension stirred at room temperature overnight to give the title compound as a white solid.
¹H NMR (400 MHz, DMSO-d6) δ d 8.87 (1H, s), 8.80 (1H, s), 8.59 (1H, d), 8.46 (1H, s), 8.34 (1H, dd), 7.85 - 7.73 (7H, m), 7.68 - 7.50 (4H, m), 7.45 - 7.37 (5H, m), 4.88 (1H, t), 4.20 (1H, s), 3.92-3.59 (12H, m) 2.69 - 2.55 (2H, m), 2.07 - 2.00 (2H, m), 1.77 - 1.61 (4H, m)

### Example 158

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a stearic acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (500mg, 0.724mmol) was dissolved in MeOH (14ml) with gentle warming and stearic acid (453 mg, 1.59 mmol) was added and warming was continued until everything was in solution. The mixture was left to cool to room temperature. The precipitated solid was collected, washed with MeOH, and air dried, affording the title compound as a solid.

### Example 159

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 2,4,6-trimethylbenzenesulfonic acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (400 mg, 0.58 mmol) and 2,4,6-trimethylbenzenesulfonic acid (116 mg, 0.58 mmol) were mixed in THF (10ml), warmed to effect solution, then stirred 10 min then evaporated to dryness affording the title compound as a solid.

### Example 160

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 2,4,6-trimethylbenzenesulfonic acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (400 mg, 0.58 mmol) and 2,4,6-trimethylbenzenesulfonic acid (232 mg, 1.16 mmol) were mixed in THF (10ml), MeOH (5 ml) was added to attain solution, then stirred 10 min and then evaporated to dryness affording the title compound as a solid.

### Example 161

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H-yl]cyclohexyl}imidazo[1,2-a]pyridine-2-carboxamide as a 2,4,6-trimethylbenzenesulfonic acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl imidazo[1,2-*a*]pyridine-2-carboxamide (400 mg, 0.58 mmol) and 2,4,6-trimethylbenzenesulfonic acid (348 mg, 1.74 mmol) were mixed in THF (10ml), MeOH (5 ml) was added, warmed until in solution then stirred 10 min then evaporated to dryness affording the title compound as a solid.

### Example 162

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H-yl]cyclohexyl}imidazo[1,2-a]pyridine-2-carboxamide as a tri-phosphoric acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (500mg, 0.724mmol) was dissolved in MeOH (14ml) with gentle warming and phosphoric acid (85 wt%, 14.7M, 0.25ml, 3.6mmol) was added. The mixture was stirred and a heavy white precipitate appeared. Stirring was continued for 4 days at room temperature and the solid was filtered off, washed with a little MeOH and air dried affording the title compound as a white powder.

Elemental analysis: Found: C, 45.48; H, 4.97; N, 11.25%; C₃₈H₃₆F₂N_{B}O₃.3.19H₃PO₄ requires C, 45.49; H, 4.58; N, 11.17%

### Example 163

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl} imidazo[1,2-a]pyridine-2-carboxamide as a phosphoric acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (230 mg, 0.33 mmol) was dissolved in methanol (10 mL) and was treated with 1M phosphoric acid (0.11ml, one third equiv) and the mixture was left to evaporate, affording the title compound as a white solid.

### Example 164

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a phosphoric acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (230 mg, 0.33 mmol) was dissolved in methanol (10 mL) and was treated with 1M phosphoric acid (0.33ml, 1 equiv) and was left to evaporate, affording the title compound as a white solid.

### Example 165

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a phosphoric acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (230 mg, 0.33 mmol) was dissolved in methanol (10 mL) and was treated with 1M phosphoric acid (0.66ml, 2 equiv) and was left to evaporate, affording the title compound as a white solid.

### Example 166

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 1.5 eq citric acid salt

6-fluoro-*N*-{*cis*-4-[6-fluoro-2,4-dioxo-1-[4'-(piperazin-1-ylmethyl)biphenyl-3-yl]-1,4-dihydropyrido [2,3-*d*]pyrimidin-3(2*H*)-yl]cyclohexyl}imidazo[1,2-*a*]pyridine-2-carboxamide (0.501 g, 0.73 mmol) was suspended in ethanol (16 ml) and warmed to 60 °C. After 30 minutes further ethanol (10 mL) was added. After 15 min, the solution was decanted from the remaining sticky solid into a separate flask and heated at 60 °C. Citric acid (0.139 g, 0.73 mmol) was added which led to a precipitate which gradually dissolved to leave a white suspension and a yellow gummy solid. The suspension was decanted and allowed to cool to room temperature, then concentrated to give the title compound as a cream-coloured solid (0.390 g).

Elemental analysis: Found: C, 55.19; H, 5.31; N, 10.82%. C₃₈H₃₆F₂N₈O₃.1.50C₆H₈O₇. 2.55H₂O requires C, 55.08; H, 5.22; N, 10.93%.

### Example 167

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a di-citric acid salt

6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide (0.506 g, 0.73 mmol) was suspended in ethanol (5 ml) and dichloromethane (2 ml) added. The solution was added to ethanol (20 ml) at 60 °C then a solution of citric acid (0.563 g, 2.93 mmol) in 2:3 water:ethanol (5 mL) was added. The mixture was allowed to cool to room temperature whereupon a precipitate formed. This was allowed to stir overnight at room temperature then filtered to afford the title compound as a cream-coloured solid (0.636 g).

Elemental analysis: Found: C, 55.19; H, 4.99; N, 10.42%. C₃gH₃₆F₂N₈O₃.1.90C₆H₈O₇. 1.05H₂O requires C, 55.21; H, 5.00; N, 10.43%.

### Example 168

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a tri-hydrobromic acid salt

6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.501 g, 0.73 mmol) was suspended in ethanol (5 ml) and dichloromethane (2 ml) added. Warm ethanol (20 ml) was added and the solution heated to 60 °C. Hydrobromic acid (48% in water) (0.3 ml, 2.65 mmol) was added dropwise and the solution allowed to cool to room temperature. The resulting precipitate was filtered off to afford the title compound as a pale yellow solid (0.527 g).

Elemental analysis: Found: C, 45.63; H, 4.80; N, 11.23%. C₃₈H₃₆F₂N₈O₃.2.85HBr. 4.35H₂O requires C, 45.63; H, 4.79; N, 11.21%.

### Example 169

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 2-naphthalene sulphonic acid salt

6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.248 g, 0.36 mmol) was suspended in ethanol (1 ml) and dichloromethane (2 ml) added. The solution was added to ethanol (10 ml) at 60 °C then a solution of naphthalene-2-sulfonic acid monohydrate (0.081 g, 0.36 mmol) in ethanol (2 ml) was added. The mixture was allowed to cool to room temperature and concentrated under vacuum to afford the title compound as heterogeneous beige foam/glass (0.324 g).

### Example 170

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 2-naphthalene sulphonic acid salt

6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.238 g, 0.34 mmol) was suspended in ethanol (1 ml) and dichloromethane (2 ml) added. The solution was added to ethanol (10 ml) at 60 °C then a solution of naphthalene-2-sulfonic acid monohydrate (0.156 g, 0.69 mmol) in ethanol (4 ml) was added. The mixture immediately formed a gel-like solid at 60 °C and was allowed to cool to room temperature, then concentrated under vacuum to afford the title compound as a cream-coloured semi-solid gel (0.376 g).

### Example 171

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 2-naphthalene sulphonic acid salt

6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.225 g, 0.33 mmol) was suspended in ethanol (1 ml) and dichloromethane (2 ml) added. The solution was added to ethanol (10 ml) at 60 °C then a solution of naphthalene-2-sulfonic acid monohydrate (0.221 g, 0.98 mmol) in ethanol (4 ml) was added. A precipitate formed at 60 °C and the mixture was allowed to cool to room temperature. The volatiles were removed in vacuo to afford the title compound as a cream-coloured powder (0.434 g).

### Example 172

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a L-malic acid salt

To 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (400 mg, 0.58 mmol) in methanol (20 mL) and dichloromethane (5 mL) was added L-malic acid (78 mg, 0.58 mmol). The resulting solution was concentrated in vacuo to leave the title compound as a solid (478 mg).

### Example 173

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a L-lactic acid salt

To 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (400 mg, 0.58 mmol) in methanol (20 mL) and dichloromethane (5 mL) was added L-lactic acid (52 mg, 0.58 mmol). The resulting solution was concentrated in vacuo to leave the title compound as a solid (452 mg).

### Example 174

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl} imidazo [1,2-a]pyridine-2-carboxamide as a succinic acid salt

To 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (400 mg, 0.58 mmol) in methanol (20 mL) and dichloromethane (5 mL) was added succinic acid (68 mg, 0.58 mmol). The resulting solution was evaporated in vacuo to leave the title compound as a solid (468 mg).

### Example 175

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a trans-butenedioic acid salt

trans-butenedioic acid (8.40 mg, 0.07 mmol) was dissolved in ethanol (1 ml) and to this solution was added 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.05 g, 0.07 mmol) predissolved in ethanol (1 ml). The mixture was stirred at room temperature for 1 hour. A further aliquot of ethanol (2 ml) was added to aid slurrying of any precipitated solid. The mixture was then slurried for 48 hours. The precipitate was collected by filtration and dried in vacuo at room temperature to give the title compound.

### Example 176

### 6-fluoro-N-{cis-4-[6-fluoro-2,4-dioxo-1-[4'-(piperazin-1-ylmethyl)biphenyl-3-yl]-1,4-dihydropyrido [2,3-d]pyrimidin-3(2H)-yl] cyclohexyl} imidazo[1,2-a]pyridine-2-carboxamide as a furan-2-carboxylic acid salt

furan-2-carboxylic acid (8.11 mg, 0.07 mmol) was dissolved in ethanol (1 ml) and to this solution was added 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.05 g, 0.07 mmol) predissolved in ethanol (1 ml). The mixture was stirred at room temperature for 1 hour. A further aliquot of ethanol (2 ml) was added to aid slurrying of any precipitated solid. The mixture was then slurried for 48 hours. The precipitate was collected by filtration and dried in vacuo at room temperature to give the title compound.

### Example 177

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide

6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.5 g, 0.72 mmol) was dissolved in ethanol (10 ml) and allowed to stand, during which time a colourless solid precipitated. This solid was collected by filtration and dried in vacuo at room temperature, then slurried with acetonitrile (3 ml) for 48 hours then filtered off dried in vacuo at room temperature to give the title compound as polymorph A.

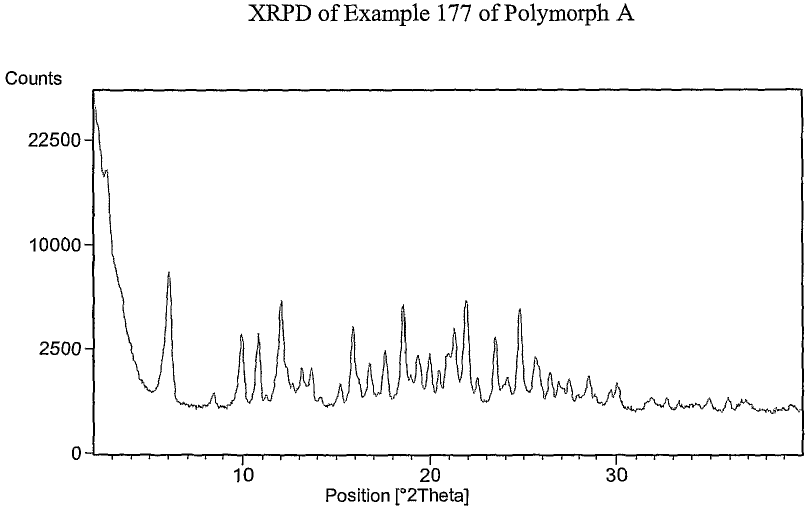

### Example 178

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a citric acid salt

Citric acid (anhydrous) (0.014 g, 0.07 mmol) was dissolved in ethanol (1 ml) and to this solution was added 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.05 g, 0.07 mmol) predissolved in ethanol (1 ml). The mixture was stirred at room temperature for 1 hour. A further aliquot of ethanol (2 ml) was added to aid slurrying of any precipitated solid. The mixture was then slurried for 48 hours. The precipitate was collected by filtration and dried in vacuo at room temperature to give the title compound.

### Example 179

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a maleic acid salt

Maleic acid (8.40 mg, 0.07 mmol) was dissolved in ethanol (1 ml) and to this solution was added 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.05 g, 0.07 mmol) predissolved in ethanol (1 ml). The mixture was stirred at room temperature for 1 hour. A further aliquot of ethanol (2 ml) was added to aid slurrying of any precipitated solid. The mixture was then slurried for 48 hours. The precipitate was collected by filtration and dried in vacuo at room temperature to give the title compound.

### Example 180

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a trans-cinnamic acid salt

trans-Cinnamic acid (0.012 ml, 0.07 mmol) was dissolved in ethanol (1 ml) and to this solution was added 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.05 g, 0.07 mmol) predissolved in ethanol (1 ml). The mixture was stirred at room temperature for 1 hour. A further aliquot of ethanol (2 ml) was added to aid slurrying of any precipitated solid. The mixture was then slurried for 48 hours. The precipitate was collected by filtration and dried in vacuo at room temperature to give the title compound.

### Example 181

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a benzenesulfonic acid salt

6-Fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.05 g, 0.07 mmol) was dissolved in methanol (1 ml) with warming and to this solution was added benzenesulfonic acid (0.023 g, 0.14 mmol) dissolved in ethanol (1.000 ml). The mixture was stirred at overnight. The solid was collected by filtration to give the title compound.

### Example 182

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a benzenesulfonic acid salt

6-Fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.5 g, 0.72 mmol) was dissolved in ethanol (10 ml) with warming and to this solution was added benzenesulfonic acid (0.343 g, 2.17 mmol). The mixture was stirred at overnight, during stirring a gum precipitated. The solvent was decanted and the residue triturated with warm ethyl acetate to give a colourless solid. This solid was filtered and dried to give the title compound.

### Example 183

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 1,5-napthalenedisulfonic acid salt

6-Fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.05 g, 0.07 mmol) was dissolved in methanol (20 mL) under reflux. To this solution was added 1,5-Napthelene disulfonic acid (0.021 g, 0.07 mmol). The mixture was evaporated to dryness and the residue was redissolved in water/ethanol mixture under reflux. The mixture was cooled between 0-5 deg overnight and the crystalline product was collected by filtration and dried in vacuo to give the title compound.

### Example 184

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a 2,5-dichlorobezenesulfonic acid salt.

The product of Example 154 (5 mg) was recrystallised from ethanol. The solution was allowed to cool to room temp and the precipitate collected by centrifugation to give the title compound as Crystalline Form A.

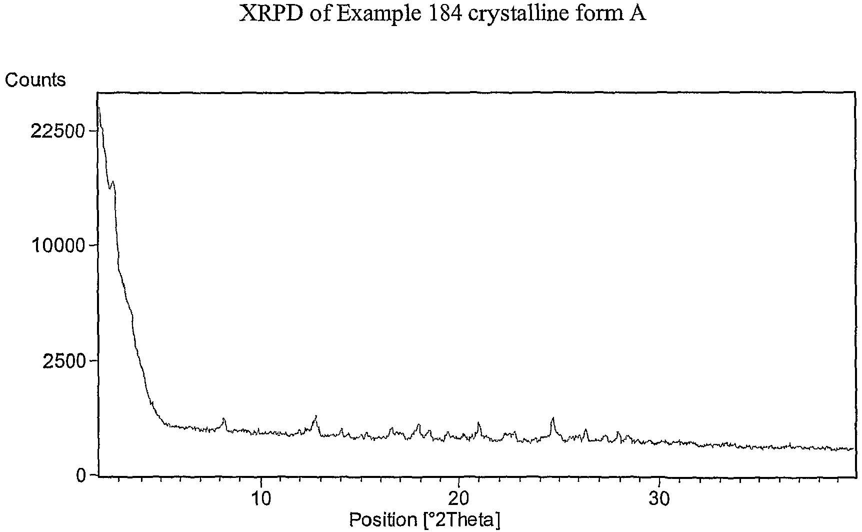

### Example 185

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide as a mono [(1S,4R)-7,7-dimethyl-2-oxobicyclo[2.2.1]hept-1-yl]methanesulfonic acid salt

To a solution of 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (2.00 g, 2.90 mmol) in ethanol (80 mL) (heated to dissolve) was added [(1S,4R)-7,7-dimethyl-2-oxobicyclo[2.2.1]hept-1-yl]methanesulfonic acid (0.673 g, 2.90 mmol) in EtOH (12 mL) (heated to dissolve). The solvent was left to evaporate over 48hours under nitrogen. This gave the title compound (2.68 g).

¹H NMR (499.914 MHz, DMSO) d 8.80 - 8.79 (m, 1H), 8.59 (d, 1H), 8.48 (s, 2H), 8.37 (s, 1H), 8.34 (dd, 1H), 7.78 - 7.60 (m, 7H), 7.46 - 7.38 (m, 4H), 4.90 - 4.85 (m, 1H), 4.17 (t, 1H), 3.59 (s, 2H), 3.10 (s, 4H), 2.86 (d, 1H), 2.72 - 2.57 (m, 7H), 2.36 (d, 1H), 2.26 - 2.20 (m, 1H), 2.00 (d, 2H), 1.93 (t, 1H), 1.87 - 1.82 (m, 1H), 1.79 (d, 1H), 1.75 - 1.64 (m, 4H), 1.30 - 1.24 (m, 2H), 1.05 (s, 3H), 0.74 (s, 3H)

### Example 186

### 6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl} imidazo[1,2-a]pyridine-2-carboxamide as a L-mandelic acid salt

6-fluoro-N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}imidazo[1,2-a]pyridine-2-carboxamide (0.5 g) was dissolved in EtOH (10 mL) and (heating was required) and the L-mandelic acid (0.110 g, 0.72 mmol) dissolved in ethanol was added. The resultant solid was filtered to give the title compound as a solid.

Some of the indicated salts above were then slurried in the conditions indicated in the table below to give the indicated crystalline form or polymorph form as identified by X-ray powder diffraction (see Figures). Unless otherwise indicated the salts were slurried at approximately 20mg/ml of solvent

| Salt Name | Sample taken from Example Number | Crystalline Form name | Polymorph Form Name | Extra experimental | Slurry Temp (Ambient or °C) | Slurry Time (days) | Slurry Solvent | Summary | Figure Number |
|---|---|---|---|---|---|---|---|---|---|
| (S)(+) 10 camphorsulfonic acid | 185 | | C | | Ambient | 8 | Dimethoxyethane | | 1 |
| (S)(+) 10 camphorsulfonic acid | 185 | | A | | Ambient | 8 | Methanol | also from a slurry in Isopropyl Alcohol | 2 |
| (S)(+) 10 camphorsulfonic acid | 185 | | B | Slurry as 24mg/ml | Ambient | 8 | Butan-2-one | | 3 |
| 1,5-Napthelene disulfonic acid | 183 | A | | | Ambient | 0 | Ethanol | | 4 |
| 1-Hydroxy-2-naphthoic acid | 145 | C | | | Ambient | 6 | Ethyl acetate | | 5 |
| 1-Hydroxy-2-naphthoic acid | 145 | B | | | Ambient | 6 | Ethanol | | 6 |
| 2,5-Dichlorobenzenesulfonic acid | 154 | A | | | 35 | 14 | Ethyl acetate | | 7 |
| 2,5-Dichlorobenzenesulfonic acid | 154 | B | | | Ambient | 15 | Butan-2-one | | 8 |
| 2-Napthalenesulfonic acid | 169 | A | | | Ambient | 7 | Ethanol | | 9 |
| 2-Napthalenesulfonic acid | 169 | B | | | Ambient | 7 | Toluene | | 10 |
| 2-Napthalenesulfonic acid | 170 | A | | | Ambient | 7 | Tetrahydrofuran | also from a slurry in Isopropyl Alcohol or Methanol or Acetonitrile or Ethanol | 11 |
| 2-Napthalenesulfonic acid | 170 | B | | | Ambient | 7 | Toluene | | 12 |
| 2-Napthalenesulfonic acid | 170 | C | | | Ambient | 7 | Dioxane | | 13 |
| Adipic acid | 150 | A | | | Ambient | 10 | Butan-2-one | also from a slurry in Isohexane | 14 |
| Benzenesulfonic acid | 182 | A | | | Ambient | 5 | Water | also from a slurry in Ethanol or Acetonitrile | 15 |
| Benzenesulfonic acid | 153 | | A | | Ambient | 15 | Nitromethane | also from a slurry in Isopropyl Alcohol or Butan-2-one or Tetrahydrofuran or Ethanol | 16 |
| Benzenesulfonic acid | 153 | | B | | 35 | 15 | Ethyl acetate | | 17 |
| Benzenesulfonic acid | 182 | B | | | Ambient | 10 | Acetonitrile | | 18 |
| Citric acid | 167 | | B | | Ambient | 7 | Acetonitrile | | 19 |
| Citric acid | 167 | | C | | Ambient | 30 | Water | | 20 |
| Citric acid | 167 | | A | | Ambient | 30 | Methanol | also from a slurry in Ethanol | 21 |
| Citric acid | 166 | | A | | Ambient | 32 | Methanol | also from a slurry in Ethanol | 22 |
| DL-Mandelic acid | 146 | A | | | Ambient | 6 | Ethyl acetate | also from a slurry in Dimethoxyethane or Ethanol or Nitromethane or Isopropyl Alcohol or butan2-one | 23 |
| HBr | 169 | | A | | Ambient | 12 | Methanol | | 24 |
| HCl | 151 | | A | | 35 | 12 | Ethanol | also from a slurry at ambient temperature in Isopropyl Alcohol or Nitromethane or Tetrahydrofuran or Dioxane or Acetonitrile or Ethyl Acetate | 25 |
| L-Mandelic acid | 186 | A | | | Ambient | 20 | Acetonitrile | also from a slurry in Butan-2-one or Dimethoxyethane or Ethanol | 26 |
| Malonic acid | 155 | | A | | Ambient | 10 | Ethanol | also from a slurry in Isopropyl Alcohol | 27 |
| Malonic acid | 155 | | B | | | 18 | Tetrahydrofuran | | 28 |
| Mesitylene sulfonic acid | 161 | A | | Slurry as 50mg/ml | Ambient | 12 | Methanol | | 29 |
| Mesitylene sulfonic acid | 161 | B | | Slurry as 50mg/ml | 35 | 12 | Tetrahydrofuran | | 30 |
| Mesitylene sulfonic acid | 160 | A | | Slurry as 50mg/ml | Ambient | 12 | Ethanol | | 31 |
| Methane sulfonic acid | 141 | | A | | Ambient | 6 | Acetonitrile | also from a slurry in Isopropyl Alcohol or Ethyl Acetate or Dioxane or Ethanol | 32 |
| Methane sulfonic acid | 139 | | B | | Ambient | 4 | Acetonitrile | | 33 |
| Methane sulfonic acid | 139 | | A | | 35 | 4 | Methanol | also from a slurry in Ethanol | 34 |
| Methane sultonic acid | 140 | | A | | Ambient | 4 | Acetonitrile | also from a slurry in Isopropyl Alcohol or Ethyl Acetate or Toluene or Ethanol or Tetrahydrofuran or Dichloromethane or Methanol or Ethanol | 35 |
| Nicotinic Acid | 149 | A | | | Ambient | 10 | Acetonitrile | | 36 |
| Phosphoric acid | 162 | | A | Slurry as 50mg/ml | Ambient | 6 | Dichloromethane | | 37 |
| Phosphoric acid | 162 | | B | Slurry as 50mg/ml | Ambient | 11 | Ethanol | also from a slurry in Methanol | 38 |
| Phosphoric acid | 165 | A | | Slurry as 30mg/ml | 35 | 6 | Methanol | | 39 |
| Phosphoric acid | 165 | B | | Slurry as 30mg/ml | 35 | 6 | Acetonitrile | also from a slurry in Tetrahydrofuran or Ethyl Acetate or Isopropyl Alcohol or Dioxane | 40 |
| Phosphoric acid | 163 | A | | Slurry as 30mg/ml | 35 | 6 | Methanol | | 41 |
| Stearic acid | 158 | D | | Slurry as 50mg/ml | Ambient | 5 | Methanol | | 42 |
| Stearic acid | 158 | B | | Slurry as 50mg/ml | Ambient | 10 | Ethyl acetate | | 43 |
| Stearic acid | 158 | C | | Slurry as 50mg/ml | Ambient | 5 | Ethyl acetate | | 44 |
| Stearic acid | 158 | A | | Slurry as 50mg/ml | Ambient | 10 | Acetonitrile | | 45 |
| Tosic acid | 148 | A | | | Ambient | 13 | Toluene | | 46 |
| Tosic acid | 148 | B | | | Ambient | 13 | Dimethoxyethane | | 47 |

### Example 187

### 6-fluoro-N-{cis-4-[6-fluoro-1-(4'-{3-[(3-hydroxypropyl)amino]propyl}biphenyl-3-yl)-2,4-dioxo-1,4-dihydropyrido [2,3-d]pyrimidin-3(2H)-yl] cyclohexyl} imidazo[1,2-a]pyridine-2-carboxamide as a methanesulfonic acid salt

6-Fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.4 g, 0.57 mmol) was dissolved in methanol (10 ml) and dichloromethane (2 ml). Methanesulfonic acid (54 mg, 0.57 mmol) was added and the solution was concentrated *in vacuo* to give the title compound as a solid (454 mg).

### Example 188

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a citric acid salt

6-Fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.4 g, 0.57 mmol) was dissolved in methanol (10 ml) and dichloromethane (2 ml). Citric acid (109 mg, 0.57 mmol) was added and the solution was concentrated *in vacuo* to give the title compound as a solid (509 mg).

### Example 189

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a 1,5-naphthalene disulfonic acid salt

6-Fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*-yl]cyclohexyl}imidazo[1,2-*a*]pyridine-2-carboxamide (0.4 g, 0.57 mmol) was dissolved in methanol (10 ml) and dichloromethane (2 ml). 1,5-Naphthalene disulfonic acid (163 mg, 0.57 mmol) was added and the solution was stirred for 15 minutes where upon a solid formed. Filtration gave the title compound as a solid (350 mg) as crystalline form B. The filtrate was concentrated to give further title compound as a solid (110 mg) as crystalline form A.

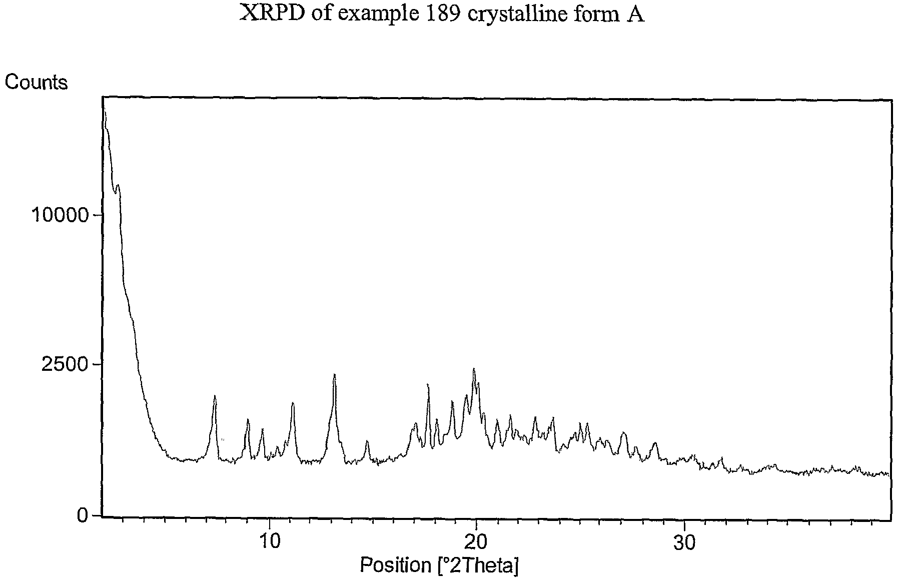

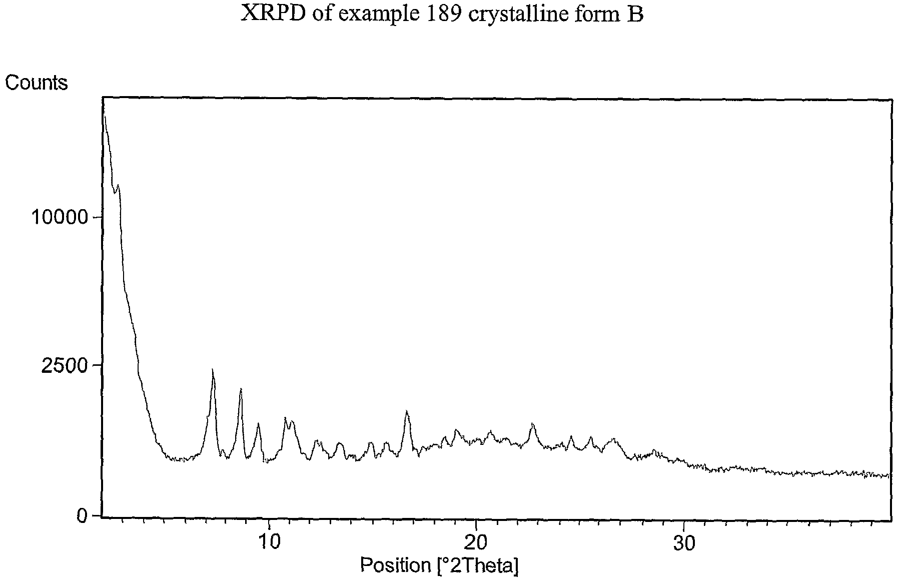

### Example 190

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl] cyclohexyl}imidazo[1,2-a]pyridine-2-carboxamide as a benzenesulfonic acid salt

6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl} imidazo[1,2-*a*]pyridine-2-carboxamide (0.4 g, 0.57 mmol) was dissolved in methanol (10 ml) and dichloromethane (2 ml). Benzenesulfonic acid (89 mg, 0.57 mmol) was added and the solution was concentrated *in vacuo* to give the title compound as a solid (489 mg).

### Example 191

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a 1-hydroxy-2-naphthoic acid salt

6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (260 mg, 0.37 mmol) was dissolved in DME (2.5 mL) and to this solution was added 1-hydroxy-2-naphthoic acid (69 mg, 0.37 mmol) in DME (2.5 mL). The resultant suspension was allowed to stir for 4 days then collected by filtration and dried *in vacuo* to give the title compound as a colorless solid (201 mg).

### Example 192

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a para-toluenesulfonic acid salt

6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (514 mg, 0.73 mmol) was dissolved in DME (5 mL) and to this solution was added p-toluenesulfonic acid monohydrate (125 mg, 0.73 mmol) in DME (5 mL). The resultant suspension was allowed to stir for two days then collected by filtration and dried in vacuo to give the title compound as a colorless solid (481 mg).

### Example 193

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a hydrochloric acid salt

6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (250 mg, 0.35 mmol) was dissolved in DME (5 mL) and to this solution was added concentrated hydrochloric acid (0.2 ml, 2 mmol). The resultant suspension was allowed to stir for four days then concnetrated and slurried in tetrahydrofuran (5 mL) for 9 days. The resultant suspension was filtered and the solid dried *in vacuo* to give the title compound as a colorless solid (196 mg).

### Example 194

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a sulfuric acid salt

6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (500 mg, 0.71 mmol) was dissolved in DME (10 mL) and to this solution was added concentrated sulfuric acid (0.038 ml, 0.71 mmol). The resultant suspension was allowed to stir for two hours then filtered and the solid dried *in vacuo* to give the title compound as a colorless solid (465 mg).

### Example 195

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a D-Mandelic acid salt

To a solution of 6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.4 g, 0.57 mmol) in DME (5 mL) (heated to dissolve) was added D-Mandelic acid (0.086 g, 0.57 mmol) in DME (3 mL) and the reaction left to stir (open to the air) at 30 °C for 2 hours (white foam crashed out) then the solution was heated to 40 °C and Nitrogen gas was blown over the solution until solvent had evaporated (20 hours). This gave the title compound as a solid.

### Example 196

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a 2,5-dichlorobenzenesulfonic acid salt

To a solution of 6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.4 g, 0.57 mmol) in DME (5 mL) (heated to dissolve) was added 2,5-Dichlorobenzenesulfonic acid (0.128 g, 0.57 mmol) in DME (3 mL) (heated to dissolve) and the reaction left to stir (open to the air) at 30 °C for 2 hours (white foam crashed out) then the solution was heated to 40 °C and Nitrogen gas was blown over the solution until solvent had evaporated (20 hours). This gave the title compound as a solid.

### Example 197

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a hydrobromide salt

6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (300 mg, 0.42 mmol) was dissolved through heating under reflux in a minimum amount of ethanol (30 mL). To this solution was added a few drops of 48% aqeuous hydroromic acid until acidic (pH 1). the mixture was allowed to cool overnight. No precipitate was observed, the mixture was then left in the refrigerator for 24 hours, during which time a pale tan coloured solid deposited, this was filtered off and dried, to give the title compound as a solid.

### Example 198

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide as a benzoic acid salt

6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (300 mg, 0.42 mmol) was dissolved through heating under reflux in a minimum amount of ethanol (30 mL). To this solution was added a solution of benzoic acid (51.8 mg, 0.42 mmol) dissolved in EtOH (2 mL) and the mixture allowed to cool overnight. No precipitate was observed, thus the mixture was evaporated to dryness and the residue tritutated with acetonitrile (10 ml) to give after several minutes stirring a colourless solid, This was filtered and dried to give the title compound as a solid.

### Example 199

### 6-fluoro-N-{cis-4-[6-fluoro-1-(4'-{3-[(3-hydroxypropyl)amino]propyl}biphenyl-3-yl)-2,4-dioxo-1,4-dihydropyrido[2,3-d]pyrimidin-3(2H)-yl]cyclohexyl}imidazo[1,2-a]pyridine-2-carboxamide

6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (3.64 g) was dissolved in ethanol with heating (350 mL), the solution was allowed to cool to room temperature to afford the title compound as a white solid (2.37 g)

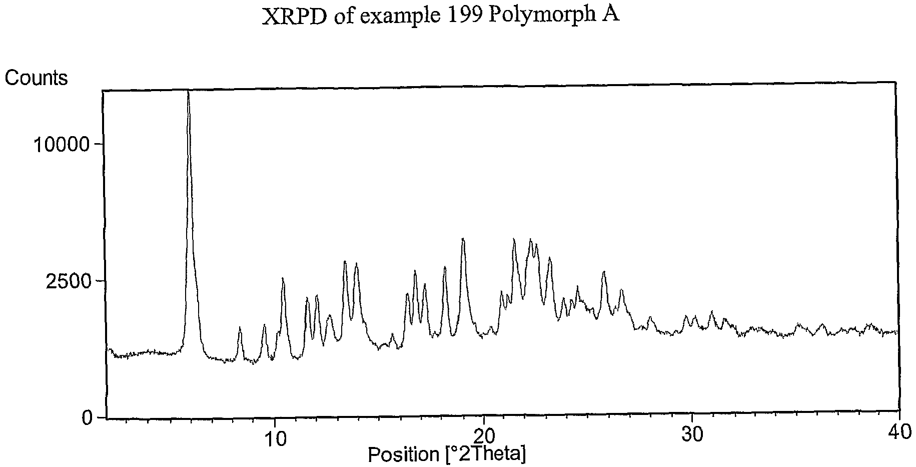

Some of the indicated salts above were then slurried in the conditions indicated in the table below to give the indicated crystalline form as identified by X-ray powder diffraction (see Figures). Unless otherwise indicated the salts were slurried at approximately 20mg/ml of solvent

| Salt name | Sample taken from Example Number | Crystalline form Name | Extra experimental | Slurry temp | Slurry Time (days) | Slurry Solvent | Summary | Figure Number |
|---|---|---|---|---|---|---|---|---|
| 1,5-napthelene disulfonic acid | 189 (from crystalline form A) | D | | Ambient | 14 | Methanol | also from slurry in Acetonitrile | 48 |
| 1,5-napthelene disulfonic acid | 189 (from crystalline form A) | E | | Ambient | 4 | Isopropanol | | 49 |
| 1,5-napthelene disulfonic acid | 189 (from crystaffine form B) | C | | Ambient | 4 | Ethanol | also from slurry in Tetrahydrofuran or Dichloromethane or Toluene | 50 |
| 1-Hydroxy-2-naphthoic acid | 191 | A | | Ambient | 7 | Isohexane | also from slurry in Dimethoxyethane or Methanol | 51 |
| 1-Hydroxy-2-naphthoic acid | 191 | B | | Ambient | 7 | Butan-2-one | also from slurry in Isopropyl Acetate or Toluene or Acetonitrile | 52 |
| 2,5-dichlorobenzenesulfonic acid | 196 | A | | Ambient | 8 | Nitromethane | also from slurry in Butan-2-one or Isopropyl Alcohol or Acetonitrile or Tetrahydrofuran or Ethyl Acetate | 53 |
| 2,5-dichlorobenzenesulfonic acid | 196 | B | | Ambient | 8 | Dimethoxyethane | | 54 |
| Benzenesulfonic acid | 190 | B | | Ambient | 4 | Ethanol | | 55 |
| Benzenesulfonic acid | 190 | A | Started in solution, solid by day 3 | Ambient | 14 | Dioxane | also from slurry in Tetrahydrofuran or Butan-2-one | 56 |
| Benzenesulfonic acid | 190 | C | | Ambient | 4 | Acetonitrile | | 57 |
| Citric acid | 188 | C | | Ambient | 14 | Tetrahydrofuran | | 58 |
| Citric acid | 188 | A | | Ambient | 14 | Ethanol | | 59 |
| Citric acid | 188 | B | | Ambient | 4 | Dioxane | | 60 |
| D-Mandelic acid | 195 | B | | Ambient | 8 | Nitromethane | | 61 |
| D-Mandelic acid | 195 | D | | Ambient | 8 | Tetrahydrofuran | also from slurry in IPA or Dimethoxyethane or Butan-2-one | 62 |
| D-Mandelic acid | 195 | A | | Ambient | 8 | Ethanol | also from slurry in Isopropyl Acetate or Ethyl Acetate | 63 |
| D-Mandelic acid | 195 | C | | Ambient | 8 | Acetonitrile | | 64 |
| Methane sulfonic acid | 187 | B | | Ambient | 4 | Toluene | also from slurry in Butan-2-one | 65 |
| Methane sulfonic acid | 187 | A | | Ambient | 4 | Tetrahydrofuran | | 66 |
| Methane sulfonic acid | 187 | C | | Ambient | 14 | Acetonitrile | also from slurry in Butan-2-one | 67 |
| p-Toluenesulfonic acid | 192 | B | | Ambient | 20 | Ethanol | also from slurry in Tetrahydrofuran | 68 |
| p-Toluenesulfonic acid | 192 | A | | Ambient | 20 | Dimethoxyethane | also from slurry in Isopropyl Acetate or Dioxan or Isohexane or Butan-2-one or Toluene | 69 |
| p-Toluenesulfonic acid | 192 | C | | Ambient | 20 | Acetonitrile | | 70 |

### Example 200

### 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide as a sulfuric acid salt

6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.82 mmol) was slurried in methanol (10 ml) and acetonitrile (10 ml). Sulfuric acid (81 mg, 0.82 mmol) was added and the resulting solution was concentrated *in vacuo* to give the title compound as a solid (581 mg).

### Example 201

### 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide as a fumaric acid salt

6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.82 mmol) was slurried in methanol (10 ml) and acetonitrile (10 ml). Fumaric acid (95 mg, 0.82 mmol) was added and the resulting solution was concentrated in *vacuo* to give the title compound as a solid (595 mg).

### Example 202

### 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide as a 1-hydroxy-2-naphthoic acid salt

6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.82 mmol) was slurried in methanol (10 ml) and acetonitrile (10 ml). 1-Hydroxy-2-naphthoic acid (155 mg, 0.82 mmol) was added and the resulting solution was concentrated *in vacuo* to give the title compound as a solid (655 mg).

### Example 203

### 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide as a methanesulfonic acid salt

6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.82 mmol) was slurried in methanol (10 ml) and acetonitrile (10 ml). Methanesulfonic acid (79 mg, 0.82 mmol) was added and the resulting solution was concentrated *in vacuo* to give the title compound as a solid (579 mg).

### Example 204

### 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide as a hydrochloric acid salt

6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl]cyclohexyl}imidazo[1,2-*a*]pyridine-2-carboxamide (0.5 g, 0.82 mmol) was slurried in methanol (10 ml) and acetonitrile (10 ml). Hydrochoric acid (10M, 82 µl, 0.82 mmol) was added and the resulting solution was concentrated *in vacuo* to give the title compound as a solid (582 mg).

### Example 205

### 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide as an acetic acid salt

6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl]cyclohexyl}imidazo[1,2-*a*]pyridine-2-carboxamide (500 mg, 0.71 mmol) and acetic acid (85 mg, 1.42 mmol) were combined in CH₂Cl₂ (10 mL) and stirred then evaporated, affording the title compound as a crisp foam (580mg).

### Example 206

### 6-fluoro-N-{cis-4-[6-fluoro-2,4-dioxo-1-[4'-(2-pyrrolidin-1-ylethoxy)biphenyl-3-yl]-1,4-dihydropyrido[2,3-d]pyrimidin-3(2H)-yl]cyclohexyl}imidazo[1,2-a]pyridine-2-carboxamide as an L-(+)-tartaric acid salt

6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl]cyclohexyl}imidazo[1,2-*a*]pyridine-2-carboxamide (0.502 g, 0.71 mmol) was dissolved in a mixture of dichloromethane (15 mL) and methanol (5 mL). A solution of L-(+)-tartaric acid (0.107 g, 0.71 mmol) in methanol (6 mL) was added to afford a clear yellow solution. After 3 h the solvents were removed in vacuo to afford a pale yellow foam (0.600 g).

Some of the indicated salts above were then slurried in the conditions indicate in the table below to give the indicated crystalline form as identified by X-ray powder diffraction (see Figures). Unless otherwise indicated the salts were slurried at approximately 20mg/ml of solvent.

| Salt Name | Sample taken from Example Number | Crystalline form Name | Slurry Temp | Slurry Time | Slurry Solvent | Summary | Figure Number |
|---|---|---|---|---|---|---|---|
| Fumaric Acid | 201 | B | Ambient | 14 | Acetonitrile | | 71 |
| 1-Hydroxy-2-naphthoic acid | 202 | A | Ambient | 14 | Ethyl acetate | also from a slurry in Ethanol or Methanol or Ethyl Acetate or Isopropyl alcohol or Acetonitrile | 72 |
| Acetic acid | 205 | A | Ambient | 1 | Ethyl acetate | also from a slurry in Acetonitrile | 73 |
| Fumaric acid | 201 | A | Ambient | 5 | Ethyl acetate | | 74 |
| L-Tartaric acid | 206 | A | Ambient | 7 | Methanol | also from a slurry in Ethanol | 75 |

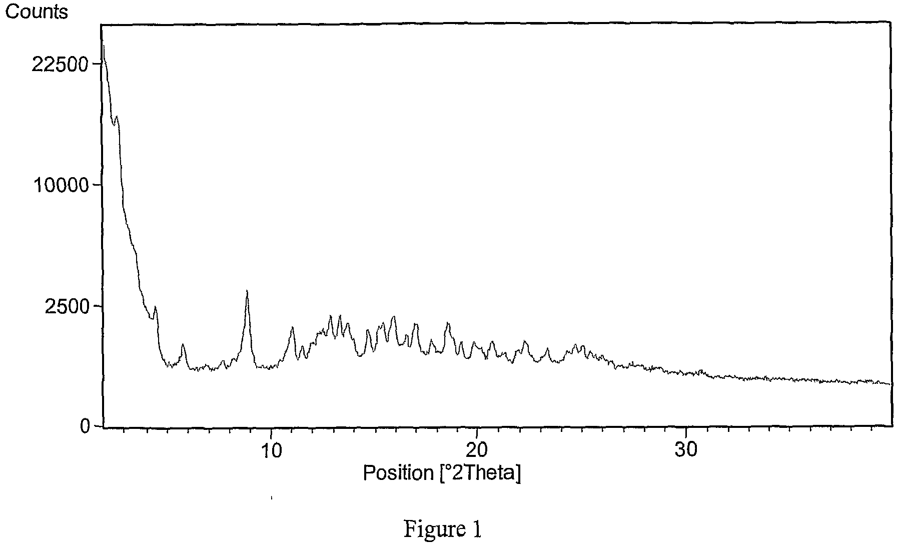

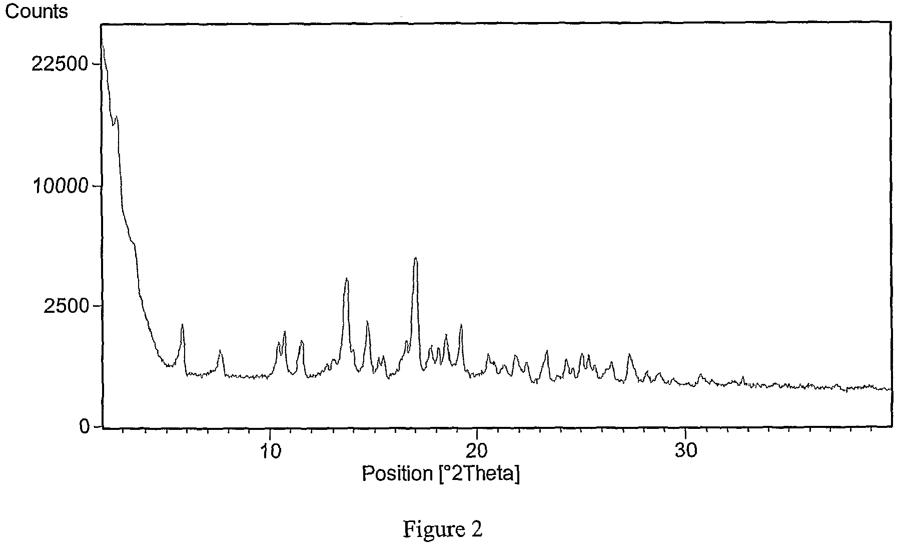

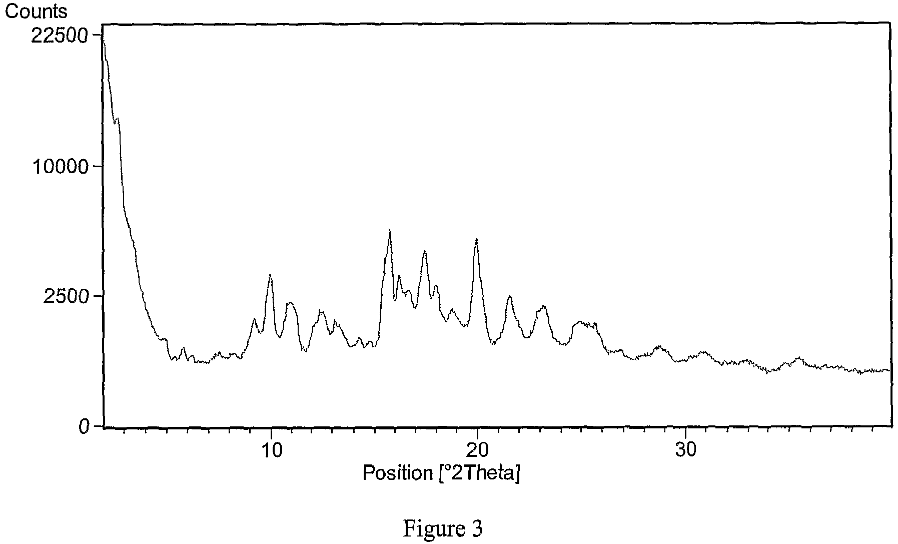

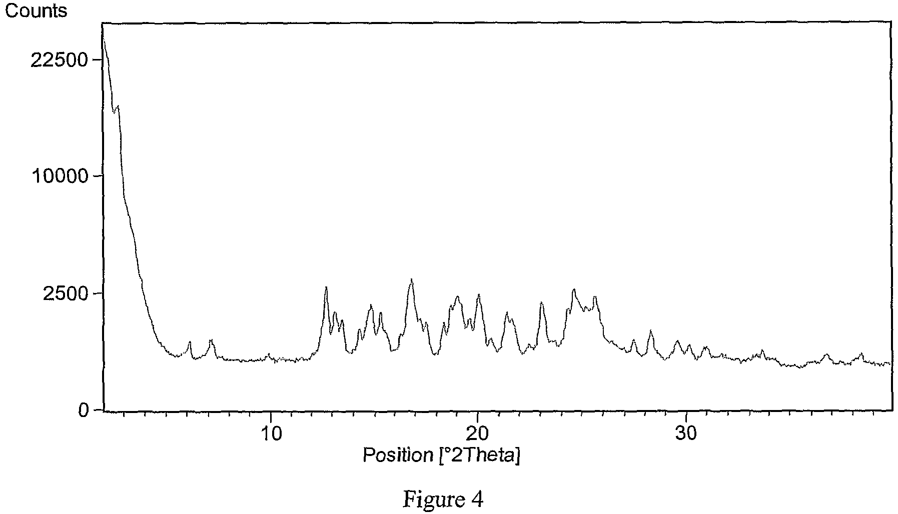

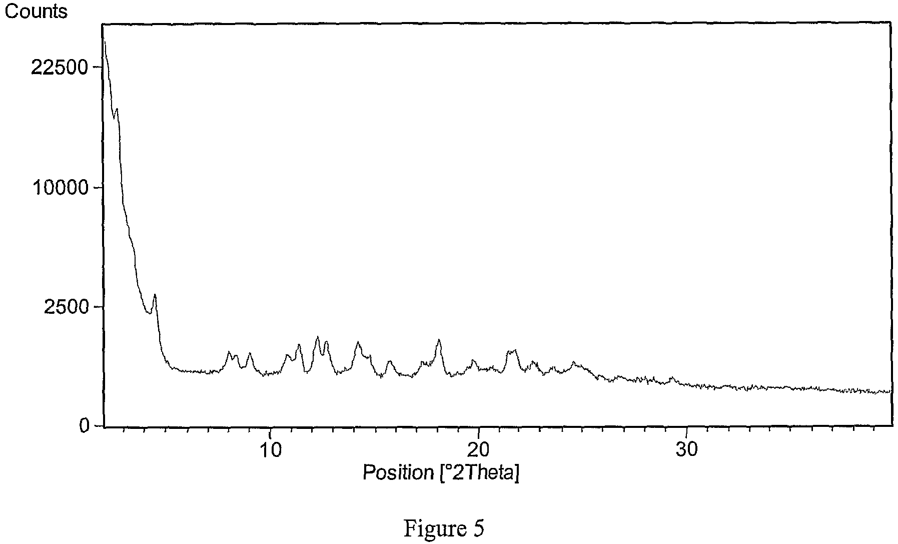

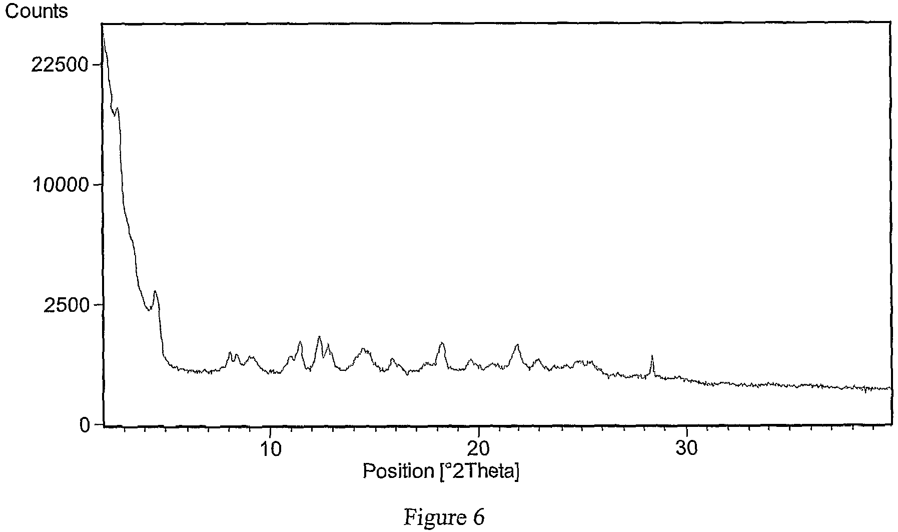

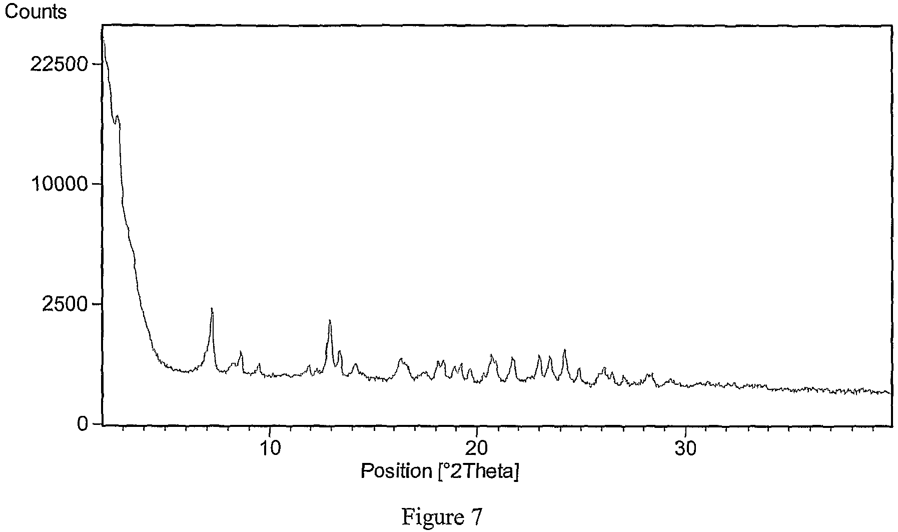

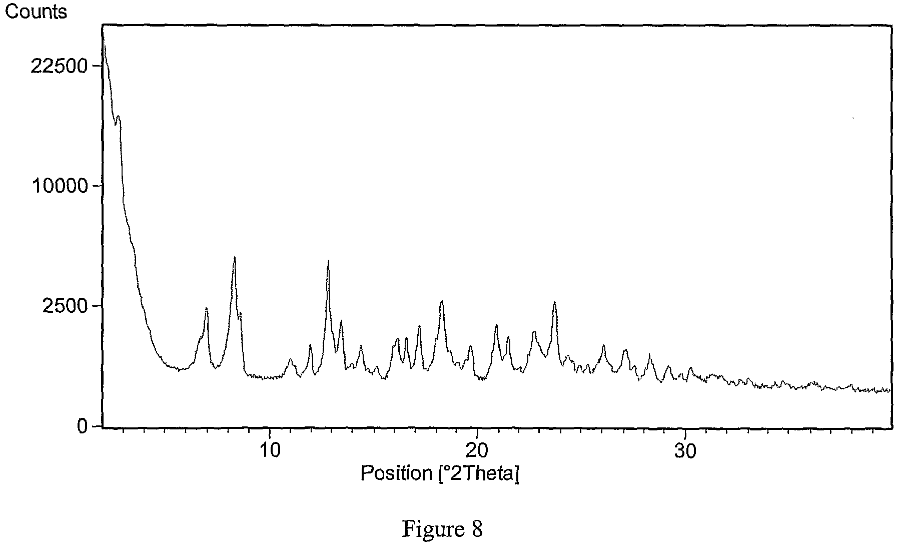

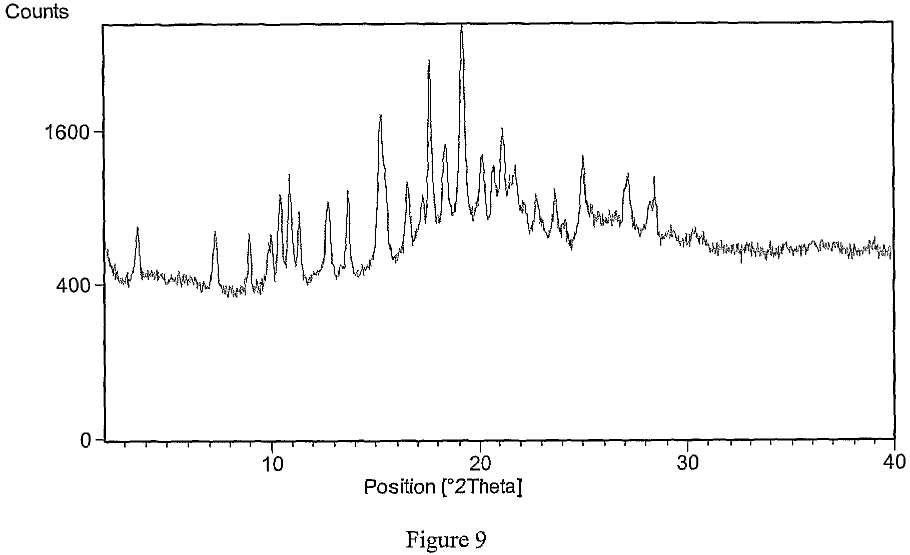

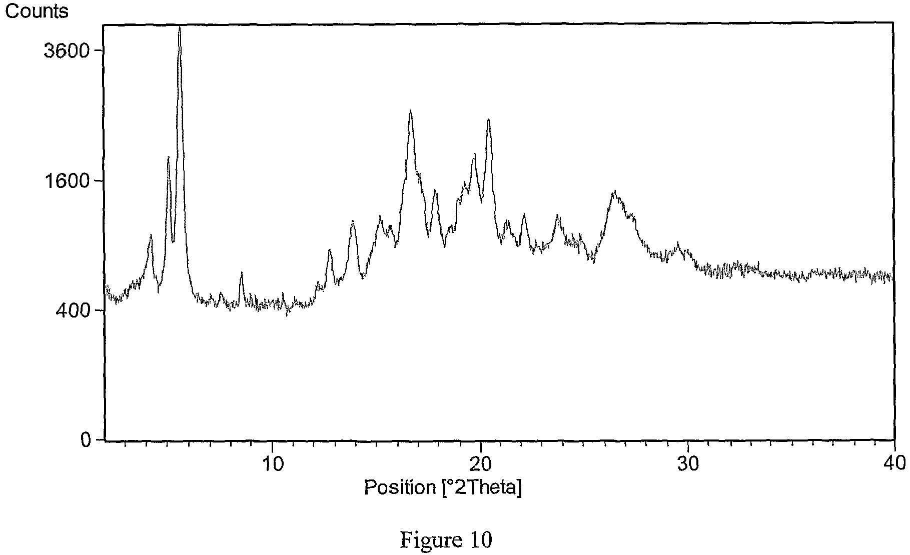

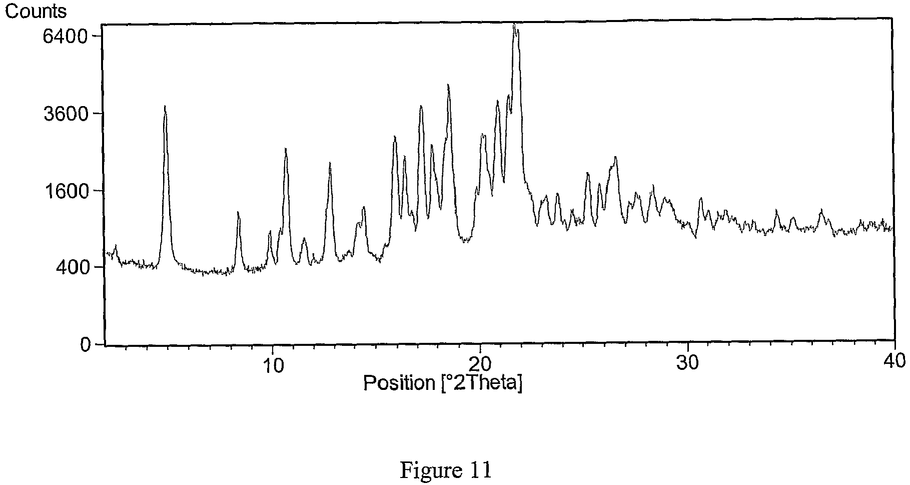

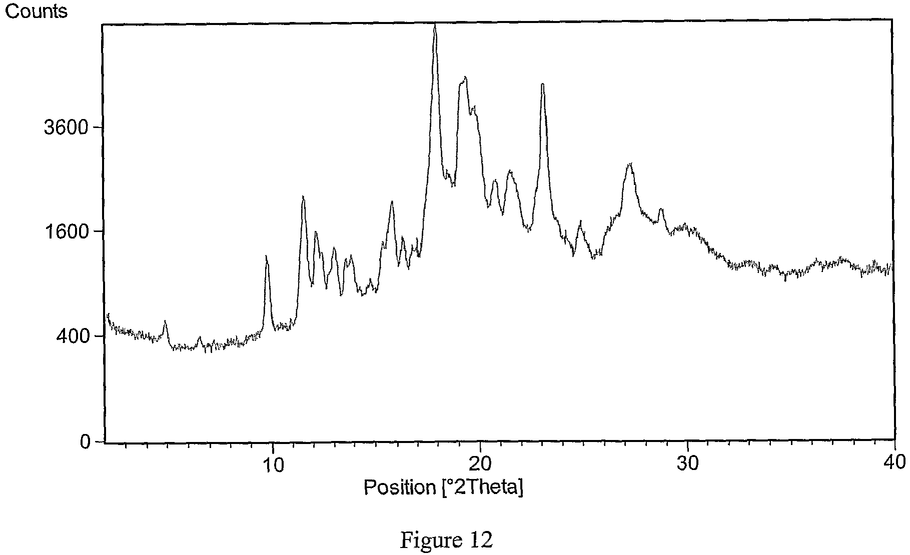

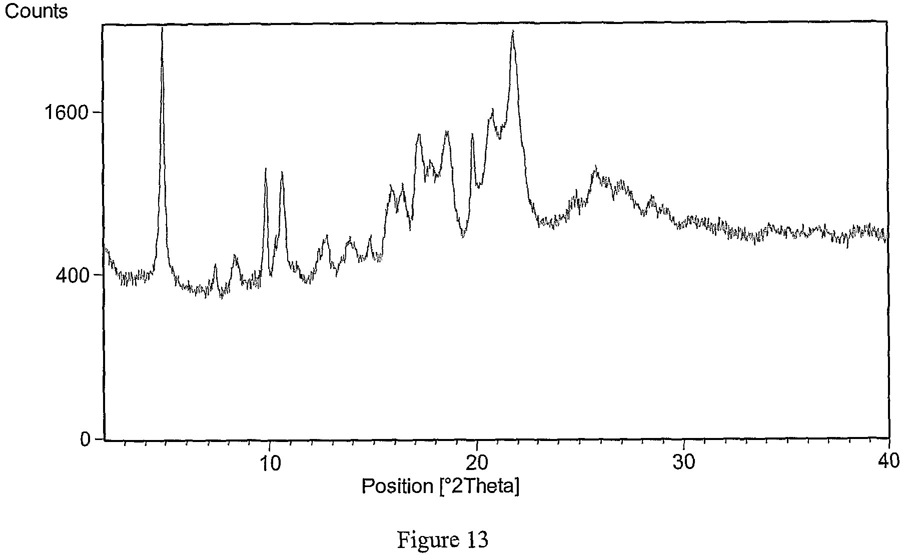

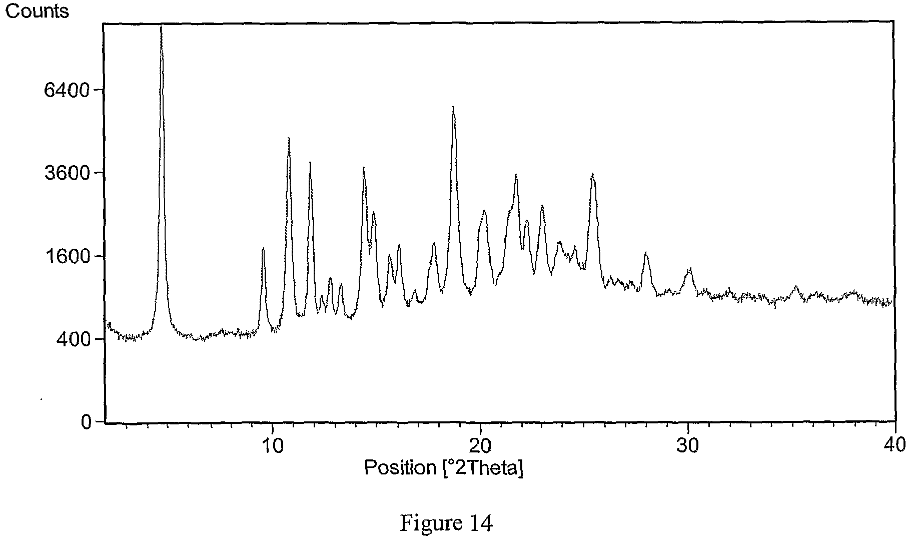

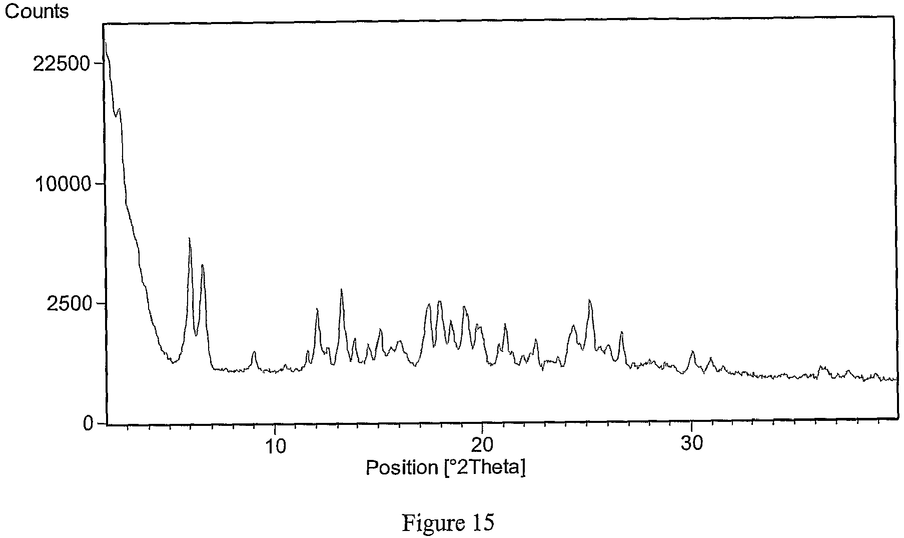

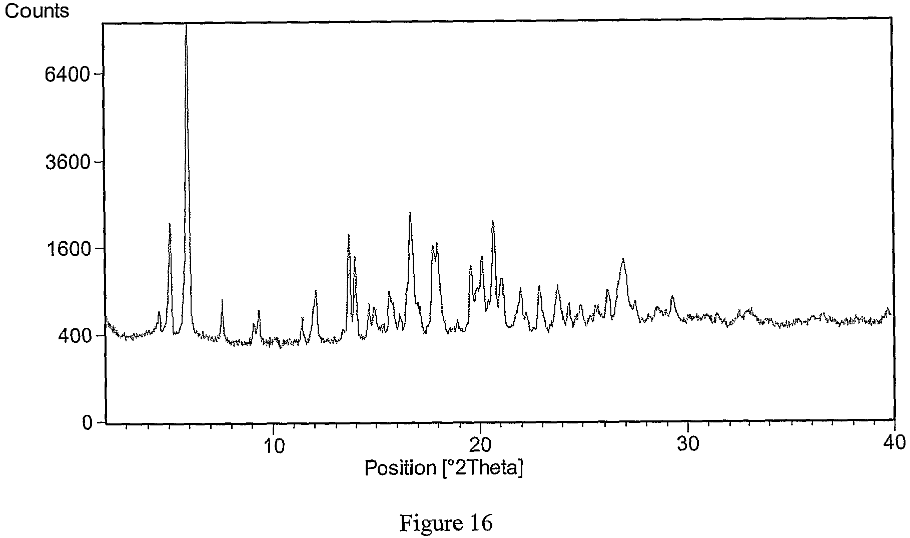

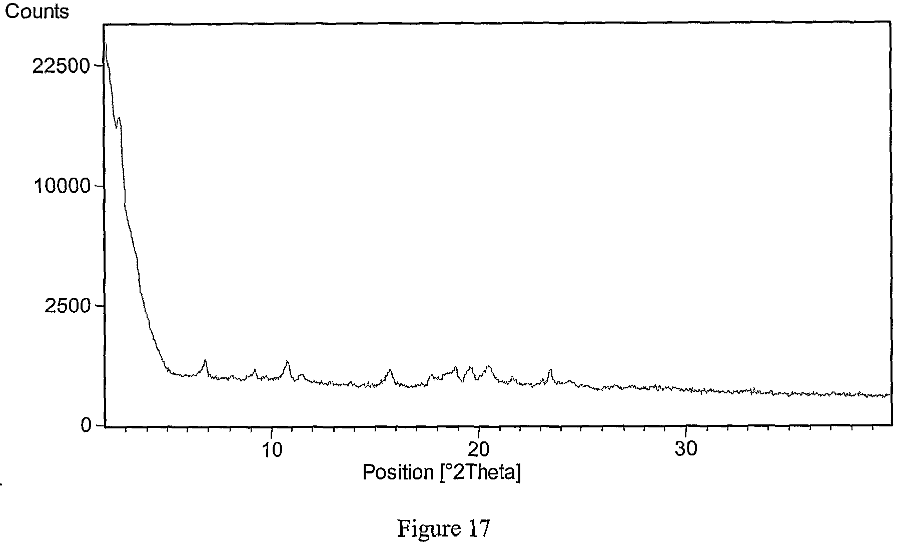

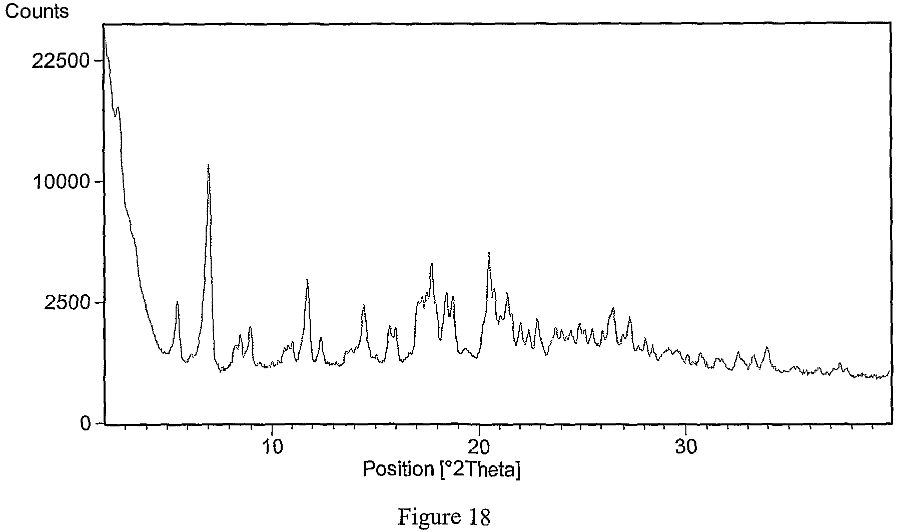

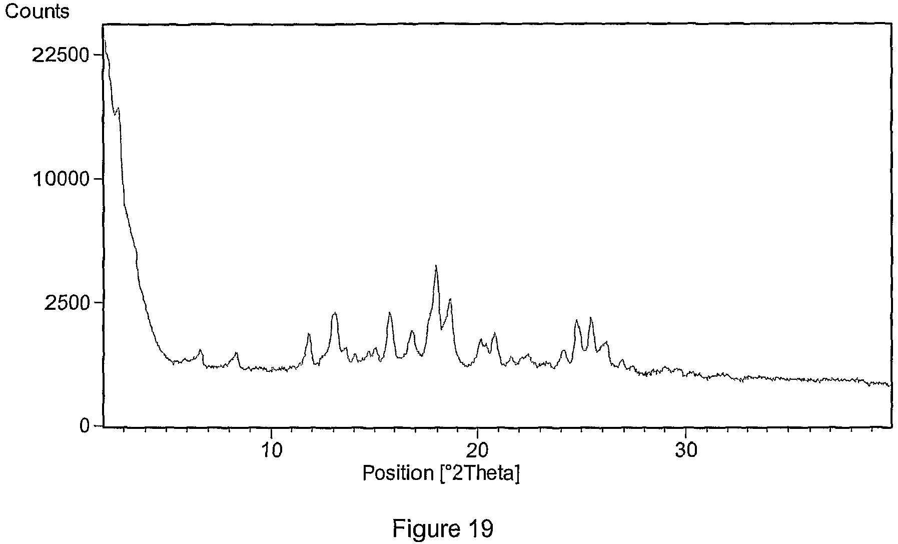

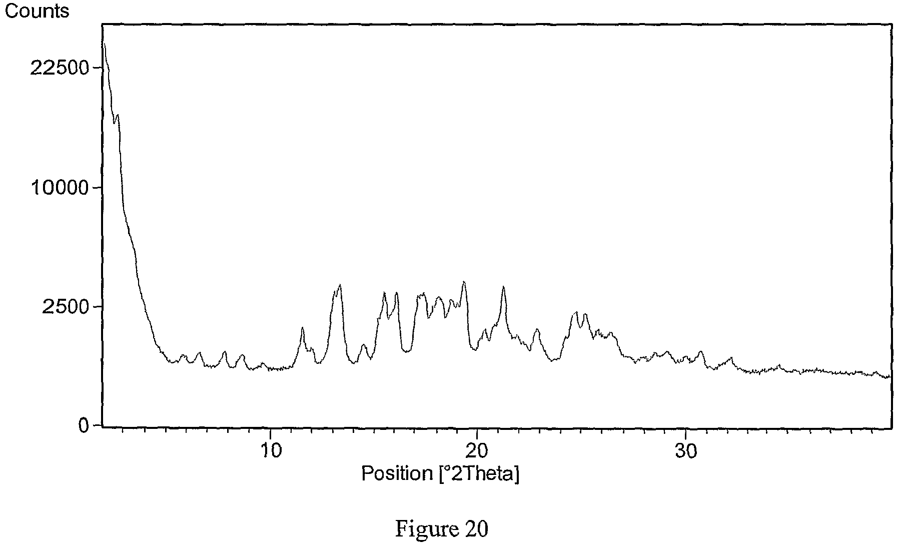

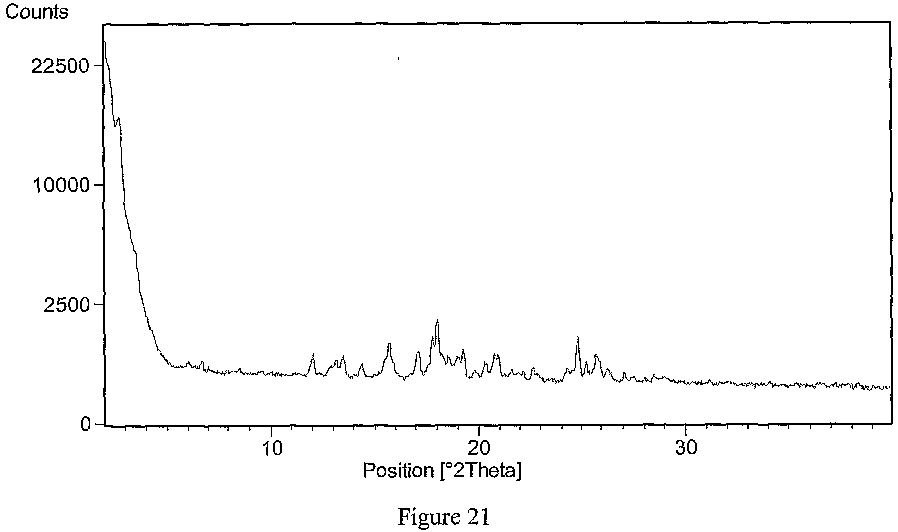

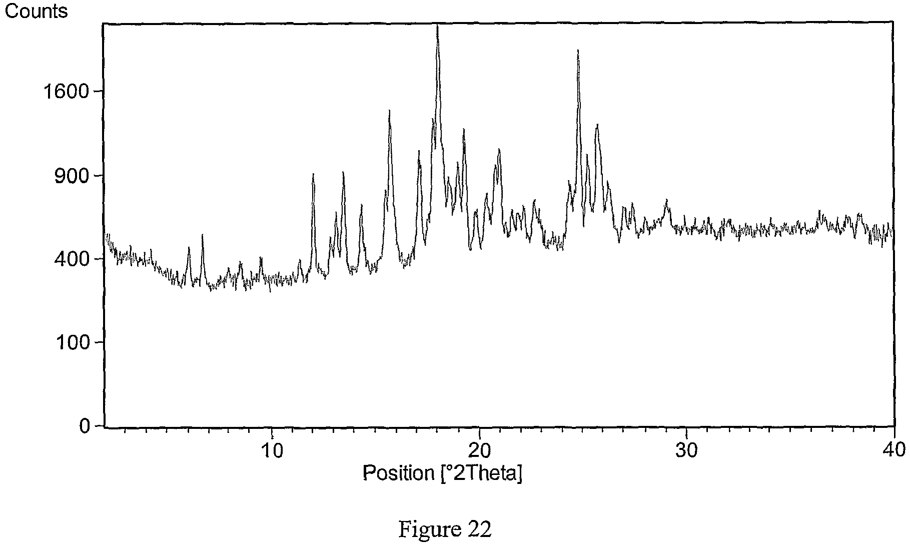

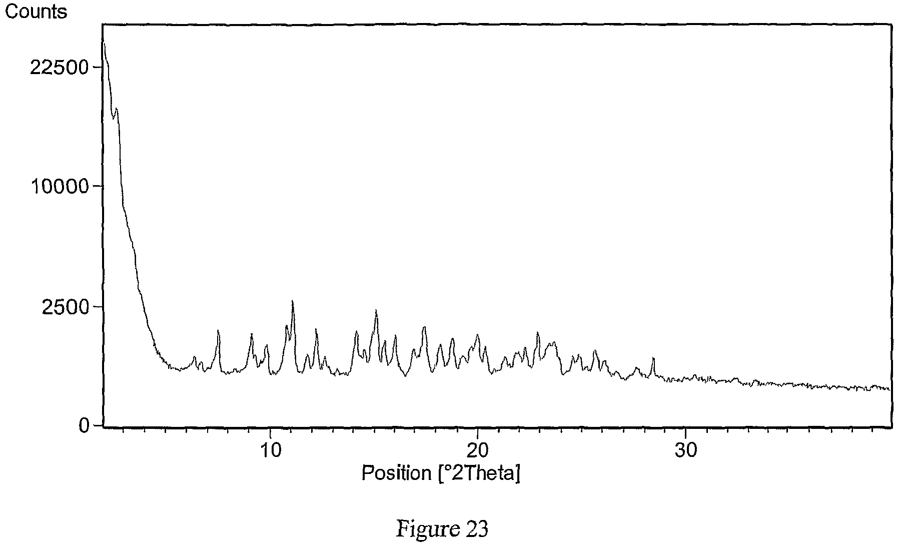

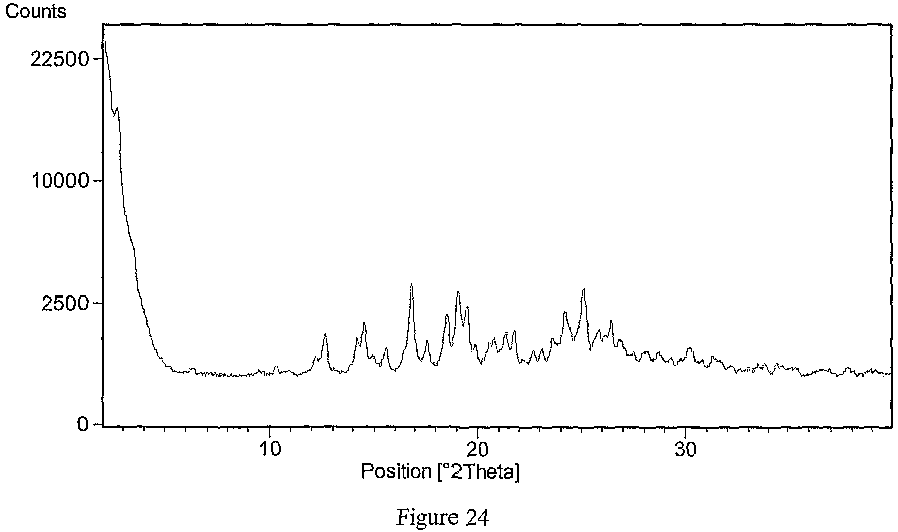

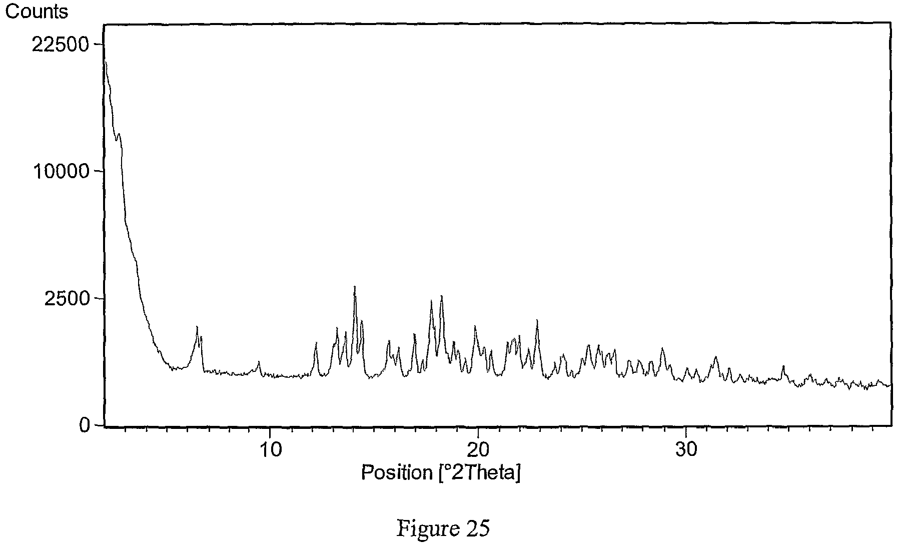

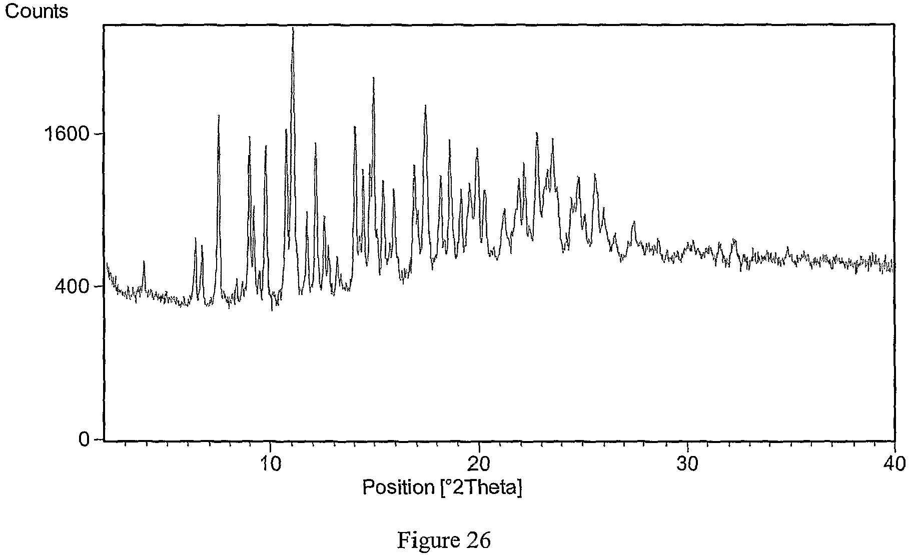

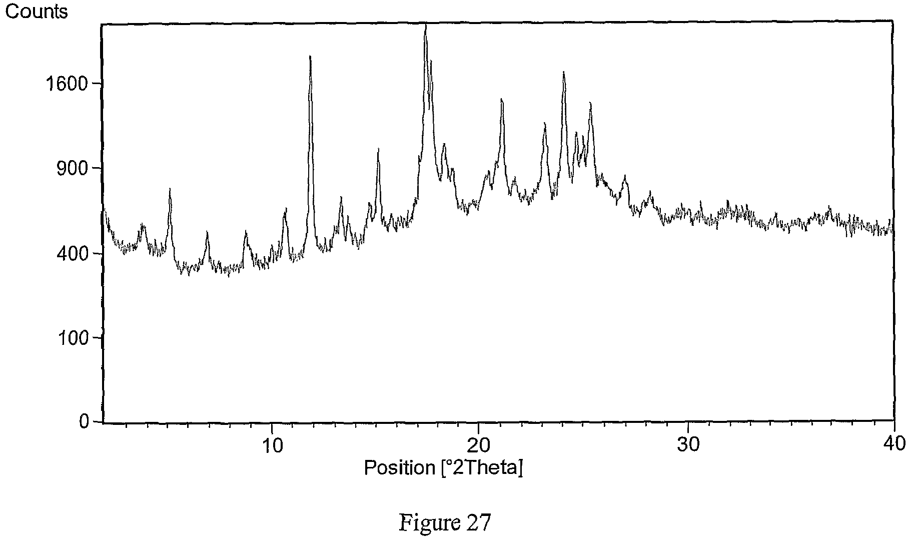

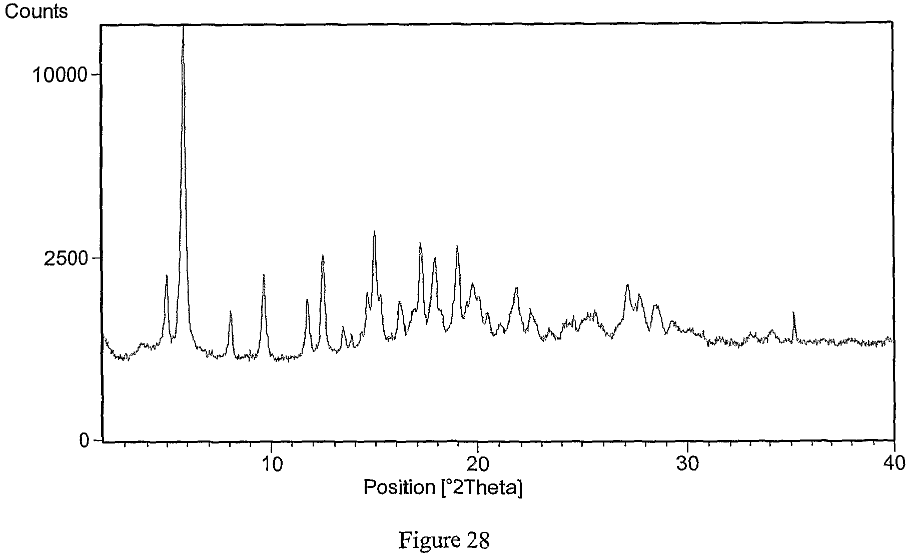

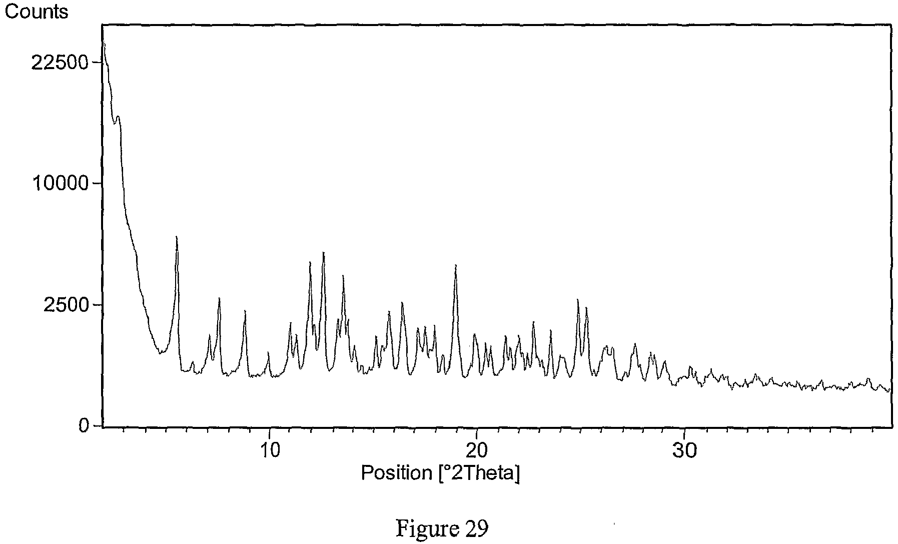

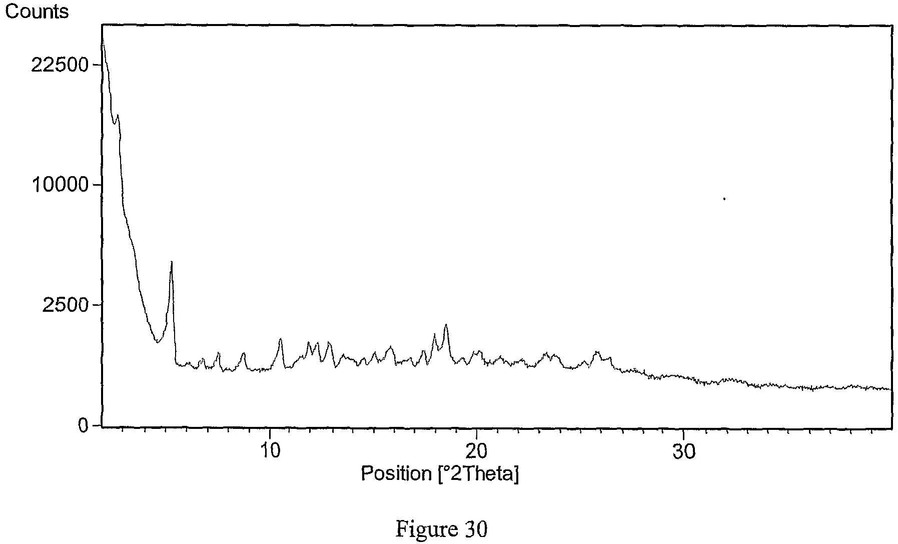

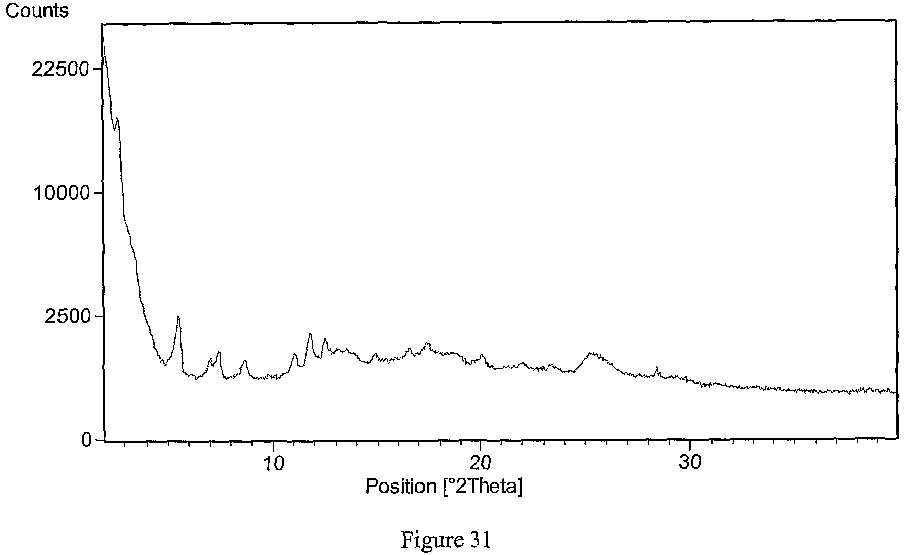

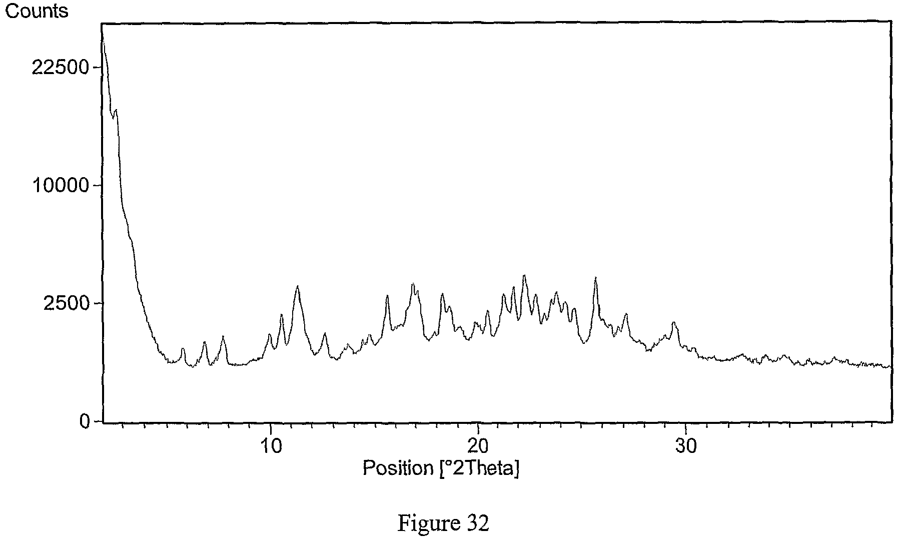

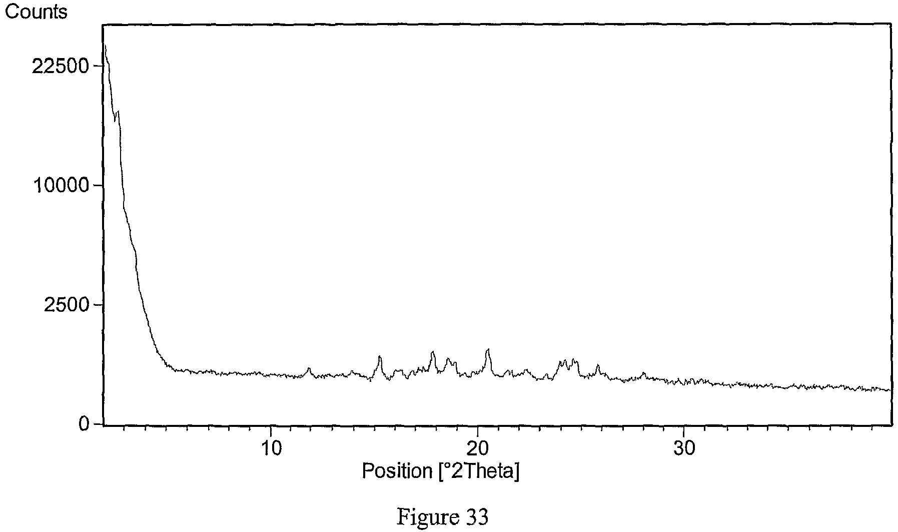

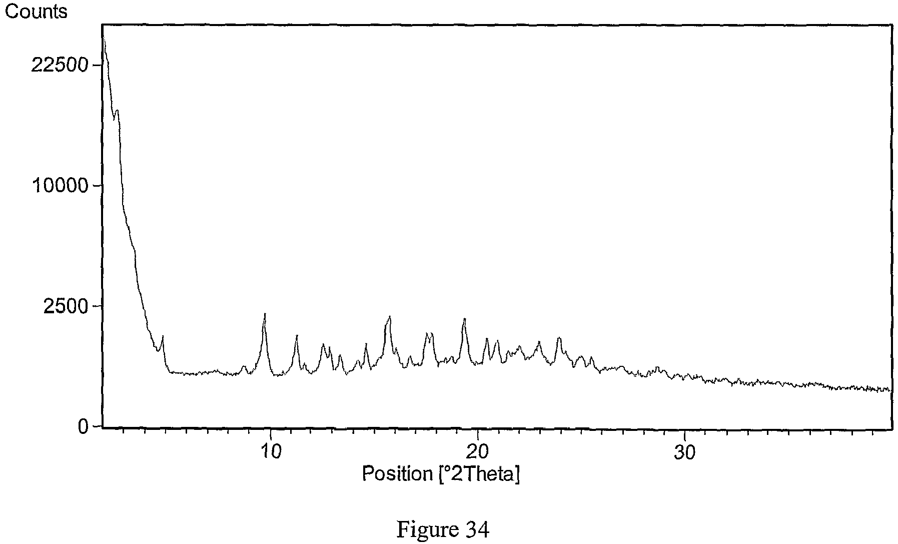

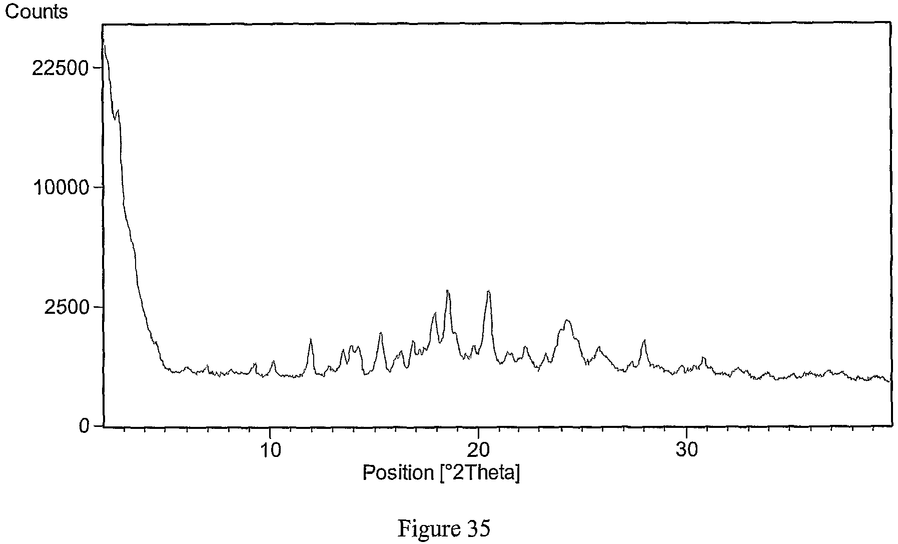

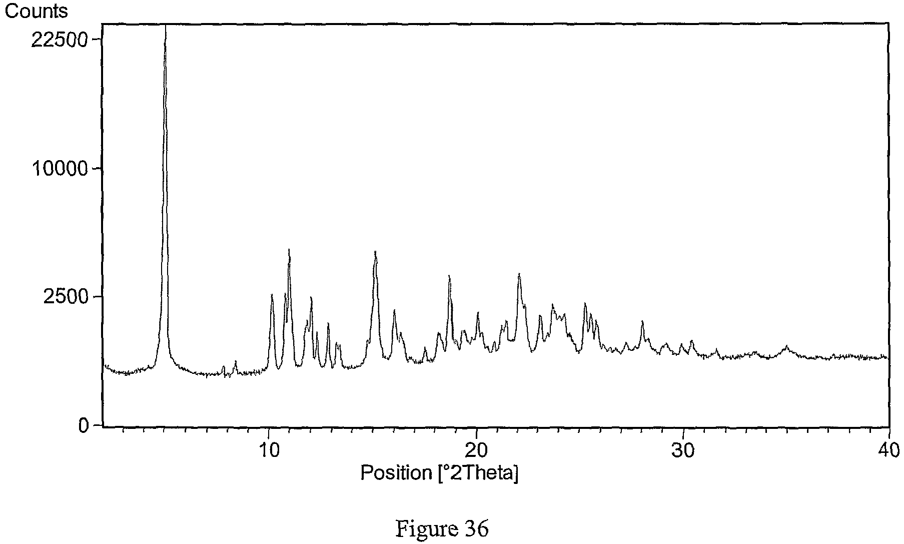

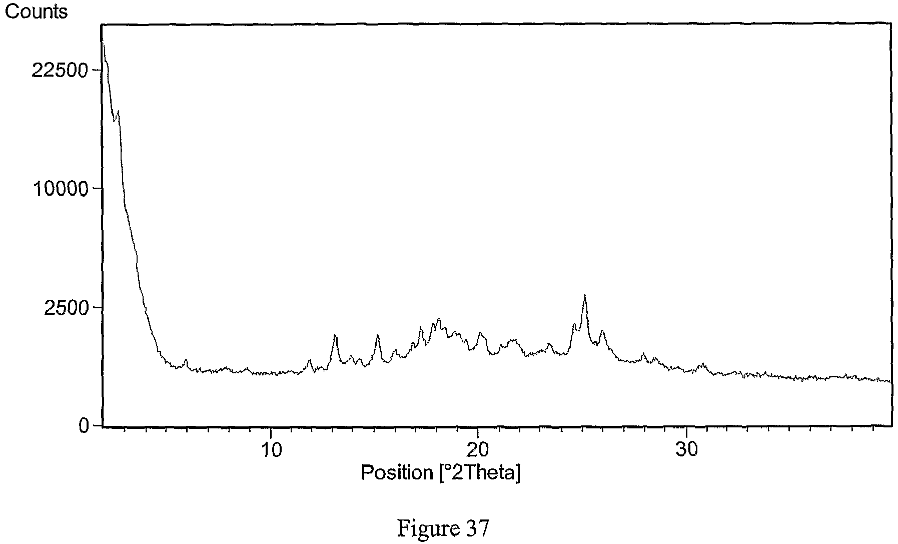

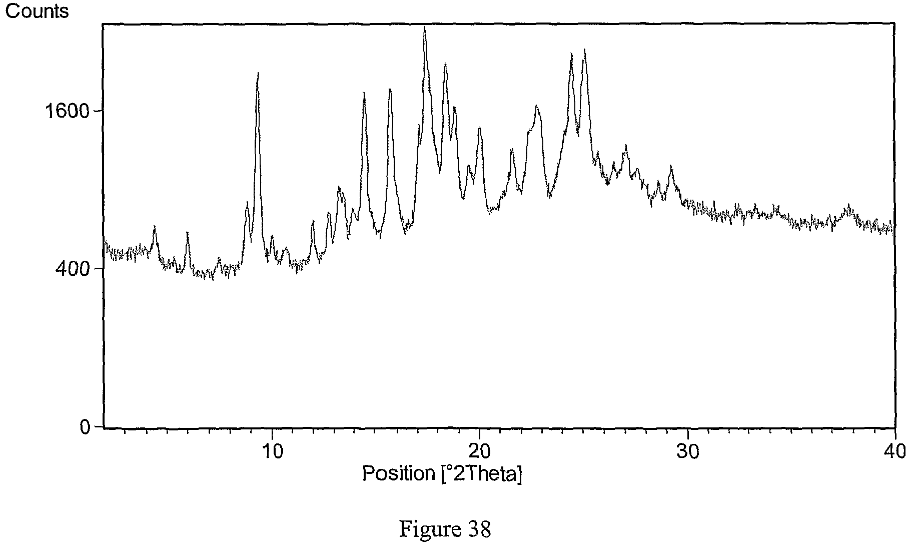

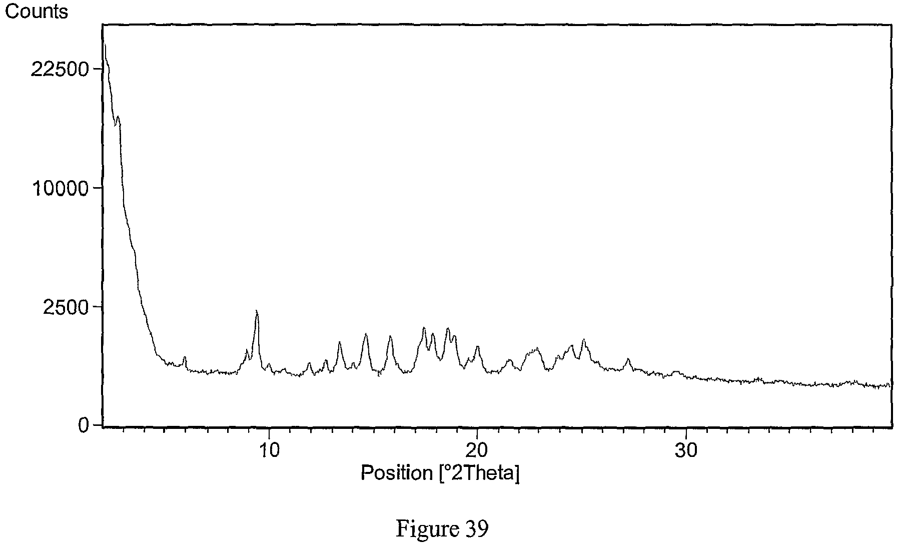

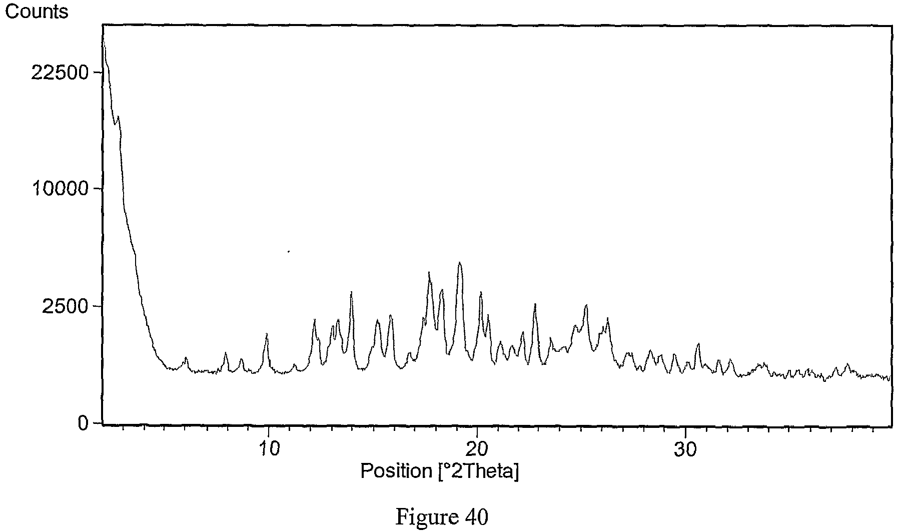

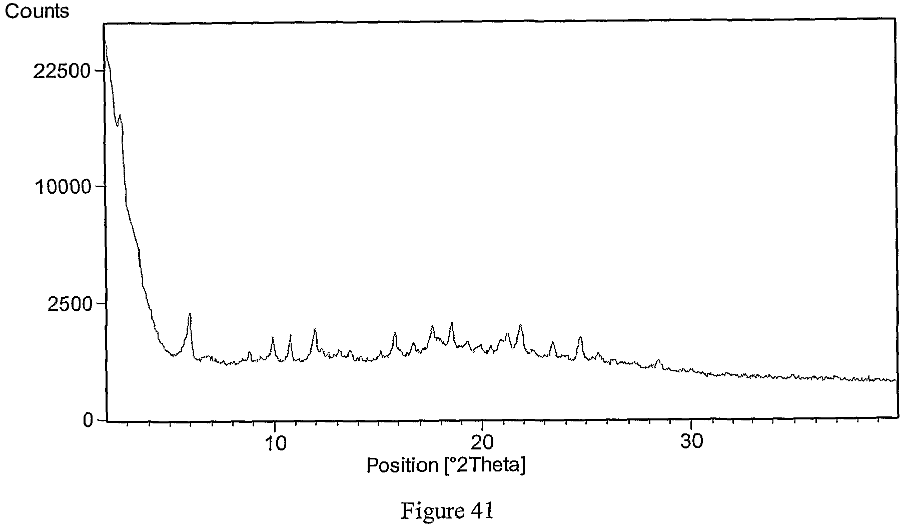

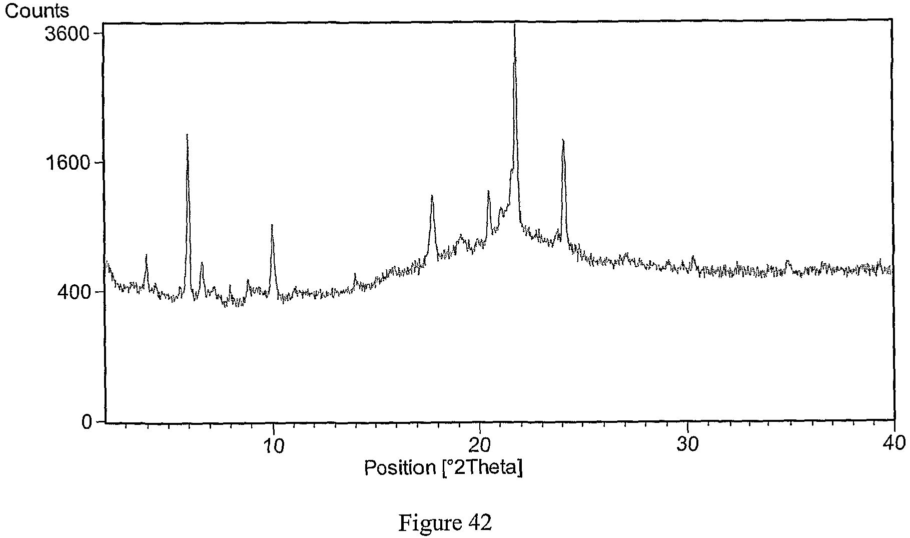

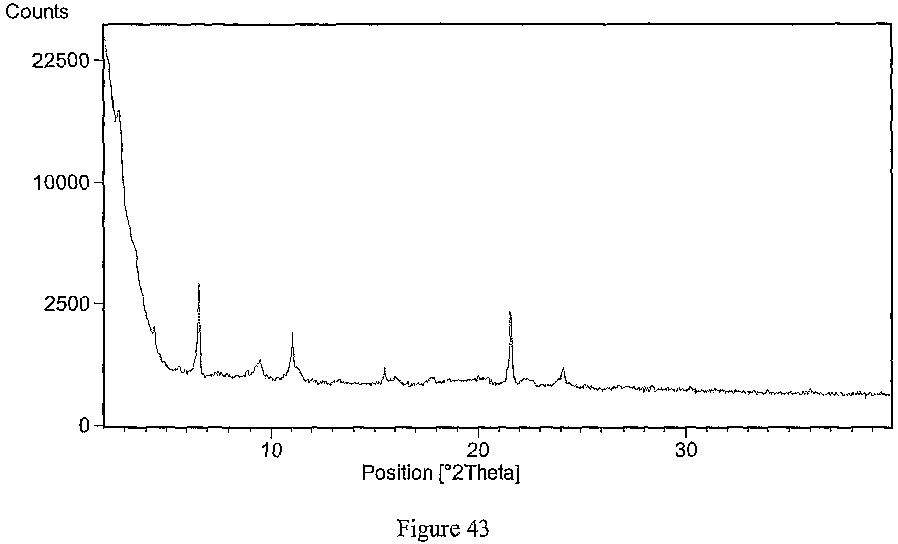

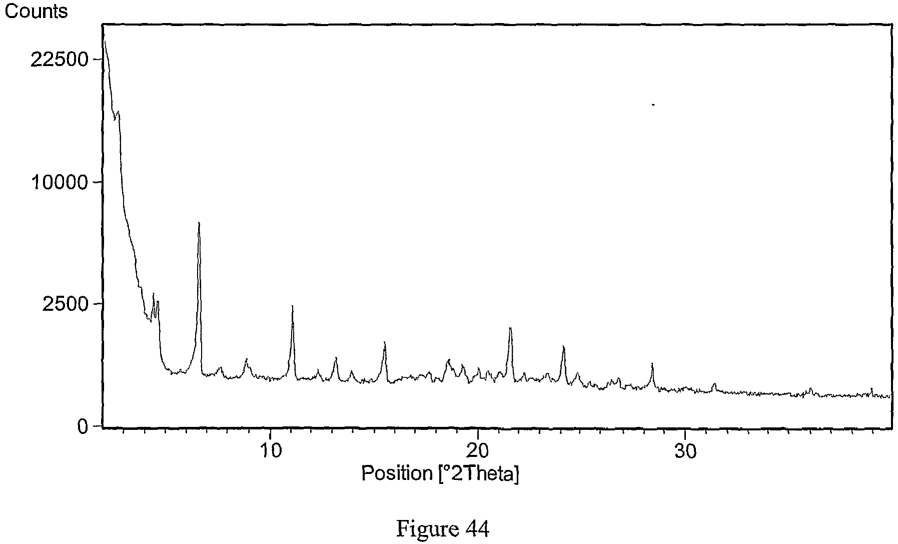

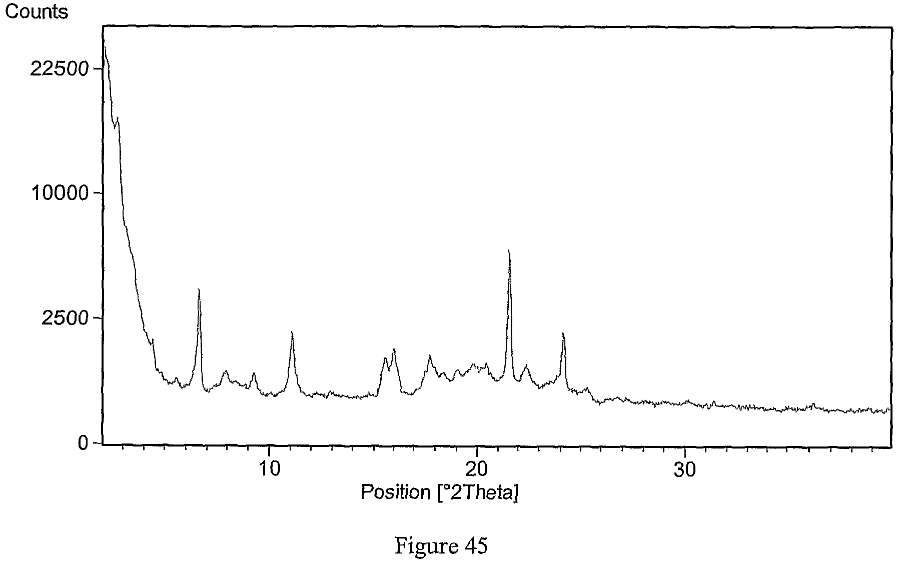

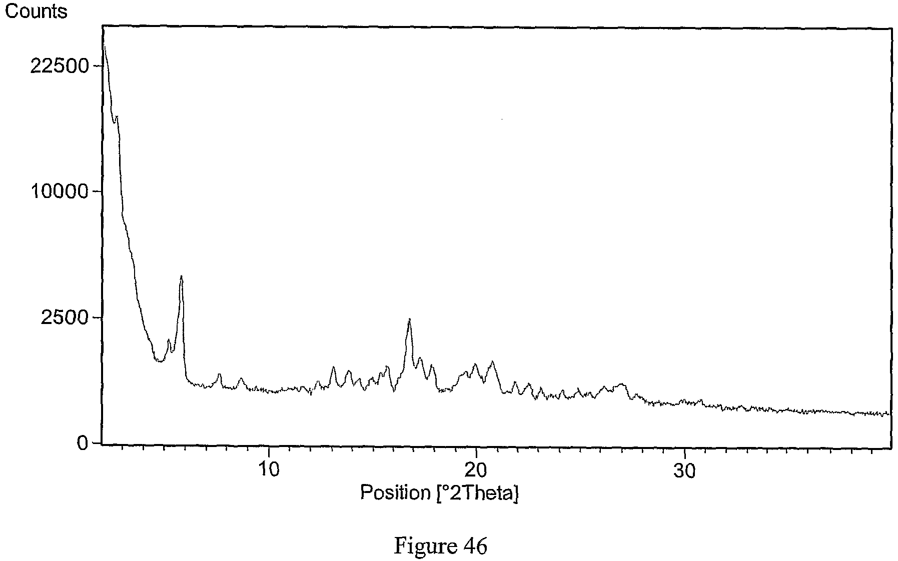

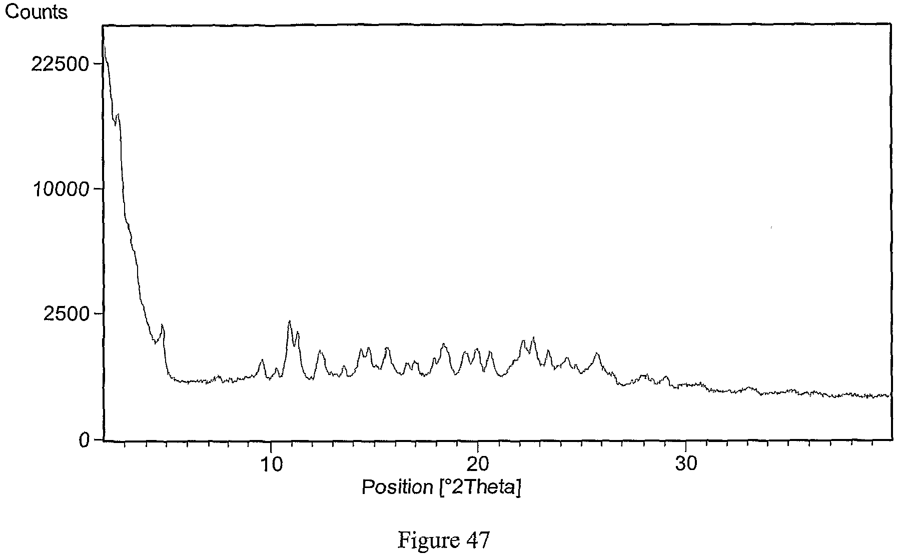

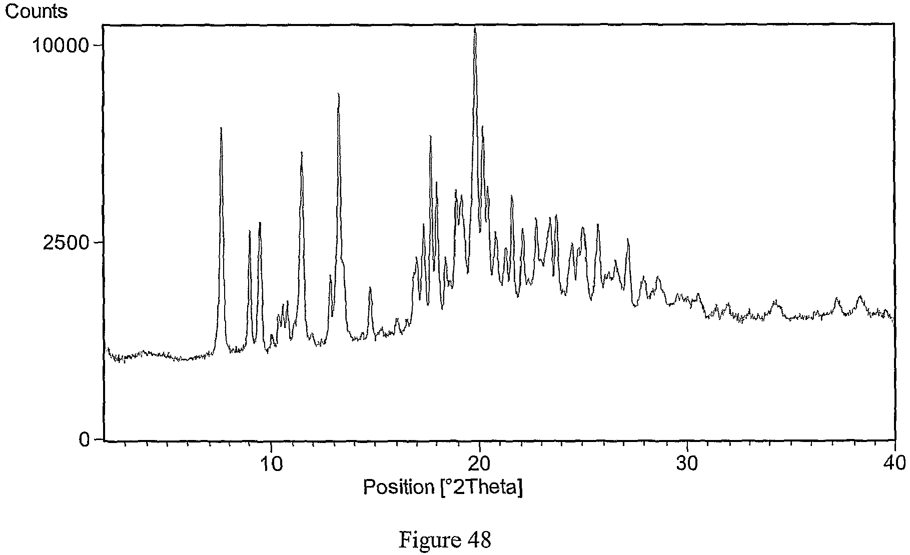

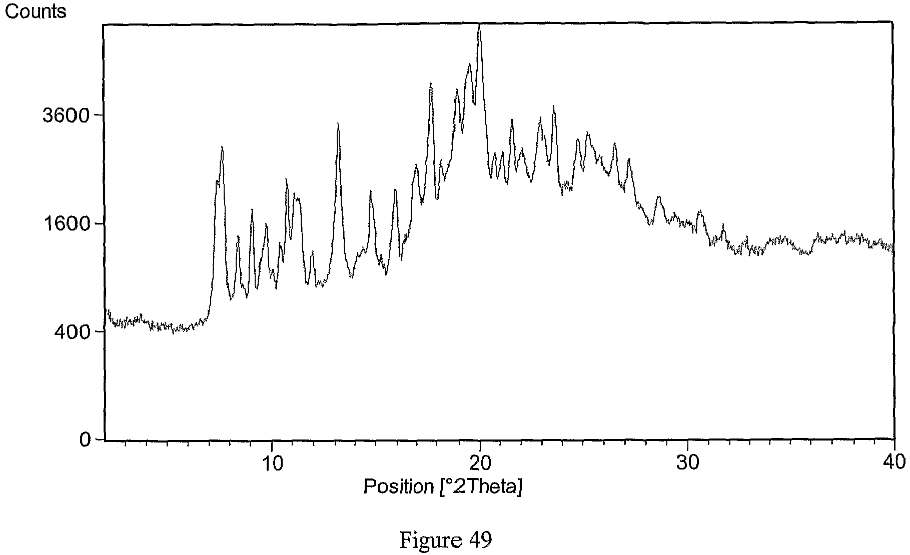

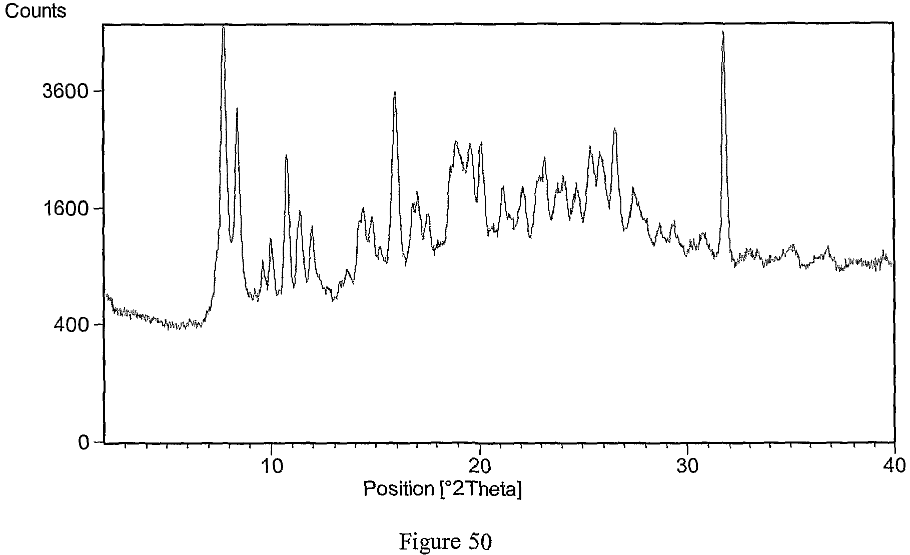

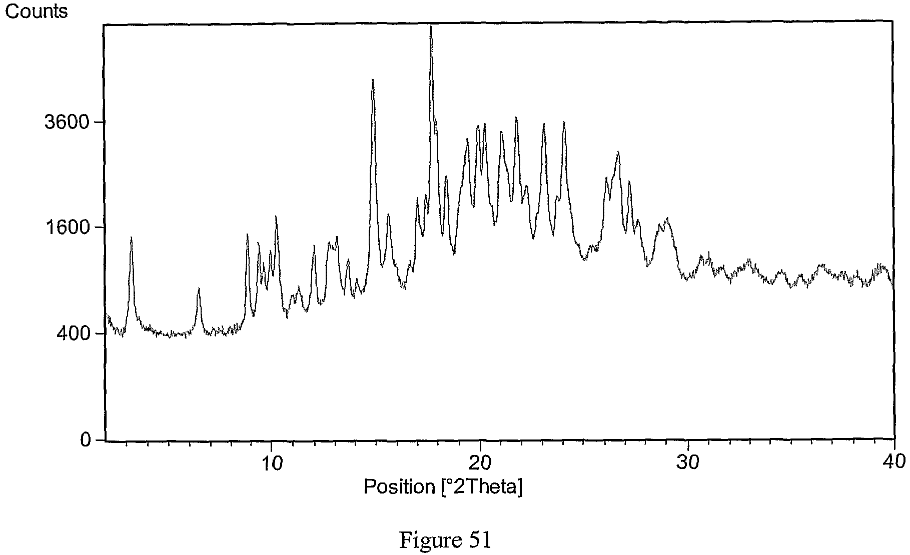

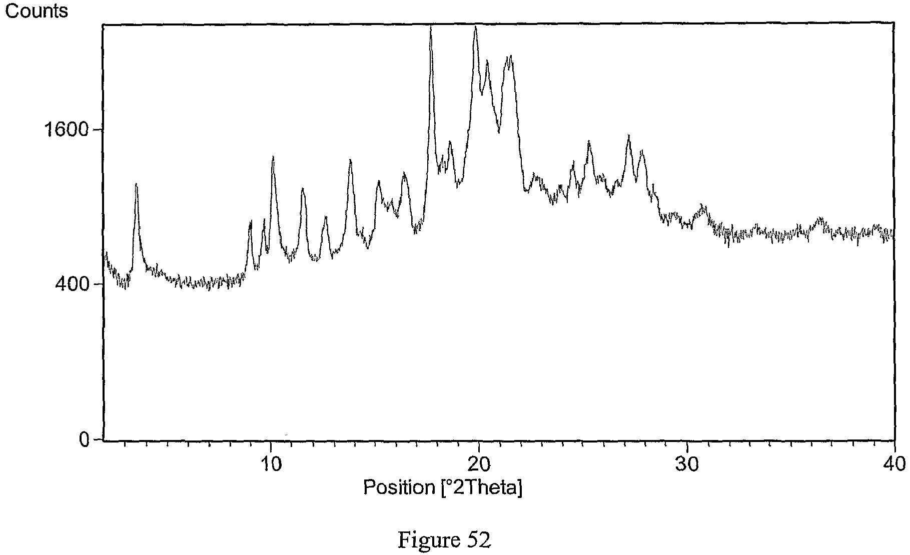

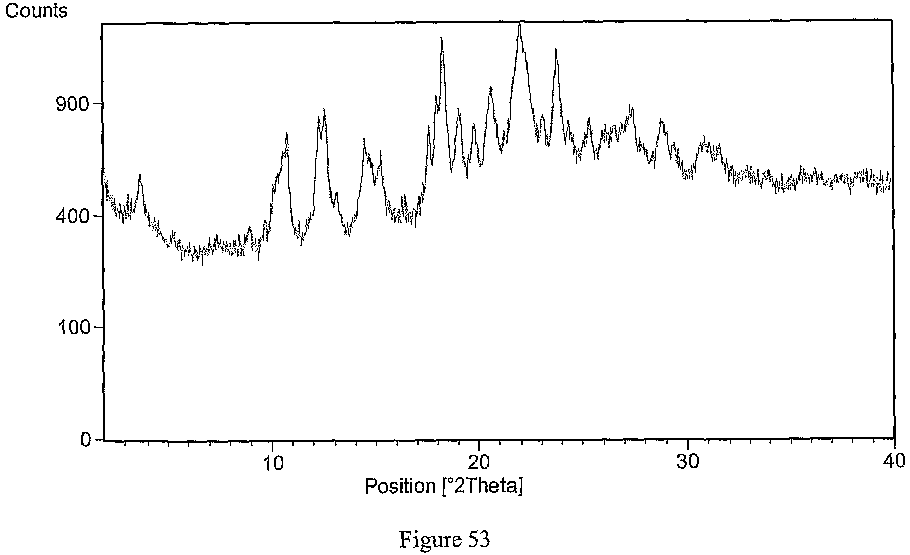

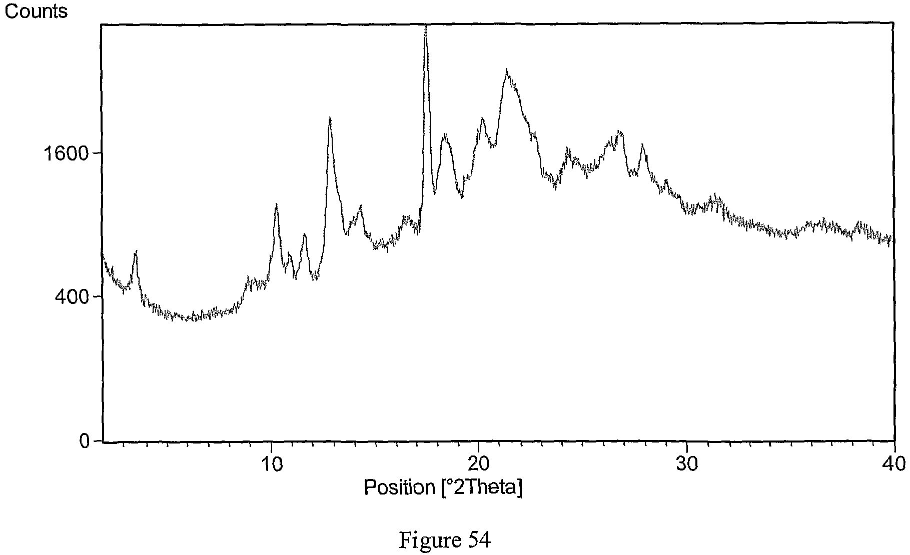

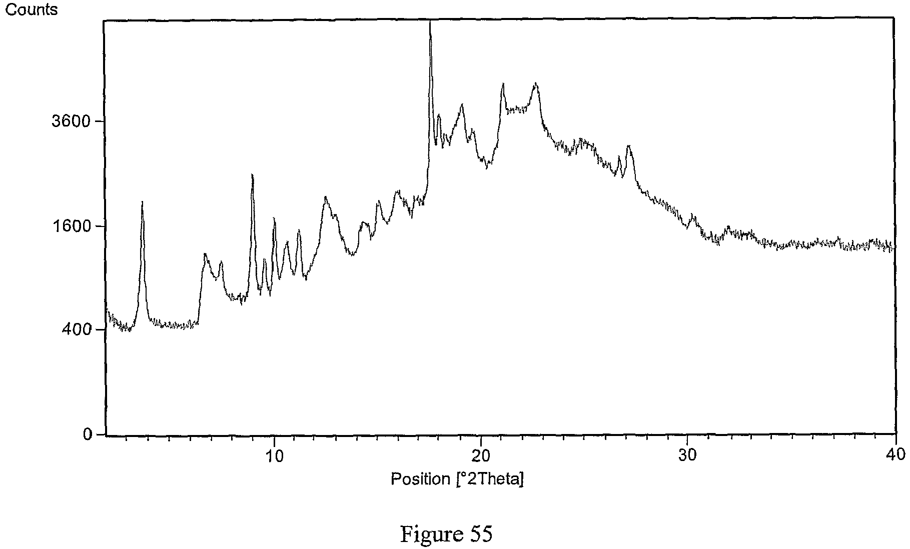

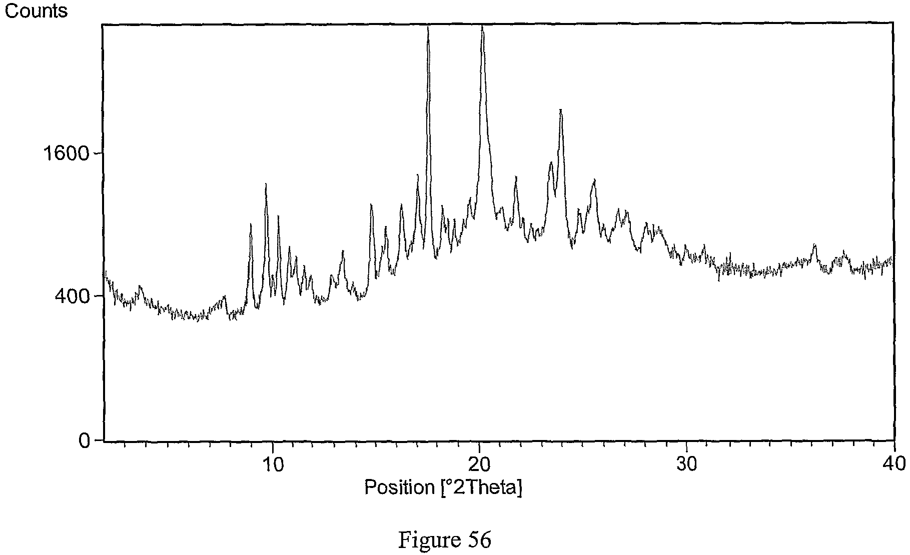

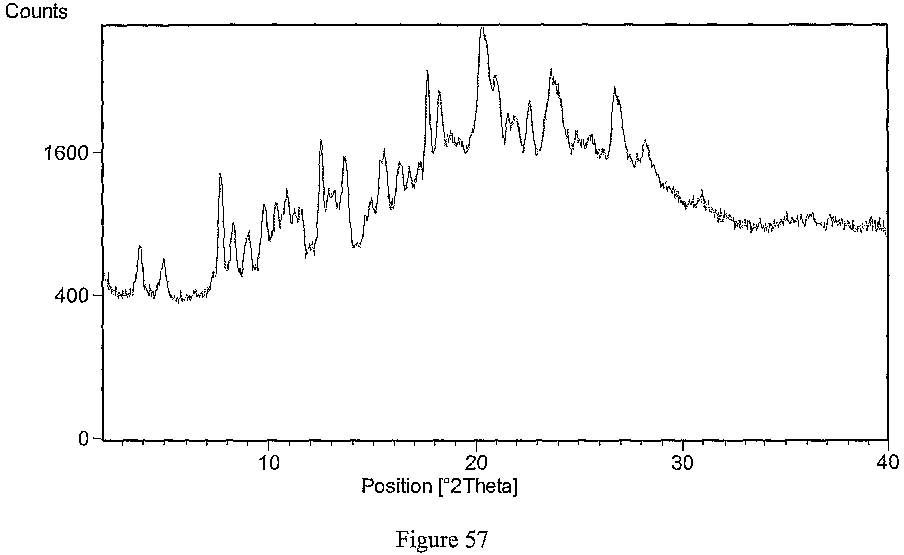

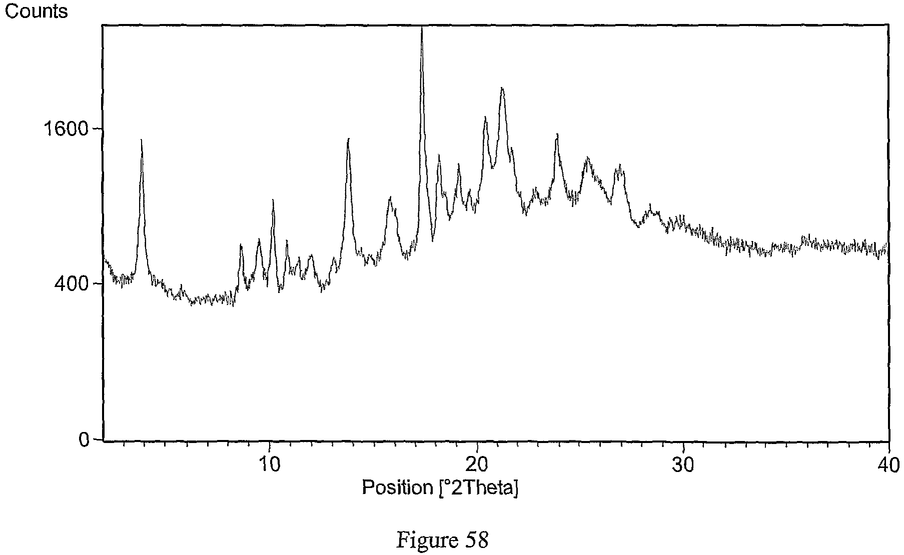

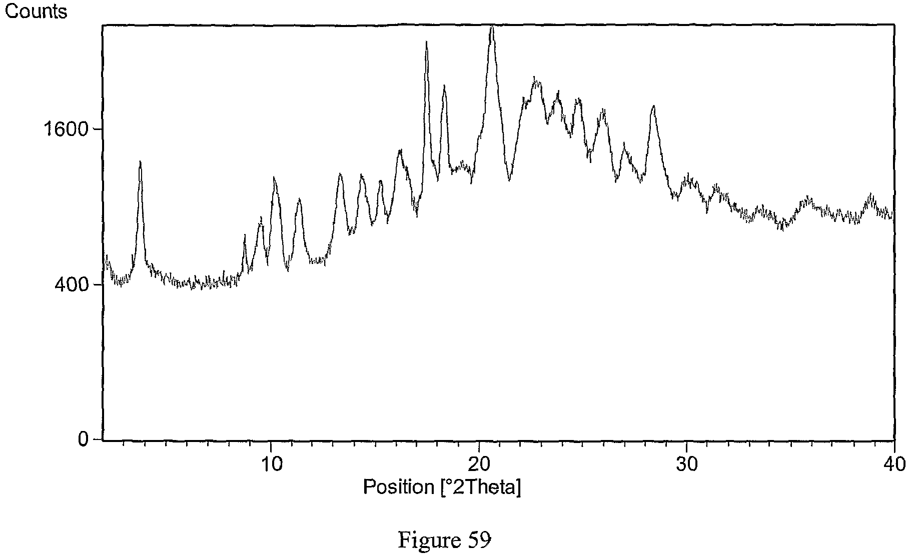

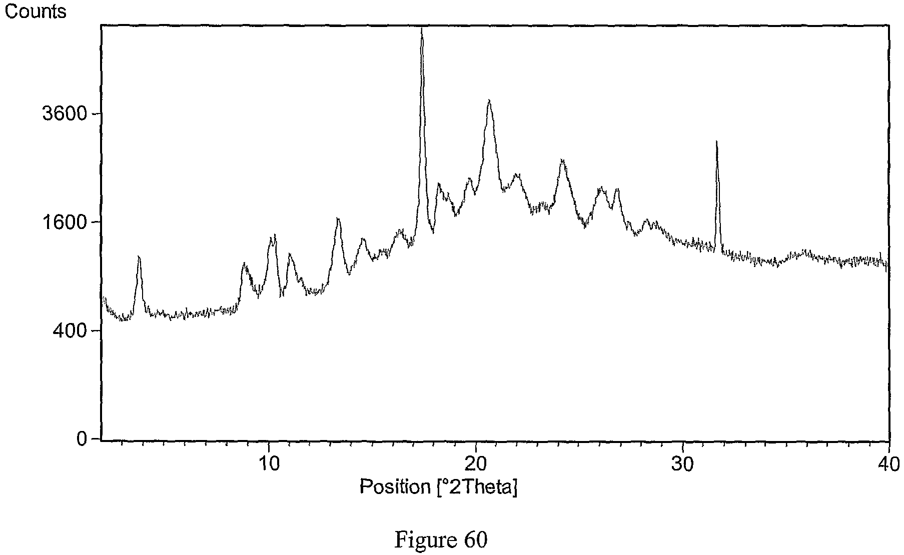

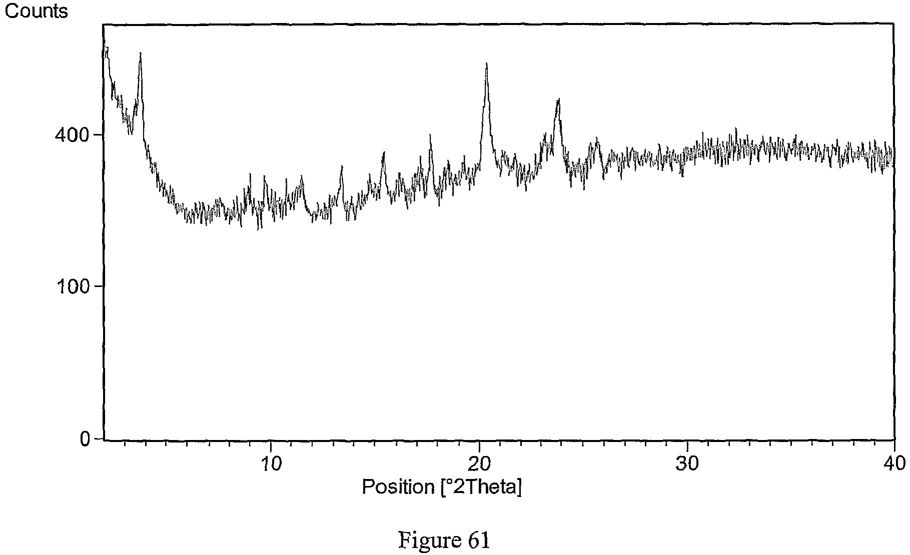

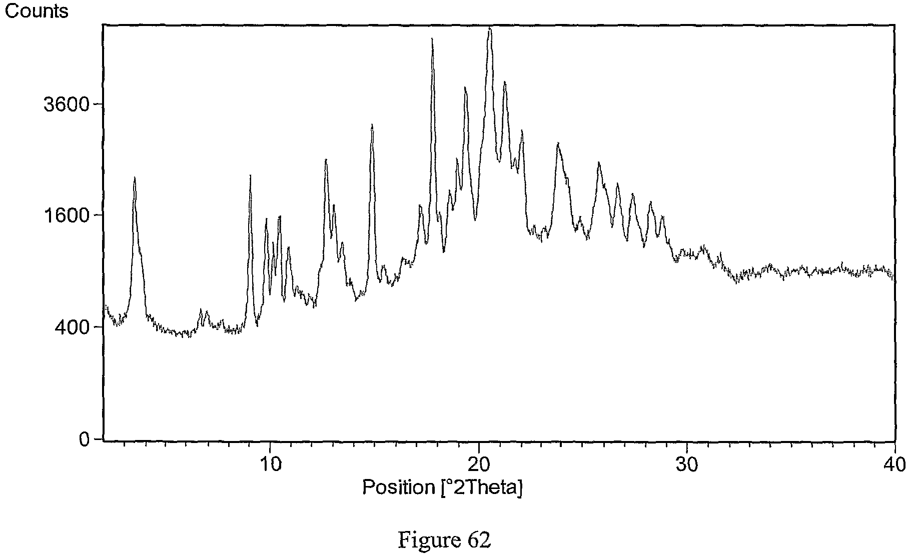

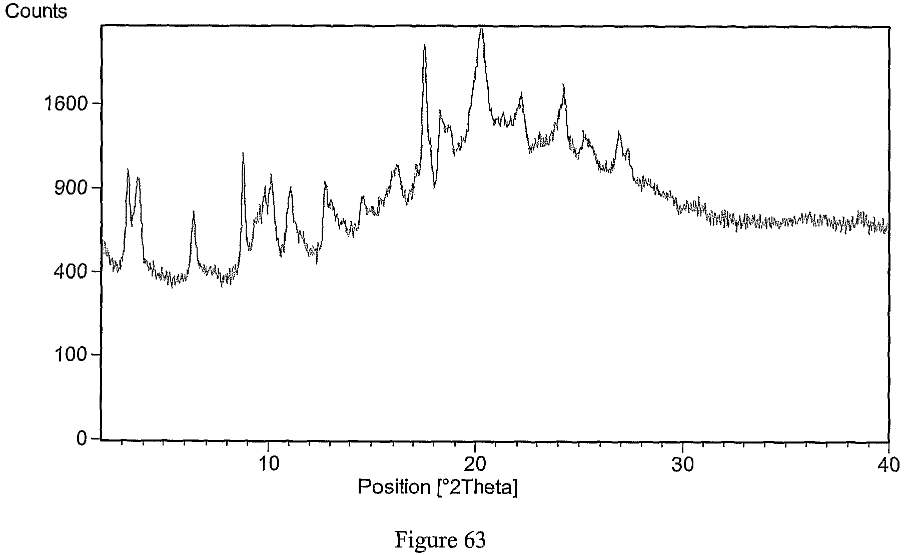

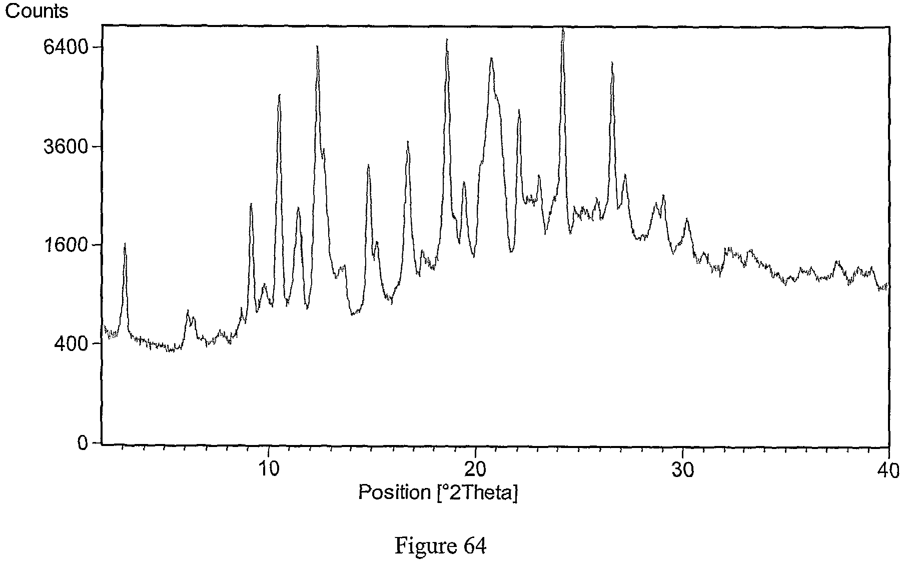

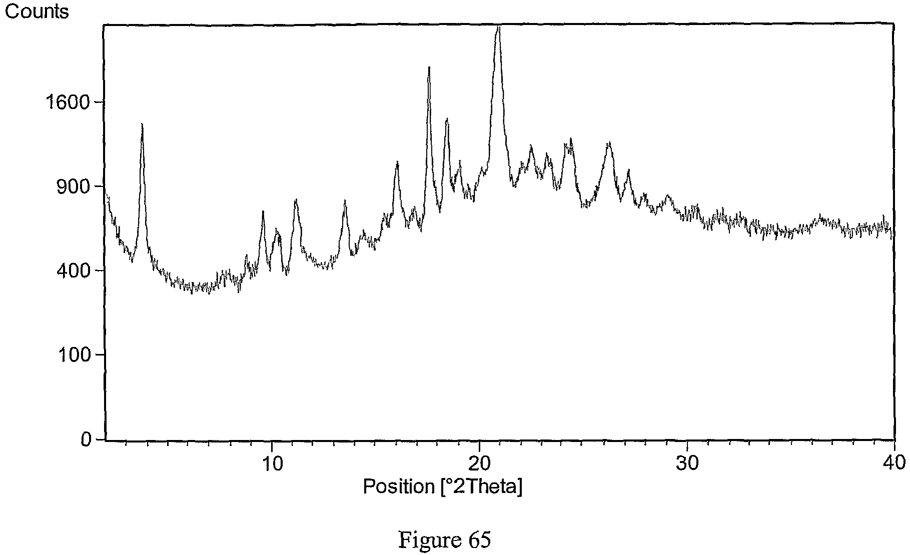

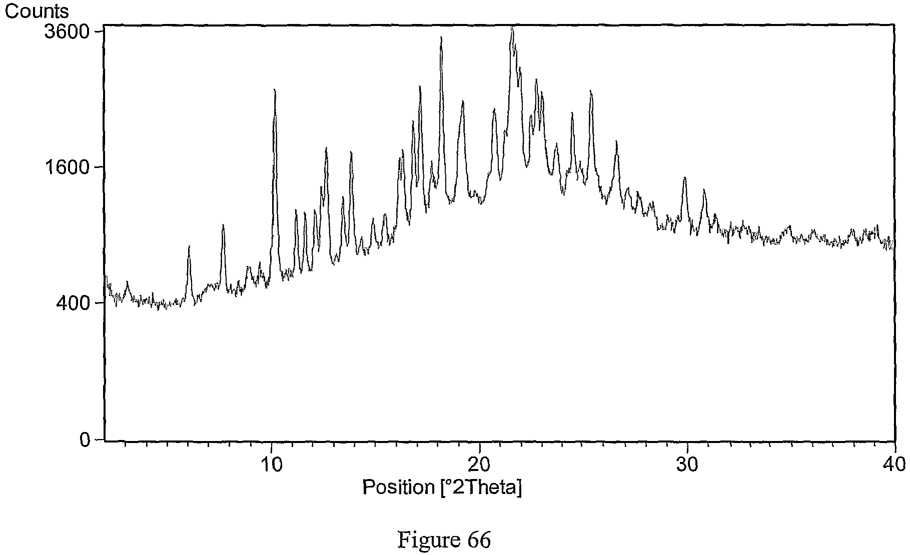

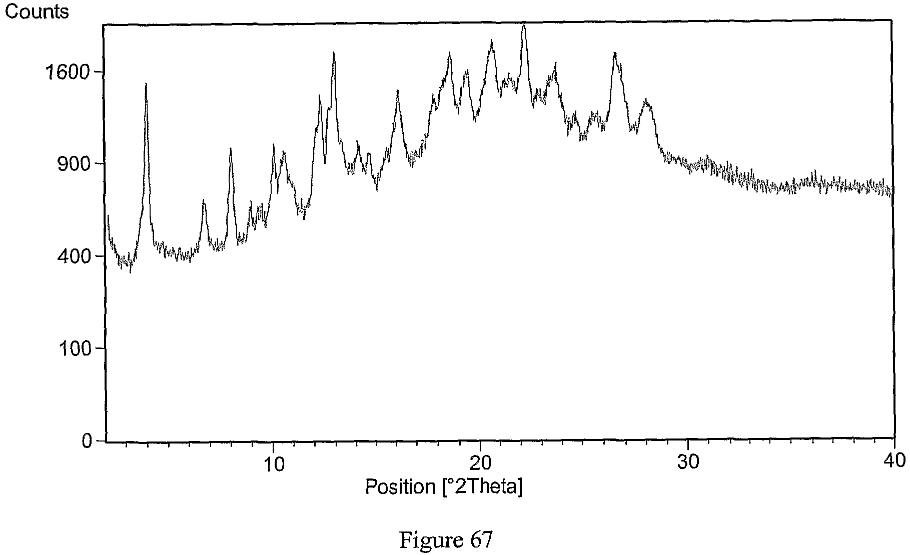

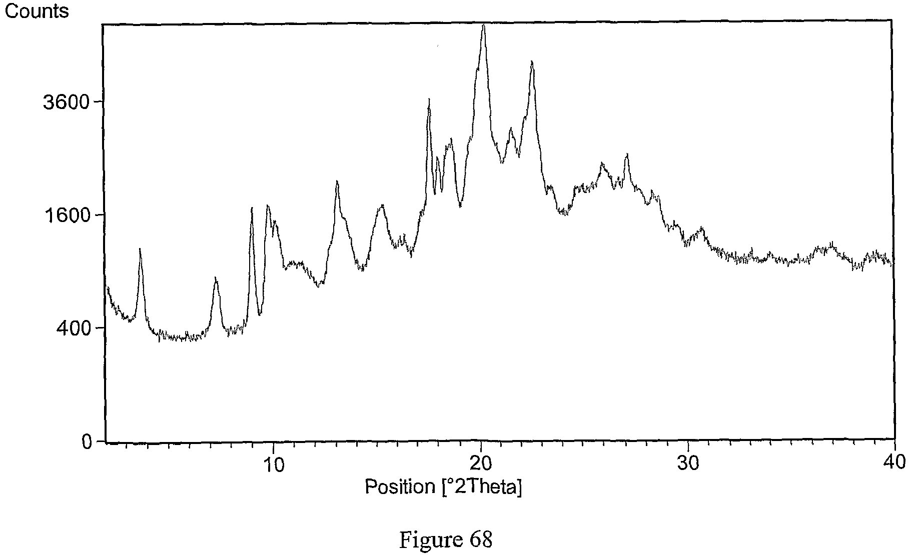

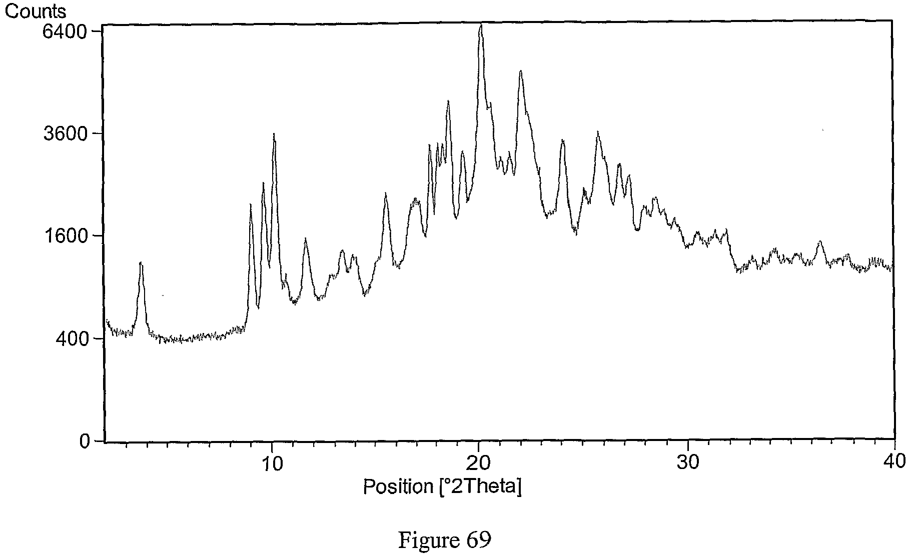

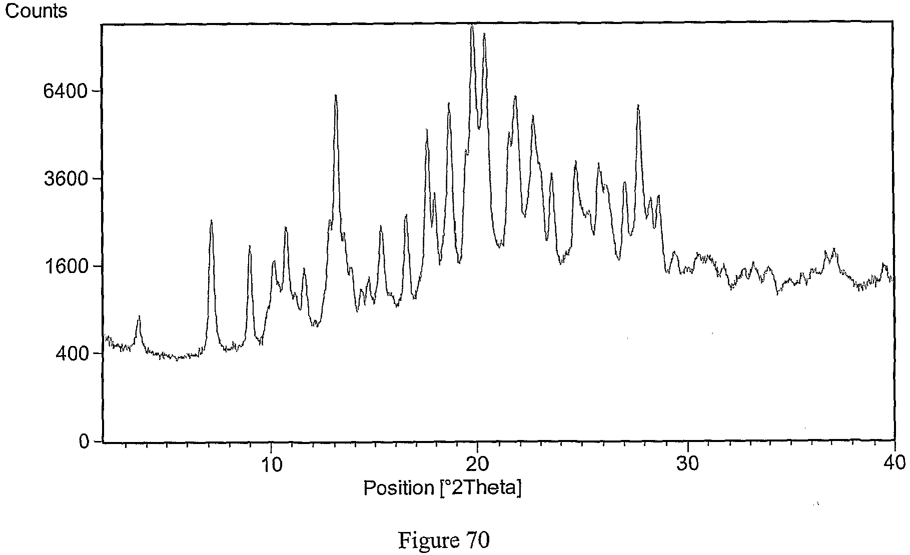

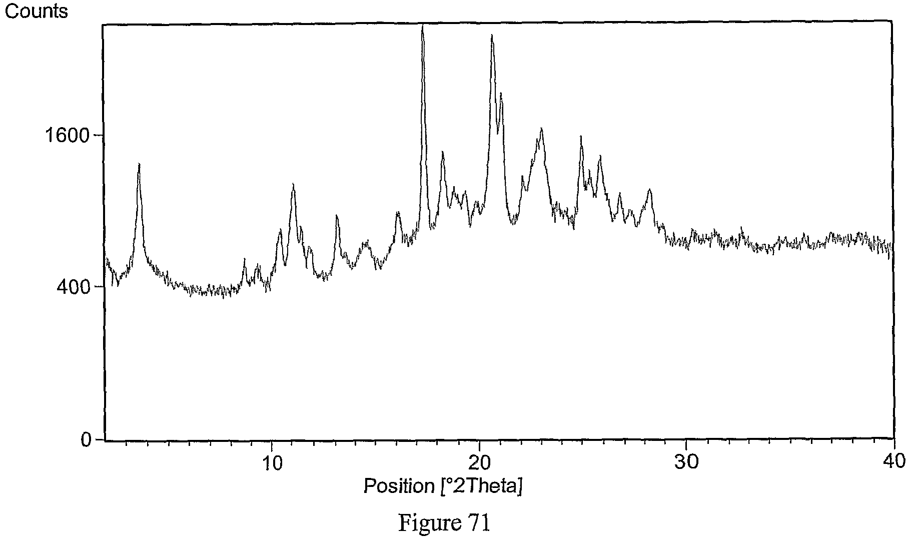

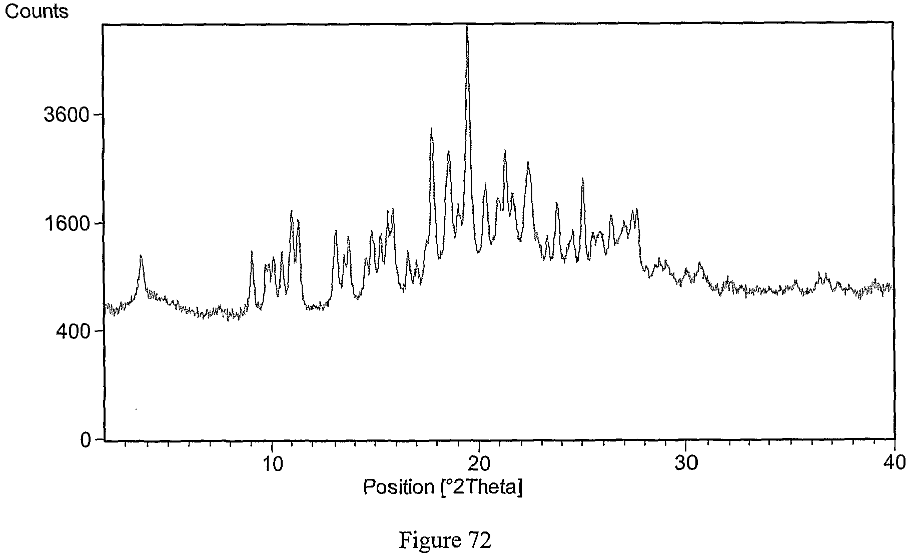

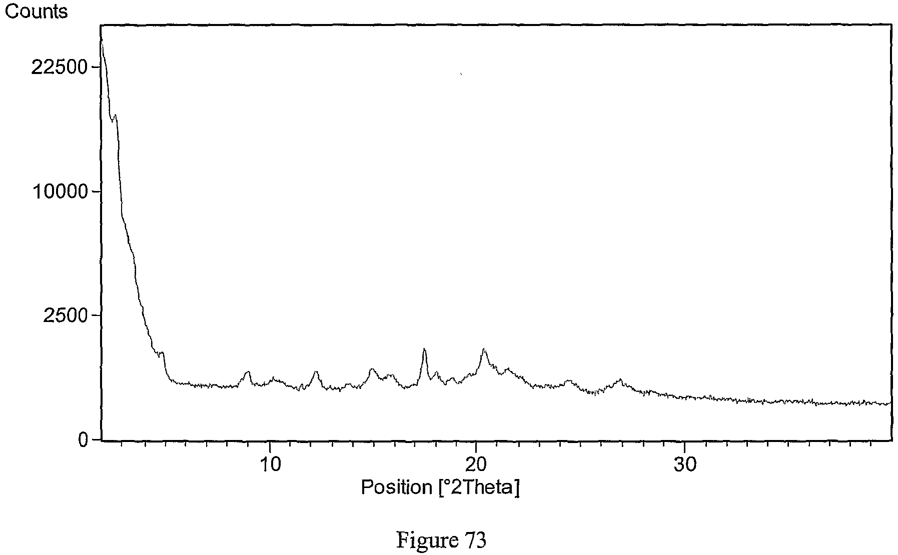

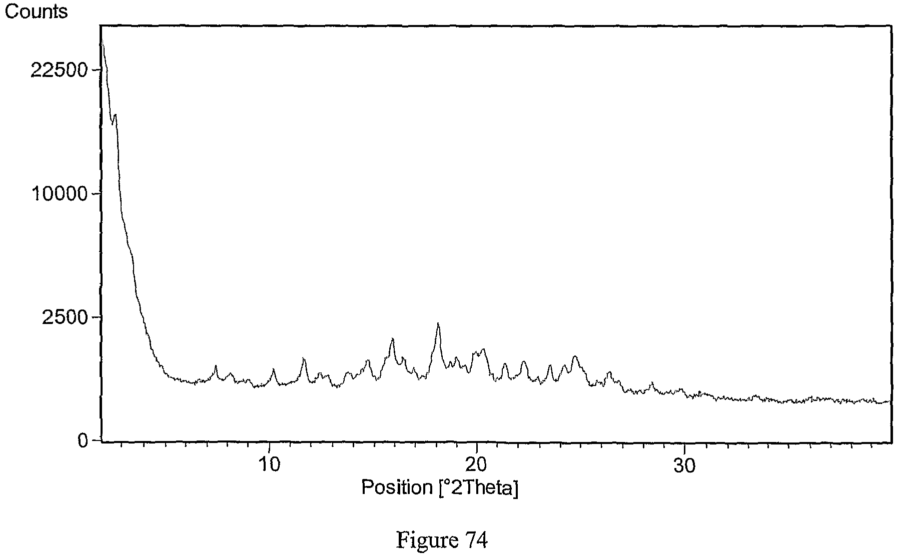

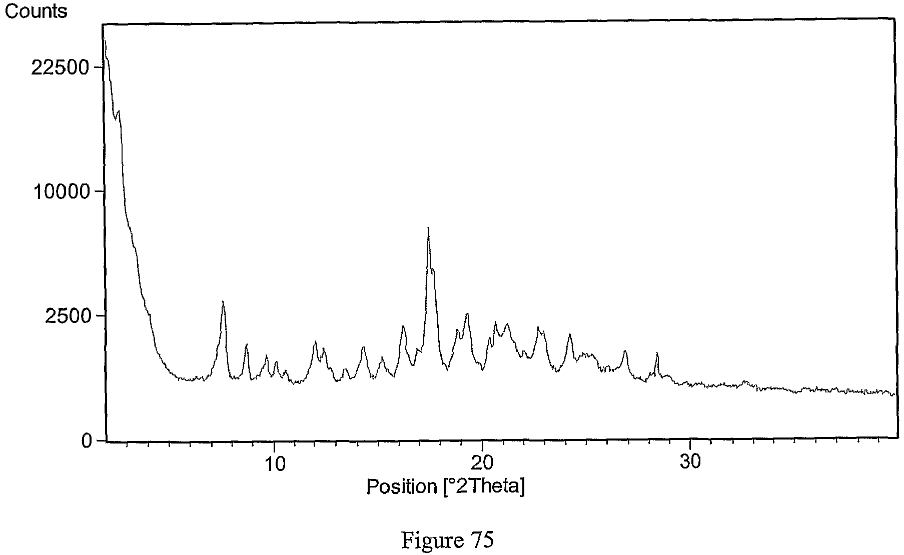

The following compounds (Table 5) were prepared in a similar manner using similar methodology described above in Example 51.

**Table 5**

| **Example Number** | **Chemistry** | **Name** | **NMR** | **M+H** |
|---|---|---|---|---|
| 207 | 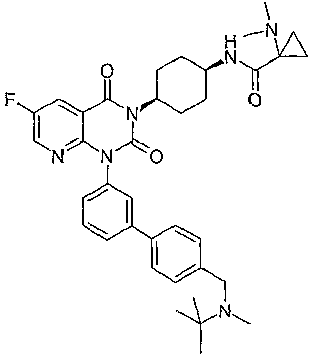 | *N*-{*cis*-4-[1-(4'-{[*tert-*butyl(methyl)amino]methyl}biphe nyl-3-yl)-6-fluoro-2,4-dioxo-1,4-dihydropyrido[2,3-*d*]pyrimidin-3(2*H*)-yl]cyclohexyl}-1-(dimethylamino)cyclopropanecarb oxamide | ¹H NMR (400 MHz, DMSO) δ 9.23 - 9.05 (m, 1H), 8.59 (d, J = 2.8 Hz, 1H), 8.32 (dd, J = 3.1, 7.4 Hz, 1H), 7.87 - 7.77 (m, 4H), 7.68 - 7.61 (m, 3H), 7.42 (d, J = 8.2 Hz, 1H), 4.87 - 4.75 (m, 1H), 4.66 (d, J = 12.8 Hz, 1H), 4.24 - 3.65 (m, 10H), 2.60 - 2.53 (m, 4H), 2.01 - 1.91 (m, 2H), 1.63 - 1.50 (m, 3H), 1.48 - 1.42 (m, 9H), 1.42 - 1.29 (m, 4H). Other resonances obscured by water peak. | 641 |
| 208 | 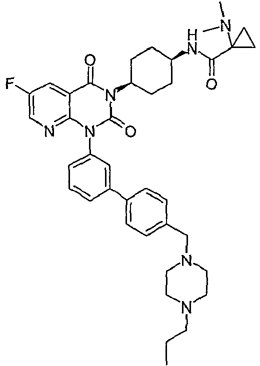 | 1-(dimethylamino)-N-{cis-4-[6-fluoro-2,4-dioxo-1-{4'-[(4-propylpiperazin-1-yl)methyl]biphenyl-3-yl}-1,4-dihydropyrido[2,3-d]pyrimidin-3(2H)-yl]cyclohexyl}cyclopropanecarbox amide | ¹H NMR (400 MHz, DMSO) δ 8.59 (d, J = 2.8 Hz, 1H), 8.31 (dd, J = 7.7, 3.1 Hz, 1H), 7.79 (d, J = 7.9 Hz, 1H), 7.71 (t, J = 7.8 Hz, 3H), 7.62 (t, J = 7.8 Hz, 1H), 7.48 (d, J = 8.2 Hz, 2H), 7.39 (d, J = 8.7 Hz, 1H), 4.81 (t, J = 12.0 Hz, 1H), 3.16 - 2.91 (m, 6H), 2.84 - 2.64 (m, 6H), 2.03 - 1.91 (m, 2H), 1.69 - 1.29 (m, 10H), 0.90 (t, J = 8.1 Hz, 4H). Other resonances obscured by DMSO peak. | 682 |
| 209 | 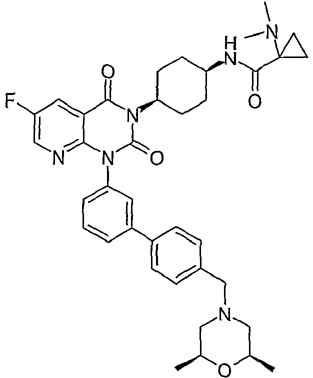 | 1-(dimethylamino)-*N*-{*cis*-4-[1-(4'-{[(2*R*,6*S*)-2,6-dimethylmorpholin-4-yl]methyl}biphenyl-3-yl)-6-fluoro-2,4-dioxo-1,4-dihydropyrido[2,3-*d*]pyrimidin-3(2*H*)-yl]cyclohexyl}cyclopropanecarbox amide | ¹H NMR (400 MHz, DMSO) δ 8.61 - 8.57 (m, 1H), 8.35 - 8.29 (m, 1H), 7.87 - 7.74 (m, 3H), 7.68 - 7.57 (m, 3H), 7.42 (d, J = 7.4 Hz, 1H), 4.87 - 4.74 (m, 1H), 4.41 - 4.30 (m, 1H), 3.90 - 3.74 (m, 2H), 3.43 - 3.24 (m, 10H), 2.80 - 2.54 (m, 4H), 2.01 - 1.87 (m, 2H), 1.64 - 1.45 (m, 2H), 1.42 - 1.17 (m, 2H), 1.18 - 0.98 (m, 10H). Resonances partially obscured by DMSO peak. | 669 |
| 210 | 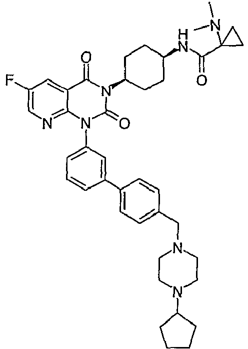 | N-{cis-4-[1-{4'-[(4-cyclopentylpiperazin-1-yl)methyl]biphenyl-3-yl}-6-fluoro-2,4-dioxo-1,4-dihydropyrido[2,3-d]pyrimidin-3(2H)-yl]cyclohexyl}-1-(dimethylamino)cyclopropanecarb oxamide | ¹H NMR (400 MHz, DMSO) δ 8.59 (d, J = 2.8 Hz, 1H), 8.31 (dd, J = 3.1, 7.7 Hz, 1H), 7.79 (d, J = 7.7 Hz, 1H), 7.75 - 7.67 (m, 3H), 7.63 (t, J = 7.8 Hz, 1H), 7.48 (d, J = 7.7 Hz, 2H), 7.39 (d, J = 7.7 Hz, 1H), 4.87 - 4.75 (m, 1H), 3.20-2.95 (m, 4H), 2.83 - 2.64 (m, 4H), 2.05 - 1.88 (m, 4H), 1.75 - 1.29 (m, 15H). Other resonances obscured by DMSO peak. | 708 |
| 211 | 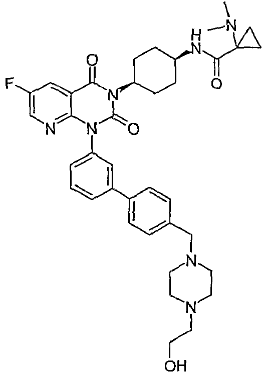 | 1-(dimethylamino)-N-{cis-4-[6-fluoro-1-(4'-{[4-(2-hydroxyethyl)piperazin-1-yl]methyl}biphenyl-3-yl)-2,4-dioxo-1,4-dihydropyrido[2,3-d]pyrimidin-3(2H)-yl]cyclohexyl}cyclopropanecarbox amide | ¹H NMR (400 MHz, DMSO) δ 8.59 (d, J = 3.1 Hz, 1H), 8.31 (dd, J = 7.7, 3.1 Hz, 1H), 7.79 (d, J = 7.7 Hz, 1H), 7.71 (t, J = 8.6 Hz, 3H), 7.62 (t, J = 7.8 Hz, 1H), 7.48 (d, J = 8.2 Hz, 2H), 7.39 (d, J = 7.9 Hz, 1H), 4.81 (t, J = 11.1 Hz, 1H), 4.09 - 3.47 (m, 10H), 3.20 - 3.10 (m, 4H), 2.77 - 2.62 (m, 4H), 2.01 - 1.89 (m, 2H), 1.63 - 1.47 (m, 5H), 1.47 - 1.24 (m, 5H). Other resonances obscured by DMSO peak. | 684 |
| 212 | 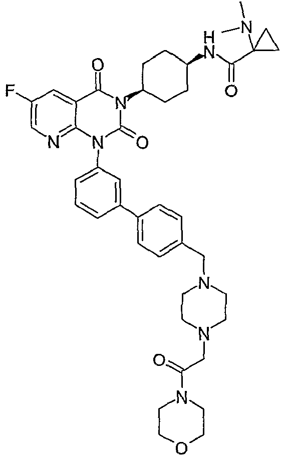 | 1-(dimethylamino)-*N-*{*cis*-4-[6-fluoro-1-(4'-{[4-(2-morpholin-4-yl-2-oxoethyl)piperazin-1-yl]methyl}biphenyl-3-yl)-2,4-dioxo-1,4-dihydropyrido[2,3-d]pyrimidin-3(2*H*)-yl]cyclohexyl}cyclopropanecarbox amide | ¹H NMR (400 MHz, DMSO) δ 8.59 (d, J = 2.8 Hz, 1H), 8.32 (dd, J = 7.7,2.8 Hz, 1H), 7.81 (d, J = 7.7 Hz, 1H), 7.74 (d, J = 7.4 Hz, 3H), 7.63 (t, J = 7.8 Hz, 1H), 7.54 (d, J = 7.9 Hz, 2H), 7.40 (d, J = 7.9 Hz, 1H), 4.81 (t, J = 12.2 Hz, 1H), 4.15 - 4.04 (m, 2H), 4.03 - 3.93 (m, 1H), 3.84 (s, 1H), 3.63 - 3.54 (m, 4H), 3.49 - 3.44 (m, 2H), 3.42 - 3.37 (m, 2H), 3.29 - 2.94 (m, 8H), 2.82 - 2.73 (m, 5H), 2.60 - 2.53 (m, 2H), 2.03 - 1.94 (m, 2H), 1.63 - 1.40 (m, 8H). Other resonances obscured by DMSO peak. | 767 |
| 213 | 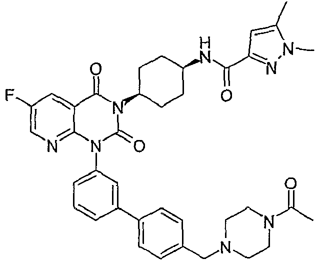 | N-{cis-4-[1-{4'-[(4-acetylpiperazin-1-yl)methyl]biphenyl-3-yl}-6-fluoro-2,4-dioxo-1,4-dihydropyrido[2,3-d]pyrimidin-3(2H)-yl]cyclohexyl}-1,5-dimethyl-1H-pyrazole-3-carboxamide | ¹H NMR (400 MHz, DMSO) δ 8.59 (d, J = 2.8 Hz, 1H), 8.34 (dd, J = 7.8, 2.9 Hz, 1H), 7.84-7.76 (m, 4H), 7.65 (t, J = 7.8 Hz, 1H), 7.59 (d, J = 8.2 Hz, 2H), 7.43 (d, J = 8.7 Hz, 1H), 7.20 (d, J = 6.9 Hz, 1H), 6.42 (s, 1H), 4.85 (t, J = 12.0 Hz, 1H), 4.38 (s, 2H), 4.08 (s, 1H), 3.75 (s, 3H), 3.59 - 3.36 (m, 6H), 3.11 (s, 1H), 2.93 (s, 1H), 2.61 - 2.52 (m, 2H), 2.25 (s, 3H), 2.04 (s, 3H), 1.96 (d, J = 12.3 Hz, 2H), 1.71 - 1.59 (m, 4H). | 693 |
| 214 | 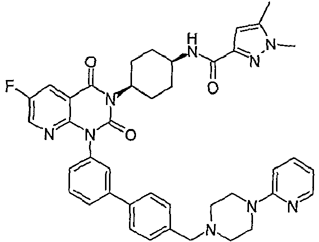 | N-{cis-4-[6-fluoro-2,4-dioxo-1-{4'-[(4-pyridin-2-ylpiperazin-1-yl)methyl]biphenyl-3-yl}-1,4-dihydropyrido[2,3-d]pyrimidin-3(2H)-yl]cyclohexyl}-1,5-dimethyl-1H-pyrazole-3-carboxamide | ¹H NMR (400 MHz, DMSO) δ 8.59 (d, J = 2.8 Hz, 1H), 8.34 (dd, J = 7.8, 2.9 Hz, 1H), 8.16 (dd, J = 4.9, 1.3 Hz, 1H), 7.85 - 7.77 (m, 4H), 7.68 - 7.60 (m, 4H), 7.43 (dd, J = 8.3, 0.4 Hz, 1H), 7.20 (d, J = 7.2 Hz, 1H), 6.95 (d, J = 8.7 Hz, 1H), 6.76 (dd, J = 6.8, 5.3 Hz, 1H), 6.42 (s, 1H), 4.85 (s, 1H), 4.45 - 4.37 (m, 4H), 4.08 (s, 2H), 3.75 (s, 3H), 3.49 - 3.39 (m, 2H), 3.18 - 3.08 (m, 4H), 2.25 (s, 3H), 1.99 - 1.93 (m, 2H), 1.71 - 1.59 (m, 4H). | 728 |
| 215 | 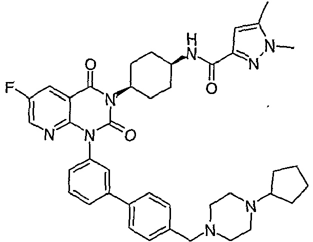 | N-{cis-4-[1-{4'-[(4-cyclopentylpiperazin-1-yl)methyl]biphenyl-3-yl}-6-fluoro-2,4-dioxo-1,4-dihydropyrido[2,3-d]pyrimidin-3(2H)-yl]cyclohexyl}-1,5-dimethyl-1H-pyrazole-3-carboxamide | ¹H NMR (400 MHz, DMSO) δ 8.59 (d, J = 2.3 Hz, 1H), 8.33 (dd, J = 7.8, 1.9 Hz, 1H), 7.79 (d, J = 7.9 Hz, 1H), 7.74 - 7.69 (m, 3H), 7.63 (t, J = 7.8 Hz, 1H), 7.49 (d, J = 7.7 Hz, 2H), 7.40 (d, J = 7.9 Hz, 1H), 7.20 (d, J = 7.2 Hz, 1H), 6.42 (s, 1H), 4.85 (t, J = 11.9 Hz, 1H), 4.08 (s, 1H), 3.75 (s, 3H), 3.54 - 3.46 (m, 5H), 3.16 - 3.06 (m, 4H), 2.61 - 2.53 (m, 2H), 2.26 (s, 3H), 2.01 - 1.93 (m, 4H), 1.70 - 1.50 (m, 12H). | 719 |
| 216 | 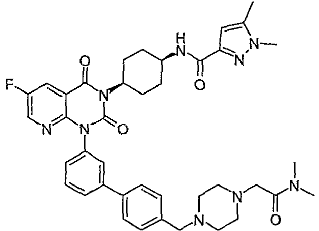 | N-{cis-4-[1-[4'-({4-[2-(dimethylamino)-2-oxoethyl]piperazin-1-yl}methyl)biphenyl-3-yl]-6-fluoro-2,4-dioxo-1,4-dihydropyrido[2,3-d)pyrimidin-3(2H)-yl)cyclohexyl}-1,5-dimethyl-1H-pyrazole-3-carboxamide | ¹H NMR (400 MHz, DMSO) δ 8.59 (d, J = 3.1 Hz, 1H), 8.33 (dd, J = 7.7, 3.1 Hz, 1H), 7.80 (d, J = 7.9 Hz, 1H), 7.76 - 7.71 (m, 3H), 7.63 (t, J = 7.8 Hz, 1H), 7.52 (d, J = 7.7 Hz, 2H), 7.40 (d, J = 7.9 Hz, 1H), 7.20 (d, J = 7.2 Hz, 1H), 6.42 (s, 1H), 4.85 (t, J = 11.9 Hz, 1H), 4.08 (s, 1H), 3.99 - 2.98 (m, 15H), 2.93 (s, 3H), 2.87 (s, 3H), 2.61 - 2.52 (m, 2H), 2.25 (s, 3H), 1.96 (d, J = 12.6 Hz, 2H), 1.71 - 1.58 (m, 4H). | 736 |
| 217 | 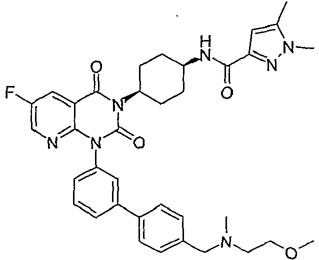 | N-{cis-4-[6-fluoro-1-(4'-{[(2-methoxyethyl)(methyl)amino]meth yl}biphenyl-3-yl)-2,4-dioxo-1,4-dihydropyrido[2,3-d]pyrimidin-3(2H)-yl]cyclohexyl}-1,5-dimethyl-1H-pyrazole-3-carboxamide | ¹H NMR (400 MHz, DMSO) δ 8.52 (d, J = 2.8 Hz, 1H), 8.25 (dd, J = 7.7, 3.1 Hz, 1H), 7.81 - 7.72 (m, 4H), 7.65 - 7.59 (m, 3H), 7.40 (d, J = 7.7 Hz, 1H), 7.11 (d, J = 7.2 Hz, 1H), 6.39 (s, 1H), 4.92 - 4.82 (m, 1H), 4.30 (s, 2H), 4.15 - 4.09 (m, 1H), 3.73 (s, 3H), 3.69 (t, J = 5.3 Hz, 2H), 3.31 (s, 3H), 3.23 - 3.19 (m, 2H), 2.71 (s, 3H), 2.63 - 2.52 (m, 2H), 2.25 (s, 3H), 1.97 (d, J = 12.8 Hz, 2H), 1.73 - 1.62 (m, 4H). | 654 |
| 218 | 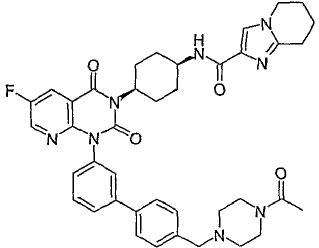 | N-{cis-4-[1-{4'-[(4-acetylpiperazin-1-yl)methyl]biphenyl-3-yl}-6-fluoro-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 8.59 (d, J = 2.6 Hz, 1H), 8.31 (dd, J = 7.7, 2.8 Hz, 1H), 8.13 - 8.06 (m, 2H), 7.85 - 7.77 (m, 4H), 7.65 (t, J = 7.9 Hz, 1H), 7.59 (d, J = 8.2 Hz, 2H), 7.42 (d, J = 8.5 Hz, 1H), 4.84 (t, J = 12.4 Hz, 1H), 4.38 (s, 2H), 4.11 - 4.07 (m, 2H), 4.03 - 4.00 (m, 1H), 2.91 - 2.87 (m, 2H), 2.68 - 2.59 (m, 2H), 2.08 - 2.03 (m, 5H), 1.96 - 1.85 (m, 4H), 1.71 - 1.58 (m, 4H). Remaining protons obscured by solvent peaks. | 719 |
| 219 | 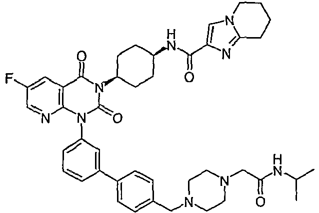 | N-{cis-4-[6-fluoro-1-[4'-({4-[2-(isopropylamino)-2-oxoethyl]piperazin-1-yl}methyl)biphenyl-3-yl]-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 8.59 (d, J = 3.1 Hz, 1H), 8.31 (dd, J = 7.7, 3.1 Hz, 1H), 8.16-8.05 (m, 2H), 7.81 (d, J = 8.0 Hz, 1H), 7.75 - 7.72 (m, 3H), 7.63 (t, J = 7.8 Hz, 1H), 7.55 - 7.51 (m, 2H), 7.39 (d, J = 8.5 Hz, 1H), 4.84 (t, J = 11.9 Hz, 1H), 4.11 - 4.08 (m, 2H), 4.03 - 3.99 (m, 1H), 3.92 - 3.83 (m, 2H), 3.11 - 2.88 (m, 6H), 2.69 - 2.59 (m, 2H), 2.09 - 2.02 (m, 2H), 1.97 - 1.85 (m, 4H), 1.71 - 1.57 (m, 4H), 1.07 (d, J = 6.7 Hz, 6H). Remaining protons obscured by solvent peaks. | 776 |
| 220 | 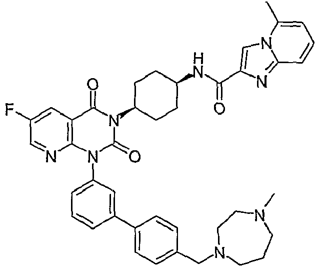 | N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-5-methylimidazo[1,2-a]pyridine-2-carboxamide | ¹H NMR (400 MHz, DMSO) δ 8.59 (d, J = 3.1 Hz, 1H), 8.45 - 8.40 (m, 1H), 8.36 - 8.33 (m, 1H), 7.93 - 7.88 (m, 1H), 7.84 - 7.77 (m, 4H), 7.65 (t, J = 7.8 Hz, 1H), 7.61 - 7.57 (m, 3H), 7.46 - 7.40 (m, 2H), 6.99 - 6.93 (m, 1H), 4.88 (t, J = 12.2 Hz, 1H), 4.38 - 4.32 (m, 1H), 4.21 - 4.15 (m, 1H), 2.84 - 2.82 (m, 2H), 2.67 - 2.59 (m, 5H), 2.14 - 2.01 (m, 4H), 1.77 - 1.65 (m, 4H). Remaining protons obscured by solvent peaks. | 715 |
| 221 | 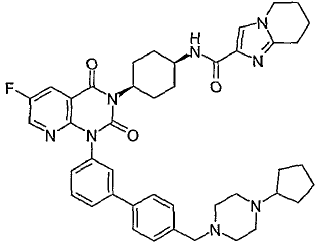 | N-{cis-4-[1-{4'-[(4-cyclopentylpiperazin-1-yl)methyl]biphenyl-3-yl}-6-fluoro-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 8.59 (d, J = 3.1 Hz, 1H), 8.31 (dd, J = 7.7, 2.8 Hz, 1H), 8.17 - 8.10 (m, 2H), 7.79 (d, J = 7.7 Hz, 1H), 7.73 - 7.68 (m, 3H), 7.62 (t, J = 7.8 Hz, 1H), 7.47 (d, J = 8.2 Hz, 2H), 7.38 (d, J = 8.5 Hz, 1H), 4.84 (t, J = 12.7 Hz, 1H), 4.12 - 4.08 (m, 2H), 4.03 - 3.99 (m, 1H), 3.83 - 2.99 (m, 11H), 2.92 - 2.89 (m, 2H), 2.70 - 2.59 (m, 2H), 2.07 (d, J = 12.6 Hz, 2H), 2.01 - 1.85 (m, 6H), 1.71 - 1.52 (m, 10H). | 745 |
| 222 | 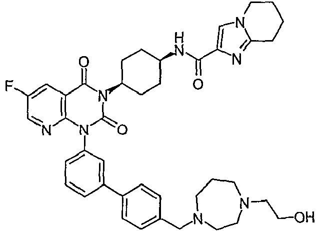 | N-{cis-4-[6-fluoro-1-(4'-{[4-(2-hydroxyethyl)-1,4-diazepan-1-yl]methyl}biphenyl-3-yl)-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 8.59 (d, J = 2.8 Hz, 1H), 8.32 (dd, J = 7.7, 2.8 Hz, 1H), 8.11-8.01 (m, 2H), 7.83 (d, J = 7.4 Hz, 1H), 7.79 - 7.76 (m, 3H), 7.64 (t, J = 8.3 Hz, 1H), 7.60-7.58 (m, 2H), 7.41 (d, J = 8.2 Hz, 1H), 4.38 - 4.31 (m, 2H), 4.11 - 4.00 (m, 3H), 3.74 - 3.70 (m, 2H), 3.27 - 3.22 (m, 2H), 2.91 - 2.86 (m, 2H), 2.68 - 2.59 (m, 2H), 2.16 - 2.12 (m, 2H), 2.06 (d, J = 12.8 Hz, 2H), 1.96 - 1.84 (m, 4H), 1.70 - 1.58 (m, 4H). Remaining protons obscured by solvent peaks. | 735 |
| 223 | 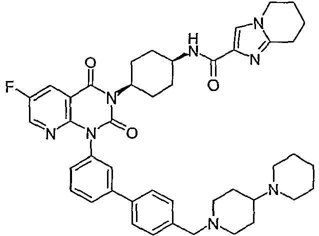 | N-{cis-4-[1-[4'-(1,4'-bipiperidin-1'-ylmethyl)biphenyl-3-yl]-6-fluoro-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 9.72 (br s, 1H), 8.59 (d, J = 2.8 Hz, 1H), 8.31 (dd, J = 7.7, 2.8 Hz, 1H), 8.11 - 8.05 (m, 2H), 7.84 - 7.77 (m, 4H), 7.65 (t, J = 7.9 Hz, 1H), 7.60 (d, J = 7.9 Hz, 2H), 7.42 (d, J = 7.9 Hz, 1H), 4.84 (t, J = 11.7 Hz, 1H), 4.35 (s, 2H), 4.09 (t, J = 5.5 Hz, 2H), 4.04 - 4.01 (m, 1H), 3.04 - 2.88 (m, 8H), 2.70 - 2.59 (m, 2H), 2.25 (d, J = 12.4 Hz, 2H), 2.06 (d, J = 13.9 Hz, 2H), 1.97 - 1.81 (m, 6H), 1.72 - 1.57 (m, 6H), 1.45 - 1.37 (m, 2H). Remaining protons obscured by solvent peaks. | 759 |
| 224 | 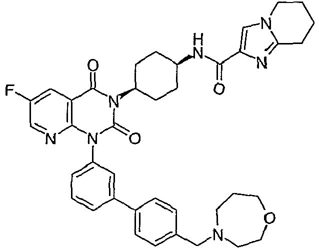 | N-{cis-4-[6-fluoro-1-[4'-(1,4-oxazepan-4-ylmethyl)biphenyl-3-yl]-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 9.82 (s, 1H), 8.59 (d, J = 2.8 Hz, 1H), 8.32 (dd, J = 7.7, 3.1 Hz, 1H), 8.08 - 7.98 (m, 2H), 7.85 - 7.77 (m, 4H), 7.66 - 7.62 (m, 3H), 7.42 (d, J = 8.5 Hz, 1H), 4.84 (t, J = 12.8 Hz, 1H), 4.45 (s, 2H), 4.09 - 4.00 (m, 3H), 3.90 - 3.84 (m, 1H), 3.78 - 3.69 (m, 4H), 2.90 - 2.86 (m, 2H), 2.67 - 2.59 (m, 2H), 2.08 - 2.02 (m, 4H), 1.95 - 1.84 (m, 4H), 1.71 - 1.58 (m, 4H). Remaining protons obscured by solvent peaks. | 692 |
| 225 | 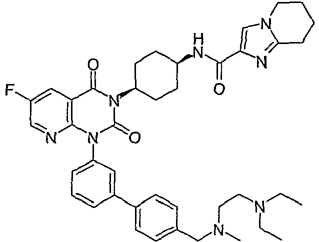 | N-{cis-4-[1-(4'-{[[2-(diethylamino)ethyl](methyl)amin o]methyl}biphenyl-3-yl)-6-fluoro-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 8.59 (d, J = 2.8 Hz, 1H), 8.31 (dd, J = 7.6,2.9 Hz, 1H), 8.12-8.01 (m, 2H), 7.82 (d, J = 7.2 Hz, 1H), 7.78 - 7.74 (m, 3H), 7.64 (t, J = 7.8 Hz, 1H), 7.60-7.55 (m, 2H), 7.41 (d, J = 7.2 Hz, 1H), 4.84 (t, J = 12.7 Hz, 1H), 4.11 - 4.06 (m, 2H), 4.04-4.00 (m, 1H), 3.19 - 3.13 (m, 4H), 2.91 - 2.86 (m, 2H), 2.69 - 2.59 (m, 4H), 2.09 - 2.02 (m, 2H), 1.95 - 1.85 (m, 4H), 1.71 - 1.58 (m, 4H), 1.20 (t, J = 6.5 Hz, 6H). Remaining resonances obscured by solvent. | 721 |
| 226 | 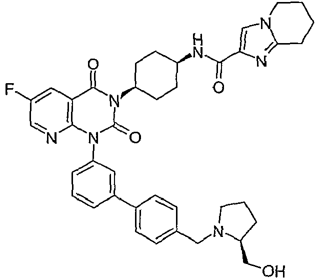 | N-{cis-4-[6-fluoro-1-(4'-{[(2S)-2-(hydroxymethyl)pyrrolidin-1-yl]methyl}biphenyl-3-yl)-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 8.59 (d, J = 2.6 Hz, 1H), 8.31 (dd, J = 7.7, 3.1 Hz, 1H), 8.09 - 8.00 (m, 2H), 7.84 (d, J = 8.4 Hz, 1H), 7.79 - 7.77 (m, 3H), 7.66 - 7.62 (m, 3H), 7.41 (d, J = 8.5 Hz, 1H), 4.84 (t, J = 11.7 Hz, 1H), 4.59 (d, J = 13.7 Hz, 1H), 4.33 - 4.29 (m, 1H), 4.10 - 4.01 (m, 3H), 3.62 - 3.59 (m, 2H), 2.91 - 2.86 (m, 2H), 2.68 - 2.59 (m, 2H), 2.16 - 1.58 (m, 14H). Remaining protons obscured by solvent peaks. | 692 |
| 227 | 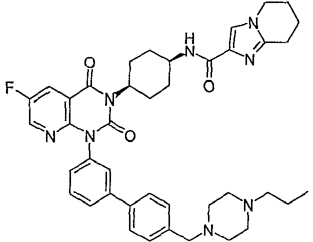 | N-{cis-4-[6-floro-2,4-dioxo-1-{4'-[(4-propylpiperazin-1-yl)methyl]biphenyl-3-yl}-1,4-dihydropyrido[2,3-d]pyrimidin-3(2H)-yl]cyclohexyl}-5,6,7,8-tetrahydroimidazo[1,2-a]pyridine-2-carboxamide | ¹H NMR (400 MHz, DMSO) δ 8.59 (d, J = 3.1 Hz, 1H), 8.31 (dd, J = 7.8, 2.9 Hz, 1H), 8.13 - 8.07 (m, 2H), 7.79 (d, J = 8.5 Hz, 1H), 7.72-7.67 (m, 3H), 7.62 (t, J = 7.8 Hz, 1H), 7.46 (d, J = 7.2 Hz, 2H), 7.38 (d, J = 8.5 Hz, 1H), 4.84 (t, J = 12.3 Hz, 1H), 4.11 - 4.07 (m, 2H), 4.04-4.00 (m, 1H), 3.80 - 3.74 (m, 2H), 3.02 - 2.88 (m, 6H), 2.69 - 2.59 (m, 2H), 2.06 (d, J = 14.1 Hz, 2H), 1.96 - 1.85 (m, 4H), 1.71 - 1.57 (m, 6H), 0.89 (t, J = 7.4 Hz, 3H). Remaining protons obscured by solvent peaks. | 719 |
| 228 | 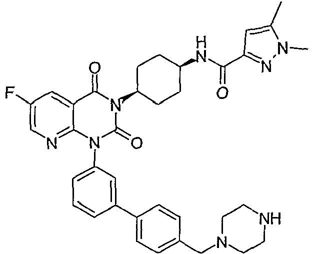 | N-{cis-4-[6-fluoro-2,4-dioxo-1-[4'-(piperazin-1-ylmethyl)biphenyl-3-yl]-1,4-dihydropyrido[2,3-d]pyrimidin-3(2H)-yl]cyclohexyl}-1,5-dimethyl-1H-pyrazole-3-carboxamide | ¹H NMR (400 MHz, CDCl₃) d 8.36 (d, J = 2.8 Hz, 1H), 8.22 (dd, J = 7.2, 3.1 Hz, 1H), 7.72 (d, J = 7.9 Hz, 1H), 7.68 - 7.62 (m, 3H), 7.52 (s, 1H), 7.45 (d, J = 7.9 Hz, 2H), 7.33 (d, J = 7.7 Hz, 1H), 7.18 (d, J = 7.7 Hz, 1H), 6.47 (s, 1H), 5.04 (t, J = 13.1 Hz, 1H), 4.33 (s, 1H), 4.07 (s, 2H), 3.73 (s, 3H), 3.48 (s, 4H), 3.33 (s, 4H), 2.73 - 2.61 (m, 2H), 2.22 (s, 3H), 2.04 (d, J = 12.6 Hz, 2H), 1.79 - 1.68 (m, 4H). | 651 |

### Example 229

### N-{cis-4-[1-[2'-Bromo-4'-(piperazin-1-ylmethyl)biphenyl-3-yl]-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

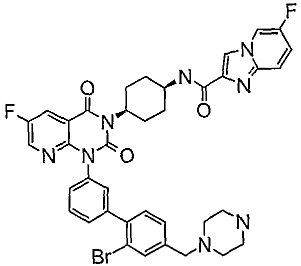

### Step (a) tert-Butyl {cis-4-[1-(2'-bromo-4'-formylbiphenyl-3-yl)-6-fluoro-2,4-dioxo-1,4-dihydropyrido[2,3-d]pyrimidin-3(2H)-yl]cyclohexyl}carbamate

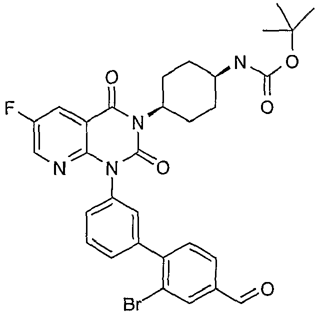

To a solution of palladium acetate (0.035 g, 0.16 mmol) dissolved in acetonitrile (10 ml) was added 2-dicyclohexylphosphino-2',6'-dimethoxy-1,1'-biphenyl (0.127 g, 0.31 mmol), and mixture stirred at room temperature, over a period of 10 min under nitrogen. To the resulting solution was added potassium carbonate (1.286 g, 9.30 mmol) dissolved in water (2 ml), followed by 3,4-dibromobenzaldehyde (0.818 g, 3.10 mmol) and *tert*-butyl {*cis*-4-[6-fluoro-2,4-dioxo-1-[3-(4,4,5,5-tetramethyl-1,3,2-dioxaborolan-2-yl)phenyl]-1,4-dihydropyrido[2,3-*d*]pyrimidin-3(2*H*)-yl]cyclohexyl}carbamate (1.8 g, 3.10 mmol) and the resultant mixture heated at 70°C for a further 2 h. The reaction mixture was diluted with ethyl acetate (15 mL), and washed with saturated brine (10 mL). The organic layer was dried over sodium sulfate. The crude product was purified by flash silica chromatography, elution gradient 30 to 50% ethyl acetate in isohexane to give the sub-title compound as a yellow gum (0.60 g).
[M-Boc]+=537/539 (MultiMode+)

### Step (b) Benzyl-4-({2-bromo-3'-[3-{cis-4-[(tert-butoxycarbonyl)amino]cyclohexyl}-6-fluoro-2,4-dioxo-3,4-dihydropyrido[2,3-d]pyrimidin-1(2H)-yl]biphenyl-4-yl} methyl)piperazine-1-carboxylate

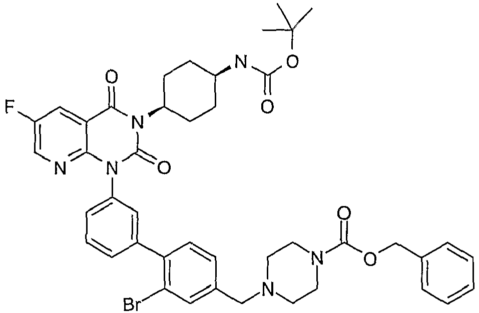

*tert*-Butyl {*cis*-4-[1-(2'-bromo-4'-formylbiphenyl-3-yl)-6-fluoro-2,4-dioxo-1,4-dihydropyrido[2,3-*d*]pyrimidin-3(2*H*)-yl]cyclohexyl}carbamate (0.6 g, 0.94 mmol) and benzyl 1-piperazine carboxylate (228 mg, 1.04 mmol) were stirred in DCM (20 mL) for 30 min then sodium triacetoxyborohydride (0.259 g, 1.22 mmol) was added and the mixture stirred for 4 h. Water was added and the mixture stirred for a further 15 h. The phases were separated. The aqueous was further extracted with DCM and the combined organic phases were dried (Na₂SO₄) and concentrated in vacuo. The crude product was purified by flash silica chromatography, elution gradient 40 to 60% ethyl acetate in isohexane. Pure fractions were evaporated to dryness to afford the sub-title compound as a colourless gum (0.60 g).
[M-Boc]+=741/743 (Multimode+)

### Step (c) Benzyl-4-({2-bromo-3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl} methyl)piperazine-1-carboxylate

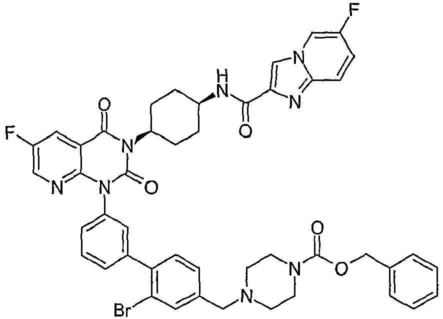

Benzyl-4-({2-bromo-3'-[3-{*cis*-4-[(*tert*-butoxycarbonyl)amino]cyclohexyl}-6-fluoro-2,4-dioxo-3,4-dihydropyrido[2,3-*d*]pyrimidin-1(2*H*)-yl]biphenyl-4-yl}methyl)piperazine-1-carboxylate (0.6 g, 0.71 mmol) was stirred in formic acid (16.67 ml, 434.54 mmol) for 5 h. The solution was diluted with methanol (20mL) and evaporated in vacuo. The residue was taken up in saturated aqueous sodium bicarbonate solution and extracted with ethyl acetate. The combined extracts were dried over sodium sulphate, filtered and evaporated to give a colourless gum. (0.33 g). This was dissolved in acetonitrile (5 mL) at room temperature with 6-fluoro-imidazole[1,2a]pyridine-2-carboxylic acid (0.098 g, 0.54 mmol) and triethylamine (0.412 mL, 2.96 mmol). 2,4,6-Tripropyl-1,3,5,2,4,6-trioxatriphosorinan-2,4,6-trioxide (1.57M in THF, 0.376 mL, 0.59 mmol) was added dropwise over 5 mins. The reaction mixture was stirred for 15 min then concentrated and slurried with water. The solid was filtered and dried in vacuo. The crude product was purified by flash silica chromatography, elution gradient 20 to 50% ethyl acetate in isohexane then by reverse phase HPLC Symmetry C8 (19mmx50mm column) eluting with TFA/MeOH 55% organic isocratic gradient to give the sub-title compound (130mg).
[M+H]+=903/905 (MultiMode+)

### Step (d) N-{cis-4-[1-[2'-Bromo-4'-(piperazin-1-ylmethyl)biphenyl-3-yl]-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

Benzyl-4-({2-bromo-3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}methyl)piperazine-1-carboxylate (80 mg, 0.09 mmol) in hydrochloric acid (5M, 5 ml, 25.00 mmol) was heated at 80 °C for 3 h. The solution was cooled and concentrated in vacuo to leave the title compound as a solid (70 mg).
[M+H]+=769 (MultiMode+)

### Example 230

### 6-Fluoro-N-{cis-4-[6-fluoro-1-[5-methyl-4'-(piperazin-1-ylmethyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

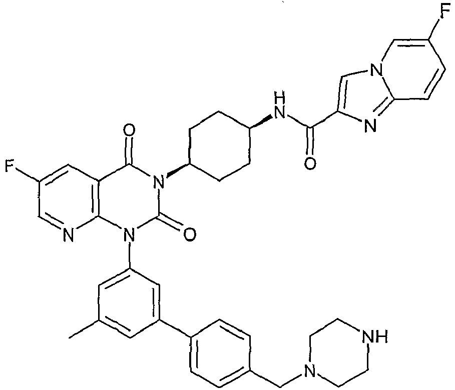

### Step (a) 2-[(3-Bromo-5-methylphenyl)amino]-5-fluoronicotinic acid

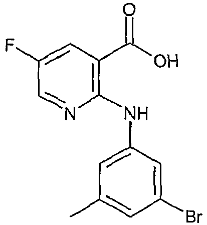

The product was prepared from 2-chloro-5-fluoronicotinic acid (8.65 g, 49.29 mmol) and 3-bromo-5-methylaniline (9.17 g, 49.29 mmol) by the method of example 246 step (a) to give the sub-title compound as a dark brown solid (10.50 g).
¹H NMR (500 MHz, DMSO) δ 10.37 (s, 1H), 8.51 (d, J = 3.0 Hz, 1H), 8.11 (dd, J = 8.9, 3.2 Hz, 1H), 8.00 (d, J = 12.0 Hz, 1H), 7.26 (d, J = 9.7 Hz, 1H), 7.03 (s, 1H), 2.30 (s, 3H).

### Step (b) tert-Butyl-{cis-4-[({2-[(3-bromo-5-methylphenyl)amino]-5-fluoropyridin-3-yl}carbonyl)amino]cyclohexyl}carbamate

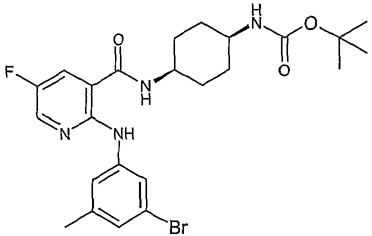

The product was prepared from 2-[(3-bromo-5-methylphenyl)amino]-5-fluoronicotinic acid (5 g, 15.38 mmol) and tert-butyl (cis-4-aminocyclohexyl)carbamate (3.30 g, 15.38 mmol) by the method of example 246 step (b) to give the sub-title compound (6.40 g).
¹H NMR (300 MHz, CDCl₃) δ 10.21 (s, 1H), 8.24 (d, J = 2.9 Hz, 1H), 7.84 (d, J = 1.7 Hz, 1H), 7.44 (dd, J = 8.3, 2.9 Hz, 1H), 7.22 (s, 1H), 6.96 (s, 1H), 6.14 (s, 1H), 4.61 (s, 1H), 4.13 - 4.01 (m, 1H), 3.68 (s, 1H), 2.30 (s, 3H), 1.90 - 1.59 (m, 8H), 1.46 (s, 9H).

### Step (c) tert-Butyl-{cis-4-[1-(3-bromo-5-methylphenyl)-6-fluoro-2,4-dioxo-1,4-dihydropyrido[2,3-d]pyrimidin-3(2H)-yl]cyclohexyl}carbamate

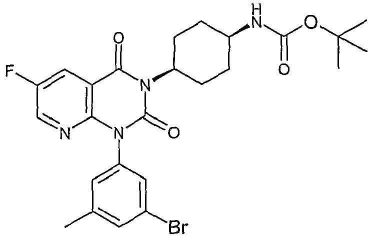

The product was prepared from *tert*-butyl-{*cis*-4-[({2-[(3-bromo-5-methylphenyl)amino]-5-fluoropyridin-3-yl}carbonyl)amino]cyclohexyl}carbamate (6.4 g, 12.27 mmol) and 1,1'-carbonyldiimidazole (3.98 g, 24.55 mmol) by the method of example 246 step (c), with heating to 70°C for 30 min, to give the sub-title compound as a white solid (5.00 g).
¹HNMR (300 MHz, DMSO) δ 8.59 (d, J = 3.1 Hz, 1H), 8.28 (dd, J = 7.7, 2.9 Hz, 1H), 7.52 (s, 1H), 7.46 (s, 1H), 7.23 (s, 1H), 6.51 (s, 1H), 4.73 (t, J = 18.0 Hz, 1H), 3.55 (s, 1H), 2.63 - 2.53 (m, 2H), 2.36 (s, 3H), 1.95 - 1.86 (m, 2H), 1.55 - 1.42 (m, 4H), 1.40 (s, 9H).

### Step (d) 3-(cis-4-Aminocyclohexyl)-1-(3-bromo-5-methylphenyl)-6-fluoropyrido[2,3-d]pyrimidine-2,4(1H,3H)-dione

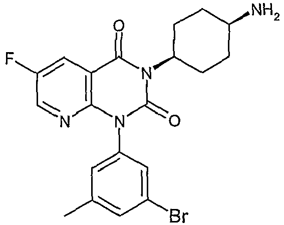

Hydrogen chloride (4M in dioxane, 10 mL, 40.00 mmol) was added to a solution of *tert-*butyl-{*cis*-4-[1-(3-bromo-5-methylphenyl)-6-fluoro-2,4-dioxo-1,4-dihydropyrido[2,3-*d*]pyrimidin-3(2*H*)-yl]cyclohex-yl}carbamate (1.0 g, 1.83 mmol) in dioxane (40 mL) and the reaction mixture stirred for 12 hours. The solvent was evaporated and the residue dried to yield the sub-title compound hydrochloride salt as the dioxane solvate (0.980 g).
¹H NMR (300 MHz, DMSO) δ 8.61 (d, J = 2.9 Hz, 1H), 8.29 (dd, J = 7.7, 3.1 Hz, 1H), 7.97 (s, 3H), 7.52 (s, 1H), 7.46 (s, 1H), 7.24 (s, 1H), 4.77 (t, J = 16.1 Hz, 1H), 3.42 - 3.36 (m, 1H), 2.60 - 2.53 (m, 2H), 2.36 (s, 3H), 1.99 - 1.57 (m, 6H).

### Step (e) N-{cis-4-[1-(3-Bromo-5-methylphenyl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

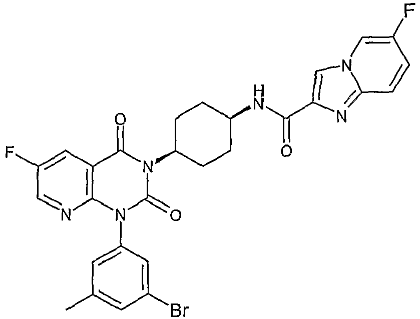

The product was prepared from 3-(*cis*-4-aminocyclohexyl)-1-(3-bromo-5-methylphenyl)-6-fluoropyrido[2,3-*d*]pyrimidine-2,4(1*H*,3*H*)-dione (0.98 g, 2.03 mmol) and 6-fluoro-imidazo[1,2-a]pyridine-2-carboxylic acid (0.438 g, 2.43 mmol) by the method of example 246 step (b) to give the sub-title compound as an off-white solid (0.97 g).
¹H NMR (300 MHz, DMSO) δ 8.80 (dd, J = 3.8, 2.5 Hz, 1H), 8.60 (d, J = 2.9 Hz, 1H), 8.37 (s, 1H), 8.32 (dd, J = 7.9, 3.1 Hz, 1H), 7.77 (dd, J = 10.0, 5.4 Hz, 1H), 7.67 (d, J = 7.3 Hz, 1H), 7.52 (s, 1H), 7.50 - 7.42 (m, 2H), 7.24 (s, 1H), 4.85 (t, J = 17.9 Hz, 1H), 4.16 (s, 1H), 2.66 - 2.54 (m, 2H), 2.36 (s, 3H), 2.05 - 1.96 (m, 2H), 1.78 - 1.59 (m, 4H).

### Step (f) 6-Fluoro-N-{cis-4-[6-fluoro-1-(4'-formyl-5-methylbiphenyl-3-yl)-2,4-dioxo-1,4-dihydropyrido [2,3-d]pyrimidin-3(2H)-yl]cyclohexyl} imidazo[1,2-a]pyridine-2-carboxamide

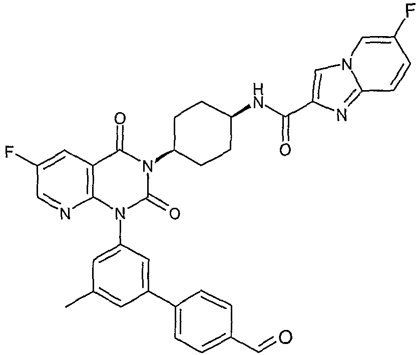

The product was prepared from *N*-{*cis*-4-[1-(3-bromo-5-methylphenyl)-6-fluoro-2,4-dioxo-1,4-dihydropyrido[2,3-*d*]pyrimidin-3(2*H*)-yl]cyclohexyl}-6-fluoroimidazo[1,2-*a*]pyridine-2-carboxamide (0.897 g, 1.47 mmol) and 4-formylbenzeneboronic acid (0.331 g, 2.21 mmol) by the method of example 71 step (b) but heating at 80°C overnight to give the sub-title compound as a beige solid (1.0 g).

¹H NMR (300 MHz, DMSO) δ 10.05 (s, 1H), 8.79 (dd, *J* = 4.2, 2.5 Hz, 1H), 8.60 (d, *J* = 2.9 Hz, 1H), 8.36 (s, 1H), 8.33 (dd, *J* = 7.8, 3.0 Hz, 1H), 8.00 (d, *J* = 8.3 Hz, 2H), 7.91 (d, *J* = 8.3 Hz, 2H), 7.78 - 7.63 (m, 4H), 7.44 (dd, *J* = 18.3, 2.3 Hz, 1H), 7.29 (s, 1H), 4.95 - 4.79 (m, 1H), 4.20 - 4.12 (m, 1H), 2.70 - 2.53 (m, 2H), 2.45 (s, 3H), 2.07 - 1.94 (m, 2H), 1.80 - 1.56 (m, 4H).

### Step (g) 6-Fluoro-N-{cis-4-[6-fluoro-1-[5-methyl-4'-(piperazin-1-ylmethyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

6-Fluoro-*N*-{*cis*-4-[6-fluoro-1-(4'-formyl-5-methylbiphenyl-3-yl)-2,4-dioxo-1,4-dihydropyrido[2,3-*d*]pyrimidin-3(2*H*)-yl]cyclohexyl}imidazo[1,2-a]pyridine-2-carboxamide (0.5 g, 0.79 mmol) and tert-butyl 1-piperazinecarboxylate (0.176 g, 0.95 mmol) were stirred together in DCM (15 mL) and acetic acid (0.047 g, 0.79 mmol) for 1 hour. To this was added sodium triacetoxyborohydride (0.250 g, 1.18 mmol) and the reaction stirred for 16 h then poured into water and extracted with DCM (2 x 20 ml). The extracts were combined and evaporated to dryness. The crude product was purified by flash silica chromatography, elution gradient 3 to 6% methanol in DCM. This product was dissolved in dioxane (10 mL) and hydrogen chloride (4M in dioxane, 5 mL, 20.00 mmol) added. The reaction mixture was stirred for 2 h, the solvent evaporated and the crude product was purified by preparative HPLC on a Sunfire column using a 75-50% gradient of aqueous 0.1% trifluoroacetic acid in acetonitrile as eluent. The fractions containing the desired compound were evaporated to dryness to afford the title compound trifluoroacetic acid salt as a white solid (80 mg).

¹H NMR (300 MHz, DMSO) δ 8.80 (dd, J = 4.4, 2.5 Hz, 1H), 8.59 (d, J = 2.9 Hz, 1H), 8.38 (s, 1H), 8.33 (dd, J = 7.9, 3.1 Hz, 1H), 7.79 - 7.66 (m, 4H), 7.61 (s, 1H), 7.52 - 7.44 (m, 4H), 7.21 (s, 1H), 4.94 - 4.81 (m, 1H), 4.23 - 4.10 (m, 1H), 3.94 - 3.72 (m, 4H), 3.25 - 3.11 (m, 4H), 2.95 - 2.76 (m, 2H), 2.66 - 2.52 (m, 2H), 2.43 (s, 3H), 2.06 - 1.95 (m, 2H), 1.81-1.59(m,4H).
[M+H]+=705 (MultiMode+)

### Example 231

### 6-Fluoro-N-{cis-4-[6-fluoro-1-[5-methyl-4'-({[2-(methylamino)ethyl] amino}methyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

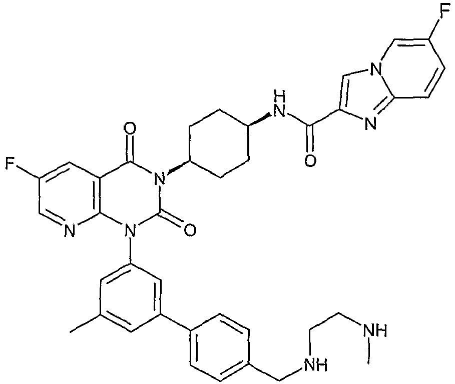

The product was prepared from 6-fluoro-*N*-{*cis*-4-[6-fluoro-1-(4'-formyl-5-methylbiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.5 g, 0.79 mmol) and Boc-N-methylethylenediamine (0.165 g, 0.95 mmol) by the method of example 230 step (g) to give the title compound trifluoroacetic acid salt as a white solid (0.220 g).

¹H NMR (300 MHz, DMSO) δ 9.33 (s, 2H), 9.10 - 8.80 (m, 2H), 8.86 - 8.81 (m, 1H), 8.61 (d, *J* = 2.9 Hz, 1H), 8.42 (s, 1H), 8.36 - 8.31 (m, 1H), 7.81 - 7.71 (m, 4H), 7.68 - 7.55 (m, 4H), 7.48 (dd, *J* = 18.3, 2.1 Hz, 1H), 7.24 (s, 1H), 4.95 - 4.85 (m, 1H), 4.34 (s, 2H), 4.22 - 3.96 (m, 2H), 3.37 - 3.24 (m, 3H), 2.72 - 2.56 (m, 5H), 2.46 (s, 3H), 2.11 - 1.95 (m, 2H), 1.82 - 1.62 (m, 4H).
[M+H]+=693 (MultiMode+)

### Example 232

### N-{cis-4-[6-Fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}acetamide

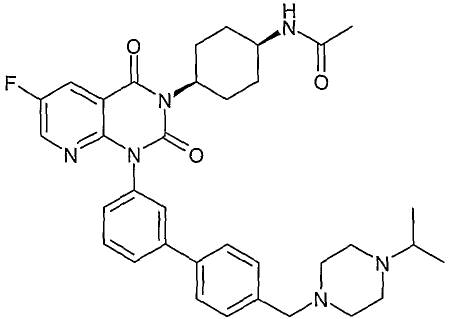

### Step (a) tert-Butyl-{cis-4-[6-fluoro-1-(4'-formylbiphenyl-3-yl)-2,4-dioxo-1,4-dihydropyrido[2,3-d]pyrimidin-3(2H)-yl]cyclohexyl}carbamate

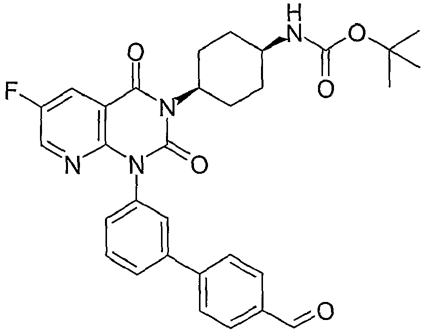

The product was prepared from *tert*-butyl {cis-4-[6-fluoro-1-(3-iodophenyl)-2,4-dioxo-1,4-dihydropyrido[2,3-d]pyrimidin-3(2H)-yl]cyclohexyl}carbamate (10.00 g, 17.23 mmol) and 4-formylbenzeneboronic acid (3.88 g, 25.84 mmol) by the method of example 71 step (b) to afford the sub-title compound as a very pale yellow solid (8.44 g).
¹H NMR (300 MHz, DMSO) δ 10.06 (s, 1H), 8.59 (d, J = 2.9 Hz, 1H), 8.30 (dd, J = 7.9, 3.1 Hz, 1H), 8.04 - 8.00 (m, 2H), 7.94 - 7.85 (m, 4H), 7.67 (t, J = 7.9 Hz, 1H), 7.48 - 7.45 (m, 1H), 6.53 - 6.49 (m, 1H), 4.81 - 4.73 (m, 1H), 3.58 - 3.53 (m, 1H), 2.67 - 2.54 (m, 2H), 1.91 (br d, J = 13.1 Hz, 2H), 1.55 - 1.46 (m, 4H), 1.39 (s, 9H).

### Step (b) tert-Butyl {cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl} carbamate

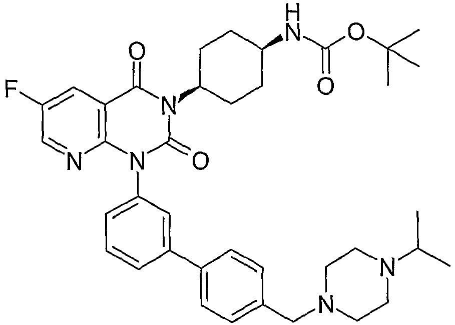

The product was prepared from *tert*-butyl-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}carbamate (2.60 g, 4.66 mmol) and 1-isopropylpiperazine (1 mL, 6.99 mmol) by the method of example 51 to afford the sub-title compound as a foam (2.55 g).
[M+H]+ = 671 (MultiMode+)

### Step (c) 3-(cis-4-Aminocyclohexyl)-6-fluoro-1-{4'-[(4-isopropylpiperazin-1-yl)methyl]biphenyl-3-yl}pyrido[2,3-d]pyrimidine-2,4(1H,3H)-dione

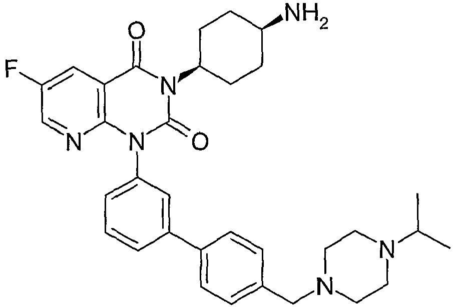

The product was prepared from *tert*-butyl {*cis*-4-[6-fluoro-1-{4'-[(4-isopropylpiperazin-1-yl)methyl]biphenyl-3-yl}-2,4-dioxo-1,4-dihydropyrido[2,3-*d*]pyrimidin-3(2*H*)-yl]cyclohexyl}carbamate (2.55 g, 3.80 mmol) by the method of example 230 step (d) to afford the sub-title compound tri-hydrochloride salt as a white solid (2.5 g).
[M+H]+=571 (MultiMode+)

### Step (d) N-{cis-4-[6-Fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}acetamide

To a solution of 3-(*cis*-4-aminocyclohexyl)-6-fluoro-1-{4'-[(4-isopropylpiperazin-1-yl)methyl]biphenyl-3-yl}pyrido[2,3-*d*]pyrimidine-2,4(1*H*,3*H*)-dione (200 mg, 0.29 mmol) in acetonitrile (5 mL) was added acetic acid (0.017 mL, 0.29 mmol) and triethylamine (298 mg, 2.94 mmol) . The mixture was stirred at room temperature for 10 min before propane phosphonic acid anhydride (1.57 M in THF, 0.2 mL, 0.29 mmol) was added and the mixture stirred at room temperature for 2 h. The mixture was poured into saturated sodium bicarbonate and the organics extracted into ethyl acetate (x 3). The ethyl acetate extracts were combined and evaporated to give a residue which was purified by reverse phase HPLC (eluent TFA(aq)/MeCN). The appropriate fractions were combined, evaporated and triturated with ether to give the title compound trifluoroacetate salt as a white solid (132 mg).
¹H NMR (400 MHz, DMSO) δ 8.51 (d, J = 3.1 Hz, 1H), 8.23 (dd, J = 7.7, 3.1 Hz, 1H), 7.75 (d, J = 7.9 Hz, 1H), 7.68 - 7.64 (m, 3H), 7.59 (t, J = 7.8 Hz, 1H), 7.44 (d, J = 8.2 Hz, 2H), 7.35 (d, J = 8.2 Hz, 1H), 7.30 (s, 1H), 4.79 (t, J = 13.1 Hz, 1H), 3.84 (s, 1H), 3.73 (s, 2H), 3.44 - 3.36 (m, 1H), 3.20 - 3.07 (m, 5H), 2.85 - 2.72 (m, 2H), 2.67 - 2.55 (m, 2H), 1.90 (d, J = 12.0 Hz, 2H), 1.85 (s, 3H), 1.59 - 1.48 (m, 5H), 1.25 (d, J = 6.7 Hz, 6H).
[M+H]+=613 (MultiMode+)

The following compounds (Table 6) were prepared in a similar manner as solids from the appropriate carboxylic acid using the method described above in example 232 step (d).

**Table 6**

| | | | | |
|---|---|---|---|---|
| 233 | 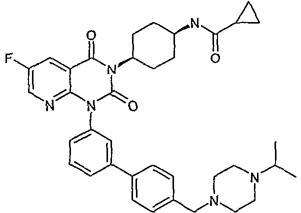 | *N*-{*cis*-4-[6-fluoro-1-{4'-[(4-isopropylpiperazin-1-yl)methyl]biphenyl-3-yl}-2,4-dioxo-1,4-dihydropyrido[2,3-d]pyrimidin-3(2*H*)-yl]cyclohexyl}cyclopropanecarbox amide | ¹H NMR (400 MHz, DMSO) δ 8.52 (t, J = 2.4 Hz, 1H), 8.23 (td, J = 5.2, 2.6 Hz, 1H), 7.75 (d, J = 7.9 Hz, 1H), 7.68 - 7.64 (m, 3H), 7.60 (td, J = 7.8, 2.2 Hz, 1H), 7.47 - 7.42 (m, 3H), 7.36 (d, J = 7.7 Hz, 1H), 4.80 (t, J = 12.0 Hz, 1H), 3.88 - 3.70 (m, 9H), 3.45 - 3.35 (m, 1H), 3.23 - 3.13 (m, 2H), 2.71 - 2.59 (m, 2H), 1.93 (d, J = 11.8 Hz, 2H), 1.72 (dd, J = 5.3, 2.7 Hz, 1H), 1.61 - 1.51 (m, 4H), 1.25 (dd, J = 6.5, 2.4 Hz, 6H), 0.70 - 0.65 (m, 2H), 0.61 - 0.57 (m, 2H). | 639 |
| 234 | 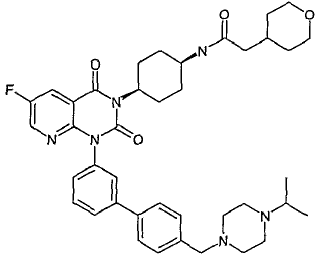 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}-2-(tetrahydro-2H-pyran-4-yl)acetamide | ¹H NMR (400 MHz, DMSO) δ 8.58 (d, J = 3.1 Hz, 1H), 8.31 (dd, J = 7.7, 3.1 Hz, 1H), 7.78 (d, J = 7.9 Hz, 1H), 7.73 - 7.67 (m, 3H), 7.64-7.60 (m, 2H), 7.49 - 7.45 (m, 2H), 7.39 (d, J = 7.7 Hz, 1H), 4.77 (t, J = 12.1 Hz, 1H), 3.82-3.69 (m, 6H), 3.48 - 3.37 (m, 2H), 3.24 (t, J = 11.5 Hz, 1H), 3.14 - 3.03 (m, 2H), 2.70 - 2.60 (m, 2H), 2.08 (d, J = 7.2 Hz, 2H), 1.93 - 1.86 (m, 4H), 1.56 - 1.49 (m, 7H), 1.26 - 1.13 (m, 11H). | 697 |
| 235 | 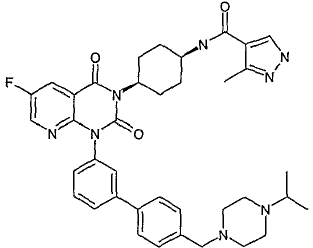 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}-3-methyl-1H-pyrazole-4-carboxamide | ¹H NMR (4006 MHz, DMSO) δ 8.58 (d, J = 2.8 Hz, 1H), 8.32 (dd, J = 7.8, 2.9 Hz, 1H), 8.04 (s, 1H), 7.78 (d, J = 7.9 Hz, 1H), 7.73 - 7.68 (m, 3H), 7.62 (t, J = 7.8 Hz, 1H), 7.47 (d, J = 8.2 Hz, 2H), 7.39 (d, J = 8.7 Hz, 1H), 7.20 (d, J = 4.9 Hz, 1H), 4.82 (t, J = 12.3 Hz, 1H), 4.03 - 3.75 (m, 8H), 3.50 - 3.36 (m, 2H), 3.16-3.02 (m, 2H), 2.73 - 2.59 (m, 2H), 2.40 (s, 3H), 2.09 - 2.02 (m, 2H), 1.63 - 1.51 (m, 4H), 1.23 (d, J = 6.4 Hz, 6H). | 679 |
| 236 | 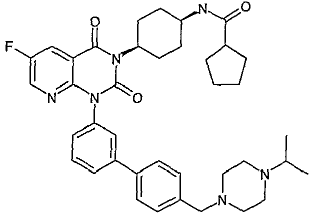 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}cyclopentanecarbox amide | ¹H NMR (400 MHz, 90°C, MeOH) δ 8.35 (d, J = 2.8 Hz, 1H), 8.22 (dd, J = 7.6,2.9 Hz, 1H), 7.73 - 7.70 (m, 1H), 7.65 - 7.55 (m, 4H), 7.43 (d, J = 8.5 Hz, 2H), 7.31 - 7.28 (m, 1H), 5.03 - 4.93 (m, 1H), 4.05 (d, J = 2.8 Hz, 1H), 3.74 (s, 2H), 3.44 (septet, J = 6.5 Hz, 1H), 3.31 - 3.24 (m, 6H), 2.86 (s, 4H), 2.75 - 2.58 (m, 3H), 1.99 - 1.93 (m, 2H), 1.90 - 1.82 (m, 2H), 1.78 - 1.54 (m, 9H), 1.34 (d, J = 6.7 Hz, 6H). | 667 |
| 237 | 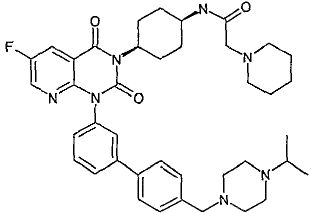 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}-2-piperidin-1-ylacetamide | ¹H NMR (400 MHz, DMSO) δ 8.52 (d, J = 3.1 Hz, 1H), 8.21 (dd, J = 7.7, 3.1 Hz, 1H), 8.15 (d, J = 5.9 Hz, 1H), 7.75 (dtd, J = 7.9, 1.3, 0.2 Hz, 1H), 7.68 - 7.63 (m, 3H), 7.60 (t, J = 7.8 Hz, 1H), 7.44 (d, J = 8.2 Hz, 2H), 7.36 - 7.33 (m, 1H), 4.87 - 4.78 (m, 1H), 4.20 - 4.02 (m, 4H), 3.95 (s, 1H), 3.87 (s, 2H), 3.71 (s, 2H), 3.43 - 3.35 (m, 1H), 3.25 - 3.06 (m, 6H), 2.89 - 2.71 (m, 2H), 2.67 - 2.56 (m, 2H), 1.97 - 1.91 (m, 2H), 1.79 - 1.73 (m, 4H), 1.69 - 1.51 (m, 6H), 1.25 (d, J = 6.7 Hz, 6H). | 696 |
| 238 | 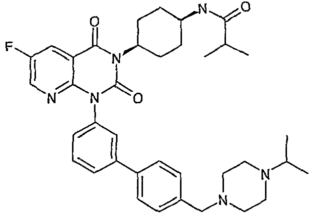 | *N*-{*cis*-4-[6-fluoro-1-{4'-[(4-isopropylpiperazin-1-yl)methyl]biphenyl-3-yl}-2,4-dioxo-1,4-dihydropyrido[2,3-*d*]pyrimidin-3(2*H*)-yl]cyclohexyl}-2-methylpropanamide | ¹H NMR (400 MHz, DMSO) δ 8.51 (d, J = 2.8 Hz, 1H), 8.23 (dd, J = 7.8, 2.9 Hz, 1H), 7.75 (dt, J = 7.9, 1.3 Hz, 1H), 7.68 - 7.64 (m, 3H), 7.59 (t, J = 7.8 Hz, 1H), 7.44 (d, J = 8.2 Hz, 2H), 7.35 (dtd, J = 7.7, 1.2, 0.1 Hz, 1H), 7.03 (d, J = 5.4 Hz, 1H), 4.84 - 4.75 (m, 1H), 3.84 (s, 1H), 3.72 (s, 2H), 3.44 - 3.36 (m, 1H), 2.67 - 2.56 (m, 2H), 1.92 (d, J = 11.5 Hz, 1H), 1.60 - 1.51 (m, 4H), 1.25 (d, J = 6.7 Hz, 6H), 1.02 (d, J = 6.7 Hz, 6H). Remaining protons obscured by solvent. | 641 |
| 239 | 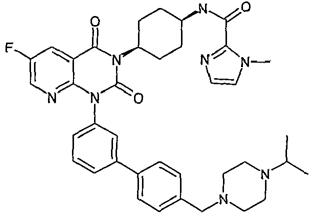 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}-1-methyl-1H-imidazole-2-carboxamide | ¹H NMR (400 MHz, DMSO) δ 8.51 (d, J = 3.1 Hz, 1H), 8.25 (dd, J = 7.9, 3.1 Hz, 1H), 7.77 - 7.65 (m, 5H), 7.60 (t, J = 7.8 Hz, 1H), 7.45 (d, J = 8.2 Hz, 2H), 7.36 (ddd, J = 7.8, 1.9, 1.0 Hz, 1H), 7.28 (s, 1H), 6.98 (d, J = 1.0 Hz, 1H), 4.92 - 4.83 (m, 1H), 4.20 - 4.11 (m, 1H), 3.96 (s, 3H), 3.76 (s, 2H), 3.44 - 3.36 (m, 1H), 3.19 (s, 4H), 2.83 (s, 4H), 2.66 - 2.54 (m, 2H), 1.96 (d, J = 13.3 Hz, 2H), 1.75 - 1.63 (m, 4H), 1.25 (d, J = 6.7 Hz, 6H). | 679 |
| 240 | 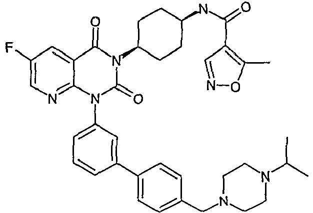 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}-5-methylisoxazole-4-carboxamide | ¹H NMR (400 MHz, DMSO) δ 8.86 (s, 1H), 8.51 (d, J = 2.8 Hz, 1H), 8.24 (dd, J = 7.9, 3.1 Hz, 1H), 7.74 (d, J = 7.7 Hz, 1H), 7.68 - 7.63 (m, 3H), 7.59 (t, J = 7.9 Hz, 1H), 7.49 (d, J = 4.6 Hz, 1H), 7.43 (d, J = 8.2 Hz, 2H), 7.35 (dt, J = 7.9, 1.2 Hz, 1H), 4.90 - 4.81 (m, 1H), 4.01 (s, 1H), 3.94 - 3.78 (m, 4H), 3.71 (s, 2H), 3.44 - 3.36 (m, 1H), 3.16 (s, 4H), 2.74 - 2.63 (m, 2H), 2.61 (s, 3H), 2.08 (d, J = 12.8 Hz, 2H), 1.68 - 1.56 (m, 4H), 1.24 (d, J = 6.7 Hz, 6H). | 680 |
| 241 | 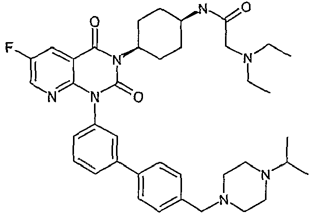 | N²,N²-diethyl-N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}glycinamide | ¹H NMR (400 MHz, CDCl₃) δ 8.37 (d, J = 3.1 Hz, 1H), 8.22 (dd, J = 7.2, 2.8 Hz, 1H), 8.07 (d, J = 6.2 Hz, 1H), 7.72 (d, J = 7.7 Hz, 1H), 7.68-7.62 (m, 3H), 7.51 - 7.47 (m, 3H), 7.30 (dd, J = 8.3, 0.4 Hz, 1H), 4.99 (t, J = 12.2 Hz, 1H), 4.14 (s, 1H), 4.10 (s, 2H), 3.87 (s, 2H), 3.53 - 3.44 (m, 9H), 3.36 - 3.28 (m, 4H), 2.70 - 2.58 (m, 2H), 2.00 (d, J = 12.6 Hz, 2H), 1.74 - 1.64 (m, 4H), 1.39 - 1.31 (m, 12H). | 684 |
| 242 | 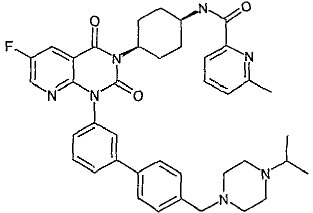 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}-6-methylpyridine-2-carboxamide | ¹H NMR (400 MHz, CDCl₃) δ 8.76 (d, J = 8.5 Hz, 1H), 8.38 (d, J = 2.8 Hz, 1H), 8.23 (dd, J = 7.2, 3.1 Hz, 1H), 7.96 (d, J = 7.7 Hz, 1H), 7.74 - 7.63 (m, 5H), 7.53 (t, J = 1.8 Hz, 1H), 7.48 (d, J = 8.2 Hz, 2H), 7.34 (d, J = 7.9 Hz, 1H), 7.24 (d, J = 7.7 Hz, 1H), 5.13 - 5.04 (m, 1H), 4.44 - 4.39 (m, 1H), 4.05 (s, 2H), 3.52 - 3.38 (m, 9H), 2.85 - 2.74 (m, 2H), 2.49 (s, 3H), 2.08 (d, J = 13.1 Hz, 2H), 1.83 - 1.71 (m, 4H), 1.37 (d, J = 6.7 Hz, 6H). | 690 |
| 243 | 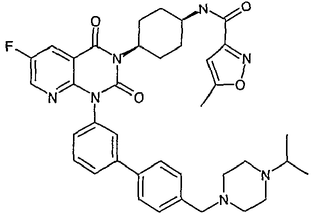 | N-{cis-4-[6-fluoro-1-{4'-[(4-isopropylpiperazin-1-yl)methyl]biphenyl-3-yl} -2,4-dioxo-1,4-dihydropyrido[2,3-d]pyrimidin-3(2H)-yl]cyclohexyl}-5-methylisoxazole-3-carboxamide | ¹H NMR (400 MHz, CDCl₃) δ 8.37 (d, J = 3.1 Hz, 1H), 8.23 (dd, J = 7.3, 2.9 Hz, 1H), 7.75-7.64 (m, 4H), 7.52 - 7.48 (m, 3H), 7.33 (d, J = 7.9 Hz, 1H), 7.16 (d, J = 7.4 Hz, 1H), 6.40 (s, 1H), 5.07 - 4.99 (m, 1H), 4.39 - 4.34 (m, 1H), 4.14 (s, 2H), 3.61 - 3.45 (m, 9H), 2.77 - 2.64 (m, 2H), 2.46 (s, 3H), 2.08 (d, J = 13.8 Hz, 2H), 1.82 - 1.68 (m, 4H), 1.38 (d, J = 6.7 Hz, 6H). | 680 |
| 244 | 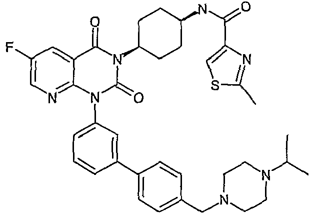 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}-2-methyl-1,3-thiazole-4-carboxamide | ¹H NMR (400 MHz, CDCl₃) δ 8.38 (d, J = 3.1 Hz, 1H), 8.23 (dd, J = 7.2, 3.1 Hz, 1H), 7.91 (s, 1H), 7.75 - 7.72 (m, 2H), 7.69 - 7.65 (m, 3H), 7.53 - 7.48 (m, 3H), 7.37 - 7.34 (m, 1H), 5.09 - 5.00 (m, 1H), 4.40 - 4.35 (m, 1H), 4.14 (s, 2H), 3.61 - 3.45 (m, 9H), 2.79 - 2.68 (m, 2H), 2.68 (s, 3H), 2.09 (d, J = 14.1 Hz, 2H), 1.84 - 1.69 (m, 4H), 1.38 (d, J = 6.7 Hz, 6H). | 696 |
| 245 | 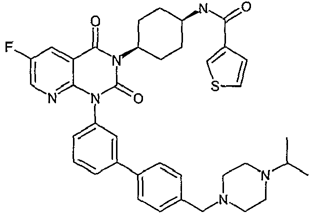 | N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl} thiophene-3-carboxamide | ¹H NMR (400 MHz, CDCl₃) δ 8.39 (d, J = 2.8 Hz, 1H), 8.24 (dd, J = 7.2, 3.1 Hz, 1H), 7.96 (s, 1H), 7.75 (dd, J = 7.8, 1.2 Hz, 1H), 7.71 - 7.66 (m, 3H), 7.54 - 7.47 (m, 4H), 7.37 - 7.33 (m, 1H), 7.31 - 7.28 (m, 1H), 6.67 (d, J = 8.5 Hz, 1H), 5.15 - 5.06 (m, 1H), 4.48 - 4.42 (m, 1H), 4.22 (s, 2H), 3.71 - 3.45 (m, 9H), 2.73 - 2.60 (m, 2H), 2.02 (d, J = 13.1 Hz, 2H), 1.80 - 1.69 (m, 4H), 1.38 (d, J = 6.7 Hz, 6H). | 681 |

### Example 246

### 6-Fluoro-3-(cis-4-{[(6-fluoroimidazo[1,2-a]pyridin-2-yl)methyl]amino}cyclohexyl)-1-[4'-hydroxy-2'-(morpholin-4-ylmethyl)biphenyl-3-yl]pyrido[2,3-d]pyrimidine-2,4(1H,3H)-dione

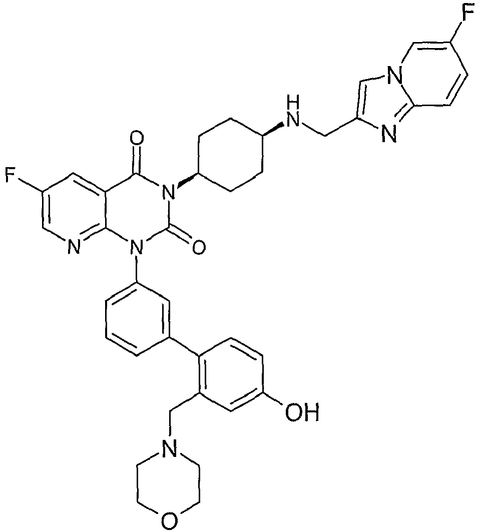

### Step (a) 5-Fluoro-2-[(3-iodophenyl)amino]nicotinic acid

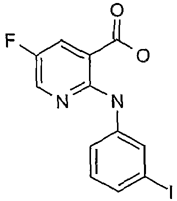

To a 5 L vessel was charged 2-chloro-5-fluoronicotinic acid (130 g, 740.55 mmol) and 3-iodoaniline (0.089 L, 740.55 mmol) in toluene (2.5 L) to give a brown suspension. The reaction was heated until gently refluxing, whereupon a solution was obtained. Tosic acid (113 g, 592.44 mmol) was then added in portions over 6 h. The reaction was then refluxed for 54 h. The reaction was cooled to room temperature and a further amount of tosic acid (21 g) and a further amount of toluene (1 L) were added and the product heated at 105 °C overnight. The resultant suspension was filtered. Water (7.3L) was added and slurried for 16 hours. This was filtered and washed with water. The solid was then dried under vacuum to give the sub-title compound (192 g).
¹H NMR (400 MHz, DMSO) δ 10.32 (s, 1H), 8.50 (d, 1H), 8.23 (t, 1H), 8.11 (q, 1H), 7.59 - 7.57 (m, 1H), 7.36 (d, 1H), 7.11 (t, 2H).

### Step (b) tert-Butyl {cis-4-[({5-fluoro-2-[(3-iodophenyl)amino]pyridin-3-yl}carbonyl)amino]cyclohexyl}carbamate

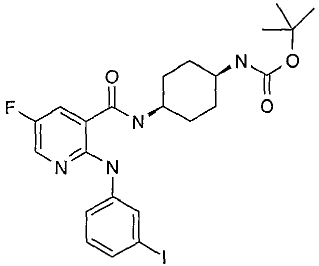

5-Fluoro-2-[(3-iodophenyl)amino]nicotinic acid (187 g, 522.19 mmol), tert-butyl (cis-4-aminocyclohexyl)carbamate (140 g, 652.74 mmol) and triethylamine (0.524 L, 3759.77 mmol) were dissolved in DMF (1.5 L) and the solution cooled to 5 °C. Propane phosphonic acid anhydride 50% in THF (0.416 L, 652.74 mmol) was added dropwise, over a period of 1h, under nitrogen. A further solution of propane phosphonic acid anhydride 50% in THF (40 mL) was added and triethylamine (30 mL). After a further hour at 10 °C, the resulting solution was stirred at room temperature for 18 h. The solution was poured onto 3% diethylether/water mixture (7.5L) and stirred overnight. The resultant precipitated was filtered and washed with water and dried in vacuo. This gave the sub-title compound (250 g).
¹H NMR (400 MHz, DMSO-*d₆*) δ 10.69 (s, 1H), 8.48 (d, 1H), 8.39 (d, 1H), 8.21 (t, 1H), 8.15 (q, 1H), 7.51 (d, 1H), 7.30 (dt, 1H), 7.07 (t, 1H), 6.64 (s, 1H), 3.84 (s, 1H), 3.43 (s, 1H), 1.78 - 1.72 (m, 4H), 1.62 - 1.50 (m, 4H), 1.40 (s, 9H).

### Step (c) tert-Butyl {cis-4-[6-fluoro-1-(3-iodophenyl)-2,4-dioxo-1,4-dihydropyrido[2,3-d]pyrimidin-3(2H)-yl]cyclohexyl}carbamate

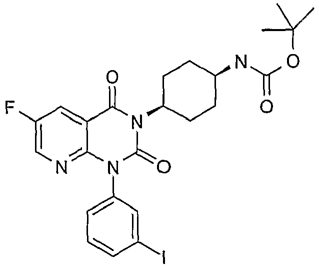

In a 10L vessel was charged tert-butyl {cis-4-[({5-fluoro-2-[(3-iodophenyl)amino]pyridin-3-yl}carbonyl)amino]cyclohexyl}carbamate (250 g, 450.94 mmol) in NMP (2.3 L) to give a brown solution. 1,1'-Carbonyldiimidazole (146 g, 901.88 mmol) was added and the solution cooled to 5 °C. Sodium hydride (36.1 g, 901.88 mmol) was added in portions over 2 h, keeping the internal temperature below 10°C throughout. Stirred for 1 hour at 15°C then poured into water (4L). The mixture was stirred overnight at room temperature. The solid was then filtered off and the filter cake washed with water (0.5L) and 2:1 t-BME/isohexane (1L). The solid product was then dried in vacuo to give the sub-title compound (254 g).
¹H NMR (400 MHz, DMSO) δ 8.58 (d , 1H), 8.28 (dd, 1H), 7.84 - 7.81 (m, 2H), 7.44 - 7.41 (m, 1H), 7.33 (t, 1H), 6.51 (s, 1H), 4.73 (t, 1H), 3.55 (s, 1H), 2.62 - 2.53 (m, 2H), 1.92 - 1.88 (m, 2H), 1.48 (m, 4H), 1.40 (s, 9H).

### Step (d) tert-Butyl-{cis-4-[6-fluoro-1-(2'-formyl-4'-hydroxybiphenyl-3-yl)-2,4-dioxo-1,4-dihydropyrido[2,3-d]pyrimidin-3(2H)-yl]cyclohexyl}carbamate

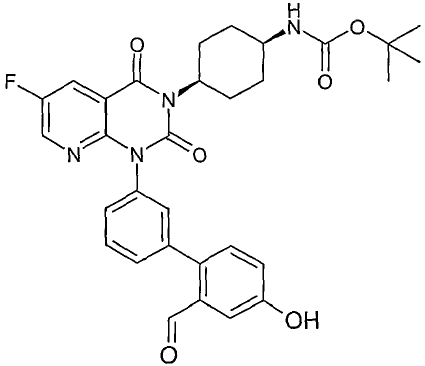

*tert*-Butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate was prepared from tert-butyl {cis-4-[6-fluoro-1-(3-iodophenyl)-2,4-dioxo-1,4-dihydropyrido[2,3-d]pyrimidin-3(2H)-yl]cyclohexyl}carbamate (13.90 g, 23.94 mmol) and 5-hydroxy-2-(4,4,5,5-tetramethyl-1,3,2-dioxaborolan-2-yl)benzaldehyde (9 g, 23.94 mmol) by the method of example 229 step (a), to give the sub-title compound (5 g).
[M-Boc]+=475 (MultiMode+)

### Step (e) tert-Butyl-{cis-4-[6-fluoro-1-[4'-hydroxy-2'-(morpholin-4-ylmethyl)biphenyl-3-yl]-2,4-dioxo-1,4-dihydropyrido[2,3-d]pyrimidin-3(2H)-yl]cyclohexyl}carbamate

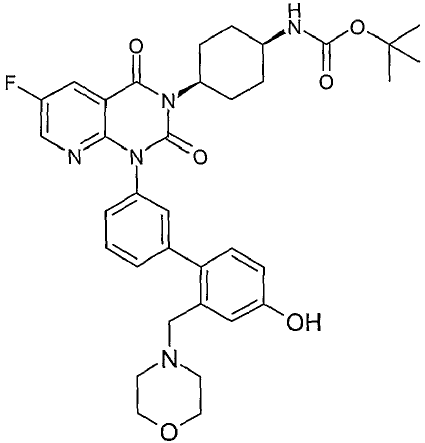

*tert-*Butyl-{*cis*-4-[6-fluoro-1-[4'-hydroxy-2'-(morpholin-4-ylmethyl)biphenyl-3-yl]-2,4-dioxo-1,4-dihydropyrido[2,3-*d*]pyrimidin-3(2*H*)-yl]cyclohexyl}carbamate (8 g, 13.92 mmol) and morpholine (1.457 mL, 16.71 mmol) were dissolved in DCM (100 mL) at 25 °C and the solution allowed to stir for 1 hour. Sodium triacetoxyborohydride (4.43 g, 20.88 mmol) was then added over a period of 10 min under air. The resultant suspension was stirred for 2 h then it was quenched by the addition of water (200 mL) and the organic layer separated, dilutes with a small amount of methanol (20 mL), dried (sodium sulphate) and concentrated to give the sub-title compound as a brown solid (8.6 g).
¹H NMR (400 MHz, CDCl₃) δ 8.37 (d, J = 3.1 Hz, 1H), 8.21 (dd, J = 7.2, 3.1 Hz, 1H), 7.59 (t, J = 7.7 Hz, 1H), 7.50 (d, J = 7.9 Hz, 1H), 7.42 (s, 1H), 7.31 - 7.18 (m, 2H), 7.00 (d, J = 2.8 Hz, 1H), 6.79 (dd, J = 8.3, 2.7 Hz, 1H), 5.52 - 5.34 (m, 1H), 5.06 - 4.91 (m, 2H), 3.93 (s, 1H), 3.58 (t, J = 4.4 Hz, 4H), 3.39 (s, 2H), 2.58 (d, J = 12.0 Hz, 2H), 2.39 (s, 4H), 1.93 (d, J= 13.3 Hz, 2H), 1.75 - 1.51 (m, 4H), 1.44 (s, 9H).

### Step (f) 3-(cis-4-Aminocyclohexyl)-6-fluoro-1-[4'-hydroxy-2'-(morpholin-4-ylmethyl)biphenyl-3-yl]pyrido[2,3-d]pyrimidine-2,4(1H,3H)-dione

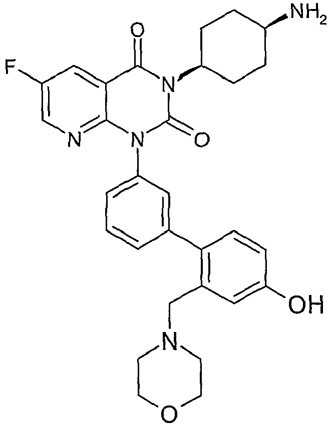

A solution of *tert*-butyl-{*cis*-4-[6-fluoro-1-[4'-hydroxy-2'-(morpholin-4-ylmethyl)biphenyl-3-yl]-2,4-dioxo-1,4-dihydropyrido[2,3-*d*]pyrimidin-3(2*H*)-yl]cyclohexyl}carbamate (1.5 g, 2.32 mmol) in formic acid (8.33 ml, 217.27 mmol) was stirred at room temperature for 5 h. The solution was diluted with methanol (20 mL) and evaporated in vacuo. The residue was taken up in saturated aqueous sodium bicarbonate solution and extracted with ethyl acetate. Combined extracts were dried over sodium sulphate, filtered and evaporated to give the sub-title compound as a colourless gum (0.81 g).
[M+H]+=546 (Multimode+)

### Step (g) 6-Fluoroimidazo[1,2-a]pyridine-2-carbaldehyde

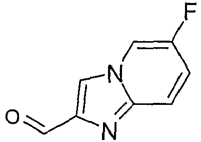

Manganese(IV) oxide (1.990 ml, 115.03 mmol) was added portionwise to (6-fluoroimidazo[1,2-a]pyridin-2-yl)methanol (1.79 g, 10.77 mmol) in DCM (108 ml) at 25 °C over a period of 5 min under nitrogen. The resulting suspension was stirred at 40 °C for 30 min. The solid oxidising agent was then filtered off and the filtrate concentrated and triturated with ether, isolated and washed with cold diethyl ether to give the sub-title compound as a pale solid (0.605 g).
¹H NMR (400 MHz, DMSO) δ 10.03 (s, 1H), 8.86 - 8.81 (m, 1H), 8.62 (d, J = 0.5 Hz, 1H), 7.77 (dd, J = 10.1, 5.3 Hz, 1H), 7.51 (dddd, J = 0.1, 10.3, 8.2, 2.2 Hz, 1H).

### Step (h) 6-Fluoro-3-(cis-4-{[(6-fluoroimidazo[1,2-a]pyridin-2-yl)methyl]amino}cyclohexyl)-1-[4'-hydroxy-2'-(morpholin-4-ylmethyl)biphenyl-3-yl]pyrido[2,3-d]pyrimidine-2,4(1H,3H)-dione

3-(*cis*-4-Aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (1.75 g, 3.21 mmol) and 6-fluoroimidazo[1,2-*a*]pyridine-2-carbaldehyde (0.684 g, 4.17 mmol) were stirred in DCM (20 mL) for 30 mins and sodium triacetoxyborohydride (0.884 g, 4.17 mmol) was added. The mixture was stirred for 1 hour then quenched with water. The phases were separated and the aqueous was extracted with DCM. The organic phases were combined, dried (Na₂SO₄) and concentrated. The crude product was purified by preparative HPLC on a Waters X-Terra column using a 95-5% gradient of aqueous 0.2% ammonia in acetonitrile as eluent. The fractions containing the desired compound were evaporated to dryness to afford the title compound as a tan solid (1.3 g).
¹H NMR (400 MHz, DMSO) δ 8.70 (dd, J = 4.4, 2.3 Hz, 1H), 8.58 (d, J = 2.8 Hz, 1H), 8.28 (dd, J = 7.7, 3.1 Hz, 1H), 7.83 (s, 1H), 7.58 - 7.49 (m, 3H), 7.41 (d, J = 7.7 Hz, 1H), 7.33 - 7.23 (m, 2H), 7.12 (d, J = 8.2 Hz, 1H), 6.88 (d, J = 2.6 Hz, 1H), 6.75 (dd, J = 8.3, 2.4 Hz, 1H), 4.77 (t, J = 13.6 Hz, 1H), 3.80 (d, J = 6.4 Hz, 2H), 3.42 (s, 4H), 3.41 - 3.34 (m, 1H), 3.27 (s, 2H), 2.85 (s, 1H), 2.79 - 2.65 (m, 2H), 2.26 (s, 4H), 1.89 (d, J = 12.8 Hz, 2H), 1.76 (s, 1H), 1.56 - 1.35 (m, 4H).
[M+H]+=694 (Multimode+)

### Example 247

### 6-Fluoro-1-[4'-hydroxy-2'-(morpholin-4-ylmethyl)biphenyl-3-yl]-3-(cis-4-{[(1-methyl-3-phenyl-1H-pyrazol-4-yl)methyl]amino}cyclohoxyl)pyrido[2,3-d]pyrimidine-2,4(1H,3H)-dione

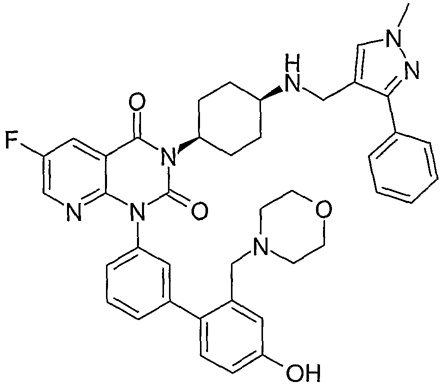

3-(*cis*-4-Aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (200 mg, 0.37 mmol) and 1-methyl-3-phenyl-1H-pyrazole-4-carbaldehyde (61.4 mg, 0.33 mmol) were dissolved in 1,2-dichloroethane (4 mL) at 25 °C and the solution allowed to stir for 10 min. NMP (1 mL) was added to the reaction mixture and the solution stirred for a further 40 min. Sodium triacetoxyborohydride (117 mg, 0.55 mmol) was then added over a period of 10 min under air. The resulting suspension was stirred at 25 °C for 16 h. A further portion of sodium triacetoxyborohydride (117 mg, 0.55 mmol) was then added and the solution was stirred for a further 4 h, before dissolving in methanol (30 mL) and loading onto a 10 g SCX column. Impurities were eluted using methanol (50 mL) and the product isolated by washing with 3.5N ammoniacal methanol (75 mL). The solvents were removed in vacuo and the resulting crude product was purified by preparative HPLC on a Phenomenex Gemini column using a 95-5% gradient of aqueous 0.2% ammonia in acetonitrile as eluent. The fractions containing the desired compound were evaporated to dryness to afford the title compound as a colourless solid (77 mg).

¹H NMR (500 MHz, DMSO) δ 9.51 (s, 1H), 8.82 (s, 1H), 8.59 (d, *J* = 3.0 Hz, 1H), 8.29 (dd, *J =* 7.7, 3.0 Hz, 1H), 7.92 - 7.50 (m, 5H), 7.45 - 7.08 (m, 5H), 6.88 (d, *J =* 2.3 Hz, 1H), 6.76 (dd, *J =* 8.4, 2.3 Hz, 1H), 4.80 (t, *J=* 11.9 Hz, 1H), 3.86 (s, 3H), 3.74 - 3.48 (m, 2H), 3.42 (s, 4H), 3.27 (s, 2H), 2.78 - 2.74 (m, 3H), 2.25 (s, 4H), 2.02 - 1.81 (m, 2H), 1.71 - 1.24 (m, 4H).
[M+H]+=716 (Multimode+)

### Example 248

### 6-Fluoro-1-[4'-hydroxy-2'-(morpholin-4-ylmethyl)biphenyl-3-yl]-3-{cis-4-[(3-phenylpropyl)amino]cyclohexyl}pyrido[2,3-d]pyrimidine-2,4(1H,3H)-dione

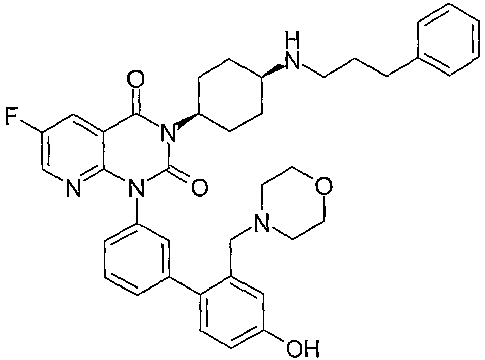

The product was prepared from 3-(*cis*-4-aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (200 mg, 0.37 mmol) and 3-phenylpropionaldehyde (0.044 mL, 0.33 mmol) by the method of example 247 but purified by preparative HPLC on a Waters X-Terra column using a 95-50% gradient of aqueous 0.1% trifluoroacetic acid in acetonitrile as eluent to give the title compound ditrifluoroacetate salt as a colourless solid (113 mg).
¹H NMR (400 MHz, DMSO) δ 10.07 - 9.95 (m, 2H), 8.60 (d, J = 2.8 Hz, 1H), 8.36 - 8.22 (m, 3H), 7.62 (s, 1H), 7.46 - 7.35 (m, 2H), 7.35 - 7.27 (m, 2H), 7.25 - 7.18 (m, 4H), 7.13 - 7.06 (m, 1H), 7.01 - 6.94 (m, 1H), 4.79 (s, 1H), 4.34 - 4.20 (m, 1H), 3.81 - 3.70 (m, 2H), 3.62 - 3.39 (m, 8H), 3.26 - 3.13 (m, 2H), 3.04 - 2.91 (m, 2H), 2.65 (t, J = 8.1 Hz, 2H), 2.10 - 1.90 (m, 2H), 1.79 (d, J = 14.1 Hz, 2H), 1.64 (d, J = 11.0 Hz, 2H).
[M+H]+=664 (MultiMode+)

### Example 249

### 3-{cis-4-[(Cyclopropylmethyl)amino]cyclohexyl}-6-fluoro-1-[4'-hydroxy-2'-(morpholin-4-ylmethyl)biphenyl-3-yl]pyrido[2,3-d]pyrimidine-2,4(1H,3H)-dione

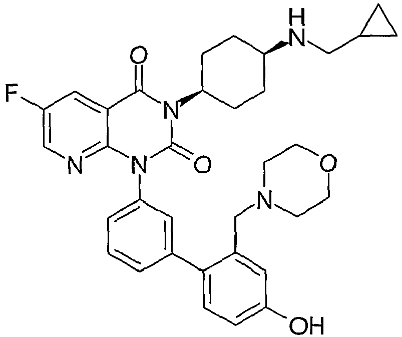

The product was prepared from 3-(*cis*-4-aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (200 mg, 0.37 mmol) and cyclopropanecarbaldehyde (0.027 mL, 0.37 mmol) by the method of example 248 to give the title compound ditrifluoroacetate salt as a colourless solid (29 mg).
¹H NMR (400 MHz, DMSO) δ 8.59 (d, J = 2.8 Hz, 1H), 8.32 (dd, J = 7.7, 3.1 Hz, 3H), 7.61 (s, 1H), 7.40 (d, J = 7.4 Hz, 3H), 7.21 (s, 1H), 7.10 (d, J = 5.9 Hz, 1H), 6.98 (s, 1H), 4.79 (d, J = 12.0 Hz, 1H), 4.27 (s, 1H), 3.74 (s, 2H), 3.60 - 3.39 (m, 8H), 2.89 (d, J = 5.6 Hz, 2H), 2.09 (d, J = 14.1 Hz, 2H), 1.83 - 1.73 (m, 2H), 1.65 (d, J = 10.5 Hz, 2H), 1.13 - 1.03 (m, 1H), 0.65 - 0.55 (m, 2H), 0.40 - 0.34 (m, 2H).
[M+H]+=600 (MultiMode+)

### Example 250

### 3-{cis-4-[(5-Chloro-2-hydroxybenzyl)amino]cyclohexyl}-6-fluoro-1-[4'-hydroxy-2'-(morpholin-4-ylmethyl)biphenyl-3-yl]pyrido[2,3-d]pyrimidine-2,4(1H,3H)-dione

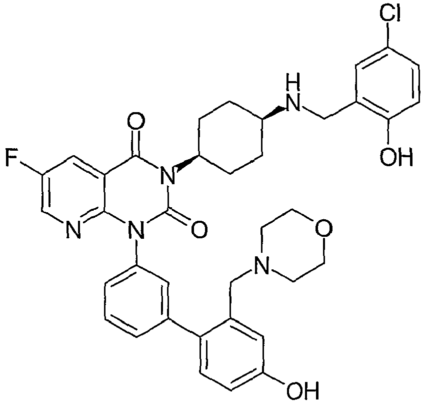

The product was prepared from 3-(*cis*-4-aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (200 mg, 0.37 mmol) and 5-chloro-2-hydroxybenzaldehyde (57.4 mg, 0.37 mmol) by the method of example 248 to give the title compound ditrifluoroacetate salt as a colourless solid (0.226 g).
¹H NMR (400 MHz, DMSO) δ 10.66 (s, 1H), 8.63 - 8.39 (m, 3H), 8.31 (dd, J = 7.7, 3.1 Hz, 1H), 7.62 (t, J = 7.7 Hz, 1H), 7.50 - 7.37 (m, 4H), 7.30 (dd, J = 8.7, 2.6 Hz, 1H), 7.22 (d, J = 8.5 Hz, 1H), 7.09 (s, 1H), 6.95 (d, J = 8.7 Hz, 2H), 4.80 (t, J = 11.7 Hz, 1H), 4.26 (s, 1H), 4.13 (s, 2H), 3.81 - 3.46 (m, 8H), 3.33 (s, 2H), 2.64 - 2.55 (m, 2H), 2.14 (d, J = 14.1 Hz, 2H), 1.80 (t, J = 13.7 Hz, 2H), 1.66 (d, J = 10.8 Hz, 2H).
[M+H]+=686 (MultiMode+)

### Example 251

### 3-(cis-4-{[(1,5-Dimethyl-1H-pyrazol-3-yl)methyl]amino}cyclohexyl)-6-fluoro-1-[4'-hydroxy-2'-(morpholin-4-ylmethyl)biphenyl-3-yl]pyrido[2,3-d]pyrimidine-2,4(1H,3H)-dione

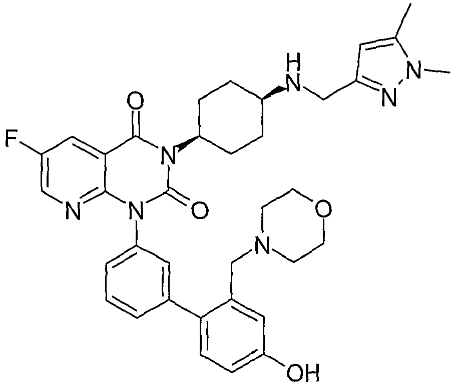

### Step (a) 1,2-Dimethyl-1H-imidazole-4-carbaldehyde

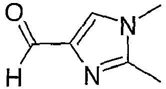

Manganese(IV) oxide (3.446 g, 39.64 mmol) was added portionwise to (1,2-dimethyl-1H-imidazol-4-yl)methanol (500 mg, 3.96 mmol) in DCM (39.600 mL) at 25 °C over a period of 5 min under nitrogen. The resulting suspension was stirred at 40 °C for 30 min. The solid oxidising agent was then filtered off using a fibreglass pad and the filtrate concentrated to give the sub-title compound as a pale yellow oil which solidified to colourless needles on standing (0.480 g).
¹H NMR (400 MHz, CDCl₃) δ 9.88 (s, 1H), 6.56 (s, 1H), 3.89 (d, J = 5.1 Hz, 3H), 2.32 (d, J = 0.5 Hz, 3H).

### Step (b) 3-(cis-4-{[(1,5-Dimethyl-1H-pyrazol-3-yl)methyl]amino}cyclohexyl)-6-fluoro-1-[4'-hydroxy-2'-(morpholin-4-ylmethyl)biphenyl-3-yl]pyrido[2,3-d]pyrimidine-2,4(1H,3H)-dione

The product was prepared from 3-(*cis*-4-aminocyclohexyl)-6-fluoro-1-[4'-hydroxy-2'-(morpholin-4-ylmethyl)biphenyl-3-yl]pyrido[2,3-d]pyrimidine-2,4(1*H*,3*H*)-dione (200 mg, 0.37 mmol) and 1,2-dimethyl-1*H*-imidazole-4-carbaldehyde (45.5 mg, 0.37 mmol) by the method of example 247 to give the title compound as a colourless solid (0.117 g).
¹H NMR (400 MHz, DMSO) δ 9.47 (s, 1H), 8.58 (d, J = 3.1 Hz, 1H), 8.28 (dd, J = 7.8, 2.9 Hz, 1H), 7.59 - 7.48 (m, 2H), 7.41 (d, J = 7.7 Hz, 1H), 7.35 - 7.25 (m, 1H), 7.13 (d, J = 8.2 Hz, 1H), 6.88 (d, J = 2.6 Hz, 1H), 6.75 (dd, J = 8.5, 2.6 Hz, 1H), 5.94 (s, 1H), 4.83 - 4.69 (m, 1H), 3.63 (s, 3H), 3.53 (s, 2H), 3.43 (s, 4H), 3.29 (s, 2H), 2.77 (s, 1H), 2.68 (d, J = 9.7 Hz, 2H), 2.27 (s, 4H), 2.19 (s, 3H), 1.84 (d, J = 12.8 Hz, 2H), 1.54 - 1.32 (m, 4H).
[M+H]+=654 (MultiMode+)

### Example 252

### 6-Fluoro-1-[4'-hydroxy-2'-(morpholin-4-ylmethyl)biphenyl-3-yl]-3-{cis-4-[(5,6,7,8-tetrahydroimidazo[1,2-a]pyridin-2-ylmethyl)amino]cyclohexyl}pyrido[2,3-d]pyrimidine-2,4(1H,3H)-dione

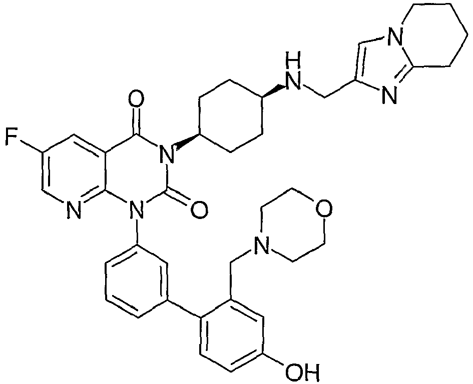

### Step (a) 5,6,7,8-Tetrahydroimidazo[1,2-a]pyridin-2-ylmethanol

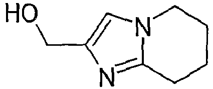

5,6,7,8-Tetrahydroimidazo[1,2-*a*]pyridine-2-carboxylic acid (230 mg, 1.38 mmol) was suspended in THF (2.287 mL) and borane-tetrahydrofyuran complex (6.92 mL, 6.92 mmol) was added. The mixture was heated at reflux for 3 h then cooled to room temperature over a period of two days, then heated to reflux for a further 16 h. Methanol (10 mL) was then added dropwise and the reaction stirred for one hour then concentrated *in vacuo.* The crude product was dissolved in methanol (5 mL) and passed through an SCX column washing with methanol (100 mL) and eluting with 3.5N ammoniacal methanol (75 mL). The basic organic layer was concentrated to give the sub-title compound as a yellow oil (134 mg).
¹H NMR (400 MHz, CDCl₃) δ 6.72 (s, 1H), 4.55 (s, 2H), 3.91 (t, J = 5.9 Hz, 2H), 2.85 (t, J = 6.3 Hz, 2H), 2.01 - 1.87 (m, 4H).

### Step (b) 5,6,7,8-Tetrahydroimidazo[1,2-a]pyridine-2-carbaldehyde

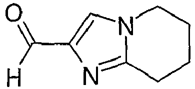

Manganese dioxide (765 mg, 8.80 mmol) was added portionwise to 5,6,7,8-tetrahydroimidazo[1,2-*a*]pyridin-2-ylmethanol (134 mg, 0.88 mmol) in DCM (5 mL) at 25 °C over a period of 5 min under nitrogen. The resulting suspension was stirred at 40 °C for 30 min. The solid oxidising agent was then filtered off using a fibreglass pad and the filtrate concentrated to give the sub-title compound as a colorless oil which crystallised on standing (75 mg).
¹H NMR (400 MHz, CDCl₃) δ 9.83 (s, 1H), 7.51 (d, J = 9.7 Hz, 1H), 4.04 (t, J = 5.9 Hz, 2H), 2.93 (t, J = 6.4 Hz, 2H), 2.09 - 1.93 (m, 4H).

### Step (c) 6-Fluoro-1-[4'-hydroxy-2'-(morpholin-4-ylmethyl)biphenyl-3-yl]-3-{cis-4-[(5,6,7,8-tetrahydroimidazo[1,2-a]pyridin-2-ylmethyl)amino]cyclohexyl}pyrido[2,3-d]pyrimidine-2,4(1H,3H)-dione

The product was prepared from 3-(*cis*-4-aminocyclohexyl)-6-fluoro-1-[4'-hydroxy-2'-(morpholin-4-ylmethyl)biphenyl-3-yl]pyrido[2,3-d]pyrimidine-2,4(1*H*,3*H*)-dione (215 mg, 0.39 mmol) and 5,6,7,8-tetrahydroimidazo[1,2-*a*]pyridine-2-carbaldehyde (53 mg, 0.35 mmol) by the method of example 247 to give the title compound as a colourless solid (34 mg).
¹H NMR (400 MHz, DMSO) δ 9.48 (s, 1H), 8.58 (d, J = 3.1 Hz, 1H), 8.27 (dd, J = 7.7, 3.1 Hz, 1H), 7.56 - 7.48 (m, 2H), 7.41 (d, J = 7.9 Hz, 1H), 7.30 (d, J = 8.2 Hz, 1H), 7.12 (d, J = 8.5 Hz, 1H), 6.80 - 6.72 (m, 2H), 4.76 (t, J = 11.8 Hz, 1H), 3.90 - 3.76 (m, 1H), 3.51 - 3.38 (m, 7H), 3.29 - 3.25 (m, 4H), 2.75 - 2.60 (m, 4H), 2.35 - 2.19 (m, 4H), 1.90 - 1.74 (m, 5H), 1.52-1.32(m,4H).
[M+H]+=680 (MultiMode+)

### Example 253

### 6-Fluoro-3-(cis-4-{[(6-fluoroimidazo[1,2-a]pyridin-2-yl)methyl]amino}cyclohexyl)-1-[4'-hydroxy-2'-(thiomorpholin-4-ylmethyl)biphenyl-3-yl]pyrido[2,3-d]pyrimidine-2,4(1H,3H)-dione

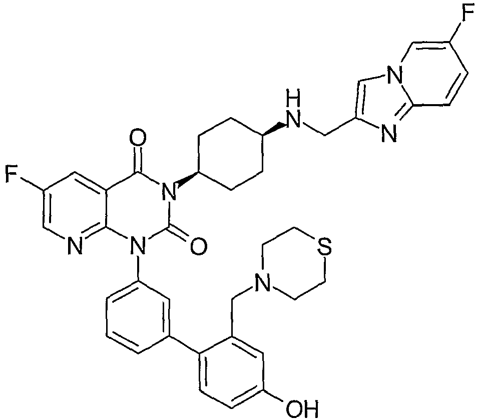

### Step (a) tert-Butyl [(6-fluoroimidazo[1,2-a]pyridin-2-yl)methyl]{cis-4-[6-fluoro-1-(3-iodophenyl)-2,4-dioxo-1,4-dihydropyrido[2,3-d]pyrimidin-3(2H)-yl]cyclohexyl} carbamate

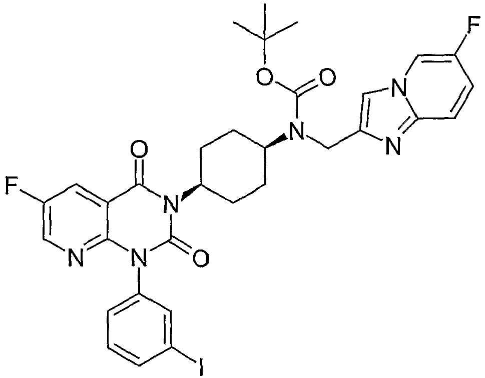

A suspension of 2-(chloromethyl)-6-fluoroimidazo[1,2-a]pyridine (2 g, 10.83 mmol), *tert-*butyl {*cis*-4-[6-fluoro-1-(3-iodophenyl)-2,4-dioxo-1,4-dihydropyrido[2,3-*d*]pyrimidin-3(2*H*)-yl]cyclohexyl}carbamate (5.20 g, 10.83 mmol) and Hünig's Base (7.57 mL, 43.34 mmol) in acetonitrile (20 mL) was heated at 80 °C for 48 h. The reaction was cooled, filtered and concentrated *in vacuo.* The residue was dissolved in DCM (50 ml) and di*-tert-*butyl carbonate (2.52 mL, 10.83 mmol) was added. The mixture was stirred for 24 h then concentrated in vacuo and purified by flash silica chromatography, elution gradient 50% ethyl acetate in isohexane. Pure fractions were evaporated to dryness to afford the sub-title compound as a tan gum (2.62 g).
¹H NMR (300 MHz, CDCl₃) δ 8.34 (d, J = 2.9 Hz, 1H), 8.16 (dd, J = 7.3, 3.1 Hz, 1H), 7.99 - 7.95 (m, 1H), 7.84 (dt, J = 7.0, 1.8 Hz, 1H), 7.63 - 7.61 (m, 1H), 7.47 (dd, J = 9.8, 5.2 Hz, 1H), 7.44 (s, 1H), 7.33 - 7.24 (m, 2H), 7.04 (dd, J = 7.7, 2.1 Hz, 1H), 5.09 - 4.96 (m, 1H), 4.79 (s, 2H), 4.21 - 4.08 (m, 1H), 2.69 - 2.48 (m, 2H), 2.28 - 2.16 (m, 2H), 1.79 - 1.62 (m, 4H), 1.43 (s, 9H).

### Step (b) tert-Butyl {cis-4-[6-fluoro-1-(2'-formyl-4'-hydroxybiphenyl-3-yl)-2,4-dioxo-1,4-dihydropyrido[2,3-d]pyrimidin-3(2H)-yl]cyclohexyl}[(6-fluoroimidazo[1,2-a]pyridin-2-yl)methyl] carbamate

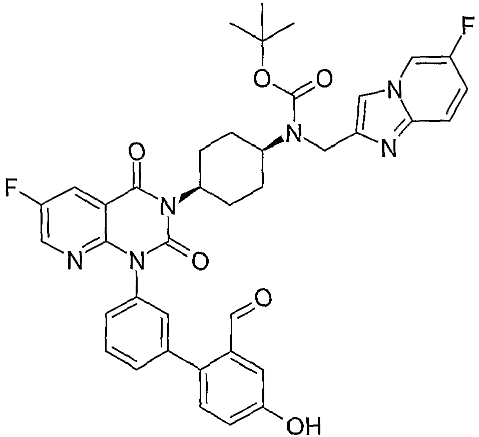

The product was prepared from *tert*-butyl [(6-fluoroimidazo[1,2-*a*]pyridin-2-yl)methyl] {*cis*-4-[6-fluoro-1-(3-iodophenyl)-2,4-dioxo-1,4-dihydropyrido[2,3-*d*]pyrimidin-3(2*H*)-yl]cyclohexyl}carbamate (0.75 g, 1.03 mmol) and 5-hydroxy-2-(4,4,5,5-tetramethyl-1,3,2-dioxaborolan-2-yl)benzaldehyde (0.255 g, 1.03 mmol) by the method of example 229 step (a) to afford the sub-title compound as a tan solid (0.625 g).
[M+H]+=723

### Step (c) 6-Fluoro-3-(cis-4-{[(6-fluoroimidazo[1,2-a]pyridin-2-yl)methyl]amino}cyclohexyl)-1-[4'-hydroxy-2'-(thiomorpholin-4-ylmethyl)biphenyl-3-yl]pyrido[2,3-d]pyrimidine-2,4(1H,3H)-dione

*tert*-Butyl- {*cis*-4-[6-fluoro-1-(2'-formyl-4'-hydroxybiphenyl-3-yl)-2,4-dioxo-1,4-dihydropyrido[2,3-d]pyrimidin-3(2*H*)-yl]cyclohexyl}[(6-fluoroimidazo[1,2-*a*]pyridin-2-yl)methyl]carbamate (150 mg, 0.21 mmol) and thiomorpholine (28 mg, 0.27 mmol) were stirred in DCM (20 mL) for 30 mins then sodium triacetoxyborohydride (57 mg, 0.27 mmol) was added. The mixture was stirred for 1 h then trifluoroacetic acid (1 ml) was added. The mixture was stirred for 2 h and then quenched with water. The phases were separated and the aqueous was extracted with DCM. The combined organic phases were combined, dried (Na₂SO₄) and concentrated. The crude product was purified by preparative HPLC on a Waters X-Terra column using a 95-5% gradient of aqueous 0.2% ammonia in acetonitrile as eluent to give the title compound as a solid (25 mg).

¹H NMR (399.826 MHz, DMSO) δ 8.70 (d, J = 4.4 Hz, 1H), 8.59 (d, J = 2.8 Hz, 1H), 8.28 (dd, J = 7.7, 2.8 Hz, 1H), 7.83 (s, 1H), 7.55 - 7.45 (m, 3H), 7.38 (d, J = 7.9 Hz, 1H), 7.30 (d, J = 8.4 Hz, 1H), 7.25 (dd, J = 18.2, 2.3 Hz, 1H), 7.10 (d, J = 8.5 Hz, 1H), 6.86 (d, J = 2.3 Hz, 1H), 6.74 (dd, J = 8.3, 2.4 Hz, 1H), 4.83 - 4.72 (m, 1H), 3.80 (d, J = 6.2 Hz, 2H), 3.31 (s, 4H), 2.85 (s, 1H), 2.80 - 2.67 (m, 2H), 2.55 - 2.39 (m, 4H), 1.93 - 1.85 (m, 2H), 1.80 - 1.73 (m, 1H), 1.55 - 1.39 (m, 5H).
[M+H]+=710 (MultiMode+)

### Example 254

### 6-Fluoro-3-(cis-4-{[(6-fluoroimidazo[1,2-a]pyridin-2-yl)methyl]amino}cyclohexyl)-1-[4'-hydroxy-2'-(1,4-oxazepan-4-ylmethyl)biphenyl-3-yl]pyrido[2,3-d]pyrimidine-2,4(1H,3H)-dione

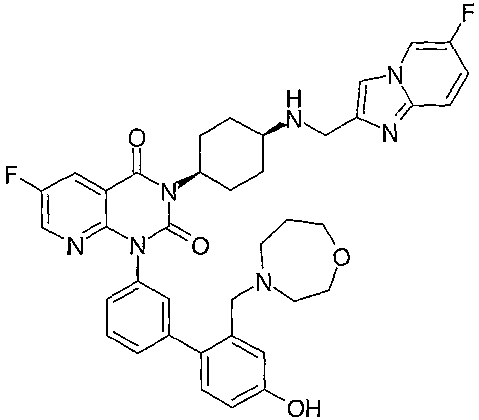

The product was prepared from *tert*-butyl {*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}[(6-fluoroimidazo[1,2-*a*]pyridin-2-yl)methyl]carbamate (150 mg, 0.21 mmol) and homomorpholine hydrochloride (29 mg, 0.21 mmol) by the method of example 253 step (c) to afford the title compound as a yellow solid (18 mg).

¹H NMR (399.826 MHz, DMSO) δ 9.49 (s, 1H), 8.70 (dd, J = 4.5, 2.4 Hz, 1H), 8.58 (s, 1H), 8.28 (dd, J = 7.7, 3.1 Hz, 1H), 7.83 (s, 1H), 7.56 - 7.48 (m, 3H), 7.39 (d, J = 7.9 Hz, 1H), 7.30 (d, J = 8.5 Hz, 1H), 7.28 - 7.22 (m, 1H), 7.10 (d, J = 8.5 Hz, 1H), 6.95 (d, J = 2.6 Hz, 1H), 6.73 (dd, J = 8.5, 2.6 Hz, 1H), 4.83 - 4.73 (m, 1H), 3.80 (d, J = 5.6 Hz, 2H), 3.57 (t, J = 6.0 Hz, 2H), 3.45 (d, J = 7.7 Hz, 4H), 3.31 (s, 2H), 2.85 (s, 1H), 2.79 - 2.65 (m, 2H), 1.94 - 1.84 (m, 2H), 1.81 - 1.72 (m, 1H), 1.67 - 1.58 (m, 2H), 1.54 - 1.37 (m, 5H).
[M+H]+=708 (Multimode+)

### Example 255

### 6-Fluoro-3-(cis-4-{[(6-fluoroimidazo[1,2-a]pyridin-2-yl)methyl]amino}cyclohexyl)-1-[4'-hydroxy-2'-(piperidin-1-ylmethyl)biphenyl-3-yl]pyrido[2,3-d]pyrimidine-2,4(1H,3H)-dione

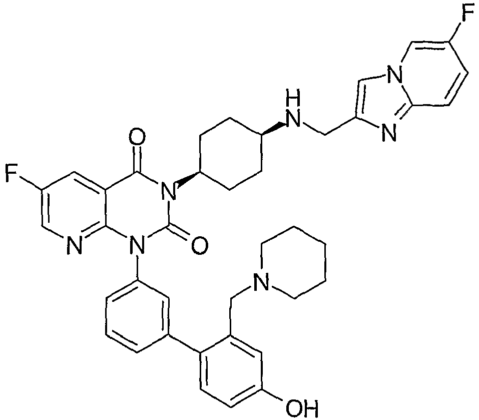

The product was prepared from *tert*-butyl {*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}[(6-fluoroimidazo[1,2-*a*]pyridin-2-yl)methyl]carbamate (150 mg, 0.21 mmol) and piperidine (0.021 mL, 0.21 mmol) by the method of example 253 step (c) to afford the title compound as a yellow solid (16 mg).
¹H NMR (400 MHz, DMSO) δ 8.70 (s, 1H), 8.59 (s, 1H), 8.28 (d, J = 4.4 Hz, 1H), 7.83 (s, 1H), 7.63 (s, 1H), 7.56 - 7.47 (m, 2H), 7.45 - 7.36 (m, 1H), 7.34 - 7.19 (m, 3H), 7.12 (d, J = 7.9 Hz, 1H), 6.85 (s, 1H), 6.73 (d, J = 7.4 Hz, 1H), 4.85 - 4.69 (m, 1H), 3.80 (s, 2H), 3.19 (s, 2H), 2.85 (s, 1H), 2.80 - 2.66 (m, 2H), 2.28 - 2.15 (m, 5H), 1.95 - 1.82 (m, 2H), 1.81 - 1.71 (m, 1H), 1.55 - 1.37 (m, 3H), 1.36 - 1.20 (m, 5H).
[M+H]+=692 (MultiMode+)

### Example 256

### 1-(2'-{[(2R,6S)-2,6-Dimethylmorpholin-4-yl]methyl}-4'-hydroxybiphenyl-3-yl)-6-fluoro-3-(cis-4-{[(6-fluoroimidazo[1,2-a]pyridin-2-yl)methyl]amino}cyclohexyl)pyrido[2,3-d]pyrimidine-2,4(1H,3H)-dione

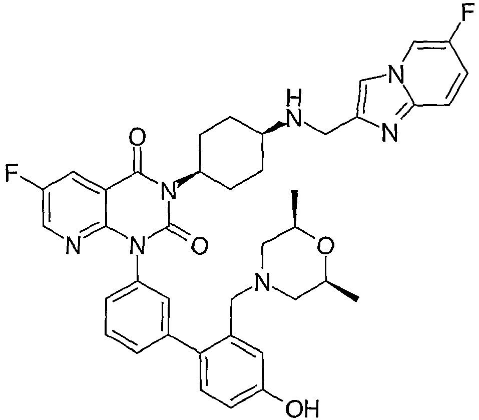

The product was prepared from *tert*-butyl {*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}[(6-fluoroimidazo[1,2-*a*]pyridin-2-yl)methyl]carbamate (150 mg, 0.21 mmol) and cis-2,6-dimethylmorpholine (23.90 mg, 0.21 mmol) by the method of example 253 step (c) to afford the title compound as a tan solid (38 mg).
¹H NMR (400 MHz, DMSO) δ 9.50 (s, 1H), 8.70 (dd, J = 4.5, 2.2 Hz, 1H), 8.60 (d, J = 3.1 Hz, 1H), 8.29 (dd, J = 7.7, 3.1 Hz, 1H), 7.84 (s, 1H), 7.60 (t, J = 1.8 Hz, 1H), 7.55 - 7.49 (m, 2H), 7.41 (d, J = 7.9 Hz, 1H), 7.32 - 7.29 (m, 1H), 7.25 (ddd, J = 9.9, 8.5, 2.4 Hz, 1H), 7.13 (d, J = 8.5 Hz, 1H), 6.86 (d, J = 2.6 Hz, 1H), 6.76 (dd, J = 8.5, 2.6 Hz, 1H), 4.82 - 4.72 (m, 1H), 3.80 (s, 2H), 3.43 - 3.18 (m, 4H), 2.85 (s, 1H), 2.81 - 2.66 (m, 2H), 2.65 - 2.56 (m, 1H), 1.89 (d, J = 12.6 Hz, 2H), 1.83 - 1.74 (m, 1H), 1.58 - 1.37 (m, 6H), 0.91 (d, J = 38.2 Hz, 6H).
[M+H]+=722 (MultiMode+)

### Example 257

### 5-[({cis-4-[6-Fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}amino)methyl]-2-methoxyphenyl acetate

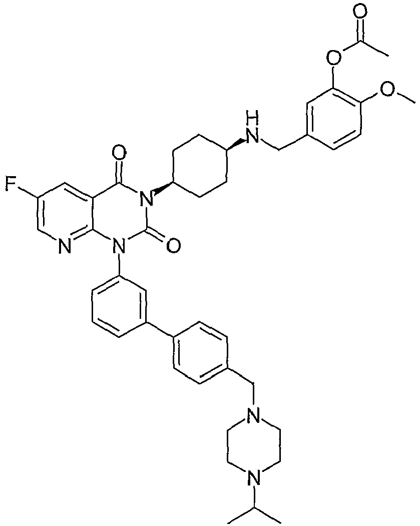

3-(*cis*-4-Aminocyclohexyl)-6-fluoro-1-{4'-[(4-isopropylpiperazin-1-yl)methyl]biphenyl-3-yl}pyrido[2,3-*d*]pyrimidine-2,4(1*H*,3*H*)-dione (200 mg, 0.35 mmol) in 1,2-dichloroethane (2 mL) was added to 5-formyl-2-methoxyphenyl acetate (102 mg, 0.53 mmol) and stirred for 15 min at room temperature. Sodium triacetoxyborohydride (149 mg, 0.70 mmol) was added and the reaction was left at room temperature overnight. Water (2ml), saturated sodium bicarbonate solution (3 mL) and DCM (2 mL) was added and the mixture was stirred 15 min, then separated. The organic solution was evaporated and the residue purified by reverse phase HPLC (ACE 5C8, 0.2% aq TFA-MeCN 95%-5% gradient). Evaporation of the product containing fractions, trituration with ether and thorough drying in vacuo afforded the title compound ditrifluoroacetate salt as a white powder (70mg). ¹H NMR (300 MHz, DMSO) δ 8.79 - 8.64 (m, 1H), 8.60 (s, 1H), 8.35 - 8.25 (m, 1H), 7.82 - 7.56 (m, 4H), 7.54 - 7.25 (m, 5H), 7.23 - 7.12 (m, 1H), 4.89 - 4.74 (m, 1H), 4.48 - 3.83 (m, 11H), 3.79 (s, 3H), 3.54 - 2.94 (m, 8H), 2.26 (s, 3H), 2.15 - 1.98 (m, 2H), 1.88 - 1.55 (m, 4H), 1.24 (d, J = 6.0 Hz, 6H). Remaining resonances partially obscured by solvent peaks.
[M+H]+=749 (MultiMode+)

### Example 258

### 3-{cis-4-[(3-Chloro-4-methoxybenzyl)amino]cyclohexyl}-6-fluoro-1-[4'-(1,4-oxazepan-4-ylmethyl)biphenyl-3-yl]pyrido[2,3-d]pyrimidine-2,4(1H,3H)-dione

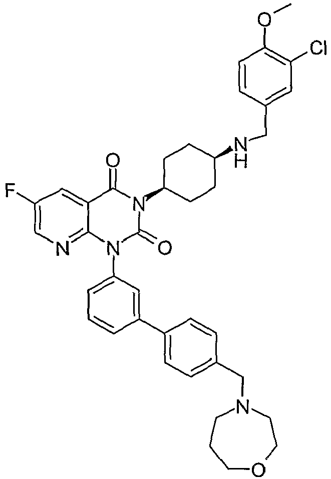

### Step (a) tert-Butyl {cis-4-[6-fluoro-1-[4'-(1,4-oxazepan-4-ylmethyl)biphenyl-3-yl]-2,4-dioxo-1,4-dihydropyrido[2,3-d]pyrimidin-3(2H)-yl]cyclohexyl}carbamate

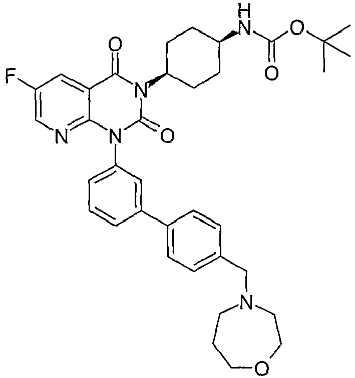

*tert*-Butyl-{*cis*-4-[6-fluoro-1-(4'-formylbiphenyl-3-yl)-2,4-dioxo-1,4-dihydropyrido[2,3-*d*]pyrimidin-3(2H)-yl]cyclohexyl}carbamate (2.234 g, 4 mmol) in 1,2-dichloroethane (30 mL) was treated with 1,4-oxazepane hydrochloride (0.826 g, 6.00 mmol) at room temperature for 15 min, then sodium triacetoxyborohydride (3.39 g, 16.00 mmol) was added and the reaction was stirred at room temperature overnight. The reaction mixture was washed with saturated sodium bicarbonate solution, dried and evaporated, then the residue was redissolved in DCM and washed with dilute aqueous HCl. Evaporation of the DCM solution afforded the sub-title compound as a white foam (2.3 g).
¹H NMR (400 MHz, CDCl₃) δ 12.66 - 12.55 (m, 1H), 8.38 (d, J = 2.8 Hz, 1H), 8.22 (dd, J = 7.2, 3.1 Hz, 1H), 7.81 - 7.72 (m, 2H), 7.72 - 7.64 (m, 3H), 7.55 - 7.51 (m, 1H), 7.37 (s, 2H), 5.09 - 4.91 (m, 2H), 4.40 - 4.12 (m, 2H), 3.96 - 3.72 (m, 3H), 3.61 - 3.48 (m, 1H), 3.23 - 2.77 (m, 2H), 2.66 - 2.52 (m, 6H), 2.09 - 1.87 (m, 2H), 1.74 - 1.59 (m, 4H), 1.42 (s, 9H).

### Step (b) 3-(cis-4-Aminocyclohexyl)-6-fluoro-1-[4'-(1,4-oxazepan-4-ylmethyl)biphenyl-3-yl]pyrido[2,3-d]pyrimidine-2,4(1H,3H)-dione

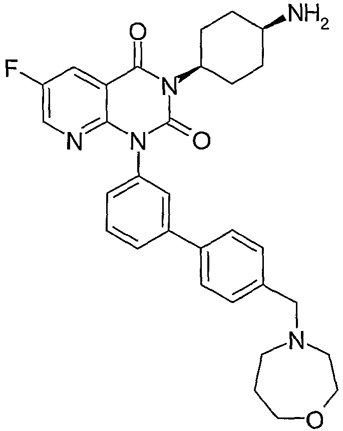

*tert-*Butyl {*cis*-4-[6-fluoro-1-[4'-(1,4-oxazepan-4-ylmethyl)biphenyl-3-yl]-2,4-dioxo-1,4-dihydropyrido[2,3-*d*]pyrimidin-3(2*H*)-yl]cyclohexyl}carbamate (2.300 g, 3.57 mmol) in DCM (20 mL) was treated with trifluoroacetic acid (3 mL, 38.94 mmol) and stirred at room temperature overnight. All volatile materials were evaporated in vacuo and the residue was redissolved in DCM and washed thoroughly with saturated aqueous sodium bicarbonate solution. Drying with Na₂SO₄ and evaporation afforded the sub-title compound as a white foam (2.0 g).
¹H NMR (400 MHz, DMSO) δ 8.58 (d, J = 2.8 Hz, 1H), 8.30 (dd, J = 7.7, 3.1 Hz, 1H), 7.76 (d, J = 7.9 Hz, 1H), 7.71 (t, J = 1.8 Hz, 1H), 7.66 - 7.57 (m, 3H), 7.42 (d, J = 8.2 Hz, 2H), 7.36 (d, J = 9.7 Hz, 1H), 4.76 (t, J = 11.7 Hz, 1H), 3.70 (t, J = 6.0 Hz, 2H), 3.66 (s, 2H), 3.63 - 3.58 (m, 2H), 3.13 (s, 1H), 2.75 - 2.57 (m, 6H), 1.85 - 1.76 (m, 2H), 1.74 - 1.65 (m, 2H), 1.65 - 1.53 (m, 2H), 1.52 - 1.43 (m, 2H). Remaining resonances partially obscured by solvent peaks.

### Step (c) 3-{cis-4-[(3-Chloro-4-methoxybenzyl)amino]cyclohexyl}-6-fluoro-1-[4'-(1,4-oxazepan-4-ylmethyl)biphenyl-3-yl]pyrido[2,3-d]pyrimidine-2,4(1H,3H)-dione

The product was prepared from 3-(*cis*-4-aminocyclohexyl)-6-fluoro-1-[4'-(1,4-oxazepan-4-ylmethyl)biphenyl-3-yl]pyrido[2,3-d]pyrimidine-2,4(1*H*,3*H*)-dione (200 mg, 0.37 mmol) and 3-chloro-4-methoxybenzaldehyde (94 mg, 0.55 mmol) by the method of example 257 to give the title compound ditrifluoroacetate salt as a white powder (69 mg).
¹H NMR (400 MHz, DMSO) δ 10.29 - 10.15 (m, 1H), 8.82 - 8.66 (m, 2H), 8.61 (d, J = 2.8 Hz, 1H), 8.31 (dd, J = 7.7, 2.8 Hz, 1H), 7.86 - 7.81 (m, 1H), 7.80 - 7.75 (m, 2H), 7.70 - 7.67 (m, 1H), 7.67 - 7.61 (m, 2H), 7.50 (d, J = 6.9 Hz, 1H), 7.42 (d, J = 7.9 Hz, 1H), 7.21 (d, J = 8.7 Hz, 1H), 4.82 (t, J = 11.3 Hz, 1H), 4.45 (s, 2H), 4.17 (s, 2H), 3.57 - 3.16 (m, 2H), 2.68 - 2.54 (m, 2H), 2.22 - 1.98 (m, 4H), 1.86 - 1.71 (m, 2H), 1.71 - 1.59 (m, 2H). Other resonances obscured by DMSO and water peaks.
[M+H]+=698 (Multimode+)

The following compounds (Table 7) were prepared in a similar manner as solids from the appropriate amine and aldehyde using the method described above in Example 257.

**Table 7**

| | | | | |
|---|---|---|---|---|
| 259 | 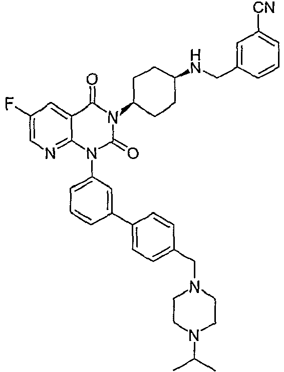 | 3-[({cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl}amino)methyl] benzonitrile | ¹H NMR (400 MHz, DMSO) δ 8.95 - 8.76 (m, 2H), 8.60 (d, J = 2.8 Hz, 1H), 8.31 (dd, J = 2.8, 7.7 Hz, 1H), 8.07 - 8.05 (m, 1H), 7.90 (d, J = 7.9 Hz, 2H), 7.79 (d, J = 7.7 Hz, 1H), 7.76 - 7.59 (m, 5H), 7.57 - 7.48 (m, 2H), 7.40 (d, J = 7.9 Hz, 1H), 4.89 - 4.77 (m, 1H), 4.35 - 4.27 (m, 2H), 3.58 - 3.40 (m, 2H), 3.42 - 3.31 (m, 2H), 2.66 - 2.54 (m, 2H), 2.20 - 2.06 (m, 2H), 1.88 - 1.73 (m, 2H), 1.74 - 1.60 (m, 2H), 1.24 (d, J = 6.4 Hz, 6H). Other resonances obscured by DMSO and water peaks. | 686 |
| 260 | 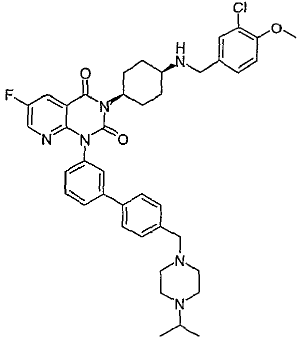 | 3-{cis-4-[(3-chloro-4-methoxybenzyl)amino]cycloh exyl}-6-fluoro-1-{4'-[(4-isopropylpiperazin-1-yl)methyl]biphenyl-3-yl}pyrido[2,3-d]pyrimidine-2,4(1H,3H)-dione | ¹H NMR (400 MHz, DMSO) δ 8.72 (s, 2H), 8.60 (d, J = 2.8 Hz, 1H), 8.31 (dd, J = 7.7,2.8 Hz, 1H), 7.79 (d, J = 7.7 Hz, 2H), 7.76 - 7.66 (m, 4H), 7.63 (t, J = 7.8 Hz, 1H), 7.54 - 7.47 (m, 3H), 7.40 (d, J = 7.7 Hz, 1H), 7.21 (d, J = 8.7 Hz, 1H), 4.88 - 4.76 (m, 1H), 4.21 - 4.12 (m, 2H), 3.99 - 3.90 (m, 2H), 3.87 (s, 3H), 3.54 - 3.41 (m, 2H), 3.34 - 3.03 (m, 4H), 2.65 - 2.55 (m, 2H), 2.09 (d, J = 13.3 Hz, 2H), 1.77 (t, J = 13.8 Hz, 2H), 1.66 (d, J = 11.0 Hz, 2H), 1.24 (d, J = 6.4 Hz, 6H). Other resonances obscured by DMSO and water peaks. | 725 |
| 261 | 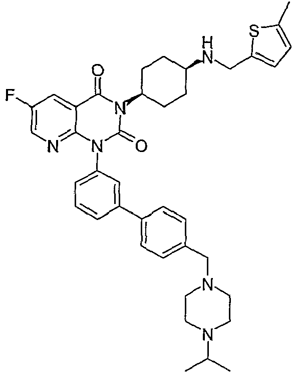 | 6-fluoro-1-{4'-[(4-isopropylpiperazin-1-yl)methyl]biphenyl-3-yl}-3-(cis-4- {[(5-methyl-2-thienyl)methyl]amino}cycloh exyl)pyrido[2,3-d]pyrimidine-2,4(1H,3H)-dione | ¹H NMR (400 MHz, DMSO) δ 8.76 (s, 2H), 8.60 (d, J = 2.6 Hz, 1H), 8.31 (dd, J = 2.6, 7.4 Hz, 1H), 7.79 (d, J = 7.7 Hz, 1H), 7.76 - 7.66 (m, 3H), 7.63 (t, J = 7.8 Hz, 1H), 7.57 (d, J = 5.1 Hz, 1H), 7.49 (d, J = 7.4 Hz, 2H), 7.39 (d, J = 7.7 Hz, 1H), 6.96 (d, J = 4.9 Hz, 1H), 4.88 - 4.77 (m, 1H), 4.41 - 4.32 (m, 2H), 3.52 - 3.29 (m, 5H), 3.23 - 3.00 (m, 4H), 2.72 - 2.54 (m, 2H), 2.26 (s, 3H), 2.16 - 2.04 (m, 3H), 1.88 - 1.73 (m, 2H), 1.72 - 1.59 (m, 2H), 1.24 (d, J = 6.4 Hz, 6H). Other resonances obscured by DMSO and water peaks. | 681 |
| 262 | 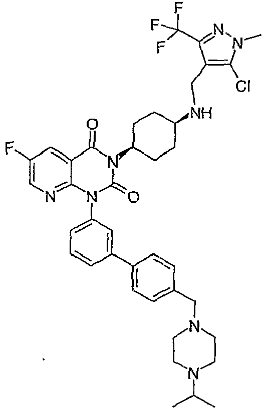 | 3-[cis-4-({[5-chloro-1-methyl-3-(trifluoromethyl)-1H-pyrazol-4-yl]methyl}amino)cyclohexyl] -6-fluoro-1-{4'-[(4-isopropylpiperazin-1-yl)methyl]biphenyl-3-yl}pyrido[2,3-d]pyrimidine-2,4(1H,3H)-dione | ¹H NMR (400 MHz, DMSO) δ 8.91 - 8.65 (m, 2H), 8.60 (d, J = 2.8 Hz, 1H), 8.30 (dd, J = 7.7, 2.8 Hz, 1H), 7.78 (d, J = 7.7 Hz, 2H), 7.75 - 7.65 (m, 3H), 7.62 (t, J = 7.8 Hz, 1H), 7.48 (d, J = 7.9 Hz, 2H), 7.39 (d, J = 7.7 Hz, 1H), 4.84 (t, J = 11.4 Hz, 1H), 4.16 (s, 2H), 3.94 (s, 3H), 3.52 - 3.32 (m, 4H), 3.22 - 2.99 (m, 3H), 2.69 - 2.52 (m, 4H), 2.20 (d, J = 13.3 Hz, 2H), 1.85 (t, J = 13.7 Hz, 2H), 1.69 (d, J = 10.0 Hz, 2H), 1.24 (d, J = 6.7 Hz, 6H). Other resonances obscured by DMSO and water peaks. | 767 |
| 263 | 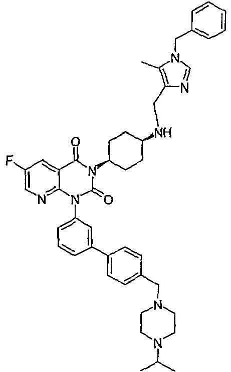 | 3-(cis-4- {[(1-benzyl-5-methyl-1H-imidazol-4-yl)methyl]amino} cyclohexyl) -6-fluoro-1-{4'-[(4-isopropylpiperazin-1-yl)methyl]biphenyl-3-yl}pyrido[2,3-d]pyrimidine-2,4(1H,3H)-dione | ¹H NMR (400 MHz, DMSO) δ 8.95 (s, 2H), 8.60 (d, J = 2.8 Hz, 1H), 8.29 (dd, J = 7.7, 2.8 Hz, 1H), 7.80 (d, J = 7.7 Hz, 1H), 7.74 - 7.66 (m, 3H), 7.63 (t, J = 7.8 Hz, 1H), 7.49 (d, J = 8.2 Hz, 2H), 7.40 - 7.27 (m, 3H), 7.21 (d, J = 7.2 Hz, 2H), 5.32 (s, 4H), 4.92-4.78 (m, 1H), 3.92 - 3.81 (m, 4H), 3.52 - 3.39 (m, 3H), 3.24 - 3.01 (m, 2H), 2.39 - 2.24 (m, 2H), 2.17 - 2.07 (m, 2H), 2.05 (s, 3H), 1.68 - 1.54 (m, 2H), 1.54-1.41 (m, 2H), 1.24 (d, J = 6.7 Hz, 6H). Some resonances obscured by DMSO and water. | 755 |
| 264 | 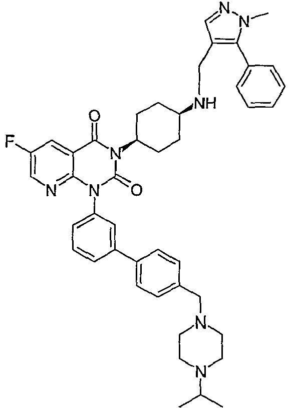 | 6-fluoro-1-{4'-[(4-isopropylpiperazin-1-yl)methyl]biphenyl-3-yl}-3-(cis-4-{[(1-methyl-5-phenyl-1H-pyrazol-4-yl)methyl] amino }cyclohexyl) pyrido[2,3-d]pyrimidine-2,4(1H,3H)-dione | ¹H NMR (400 MHz, DMSO) δ 8.73 - 8.63 (m, 2H), 8.60 (d, J = 2.8 Hz, 1H), 8.30 (dd, J = 7.7, 2.8 Hz, 1H), 7.79 (d, J = 8.2 Hz, 1H), 7.76 - 7.72 (m, 3H), 7.69 (d, J = 7.9 Hz, 2H), 7.62 (t, J = 7.8 Hz, 1H), 7.59 - 7.47 (m, 6H), 7.39 (d, J = 9.3 Hz, 1H), 7.39 (d, J = 7.8 Hz, 1H), 4.76 (t, J = 10.9 Hz, 1H), 3.72 (s, 3H), 3.55 - 3.44 (m, 4H), 3.30 - 3.02 (m, 6H), 2.83 - 2.66 (m, 2H), 1.88 - 1.78 (m, 2H), 1.69 - 1.53 (m, 4H), 1.24 (d, J = 6.4 Hz, 6H). Some resonances obscured by DMSO ands water peaks. | 741 |
| 265 | 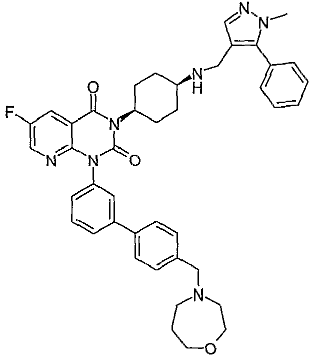 | 6-fluoro-3-(cis-4-{[(1-methyl-5-phenyl-1H-pyrazol-4-yl)methyl]amino}cyclohexyl) -1-[4'-(1,4-oxazepan-4-ylmethyl)biphenyl-3-yl]pyrido[2,3-d]pyrimidine-2,4(1H,3H)-dione | ¹H NMR (400 MHz, DMSO) δ 10.29 - 10.08 (m, 1H), 8.75 - 8.62 (m, 2H), 8.60 (d, J = 2.6 Hz, 1H), 8.30 (dd, J = 7.4,2.6 Hz, 1H), 7.83 (d, J = 7.9 Hz, 1H), 7.80 - 7.71 (m, 4H), 7.69 - 7.60 (m, 3H), 7.60 - 7.46 (m, 6H), 7.41 (d, J = 7.7 Hz, 1H), 4.82 - 4.70 (m, 1H), 4.45 (s, 2H), 3.92 (s, 2H), 3.45 - 3.10 (m, 7H), 2.21 - 1.95 (m, 2H), 1.93 - 1.74 (m, 2H), 1.72 - 1.47 (m, 4H). Other resonances obscured by DMSO and water peaks. | 714 |
| 266 | 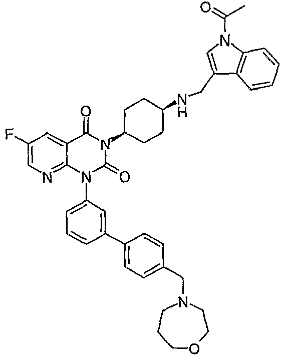 | 3-(cis-4-{[(1-acetyl-1H-indol-3-yl)methyl] amino }cyclohexyl) -6-fluoro-1-[4'-(1,4-oxazepan-4-ylmethyl)biphenyl-3-yl]pyrido[2,3-d]pyrimidine-2,4(1H,3H)-dione | ¹H NMR (400 MHz, DMSO) δ 10.31 - 10.16 (m, 1H), 8.93 - 8.74 (m, 2H), 8.61 (d, J = 2.3 Hz, 1H), 8.38 - 8.28 (m, 2H), 8.12 (s, 1H), 7.84 (t, J = 8.8 Hz, 2H), 7.81 - 7.74 (m, 3H), 7.69 - 7.61 (m, 3H), 7.46 - 7.32 (m, 3H), 4.92 - 4.77 (m, 1H), 4.45 (s, 2H), 4.40 (s, 2H), 2.71 - 2.55 (m, 5H), 2.26 - 1.95 (m, 4H), 1.88 - 1.74 (m, 2H), 1.73 - 1.59 (m, 2H). Other resonances obscured by DMSO and water peaks. | 715 |
| 267 | 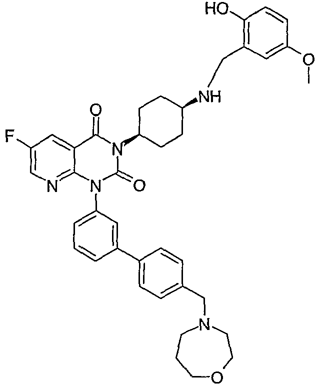 | 6-fluoro-3-{cis-4-[(2-hydroxy-5-methoxybenzyl)amino] cycloh exyl}-1-[4'-(1,4-oxazepan-4-ylmethyl)biphenyl-3-yl]pyrido[2,3-d]pyrimidine-2,4(1H,3H)-dione | ¹H NMR (400 MHz, DMSO) δ 10.16 - 10.04 (m, 1H), 9.91 - 9.82 (m, 1H), 8.61 (d, *J* = 3.1 Hz, 1H), 8.51 - 8.42 (m, 2H), 8.31 (dd, *J* = 7.7, 3.0 Hz, 1H), 7.83 (d, *J* = 7.9 Hz, 1H), 7.80 - 7.74 (m, 3H), 7.67 - 7.61 (m, 3H), 7.42 (d, *J* = 7.9 Hz, 1H), 7.00 (s, 1H), 6.85 (d, *J* = 1.5 Hz, 2H), 4.83 (t, *J* = 11.7 Hz, 1H), 4.45 (s, 2H), 4.12 (s, 2H), 3.92 - 3.70 (m, 5H), 3.69 (s, 3H), 3.55 - 3.45 (m, 1H), 3.44 - 3.34 (m, 1H), 3.25 - 3.13 (m, 1H), 2.66 - 2.54 (m, 2H), 2.18 - 2.01 (m, 4H), 1.79 (t, *J* = 14.1 Hz, 2H), 1.73 - 1.60 (m, 2H). Some resonances partially obscured by DMSO and water. | 680 |

### Example 268

### 6-Fluoro-N-{cis-4-[6-fluoro-2,4-dioxo-1-{4'-[(piperidin-4-ylamino)methyl]biphenyl-3-yl}-1,4-dihydropyrido[2,3-d]pyrimidin-3(2H)-yl]cyclohexyl}imidazo[1,2-a]pyridine-2-carboxamide

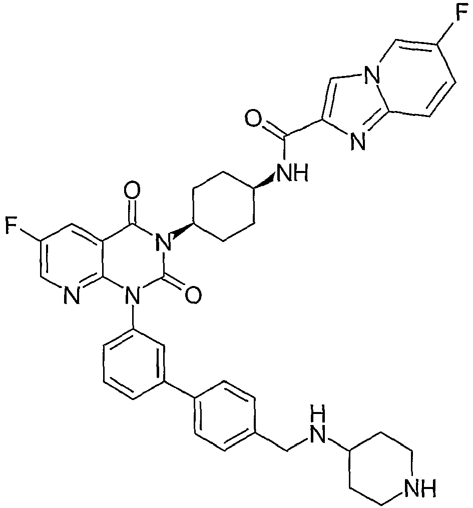

A solution of 6-fluoro-*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.8 g, 1.29 mmol) and *tert*-butyl 4-aminopiperidine-1-carboxylate (0.310 g, 1.55 mmol) in DCM (30 mL) was stirred for 30 mins then sodium triacetoxyborohydraide (0.328 g, 1.55 mmol) was added. The mixture was stirred at room temperature for 24 h. TFA (1 ml) was added to the reaction, which was stirred for 2 h and then quenched with water and methanol (0.5 ml) and concentrated in vacuo. Water was added then stirred for 5 h and filtered to leave a brown solid. The crude product was purified by flash silica chromatography, elution gradient 0 to 4% methanol in ethyl acetate. Pure fractions were evaporated to dryness to afford the title compound (0.230 g).
¹H NMR (300 MHz, DMSO) δ 9.02 (s, 1H), 8.80 (s, 1H), 8.59 (s, 1H), 8.39 - 8.33 (m, 2H), 7.85 - 7.72 (m, 5H), 7.70 - 7.58 (m, 4H), 7.49 - 7.40 (m, 2H), 4.95 - 4.79 (m, 1H), 4.29 - 4.21 (m, 2H), 4.21 - 4.13 (m, 1H), 3.03 - 2.85 (m, 2H), 2.65 - 2.53 (m, 2H), 2.32 - 2.20 (m, 4H), 2.08 - 1.96 (m, 3H), 1.80 - 1.61 (m, 6H).
[M+H]+=705 (MultiMode+)

### Example 269

### 6-Fluoro-N-cis-4-[6-fluoro-1-{4'-[(4-methylpiperazin-1-yl)methyl]biphenyl-3-yl}-2,4-dioxo-1,4-dihydropyrido[2,3-d]pyrimidin-3(2H)-yl]cyclohexyl}imidazo[1,2-a]pyridine-2-carboxamide

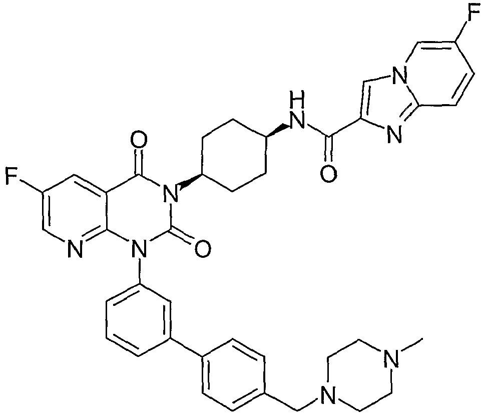

The product was prepared from 6-fluoro-*N*-*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl} imidazo[1,2-a]pyridine-2-carboxamide (250 mg, 0.40 mmol) and 1-methylpiperazine (44.7 µl, 0.40 mmol) by the method of example 56 to afford the title compound as a colourless solid (0.191 g).

¹H NMR (400 MHz, DMSO) δ 8.83 (dd, J = 4.0, 2.2 Hz, 1H), 8.59 (d, J = 2.8 Hz, 1H), 8.42 (s, 1H), 8.34 (dd, J = 7.7, 3.1 Hz, 1H), 7.83 - 7.70 (m, 6H), 7.64 (t, J = 7.8 Hz, 1H), 7.55 - 7.45 (m, 4H), 7.41 (d, J = 7.7 Hz, 1H), 4.88 (t, J = 12.0 Hz, 1H), 4.17 (s, 1H), 4.02 (s, 1H), 3.49 - 3.06 (m, 4H), 2.82 (s, 3H), 2.61 (dd, J = 35.5, 11.9 Hz, 6H), 2.02 (d, J = 12.8 Hz, 2H), 1.78 - 1.61 (m, 4H).
[M+H]+=705 (MultiMode+)

Example 270

### N-{cis-4-[1-(4'-{[(3R,5S)-3,5-Dimethylpiperazin-1-yl]methyl}biphenyl-3-yl)-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

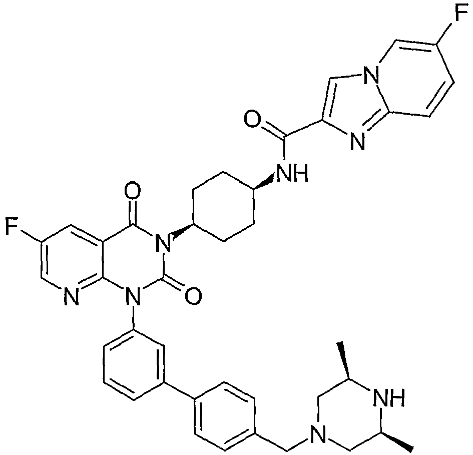

The product was prepared from 6-fluoro-*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl} imidazo [1,2-*a*]pyridine-2-carboxamide (0.5 g, 0.81 mmol) and cis 2,6-dimethylpiperazine (0.092 g, 0.81 mmol) by the method of example 56 to afford the title compound as a colourless solid (0.379 g).
¹H NMR (400 MHz, DMSO) δ 9.33 (br s, 1H), 8.83 (dd, J = 4.1, 2.8 Hz, 1H), 8.59 (d, J = 2.8 Hz, 2H), 8.41 (s, 1H), 8.34 (dd, J = 7.7, 3.1 Hz, 1H), 7.81 (d, J = 7.9 Hz, 1H), 7.78 - 7.69 (m, 6H), 7.63 (t, J = 7.8 Hz, 1H), 7.49 (td, J = 12.1, 8.2 Hz, 3H), 7.43 - 7.38 (m, 1H), 4.88 (t, J = 12.0 Hz, 1H), 4.17 (s, 1H), 3.99 (s, 2H), 3.43 (s, 2H), 3.26 (d, J = 11.3 Hz, 2H), 2.72 - 2.38 (m, 4H), 2.02 (d, J = 13.1 Hz, 2H), 1.79 - 1.59 (m, 4H), 1.20 (d, J = 6.7 Hz, 6H).
[M+H]+=719 (MultiMode+)

### Example 271

### 6-Fluoro-N-{cis-4-[6-fluoro-1-(4'-{[(3S)-3-methylpiperazin-1-yl]methyl}biphenyl-3-yl)-2,4-dioxo-1,4-dihydropyrido[2,3-d]pyrimidin-3(2H)-yl]cyclohexyl}imidazo[1,2-a]pyridine-2-carboxamide

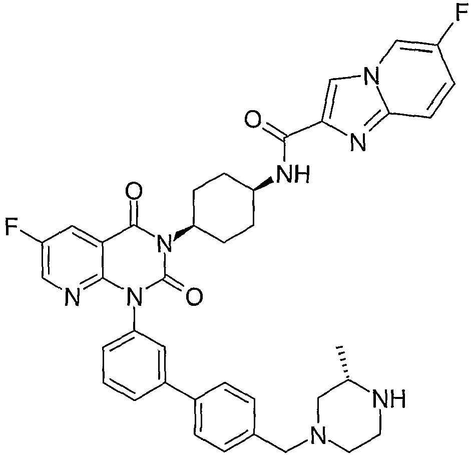

The product was prepared from 6-fluoro-*N*-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}imidazo[1,2-a]pyridine-2-carboxamide (0.5 g, 0.81 mmol) and *tert*-butyl (2*S*)-2-methylpiperazine-1-carboxylate (0.161 g, 0.81 mmol) by the method of example 56 to afford the title compound as a colourless solid (0.490 g).
¹H NMR (400 MHz, DMSO) δ 9.30 (br s, 1H), 8.96 - 8.76 (m, 2H), 8.59 (d, J = 3.1 Hz, 1H), 8.41 (s, 1H), 8.34 (dd, J = 7.7, 3.1 Hz, 1H), 7.84 - 7.37 (m, 10H), 4.88 (t, J = 11.9 Hz, 1H), 4.12 (d, J = 37.7 Hz, 3H), 3.51 - 3.06 (m, 5H), 2.86 - 2.55 (m, 4H), 2.02 (d, J = 13.1 Hz, 2H), 1.78 - 1.57 (m, 4H), 1.21 (d, J = 6.7 Hz, 3H).
[M+H]+=705 (MultiMode+)

### Example 272

### N-{cis-4-[1-{4'-[(Dimethylamino)methyl]biphenyl-3-yl}-6-fluoro-2,4-dioxo-1,4-dihydropyrido[2,3-d]pyrimidin-3(2H)-yl]cyclohexyl}-2-(2-hydroxyethoxy)benzamide

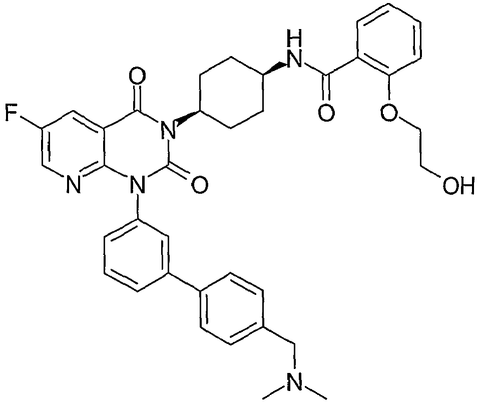

### Step (a) N-{cis-4-[1-{4'-[(Dimethylamino)methyl]biphenyl-3-yl}-6-fluoro-2,4-dioxo-1,4-dihydropyrido[2,3-d]pyrimidin-3(2H)-yl]cyclohexyl}-2-[2-(tetrahydro-2H-pyran-2-yloxy)ethoxy]benzamide

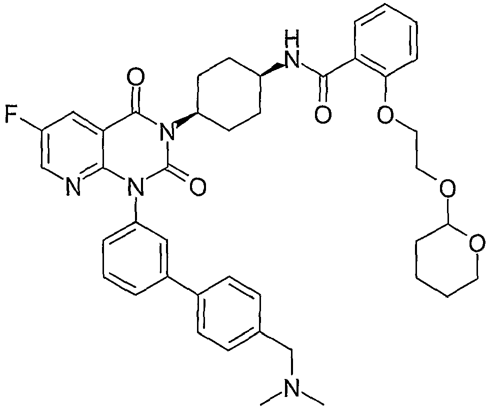

To a stirred solution of 3-(*cis*-4-aminocyclohexyl)-1-{4'-[(dimethylamino)methyl]biphenyl-3-yl}-6-fluoropyrido[2,3-*d*]pyrimidine-2,4(1*H*,3*H*)-dione dihydrochloride (0.2 g, 0.36 mmol) in DMF (4 mL) was added 2-[2-(tetrahydro-2*H*-pyran-2-yloxy)ethoxy]benzoic acid (0.114 g, 0.43 mmol), Hünig's base (0.187 mL, 1.07 mmol) and finally HATU (0.163 g, 0.43 mmol). The reaction mixture was stirred at room temperature overnight. The reaction mixture was diluted with ethyl acetate (20 mL) and washed with water (2 x 30 mL). The aqueous layer was back-extracted with ethyl acetate (30 mL) and the combined organics washed with saturated brine (30 mL), dried over magnesium sulphate, filtered and evaporated to afford the sub-title compound as a pale yellow solid (0.296 g).
¹H NMR (400 MHz, DMSO) δ 8.59 (d, J = 2.8 Hz, 1H), 8.36 (d, J = 6.0 Hz, 1H), 8.32 (dd, J = 7.8, 2.9 Hz, 1H), 7.98 - 7.93 (m, 2H), 7.82 (d, J = 14.2 Hz, 1H), 7.78 - 7.75 (m, 3H), 7.63 (t, J = 7.9 Hz, 1H), 7.55 (d, J = 8.2 Hz, 1H), 7.48 (s, 1H), 7.40 (d, J = 13.0 Hz, 1H), 7.21 (d, J = 11.1 Hz, 1H), 7.06 (t, J = 9.5 Hz, 1H), 4.91 - 4.79 (m, 1H), 4.60 (s, 1H), 4.42 - 4.33 (m, 2H), 4.21 - 4.11 (m, 2H), 3.96 - 3.76 (m, 2H), 3.70 - 3.61 (m, 1H), 3.34 (s, 2H), 2.88 (s, 6H), 2.65 - 2.60 (m, 6H), 1.73 - 1.59 (m, 4H), 1.46 - 1.33 (m, 4H).

### Step (b) N-{cis-4-[1-{4'-[(Dimethylamino)methyl]biphenyl-3-yl}-6-fluoro-2,4-dioxo-1,4-dihydropyrido[2,3-d]pyrimidin-3(2H)-yl]cyclohexyl}-2-(2-hydroxyethoxy)benzamide

To a mixture of *N*-{*cis*-4-[1-{4'-[(dimethylamino)methyl]biphenyl-3-yl}-6-fluoro-2,4-dioxo-1,4-dihydropyrido[2,3-*d*]pyrimidin-3(2*H*)-yl]cyclohexyl}-2-[2-(tetrahydro-2*H*-pyran-2-yloxy)ethoxy]benzamide (0.29 g, 0.39 mmol) in THF (2 mL) and water (1 ml) was added acetic acid (4.5 ml, 78.61 mmol) and the reaction mixture was heated at 80 °C for 18 h. The reaction was allowed to cool to room temperature and poured onto saturated sodium bicarbonate solution (50mL). The product was extracted with DCM (150mL). The DCM was evaporated and the residue dissolved in methanol. The solvents were removed by evaporation in vacuo and the residue was stirred in ether overnight. The crude product was purified by preparative HPLC on a Waters X-Bridge column using a 65-45 % gradient of aqueous 0.2% ammonia in acetonitrile as eluent. The fractions containing the desired compound were lyophilsed to afford the title compound as a white solid (44 mg).
¹H NMR (400 MHz, DMSO) δ 8.58 (d, J = 2.8 Hz, 1H), 8.43 (d, J = 6.2 Hz, 1H), 8.30 (dd, J = 7.7, 3.1 Hz, 1H), 7.94 (d, J = 6.2 Hz, 1H), 7.76 (d, J = 7.9 Hz, 1H), 7.71 (s, 1H), 7.60 (q, J = 7.9 Hz, 3H), 7.47 (t, J = 7.0 Hz, 1H), 7.40 - 7.34 (m, 2H), 7.20 (d, J = 8.2 Hz, 1H), 7.05 (t, J = 7.4 Hz, 1H), 4.91 - 4.76 (m, 2H), 4.27 (s, 2H), 4.16 (s, 1H), 3.84 - 3.77 (m, 2H), 3.46 (s, 2H), 2.73 - 2.60 (m, 2H), 2.19 (s, 6H), 2.08 - 1.99 (m, 2H), 1.71 - 1.59 (m, 4H).
[M+H]+=652 (MultiMode+)

### Example 273

### N-{cis-4-[1-{2'-[(Dimethylamino)methyl]-4'-fluorobiphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

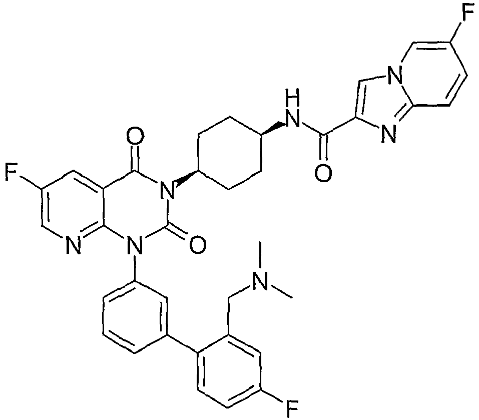

### Step (a) N-{cis-4-[1-[2'-(Aminomethyl)-4'-fluorobiphenyl-3-yl]-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

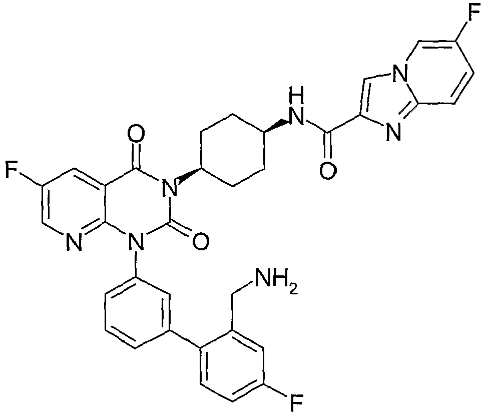

Acetonitrile (4 mL) was added to palladium (II) acetate (10.48 mg, 0.05 mmol) and 2-dicyclohexylphosphino-2',6'-dimethoxybiphenyl (0.038 g, 0.09 mmol) and the mixture was stirred at room temperature for 10 min. Potassium carbonate (0.194 g, 1.40 mmol) in water (2 mL), 6-fluoro-N- {cis-4-[6-fluoro-1-(3-iodophenyl)-2,4-dioxo-1,4-dihydropyrido[2,3-d]pyrimidin-3(2H)-yl]cyclohexyl}imidazo[1,2-a]pyridine-2-carboxamide (0.3 g, 0.47 mmol), and (2-{[(tert-butoxycarbonyl)amino]methyl}-4-fluorophenyl)boronic acid (0.126 g, 0.47 mmol) were added and the reaction mixture was heated at 80 °C overnight. The reaction mixture was directly loaded onto a ChemElut cartridge and the product eluted with DCM. The solvent was evaporated and the residue dissolved in 1,4-dioxane (5 mL). Hydrogen chloride in dioxane (4M) (5 ml, 20.00 mmol) was added and the reaction mixture was stirred at room temperature for 5 h. The solvents were removed in vacuo and the residue triturated in ether overnight. The solid was removed by filtration to give the sub-title compound as a beige solid (0.363 g).
¹H NMR (400 MHz, DMSO) δ 8.94 (s, 1H), 8.70 (d, J = 3.1 Hz, 1H), 8.54 - 8.47 (m, 3H), 8.33 (d, J = 16.1 Hz, 1H), 7.90 - 7.85 (m, 1H), 7.82 - 7.78 (m, 1H), 7.68 - 7.56 (m, 2H), 7.50 - 7.40 (m, 2H), 7.32 (d, J = 13.7 Hz, 2H), 4.87 (t, J = 14.7 Hz, 1H), 4.17 - 4.11 (m, 1H), 4.04 - 3.99 (m, 2H), 2.64 - 2.59 (m, 2H), 2.03 (d, J = 29.8 Hz, 2H), 1.74 - 1.61 (m, 4H).

### Step (b) N-{cis-4-[1-{2'-[(Dimethylamino)methyl]-4'-fluorobiphenyl-3-yl}-6-fluoro-2,4-dioxo-1,4-dihydropyrido[2,3-d]pyrimidin-3(2H)-yl]cyclohexyl}-6-fluoroimidazo[1,2-a]pyridine-2-carboxamide

A mixture of *N*-{*cis*-4-[1-[2'-(aminomethyl)-4'-fluorobiphenyl-3-yl]-6-fluoro-2,4-dioxo-1,4-dihydropyrido[2,3-*d*]pyrimidin-3(2*H*)-yl]cyclohexyl}-6-fluoroimidazo[1,2-*a*]pyridine-2-carboxamide (0.363 g, 0.57 mmol) and formaldehyde (37% wt% in water, 0.2 mL, 2.69 mmol) in 1,2-dichloroethane (10 mL) was stirred at room temperature for 10 min. To this was added sodium triacetoxyborohydride (0.241 g, 1.14 mmol) and the reaction mixture was stirred at room temperature for 1 hour. The reaction mixture was concentrated in vaccuo. The residue was dissolved in methanol (5 mL), and loaded on to a 10g SCX cartridge. The impurities were washed through with methanol (70 mL) and discarded. The product was eluted with 1N methanolic ammonia (100 mL) and evaporated *in vacuo*. The crude product was purified by preparative HPLC on a Waters X-Bridge column using a 55-35% gradient of aqueous 0.2% ammonia in acetonitrile as eluent then by flash silica chromatography, eluting with 5% methanol in DCM. Pure fractions were evaporated to dryness to afford the title compound as a white solid (60 mg).
¹H NMR (400 MHz, DMSO) δ 8.80 (ddt, J = 4.1, 2.6, 0.1 Hz, 1H), 8.60 (d, J= 2.8 Hz, 1H), 8.37 (s, 1H), 8.32 (dd, J = 7.7, 3.1 Hz, 1H), 7.74 (dd, J = 9.9, 5.3 Hz, 1H), 7.67 (d, J = 7.2 Hz, 1H), 7.59 (t, J = 8.1 Hz, 1H), 7.49 - 7.38 (m, 4H), 7.37 - 7.28 (m, 2H), 7.18 (td, J = 11.1, 5.0 Hz, 1H), 4.88 (t, J = 16.5 Hz, 1H), 4.19 (s, 1H), 2.69 - 2.55 (m, 2H), 2.08 - 1.98 (m, 8H), 1.76 - 1.61 (m, 4H). Other resonances obscured by water peak.
[M+H]+=668 (MultiMode+)

### Example 274

### 6-Fluoro-N-{cis-4-[6-fluoro-1-[2'-hydroxy-4'-(piperazin-1-ylmethyl)biphenyl-3-yl]-2,4-dioxo-1,4-dihydropyrido[2,3-d]pyrimidin-3(2H)-yl]cyclohexyl}imidazo[1,2-a]pyridine-2-carboxamide

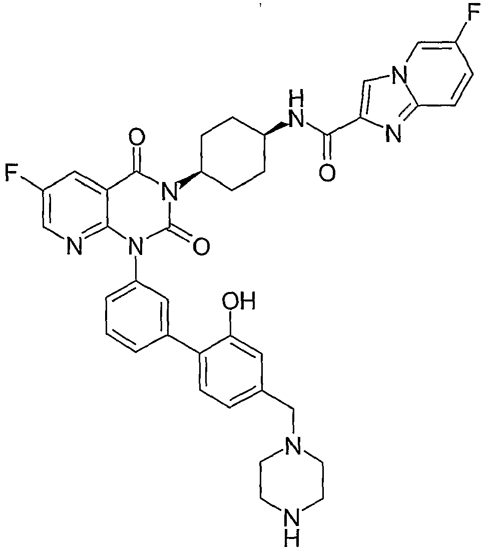

### Step (a) tert-Butyl 4-({3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]-2-hydroxybiphenyl-4-yl}methyl)piperazine-1-carboxylate

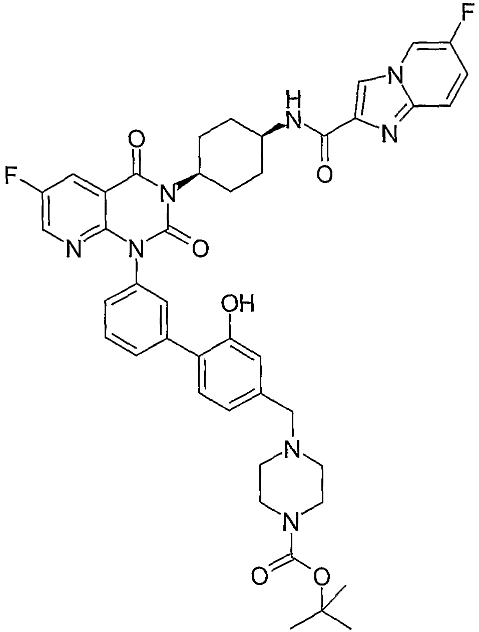

To a solution of 6-fluoro-*N*-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide (0.25 g, 0.39 mmol) in 1,2-dichloroethane (10 mL) was added tert-butyl 1-piperazinecarboxylate (0.110 g, 0.59 mmol) and acetic acid (0.045 mL, 0.79 mmol). The reaction mixture was stirred for 10 min before addition of sodium triacetoxyborohydride (0.125 g, 0.59 mmol). The reaction mixture was stirred at room temperature overnight then concentrated in vaccuo. The residue was dissolved in methanol (5 mL), and loaded on to a 10g SCX cartridge. The impurities were washed through with methanol (70 mL) and discarded. The product was eluted with 1N methanolic ammonia (100 mL) and evaporated *in vacuo*. The residue was left on the high vac overnight to give the sub-title compound (0.156 g).
[M+H]+=807

### Step (b) 6-Fluoro-N-{cis-4-[6-fluoro-l-[2'-hydroxy-4'-(piperazin-1-ylmethyl)biplienyl-3-yl]-2,4-dioxo-1,4-dihydropyrido[2,3-d]pyrimidin-3(2H)-yl]cyclohexyl}imidazo[1,2-a]pyridine-2-carboxamide

Hydrogen chloride in 1,4-dioxane (4M) (1 mL, 4.00 mmol) was added to a solution of *tert-*butyl-4-({3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]-2-hydroxybiphenyl-4-yl}methyl)piperazine-1-carboxylate (0.15 g, 0.19 mmol) in 1,4-dioxane (10 mL) and the reaction mixture was stirred at room temperature overnight. The solvents were evaporated and the residue was purified by preparative HPLC on a Sunfire column using a 90-70% gradient of aqueous 0.1% trifluoroacetic acid in acetonitrile as eluent. The fractions containing the desired compound were lyophilised to afford the title compound as a white solid (27 mg).
¹H NMR (400 MHz, DMSO) δ 8.70 (s, 1H), 8.58 (d, J = 3.1 Hz, 1H), 8.31 (dd, J = 7.7, 3.1 Hz, 1H), 7.86 - 7.78 (m, 3H), 7.55 - 7.49 (m, 3H), 7.49 - 7.43 (m, 1H), 7.40 - 7.36 (m, 2H), 7.03 (s, 1H), 6.94 (d, J = 19.2 Hz, 1H), 4.86 (t, J = 22.2 Hz, 1H), 4.11 (s, 1H), 3.63 (s, 2H), 3.26 - 3.16 (m, 4H), 2.90 - 2.71 (m, 4H), 2.04 - 1.90 (m, 4H), 1.75 - 1.57 (m, 4H).
[M+H]+=707 (MultiMode+)

### Example 275

### N-{cis-4-[6-Fluoro-1-[2'-(morpholin-4-ylmethyl)biphenyl-3-yl]-2,4-dioxo-1,4-dihydropyrido[2,3-d]pyrimidin-3(2H)-yl]cyclohexyl}-1-(propylamino)cyclopentanecarboxamide

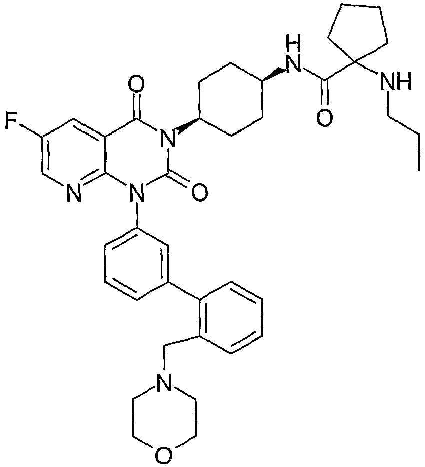

1-Amino-*N*-{*cis*-4-[6-fluoro-1-[2'-(morpholin-4-ylmethyl)biphenyl-3-yl]-2,4-dioxo-1,4-dihydropyrido[2,3-*d*]pyrimidin-3(2*H*)-yl]cyclohexyl}cyclopentanecarboxamide (0.54 g, 0.84 mmol) and propionic aldehyde (0.049 g, 0.84 mmol) in 1,2-dichloroethane (20 ml) were treated with sodium triacetoxyborohydride (0.214 g, 1.01 mmol) and the resultant mixture stirred overnight. The mixture was evaporated to dryness and the crude product was purified by preparative HPLC on a Waters X-Terra column using a 95-50% gradient of aqueous 0.1% trifluoroacetic acid in acetonitrile as eluent. The fractions containing the desired compound were evaporated to dryness to afford the title compound as a colourless solid (0.372 g).
¹H NMR (400 MHz, DMSO) δ 10.40 (s, 1H), 9.07 (s, 2H), 8.59 (d, *J* = 2.8 Hz, 1H), 8.31 (dd, *J*= 7.7, 2.8 Hz, 1H), 7.71 (s, 1H), 7.67 (t, *J*= 7.9 Hz, 1H), 7.58 - 7.50 (m, 3H), 7.49 - 7.44 (m, 1H), 7.44 - 7.39 (m, 1H), 4.81 (t, *J*= 11.8 Hz, 1H), 4.33 (s, 2H), 3.89 (s, 1H), 3.79 - 3.53 (m, 4H), 3.27 - 3.06 (m, 2H), 2.81 - 2.55 (m, 6H), 2.37 - 2.23 (m, 2H), 2.05 - 1.91 (m, 4H), 1.85 - 1.69 (m, 5H), 1.68 - 1.51 (m, 7H), 0.90 (t, *J*= 7.3 Hz, 3H).
[M+H]+=683 (MultiMode+)

### Example 277

### 1-{2'-[(dimethylamino)methyl]-4'-hydroxybiphenyl-3-yl}-6-fluoro-3-(cis-4-{[(6-fluoroimidazo[1,2-a]pyridin-2-yl)methyl]amino}cyclohexyl)pyrido[2,3-d]pyrimidine-2,4(1H,3H)-dione

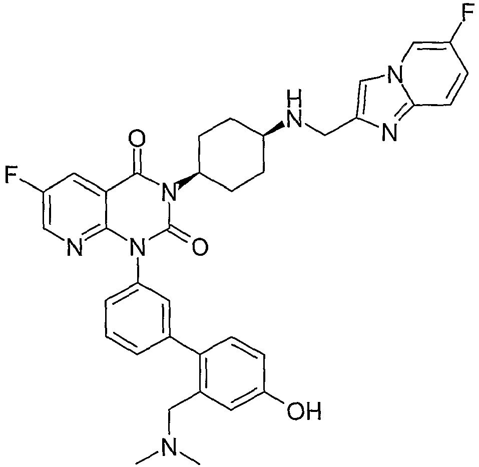

### Step (a) tert-Butyl {cis-4-[1-{2'-[(dimethylamino)methyl]-4'-hydroxybiphenyl-3-yl}-6-fluoro-2,4-dioxo-1,4-dihydropyrido[2,3-d]pyrimidin-3(2H)-yl]cyclohexyl}carbamate

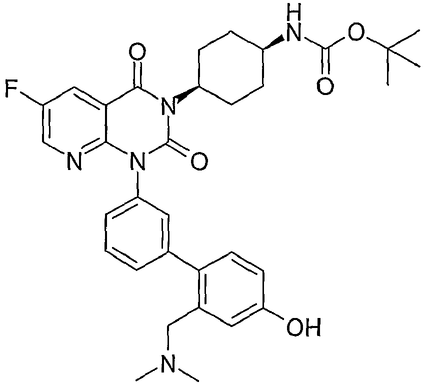

The product was prepared from *tert*-butyl-{*cis*-4-[6-fluoro-1-(2'-formyl-4'-hydroxybiphenyl-3-yl)-2,4-dioxo-1,4-dihydropyrido[2,3*-d*]pyrimidin-3(2*H*)-yl]cyclohexyl}carbamate (0.5 g, 0.87 mmol) and dimethylamine (1.305 ml, 2.61 mmol) by the method of example 274 step (a) to afford the sub-title compound (0.50 g).
[M+H]+=604

### Step (b) 3-(cis-4-Aminocyclohexyl)-1-{2'-[(dimethylamino)methyl]-4'-hydroxybiphenyl-3-yl}-6-fluoropyrido[2,3-d]pyrimidine-2,4(1H,3H)-dione

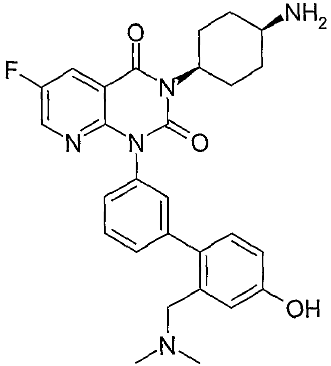

The product was prepared from *tert*-butyl {*cis*-4-[1-{2'-[(dimethylamino)methyl]-4'-hydroxybiphenyl-3-yl}-6-fluoro-2,4-dioxo-1,4-dihydropyrido[2,3-*d*]pyrimidin-3(2*H*)-yl]cyclohexyl}carbamate (0.5 g, 0.83 mmol) by the method of example 230 step (d) to give the sub-title compound (0.410 g).
[M+H]+=604

### Step (c) 1-{2'-[(dimethylamino)methyl]-4'-hydroxybiphenyl-3-yl}-6-fluoro-3-(cis-4-{[(6-fluoroimidazo[1,2-a]pyridin-2-yl)methyl]amino}cyclohexyl)pyrido[2,3-d]pyrimidine-2,4(1H,3H)-dione

To a solution of 6-fluoroimidazo[1,2-*a*]pyridine-2-carbaldehyde (0.082 g, 0.50 mmol) in 1,2-dichloroethane was added 3-(*cis*-4-aminocyclohexyl)-1-{2'-[(dimethylamino)methyl]-4'-hydroxybiphenyl-3-yl}-6-fluoropyrido[2,3-*d*]pyrimidine-2,4(1*H*,3*H*)-dione (0.263 g, 0.52 mmol) and acetic acid (0.038 g, 0.63 mmol) and the reaction mixture stirred at 25 °C for 10 mins. Sodium triacetoxyborohydride (0.111 g, 0.52 mmol) was added and the reaction stirred for 14 hours. The crude material was dissolved in methanol (5 mL), and loaded on a 20g SCX cartridge. The impurities were washed through with methanol (250 mL) and discarded. The product was eluted with 1N methanolic ammonia (250 mL) and evaporated. The crude product was purified by preparative HPLC on a Waters X-Terra column using a 95-50% gradient of aqueous 0.1 % trifluoroacetic acid in acetonitrile as eluent then further purified by preparative HPLC on a Waters X-Terra column using a 95-5% gradient of aqueous 0.1% ammonium acetate in methanol as eluent. The fractions containing the desired compound were evaporated to dryness to afford the title compound (0.104 g).
¹H NMR (400 MHz, DMSO) δ 8.71 - 8.70 (m, 1H), 8.59 - 8.58 (m, 1H), 8.29 - 8.26 (m, 1H), 7.84 (s, 1H), 7.55 - 7.50 (m, 2H), 7.43 - 7.41 (m, 2H), 7.32 - 7.23 (m, 2H), 7.10 (d, J = 8.2 Hz, 1H), 6.92 (d, J = 2.6 Hz, 1H), 6.73 (dd, J = 8.2, 2.6 Hz, 1H), 4.81 - 4.75 (m, 1H), 3.80 (s, 2H), 3.24 (s, 2H), 2.85 - 2.67 (m, 3H), 2.54 (s, 1H), 2.06 (s, 3H) 1.90 - 1.87 (m, 7H), 1.51 - 1.40 (m, 4H).
[M+H]+=652 (MultiMode+)

The following compounds (Table 8) were prepared as solids from 3-(*cis*-4-aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione and the appropriate aldehyde using the method described above in Example 247.

**Table 8**

| **Example Number** | **Chemistry** | **Name** | **NMR** | **M+H** |
|---|---|---|---|---|
| 278 | 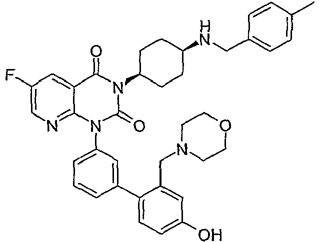 | 6-fluoro-1-[4'-hydroxy-2'-(morpholin-4-ylmethyl)biphenyl-3-yl]-3-{*cis*-4-[(4-methylbenzyl)amino]cyclohexyl}pyr ido[2,3-*d*]pyrimidine-2,4(1*H*,3*H*)-dione | ¹H NMR (400 MHz, DMSO) δ 9.48 (s, 1H), 8.58 (d, J = 3.1 Hz, 1H), 8.29 (dd, J = 7.7, 3.1 Hz, 1H), 7.60 - 7.46 (m, 2H), 7.41 (d, J = 7.7 Hz, 1H), 7.31 (d, J = 7.9 Hz, 1H), 7.24 (d, J = 7.9 Hz, 2H), 7.12 (dd, J = 11.1, 8.1 Hz, 3H), 6.88 (d, J = 2.6 Hz, 1H), 6.76 (dd, J = 8.3, 2.4 Hz, 1H), 4.76 (t, J = 12.0 Hz, 1H), 3.66 (s, 2H), 3.43 (s, 4H), 3.28 (s, 4H), 2.79 - 2.64 (m, 4H), 2.27 (s, 4H), 1.85 (d, J = 13.1 Hz, 2H), 1.50 - 1.35 (m, 4H). | 650 |
| 279 | 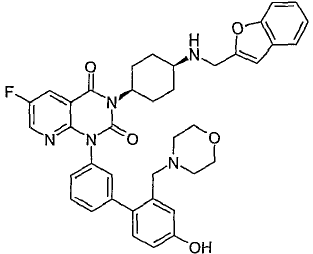 | 3-{*cis*-4-[(1-benzofuran-2-ylmethyl)amino]cyclohexyl}-6-fluoro-1-[4'-hydroxy-2'-(morpholin-4-ylmethyl)biphenyl-3-yl]pyrido[2,3-d]pyrimidine-2,4(1*H*,3*H*)-dione | ¹H NMR (400 MHz, DMSO) δ 9.48 (s, 1H), 8.58 (d, J = 3.1 Hz, 1H), 8.29 (dd, J = 7.7, 3.1 Hz, 1H), 7.59 - 7.47 (m, 3H), 7.41 (d, J = 7.7 Hz, 1H), 7.31 (d, J = 7.9 Hz, 1H), 7.27 - 7.09 (m, 3H), 6.88 (d, J = 2.6 Hz, 1H), 6.78 - 6.72 (m, 2H), 4.77 (t, J = 12.3 Hz, 1H), 3.87 (d, J = 4.4 Hz, 2H), 3.43 (s, 4H), 3.29 (d, J = 6.7 Hz, 2H), 2.85 - 2.65 (m, 3H), 2.27 (s, 4H), 1.98 - 1.83 (m, 3H), 1.53 - 1.37 (m, 4H). | 676 |
| 280 | 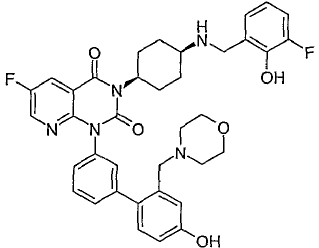 | 6-fluoro-3-{*cis*-4-[(3-fluoro-2-hydroxybenzyl)amino]cyclohexyl}-1-[4'-hydroxy-2'-(morpholin-4-ylmethyl)biphenyl-3-yl]pyrido[2,3-d]pyrimidine-2,4(1*H*,3*H*)-dione | ¹H NMR (400 MHz, DMSO) δ 8.59 (d, J = 3.1 Hz, 1H), 8.31 (dd, J = 7.7, 3.1 Hz, 1H), 7.60 - 7.49 (m, 2H), 7.42 (d, J = 7.7 Hz, 1H), 7.32 (d, J = 7.9 Hz, 1H), 7.13 (d, J = 8.2 Hz, 1H), 7.05 - 6.97 (m, 1H), 6.95 - 6.86 (m, 2H), 6.78 - 6.65 (m, 2H), 4.79 (t, J = 12.0 Hz, 1H), 3.90 (s, 2H), 3.43 (t, J = 4.0 Hz, 4H), 3.27 (d, J = 10.0 Hz, 2H), 2.77 (s, 1H), 2.73 - 2.57 (m, 2H), 2.28 (s, 4H), 1.92 (d, J = 12.6 Hz, 2H), 1.62 - 1.44 (m, 4H). | 670 |
| 281 | 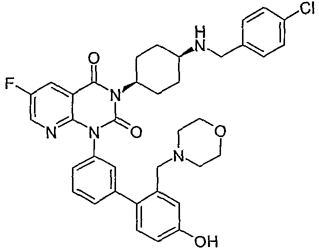 | 3-{*cis*-4-[(4-chlorobenzyl)amino]cyclohexyl}-6-fluoro-1-[4'-hydroxy-2'-(morpholin-4-ylmethyl)biphenyl-3-yl]pyrido[2,3-d]pyrimidine-2,4(1*H*,3*H*)-dione | ¹H NMR (400 MHz, DMSO) δ 9.49 (s, 1H), 8.58 (d, J = 2.3 Hz, 1H), 8.29 (dd, J = 7.7, 2.1 Hz, 1H), 7.59 - 7.50 (m, 2H), 7.45 - 7.28 (m, 6H), 7.13 (d, J = 8.2 Hz, 1H), 6.88 (s, 1H), 6.76 (dd, J = 8.3, 1.2 Hz, 1H), 4.77 (t, J = 12.2 Hz, 1H), 3.73 (s, 2H), 3.43 (s, 4H), 3.28 (s, 2H), 2.82 - 2.64 (m, 3H), 2.34 - 2.19 (m, 4H), 1.87 (d, J = 11.3 Hz, 2H), 1.56 - 1.38 (m, 4H). | 670 |
| 282 | 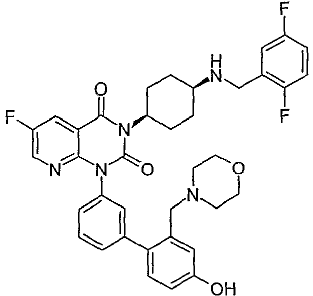 | 3-{*cis*-4-[(2,5-difluorobenzyl)amino]cyclohexyl}-6-fluoro-1-[4'-hydroxy-2'-(morpholin-4-ylmethyl)biphenyl-3-yl]pyrido[2,3-*d*]pyrimidine-2,4(1*H*,3*H*)-dione | ¹H NMR (400 MHz, DMSO) δ 9.48 (s, 1H), 8.59 (s, 1H), 8.29 (dd, J = 7.6, 2.9 Hz, 1H), 7.59 - 7.50 (m, 2H), 7.41 (d, J = 7.7 Hz, 2H), 7.31 (d, J = 7.7 Hz, 2H), 7.12 (d, J = 8.5 Hz, 2H), 6.88 (d, J = 2.6 Hz, 1H), 6.75 (dd, J = 8.2, 2.6 Hz, 1H), 4.85 - 4.72 (m, 1H), 3.73 (s, 2H), 3.45 (d, J = 11.3 Hz, 4H), 3.29 (d, J = 5.1 Hz, 2H), 2.74 (s, 3H), 2.27 (s, 4H), 1.85 (s, 2H), 1.43 (s, 4H). | 672 |
| 283 | 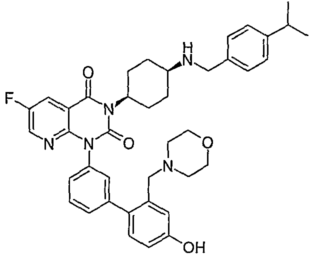 | 6-fluoro-1-[4'-hydroxy-2'-(morpholin-4-ylmethyl)biphenyl-3-yl]-3-{*cis*-4-[(4-isopropylbenzyl)amino]cyclohexyl}p yrido[2,3-*d*]pyrimidine-2,4(1*H*,3*H*)-dione | ¹H NMR (400 MHz, DMSO) δ 9.48 (s, 1H), 8.58 (d, J = 3.1 Hz, 1H), 8.29 (dd, J = 7.8, 2.9 Hz, 1H), 7.60 - 7.49 (m, 2H), 7.41 (d, J = 7.7 Hz, 1H), 7.33 - 7.24 (m, 3H), 7.18 - 7.09 (m, 3H), 6.88 (d, J = 2.6 Hz, 1H), 6.75 (dd, J = 8.3, 2.7 Hz, 1H), 4.83 - 4.71 (m, 1H), 3.65 (s, 2H), 3.43 (t, J = 4.1 Hz, 4H), 3.29 (d, J = 8.5 Hz, 2H), 2.90 - 2.66 (m, 4H), 2.27 (s, 4H), 1.87 (d, J = 12.0 Hz, 2H), 1.52 - 1.38 (m, 4H), 1.18 (d, J = 6.9 Hz, 6H). | 678 |
| 284 | 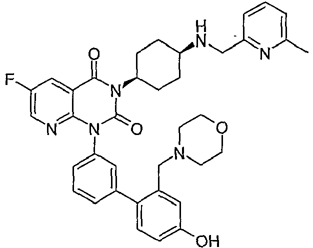 | 6-fluoro-1-[4'-hydroxy-2'-(morpholin-4-ylmethyl)biphenyl-3-yl]-3-(*cis*-4-{[(6-methylpyridin-2-yl)methyl]amino}cyclohexyl)pyrido[ 2,3-*d*]pyrimidine-2,4(1*H*,3*H*)-dione | ¹H NMR (400 MHz, DMSO) δ 8.58 (d, J = 3.1 Hz, 1H), 8.28 (dd, J = 7.7, 3.1 Hz, 1H), 7.64 - 7.48 (m, 3H), 7.41 (d, J = 7.9 Hz, 1H), 7.34 - 7.24 (m, 2H), 7.10 (dd, J = 20.1, 7.8 Hz, 2H), 6.87 (d, J = 2.6 Hz, 1H), 6.75 (dd, J = 8.2, 2.6 Hz, 1H), 4.77 (t, J = 12.0 Hz, 1H), 3.75 (d, J = 6.9 Hz, 2H), 3.42 (d, J = 4.4 Hz, 4H), 3.28 (s, 2H), 2.81 - 2.64 (m, 3H), 2.43 (s, 3H), 2.27 (s, 4H), 1.99 (d, J = 4.9 Hz, 1H), 1.86 (d, J = 12.3 Hz, 2H), 1.54 - 1.36 (m, 4H). | 651 |
| 285 | 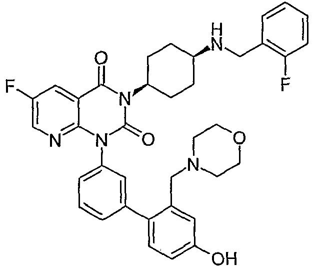 | 6-fluoro-3-{*cis*-4-[(2-fluorobenzyl)amino]cyclohexyl}-1-[4'-hydroxy-2'-(morpholin-4-ylmethyl)biphenyl-3-yl]pyrido[2,3-*d*]pyrimidine-2,4(1*H*,3*H*)-dione | ¹H NMR (400 MHz, DMSO) δ 9.56 - 9.41 (m, 1H), 8.58 (d, J = 2.8 Hz, 1H), 8.29 (dd, J = 7.8, 2.9 Hz, 1H), 7.59 - 7.48 (m, 3H), 7.45 - 7.38 (m, 1H), 7.33 - 7.23 (m, 2H), 7.19 - 7.09 (m, 3H), 6.88 (d, J = 2.6 Hz, 1H), 6.75 (dd, J = 8.5, 2.6 Hz, 1H), 4.77 (s, 1H), 3.74 (d, J = 6.4 Hz, 2H), 3.43 (t, J = 4.2 Hz, 4H), 3.28 (s, 2H), 2.73 (d, J = 16.1 Hz, 3H), 2.27 (s, 4H), 1.86 (d, J = 13.3 Hz, 2H), 1.54 - 1.35 (m, 4H). | 654 |

### Example 286

### 6-fluoro-3-{cis-4-[[(6-fluoroimidazo[1,2-a]pyridin-2-yl)methyl](methyl)amino]cyclohexyl}-1-[4'-hydroxy-2'-(morpholin-4-ylmethyl)biphenyl-3-yl]pyrido[2,3-d]pyrimidine-2,4(1H,3H)-dione

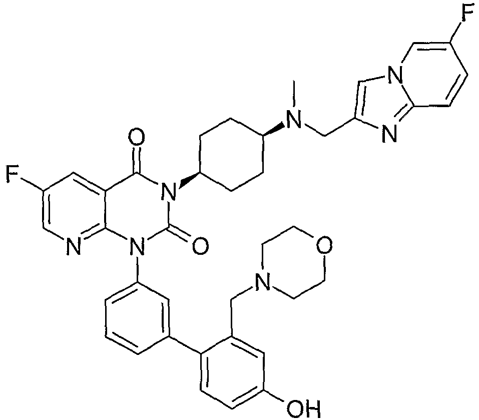

3-(*cis*-4-Aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (184 mg, 0.34 mmol) and 6-fluoroimidazo[1,2-a]pyridine-2-carbaldehyde (55.4 mg, 0.34 mmol) were dissolved in 1,2-dichloroethane (4 mL) and N-methyl-2-pyrrolidinone (1 mL) at 25 °C and the solution allowed to stir for 1 h. Sodium triacetoxyborohydride (117 mg, 0.55 mmol) was then added over a period of 10 mins under air. The resulting suspension was stirred at 25 °C for 16 h, then formaldehyde (41 mg, 0.51 mmol) was added and the suspension stirred a further 16 h. The crude suspension was then loaded on to a 10g SCX column, washed with methanol (100 mL) and eluted with 3.5N ammoniacal methanol (75 mL). The crude compound was obtained after concentration in vacuo and purified by preparative HPLC on a Phenomenex Gemini column using a 75-5% gradient of aqueous 0.2% ammonia in acetonitrile as eluent. The fractions containing the desired compound were evaporated to dryness to afford the title compound as a colourless solid (0.134 g).
¹H NMR (400 MHz, DMSO) δ 9.48 (s, 1H), 8.66 - 8.56 (m, 2H), 8.34 (dd, J = 7.8, 2.9 Hz, 1H), 7.91 (s, 1H), 7.61 - 7.50 (m, 3H), 7.43 (d, J = 7.9 Hz, 1H), 7.35 - 7.29 (m, 1H), 7.29 - 7.20 (m, 1H), 7.14 (d, J = 8.5 Hz, 1H), 6.88 (d, J = 2.6 Hz, 1H), 6.76 (dd, J = 8.2,2.6 Hz, 1H), 4.91 - 4.81 (m, 1H), 3.81 (s, 2H), 3.43 (t, J = 4.1 Hz, 4H), 3.29 (s, 2H), 2.80 (dd, J = 24.9, 11.5 Hz, 2H), 2.42 - 2.35 (m, 1H), 2.31 - 2.15 (m, 9H), 1.42 (s, 4H).
[M+H]+=708 (Multimode+)

### Example 287

### N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-N²,N²-dimethylglycinamide

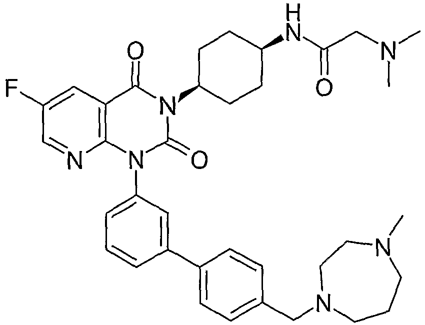

### Step (a) tert-butyl {cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}carbamate

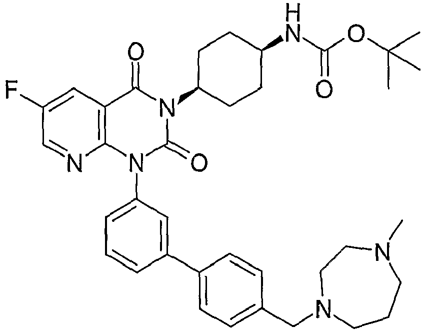

1-Methyl-1,4-diazepane (0.7 mL, 5.63 mmol) was added to a solution of *tert*-butyl-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}carbamate (2.009 g, 3.60 mmol) in DCM (40 mL). Anhydrous sodium sulphate (∼0.5 g) was added. The mixture was stirred at room temperature for 50 mins before sodium triacetoxyborohydride (1.143 g, 5.39 mmol) was added and the mixture stirred at room temperature overnight. The reaction mixture was diluted with DCM, washed with saturated sodium hydrogen carbonate, water and saturated brine, dried over sodium sulfate, filtered and concentrated. The crude material was dissolved in methanol and loaded onto a pre-washed SCX cartridge. Neutral impurities were removed by washing with methanol, then the product was eluted with 10-20% 7N ammonia in methanol in methanol to afford the sub-title compound as a pale yellow foam (1.66 g).
[M+H]+ = 657

### Step (b) 3-(cis-4-aminocyclohexyl)-6-fluoro-1-{4'-[(4-methyl-1,4-diazepan-1-yl)methyl]biphenyl-3-yl}pyrido[2,3-d]pyrimidine-2,4(1H,3H)-dione

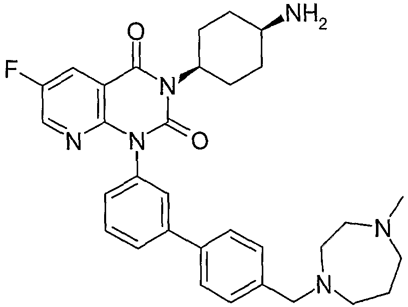

*tert*-butyl {*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}carbamate (1.661 g, 2.53 mmol) was dissolved in 1,4-dioxane (10 ml) and cooled in ice. Hydrogen chloride (∼4M in 1,4-dioxane) (12.64 ml, 50.58 mmol) was added and the reaction mixture stirred at ∼5°C for 1 h then at room temperature for 4 h before being concentrated in vacuo to afford the sub-title compound trihydrochloride salt as a white solid (1.985 g).
[M+H]+=557 (Multimode+)

### Step (c) N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl]-N²,N²-dimethylglycinamide

A solution of 3-(*cis*-4-aminocyclohexyl)-6-fluoro-1-{4'-[(4-methyl-1,4-diazepan-1-yl)methyl]biphenyl-3-yl}pyrido[2,3-d]pyrimidine-2,4(1*H*,3*H*)-dione (0.243 g, 0.36 mmol), *N,N*-dimethylglycine (0.040 g, 0.38 mmol) and triethylamine (0.55 ml, 3.96 mmol) in acetonitrile (10 ml) was stirred for 30 mins then 1-propanephosphonic acid cyclic anhydride (T3P, 1.57M in THF) (0.25 ml, 0.39 mmol) added dropwise. The reaction mixture was stirred overnight then poured onto saturated sodium hydrogen carbonate (10ml) and extracted with ethyl acetate (x 3). The combined organic extracts were washed with water, concentrated and purified by preparative HPLC on a Waters X-Bridge column using a 95-5% gradient of aqueous 0.2% trifluoroacetic acid in acetonitrile as eluent. The fractions containing the desired compound were evaporated to dryness to afford the title compound ditrifluoroacetate as a white foam (0.212 g).
¹H NMR (400 MHz, DMSO) δ 9.66 (br s, 1H), 8.59 (d, J = 3.1 Hz, 1H), 8.45 (d, J = 5.6 Hz, 1H), 8.30 (dd, J = 7.7, 3.1 Hz, 1H), 7.83 - 7.74 (m, 4H), 7.66 - 7.58 (m, 3H), 7.41 (d, J = 7.7 Hz, 1H), 4.79 (t, J = 12.0 Hz, 1H), 4.34 - 4.28 (m, 1H), 3.96 - 3.90 (m, 2H), 2.84 (s, 3H), 2.80 (s, 6H), 2.67 - 2.58 (m, 2H), 2.14 - 2.07 (m, 2H), 1.90 (d, J = 12.5 Hz, 2H), 1.67 - 1.55 (m, 4H). Remaining protons obscured by solvent peaks.
[M+H]+=642 (Multimode+)

### Example 288

### N-{cis-4-[1-{4'-[(4-cyclopentylpiperazin-1-yl)methyl]biphenyl-3-yl}-6-fluoro-2,4-dioxo-1,4-dihydropyrido[2,3-d]pyrimidin-3(2H)-yl]cyclohexyl}-5-methylpyrazine-2-carboxamide

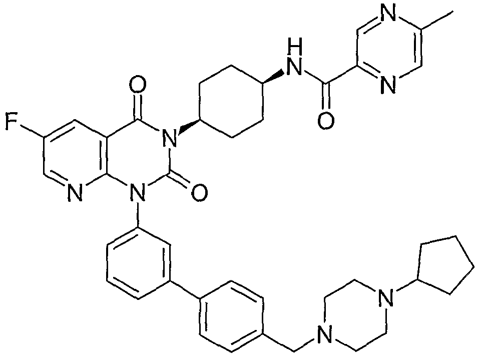

### Step (a) tert-butyl{cis-4-[1-{4'-[(4-cyclopentylpiperazin-1-yl)methyl]biphenyl-3-yl}-6-fluoro-2,4-dioxo-1,4-dihydropyrido[2,3-d]pyrimidin-3(2H)-yl]cyclohexyl}carbamate

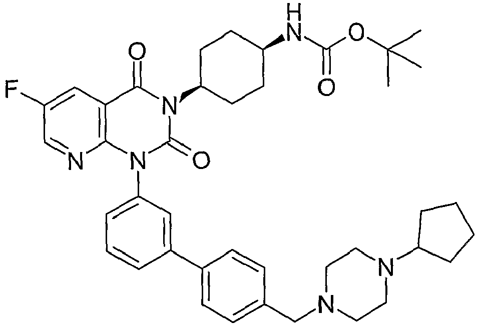

The product was prepared from *tert*-butyl-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}carbamate (2.074 g, 3.71 mmol) and 1-cyclopentylpiperazine (0.859 g, 5.57 mmol) by the method of example 287 step (a) to give the sub-title compound as a pale yellow foam (2.393 g).
[M+H]+=697

### Step (b) 3-(cis-4-aminocyclohexyl)-1-{4'-[(4-cyclopentylpiperazin-1-yl)methyl]biphenyl-3-yl}-6-fluoropyrido[2,3-d]pyrimidine-2,4(1H,3H)-dione

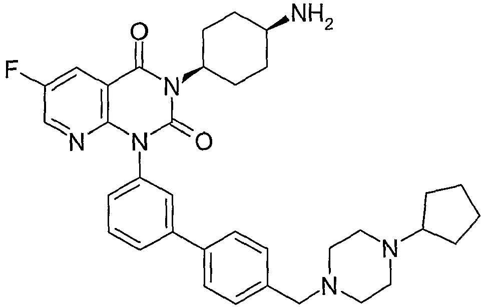

The product was prepared from *tert*-butyl {*cis*-4-[1-{4'-[(4-cyclopentylpiperazin-1-yl)methyl]biphenyl-3-yl}-6-fluoro-2,4-dioxo-1,4-dihydropyrido[2,3-*d*]pyrimidin-3(2*H*)-yl]cyclohexyl}carbamate (2.393 g, 3.43 mmol) by the method of example 287 step (b) to give the sub-title compound trihydrochloride salt as a pale yellow solid (2.998 g).
[M+H]+=597

### Step (c) N-{cis-4-[1-{4'-[(4-cyclopentylpiperazin-1-yl)methyl]biphenyl-3-yl}-6-fluoro-2,4-dioxo-1,4-dihydropyrido[2,3-d]pyrimidin-3(2H)-yl]-cyclohexyl}-5-methylpyrazine-2-carboxamide

The product was prepared from 3-(*cis*-4-aminocyclohexyl)-1-{4'-[(4-cyclopentylpiperazin-1-yl)methyl]biphenyl-3-yl}-6-fluoropyrido[2,3-*d*]pyrimidine-2,4(1*H*,3*H*)-dione (0.296 g, 0.42 mmol) and 5-methylpyrazine-2-carboxylic acid (0.069 g, 0.50 mmol) by the method of example 287 step (c) to give the title compound ditrifluoroacetate salt as a white foam (95 mg).
¹H NMR (400 MHz, DMSO) δ 9.07 (s, 1H), 8.64 - 8.59 (m, 2H), 8.33 (dd, J = 7.7, 3.1 Hz, 1 H), 8.18 (d, J = 7.2 Hz, 1H), 7.82 - 7.72 (m, 3 H), 7.64 (t, J = 7.8 Hz, 1H), 7.52 (d, J = 7.9 Hz, 2H), 7.41 (d, J = 7.9 Hz, 1H), 4.89 (t, J = 12.2 Hz, 1H), 4.21 - 4.17 (m, 1H), 3.56 - 3.49 (m, 2H), 3.30 - 3.08 (m, 4H), 2.67 - 2.54 (m, 5H), 2.04 - 1.97 (m, 4H), 1.77 - 1.54 (m, 10H). Remaining protons obscured by solvent peaks.
[M+H]+=717 (Multimode+)

### Example 289

### N-{cis-4-[1-[4'-(1,4'-bipiperidin-1'-ylmethyl)biphenyl-3-yl]-6-fluoro-2,4-dioxo-1,4-dihydropyrido[2,3-d]pyrimidin-3(2H)-yl]cyclohexyl}-1,5-dimethyl-1H-pyrazole-3-carboxamide

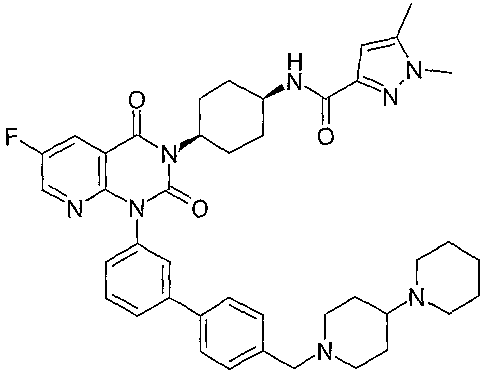

### Step (a) tert-butyl {cis-4-[1-[4'-(1,4'-bipiperidin-1'-ylmethyl)biphenyl-3-yl]-6-fluoro-2,4-dioxo-1,4-dihydropyrido[2,3-d]pyrimidin-3(2H)-yl]cyclohexyl}-carbamate

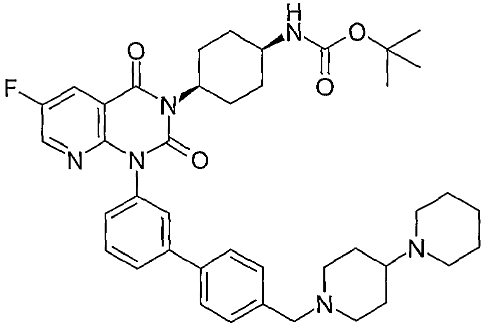

The product was prepared from *tert*-butyl-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}carbamate (1.500 g, 2.69 mmol) and 1,4'-bipiperidine (0.565 g, 3.36 mmol) by the method of example 287 step (a) to give the sub-title compound as a pale yellow foam (1.585 g).
[M+H]+=711

### Step (b) 3-(cis-4-aminocyclohexyl)-1-[4'-(1,4'-bipiperidin-1'-ylmethyl)biphenyl-3-yl]-6-fluoropyrido[2,3-d]pyrimidine-2,4(1H,3H)-dione

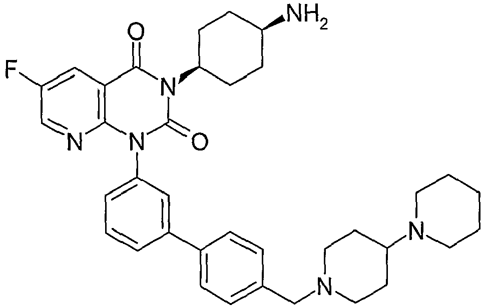

The product was prepared from *tert*-butyl {*cis*-4-[1-[4'-(1,4'-bipiperidin-1'-ylmethyl)biphenyl-3-yl]-6-fluoro-2,4-dioxo-1,4-dihydropyrido[2,3-*d*]pyrimidin-3(2*H*)-yl]cyclohexyl}carbamate (1.585 g, 2.23 mmol) by the method of example 287 step (b) to give the sub-title compound trihydrochloride salt as a cream-coloured solid (1.944 g).
[M+H]+=611

### Step (c) N-{cis-4-[1-[4'-(1,4'-bipiperidin-1'-ylmethyl)biphenyl-3-yl]-6-fluoro-2,4-dioxo-1,4-dihydropyrido[2,3-d]pyrimidin-3(2H)-yl]cyclohexyl}-1,5-dimethyl-1H-pyrazole-3-carboxamide

The product was prepared from 3-(*cis*-4-aminocyclohexyl)-1-[4'-(1,4'-bipiperidin-1'-ylmethyl)biphenyl-3-yl]-6-fluoropyrido[2,3-*d*]pyrimidine-2,4(1*H*,3*H*)-dione (0.221 g, 0.31 mmol) and 1,5-dimethyl-1H-pyrazole-3-carboxylic acid (0.052 g, 0.37 mmol) by the method of example 287 step (c) to give the title compound ditrifluoroacetate salt as a white foam (0.164 g).
¹H NMR (400 MHz, DMSO) δ 8.5 (d, J = 3.1 Hz, 1H), 8.34 (dd, J = 7.8, 2.9 Hz, 1H), 7.84 - 7.77 (m, 4H), 7.65 (t, J = 7.8 Hz, 1H), 7.60 (d, J = 7.4 Hz, 2H), 7.43 (d, J = 8.5 Hz, 1H), 7.20 (d, J = 6.9 Hz, 1H), 6.42 (s, 1H), 4.88 - 4.81 (m, 1H), 4.37 - 4.32 (m, 2H), 4.10 - 4.07 (m, 1H), 3.75 (s, 3H), 3.02 - 2.88 (m, 4H), 2.57 - 2.51 (m, 2H), 2.25 - 2.21 (m, 5H), 1.98 - 1.83 (m, 6H), 1.72 - 1.60 (m, 6H), 1.44 - 1.27 (m, 2H). Remaining protons obscured by solvent peaks.
[M+H]+=733 (Multimode+)

### Example 290

### N-{cis-4-[1-(4'-{[(3R,5S)-3,5-dimethylpiperazin-1-yl]methyl}biphenyl-3-yl)-6-fluoro-2,4-dioxo-1,4-dihydropyrido[2,3-d]pyrimidin-3(2H)-yl]cyclohexyl}-6-methylpyridine-2-carboxamide

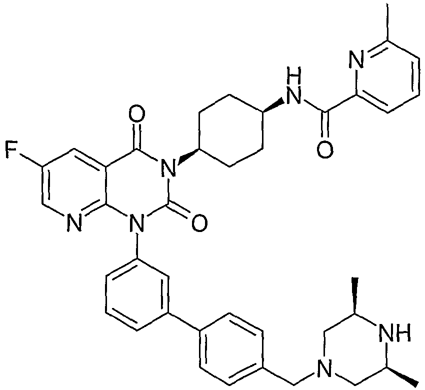

### Step (a) tert-butyl {cis-4-[1-(4'-{[(3R,5S)-3,5-dimethylpiperazin-1-yl]methyl)biphenyl-3-yl)-6-fluoro-2,4-dioxo-1,4-dihydropyrido[2,3-d]pyrimidin-3(2H)-yl]cyclohexyl}carbamate

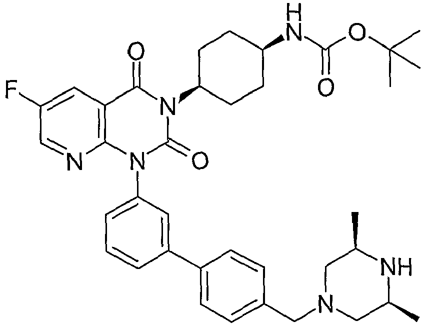

The product was prepared from *tert*-butyl-{*cis*-4-[6-fluoro-1-(4'-formylbiphenyl-3-yl)-2,4-dioxo-1,4-dihydropyrido[2,3-*d*]pyrimidin-3(2*H*)-yl]cyclohexyl}carbamate (2.000 g, 3.58 mmol) and cis-2,6-dimethylpiperazine (0.409 g, 3.58 mmol) by the method of example 287 step (a) to give the sub-title compound as a foam (1.92 g).
[M+H]+=657 (Multimode+)

### Step (b) 3-(cis-4-aminocyclohexyl)-1-(4'-{[(3R,5S)-3,5-dimethylpiperazin-1-yl]methyl}biphenyl-3-yl)-6-fluoropyrido[2,3-d]pyrimidine-2,4(1H,3H)-dione

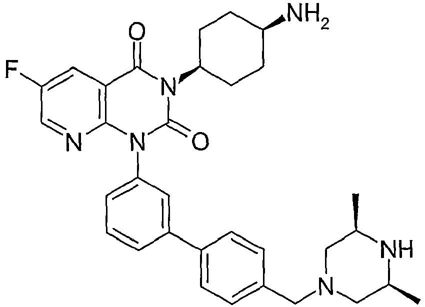

The product was prepared from *tert*-butyl {*cis*-4-[1-(4'-{[(3*R*,5*S*)-3,5-dimethylpiperazin-1-yl]methyl}biphenyl-3-yl)-6-fluoro-2,4-dioxo-1,4-dihydropyrido[2,3-*d*]pyrimidin-3(2*H*)-yl]cyclohexyl}carbamate (1.88 g, 2.86 mmol) by the method of example 287 step (b) to give the sub-title compound trihydrochloride salt as a white solid (2.05 g).
[M+H]+=557

### Step (c) N-{cis-4-[1-(4'-{[(3R,5S)-3,5-dimethylpiperazin-1-yl]methyl}biphenyl-3-yl)-6-fluoro-2,4-dioxo-1,4-dihydropyrido[2,3-d]pyrimidin-3(2H)-yl]cyclohexyl}-6-methylpyridine-2-carboxamide

The product was prepared from 3-(*cis*-4-aminocyclohexyl)-1-(4'-{[(3*R*,5*S*)-3,5-dimethylpiperazin-1-yl]methyl}biphenyl-3-yl)-6-fluoropyrido[2,3-*d*]pyrimidine-2,4(1*H*,3*H*)-dione (0.200 g, 0.34 mmol) and 6-methylpicolinic acid (46 mg, 0.34 mmol) by the method of example 287 step (c) to give the title compound ditrifluoroacetate salt as a white solid (98 mg).
¹H NMR (400 MHz, CDCl₃) δ 8.77 (d, J = 8.7 Hz, 1H), 8.37 (d, J = 3.1 Hz, 1H), 8.23 (dd, J = 7.2, 3.1 Hz, 1H), 7.95 (d, J = 7.7 Hz, 1H), 7.75 - 7.63 (m, 5H), 7.53 (t, J = 1.8 Hz, 1H), 7.47 (d, J = 8.2 Hz, 2H), 7.34 (ddt, J = 7.8, 0.2, 1.3 Hz, 1H), 7.26 - 7.22 (m, 1H), 5.13 - 5.03 (m, 1H), 4.43 - 4.39 (m, 1H), 4.05 (s, 2H), 3.71 - 3.65 (m, 2H), 3.24 (d, J = 10.3 Hz, 2H), 3.05 (t, J = 12.0 Hz, 2H), 2.84 - 2.74 (m, 2H), 2.49 (s, 3H), 2.07 (d, J = 13.3 Hz, 2H), 1.82 - 1.71 (m, 4H), 1.32 (d, J = 6.4 Hz, 6H).
[M+H]+=676 (Multimode+)

The following compounds (Table 9) were prepared in a similar manner as solids from the appropriate amine and acid using the method described above in example 287 step (c).

**Table 9**

| **Example Number** | **Chemistry** | **Name** | **NMR** | **M+H** |
|---|---|---|---|---|
| 291 | 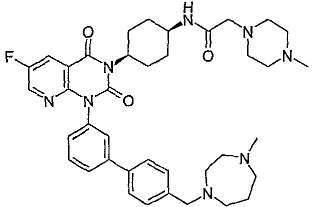 | *N*-{*cis*-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2*H*)-yl]cyclohexyl}-2-(4-methylpiperazin-1-yl)acetamide | ¹H NMR (400 MHz, DMSO) δ 8.60 (d, J = 2.8 Hz, 1H), 8.34 (dd, J = 7.7, 3.1 Hz, 1H), 7.84 - 7.74 (m, 5H), 7.67 - 7.60 (m, 3H), 7.43 (d, J = 7.7 Hz, 1H), 4.86 (t, J = 12.0 Hz, 1H), 4.39 - 4.34 (m, 2H), 3.98 - 3.92 (m, 2H), 3.37 - 2.98 (m, 8H), 2.84 (s, 3H), 2.59 - 2.53 (m, 2H), 2.17 - 2.11 (m, 2H), 1.86 (d, J = 12.3 Hz, 2H), 1.65 - 1.57 (m, 4H). Remaining protons obscured by solvent peaks. | 697 |
| 292 | 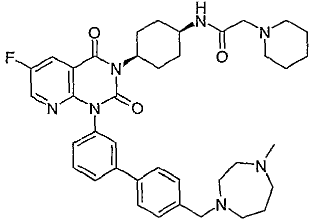 | *N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2H)-yl]cyclohexyl}-2-piperidin-1-ylacetamide | ¹H NMR (400 MHz, DMSO) δ 9.54 (br s, 1H), 8.59 (d, J = 3.1 Hz, 1H), 8.44 (d, J = 5.4 Hz, 1H), 8.30 (dd, J = 7.7, 3.1 Hz, 1H), 7.83 - 7.76 (m, 4H), 7.66 - 7.59 (m, 3*H*), 7.41 (d, J = 7.7 Hz, 1H), 4.80 (t, J = 11.5 Hz, 1H), 4.35 - 4.30 (m, 2H), 3.94 - 3.89 (m, 4H), 3.30 - 2.92 (m, 6H), 2.84 (s, 3H), 2.67 - 2.57 (m, 2H), 2.15 - 2.10 (m, 2H), 1.90 (d, J = 12.6 Hz, 2H), 1.77 - 1.34 (m, 10H). Remaining protons obscured by solvent peaks. | 682 |
| 293 | 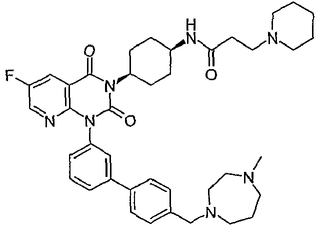 | *N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}-3-piperidin-1-ylpropanamide | ¹H NMR (400 MHz, DMSO) δ 9.20 (br s, 1H), 8.59 (d, J = 3.1 Hz, 1H), 8.30 (dd, J = 7.7, 3.1 Hz, 1H), 7.98 (d, J = 5.1 Hz, 1H), 7.82 (d, J = 7.9 Hz, 2H), 7.78 - 7.75 (m, 3H), 7.66 - 7.58 (m, 3H), 7.41 (d, J = 8.2 Hz, 1H), 4.79 (t, J = 11.8 Hz, 1H), 4.34 - 4.28 (m, 1H), 3.85 - 3.81 (m, 1H), 3.29 - 3.24 (m, 2H), 2.90 - 2.81 (m, 5H), 2.67 - 2.58 (m, 4H), 2.14 - 2.09 (m, 2H), 1.92 (d, J = 11.5 Hz, 2H), 1.78 (d, J = 14.1 Hz, 2H), 1.65 - 1.51 (m, 8H). Remaining protons obscured by solvent peaks. | 696 |
| 294 | 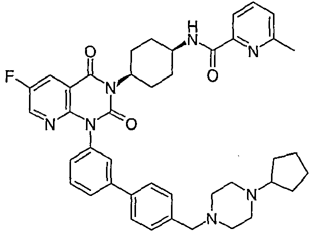 | *N*-{*cis*-4-[1-{4'-[(4-cyclopentylpiperazin-1-yl)methyl]biphenyl-3-yl}-6-fluoro-2,4-dioxo-1,4-dihydropyrido[2,3-*d*]pyrimidin-3(2*H*)-yl]cyclohexyl}-6-methylpyridine-2-carboxamide | ¹H NMR (400 MHz, DMSO) δ 8.63 - 8.59 (m, 2H), 8.32 (dd, J = 7.7, 2.8 Hz, 1H), 7.89 - 7.70 (m, 6H), 7.63 (t, J = 7.8 Hz, 1H), 7.51 - 7.40 (m, 4H), 4.93 (t, J = 11.5 Hz, 1H), 4.23 - 4.19 (m, 1H), 3.95 - 3.89 (m, 2H), 3.19 - 3.04 (m, 4H), 2.68 - 2.58 (m, 2H), 2.43 (s, 3H), 2.00 - 1.90 (m, 4H), 1.76 - 1.53 (m, 10H). Remaining protons obscured by solvent peaks. | 716 |
| 295 | 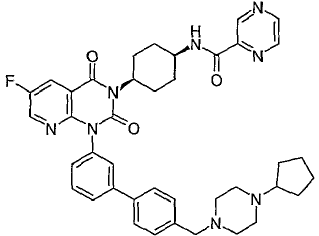 | *N*-{*cis*-4-[1-{4'-[(4-cyclopentylpiperazin-1-yl)methyl]biphenyl-3-yl}-6-fluoro-2,4-dioxo-1,4-dihydropyrido[2,3-*d*]pyrimidin-3(2*H*)-yl]cyclohexyl}pyrazine-2-carboxamide | ¹H NMR (400 MHz, DMSO) δ 9.21 (s, 1H), 8.89 (d, J = 2.3 Hz, 1H), 8.77 - 8.76 (m, 1H), 8.59 (d, J = 2.8 Hz, 1H), 8.34 (dd, J = 7.8, 2.9 Hz, 1H), 8.25 (d, J = 7.2 Hz, 1H), 7.79 (d, J = 8.5 Hz, 1H), 7.73 - 7.68 (m, 3H), 7.62 (t, J = 7.7 Hz, 1H), 7.46 - 7.45 (m, 2H), 7.39 (d, J = 7.7 Hz, 1H), 4.88 (t, J = 12.2 Hz, 1H), 4.21 - 4.19 (m, 1H), 3.81 - 3.74 (m, 1H), 3.10 - 2.97 (m, 4H), 2.67 - 2.56 (m, 2H), 2.04 - 1.95 (m, 4H), 1.78 - 1.52 (m, 10H). Remaining protons obscured by solvent peaks. | 703 |
| 296 | 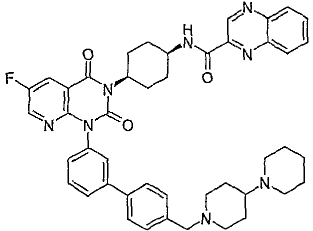 | *N*-{*cis*-4-[1-[4'-(1,4'-bipiperidin-1'-ylmethyl)biphenyl-3-yl]-6-fluoro-2,4-dioxo-1,4-dihydropyrido[2,3-*d*]pyrimidin-3(2*H*)-yl]cyclohexyl}quinoxaline-2-carboxamide | ¹H NMR (400 MHz, DMSO) δ 9.48 (s, 1H), 8.63 (d, J = 7.9 Hz, 1H), 8.61 (d, J = 3.1 Hz, 1H), 8.37 (dd, J = 7.7, 3.1 Hz, 1H), 8.19 (d, J = 8.2 Hz, 1H), 7.99 - 7.81 (m, 7H), 7.70 (t, J = 7.8 Hz, 1H), 7.58 (d, J = 7.7 Hz, 2H), 7.48 (d, J = 8.2 Hz, 1H), 5.02 - 4.95 (m, 1H), 4.36 - 4.28 (m, 2H), 3.01 - 2.87 (m, 4H), 2.76 - 2.67 (m, 2H), 2.25 - 2.17 (m, 2H), 2.03 - 2.00 (m, 2H), 1.90 - 1.64 (m, 12H), 1.45 - 1.24 (m, 2H). Remaining protons obscured by solvent peaks. | 767 |
| 297 | 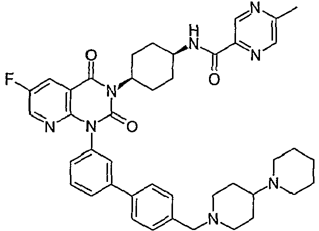 | *N*-{*cis*-4-[1-[4'-(1,4'-bipiperidin-1'-ylmethyl)biphenyl-3-yl]-6-fluoro-2,4-dioxo-1,4-dihydropyrido[2,3-*d*]pyrimidin-3(2*H*)-yl]cyclohexyl}-5-methylpyrazine-2-carboxamide | ¹H NMR (400 MHz, DMSO) δ 9.07 (s, 1H), 8.64 (s, 1H), 8.59 (d, J = 3.1 Hz, 1H), 8.35 (dd, J = 7.8,2.9 Hz, 1H), 8.18 (d, J = 7.2 Hz, 1H), 7.85 - 7.78 (m, 4H), 7.65 (t, J = 7.9 Hz, 1H), 7.59 (d, J = 6.9 Hz, 2H), 7.43 (d, J = 7.9 Hz, 1H), 4.93 - 4.83 (m, 1H), 4.37 - 4.31 (m, 2H), 4.21 - 4.17 (m, 1H), 3.02 - 2.86 (m, 4H), 2.67 - 2.55 (m, 5H), 2.26 - 2.18 (m, 2H), 2.03 - 1.99 (m, 2H), 1.90 - 1.64 (m, 12H), 1.48 - 1.24 (m, 2H). Remaining protons obscured by solvent peaks. | 731 |
| 298 | 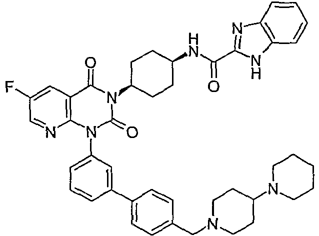 | *N*-{*cis*-4-[1-[4'-(1,4'-bipiperidin-1'-ylmethyl)biphenyl-3-yl]-6-fluoro-2,4-dioxo-1,4-dihydropyrido[2,3-*d*]pyrimidin-3(2*H*)-yl]cyclohexyl}-1*H*-benzimidazole-2-carboxamide | ¹H NMR (400 MHz, DMSO) δ 8.59 (d, J = 3.1 Hz, 1H), 8.36 (dd, J = 7.9, 2.4 Hz, 1H), 8.03 (d, J = 6.9 Hz, 1H), 7.84 - 7.78 (m, 4H), 7.68 - 7.58 (m, 5H), 7.44 (d, J = 7.7 Hz, 1H), 7.30 - 7.28 (m, 2H), 4.92 - 4.86 (m, 1H), 4.38 - 4.32 (m, 2H), 4.22 - 4.18 (m, 1H), 3.01 - 2.87 (m, 4H), 2.70 - 2.62 (m, 2H), 2.25 - 2.18 (m, 2H), 2.07 (d, J = 10.4 Hz, 2H), 1.90 - 1.60 (m, 12H), 1.46 - 1.35 (m, 2H). Remaining protons obscured by solvent peaks. | 755 |
| 299 | 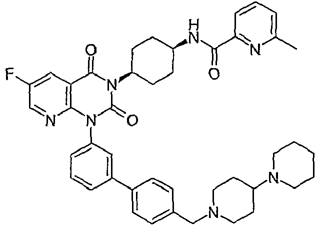 | *N*-{*cis*-4-[1-[4'-(1,4'-bipiperidin-1'-ylmethyl)biphenyl-3-yl]-6-fluoro-2,4-dioxo-1,4-dihydropyrido[2,3-*d*]pyrimidin-3(2*H*)-yl]cyclohexyl}-6-methylpyridine-2-carboxamide | ¹H NMR (400 MHz, DMSO) δ 8.62 (d, J = 7.8 Hz, 1H), 8.59 (d, J = 3.1 Hz, 1H), 8.31 (dd, J = 7.7, 3.1 Hz, 1H), 7.89 - 7.82 (m, 2H), 7.77 (d, J = 7.7 Hz, 1H), 7.72 - 7.71 (m, 1H), 7.63 - 7.58 (m, 3H), 7.44 (d, J = 8.2 Hz, 1H), 7.38 (d, J = 8.2 Hz, 3H), 4.97 - 4.89 (m, 1H), 4.23 - 4.18 (m, 1H), 3.50 - 3.46 (m, 2H), 2.90 - 2.83 (m, 2H), 2.67 - 2.58 (m, 4H), 2.44 - 2.40 (m, 3H), 1.94 - 1.88 (m, 4H), 1.77 - 1.66 (m, 6H), 1.52 - 1.35 (m, 6H). Remaining protons obscured by solvent peaks. | 730 |
| 300 | 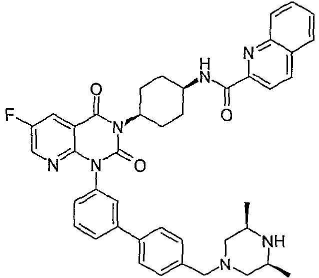 | *N*-{*cis*-4-[1-(4'-{[(3*R*,5*S*-3,5-dimethylpiperazin-1-yl]methyl}biphenyl-3-yl)-6-fluoro-2,4-dioxo-1,4-dihydropyrido[2,3-*d*]pyrimidin-3(2*H*)-yl]cyclohexyl}quinoline-2-carboxamide | ¹H NMR (400 MHz, CDCl₃) δ 8.97 (d, J = 8.7 Hz, 1H), 8.38 (d, J = 3.1 Hz, 1H), 8.30 - 8.24 (m, 3H), 8.04 (s, 1H), 7.85 (d, J = 8.2 Hz, 1H), 7.79 (d, J = 7.9 Hz, 1H), 7.72 - 7.55 (m, 6H), 7.45 (d, J = 8.2 Hz, 2H), 7.38 (d, J = 7.7 Hz, 1H), 5.17 - 5.09 (m, 1H), 4.50 (d, J = 8.7 Hz, 1H), 4.03 (s, 2H), 3.69 - 3.62 (m, 2H), 3.22 (d, J = 11.0 Hz, 2H), 3.01 (t, J = 11.9 Hz, 2H), 2.93 - 2.83 (m, 2H), 2.12 (d, J = 12.1 Hz, 2H), 1.87 - 1.75 (m, 4H), 1.29 (d, J = 6.4 Hz, 6H). | 712 |
| 301 | 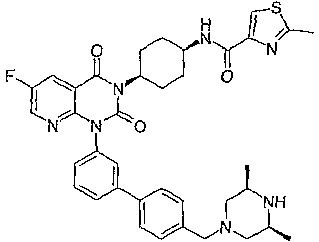 | *N*-{*cis*-4-[1-(4'-{[(3*R*,5*S*)-3,5-dimethylpiperazin-1-yl]methyl} biphenyl-3-yl)-6-fluoro-2,4-dioxo-1,4-dihydropyrido[2,3-*d*]pyrimidin-3(2*H*)-yl]cyclohexyl}-2-methyl-1,3-thiazole-4-carboxamide | ¹H NMR (400 MHz, CDCl₃) δ 8.37 (d, J = 3.1 Hz, 1H), 8.23 (dd, J = 7.2, 3.1 Hz, 1H), 7.91 (s, 1H), 7.76 - 7.72 (m, 2H), 7.69 - 7.63 (m, 3H), 7.53 (t, J = 1.8 Hz, 1H), 7.46 (d, J = 8.2 Hz, 2H), 7.34 (d, J = 7.7 Hz, 1H), 5.04 (t, J = 12.2 Hz, 1H), 4.37 (s, 1H), 4.02 (s, 2H), 3.71 - 3.62 (m, 2H), 3.22 (d, J = 10.8 Hz, 2H), 3.01 (t, J = 11.7 Hz, 2H), 2.78 - 2.69 (m, 2H), 2.68 (s, 3H), 2.09 (d, J = 13.8 Hz, 2H), 1.84 - 1.68 (m, 4H), 1.32 (d, J = 6.7 Hz, 6H). | 682 |
| 302 | 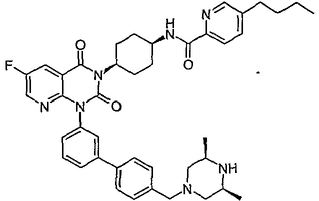 | 5-butyl-*N*-{*cis*-4-[1-(4'-{[(3*R*,5*S-*3,5-dimethylpiperazin-1-yl]methyl}biphenyl-3-yl)-6-fluoro-2,4-dioxo-1,4-dihydropyrido[2,3-*d*]pyrimidin-3(2*H*)-yl]cyclohexyl}pyridine-2-carboxamide | ¹H NMR (400 MHz, CDCl₃) δ 8.45 (d, J = . 7.9 Hz, 1H), 8.40 (s, 1H), 8.37 (d, J = 3.1 Hz, 1H), 8.24 (dd, J = 7.2, 3.1 Hz, 1H), 8.07 (d, J = 7.9 Hz, 1H), 7.74 (d, J = 7.9 Hz, 1H), 7.69 - 7.60 (m, 4H), 7.53 (t, J = 1.8 Hz, 1H), 7.46 (d, J = 8.2 Hz, 2H), 7.34 (d, J = 8.5 Hz, 1H), 5.08 - 5.00 (m, 1H), 4.41 - 4.37 (m, 1H), 4.00 (s, 2H), 3.68 - 3.60 (m, 2H), 3.21 (d, J = 11.0 Hz, 2H), 3.01 - 2.93 (m, 2H), 2.82 - 2.71 (m, 2H), 2.66 (t, J = 7.7 Hz, 2H), 2.11 (d, J = 13.3 Hz, 2H), 1.86 - 1.70 (m, 4H), 1.61 (quintet, J = 7.6 Hz, 2H), 1.40 - 1.29 (m, 8H), 0.93 (t, J = 7.3 Hz, 3H). | 718 |
| 303 | 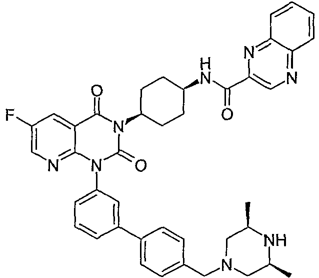 | *N*-{*cis*-4-[1-(4'-{[(3*R*,5*S*)-3,5-dimethylpiperazin-1-yl]methyl}biphenyl-3-yl)-6-fluoro-2,4-dioxo-1,4-dihydropyrido[2,3-*d*]pyrimidin-3(2*H*)-yl]cyclohexyl}quinoxaline-2-carboxamide | ¹H NMR (400 MHz, CDCl₃) δ 9.65 (s, 1H), 8.60 (d, J = 8.5 Hz, 1H), 8.38 (d, J = 3.1 Hz, 1H), 8.25 (dd, J = 7.2, 2.8 Hz, 1H), 8.16 (d, J = 8.5 Hz, 1H), 8.03 (s, 1H), 7.86 - 7.65 (m, 6H), 7.56 (d, J = 1.8 Hz, 1H), 7.47 (d, J = 8.2 Hz, 2H), 7.37 (d, J = 7.7 Hz, 1H), 5.13 (t, J = 12.8 Hz, 1H), 4.53 - 4.49 (m, 1H), 4.04 (s, 2H), 3.71 - 3.64 (m, 2H), 3.22 (d, J = 11.0 Hz, 2H), 3.03 (t, J = 12.0 Hz, 2H), 2.90 - 2.78 (m, 2H), 2.13 (d, J= 13.1 Hz, 2H), 1.89 - 1.76 (m, 4H), 1.31 (d, J = 6.7 Hz, 6H). | 713 |
| 304 | 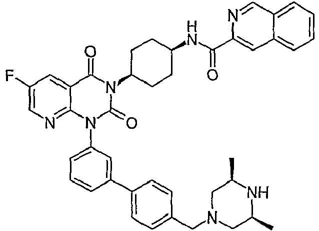 | *N*-{*cis*-4-[1-(4'-{[(3*R*,5*S*)-3,5-dimethylpiperazin-1-yl]methyl}biphenyl-3-yl)-6-fluoro-2,4-dioxo-1,4-dihydropyrido[2,3-*d*]pyrimidin-3(2*H*)-yl]cyclohexyl} isoquinoline-3-carboxamide | ¹H NMR (400 MHz, CDCl₃) δ 9.19 (s, 1H), 8.72 (d, J = 7.9 Hz, 1H), 8.59 (s, 1H), 8.37 (d, J = 3.1 Hz, 1H), 8.25 (dd, J = 7.3, 2.9 Hz, 1H), 8.02 (d, J = 8.2 Hz, 1H), 7.97 (d, J = 7.9 Hz, 1H), 7.78 - 7.65 (m, 5H), 7.54 (t, J = 1.7 Hz, 2H), 7.47 (d, J = 7.9 Hz, 2H), 7.35 (d, J = 8.5 Hz, 1H), 5.07 (t, J = 12.0 Hz, 1H), 4.46 (s, 1H), 4.08 (s, 2H), 3.74 - 3.65 (m, 2H), 3.26 (d, J = 11.0 Hz, 2H), 3.08 (t, J = 12.0 Hz, 2H), 2.81 (dd, J = 23.1, 12.6 Hz, 2H), 2.16 (d, J = 13.6 Hz, 2H), 1.90 - 1.73 (m, 4H), 1.31 (d, J = 6.4 Hz, 6H). | 712 |
| 305 | 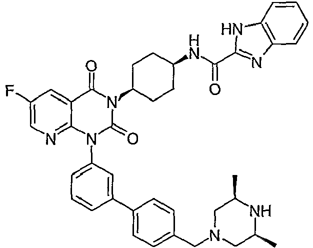 | *N*-{*cis*-4-[1-(4'-{[(3*R*,5*S*-3,5-dimethylpiperazin-1-yl]methyl}biphenyl-3-yl)-6-fluoro-2,4-dioxo-1,4-dihydropyrido[2,3-*d*]pyrimidin-3(2*H*)-yl]cyclohexyl}-1*H*-benzimidazole-2-carboxamide | ¹H NMR (400 MHz, CDCl₃) δ 8.37 (d, J = 2.8 Hz, 1H), 8.24 (dd, J = 7.2, 3.1 Hz, 1H), 7.76 - 7.73 (m, 1H), 7.70 - 7.64 (m, 5H), 7.55 - 7.52 (m, 3H), 7.38 - 7.33 (m, 4H), 5.08 - 4.99 (m, 1H), 4.33 (s, 1H), 4.21 (s, 2H), 3.73 - 3.65 (m, 2H), 3.41 - 3.19 (m, 4H), 2.90 - 2.78 (m, 2H), 2.20 (d, J = 13.6 Hz, 2H), 1.88 - 1.73 (m, 4H), 1.36 (d, J = 6.4 Hz, 6H). | 701 |
| 306 | 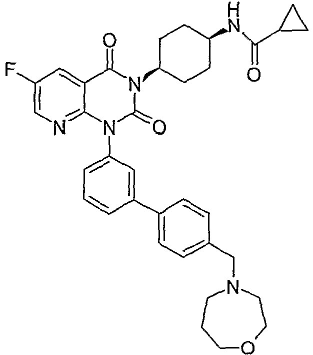 | *N*-{*cis*-4-[6-fluoro-1-[4'-(1,4-oxazepan-4-ylmethyl)biphenyl-3-yl]-2,4-dioxo-1,4-dihydropyrido[2,3-*d*]pyrimidin-3(2*H*)-yl]cyclohexyl}cyclopropanecarboxa mide | ¹H NMR (400 MHz, CDCl₃) δ 8.39 (d, J = 3.1 Hz, 1H), 8.23 (dd, J = 7.2,2.8 Hz, 1H), 7.75 (d, J = 7.9 Hz, 1H), 7.71 - 7.66 (m, 3H), 7.55 - 7.48 (m, 3H), 7.35 (d, J = 7.9 Hz, 1H), 6.24 (d, J = 7.9 Hz, 1H), 5.05 (t, J = 12.4 Hz, 1H), 4.34 - 4.30 (m, 2H), 4.30 - 4.24 (m, 1H), 3.96 - 3.90 (m, 2H), 3.89 - 3.63 (m, 6H), 3.87 - 3.81 (m, 2H), 2.70 - 2.54 (m, 2H), 1.93 (d, J = 17.3 Hz, 2H), 1.76 - 1.61 (m, 4H), 1.46 - 1.37 (m, 1H), 1.01 - 0.94 (m, 2H), 0.79 - 0.70 (m, 2H). | 612 |
| 307 | 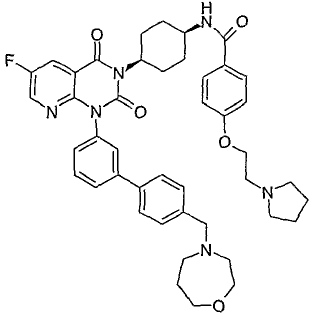 | *N*-{*cis*-4-[6-fluoro-1-[4'-(1,4-oxazepan-4-ylmethyl)biphenyl-3-yl]-2,4-dioxo-1,4-dihydropyrido[2,3-*d*]pyrimidin-3(2*H*)-yl]cyclohexyl}-4-(2-pyrrolidin-1-ylethoxy)benzamide | ¹H NMR (400 MHz, CDCl₃) δ 8.39 (d, J = 2.8 Hz, 1H), 8.25 (dd, J = 7.2, 2.8 Hz, 1H), 7.83 (d, J = 7.9 Hz, 2H), 7.75 (d, J = 7.9 Hz, 1H), 7.71 - 7.64 (m, 3H), 7.56 - 7.47 (m, 3H), 7.34 (d, J = 7.7 Hz, 1H), 6.86 (d, J = 7.7 Hz, 2H), 6.73 (d, J = 7.7 Hz, 1H), 5.19 - 5.04 (m, 1H), 4.51 - 4.06 (m, 11H), 4.00 - 3.77 (m, 6H), 3.63 - 3.49 (m, 3H), 3.08 - 2.95 (m, 3H), 2.74 - 2.59 (m, 2H), 2.22 - 1.98 (m, 6H), 1.83 - 1.69 (m, 4H). | 761 |
| 308 | 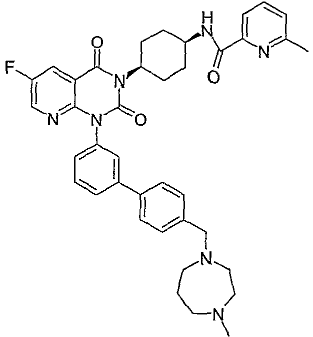 | *N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}-6-methylpyridine-2-carboxamide | ¹H NMR (400 MHz, CDCl₃) δ 8.78 (d, J = 8.5 Hz, 1H), 8.39 - 8.37 (m, 1H), 8.24 (dd, J = 7.4, 3.1 Hz, 1H), 7.97 (d, J = 7.4 Hz, 1H), 7.76 - 7.64 (m, 5H), 7.57 - 7.52 (m, 3H), 7.38 - 7.35 (m, 1H), 7.27 - 7.23 (m, 2H), 5.14 - 5.04 (m, 1H), 4.46 - 4.39 (m, 1H), 4.28 (s, 2H), 4.00 - 3.31 (m, 8H), 2.90 (s, 3H), 2.87 - 2.72 (m, 2H), 2.50 (s, 5H), 2.13 - 2.01 (m, 2H), 1.86 - 1.69 (m, 4H). | 676 |
| 309 | 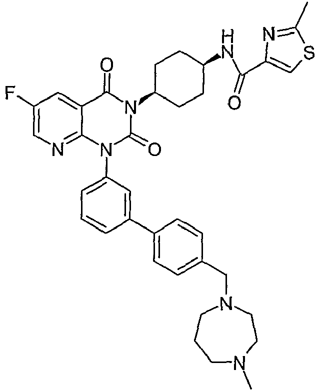 | *N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}-2-methyl-1,3-thiazole-4-carboxamide | ¹H NMR (400 MHz, CDCl₃) δ 8.37 (d, J = 2.8 Hz, 1H), 8.23 (dd, J = 7.2, 3.1 Hz, 1H), 7.91 (s, 1H), 7.76 - 7.71 (m, 2H), 7.71 - 7.65 (m, 3H), 7.56 - 7.52 (m, 3H), 7.38 - 7.34 (m, 1H), 5.04 (t, J = 12.2 Hz, 1H), 4.40 - 4.34 (m, 1H), 4.28 (s, 2H), 3.97 - 3.33 (m, 8H), 2.89 (s, 3H), 2.80 - 2.65 (m, 6H), 2.52 - 2.45 (m, 2H), 2.14 - 2.04 (m, 2H), 1.85 - 1.67 (m, 4H). | 682 |
| 310 | 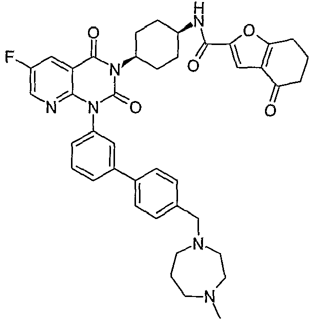 | *N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}-4-oxo-4,5,6,7-tetrahydro-1-benzofuran-2-carboxamide | ¹H NMR (400 MHz, CDCl₃) δ 10.06 (d, J = 6.2 Hz, 1H), 8.35 (d, J = 3.1 Hz, 1H), 8.22 (dd, J = 7.4, 3.1 Hz, 1H), 8.03 (s, 1H), 7.73 - 7.62 (m, 5H), 7.56 - 7.49 (m, 4H), 7.34 (dq, J = 7.7, 1.0 Hz, 1H), 4.98 (t, J = 12.3 Hz, 1H), 4.30 - 4.23 (m, 3H), 3.91 - 3.37 (m, 6H), 2.97 - 2.82 (m, 8H), 2.68 - 2.59 (m, 2H), 2.56 - 2.44 (m, 2H), 2.25 - 2.11 (m, 4H), 1.83 - 1.68 (m, 4H). | 719 |

### Example 311

### N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-2-methylalaninamide

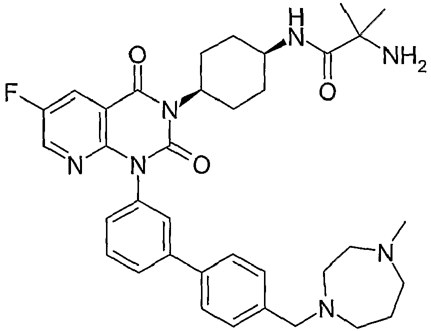

### Step (a) tert-butyl[2-({cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}amino)-1,1-dimethyl-2-oxoethyl]carbamate

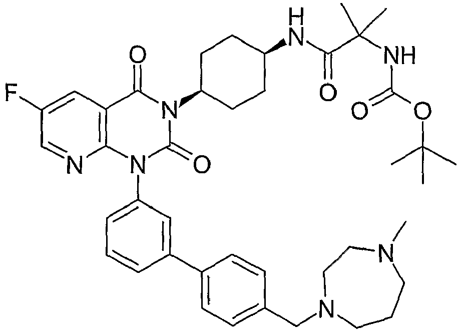

The product was prepared from 3-(*cis*-4-aminocyclohexyl)-6-fluoro-1-{4'-[(4-methyl-1,4-diazepan-1-yl)methyl]biphenyl-3-yl}pyrido[2,3-*d*]pyrimidine-2,4(1*H*,3*H*)-dione (0.245 g, 0.37 mmol) and 2-(*tert*-butoxycarbonylamino)-2-methylpropanoic acid (0.078 g, 0.39 mmol) by the method of example 287 step (c) without HPLC purification to give the sub-title compound as a white foam (0.226 g).
[M+H]+=742

Step (b) *N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}-2-methylalaninamide Hydrogen chloride (4M in 1,4-dioxane) (1.5 mL, 6.00 mmol) was added dropwise to a stirred solution of *tert*-butyl [2-({*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}amino)-1,1-dimethyl-2-oxoethyl]carbamate (0.226 g, 0.30 mmol) in 1,4-dioxane (3 mL). The resulting mixture was stirred at room temperature for 6 h then concentrated under vacuum. The crude product was purified by preparative HPLC on a Waters X-Bridge column using an 85-15% gradient of aqueous 0.2% trifluoroacetic acid in acetonitrile as eluent. The fractions containing the desired compound were evaporated to dryness to afford the title compound ditrifluoroacetate salt as a white foam (60 mg).
¹H NMR (400 MHz, DMSO) δ 8.59 (d, J = 2.8 Hz, 1H), 8.32 (dd, J = 7.7, 3.1 Hz, 1H), 8.14 - 8.09 (m, 3H), 7.83 (d, J = 8.3 Hz, 1H), 7.78 - 7.75 (m, 3H), 7.69 - 7.58 (m, 5H), 7.41 (d, J = 7.7 Hz, 1H), 4.81 (t, J = 11.7 Hz, 1H), 4.36 - 4.30 (m, 1H), 3.83 - 3.79 (m, 1H), 2.84 (s, 3H), 2.70 - 2.62 (m, 2H), 2.14 - 2.01 (m, 4H), 1.64 - 1.53 (m, 10H). Remaining protons obscured by solvent peaks.
[M+H]+=642 (Multimode+)

### Example 312

### N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-L-phenylalaninamide

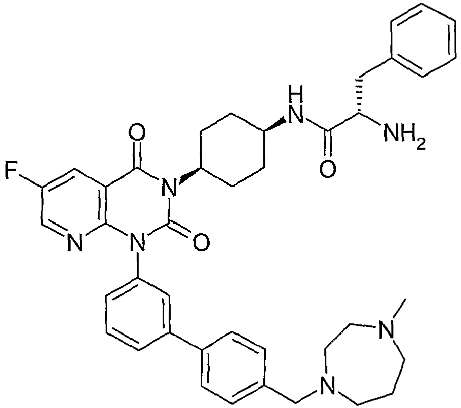

### Step (a) tert-butyl [(1S)-1-benzyl-2-({cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}amino)-2-oxoethyl]carbamate

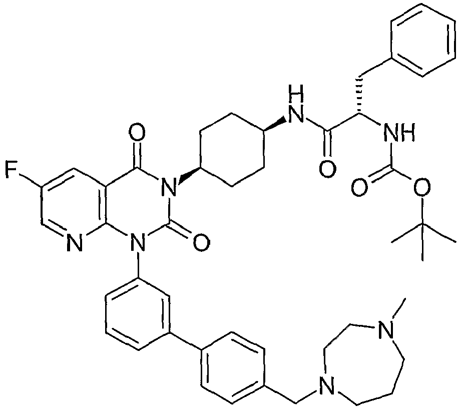

The product was prepared from 3-(*cis*-4-aminocyclohexyl)-6-fluoro-1-{4'-[(4-methyl-1,4-diazepan-1-yl)methyl]biphenyl-3-yl}pyrido[2,3-*d*]pyrimidine-2,4(1*H*,3*H*)-dione (0.244 g, 0.37 mmol) and (S)-2-(*tert*-butoxycarbonylamino)-3-phenylpropanoic acid (0.097 g, 0.37 mmol) using the method of example 311 step (a) to give the sub-title compound as a colourless glass (0.249 g).
[M-Boc]+=704

Step (b) *N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}-L-phenylalaninamide The product was prepared from *tert*-butyl [(1*S*)-1-benzyl-2-({*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}amino)-2-oxoethyl]carbamate (0.249 g, 0.31 mmol) using the method of example 311 step (b) to give the title compound ditrifluoroacetate salt as a white foam (0.192 g).
¹H NMR (400 MHz, DMSO) δ 8.59 (d, J = 2.8 Hz, 1H), 8.30 (dd, J = 7.8, 2.9 Hz, 1H), 8.21 - 8.11 (m, 4H), 7.83 - 7.73 (m, 4H), 7.66 - 7.54 (m, 3H), 7.41 (d, J = 7.2 Hz, 1H), 7.31 - 7.20 (m, 5H), 4.79 - 4.70 (m, 1H), 4.36 - 4.13 (m, 2H), 3.87 - 3.82 (m, 1H), 3.07 - 2.97 (m, 4H), 2.83 (s, 3H), 2.67 - 2.61 (m, 2H), 2.14 - 2.05 (m, 2H), 1.87 (d, J = 14.1 Hz, 2H), 1.70 - 1.44 (m, 4H). Remaining protons obscured by solvent peaks.
[M+H]+=704 (Multimode+)

### Example 313

### N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-D-valinamide

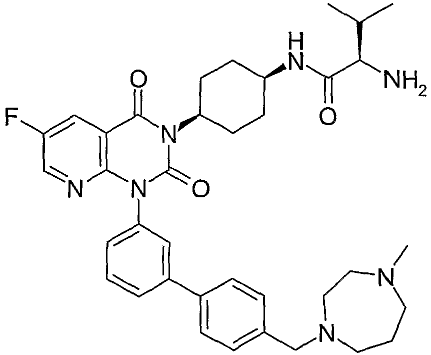

### Step (a) tert-butyl {(1R)-1-[({cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl} amino)carbonyl]-2-methylpropyl} carbamate

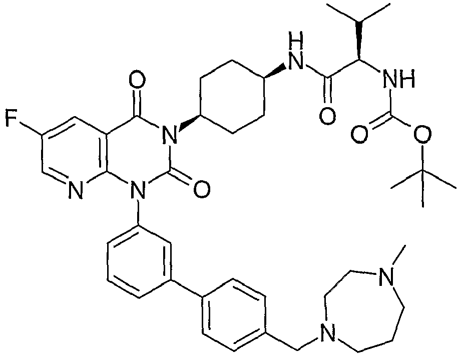

The product was prepared from 3-(*cis*-4-aminocyclohexyl)-6-fluoro-1-{4'-[(4-methyl-1,4-diazepan-1-yl)methyl]biphenyl-3-yl}pyrido[2,3-*d*]pyrimidine-2,4(1*H*,3*H*)-dione (0.242 g, 0.36 mmol) and (R)-2-(tert-butoxycarbonylamino)-3-methylbutanoic acid (0.083 g, 0.38 mmol) using the method of example 311 step (a) to give the sub-title compound as a colourless oil (0.240 g).
[M+H]+=756

### Step (b) N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-D-valinamide

The product was prepared from *tert*-butyl {(1*R*)-1-[({*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}amino)carbonyl]-2-methylpropyl}carbamate (0.275 g, 0.31 mmol) using the method of example 311 step (b) to give the title compound ditrifluoroacetate salt as a white foam (0.159 g).
¹H NMR (400 MHz, DMSO) δ 8.59 (d, J = 2.8 Hz, 1H), 8.31 - 8.27 (m, 2H), 8.10 - 8.04 (m, 3H), 7.82 (d, J = 7.9 Hz, 1H), 7.78 - 7.75 (m, 2H), 7.66 - 7.58 (m, 3H), 7.41 (d, J = 7.9 Hz, 1H), 4.82 - 4.76 (m, 1H), 4.36 - 4.28 (m, 1H), 3.94 - 3.89 (m, 1H), 3.74 - 3.69 (m, 1H), 2.84 (s, 3H), 2.71 - 2.58 (m, 2H), 2.15 - 2.05 (m, 2H), 1.95 - 1.84 (m, 2H), 1.72 - 1.53 (m, 4H), 0.95 (d, J = 6.4 Hz, 6H). Remaining protons obscured by solvent peaks.
[M+H]+=656 (Multimode+)

### Example 314

### N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-L-valinamide

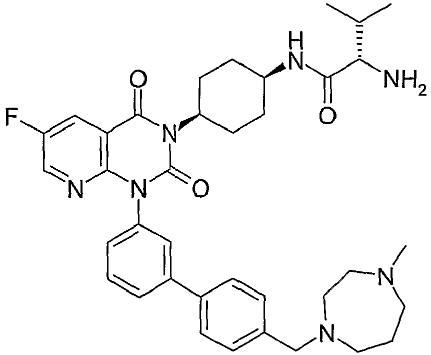

### Step (a) tert-butyl {(1S)-1-[({cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}amino)carbonyl]-2-methylpropyl}carbamate

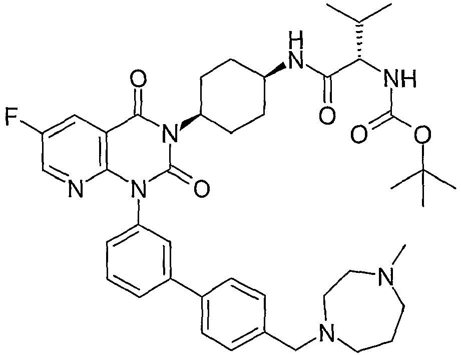

The product was prepared from 3-(*cis*-4-aminocyclohexyl)-6-fluoro-1-{4'-[(4-methyl-1,4-diazepan-1-yl)methyl]biphenyl-3-yl}pyrido[2,3-*d*]pyrimidine-2,4(1*H*,3*H*)-dione (0.248 g, 0.37 mmol) and (S)-2-(tert-butoxycarbonylamino)-3-methylbutanoic acid (0.085 g, 0.39 mmol) using the method of example 311 step (a) to give the sub-title compound as a colourless oil (0.247 g).
[M+H]+=756

### Step (b) N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-L-valinamide

The product was prepared from *tert*-butyl {(1*S*)-1-[({*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}amino)carbonyl]-2-methylpropyl}carbamate (0.281 g, 0.37 mmol) using the method of example 311 step (b) to give the title compound ditrifluoroacetate salt as a white foam (0.181 g).
¹H NMR (400 MHz, DMSO) δ 8.59 (d, J = 3.1 Hz, 1H), 8.31 - 8.27 (m, 2H), 8.09 - 8.04 (m, 3H), 7.82 (d, J = 7.4 Hz, 1H), 7.78 - 7.75 (m, 2H), 7.66 - 7.58 (m, 3H), 7.41 (d, J = 7.4 Hz, 1H), 4.82 - 4.76 (m, 1H), 4.33 - 4.23 (m, 1H), 3.94 - 3.89 (m, 1H), 3.74 - 3.69 (m, 1H), 2.84 (s, 3H), 2.71 - 2.58 (m, 2H), 2.09 (m, 2H), 1.96 - 1.84 (m, 2H), 1.72 - 1.53 (m, 4H), 0.96 (d, J = 6.7 Hz, 6H). Remaining protons obscured by solvent peaks.
[M+H]+=656 (Multimode+)

### Example 315

### 6-fluoro-3-(cis-4-{[(6-fluoroimidazo[1,2-a]pyridin-2-yl)methyl]amino}cyclohexyl)-1-(4'-hydroxy-2'-{[[(1S)-2-methoxy-1-methylethyl](methyl)amino]methyl}biphenyl-3-yl)pyrido[2,3-d]pyrimidine-2,4(1H,3H)-dione

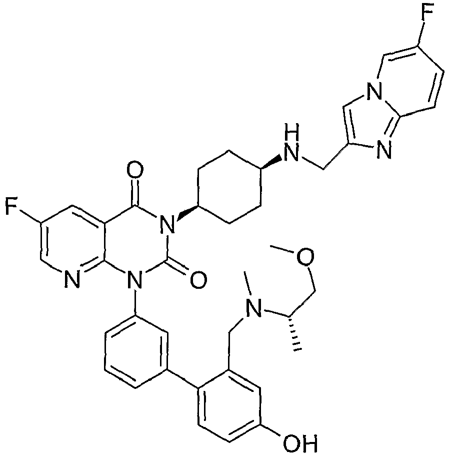

A mixture of (S)-1-methoxypropan-2-amine (140 mg, 1.57 mmol), *tert*-butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) and sodium triacetoxyborohydride (332 mg, 1.57 mmol) was stirred in DCE (20 mL) overnight. To this solution was added formaldehyde (0.288 mL, 4.18 mmol) followed by a further aliquot of sodium triacetoxyborohydride (332 mg, 1.57 mmol). This mixture was then allowed to stir for 2 h. The reaction mixture was washed with 10% sodium bicarbonate solution (30 ml) and dried over sodium sulfate. The organics were filtered and evaporated to dryness. This residue was then taken up into 50:50 DCM: trifluoroacetic acid (10 ml) and allowed to stand for 2 h. The mixture was evaporated to dryness, extracted into DCM and washed with 10% sodium bicarbonate solution (30 ml) and dried over sodium sulfate. The organics were filtered and evaporated to dryness. This residue was taken up into DCE (20 mL) and to this solution was added 6-fluoroimidazo[1,2-a]pyridine-2-carbaldehyde (206 mg, 1.25 mmol) and the mixture allowed to stir for 1 hour, followed by the addition of sodium triacetoxyborohydride (332 mg, 1.57 mmol) and the resultant mixture stirred overnight. The organics were washed with 10% sodium bicarbonate solution (30 ml) and dried over sodium sulfate. The crude product was purified by preparative HPLC on a Phenomenex Gemini column using a 75-5% gradient of aqueous 0.2% ammonia in acetonitrile as eluent. The fractions containing the desired compound were evaporated to dryness to afford the title compound as a colourless solid (0.176 g).
¹H NMR (400 MHz, DMSO) δ 9.42 (s, 1H), 8.70 (dd, J = 4.6, 2.6 Hz, 1H), 8.57 (d, J = 3.1 Hz, 1H), 8.28 (dd, J = 7.8, 2.9 Hz, 1H), 7.83 (s, 1H), 7.52 (dd, J = 15.2, 1.9 Hz, 3H), 7.39 (d, J = 7.9 Hz, 1H), 7.33 - 7.20 (m, 2H), 7.12 (d, J = 8.5 Hz, 1H), 6.92 (d, J = 2.6 Hz, 1H), 6.73 (dd, J = 8.5, 2.6 Hz, 1H), 4.77 (t, J = 37.6 Hz, 1H), 3.80 (s, 2H), 3.42 (s, 2H), 3.32 (s, 3H), 3.24 (dd, J= 9.6, 6.3 Hz, 1H), 3.12 - 2.95 (m, 4H), 2.88 - 2.64 (m, 4H), 2.00 (s, 3H), 1.89 (d, J = 13.1 Hz, 2H), 1.46 (dd, J = 29.3,13.7 Hz, 4H), 0.77 (d, J = 6.7 Hz, 3H).
[M+H]+=710 (Multimode+)

### Example 316

### 6-fluoro-3-(cis-4-{[(6-fluoroimidazo[1,2-a]pyridin-2-yl)methyl]amino}cyclohexyl)-1-(4'-hydroxy-2'-{[(3R)-3-methylmorpholin-4-yl]methyl}biphenyl-3-yl)pyrido[2,3-d]pyrimidine-2,4(1H,3H)-dione

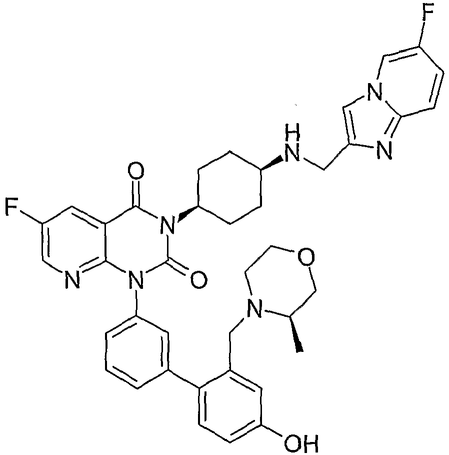

To a mixture of *tert*-butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (568 mg, 0.99 mmol) and (R)-3-methylmorpholine hydrochloride (200mg, 1.98 mmol) in DCE (10 mL) was added triethylamine (0.276 mL, 1.98 mmol) and the mixture stirred for 1 hour at room temperature. To this mixture was then added sodium triacetoxyborohydride (419 mg, 1.98 mmol) and the mixture stirred overnight. The mixture was washed with saturated sodium bicarbonate solution and dried over sodium sulfate. The organics were evaporated to dryness and the residue taken up into DCM (20 ml). To this solution was added TFA (10 mL) and the mixture allowed to stand at room temp for 1 hour before evaporation to dryness. The residue was dissolved in water (30 ml), neutralised with 0.88 ammonium hydroxide. The solid was filtered off and dried. A mixture of this crude compound, 6-fluoroimidazo[1,2-a]pyridine-2-carbaldehyde (138 mg, 0.84 mmol), and sodium triacetoxyborohydride (178 mg, 0.84 mmol) in a solvent of DCE (20 mL) was stirred overnight. The reaction mixture was washed with 10% sodium bicarbonate solution and the organic layer separated and dried over sodium sulfate. The crude product was purified by preparative HPLC on a Waters X-Terra column using a 95-50% gradient of aqueous 0.1% trifluoroacetic acid in acetonitrile as eluent. The fractions containing the desired compound were evaporated to dryness to afford the title compound ditrifluoroacetate salt as a colourless solid (0.247 g).
¹H NMR (299.947 MHz, DMSO) d 8.78 (ddd, J = 4.6, 2.5, 0.8 Hz, 1H), 8.52 (d, J = 3.1 Hz, 1H), 8.23 (dd, J = 7.7, 3.1 Hz, 1H), 8.06 (s, 1H), 7.65 - 7.53 (m, 2H), 7.43 - 7.30 (m, 4H), 7.16 (d, J = 8.3 Hz, 1H), 7.06 (1, J = 2.3 Hz, 1H), 6.89 (d, J = 7.5 Hz, 1H), 4.83 (t, J = 11.4 Hz, 2H), 4.34 (s, 5H), 3.77 - 3.26 (m, 5H), 2.86 - 2.52 (m, 5H), 2.15 (d, J = 14.6 Hz, 2H), 1.87 - 1.58 (m, 4H), 1.03 (d, J = 5.6 Hz, 3H)
[M+H]+=708 (Multimode+)

### Example 317

### 6-fluoro-3-(cis-4-{[(6-fluoroimidazo[1,2-a]pyridin-2-yl)methyl]amino}cyclohexyl)-1-(4'-hydroxy-2'-{[(3S)-3-methylmorpholin-4-yl]methyl}biphenyl-3-yl)pyrido[2,3-d]pyrimidine-2,4(1H,3H)-dione

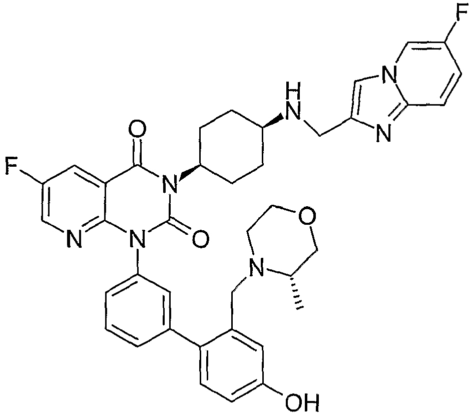

The product was prepared from *tert*-butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (0.450 g, 0.78 mmol) and (S)-3-methylmorpholine hydrochloride (158 mg, 1.57 mmol) by the method of example 316 but the crude product was purified by reverse phase HPLC on a Phenomenex Gemini column using a 95-5% gradient of aqueous 0.2% ammonia in acetonitrile as eluent. The fractions containing the desired compound were evaporated to dryness to afford the title compound as a colourless solid (0.143 g).
¹H NMR (400 MHz, DMSO) δ 9.43 (s, 1H), 8.70 (dd, J = 4.6, 2.3 Hz, 1H), 8.56 (d, J = 2.8 Hz, 1H), 8.28 (dd, J = 7.7, 3.1 Hz, 1H), 7.83 (s, 1H), 7.57 - 7.48 (m, 2H), 7.43 - 7.35 (m, 3H), 7.33 - 7.21 (m, 2H), 7.07 (d, J = 8.5 Hz, 1H), 6.97 (d, J = 2.6 Hz, 1H), 6.71 (dd, J = 8.2, 2.6 Hz, 1H), 4.78 (t, J = 29.6 Hz, 1H), 3.79 (d, J = 12.8 Hz, 3H), 3.49 (s, 2H), 3.41 - 3.27 (m, 1H), 3.07 (d, J = 13.3 Hz, 2H), 2.85 (s, 1H), 2.74 (d, J = 12.6 Hz, 2H), 2.36 - 2.24 (m, 1H), 1.97 (m, 1H), 1.89 (d, J = 13.1 Hz, 2H), 1.55 - 1.39 (m, 4H), 0.80 (s, 3H).
[M+H]+=708 (Multimode+)

### Example 318

### 6-fluoro-3-(cis-4-{[(6-fluoroimidazo[1,2-a]pyridin-2-yl)methyl]amino}cyclohexyl)-1-(4'-hydroxy-2'-{[[(2S)-2-hydroxypropyl](methyl)amino]methyl}biphenyl-3-yl)pyrido[2,3-d]pyrimidine-2,4(1H,3H)-dione

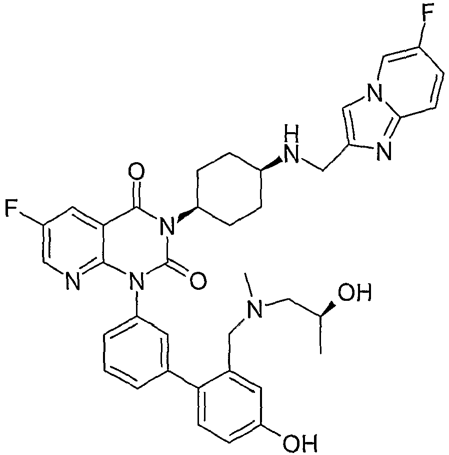

The product was prepared from (S)-1-aminopropan-2-ol (78 mg, 1.04 mmol) and *tert*-butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) by the method of example 315. The crude product was purified by preparative HPLC on a Phenomenex Gemini column using a 95-5% gradient of aqueous 0.1% trifluoroacetic acid in acetonitrile as eluent. The fractions containing the desired compound were evaporated to dryness to afford the title compound ditrifluoroacetate salt (0.130 g).
¹H NMR (300 MHz, DMSO) δ 9.2 (s, 1H), 8.91 (dd, J = 4.6, 2.5 Hz, 3H), 8.61 (d, J = 2.7 Hz, 1H), 8.31 (dd, J = 7.6, 3.0 Hz, 1H), 8.12 (d, J = 9.0 Hz, 1H), 7.71 - 7.58 (m, 2H), 7.48 - 7.34 (m, 4H), 7.25 - 7.15 (m, 2H), 6.97 (dd, J = 8.4, 2.2 Hz, 1H), 4.78 (t, J = 20.4 Hz, 1H), 4.36 (s, 3H), 3.92 (s, 1H), 3.38 (s, 1H), 2.67 - 2.52 (m, 9H), 2.19 - 2.06 (m, 2H), 1.81 - 1.56 (m, 4H), 1.10 - 0.85 (m, 3H).
[M+H]+=696 (Multimode+)

### Example 319

### 6-fluoro-3-(cis-4-{[(6-fluoroimidazo[1,2-a]pyridin-2-yl)methyl]amino}cyclohexyl)-1-(4'-hydroxy-2'-{[[(1R)-2-hydroxy-1-methylethyl](methyl)amino]methyl}biphenyl-3-yl)pyrido[2,3-d]pyrimidine-2,4(1H,3H)-dione

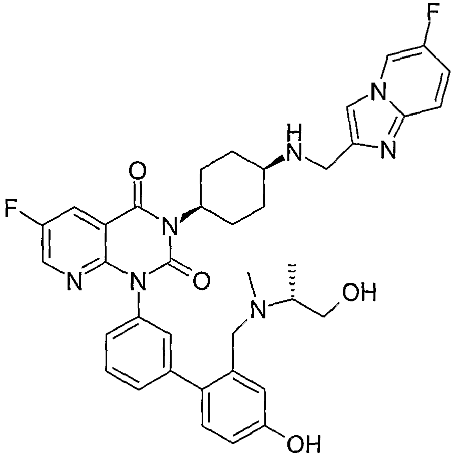

The product was prepared from (R)-2-aminopropan-1-ol (78 mg, 1.04 mmol) and *tert*-butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) by the method of example 315. The crude product was purified by preparative HPLC on a Phenomenex Gemini column using a 95-5% gradient of aqueous 0.1% trifluoroacetic acid in acetonitrile as eluent. The fractions containing the desired compound were evaporated to dryness to afford the title compound ditrifluoroacetate salt as a colourless solid (0.163 g).
¹H NMR (300 MHz, DMSO) δ 8.90 (dd, J = 4.5, 2.4 Hz, 3H), 8.57 (s, 1H), 8.31 (dd, J = 7.7, 2.9 Hz, 1H), 8.10 (s, 1H), 7.71 - 7.57 (m, 2H), 7.47 - 7.32 (m, 4H), 7.22 (d, J = 8.3 Hz, 2H), 6.97 (dd, J = 8.3, 2.3 Hz, 2H), 4.79 (s, 1H), 4.41 - 4.18 (m, 4H), 3.41 (d, J = 26.3 Hz, 3H), 2.65 - 2.26 (m, 8H), 2.13 (d, J = 14.4 Hz, 1H), 1.83 - 1.56 (m, 5H), 1.10 - 0.98 (d, 1H), 0.69 (d, 2H).
[M+H]+=696 (Multimode+)

### Example 320

### 6-fluoro-3-(cis-4-{[(6-fluoroimidazo[1,2-a]pyridin-2-yl)methyl]amino}cyclohexyl)-1-(4'-hydroxy-2'-{[[(2R)-2-hydroxypropyl](methyl)amino]methyl}biphenyl-3-yl)pyrido[2,3-d]pyrimidine-2,4(1H,3H)-dione

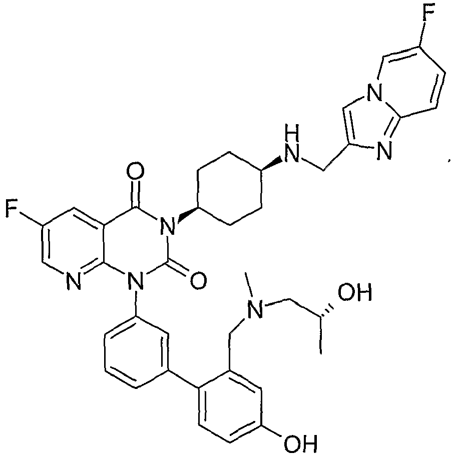

The product was prepared from (R)-1-aminopropan-2-ol (78 mg, 1.04 mmol) and *tert-*butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) by the method of example 315. The crude product was purified by preparative HPLC on a Phenomenex Gemini column using a 95-5% gradient of aqueous 0.1% trifluoroacetic acid in acetonitrile as eluent. The fractions containing the desired compound were evaporated to dryness to afford the title compound ditrifluoroacetate salt as a colourless solid (0.114 g).
¹H NMR (300 MHz, DMSO) δ 9.2 (s, 1H), 8.91 (dd, J = 4.6, 2.5 Hz, 3H), 8.61 (d, J = 2.7 Hz, 1H), 8.31 (dd, J = 7.6, 3.0 Hz, 1H), 8.12 (d, J = 9.0 Hz, 1H), 7.71 - 7.58 (m, 2H), 7.48 - 7.34 (m, 4H), 7.25 - 7.15 (m, 2H), 6.97 (dd, J = 8.4, 2.2 Hz, 1H), 4.78 (t, J = 20.4 Hz, 1H), 4.36 (s, 3H), 3.92 (s, 1H), 3.38 (s, 1H), 2.67 - 2.52 (m, 9H), 2.19 - 2.06 (m, 2H), 1.81 - 1.56 (m, 4H), 1.10 - 0.85 (m, 3H).
[M+H]+=696 (MultiMode+)

### Example 321

### 6-fluoro-3-(cis-4-{[(6-fluoroimidazo[1,2-a]pyridin-2-yl)methyl]amino}cyclohexyl)-1-(4'-hydroxy-2'-{[[1-(hydroxymethyl)cyclopropyl](methyl)amino]methyl}biphenyl-3-yl)pyrido[2,3-d]pyrimidine-2,4(1H,3H)-dione

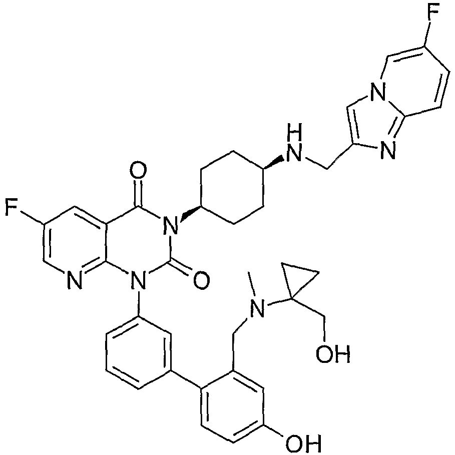

The product was prepared from (1-aminocyclopropyl)methanol (91 mg, 1.04 mmol) and *tert-*butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) by the method of example 315. The crude product was purified by preparative HPLC on a Phenomenex Gemini column using a 95-5% gradient of aqueous 0.1% ammonium acetate in acetonitrile as eluent. The fractions containing the desired compound were evaporated to dryness to afford the title compound as a colourless solid (41 mg).
¹H NMR (300 MHz, DMSO) δ 9.35 (s, 1H), 8.71 (s, 1H), 8.59 (d, J = 1.9 Hz, 1H), 8.32 - 8.23 (m, 1H), 7.84 (s, 1H), 7.57 - 7.47 (m, 2H), 7.37 - 7.21 (m, 3H), 7.01 (d, J = 8.3 Hz, 1H), 6.87 (s, 1H), 6.67 (d, J = 8.1 Hz, 1H), 4.78 (t, J = 27.2 Hz, 1H), 4.36 (d, J = 5.0 Hz, 1H), 3.79 (d, J = 8.3 Hz, 4H), 2.88 - 2.80 (m, 2H), 2.72 (s, 2H), 2.15 (s, 3H), 1.90 (d, J = 12.3 Hz, 3H), 1.49 (d, J = 23.5 Hz, 4H), 0.43 (d, J = 26.0 Hz, 4H).
[M+H]+=708 (Multimode+)

### Example 322

### 6-fluoro-3-(cis-4-{[(6-fluoroimidazo[1,2-a]pyridin-2-yl)methyl]amino}cyclohexyl)-1-(4'-hydroxy-2'-{[[(1-hydroxycyclopropyl)methyl](methyl)amino]methyl}biphenyl-3-yl)pyrido[2,3-d]pyrimidine-2,4(1H,3H)-dione

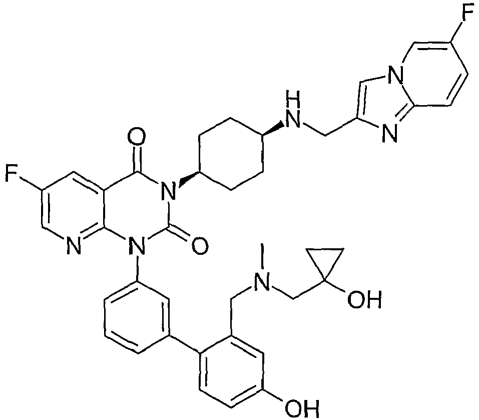

The product was prepared from 1-(aminomethyl)cyclopropanol (0.136 g, 1.57 mmol) and *tert-*butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) by the method of example 315. The crude product was purified by preparative HPLC on a Phenomenex Gemini column using a 90-40 % gradient of aqueous 0.2% TFA in acetonitrile as eluent. The fractions containing the desired compound were evaporated to dryness to afford the title compound ditrifluoroacetate salt (20 mg).
¹H NMR (400 MHz, DMSO) δ 10.03 (s, 1H), 9.49 (s, 1H), 9.00 - 8.86 (m, 2H), 8.59 (d, J = 2.8 Hz, 1H), 8.31 (dd, J = 7.7, 2.8 Hz, 1H), 8.10 (s, 1H), 7.71 - 7.57 (m, 2H), 7.45 - 7.33 (m, 4H), 7.25 - 7.08 (m, 2H), 6.97 (dd, J = 8.5, 2.3 Hz, 1H), 4.81 - 4.72 (m, 1H), 4.36 (s, 2H), 4.29 - 4.20 (m, 1H), 3.40 - 3.35 (m, 3H), 3.07 (d, J = 27.1 Hz, 1H), 2.78 - 2.69 (m, 1H), 2.59 (s, 3H), 2.13 (d, J = 14.1 Hz, 2H), 1.78 - 1.60 (m, 4H), 1.25 (s, 1H), 0.78 - 0.56 (m, 4H).
[M+H]+=708 (Multimode+)

### Example 323

### 1-(2'-{[cyclopropyl(methyl)amino]methyl}-4'-hydroxybiphenyl-3-yl)-6-fluoro-3-(cis-4-{[(6-fluoroimidazo[1,2-a]pyridin-2-yl)methyl]amino}cyclohexyl)pyrido[2,3-d]pyrimidine-2,4(1H,3H)-dione

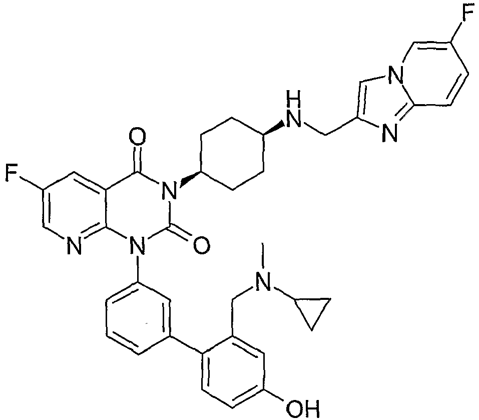

The product was prepared from cyclopropanamine (0.089 g, 1.57 mmol) and *tert*-butyl-{*cis-*4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) by the method of example 315. The crude product was purified by preparative HPLC on a Phenomenex Gemini column using a 90-40 % gradient of aqueous 0.2% TFA in acetonitrile as eluent. The fractions containing the desired compound were evaporated to dryness to afford the title compound ditrifluoroacetate salt (25 mg).
¹H NMR (400 MHz, DMSO) δ 9.05 - 8.92 (m, 2H), 8.66 (d, J = 3.1 Hz, 1H), 8.36 (dd, J = 7.7, 3.1 Hz, 1H), 8.16 (s, 1H), 7.76 - 7.63 (m, 2H), 7.52 - 7.43 (m, 3H), 7.28 (d, J = 8.5 Hz, 1H), 7.12 (s, 1H), 7.03 (d, J = 6.9 Hz, 1H), 4.84 (t, J = 13.9 Hz, 1H), 4.50 - 4.39 (m, 3H), 3.48 (s, 1H), 2.74 - 2.59 (m, 5H), 2.48 - 2.40 (m, 1H), 2.19 (d, J = 14.1 Hz, 2H), 1.81 (t, J = 14.2 Hz, 2H), 1.70 (d, J = 10.5 Hz, 2H), 0.92 - 0.48 (m, 5H).
[M+H]+=678 (Multimode+)

### Example 324

### 6-fluoro-3-(cis-4-{[(6-fluoroimidazo[1,2-a]pyridin-2-yl)methyl]amino}cyclohexyl)-1-(4'-hydroxy-2'-{[[(1S)-2-hydroxy-1-methylethyl](methyl)amino]methyl}biphenyl-3-yl)pyrido[2,3-d]pyrimidine-2,4(1H,3H)-dione

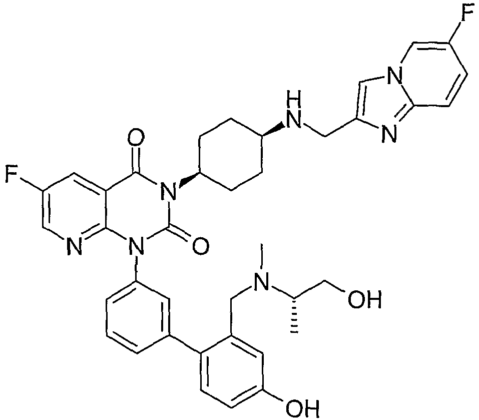

The product was prepared from (S)-2-aminopropan-1-ol (0.118 g, 1.57 mmol) and *tert*-butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) by the method of example 315. The crude product was purified by preparative HPLC on a Phenomenex Gemini column using a 90-40 % gradient of aqueous 0.2% TFA in acetonitrile as eluent. The fractions containing the desired compound were evaporated to dryness to afford the title compound ditrifluoroacetate salt (0.132 g).
¹H NMR (400 MHz, DMSO) δ 10.03 (s, 1H), 8.90 (dd, J = 4.4, 2.3 Hz, 2H), 8.63 - 8.53 (m, 1H), 8.31 (d, J = 4.9 Hz, 1H), 8.10 (s, 1H), 7.70 - 7.58 (m, 2H), 7.47 - 7.39 (m, 3H), 7.35 (s, 1H), 7.26 - 7.19 (m, 2H), 6.97 (dd, J = 8.5, 2.3 Hz, 1H), 4.78 (t, J = 16.2 Hz, 1H), 4.39 (s, 3H), 3.52 - 3.30 (m, 4H), 3.04 (s, 1H), 2.63 - 2.55 (m, 2H), 2.54 (d, J = 3.3 Hz, 3H), 2.33 (d, J = 1.8 Hz, 1H), 2.13 (d, J = 14.6 Hz, 2H), 1.77 (d, J = 14.6 Hz, 2H), 1.68 - 1.60 (m, 2H), 1.09 - 1.01 (m, 1H), 0.73 - 0.67 (m, 2H).
[M+H]+=696 (Multimode+)

### Example 325

### 1-(2'-{[(cyclopropylmethyl)(methyl)amino]methyl}-4'-hydroxybiphenyl-3-yl)-6-fluoro-3-(cis-4-{[(6-fluoroimidazo[1,2-a]pyridin-2-yl)methyl]amino}cyclohexyl)pyrido[2,3-d]pyrimidine-2,4(1H,3H)-dione

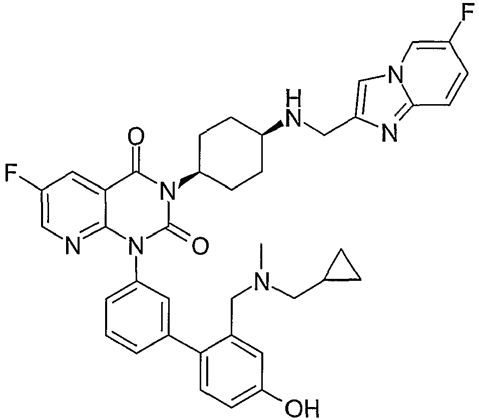

The product was prepared from cyclopropylmethanamine (0.111 g, 1.57 mmol) and *tert-*butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (600 mg, 1.04 mmol) by the method of example 315. The crude product was purified by preparative HPLC on a Phenomenex Gemini column using a 90-40 % gradient of aqueous 0.2% TFA in acetonitrile as eluent. The fractions containing the desired compound were evaporated to dryness to afford the title compound ditrifluoroacetate salt (0.130 g).
¹H NMR (400 MHz, DMSO) δ 9.42 (s, 1H), 8.82 (s, 1H), 8.67 (dd, J = 4.4, 2.6 Hz, 1H), 8.33 (d, J = 2.8 Hz, 1H), 8.05 (dd, J = 7.7, 3.1 Hz, 1H), 7.87 (s, 1H), 7.43 (dd, J = 9.9, 5.3 Hz, 1H), 7.37 (t, J = 7.7 Hz, 1H), 7.23 - 7.11 (m, 4H), 6.98 (d, J = 8.5 Hz, 1H), 6.86 (d, J = 2.3 Hz, 1H), 6.73 (dd, J = 8.5, 2.3 Hz, 1H), 4.53 (t, J = 11.7 Hz, 1H), 4.07 - 3.90 (m, 4H), 3.14 (s, 1H), 2.67 - 2.59 (m, 1H), 2.51 - 2.42 (m, 1H), 2.42 - 2.27 (m, 5H), 1.89 (d, J = 13.8 Hz, 2H), 1.50 (t, J = 13.8 Hz, 2H), 1.40 (d, J = 11.3 Hz, 2H), 0.65 - 0.58 (m, 1H), 0.26 (s, 2H), 0.10 (s, 2H).
[M+H]+=692 (Multimode+)

### Example 326

### 6-fluoro-3-(cis-4-{[(6-fluoroimidazo[1,2-a]pyridin-2-yl)methyl]amino}cyclohexyl)-1-(4'-hydroxy-2'-{[(3R)-3-hydroxypyrrolidin-1-yl]methyl}biphenyl-3-yl)pyrido[2,3-d]pyrimidine-2,4(1H,3H)-dione

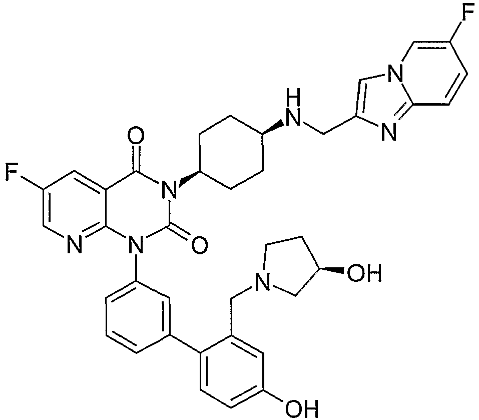

*tert*-Butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (300 mg, 0.52 mmol) and (R)-pyrrolidin-3-ol (50.0 mg, 0.57 mmol) were stirred at room temperature in DCM (20 mL) for 15 mins then sodium triacetoxyborohydride (133 mg, 0.63 mmol) was added. The mixture was stirred at room temperature for 20 h then TFA (2 mL, 25.96 mmol) was added. The mixture was stirred for 3 h and concentrated in vacuo. Saturated sodium hydrogen carbonate was added and the mixture was extracted with ethyl acetate, which was dried with sodium sulfate and concentrated to leave a solid (100 mg). This was stirred with 6-fluoroimidazo[1,2-a]pyridine-2-carbaldehyde (30 mg, 0.18 mmol) at room temperature in DCM (20 mL) for 15 mins then sodium triacetoxyborohydride (47 mg, 0.22 mmol) was added. The mixture was stirred for 3 h and concentrated in vacuo. Water was added and the mixture extracted with ethyl acetate, which was dried with sodium sulfate and concentrated in vacuo. The crude product was purified by preparative HPLC on a Waters X-Terra column using a 75-5% gradient of aqueous 0.2% ammonia in acetonitrile as eluent. The fractions containing the desired compound were evaporated to dryness to afford the title compound as a solid (56 mg). ¹H NMR (300 MHz, DMSO) δ 8.70 (dd, J = 4.7, 2.4 Hz, 1H), 8.58 (d, J = 3.1 Hz, 1H), 8.27 (dd, J = 7.7, 2.9 Hz, 1H), 7.83 (s, 1H), 7.58 - 7.47 (m, 3H), 7.44 (d, J = 7.7 Hz, 1H), 7.32 - 7.20 (m, 2H), 7.11 (d, J = 8.5 Hz, 1H), 6.89 (d, J = 2.3 Hz, 1H), 6.72 (dd, J = 8.4, 2.4 Hz, 1H), 4.86 - 4.70 (m, 1H), 4.11 - 4.02 (m, 1H), 3.80 (d, J = 6.7 Hz, 2H), 3.37 (s, 2H), 2.89 - 2.80 (m, 1H), 2.79 - 2.65 (m, 2H), 2.33 - 2.19 (m, 3H), 1.96 - 1.82 (m, 2H), 1.81 - 1.71 (m, 1H), 1.55 - 1.35 (m, 4H).
[M+H]+=694 (Multimode+)

The following compounds (Table 10) were prepared in a similar manner as solids from *tert-*butyl-{*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate, 6-fluoroimidazo[1,2-a]pyridine-2-carbaldehyde and the appropriate amine using the method described above in example 326.

**Table 10**

| **Example Number** | **Chemistry** | **Name** | **NMR** | **M+H** |
|---|---|---|---|---|
| 327 | 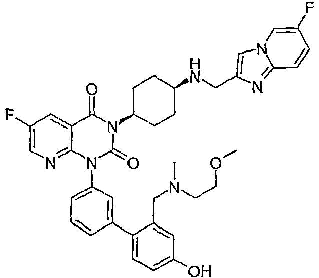 | 6-fluoro-3-(*cis*-4-{[(6-fluoroimidazo[1,2-*a*]pyridin-2-yl)methyl]amino}cyclohexyl)-1-(4'-hydroxy-2'-{[(2-methoxyethyl)(methyl)amino]methyl }biphenyl-3-yl)pyrido[2,3-*d*]pyrimidine-2,4(1*H*,3*H*)-dione | ¹H NMR (300 MHz, DMSO) δ 8.70 (dd, J = 4.3, 2.2 Hz, 1H), 8.58 (d, J = 2.9 Hz, 1H), 8.28 (dd, J = 7.7,3.1 Hz, 1H), 7.83 (s, 1H), 7.56 - 7.46 (m, 3H), 7.40 (d, J = 7.9 Hz, 1H), 7.31 - 7.23 (m, 2H), 7.09 (d, J = 8.5 Hz, 1H), 6.86 (d, J = 2.3 Hz, 1H), 6.70 (dd, J = 8.3, 2.5 Hz, 1H), 4.85 - 4.72 (m, 1H), 3.80 (d, J = 6.9 Hz, 2H), 3.35 - 3.22 (m, 4H), 3.05 (s, 3H), 2.85 (s, 1H), 2.80 - 2.65 (m, 2H), 2.44 - 2.33 (m, 2H), 1.95 - 1.84 (m, 2H), 1.81 - 1.71 (m, 1H), 1.56 - 1.37 (m, 4H). | 696 |
| 328 | 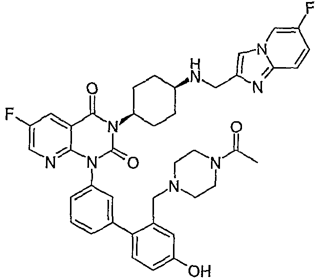 | 1-{2'-[(4-acetylpiperazin-1-yl)methyl]-4'-hydroxybiphenyl-3-yl}-6-fluoro-3-(*cis*-4-{[(6-fluoroimidazo[1,2-*a*]pyridin-2-yl)methyl]amino}cyclohexyl)pyrido[ 2,3-*d*]pyrimidine-2,4(1*H*,3*H*)-dione | ¹H NMR (300 MHz, DMSO) δ 8.71 (dd, J = 4.6, 2.3 Hz, 1H), 8.57 (d, J = 3.1 Hz, 1H), 8.28 (dd, J = 7.8, 3.0 Hz, 1H), 7.84 (s, 1H), 7.57 - 7.48 (m, 3H), 7.39 (d, J = 7.7 Hz, 1H), 7.32 - 7.21 (m, 2H), 7.11 (d, J = 8.3 Hz, 1H), 6.88 (d, J = 2.5 Hz, 1H), 6.75 (dd, J = 8.4, 2.4 Hz, 1H), 4.83 - 4.71 (m, 1H), 3.80 (d, J = 5.0 Hz, 2H), 3.41 - 3.16 (m, 6H), 2.85 (s, 1H), 2.80 - 2.65 (m, 2H), 2.32 - 2.15 (m, 4H), 1.91 (s, 3H), 1.87 (s, 1H), 1.80 - 1.72 (m, 1H), 1.56 - 1.36 (m, 4H). | 735 |
| 329 | 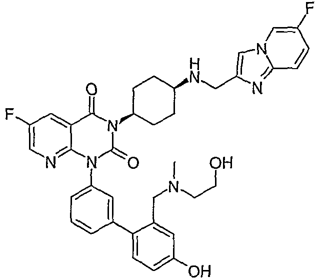 | 6-fluoro-3-(*cis*-4-{[(6-fluoroimidazo[1,2-*a*]pyridin-2-yl)methyl]amino}cyclohexyl)-1-(4'-hydroxy-2'-{[(2-hydroxyethyl)(methyl)amino]methyl }biphenyl-3-yl)pyrido[2,3-*d*]pyrimidine-2,4(1*H*,3*H*)-dione | ¹H NMR (400 MHz, DMSO) δ 9.60 - 9.45 (m, 1H), 9.07 - 8.93 (m, 2H), 8.93 (s, 1H), 8.60 (d, J = 2.8 Hz, 1H), 8.31 (dd, J = 7.6, 2.7 Hz, 1H), 8.12 (s, 1H), 7.73 - 7.65 (m, 1H), 7.62 (t, J = 7.8 Hz, 1H), 7.50 - 7.34 (m, 4H), 7.22 (d, J = 8.2 Hz, 1H), 7.13 (s, 1H), 6.98 (d, J = 8.5 Hz, 1H), 4.86 - 4.73 (m, 1H), 4.38 (s, 2H), 4.32 - 4.21 (m, 2H), 3.61 (s, 2H), 3.39 (s, 1H), 3.09 - 2.93 (m, 1H), 2.89 - 2.73 (m, 1H), 2.66 - 2.49 (m, 2H), 2.56 (s, 3H), 2.17 - 2.10 (m, 2H), 1.83 - 1.71 (m, 2H), 1.69 - 1.60 (m, 2H). | 682 |
| 330 | 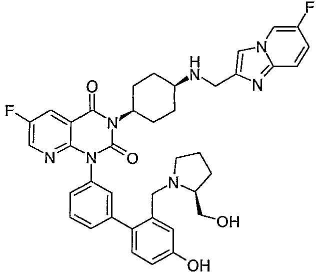 | 6-fluoro-3-(*cis*-4- {[(6-fluoroimidazo[1,2-a]pyridin-2-yl)methyl]amino}cyclohexyl)-1-(4'-hydroxy-2'-{[(2*S*)-2-(hydroxymethyl)pyrrolidin-1-yl]methyl}biphenyl-3-yl)pyrido[2,3-*d*]pyrimidine-2,4(1*H*,3*H*)-dione | ¹H NMR (400 MHz, DMSO) 5 9.47 - 9.34 (m, 1H), 9.14 - 9.00 (m, 2H), 8.96 (s, 1H), 8.60 (s, 1H), 8.31 (dd, J =7.7, 2.8 Hz, 1H), 8.16 (s, 1H), 7.72 (dd, J = 10.0, 5.1 Hz, 1H), 7.63 (t, J = 7.8 Hz, 1H), 7.49 (dd, J = 18.2, 2.3 Hz, 1H), 7.45 - 7.36 (m, 2H), 7.22 (d, J = 8.2 Hz, 1H), 7.14 (d, J = 1.8 Hz, 1H), 6.98 (dd, J = 8.5, 2.1 Hz, 1H), 4.85 - 4.77 (m, 1H), 4.48 - 4.22 (m, 2H), 4.40 (s, 2H), 3.52 (d, J = 3.3 Hz, 2H), 3.44 - 3.29 (m, 2H), 3.28 - 3.18 (m, 1H), 2.82 - 2.70 (m, 1H), 2.67 - 2.55 (m, 2H), 2.22 - 2.09 (m, 2H), 1.95 - 1.83 (m, 1H), 1.82 - 1.59 (m, 7H). | 708 |
| 331 | 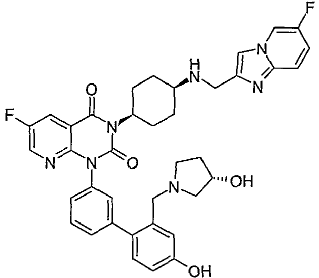 | 6-fluoro-3-(*cis*-4-{[(6-fluoroimidazo[1,2-*a*]pyridin-2-yl)methyl]amino}cyclohexyl)-1-(4-hydroxy-2'-{[(3*S*)-3-hydroxypyrrolidin-1-yl]methyl}biphenyl-3-yl)pyrido[2,3-*d*]pyrimidine-2,4(1*H*,3*H*)-dione | ¹H NMR (400 MHz, DMSO) δ 8.98 - 8.86 (m, 2H), 8.90 (dd, J = 4.4, 2.3 Hz, 1H), 8.59 (d, J = 3.1 Hz, 1H), 8.34 - 8.27 (m, 1H), 8.10 (s, 1H), 7.67 (dd, J = 10.0, 5.1 Hz, 2H), 7.61 (t, J = 7.7 Hz, 2H), 7.47 - 7.36 (m, 4H), 7.21 (d, J = 8.2 Hz, 1H), 7.11 (dd, J = 21.0, 2.3 Hz, 1H), 6.95 (dd, J = 8.5, 2.6 Hz, 1H), 4.85 - 4.74 (m, 1H), 4.37 (s, 2H), 4.30 (s, 2H), 3.61 - 3.29 (m, 3H), 3.14 - 3.06 (m, 1H), 2.93 - 2.71 (m, 2H), 2.65 - 2.53 (m, 2H), 2.19 - 2.08 (m, 2H), 2.06 - 1.87 (m, 2H), 1.80 - 1.61 (m, 6H). | 694 |
| 332 | 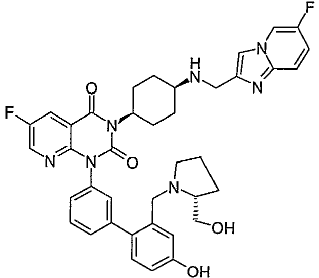 | 6-fluoro-3-(*cis*-4-{[(6-fluoroimidazo[1,2-*a*]pyridin-2-yl)methyl]amino}cyclohexyl)-1-(4'-hydroxy-2'-{[(2*R*)-2-(hydroxymethyl)pyrrolidin-1-yl]methyl}biphenyl-3-yl)pyrido[2,3-d]pyrimidine-2,4(1*H*,3*H*)-dione | ¹H NMR (400 MHz, DMSO) d 9.47 - 9.34 (m, 1H), 9.14 - 9.00 (m, 2H), 8.96 (s, 1H), 8.60 (s, 1H), 8.31 (dd, J = 7.7,2.8 Hz, 1H), 8.16 (s, 1H), 7.72 (dd, J = 10.0, 5.1 Hz, 1H), 7.63 (t, J = 7.8 Hz, 1H), 7.49 (dd, J = 18.2, 2.3 Hz, 1H), 7.45 - 7.36 (m, 2H), 7.22 (d, J = 8.2 Hz, 1H), 7.14 (d, J = 1.8 Hz, 1H), 6.98 (dd, J = 8.5, 2.1 Hz, 1H), 4.85 - 4.77 (m, 1H), 4.48 - 4.22 (m, 2H), 4.40 (s, 2H), 3.52 (d, J = 3.3 Hz, 2H), 3.44 - 3.29 (m, 2H), 3.28 - 3.18 (m, 1H), 2.82 - 2.70 (m, 1H), 2.67 - 2.55 (m, 2H), 2.22 - 2.09 (m, 2H), 1.95 - 1.83 (m, 1H), 1.82 - 1.59 (m, 7H) | 708 |

### Example 333

### N-{cis-4-[6-fluoro-1-[4'-hydroxy-2'-(morpholin-4-ylmethyl)biphenyl-3-yl]-2,4-dioxo-1,4-dihydropyrido[2,3-d]pyrimidin-3(2H)-yl]cyclohexyl}-2-piperidin-1-ylacetamide

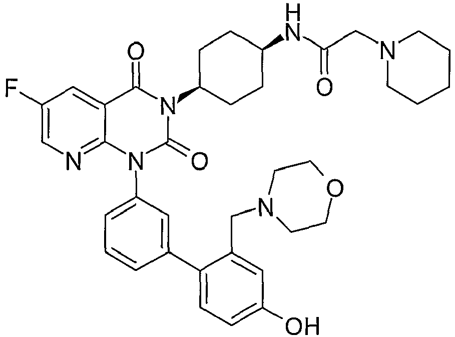

To a solution of 2-(piperidin-1-yl)acetic acid (0.052 g, 0.37 mmol) in NMP (4 mL) was added *N*-ethyldiisopropylamine (0.190 mL, 1.10 mmol) followed by HATU (0.167 g, 0.44 mmol) and the reaction stirred for 5 mins. 3-(*cis*-4-Aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H-*dione (0.2 g, 0.37 mmol) was then added and the reaction stirred for 3 h at room temperature. Water (20ml) was added and the reaction stirred for 5 mins. The resultant precipitate was isolated by filtration. The crude product was purified by preparative HPLC on a Phenomenex Gemini column using a 95-5% gradient of aqueous 0.2% ammonia in acetonitrile as eluent. The fractions containing the desired compound were evaporated to dryness to afford the title compound as a solid (70 mg).
¹H NMR (400 MHz, DMSO) δ 9.48 (s, 1H), 8.59 (d, J = 2.8 Hz, 1H), 8.26 (dd, J = 7.7, 2.8 Hz, 1H), 7.76 (d, J = 8.2 Hz, 1H), 7.58 - 7.57 (m, 1H), 7.54 - 7.50 (m, 1H), 7.42 (d, J = 7.7 Hz, 1H), 7.31 (dd, J = 8.1, 1.4 Hz, 1H), 7.11 (d, J = 8.2 Hz, 1H), 6.88 (d, J = 2.6 Hz, 1H), 6.75 (dd, J = 8.5, 2.6 Hz, 1H), 4.88 - 4.82 (m, 1H), 4.02 - 4.00 (m, 1H), 3.45 - 3.43 (m, 4H), 3.28 (s, 1H), 2.84 (s, 2H), 2.55 - 2.52 (m, 1H), 2.38 (s, 4H), 2.28 (s, 4H), 1.78 - 1.73 (m, 2H), 1.65 - 1.57 (m, 5H), 1.48 (s, 4H), 1.24 (s, 2H).
[M+H]+=671 (Multimode+)

### Example 334

### 6-fluoro-1-[4'-hydroxy-2'-(morpholin-4-ylmethyl)biphenyl-3-yl]-3-(cis-4-{[(6-methylimidazo[1,2-a]pyridin-2-yl)methyl]amino}cyclohexyl)pyrido[2,3-d]pyrimidine-2,4(1H,3H)-dione

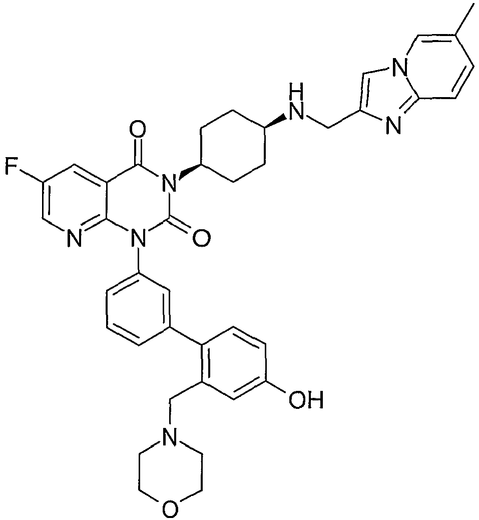

### Step (a) Ethyl 6-methylimidazo[1,2-a]pyridine-2-carboxylate

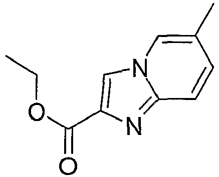

Ethyl bromopyruvate (10.04 g, 55.48 mmol) was added slowly to 5-methylpyridin-2-amine (4 g, 36.99 mmol) in ethanol (60 ml) at ambient temperature. The reaction mixture was stirred at reflux for 19 h. Ethyl bromopyruvate (2.321 ml, 18.49 mmol) was added and the reaction mixture was refluxed for a further 19 h. The solvents were evaporated and 1N HCl (100 mL) was added to the residue. The aqueous layer was washed with ethyl acetate, basified with aq. saturated sodium bicarbonate solution, and the product extracted into ethyl acetate. The combined organic phase was washed with brine and dried over anhydrous sodium sulfate, filtered and evaporated. The crude residue was purified by flash silica chromatography, elution gradient 40% ethyl acetate in DCM. Pure fractions were evaporated to dryness to afford the sub-title compound as a yellow solid (1.1 g). ¹H NMR (400 MHz, CDCl₃) δ 8.09 (s, 1H), 7.90 (d, 1H), 7.57 (d, J = 9.2 Hz, 1H), 7.09 (dd, 1H), 4.45 (q, 2H), 2.33 (d, 3H), 1.44 (t, 3H).
[M+H]+=205

### Step (b) 6-Methylimidazo[1,2-a]pyridin-2-yl)methanol

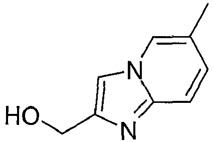

A solution of ethyl 6-methylimidazo[1,2-a]pyridine-2-carboxylate (1.11 g, 5.44 mmol) in tetrahydrofuran (20 mL) was added dropwise to lithium aluminium hydride (1M in THF) (5.44 mL, 5.44 mmol) at 5 °C and the reaction mixture was stirred at 5 °C for 1 h. The reaction was quenched by dropwise addition of ethyl acetate. After allowing to warm to room temperature, the mixture was partitioned between DCM and water. The organic layer was dried over anhydrous magnesium sulfate, filtered and concentrated to afford the sub-title compound as a pale brown solid (0.84 g).
¹H NMR (400 MHz, CDCl₃) δ 7.87 (s, 1H), 7.47 - 7.44 (m, 2H), 7.02 (dd, J = 9.1, 1.2 Hz, 1H), 4.82 (s, 2H), 2.31 (s, 3H).
[M+H]+=163

### Step (c) 6-Methylimidazo[1,2-a]pyridine-2-carbaldehyde

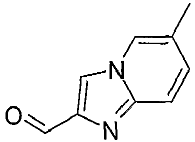

To a solution of (6-methylimidazo[1,2-a]pyridin-2-yl)methanol (0.84 g, 5.18 mmol) in DCM (15 ml) was added manganese dioxide (0.450 g, 5.18 mmol) and the reaction mixture was stirred under nitrogen at ambient temperature for 1.5 h and left to stand for a further 18 h. The reaction mixture was heated to reflux for 3 h before further manganese dioxide (2.5 g, 28.76 mmol) and DCM (15.00 ml) was added. The reaction mixture was stirred at reflux for 3 h and left to stand at room temperature for 67 h. The mixture was filtered through a pad of celite, washing with DCM. The filtrate was concentrated, triturated with ether and filtered to give the sub title compound as a beige solid (0.283 g). ¹H NMR (400 MHz, CDCl₃) δ 10.13 (s, 1H), 8.07 (s, 1H), 7.94 (d, J = 1.3 Hz, 1H), 7.58 (d, J = 9.5 Hz, 1H), 7.14 (dd, J = 9.5, 1.5 Hz, 1H), 2.38 (s, 3H).
[M+H]=161

### Step (d) 6-fluoro-1-[4'-hydroxy-2'-(morpholin-4-ylmethyl)biphenyl-3-yl]-3-(cis-4-{[(6-methylimidazo[1,2-a]pyridin-2-yl)methyl]amino}cyclohexyl)pyrido[2,3-d]pyrimidine-2,4(1H,3H)-dione

To 3-(*cis*-4-aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (0.25 g, 0.46 mmol) was added DCE (20 mL) and NMP (1 mL) and the suspension was stirred vigorously for 1.5 h. 6-Methylimidazo[1,2-a]pyridine-2-carbaldehyde (0.066 g, 0.41 mmol) was added and the reaction mixture was stirred at ambient temperature for 1 h. Four drops of acetic acid were added and the reaction mixture was stirred at ambient temperature for 17 h. Sodium triacetoxyborohydride (0.194 g, 0.92 mmol) was added and the reaction mixture was stirred at ambient temperature for a further 3 h and left to stand at ambient temperature for 19 h. The reaction mixture was diluted with DCM and the organics washed with saturated aqueous sodium bicarbonate, brine, dried over magnesium sulfate, filtered and the solvent evaporated. The crude product was purified by preparative HPLC on a Phenomenex Gemini column using a 75-5% gradient of aqueous 0.2% ammonia in acetonitrile as eluent. The fractions containing the desired compound were lyophilised to afford the title compound as a white solid (0.160 g).
¹H NMR (400 MHz, DMSO) δ 8.58 (d, J = 3.1 Hz, 1H), 8.30 - 8.23 (m, 2H), 7.70 (s, 1H), 7.59 - 7.49 (m, 2H), 7.43 - 7.34 (m, 2H), 7.31 (d, 1H), 7.13 (d, 1H), 7.03 (dd, 1H), 6.88 (d, 1H), 6.75 (dd, 1H), 4.78 (t, 1H), 3.77 (d, 2H), 3.43 (s, 4H), 3.28 (s, 2H), 2.85 (s, 1H), 2.80 - 2.67 (m, 2H), 2.25 (s, 7H), 1.92 - 1.85 (m, 2H), 1.72 (s, 1H), 1.53 - 1.38 (m, 4H).
[M+H]+=690 (MultiMode+)

### Example 335

### 6-fluoro-1-[4'-hydroxy-2'-(morpholin-4-ylmethyl)biphenyl-3-yl]-3-(cis-4-{[(5-methylimidazo[1,2-a]pyridin-2-yl)methyl]amino}cyclohexyl)pyrido[2,3-d]pyrimidine-2,4(1H,3H)-dione

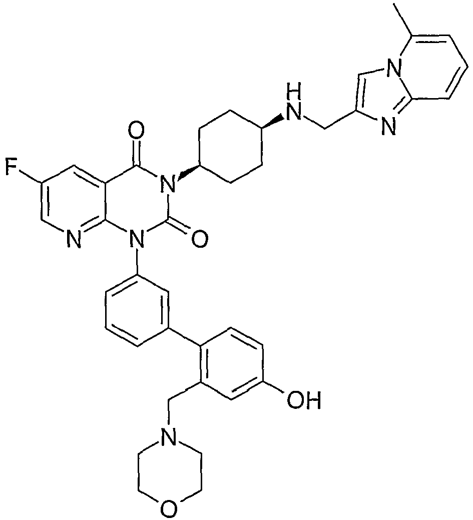

To 3-(*cis*-4-aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (0.25 g, 0.46 mmol) in DCE (20 mL) was added 5-methylimidazo[1,2-a]pyridine-2-carbaldehyde (0.066 g, 0.41 mmol) and four drops of acetic acid. The reaction mixture was stirred at ambient temperature for 17 h. Sodium triacetoxyborohydride (0.194 g, 0.92 mmol) was added and the reaction mixture was stirred at ambient temperature for 6.5 h. The reaction mixture was diluted with DCM and the organics washed with saturated aqueous sodium bicarbonate, brine, dried over magnesium sulfate and the solvent evaporated. The crude product was purified by preparative HPLC on a Phenomenex Gemini column using a 75-5% gradient of aqueous 0.2% ammonia in acetonitrile as eluent. The fractions containing the desired compound were lyophilised to afford the title compound as a white solid (0.161 g).
¹H NMR (400 MHz, DMSO) δ 9.49 (s, 1H), 8.58 (d, 1H), 8.27 (dd, 1H), 7.71 (s, 1H), 7.58 - 7.49 (m, 2H), 7.44 - 7.35 (m, 2H), 7.33 - 7.29 (m, 1H), 7.18 - 7.11 (m, 2H), 6.88 (d, 1H), 6.75 (dd, 1H), 6.70 (d, 1H), 4.80 (t, 1H), 3.83 (s, 2H), 3.42 (t, 4H), 3.27 (s, 2H), 2.89 (s, 1H), 2.84 - 2.71 (m, 2H), 2.53 (s, 3H), 2.26 (s, 4H), 1.90 (d, 2H), 1.77 (d, 1H), 1.54 - 1.38 (m, 4H).
[M+H]+=690 (Multimode+)

### Example 336

### 6-fluoro-1-[4'-hydroxy-2'-(morpholin-4-ylmethyl)biphenyl-3-yl]-3-(cis-4-{[(3-methylimidazo[1,2-a]pyridin-2-yl)methyl]amino}cyclohexyl)pyrido[2,3-d]pyrimidine-2,4(1H,3H)-dione

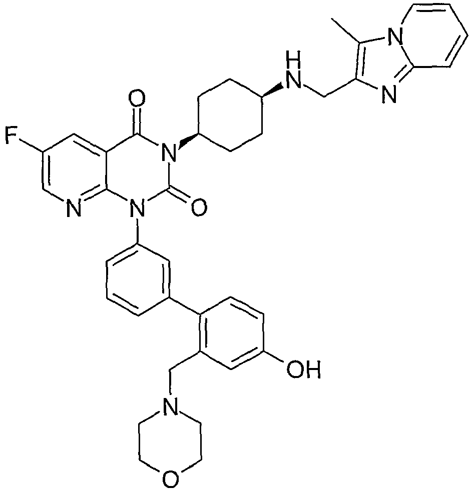

### Step (a) Methyl and ethyl 3-methylimidazo[1,2-a]pyridine-2-carboxylate

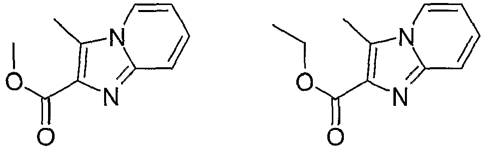

To a solution of methyl 3-bromo-2-oxobutanoate (6.23 g, 31.95 mmol) in ethanol (50 mL) was added pyridin-2-amine (2.506 g, 26.62 mmol) and the mixture was heated at reflux for 6 h followed by stirring at ambient temperature for 2 days. The suspension was heated at reflux for a further 6 h followed by stirring at ambient temperature for 17 h. The solvents were evaporated and the crude product was purified by flash silica chromatography, eluting with 3% (10% methanolic ammonia in 90% methanol) in 97% DCM. Pure fractions were evaporated to dryness to afford the sub-title compounds as a orange oil as a mixture of the methyl and ethyl esters (2.48 g).
¹H NMR (400 MHz, CDCl₃) δ 7.91 (dd, 1H), 7.68 - 7.63 (m, 1H), 7.27 - 7.19 (m, 1H), 6.90 (tdd, 1H), 4.47 (q, 2H), 4.00 (s, 3H), 2.81 (s, 3H), 1.46 (t, 3H).
[M+H]+=191 corresponding to methyl ester; [M+H]+=204 corresponding to ethyl ester

### Step (b) (3-Methylimidazo[1,2-a]pyridin-2-yl)methanol

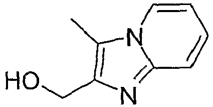

A solution of methyl and ethyl 3-methylimidazo[1,2-a]pyridine-2-carboxylate (2.48 g, 12.14 mmol) in tetrahydrofuran (40 mL) was added dropwise to lithium aluminium hydride (1M in THF) (12.14 mL, 12.14 mmol) at 5 °C and the reaction mixture was stirred at 5 °C for 1 h. A saturated aqueous solution of Rochelle's salt was added dropwise to the reaction mixture and the mixture was filtered. The filtrate was dried over magnesium sulfate, filtered and the solvents evaporated to afford the sub title compound (1.590 g).
¹H NMR (400 MHz, CDCl₃) δ 7.83 (d, 1H), 7.56 (d, 1H), 7.15 (ddd, 1H), 6.82 (td, 1H), 4.85 (s, 2H), 2.49 (s, 3H).
[M+H]+=163

### Step (c) 3-Methylimidazo[1,2-a]pyridine-2-carbaldehyde

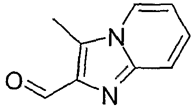

Manganese dioxide (8.52 g, 98.03 mmol) was added portionwise over 5 min to a solution of (3-methylimidazo[1,2-a]pyridin-2-yl)methanol (1.59 g, 9.80 mmol) in DCM (100 mL) under nitrogen. The reaction mixture was heated to reflux for 3 h. The mixture was filtered through a pad of celite, followed by further filtration of the filtrate through 3 glass fibre filters, washing with DCM. The filtrate was concentrated to afford the sub title compound as a yellow solid (1.20 g).
¹H NMR (400 MHz, CDCl₃) δ 10.26 (s, 1H), 7.95 (d, 1H), 7.66 (d, 1H), 7.30 - 7.25 (m, 1H), 6.94 (dd, 1H), 2.80 (s, 3H).
[M+H]+=161

### Step (d) 6-fluoro-1-[4'-hydroxy-2'-(morpholin-4-ylmethyl)biphenyl-3-yl]-3-(cis-4-{[(3-methylimidazo[1,2-a]pyridin-2-yl)methyl]amino}cyclohexyl)pyrido[2,3-d]pyrimidine-2,4(1H,3H)-dione

To -(*cis*-4-aminocyclohexyl)-6-fluoro-1-[4'-hydroxy-2'-(morpholin-4-ylmethyl)biphenyl-3-yl]pyrido[2,3-*d*]pyrimidine-2,4(1*H*,3*H*)-dione (0.25 g, 0.46 mmol) in DCE (20 mL) was added 3-methylimidazo[1,2-a]pyridine-2-carbaldehyde (0.066 g, 0.41 mmol) and 4 drops of acetic acid. The reaction mixture was stirred at ambient temperature for 19h. Sodium triacetoxyborohydride (0.194 g, 0.92 mmol) was added and the reaction mixture was stirred at ambient temperature for 5 h. The reaction mixture was diluted with DCM and the organics washed with saturated aqueous sodium bicarbonate, brine, dried over magnesium sulfate and the solvent evaporated. The crude product was purified by preparative HPLC on a Phenomenex Gemini column using a 75-5% gradient of aqueous 0.2% ammonia in acetonitrile as eluent. The fractions containing the desired compound were lyophilised to afford the title compound as an off white solid (0.120g).
¹H NMR (400 MHz, DMSO) δ 9.50 (s, 1H), 8.58 (d, 1H), 8.27 (dd, 1H), 8.19 (d, 1H), 7.57 - 7.45 (m, 3H), 7.41 (d, 1H), 7.30 (d, 1H), 7.19 - 7.11 (m, 2H), 6.91 - 6.86 (m, 2H), 6.76 (dd, 1H), 4.77 (t, 1H), 3.79 (s, 2H), 3.43 (t, 4H), 3.28 (s, 2H), 2.83 (s, 1H), 2.79 - 2.68 (m, 2H), 2.46 (s, 3H), 2.27 (s, 4H), 1.93 - 1.85 (m, 2H), 1.64 (s, 1H), 1.53 - 1.38 (m, 4H).
[M+H]+=690 (Multimode+)

### Example 337

### 6-fluoro-3-(cis-4-{[(6-fluoroimidazo[1,2-a]pyridin-2-yl)methyl]amino}cyclohexyl)-1-[5'-hydroxy-2'-(morpholin-4-ylmethyl)biphenyl-3-yl]pyrido[2,3-d]pyrimidine-2,4(1H,3H)-dione

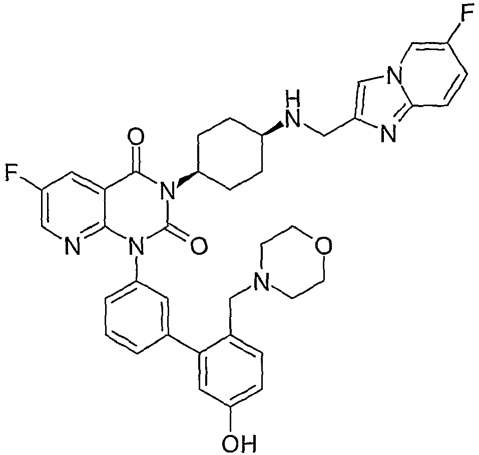

### Step (a) tert-butyl {cis-4-[6-fluoro-1-(2'-formyl-5'-hydroxybiphenyl-3-yl)-2,4-dioxo-1,4-dihydropyrido[2,3-d]pyrimidin-3(2H)-yl]cyclohexyl} carbamate

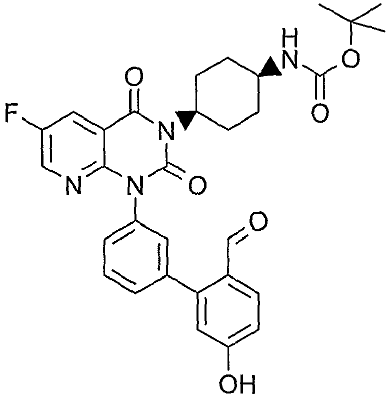

A mixture of palladium diacetate (19 mg, 0.09 mmol) and dicyclohexyl(2',6'-dimethoxybiphenyl-2-yl)phosphine (71 mg, 0.17 mmol) was stirred for 10 mins in a solvent of dry acetonitrile (16 ml). To this mixture was added consecutively, a solution of potassium carbonate (0.357 g, 2.58 mmol) in water (5 ml), 2-chloro-4-hydroxybenzaldehyde (0.135 g, 0.86 mmol) and *tert-*butyl {*cis*-4-[6-fluoro-2,4-dioxo-1-[3-(4,4,5,5-tetramethyl-1,3,2-dioxaborolan-2-yl)phenyl]-1,4-dihydxopyrido[2,3-*d*]pyrimidin-3(2*H*)-yl]cyclohexyl}carbamate (0.5 g, 0.86 mmol). The resultant mixture was stirred and heated at 70 °C for 4 h. The mixture was allowed to cool to room temperature and concentrated in vacuo. The crude product was added to a silica gel column and was eluted with 30% to 50% ethyl acetate : isohexane mixture. The pure fractions were combined and evaporated to dryness to give the sub-title compound as a pale yellow solid (0.255 g).
¹H NMR (300 MHz, CDCl₃) δ 9.87 (s, 1H), 8.33 - 8.27 (m, 1H), 8.13 (dd, J = 6.8, 2.6 Hz, 1H), 7.70 (d, J = 7.5 Hz, 1H), 7.59 (t, J = 7.7 Hz, 1H), 7.49 - 7.42 (m, 1H), 7.33 (d, J = 7.9 Hz, 1H), 7.27 - 7.22 (m, 1H), 6.90 - 6.81 (m, 2H), 4.99 - 4.80 (m, 2H), 3.84 (s, 1H), 2.59 - 2.40 (m, 2H), 1.90 - 1.71 (m, 2H), 1.65 - 1.46 (m, 4H), 1.35 (s, 9H).
[M+H]+=646

### Step (b) 3-(cis-4-aminocyclohexyl)-6-fluoro-1-[5'-hydroxy-2'-(morpholin-4-ylmethyl)biphenyl-3-yl]pyrido[2,3-d]pyrimidine-2,4(1H,3H)-dione

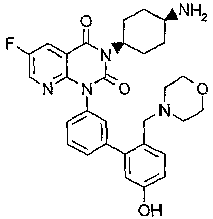

A solution of *tert*-butyl {*cis*-4-[6-fluoro-1-(2'-formyl-5'-hydroxybiphenyl-3-yl)-2,4-dioxo-1,4-dihydropyrido[2,3-*d*]pyrimidin-3(2*H*)-yl]cyclohexyl}carbamate (250 mg, 0.44 mmol) and morpholine (0.042 mL, 0.48 mmol) was stirred for 15 mins and sodium triacetoxyborohydride (101 mg, 0.48 mmol) was added. The mixture was stirred for 2 h. Morpholine (20 mg) was added and after 15 mins sodium triacetoxyborohydride (50 mg). The reaction mixture was stirred for 1 h. Trifluoroacetic acid (1 mL, 12.98 mmol) was added and the mixture stirred for a further 2 h then concentrated *in vacuo* to leave a solid (0.280 g).
[M+H]+=546

### Step (c) 6-fluoro-3-(cis-4-{[(6-fluoroimidazo[1,2-a]pyridin-2-yl)methyl]amino}cyclohexyl)-1-[5'-hydroxy-2'-(morpholin-4-ylmethyl)biphenyl-3-yl]pyrido[2,3-d]pyrimidine-2,4(1H,3H)-dione

A solution of 3'-(3-((1s,4s)-4-aminocyclohexyl)-6-fluoro-2,4-dioxo-3,4-dihydropyrido[2,3-d]pyrimidin-1(2H)-yl)-5-hydroxybiphenyl-2-carbaldehyde (250 mg, 0.53 mmol) and 6-fluoroimidazo[1,2-a]pyridine-2-carbaldehyde (104 mg, 0.63 mmol) in dichloromethane (20 mL) was stirred for 15 mins and sodium triacetoxyborohydride (134 mg, 0.63 mmol) was added and stirred for 2h. Methanol (1 ml) was added, stirred for 2h and concentrated in vacuo to leave a solid. The crude product was purified by preparative HPLC on a Phenomenex Gemini column using a 83% gradient of aqueous 0.1% trifluoroacetic acid in acetonitrile as eluent. The fractions containing the desired compound were evaporated to dryness to afford the title compound as a white solid (79 mg).
¹H NMR (300 MHz, DMSO) δ 10.19 - 10.00 (m, 1H), 9.01 - 8.82 (m, 2H), 8.59 (s, 1H), 8.33 (dd, J = 7.7, 1.2 Hz, 1H), 8.11 (s, 1H), 7.71 - 7.59 (m, 2H), 7.55 - 7.40 (m, 5H), 6.91 (d, J = 8.7 Hz, 1H), 6.78 (s, 1H), 4.84 - 4.71 (m, 1H), 4.37 (s, 2H), 4.21 (s, 2H), 3.83 - 3.67 (m, 2H), 3.65 - 3.49 (m, 2H), 3.43 - 3.32 (m, 1H), 3.21 - 3.03 (m, 2H), 2.72 - 2.53 (m, 4H), 2.19 - 2.04 (m, 2H), 1.85 - 1.59 (m, 4H).
[M+H]+=694 (Multimode+)

### Example 338

### 6-fluoro-3-(cis-4-{[(6-fluoroimidazo[1,2-a]pyridin-2-yl)methyl]amino}cyclohexyl)-1-[4'-(1,4-oxazepan-4-ylmethyl)biphenyl-3-yl]pyrido[2,3-d]pyrimidine-2,4(1H,3H)-dione

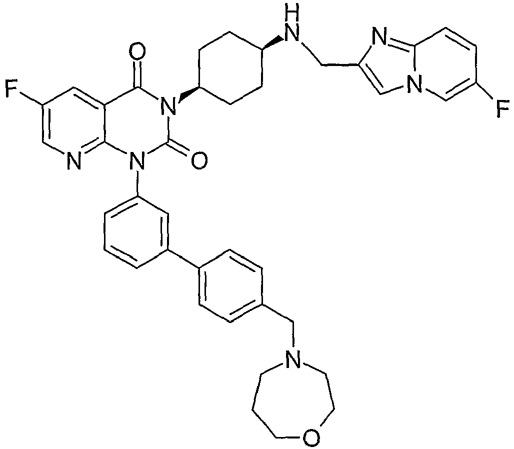

3-(*cis*-4-Aminocyclohexyl)-6-fluoro-1-[4'-(1,4-oxazepan-4-ylmethyl)biphenyl-3-yl]pyrido[2,3-*d*]pyrimidine-2,4(1*H*,3*H*)-dione (200 mg, 0.37 mmol) in DCE (2 mL) was treated with 6-fluoroimidazo[1,2-a]pyridine-2-carbaldehyde (75 mg, 0.46 mmol) and stirred for 15 min at room temperature. Sodium triacetoxyborohydride (156 mg, 0.74 mmol) was added and the reaction was stirred at room temperature overnight. The reaction was diluted with DCM (3 ml), water (2 ml) and saturated aqueous sodium hydrogen carbonate solution (3 ml) was added and the mixture was stirred vigorously for 15 min. The organic phase was separated and evaporated. The residue was taken in a little acetonitrile, filtered and purified by preparative HPLC on a ACE 5C8 column using a 95-25% gradient of aqueous 0.2% aqueous trifluoroacetic acid in acetonitrile as eluent. The product containing fractions were combined and evaporated to give the title compound ditrifluoroacetate salt as a fluffy cream powder (78 mg).
¹H NMR (400 MHz, CDCl₃) δ 8.37 (d, J = 3.1 Hz, 1H), δ.31 (s, 1H), 8.24 (s, 1H), 8.18 (dd, J = 7.2, 3.1 Hz, 1H), 7.78 (dd, J = 10.0, 4.6 Hz, 1H), 7.69 - 7.62 (m, 3H), 7.59 (t, J = 7.8 Hz, 1H), 7.52 - 7.45 (m, 4H), 7.29 - 7.27 (m, 1H), 5.13 - 4.98 (m, 1H), 4.55 (s, 2H), 4.29 (s, 2H), 3.96 - 3.85 (m, 2H), 3.82 (t, J = 6.3 Hz, 2H), 3.60 - 3.52 (m, 1H), 2.78 - 2.62 (m, 2H), 2.45 - 2.34 (m, 3H), 1.92 - 1.76 (m, 4H). Other protons obscured by solvent peaks.
[M+H]+=692 (Multimode+)

### Example 339

### 3-(cis-4-{bis[(6-fluoroimidazo[1,2-a]pyridin-2-yl)methyl]amino}cyclohexyl)-6-fluoro-1-[4'-(1,4-oxazepan-4-ylmethyl)biphenyl-3-yl]pyrido[2,3-d]pyrimidine-2,4(1H,3H)-dione

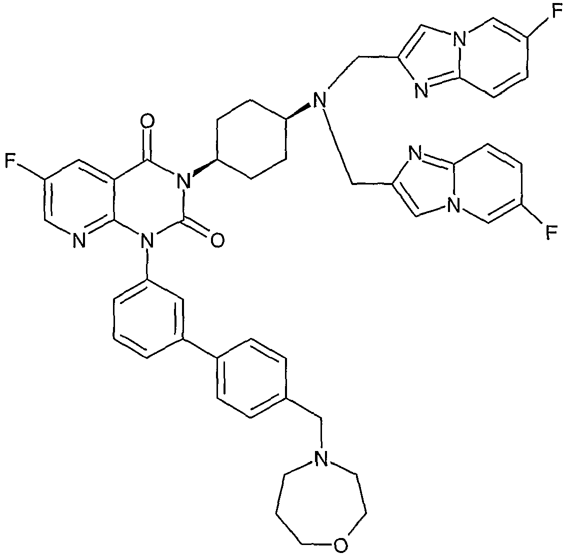

Also isolated from the reaction to prepare example 338 was the title compound ditrifluoroacetate salt as a white powder (19 mg).
¹H NMR (400 MHz, CDCl₃) δ 8.49 (s, 2H), 8.40 (d, J = 3.1 Hz, 1H), 8.26 (dd, J = 6.9, 3.1 Hz, 1H), 8.19 (s, 2H), 7.90 - 7.83 (m, 3H), 7.74 (t, J = 7.6 Hz, 1H), 7.71 (d, J = 8.2 Hz, 2H), 7.61 - 7.59 (m, 1H), 7.53 (d, J = 8.2 Hz, 2H), 7.47 (dt, J = 2.1, 8.7 Hz, 2H), 7.39 (d, J = 8.5 Hz, 1H), 5.26 - 5.14 (m, 1H), 4.29 (s, 2H), 4.14 (s, 4H), 3.96 - 3.89 (m, 2H), 3.83 (t, J = 6.3 Hz, 2H), 2.97 - 2.91 (m, 1H), 2.89 - 2.75 (m, 2H), 2.32 (d, J = 14.6 Hz, 3H), 1.79 - 1.58 (m, 5H).
[M+H]+=840 (Multimode+)

### Example 340

### N-{4-[2-(diethylamino)ethoxy]benzyl}-N-{cis-4-[6-fluoro-1-{4'-[(4-isopropylpiperazin-1-yl)methyl]biphenyl-3-yl}-2,4-dioxo-1,4-dihydropyrido[2,3-d]pyrimidin-3(2H)-yl]cyclohexyl} acetamide

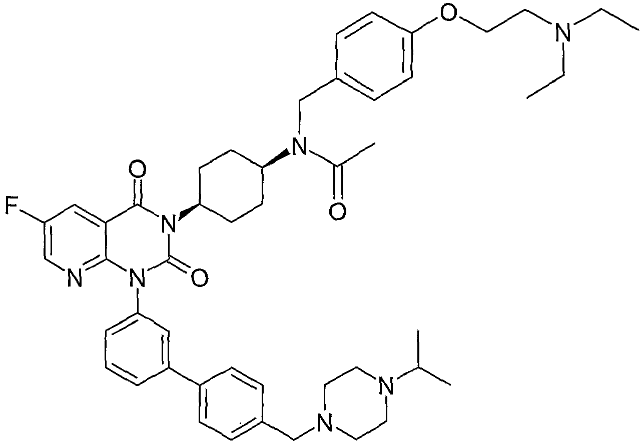

3-(*cis*-4-Aminocyclohexyl)-6-fluoro-1-{4'-[(4-isopropylpiperazin-1-yl)methyl]biphenyl-3-yl}pyrido[2,3-*d*]pyrimidine-2,4(1*H*,3*H*)-dione (200 mg, 0.35 mmol) in DCE (2 mL) was treated with 4-(2-(diethylamino)ethoxy)benzaldehyde (97 mg, 0.44 mmol) at room temperature for 15 min, then sodium triacetoxyborohydride (149 mg, 0.70 mmol) was added and the whole mixture was stirred at room temperature for 3 days. The reaction was diluted with DCM (3 ml) and water (2 ml), and saturated aqueous sodium bicarbonate solution (3 ml) was added and the mixture was stirred vigorously for 15 min. The organic phase was separated and evaporated and the residue was taken up in acetonitrile (3 mL) and treated with acetyl chloride (0.031 mL, 0.44 mmol) and triethylamine (0.073 mL, 0.53 mmol). The mixture was stirred at room temperature for 24 h then diluted with DCM, and stirred well with water and saturated aqueous sodium bicarbonate solution. The organic phase was separated and evaporated. The crude material was purified by preparative HPLC on a ACE 5C8 column using a 95-5% gradient of 0.2% aqueous ammonia in acetonitrile as eluent then by preparative HPLC on a ACE 5C8 column using a 95-25% gradient of 0.2% aqueous trifluoroacetic acid in acetonitrile as eluent. The fractions containing the desired compound were evaporated to afford the title compound ditrifluoroacetate salt as a white solid (36 mg).
¹H NMR (400 MHz, CDCl₃) δ 8.37 (d, J = 2.8 Hz, 1H), 8.22 (dd, J = 7.2, 3.1 Hz, 1H), 7.74 (d, J = 7.9 Hz, 1H), 7.72 - 7.64 (m, 4H), 7.55 - 7.48 (m, 4H), 7.40 - 7.30 (m, 1H), 7.14 (d, J = 8.2 Hz, 2H), 6.91 - 6.73 (m, 3H), 5.17 - 5.06 (m, 1H), 4.86 (s, 2H), 4.35 (s, 2H), 4.25 (s, 2H), 3.79 - 3.41 (m, 12H), 3.40 - 3.17 (m, 4H), 2.64 - 2.45 (m, 2H), 2.15 - 1.93 (m, 4H), 1.82 - 1.60 (m, 4H), 1.42 - 1.30 (m, 12H).
[M+H]+=818 (Multimode+)

### Example 341

### 3-(cis-4-{bis[(1-acetyl-1H-indol-3-yl)methyl]amino}cyclohexyl)-6-fluoro-1-{4'-[(4-isopropylpiperazin-1-yl)methyl]biphenyl-3-yl}pyrido[2,3-d]pyrimidine-2,4(1H,3H)-dione

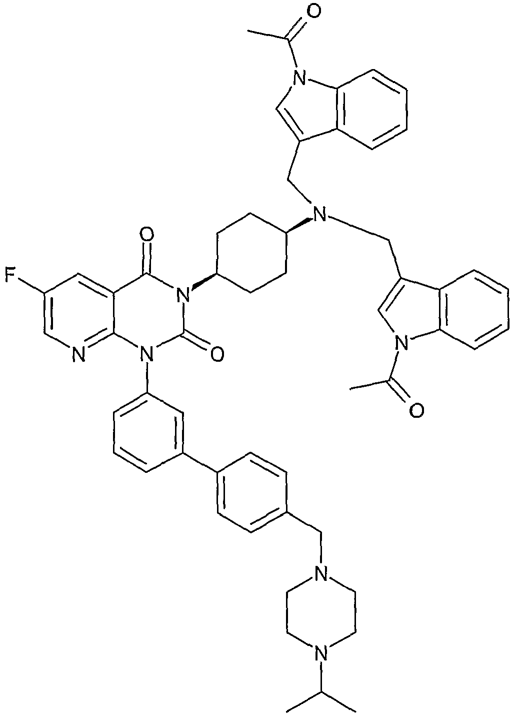

3-(*cis*-4-Aminocyclohexyl)-6-fluoro-1-{4'-[(4-isopropylpiperazin-1-yl)methyl]biphenyl-3-yl}pyrido[2,3-*d*]pyrimidine-2,4(1*H*,3*H*)-dione (200 mg, 0.35 mmol) in DCE (2 mL) was added to 1-acetyl-1H-indole-3-carbaldehyde (98 mg, 0.53 mmol) and stirred for 15 min at room temperature. Sodium triacetoxyborohydride (149 mg, 0.70 mmol) was added and the reaction was left at room temperature overnight. Water (2 ml), saturated aqueous sodium bicarbonate solution (3 ml) and DCM (2 ml) were added and the mixture was stirred 15 min, then separated. The organic solution was evaporated, and the crude material was purified by by preparative HPLC on a ACE 5C8 column using a 75-5% gradient of 0.2% aqueous trifluoroacetic acid in acetonitrile as eluent. The fractions containing the desired compound were evaporated to afford the title compound ditrifluoroacetate salt as a pale pink solid (51 mg).
¹H NMR (400 MHz, DMSO) δ 9.93 - 9.84 (m, 1H), 8.64 - 8.58 (m, 1H), 8.33 (dd, J = 7.7, 2.8 Hz, 1H), 8.27 - 8.19 (m, 1H), 8.13 - 8.03 (m, 2H), 7.93 - 7.84 (m, 1H), 7.83 - 7.66 (m, 7H), 7.57 - 7.43 (m, 3H), 7.37 - 7.26 (m, 2H), 7.16 - 7.07 (m, 1H), 7.05 - 6.99 (m, 1H), 5.11 - 4.99 (m, 1H), 4.80 - 4.68 (m, 1H), 4.68 - 4.58 (m, 1H), 3.24 - 2.99 (m, 6H), 2.84 - 2.61 (m, 2H), 2.35 - 2.20 (m, 1H), 2.17 - 1.99 (m, 4H), 1.23 (d, J = 6.4 Hz, 6H). Other resonances obscured by DMSO and water signals.
[M+H]+=913 (Multimode+)

### Example 342

### 6-fluoro-N-{cis-4-[6-fluoro-1-{4'-[2-(isopropylamino)ethoxy]biphenyl-3-yl}-2,4-dioxo-1,4-dihydropyrido[2,3-d]pyrimidin-3(2H)-yl]cyclohexyl}imidazo[1,2-a]pyridine-2-carboxamide

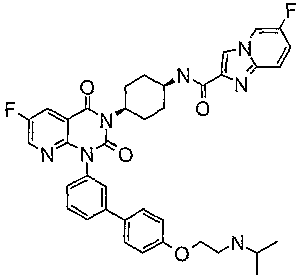

*N*-{*cis*-4-[1-[4'-(2-Chloroethoxy)biphenyl-3-yl]-6-fluoro-2,4-dioxo-1,4-dihydropyrido[2,3-d]pyrimidin-3(2*H*)-yl]cyclohexyl}-6-fluoroimidazo[1,2-a]pyridine-2-carboxamide (0.2 g, 0.30 mmol), [potassium carbonate (0.412 g, 2.98 mmol), potassium iodide (0.05 g, 0.30 mmol) and 2-aminopropane (1 mL, 11.74 mmol) were suspended in acetonitrile (5 mL) and heated to 80 °C for 5 h. A further portion of 2-aminopropane (1 mL, 11.74 mmol) was added and the reaction heated a further 16 h then poured into water and extracted with ethyl acetate. The combined extracts were washed with brine and the solvent evaporated. The crude product was purified by preparative HPLC on a Sunfire column using a 95-50% gradient of aqueous 0.1% trifluoroacetic acid in acetonitrile as eluent. The fractions containing the desired compound were evaporated to dryness to afford the title compound trifluoroacetate salt as a pale pink solid (30 mg).
¹H NMR (500 MHz, DMSO) δ 8.82 (d, J = 2.3 Hz, 1H), 8.66 (s, 2H), 8.59 (d, J = 3.0 Hz, 1H), 8.40 (s, 1H), 8.34 (dd, J = 7.7, 3.0 Hz, 1H), 7.76 - 7.65 (m, 5H), 7.59 (t, J = 7.9 Hz, 1H), 7.48 (dd, J = 18.4, 2.0 Hz, 1H), 7.34 (d, J = 8.0 Hz, 1H), 7.10 (d, J = 8.7 Hz, 2H), 4.88 (t, J = 11.9 Hz, 1H), 4.28 (t, J = 5.0 Hz, 2H), 4.16 (d, J = 3.0 Hz, 1H), 3.44 - 3.34 (m, 3H), 2.66 - 2.57 (m, 2H), 2.02 (d, J = 13.0 Hz, 2H), 1.76 - 1.63 (m, 4H), 1.27 (d, J = 6.3 Hz, 6H).
[M+H]+=694 (Multimode+)

### Example 343

### 1-{4'-[(dimethylamino)methyl]biphenyl-3-yl}-6-fluoro-3-{cis-4-[(imidazo[1,2-a]pyridin-2-ylmethyl)amino]cyclohexyl}pyrido[2,3-d]pyrimidine-2,4(1H,3H)-dione

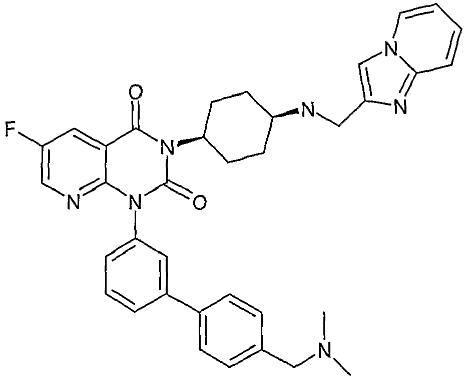

To a solution of imidazo[1,2-a]pyridine-2-carbaldehyde (71 mg, 0.49 mmol) dissolved in DCE (20ml) was added 3-(*cis*-4-aminocyclohexyl)-1-{4'-[(dimethylamino)methyl]biphenyl-3-yl}-6-fluoropyrido[2,3-d]pyrimidine-2,4(1*H*,3*H*)-dione (0.238 g, 0.49 mmol), followed by sodium triacetoxyborohydride (0.103 g, 0.49 mmol). The resulting mixture was stirred for 4 h at room temperature. The reaction mixture was diluted with DCM (10 mL), and washed with saturated brine (20 mL). The organic layer was dried over sodium sulfate, filtered and evaporated to afford crude product. The crude product was purified by preparative HPLC on a Waters X-Terra column using a 50-5% gradient of aqueous 0.1% trifluoroacetic acid in acetonitrile as eluent. The fractions containing the desired compound were evaporated to dryness to afford the title compound ditrifluoroacetate salt as a colourless solid (0.351 g).
¹H NMR (300 MHz, DMSO) δ 10.08 (s, 1H), 8.96 (s, 1H), 8.68 (d, J = 6.9 Hz, 1H), 8.61 (d, J = 3.1 Hz, 1H), 8.31 (dd, J = 7.7, 3.1 Hz, 1H), 8.13 (s, 1H), 7.87 - 7.74 (m, 4H), 7.67 - 7.56 (m, 4H), 7.46 - 7.37 (m, 2H), 7.04 (t, J = 6.8 Hz, 1H), 4.82 (t, J = 11.5 Hz, 1H), 4.44 - 4.27 (m, 4H), 3.40 (s, 1H), 2.76 (d, J = 3.7 Hz, 6H), 2.67 - 2.52 (m, 3H), 2.20 - 2.07 (m, 2H), 1.84 - 1.58 (m, 4H).
[M+H]+=618 (Multimode+)

### Example 344

### 1-amino-N-[cis-4-(1-biphenyl-3-yl-6-fluoro-2,4-dioxo-1,4-dihydropyrido[2,3-d]pyrimidin-3 (2H)-yl)cyclohexyl] cyclopropanecarboxamide

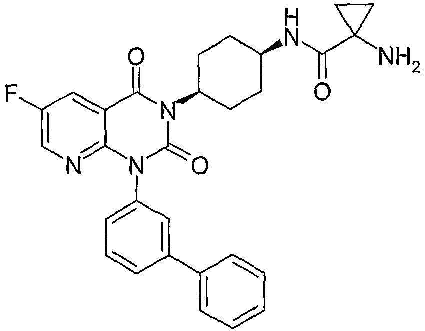

To a mixture of 1-BOC-amino-cyclopropanecarboxylic acid (68 mg, 0.34 mmol) and Hünigs base (0.194 g, 1.5 mmol) in dry DMF (5 ml) was added HATU (128 mg, 0.34mmol). The mixture was allowed to stir for 10 mins at room temperature. To this mixture was added 3-(*cis*-4-aminocyclohexyl)-1-biphenyl-3-yl-6-fluoropyrido[2,3-*d*]pyrimidine-2,4(1*H*,3*H*)-dione (115mg, 0.31mmol) and the mixture stirred overnight. The mixture was poured into water and the solid collected. The above solid was then dissolved in 4N HCl/dioxane (2 ml) and left for 1 hour, evaporated to dryness and the crude product was purified by preparative HPLC on a Waters X-Terra column using a 70-50% gradient of aqueous 0.1% ammonia in acetonitrile as eluent. The fractions containing the desired compound were evaporated to dryness to afford the title compound (65 mg).
¹H NMR (300 MHz, DMSO) δ 8.68 (m, 2H), 8.60 (m1H), 8.32 (m,1H), 7.70 (m, 4H), 7.49 (m, 2H), 7.39 (m, 2H), 7.12 (s, 1H), 4.82 (m, 1H), 3.80 (m, 1H), 3.41 (m, 3H), 2.61 (m, 2H), 2.02 (d, J = 13.1 Hz, 2H), 1.54 (m, 4H), 1.28 (m, 2H).
[M+H]+=514 (Multimode+)

### Example 345

### 6-fluoro-N-{cis-4-[6-fluoro-2,4-dioxo-1-[4'-(piperidin-4-ylmethyl)biphenyl-3-yl]-1,4-dihydropyrido[2,3-d]pyrimidin-3(2H)-yl]cyclohexyl}imidazo[1,2-a]pyridine-2-carboxamide

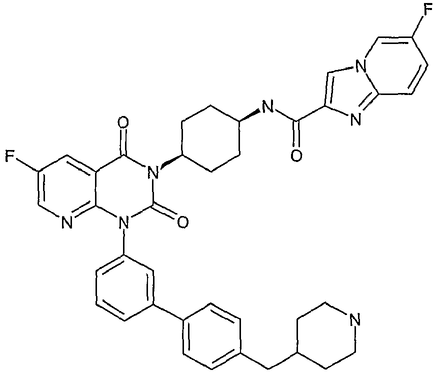

### Step (a) tert-butyl 4-({3'-[6-fluoro-3-(cis-4-{[(6-fluoroimidazo[1,2-a]pyridin-2-yl)carbonyl]amino}cyclohexyl)-2,4-dioxo-3,4-dihydropyrido[2,3-d]pyrimidin-1(2H)-yl]biphenyl-4-yl}methyl)piperidine-1-carboxylate

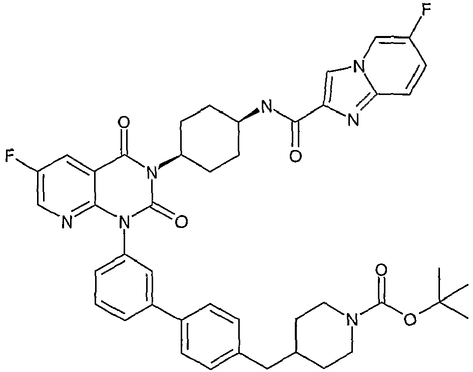

To a suspension of Palladium(II) acetate (0.035 g, 0.16 mmol) in acetonitrile (15 mL) was added 2-(Dicyclohexylphosphino)-2',4',6'-tri-i-propyl-1,1'-biphenyl (0.149 g, 0.31 mmol). The mixture was stirred under nitrogen for 10 minutes. Potassium carbonate (0.282 mL, 4.68 mmol) was then added as a solution in water (10 mL) followed by 6-fluoro-*N*-{*cis*-4-[6-fluoro-2,4-dioxo-1-[3-(4,4,5,5-tetramethyl-1,3,2-dioxaborolan-2-yl)phenyl]-1,4-dihydropyrido[2,3-*d*]pyrimidin-3(2*H*)-yl]cyclohexyl}imidazo[1,2-*a*]pyridine-2-carboxamide (1.002 g, 1.56 mmol) and then tert-butyl 4-(4-bromobenzyl)piperidine-1-carboxylate (0.663 g, 1.87 mmol). The mixture was heated to 80°C for 4 hours. The mixture was cooled and left to stir at room temperature overnight. The mixture was then poured into water. An attempted extraction with ethylaceate then formed an emulsion. The mixture was then filtered, separated and the organic layer evaporated to give a crude product. This was purifed by column chromatography using ethyl acetate as eluent to give the sub-title compound (450 mg).
[M+H]+ = 790 (MultiMode+)

### Step (b) 6-fluoro-N-{cis-4-[6-fluoro-2,4-dioxo-1-[4'-(piperidin-4-ylmethyl)biphenyl-3-yl]-1,4-dihydropyrido[2,3-d]pyrimidin-3(2H)-yl]cyclohexyl}imidazo[1,2-a]pyridine-2-carboxamide

To a solution of *tert*-butyl 4-({3'-[6-fluoro-3-(*cis*-4-{[(6-fluoroimidazo[1,2-*a*]pyridin-2-yl)carbonyl]amino}cyclohexyl)-2,4-dioxo-3,4-dihydropyrido[2,3-*d*]pyrimidin-1(2*H*)-yl]biphenyl-4-yl}methyl)piperidine-1-carboxylate in DCM (2 mL) was added trifluoroacetic acid (2 ml, 25.96 mmol). The mixture was stirred at room temperature for 2 hours. The solvents were removed in vacuo. The crude product was purified by reverse phase preparative HPLC on an ACE column (available from Highchrom Limited - www.highchrom.co.uk) using a 95-5% gradient of aqueous 0.2% TFA in acetonitrile as eluent. The fractions containing the desired compound were evaporated to dryness to afford a solid, which was dried overnight at 40°C under vacuum to give the title compound (15 mg).
[M+H]+ = 690 (MultiMode+)

### Example 346

### 6-Fluoro-3-(cis-4-{[(6-fluoroimidazo[1,2-a]pyridin-2-yl)methyl]amino}cyclohexyl)-1-[3'-hydroxy-2'-(morpholin-4-ylmethyl)biphenyl-3-yl]pyrido[2,3-d]pyrimidine-2,4(1H,3H)-dione

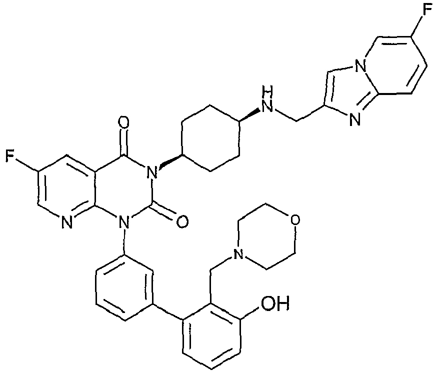

### Step (a) 2-Formyl-3-methoxyphenyl trifluoromethanesulfonate

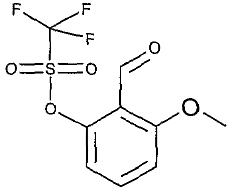

To a solution of 2-hydroxy-6-methoxybenzaldehyde (2.70 g, 17.72 mmol) and pyridine (1.720 mL, 21.27 mmol) in dichloromethane (50 mL) at 0°C was added trifluoromethanesulfonic anhydride (2.98 mL, 17.72 mmol). The orange suspension was left to warm slowly and stirred for 20 h. Water was added and acidified with aqueous HCl (1M). The organics were separated and washed with sat. aq. NaHCO₃. The organics were dried (Na₂SO₄) and concentrated to give the sub-title compound(3.5 g)
GCMS [M] 284

### Step (b) 3-Methoxy-2-(morpholin-4-ylmethyl)phenyl trifluoromethanesulfonate

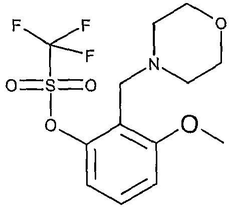

2-Formyl-3-methoxyphenyl trifluoromethanesulfonate (216 mg, 0.76 mmol) and morpholine (0.079 mL, 0.91 mmol) were stirred at room temperature in dichloromethane (20 mL) for 15 mins whereupon sodium triacetoxyborohydride (193 mg, 0.91 mmol) was added. The mixture was stirred at room temperature for 20 h and trifluoroacetic acid (2 mL, 25.96 mmol) was added. The mixture was stirred for 3 h and concentrated *in vacuo.* Saturated sodium hydrogen carbonate was added and then extracted with ethyl acetate, which was dried and concentrated to leave a solid. The crude product was purified by flash silica chromatography, elution gradient 70% ethyl acetate in isohexane. Pure fractions were evaporated to dryness to afford the sub-title compound as a yellow oil (100 mg)
[M+H]+ = 356 (MultiMode+)

### Step (c) 3-Hydroxy-2-(morpholin-4-ylmethyl)phenyl trifluoromethanesulfonate

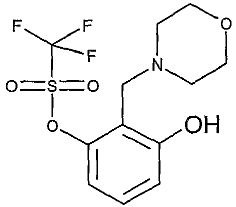

Boron tribromide (7.60 mL, 7.60 mmol, 1M in DCM) was added to a solution of 3-methoxy-2-(morpholinomethyl)phenyl trifluoromethanesulfonate (2.7 g, 7.60 mmol) in dichloromethane (20 mL) and stirred for 24 h. HCl (2M) was added and stirred for 2 h. The phases were separated and the aqueous was extracted with DCM. The combined organic phases were dried (Na₂SO₄) and concentrated. The crude product was purified by flash silica chromatography, elution with 10% ethyl acetate in isohexane. Pure fractions were evaporated to dryness to afford the sub-title compound as a tan solid (1.10 g)
[M+H]+ = 342 (MultiMode+)

### Step (d) tert-Butyl {cis-4-[6-fluoro-1-[3'-hydroxy-2'-(morpholin-4-ylmethyl)biphenyl-3-yl]-2,4-dioxo-1,4-dihydropyrido[2,3-d]pyrimidin-3(2H)-yl]cyclohexyl}carbamate

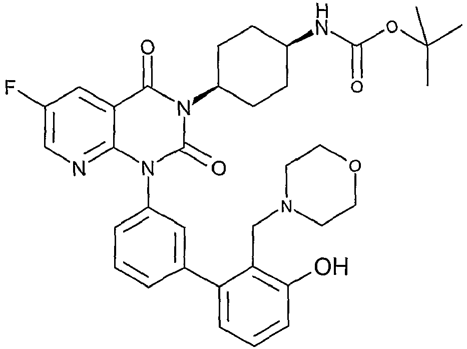

A mixture of diacetoxypalladium (0.039 g, 0.17 mmol) and dicyclohexyl(2',6'-dimethoxybiphenyl-2-yl)phosphine (0.141 g, 0.34 mmol) was stirred for 10 mins in a solvent of dry acetonitrile (16 ml). To this mixture was added consecutively, a solution of potassium carbonate (0.714 g, 5.17 mmol) in water (5 ml), 3-hydroxy-2-(morpholinomethyl)phenyl trifluoromethanesulfonate (0.588 g, 1.72 mmol) and *tert-butyl* {4-[6-fluoro-2,4-dioxo-1-[3-(4,4,5,5-tetramethyl-1,3,2-dioxaborolan-2-yl)phenyl]-1,4-dihydropyrido[2,3-*d*]pyrimidin-3(2*H*)-yl]cyclohexyl}carbamate (1 g, 1.72 mmol). The resultant mixture was stirred and heated at 70 °C for 4 h. The mixture was allowed to cool to room temperature and concentrated *in vacuo.* The crude product was added to a silica gel column and was eluted with 30% to 50% ethyl acetate : isohexane mixture. The pure fractions were combined and evaporated to dryness to give the sub-title compound (1.0 g)
[M+H]+ = 646 (MultiMode+)

### Step (e) 3-(cis-4-Aminocyclohexyl)-6-fluoro-1-[3'-hydroxy-2'-(morpholin-4-ylmethyl)biphenyl-3-yl]pyrido[2,3-d]pyrimidine-2,4(1H,3H)-dione

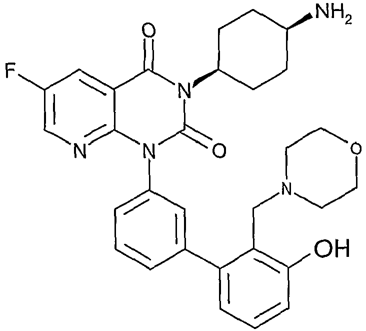

Trifluoroacetic acid (3 mL, 38.94 mmol) was added to *tert-*Butyl {*cis*-4-[6-fluoro-1-[3'-hydroxy-2'-(morpholin-4-ylmethyl)biphenyl-3-yl]-2,4-dioxo-1,4-dihydropyrido[2,3-*d*]pyrimidin-3(2*H*)-yl]cyclohexyl]carbamate (500 mg, 0.77 mmol) in dichloromethane (10 mL) and stirred for 3 h. Concentrated *in vacuo* to give the sub-title compound as an orange oil (600 mg)
[M+H]+ = 546 (MultiMode+)

### Step (e) 6-Fluoro-3-(cis-4-{[(6-fluoroimidazo[1,2-a]pyridin-2-yl)methyl]amino}cyclohexyl)-1-[3'-hydroxy-2'-(morpholin-4-ylmethyl)biphenyl-3-yl]pyrido[2,3-d]pyrimidine-2,4(1H,3H)-dione

3-(*cis*-4-Aminocyclohexyl)-6-fluoro-1-[3'-hydroxy-2'-(morpholin-4-ylmethyl)biphenyl-3-yl]pyrido[2,3-*d*]pyrimidine-2,4(1*H*,3*H*)-dione (0.420 g, 0.77 mmol) and 6-fluoroimidazo[1,2-*a*]pyridine-2-carbaldehyde (0.063 g, 0.39 mmol) in dichloromethane (20 mL) were stirred for 30 mins. Sodium triacetoxyborohydride (0.082 g, 0.39 mmol) was added and the mixture was stirred for 2 h. Further 6-fluoroimidazo[1,2-*a*]pyridine-2-carbaldehyde (0.063 g, 0.39 mmol) was added and 5 mins later, sodium triacetoxyborohydride (0.082 g, 0.39 mmol) were added. Stirred for 2 h and methanol (1 ml) was added. Stirred for 1 h and concentrated *in vacuo*. The crude product was purified by preparative HPLC on a Waters X-Terra column using a 85% isocratic aqueous 0.1% trifluoroacetic acid in acetonitrile as eluent. The fractions containing the desired compound were evaporated to dryness to give the title compound (30 mg)
¹H NMR (300 MHz, DMSO) 5 8.97 - 8.86 (m, 3H), 8.63 - 8.58 (m, 1H), 8.37 - 8.30 (m, 1H), 8.12 - 8.08 (m, 1H), 7.71 - 7.62 (m, 2H), 7.51 - 7.36 (m, 5H), 7.08 - 7.01 (m, 1H), 6.88 - 6.81 (m, 1H), 4.85 - 4.71 (m, 1H), 4.37 (s, 2H), 4.23 (s, 2H), 3.67 (s, 5H), 3.44 - 3.34 (m, 2H), 2.64 - 2.55 (m, 4H), 2.20 - 2.08 (m, 2H), 1.83 - 1.59 (m, 4H)
[M+H]⁺ = 694 (MultiMode+)

### Example 347

### N-{cis-4-[1-{2'-[(dimethylamino)methyl]-4'-hydroxybiphenyl-3-yl}-6-fluoro-2,4-dioxo-1,4-dihydropyrido[2,3-d]pyrimidin-3(2H)-yl]cyclohexyl}-5,6,7,8-tetrahydroimidazo[1,2-a]pyridine-2-carboxamide

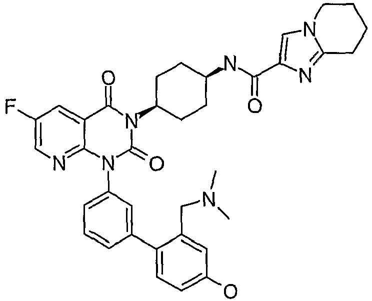

To a solution of 5,6,7,8-tetrahydroimidazo[1,2-a]pyridine-2-carboxylic acid (0.099 g, 0.60 mmol) in DMF (5 mL) was added N,N-Diisopropylethylamine (0.311 mL, 1.79 mmol) and O-(7-Azabenzotriazol-1-yl)-N,N,N',N'-tetramethyluronium hexafluorophosphate (0.272 g, 0.71 mmol) and the reaction stirred for 5 mins. 3-(*cis*-4-aminocyclohexyl)-1-{2'-[(dimethylamino)methyl]-4'-hydroxybiphenyl-3-yl}-6-fluoropyrido[2,3-*d*]pyrimidine-2,4(1*H*,3*H*)-dione (0.3 g, 0.60 mmol) was then added and the reaction left to stir for 5hrs. Water was added and the resultant precipitate filtered, and dried. The crude product was purified by preparative HPLC on a Phenomenex Gemini column using a 95-5% gradient of aqueous 0.2% ammonia in acetonitrile as eluent. The fractions containing the desired compound were evaporated to dryness to afford a solid. The compound was then slurried in ether for 48hrs, filtered and dried to give the title compound (0.083 g)

¹H NMR (299.947 MHz, DMSO) δ 8.59 (d, J = 2.9 Hz, 1H), 8.30 (dd, J = 7.8, 3.0 Hz, 1H), 7.56 - 7.49 (m, 2H), 7.44 - 7.42 (m, 3H), 7.33 - 7.28 (m, 2H), 7.11 (d, J = 8.5 Hz, 1H), 6.92 (d, J = 2.7 Hz, 1H), 6.73 (dd, J = 8.4, 2.6 Hz, 1H), 4.83 (t, J = 11.7 Hz, 1H), 4.08 (s, 1H), 3.98 - 3.94 (m, 2H), 3.25 (s, 2H), 2.76 - 2.72 (m, 3H), 2.07 (s, 6H), 1.93 - 1.80 (m, 6H), 1.71-1.58 (m, 4H)
[M+H]+ = 652 (MultiMode+)

### Example 348

### N-{cis-4-[1-{2'-[(dimethylamino)methyl]-4'-hydroxybiphenyl-3-yl}-6-fluoro-2,4-dioxo-1,4-dihydropyrido[2,3-d]pyrimidin-3(2H)-yl]cyclohexyl}-1,5-dimethyl-1H-pyrazole-3-carboxamide

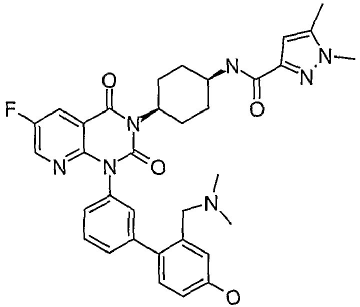

To a solution of 1,5-Dimethyl-1H-pyrazole-3-carboxylic acid (0.083 g, 0.60 mmol) in DMF (5 mL) was added N,N-Diisopropylethylamine (0.311 mL, 1.79 mmol) and O-(7-Azabenzotriazol-1-yl)-N,N,N',N'-tetramethyluronium hexafluorophosphate (0.272 g, 0.71 mmol) and the reaction stirred for 5 mins. 3-(*cis*-4-aminocyclohexyl)-1-{2'-[(dimethylamino)methyl]-4'-hydroxybiphenyl-3-yl}-6-fluoropyrido[2,3-*d*]pyrimidine-2,4(1*H*,3*H*)-dione (0.3 g, 0.60 mmol) was then added and the reaction left to stir for 5hrs. Water was added and the resultant precipitate filtered, dried. The crude product was purified by preparative HPLC on a Phenomenex Gemini column using a 95-5% gradient of aqueous 0.2% ammonia in acetonitrile as eluent. The fractions containing the desired compound were evaporated to dryness to afford the title compound (0.045 g)

¹H NMR (299.947 MHz, DMSO) δ 9.47 (s, 1H), 8.62 - 8.61 (m, 1H), 8.34 - 8.30 (m, 1H), 7.54 (d, J = 8.1 Hz, 1H), 7.46 - 7.43 (m, 2H), 7.34 (d, J = 1.0 Hz, 1H), 7.21 (d, J = 7.1 Hz, 1H), 7.13 (d, J = 8.3 Hz, 1H), 6.94 (d, J = 2.3 Hz, 1H), 6.77 (s, 1H), 6.43 (s, 1H), 4.90 - 4.82 (m, 1H), 4.10 (s, 1H), 3.78 (s, 3H), 3.27 (s, 2H), 2.62 - 2.58 (m, 2H), 2.28 (s, 3H), 2.08 (s, 6H), 1.98 - 1.94 (m, 2H), 1.71 - 1.59 (m, 4H)
[M+H]+ = 626 (MultiMode+)

### Example 349

### N-{cis-4-[1-[4'-(1,4-diazepan-1-ylmethyl)biphenyl-3-yl]-6-fluoro-2,4-dioxo-1,4-dihydropyrido[2,3-d]pyrimidin-3(2H)-yl]cyclohexyl}-2-methyl-1,3-thiazole-4-carboxamide

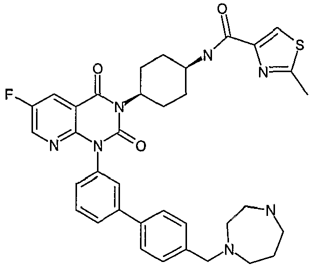

### Step (a) benzyl 4-({3'-[3-{cis-4-[(tert-butoxycarbonyl)amino]cyclohexyl}-6-fluoro-2,4-dioxo-3,4-dihydropyrido[2,3-d]pyrimidin-1(2H)-yl]biphenyl-4-yl}methyl)-1,4-diazepane-1-carboxylate

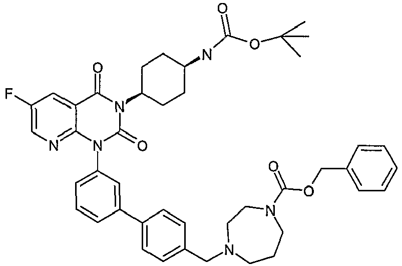

To a solution of *tert-butyl* {*cis*-4-[6-fluoro-1-(4'-formylbiphenyl-3-yl)-2,4-dioxo-1,4-dihydropyrido[2,3-*d*]pyrimidin-3(2H)-yl]cyclohexyl}carbamate (3.013 g, 5.39 mmol) in DCM (60 mL) was added sodium sulfate (anhydrous) (0.766 g, 5.39 mmol) and benzyl 1,4-diazepane-1-carboxylate (1.789 mL, 8.63 mmol). The mixture was stirred at room temperature for 30min and then sodium triacetoxyborohydride (1.143 g, 5.39 mmol) was added and the reaction was permitted to stir at ambient temperature overnight. The reaction was diluted with CH₂Cl₂ (50ml), washed thoroughly with saturated aq. NaHC03 solution, dried and evaporated at 40°C affording a grey foam. The foam (dissolved in MeOH) was applied to a large SCX cartridge (pre-washed with MeOH), washed well with MeOH and eluted with MeOH-7N ammonia in MeOH (4:1) affording the sub-title compound (3.03 g). This was used without further purification.

¹H NMR (400 MHz, CDCL3) δ 8.37 (d, J = 2.8 Hz, 1H), 8.21 (dd, J = 3.3, 7.2 Hz, 1H), 7.77 - 7.72 (m, 1H), 7.67 - 7.62 (m, 1H), 7.59 - 7.53 (m, 2H), 7.53 - 7.49 (m, 1H), 7.41 - 7.26 (m, 16H), 5.14 (s, 4H), 5.04 - 4.93 (m, 2H), 3.97 - 3.89 (m, 1H), 2.97 - 2.80 (m, 5H), 2.73 - 2.52 (m, 7H), 2.06 (s, 2H), 1.97 - 1.57 (m, 16H), 1.43 (s, 9H), 1.26 (t, J = 7.1 Hz, 3H) Spectrum shows presence of 1 mole EtOAc and approx I mole N-Cbz-homopiperazine

### Step (b) benzyl 4-({3'-[3-(cis-4-aminocyclohexyl)-6-fluoro-2,4-dioxo-3,4-dihydropyrido[2,3-d]pyrimidin-1(2H)-yl]biphenyl-4-yl} methyl)-1,4-diazepane-1-carboxylate

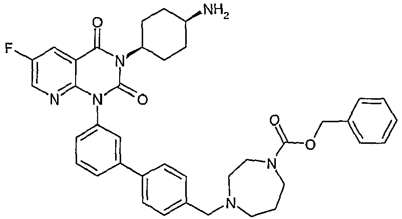

To a solution of benzyl 4-({3'-[3-{*cis*-4-[(tert-butoxycarbonyl)amino]cyclohexyl}-6-fluoro-2,4-dioxo-3,4-dihydropyrido[2,3-*d*]pyrimidin-1(2*H*)-yl]biphenyl-4-yl}methyl)-1,4-diazepane-1-carboxylate (2.3 g, 2.96 mmol) in 1,4-dioxane (10 mL) was added 4.0M Hydrogen chloride in 1,4 Dioxane (10 mL, 40.00 mmol). The reaction was stirred at room temperature overnight. The mixture was evaporated to dryness to give a white solid (3 g) This was used without further purification.
[M+H]+ = 677 (MultiMode+)

### Step (c) N-{cis-4-[1-[4'-(1,4-diazepan-1-ylmethyl)biphenyl-3-yl]-6-fluoro-2,4-dioxo-1,4-dihydropyrido[2,3-d]pyrimidin-3(2H)-yl]cyclohexyl}-2-methyl-1,3-thiazole-4-carboxamide

To a suspension of benzyl 4-({3'-[3-(*cis*-4-aminocyclohexyl)-6-fluoro-2,4-dioxo-3,4-dihydropyrido[2,3-*d*]pyrimidin-1(2*H*)-yl]biphenyl-4-yl}methyl)-1,4-diazepane-1-carboxylate (300 mg, 0.44 mmol) and 2-methylthiazole-4-carboxylic acid (63.5 mg, 0.44 mmol) in acetonitrile (5 mL) was added Triethylamine (0.309 mL, 2.22 mmol). After stirring at room temperature for 10 mins 1.57M solution of 1-Propanephosphonic acid cyclic anhydride in THF (0.296 mL, 0.47 mmol) was added and the mixture was left to stir for 2 hours. The mixture was poured into sat NaHCO3(aq) and the organics extracted into ethyl acetate The ethylacetate extractions were combined and evaporated to give the crude product. This was dissolved in acetic acid 2ml and 33% HBr in acetic acid (2ml) was added. The reaction was stirred for 10mins. The solvent were removed in vacuo The crude product was purified by reverse phase preparative HPLC on an ACE column (available from Highchrom Limited - www.highchrom.co.uk) using a 95-5% gradient of aqueous 0.2% TFA in acetonitrile as eluent. The appropriate fractions were combined, evaporated to dryness and triturated with ether to give a white solid. The solid was isolated by filtration and dried overnight under vacuum at 40°C. This gave the title compound (111 mg)

1H NMR (400 MHz, CDCL3) δ 8.36 (d, J = 3.1 Hz, 1H), 8.22 (dd, J = 7.0, 2.9 Hz, 1H), 7.90 (s, 1H), 7.72 (d, J = 7.9 Hz, 2H), 7.68 - 7.64 (m, 3H), 7.55 - 7.50 (m, 3H), 7.34 (d, J = 7.7 Hz, 1H), 5.03 (t, J = 12.0 Hz, 1H), 4.37 - 4.32 (m, 1H), 4.25 (s, 2H), 3.73 - 3.64 (m, 4H), 3.46 - 3.36 (m, 4H), 2.76 - 2.65 (m, 5H), 2.34 - 2.26 (m, 2H), 2.08 (d, J = 13.1 Hz, 2H), 1.81 - 1.68 (m, 4H)
[M+H]+ = 668 (MultiMode+)

The following compounds (Table 11) were prepared in a similar manner as solids from the appropriate carboxylic acid using the method described in Example 349 step (c).

**Table 11**

| **Example Number** | **Chemistry** | **Name** | **NMR** | **M+H** |
|---|---|---|---|---|
| 350 | 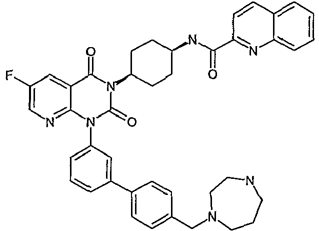 | N-{cis-4-[1-[4'-(1,4-diazepan-1-ylmethyl)biphenyl-3-yl]-6-fluoro-2,4-dioxo-1,4-dihydropyrido[2,3-d]pyrimidin-3 (2H)-yl]cyclohexyl]quinoline-2-carboxamide | ¹H NMR (400 MHz, CDCL3) δ 8.96 (d, J = 8.7 Hz, 1H), 8.36 (d, J = 3.1 Hz, 1H), 8.29 - 8.23 (m, 3H), 8.07 - 8.02 (m, 1H), 7.83 (d, J = 8.2 Hz, 1H), 7.76 (d, J = 8.2 Hz, 1H), 7.71 - 7.60 (m, 4H), 7.59 - 7.53 (m, 2H), 7.50 (d, J = 8.2 Hz, 2H), 7.38 (d, J = 7.7 Hz, 1H), 5.15 - 5.07 (m, 1H), 4.50 - 4.45 (m, 1H), 4.26 (s, 2H), 3.73 - 3.68 (m, 4H), 3.44 - 3.38 (m, 4H), 2.94 - 2.81 (m, 2H), 2.31 - 2.26 (m, 2H), 2.11 (d, J = 13.3 Hz, 2H), 1.85 - 1.74 (m, 4H) | 698 |
| 351 | 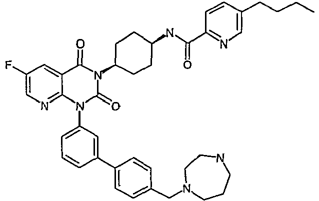 | 5-butyl-N-{cis-4-[1-[4'-(1,4-diazepan-1-ylmethyl)biphenyl-3-yl]-6-fluoro-2,4-dioxo-1,4-dihydropyrido[2,3-d]pyrimidin-3(2H)-yl]cyclohexyl}pyridine-2-carboxamide | ¹H NMR (400 MHz, CDCL3) δ 8.44 (d, J = 7.9 Hz, 1H), 8.39 (s, 1H), 8.35 (d, J = 3.1 Hz, 1H), 8.23 (dd, J = 7.2, 3.1 Hz, 1H), 8.06 (d, J = 7.9 Hz, 1H), 7.72 (d, J = 7.9 Hz, 1H), 7.69 - 7.65 (m, 3H), 7.61 (dd, J = 7.9, 1.8 Hz, 1H), 7.54 - 7.51 (m, 3H), 7.34 (d, J = 7.7 Hz, 1H), 5.02 (t, J = 12.3 Hz, 1H), 4.40 - 4.34 (m, 1H), 4.24 (s, 2H), 3.72 - 3.65 (m, 4H), 3.46 - 3.38 (m, 4H), 2.81 - 2.70 (m, 2H), 2.65 (t, J = 7.7 Hz, 2H), 2.33 - 2.28 (m, 2H), 2.10 (d, J = 13.6 Hz, 2H), 1.83 - 1.69 (m, 4H), 1.60 (quintet, J = 7.6 Hz, 2H), 1.35 (sextet, J = 7.4 Hz, 2H), 0.92 (t, J = 7.4 Hz, 3H) | 704 |
| 352 | 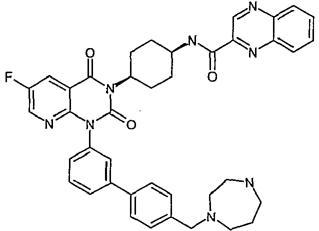 | N-{cis-4-[1-[4'-(1,4-diazepan-1-ylmethyl)biphenyl-3-yl]-6-fluoro-2,4-dioxo-1,4-dihydropyrido[2,3-d]pyrimidin-3(2H)-yl]cyclohexyl}quinoxaline-2-carboxamide | ¹H NMR (400 MHz, CDCL3) δ 9.63 (s, 1H), 8.60 (d, J = 8.5 Hz, 1H), 8.37 (d, J = 3.1 Hz, 1H), 8.24 (dd, J = 7.2, 3.1 Hz, 1H), 8.14 (d, J = 8.2 Hz, 1H), 8.03 (s, 1H), 7.85 - 7.66 (m, 6H), 7.56 (s, 1H), 7.50 (d, J = 7.9 Hz, 2H), 7.37 (d, J = 7.7 Hz, 1H), 5.16 - 5.06 (m, 1H), 4.52 - 4.47 (m, 1H), 4.24 (s, 2H), 3.71 - 3.65 (m, 4H), 3.43 - 3.37 (m, 4H), 2.83 (q, J = 12.6 Hz, 2H), 2.33 - 2.26 (m, 2H), 2.12 (d, J = 12.8 Hz, 2H), 1.87 - 1.75 (m, 4H) | 699 |
| 353 | 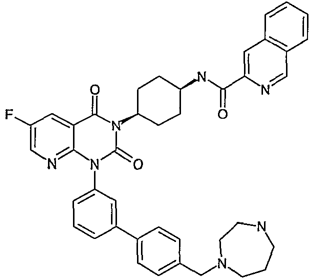 | N-{cis-4-[1-[4'-(1,4-diazepan-1-ylmethyl)biphenyl-3-yl]-6-fluoro-2,4-dioxo-1,4-dihydropyrido[2,3-d]pyrimidin-3(2H)-yl]cyclohexyl}isoquinoline-3-carboxamide | ¹H NMR (400 MHz, CDCL3) δ 9.16 (s, 1H), 8.70 (d, J = 7.9 Hz, 1H), 8.56 (s, 1H), 8.35 (d, J = 3.1 Hz, 1H), 8.23 (dd, J = 7.3, 2.9 Hz, 1H), 8.00 (d, J = 8.2 Hz, 1H), 7.95 (d, J = 8.2 Hz, 1H), 7.76 - 7.62 (m, 6H), 7.54 - 7.47 (m, 3H), 7.34 (d, J = 7.7 Hz, 1H), 5.09 - 5.01 (m, 1H), 4.48 - 4.43 (m, 1H), 4.23 (s, 2H), 3.70 - 3.64 (m, 4H), 3.43 - 3.36 (m, 4H), 2.86 - 2.74 (m, 2H), 2.32 - 2.25 (m, 2H), 2.15 (d, J= 13.6 Hz, 2H), 1.87-1.72 (m, 4H) | 698 |
| 354 | 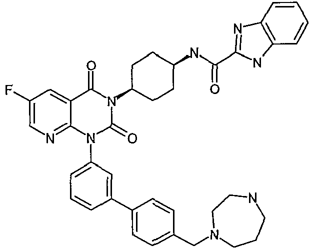 | N-{cis-4-[1-[4'-(1,4-diazepan-1-ylmethyl)biphenyl-3-yl]-6-fluoro-2,4-dioxo-1,4-dihydropyrido[2,3-d]pyrimidin-3(2H)-yl]cyclohexyl}-1 H-benzimidazole-2-carboxamide | ¹H NMR (400 MHz, DMSO) δ 10.67-10.51 (m, 1H), 9.14 - 8.99 (m, 2H), 8.59 (d, J = 2.8 Hz, 1H), 8.36 (dd, J = 7.7, 3.1 Hz, 1H), 8.04 (d, J = 7.2 Hz, 1H), 7.85 - 7.77 (m, 4H), 7.67 - 7.59 (m, 5H), 7.43 (d, J = 8.5 Hz, 1H), 7.31 - 7.26 (m, 2H), 4.94 - 4.84 (m, 1H), 4.42 - 4.31 (m, 2H), 4.22 - 4.18 (m, 1H), 3.65 - 3.18 (m, 8H), 2.74 - 2.58 (m, 2H), 2.12 - 2.03 (m, 4H), 1.79 - 1.63 (m, 4H) | 687 |
| 355 | 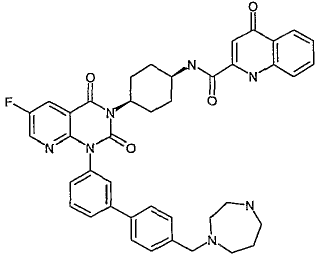 | N-{cis-4-[1-[4'-(1,4-diazepan-1-ylmethyl)biphenyl-3-yl]-6-fluoro-2,4-dioxo-1,4-dihydropyrido [2,3-d]pyrimidin-3(2H)-yl]cyclohexyl}-4-oxo-1,4-dihydroquinoline-2-carboxamide | ¹H NMR (400 MHz, CDCL3) δ 8.37 (d, J = 3.1 Hz, 1H), 8.28 (dd, J = 7.2, 3.1 Hz, 1H), 8.21 (d, J = 8.2 Hz, 1H), 7.68 - 7.46 (m, 10H), 7.40 (t, J = 7.4 Hz, 1H), 7.33 - 7.29 (m, 1H), 7.00 (s, 1H), 5.09 (t, J = 12.2 Hz, 1H), 4.34 - 4.29 (m, 3H), 3.65 (s, 4H), 3.49 - 3.39 (m, 4H), 2.80 - 2.71 (m, 2H), 2.36 - 2.29 (m, 2H), 2.17 (d, J = 12.8 Hz, 2H), 1.83 - 1.72 (m, 4H) | 714 |
| 356 | 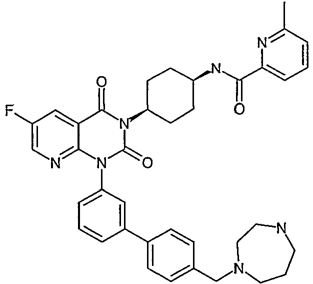 | N-{cis-4-[1-[4'-(1,4-diazepan-1-ylmethyl)biphenyl-3-yl]-6-fluoro-2,4-dioxo-1,4-dihydropyrido[2,3-d]pyrimidin-3(2H)-yl]cyclohexyl}-6-methylpyridine-2-carboxamide | ¹H NMR (400 MHz, CDCL3) δ 8.76 (d, J = 8.5 Hz, 1H), 8.36 (d, J = 3.1 Hz, 1H), 8.22 (dd, J = 7.2, 2.8 Hz, 1H), 7.94 (d, J = 7.7 Hz, 1H), 7.74 - 7.61 (m, 5H), 7.54 - 7.50 (m, 3H), 7.34 (d, J = 7.9 Hz, 1H), 7.23 (d, J = 7.7 Hz, 1H), 5.06 (t, J = 12.0 Hz, 1H), 4.42 - 4.36 (m, 1H), 4.25 (s, 2H), 3.72 - 3.66 (m, 4H), 3.44 - 3.39 (m, 4H), 2.83 - 2.72 (m, 2H), 2.49 (s, 3H), 2.31 (s, 2H), 2.06 (d, J = 12.8 Hz, 2H), 1.81 - 1.69 (m, 4H) | 662 |

The following compounds (Table 12) were prepared in a similar manner using the methods described in example 232.

**Table 12**

| **Example Number** | **Chemistry** | **Name** | **NMR** | **M+H** |
|---|---|---|---|---|
| 357 | 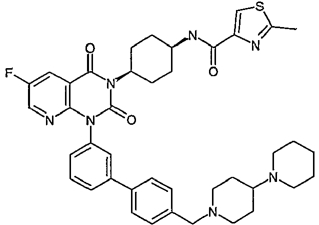 | N-{cis-4-[1-[4'-(1,4'-bipiperidin-1'-ylmethyl)biphenyl-3-yl]-6-fluoro-2,4-dioxo-1,4-dihydropyrido [2,3-d]pyrimidin-3(2H)-yl]cyclohexyl}-2-methyl-1,3-thiazole-4-carboxamide | ¹H NMR (400 MHz, DMSO) δ 8.59 (d, J = 3.1 Hz, 1H), 8.32 (dd, J = 7.7, 3.1 Hz, 1H), 8.11 (s, 1H), 7.81 - 7.66 (m, 3H), 7.66 - 7.55 (m, 3H), 7.42 - 7.33 (m, 3H), 4.87 (t, J = 12.5 Hz, 1H), 4.15 - 4.11 (m, 1H), 3.46 (s, 2H), 2.87 - 2.83 (m, 2H), 2.66 (s, 3H), 2.61 - 2.51 (m, 2H), 2.45 - 2.39 (m, 4H), 1.99 - 1.88 (m, 4H), 1.73 - 1.63 (m, 6H), 1.48 - 1.37 (m, 8H). Remaining proton obscured by solvent peak. | 736 |
| 358 | 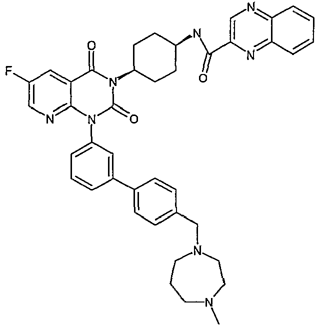 | N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}quinoxaline-2-carboxamide | ¹H NMR (400 MHz, CDCL3) δ 9.65 (s, 1H), 8.64 (d, J = 8.7 Hz, 1H), 8.39 (d, J = 3.1 Hz, 1H), 8.26 (dd, J = 7.2, 2.8 Hz, 1H), 8.19 (d, J = 8.5 Hz, 1H), 8.09 - 8.02 (m, 1H), 7.86 (t, J = 7.6 Hz, 1H), 7.78 (t, J = 8.6 Hz, 2H), 7.74 - 7.68 (m, 3H), 7.54 (t, J = 8.2 Hz, 3H), 7.39 (d, J = 7.7 Hz, 1H), 5.20 - 5.09 (m, 1H), 4.56 - 4.49 (m, 1H), 4.34 (s, 3H), 4.03 - 2.90 (m, 10H), 2.91 - 2.76 (m, 3H), 2.55 - 2.46 (m, 2H), 2.20 - 2.08 (m, 2H), 1.91 - 1.74 (m, 4H) | 713 |
| 359 | 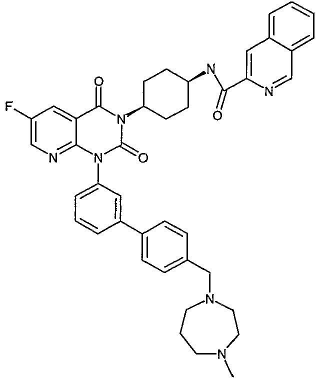 | N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}isoquinoline-3-carboxamide | ¹H NMR (400 MHz, CDCL3) δ 9.21 (s, 1H), 8.73 (d, J = 7.4 Hz, 1H), 8.60 (s, 1H), 8.37 (d, J = 2.8 Hz, 1H), 8.25 (dd, J = 6.9, 2.6 Hz, 1H), 8.04 (d, J = 7.9 Hz, 1H), 7.98 (d, J = 7.9 Hz, 1H), 7.80 - 7.65 (m, 6H), 7.58 - 7.51 (m, 3H), 7.37 (d, J = 6.9 Hz, 1H), 5.07 (t, J = 10.2 Hz, 1H), 4.52 - 4.44 (m, 1H), 4.29 (s, 2H), 4.01 - 3.20 (m, 9H), 2.90 (s, 3H), 2.88 - 2.74 (m, 2H), 2.56 - 2.43 (m, 2H), 2.22 - 2.11 (m, 2H), 1.92 - 1.68 (m, 4H) | 712 |
| 360 | 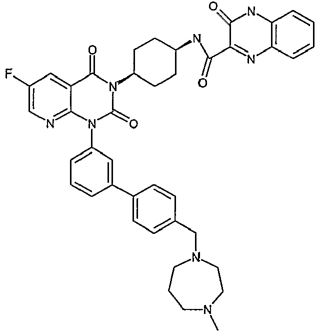 | N-{cis-4-[6-fluoro-1-{4'-[(4-methyl-1,4-diazepan-1-yl)methyl]biphenyl-3-yl}-2,4-dioxo-1,4-dihydropyrido[2,3-d]pyrimidin-3(2H)-yl]cyclohexyl}-3-oxo-3,4-dihydroquinoxaline-2-carboxamide | ¹H NMR (400 MHz, DMSO + CDC13) δ 9.91 - 9.76 (m, 1H), 8.57 - 8.48 (m, 1H), 8.32 - 8.14 (m, 2H), 7.94 - 7.87 (m, 1H), 7.84 - 7.50 (m, 9H), 7.43 - 7.33 (m, 3H), 4.96 - 4.84 (m, 1H), 4.32 - 4.09 (m, 4H), 2.85 (s, 3H), 2.77 - 2.60 (m, 2H), 2.18 - 1.95 (m, 4H), 1.84 - 1.60 (m, 4H), 1.60 - 1.41 (m, 1H) Other resonances obscured by DMSO and water signals | 729 |
| 361 | 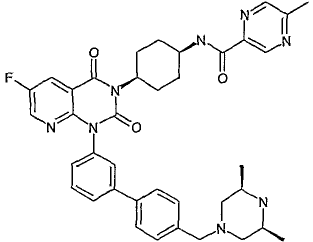 | N-{cis-4-[1-(4'-{[(3R,5S)-3,5-dimethylpiperazin-1-yl]methyl}biphenyl-3-yl)-6-fluoro-2,4-dioxo-1,4-dihydropyrido[2,3-d]pyrimidin-3(2H)-yl]cyclohexyl}-5-methylpyrazine-2-carboxamide | ¹H NMR (400 MHz, CDCL3) δ 9.24 (d, J = 1.0 Hz, 1H), 8.40 (s, 1H), 8.37 (d, J = 2.8 Hz, 1H), 8.23 (dd, J = 7.3, 2.9 Hz, 1H), 8.18 (d, J = 7.9 Hz, 1H), 7.75 (d, J = 7.9 Hz, 1H), 7.69 - 7.63 (m, 3H), 7.53 (t, J = 1.8 Hz, 1H), 7.45 (d, J = 8.2 Hz, 2H), 7.35 - 7.31 (m, 1H), 5.05 (t, J = 12.3 Hz, 1H), 4.44 - 4.39 (m, 1H), 3.96 (s, 2H), 3.64 - 3.58 (m, 2H), 3.17 (d, J = 11.0 Hz, 2H), 2.90 (t, J =11.8 Hz, 2H), 2.79 - 2.67 (m, 2H), 2.63 (s, 3H), 2.10 (d, J = 13.3 Hz, 2H), 1.86 - 1.70 (m, 4H), 1.32 (d, J = 6.4 Hz, 6H) | 677 |
| 362 | 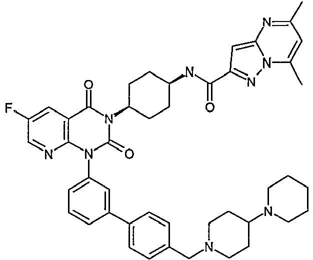 | N-{cis-4-[1-[4'-(1,4'-bipiperidin-1'-ylmethyl)biphenyl-3-yl]-6-fluoro-2,4-dioxo-1,4-dihydropyrido[2,3-d]pyrimidin-3(2H)-yl]cyclohexyl}-5,7-dimethylpyrazolo[1,5-a]pyrimidine-2-carboxamide | ¹H NMR (400 MHz, DMSO) δ 8.59 (d, J = 3.1 Hz, 1H), 8.30 (dd, J = 7.7, 3.1 Hz, 1H), 7.84 (d, J = 7.7 Hz, 1H), 7.78 (d, J = 7.7 Hz, 1H), 7.71 - 7.70 (m, 1H), 7.63 - 7.58 (m, 3H), 7.37 (d, J = 8.2 Hz, 3H), 6.98 (s, 1H), 6.91 (s, 1H), 4.98 - 4.91 (m, 1H), 4.24 - 4.20 (m, 1H), 3.46 (s, 2H), 3.29 (s, 1H), 2.87 - 2.82 (m, 2H), 2.72 - 2.61 (m, 2H), 2.44 - 2.39 (m, 2H), 2.19 - 1.87 (m, 4H), 1.76 - 1.62 (m, 6H), 1.49 - 1.33 (m, 8H). Remaining protons obscured by solvent peaks. | 784 |
| 363 | 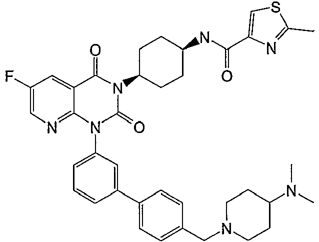 | N-{cis-4-[1-(4'-{[4-(dimethylamino)piperidin-1-yl]methyl}biphenyl-3-yl)-6-fluoro-2,4-dioxo-1,4-dihydropyrido[2,3-d]pyrimidin-3(2H)-yl]cyclohexyl}-2-methyl-1,3-thiazole-4-carboxamide | | 696 |
| 364 | 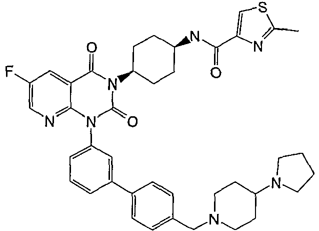 | N-{cis-4-[6-fluoro-2,4-dioxo-1-{4'-[(4-pyrrolidin-1-ylpiperidin-1-yl)methyl]biphenyl-3-yl}-1,4-dihydropyrido[2,3-d]pyrimidin-3(2H)-yl]cyclohexyl}-2-methyl-1,3-thiazole-4-carboxamide | | 722 |
| 365 | 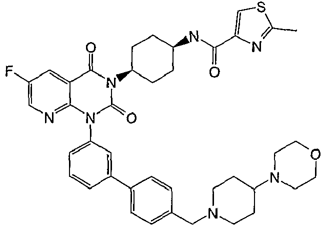 | N-{cis-4-[6-fluoro-1-{4'-[(4-morpholin-4-ylpiperidin-1-yl)methyl]biphenyl-3-yl}-2,4-dioxo-1,4-dihydropyrido[2,3-d]pyrimidin-3(2H)-yl]cyclohexyl}-2-methyl-1,3-thiazole-4-carboxamide | | 738 |
| 366 | 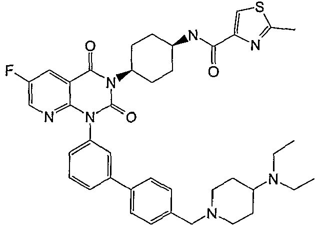 | N-{cis-4-[1-(4'-{[4-(diethylamino)piperidin-1-yl]methyl}biphenyl-3-yl)-6-fluoro-2,4-dioxo-1,4-dihydropyrido[2,3-d]pyrimidin-3(2H)-yl]cyclohexyl}-2-methyl-1,3-thiazole-4-carboxamide | | 724 |
| 367 | 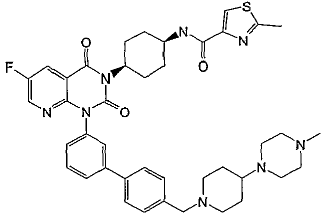 | N-{cis-4-[6-fluoro-1-(4'-{[4-(4-methylpiperazin-1-yl)piperidin-1-yl]methyl}biphenyl-3-yl)-2,4-dioxo-1,4-dihydropyrido[2,3-d]pyrimidin-3(2H)-yl]cyclohexyl}-2-methyl-1,3-thiazole-4-carboxamide | | 751 |
| 368 | 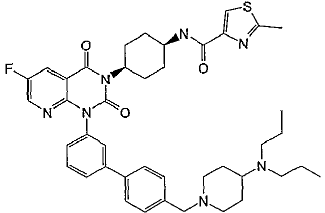 | N-{cis-4-[1-(4'-{[4-(dipropylamino)piperidin-1-yl]methyl}biphenyl-3-yl)-6-fluoro-2,4-dioxo-1,4-dihydropyrido[2,3-d]pyrimidin-3(2H)-yl]cyclohexyl}-2-methyl-1,3-thiazole-4-carboxamide | | 752 |

### Example 369

### 3-(cis-4-aminocyclohexyl)-6-fluoro-1-{4'-hydroxy-2'-[(5-oxo-1,4-diazepan-1-yl)methyl]biphenyl-3-yl}pyrido[2,3-d]pyrimidine-2,4(1H,3H)-dione

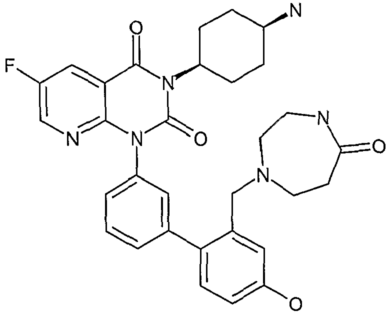

### Step (a) tert-butyl {cis-4-[6-fluoro-1-{4'-hydroxy-2'-[(5-oxo-1,4-diazepan-1-yl)methyl]biphenyl-3-yl}-2,4-dioxo-1,4-dihydropyrido[2,3-d]pyrimidin-3(2H)-yl]cyclohexyl} carbamate

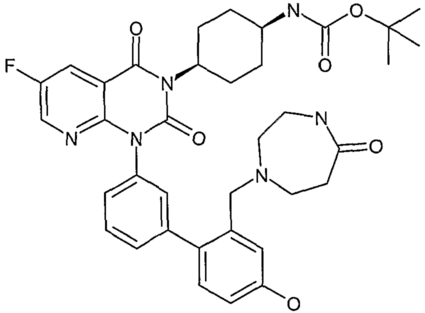

To a solution of *tert-*butyl {*cis*-4-[6-fluoro-1-(2'-formyl-4'-hydroxybiphenyl-3-yl)-2,4-dioxo-1,4-dihydropyrido[2,3-*d*]pyrimidin-3(2*H*)-yl]cyclohexyl}carbamate (2.5 g, 4.35 mmol) in 1,2-dichloroethane (30 mL) was added 1,4-diazepan-5-one (0.596 g, 5.22 mmol) and acetic acid (0.249 mL, 4.35 mmol) and the reaction mixture was stirred at ambient temperature for 4h. sodium triacetoxyborohydide (1.844 g, 8.70 mmol) followed by 1,2 dichloroethane (30 mL) was added and the reaction mixture was stirred at ambient temperature for 24 h. The mixture was washed with saturated sodium bicarbonate solution, brine and dried over sodium sulfate. The organics were evaporated to dryness. The crude product was purified by flash silica chromatography, elution gradient 3 to 5% (7N methanolic ammonia) in dichloromethane. Pure fractions were evaporated to dryness to afford the sub-title compound (2.21 g).
[M+H]+ = 673 (MultiMode+)

### Step (b) 3-(cis-4-aminocyclohexyl)-6-fluoro-1-{4'-hydroxy-2'-[(5-oxo-1,4-diazepan-1-yl)methyl]biphenyl-3-yl}pyrido[2,3-d]pyrimidine-2,4(1H,3H)-dione

A mixture of trifluoroacetic acid (25 mL, 324.49 mmol) and dichloromethane (25 mL) was added to tert-butyl {cis-4-[6-fluoro-1-{4'-hydroxy-2'-[(5-oxo-1,4-diazepan-1-yl)methyl]biphenyl-3-yl}-2,4-dioxo-1,4-dihydropyrido[2,3-d]pyrimidin-3(2H)-yl]cyclohexyl}carbamate (2.21 g, 3.29 mmol) and the mixture was allowed to stand at room temperature for 1 h. The solvents were evaporated and the residue was dissolved in water and basified by addition of aqueous ammonia. The precipitate was filtered and dried in the oven at 40 C for 16 h to give the crude title product (1.19 g). The crude product (0.1g) was purified by preparative HPLC on a Phenomenex Gemini column using a 95-5% gradient of aqueous 0.2% ammonia in acetonitrile as eluent. The fractions containing the desired compound were evaporated to dryness to afford the title compound as a white solid (0.088g).
¹H NMR (399.826 MHz, DMSO) δ 8.56 (d, J = 2.8 Hz, 1H), 8.29 (dd, J = 7.7,3.1 Hz, 1H), 7.55 - 7.48 (m, 2H), 7.46 (t, J = 5.4 Hz, 1H), 7.38 (d, J = 7.7 Hz, 1H), 7.30 (d, J = 7.9 Hz, 1H), 7.11 (d, J = 8.2 Hz, 1H), 6.90 (d, J = 2.6 Hz, 1H), 6.75 (dd, J = 8.3, 2.4 Hz, 1H), 4.74 (ddd, J = 12.2, 8.6, 3.7 Hz, 1H), 3.43 - 3.38 (m, 2H), 3.06 (s, 1H), 2.96 (s, 2H), 2.70 (td, J = 12.8, 9.3 Hz, 2H), 2.39 (s, 4H), 2.23 (d, J = 4.6 Hz, 2H), 1.64 (d, J = 12.0 Hz, 2H), 1.53 (t, J = 13.1 Hz, 2H), 1.42 (d, J = 10.3 Hz, 2H)
[M+H]+ = 573 (MultiMode+)

The following compounds (Table 13) were prepared in a similar manner using the methodology described in example 246.

**Table 13**

| **Example Number** | **Chemistry** | **Name** | **NMR** | **M+H** |
|---|---|---|---|---|
| 370 | 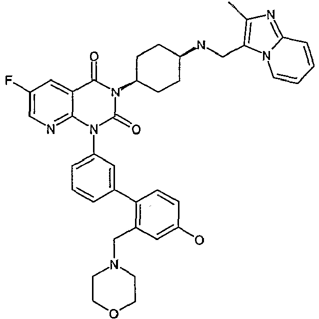 | 6-fluoro-1-[4'-hydroxy-2'-(morpholin-4-ylmethyl)biphenyl-3-yl]-3-(cis-4- {[(2-methylimidazo[1,2-a]pyridin-3-yl)methyl] amino } cyclohexyl)pyrido[ 2,3-d]pyrimidine-2,4(1H,3H)-dione | ¹H NMR (300 MHz, DMSO) δ 9.50 (s, 1H), 8.64 (d, J = 6.5 Hz, 1H), 8.59 (d, J = 2.9 Hz, 1H), 8.31 (dd, J = 7.8, 3.0 Hz, 1H), 7.61 - 7.51 (m, 2H), 7.47 - 7.37 (m, 3H), 7.33 (d, J = 7.7 Hz, 1H), 7.17 - 7.07 (m, 2H), 6.88 (d, J = 2.5 Hz, 1H), 6.76 (dd, J = 8.4,2.4 Hz, 1H), 6.73 - 6.65 (m, 1H), 4.88 - 4.75 (m, 1H), 3.99 (s, 2H), 3.46 - 3.39 (m, 2H), 3.28 (s, 1H), 2.85 - 2.72 (m, 4H), 2.33 (s, 3H), 2.30 - 2.21 (m, 4H), 1.89 (d, J= 12.3 Hz, 2H), 1.55 - 1.39 (m, 6H) | 690 |
| 371 | 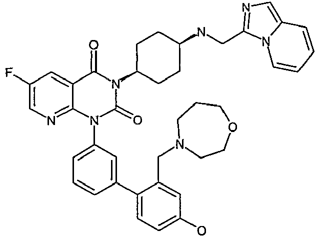 | 6-fluoro-1-[4'-hydroxy-2'-(1,4-oxazepan-4-ylmethyl)biphenyl-3-yl]-3-{cis-4-[(imidazo[1,5-a]pyridin-3-ylmethyl)amino]cyclohexyl}pyrido[2 ,3-d]pyrimidine-2,4(1H,3H)-dione | ¹H NMR (400 MHz, DMSO) δ 9.47 (s, 1H), 8.59 (d, J = 3.1 Hz, 1H), 8.54 (d, J = 7.2 Hz, 1H), 8.30 (dd, J = 7.7, 3.1 Hz, 1H), 7.57 - 7.47 (m, 3H), 7.41 (d, J = 7.9 Hz, 1H), 7.32 (ddd, J = 7.8, 1.9, 1.0 Hz, 1H), 7.25 (s, 1H), 7.12 (d, J = 8.2 Hz, 1H), 6.95 (d, J = 2.6 Hz, 1H), 6.74 (dd, J = 8.2, 2.6 Hz, 1H), 6.69 (dd, J = 9.1, 6.3 Hz, 1H), 6.51 (d, J = 5.6 Hz, 1H), 4.80 (s, 1H), 4.10 (s, 2H), 3.56 (t, J = 6.0 Hz, 2H), 3.48 - 3.41 (m, 4H), 2.82 - 2.69 (m, 3H), 1.98 - 1.83 (m, 3H), 1.67 - 1.59 (m, 2H), 1.52 - 1.38 (m, 4H) Remaining protons obscured by solvent peaks. | 690 |
| 372 | 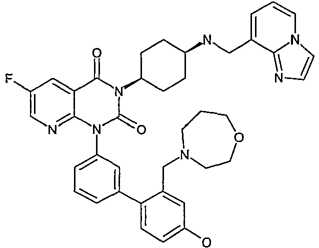 | 6-fluoro-1-[4'-hydroxy-2'-(1,4-oxazepan-4-ylmethyl)biphenyl-3-yl]-3-{cis-4-[(imidazo[1,2-a]pyridin-8-ylmethyl)amino]cyclohexyl}pyrido[2 ,3-d]pyrimidine-2,4(1H,3H)-dione | ¹H NMR (400 MHz, DMSO) δ 9.49 (s, 1H), 8.58 (d, J = 3.1 Hz, 1H), 8.46 (d, J = 6.7 Hz, 1H), 8.29 (dd, J = 7.7,3.1 Hz, 1H), 7.94 (s, 1H), 7.55 - 7.47 (m, 3H), 7.40 (d, J = 7.7 Hz, 1H), 7.33 - 7.26 (m, 2H), 7.11 (d, J = 8.5 Hz, 1H), 6.96 (d, J = 2.6 Hz, 1H), 6.87 (t, J = 6.8 Hz, 1H), 6.74 (dd, J = 8.3, 2.4 Hz, 1H), 4.79 (t, J = 12.0 Hz, 1H), 4.10 (s, 2H), 3.57 (t, J = 5.9 Hz, 2H), 3.48 - 3.42 (m, 2H), 3.36 (s, 6H), 2.88 - 2.70 (m, 3H), 1.91 (d, J = 13.1 Hz, 2H), 1.64 (s, 2H), 1.49 (dd, J = 30.9, 13.7 Hz, 4H) | 690 |
| 373 | 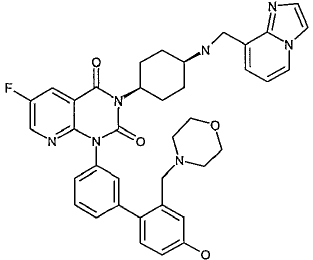 | 6-fluoro-1-[4'-hydroxy-2'-(morpholin-4-ylmethyl)biphenyl-3-yl]-3-{cis-4-[(imidazo[1,2-a]pyridin-8-ylmethyl)amino] cyclohexyl} pyrido [2 ,3-d]pyrimidine-2,4(1H,3H)-dione | ¹H NMR (300 MHz, DMSO) δ 9.50 (s, 1H), 8.58 (d, J = 2.9 Hz, 1H), 8.43 (d, J = 6.7 Hz, 1H), 8.29 (dd, J = 7.7, 3.1 Hz, 1H), 7.93 (s, 1H), 7.58 - 7.49 (m, 3H), 7.41 (d, J = 7.7 Hz, 1H), 7.35 - 7.21 (m, 2H), 7.13 (d, J = 8.3 Hz, 1H), 6.92 - 6.80 (m, 2H), 6.75 (dd, J = 8.3, 2.5 Hz, 1H), 4.77 (t, J = 12.0 Hz, 1H), 4.02 (d, J = 6.5 Hz, 2H), 3.43 (s, 4H), 2.76 (s, 3H), 2.35 - 2.11 (m, 6H), 1.86 (d, J = 12.9 Hz, 2H), 1.56 - 1.34 (m, 5H) | 676 |
| 374 | 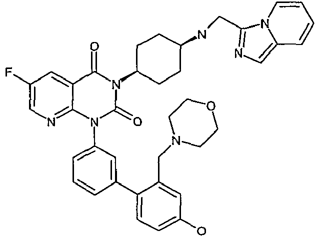 | 6-fluoro-1-[4'-hydroxy-2'-(morpholin-4-ylmethyl)biphenyl-3-yl]-3-{cis-4-[(imidazo[1,5-a]pyridin-3-ylmethyl)amino]cyclohexyl}pyrido[2 ,3-d]pyrimidine-2,4(1H,3H)-dione | .¹H NMR (300 MHz, DMSO) δ 9.50 (s, 1H), 8.65 - 8.47 (m, 2H), 8.31 (dt, J = 6.2, 1.4 Hz, 1H), 7.64 - 7.39 (m, 4H), 7.32 (d, J = 6.7 Hz, 1H), 7.25 (s, 1H), 7.14 (d, J = 8.3 Hz, 1H), 6.88 (s, 1H), 6.80 - 6.64 (m, 2H), 6.50 (s, 1H), 4.79 (s, 1H), 4.10 (d, J = 5.6 Hz, 2H), 3.47 - 3.38 (m, 6H), 2.75 (d, J = 11.7 Hz, 3H), 2.27 (s, 4H), 2.01 - 1.80 (m, 3H), 1.56 - 1.31 (m, 4H) | 676 |
| 375 | 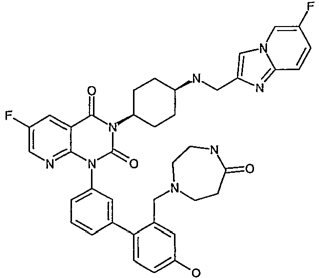 | 6-fluoro-3-(cis-4-{[(6-fluoroimidazo[1,2-a]pyridin-2-yl)methyl]amino}cyclohexyl)-1-{4'-hydroxy-2'-[(5-oxo-1,4-diazepan-1-yl)methyl]biphenyl-3-yl}pyrido[2,3-d]pyrimidine-2,4(1H,3H)-dione | ¹H NMR (400 MHz, DMSO) δ 8.71 (dd, J = 4.6, 2.1 Hz, 1H), 8.56 (d, J = 3.1 Hz, 1H), 8.28 (dd, J = 7.7, 3.1 Hz, 1H), 7.84 (s, 1H), 7.56 - 7.46 (m, 4H), 7.38 (d, J = 7.9 Hz, 1H), 7.32 - 7.22 (m, 2H), 7.10 (d, J = 8.2 Hz, 1H), 6.89 (d, J = 2.6 Hz, 1H), 6.75 (dd, J = 8.2, 2.6 Hz, 1H), 4.83 - 4.71 (m, 1H), 3.80 (d, J = 6.4 Hz, 2H), 3.39 (d, J = 8.2 Hz, 2H), 2.95 (s, 2H), 2.85 (s, 1H), 2.79 - 2.67 (m, 2H), 2.39 (s, 4H), 2.23 (s, 2H), 1.89 (d, J = 12.3 Hz, 2H), 1.80 - 1.71 (m, 1H), 1.53 - 1.39 (m, 4H) | 721 |
| 376 | 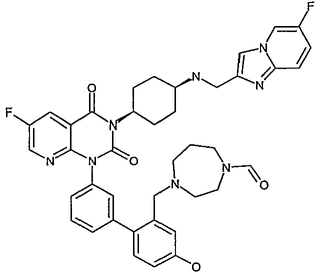 | 4-({3'-[6-fluoro-3-(cis-4-{[(6-fluoroimidazo[1,2-a]pyridin-2-yl)methyl]amino}cyclohexyl)-2,4-dioxo-3,4-dihydropyrido[2,3-d]pyrimidin-1(2H)-yl]-4-hydroxybiphenyl-2-yl}methyl)-1,4-diazepane-1-carbaldehyde | ¹H NMR (400 MHz, DMSO) δ 8.71 (dd, J = 4.5, 2.4 Hz, 1H), 8.57 (t, J = 3.1 Hz, 1H), 8.28 (dd, J = 7.8, 2.9 Hz, 1H), 7.93 (d, J = 11.3 Hz, 1H), 7.83 (s, 1H), 7.51 (dd, J = 10.1, 5.0 Hz, 2H), 7.43 (d, J = 1.8 Hz, 1H), 7.37 (d, J = 7.7 Hz, 1H), 7.32 - 7.21 (m, 2H), 7.09 (dd, J = 8.5, 2.1 Hz, 1H), 6.92 (dd, J = 4.4, 2.6 Hz, 1H), 6.73 (dd, J = 8.3, 2.4 Hz, 1H), 4.81 - 4.75 (m, 1H), 3.80 (d, J = 5.6 Hz, 2H), 3.46 (d, J = 5.9 Hz, 2H), 3.29 - 3.17 (m, 4H), 2.88 - 2.83 (m, 1H), 2.78 - 2.66 (m, 2H), 2.46 - 2.41 (m, 4H), 1.92 - 1.86 (m, 2H), 1.79 - 1.72 (m, 1H), 1.63 - 1.56 (m, 2H), 1.52 - 1.40 (m, 4H) | 735 |
| 377 | 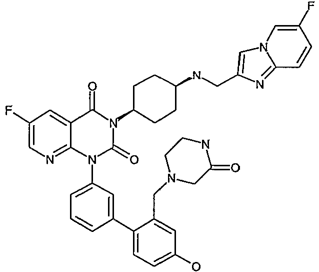 | 6-fluoro-3-(cis-4-{[(6-fluoroimidazo[1,2-a]pyridin-2-yl)methyl]amino}cyclohexyl)-1-{4'-hydroxy-2'-[(3-oxopiperazin-1-yl)methyl]biphenyl-3-yl}pyrido[2,3-d]pyrimidine-2,4(1H,3H)-dione | 1H NMR (299.947 MHz, DMSO) d 8.91 (dd, J = 4.5, 2.4 Hz, 2H), 8.61 (d, J = 3.1 Hz, 1H), 8.31 (dd, J = 7.6, 3.0 Hz, 1H), 8.11 (s, 1H), 7.68 (dd, J = 9.8, 5.2 Hz, 1H), 7.59 (t, J = 7.8 Hz, 1H), 7.49 - 7.33 (m, 4H), 7.20 (d, J = 8.5 Hz, 1H), 7.03 (s, 1H), 6.89 (d, J = 7.3 Hz, 1H), 4.79 (d, J = 11.5 Hz, 1H), 4.37 (s, 2H), 4.11 - 3.64 (m, 2H), 3.44 - 2.92 (m, 4H), 2.61 (t, J = 11.7 Hz, 1H), 2.13 (d, J = 13.1 Hz, 2H), 1.88 - 1.55 (m, 3H), remainding peaks hidden under solvent | 707 |
| 378 | 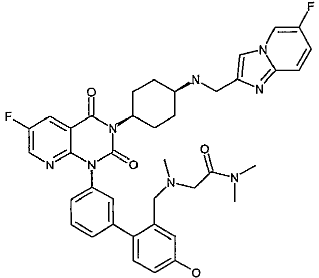 | N~2~({3'-[6-fluoro-3-(cis-4-{[(6-fluoroimidazo[1,2-a]pyridin-2-yl)methyl]amino}cyclohexyl)-2,4-dioxo-3,4-dihydropyrido[2,3-d]pyrimidin-1(2H)-yl]-4-hydroxybiphenyl-2-yl}methyl)-N,N,N~2~-trimethylglycinamide | ¹H NMR (300 MHz, DMSO) δ 8.93 (dd, J = 4.5, 2.4 Hz, 2H), 8.60 (s, 1H), 8.33 (dd, J = 7.6,3.0 Hz, 1H), 8.13 (s, 1H), 7.75 - 7.57 (m, 2H), 7.50 - 7.33 (m, 4H), 7.22 (s, 2H), 7.01 - 6.93 (m, 1H), 5.14 - 3.93 (m, 11H), 2.88 (d, J =16.5 Hz, 6H), 2.70 - 2.54 (m, 1H), 2.20 - 2.06 (m, 2H), 1.86 - 1.56 (m, 4H), remainding peaks hidden under solvent | 723 |
| 379 | 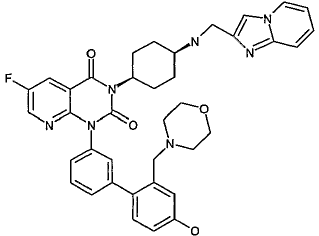 | 6-fluoro-1-[4'-hydroxy-2'-(morpholin-4-ylmethyl)biphenyl-3-yl]-3-{cis-4-[(imidazo[1,2-a]pyridin-2-ylmethyl)amino]cyclohexyl}pyrido[2 ,3-d]pyrimidine-2,4(1H,3H)-dione | ¹H NMR (300MHz, DMSO) δ 8.94 (s, 2H), 8.66 (d, J = 20.7 Hz, 1H), 8.59 (d, J = 2.9 Hz, 1H), 8.32 (dd, J=7.6, 3.0 Hz, 1H), 8.11 (s, 1H), 7.66 - 7.58 (m, 2H), 7.45 - 7.34 (m, 4H), 7.23 (d, J = 8.3 Hz, 1H), 7.10 (s, 1H), 7.04 - 6.93 (m, 2H), 4.79 (t, J = 18.7 Hz, 1H), 4.37 (s, 2H), 4.26 (s, 2H), 3.86 - 3.49 (m, 4H), 3.39 (s, 1H), 3.18 (s, 2H), 2.77 - 2.54 (m, 4H), 2.14 (d, J = 14.8 Hz, 2H), 1.83 - 1.56 (m, 4H) | 676 |
| 380 | 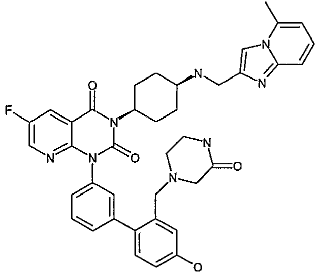 | 6-fluoro-1-{4'-hydroxy-2'-[(3-oxopiperazin-1-yl)methyl]biphenyl-3-yl}-3-(cis-4-{[(5-methylimidazo[1,2-a]pyridin-2-yl)methyl]amino}cyclohexyl)pyrido[ 2,3-d]pyrimidine-2,4(1H,3H)-dione | ¹H NMR (300MHz, DMSO) δ 8.99 (s, 1H), 8.61 (d, J = 2.7 Hz, 1H), 8.30 (dd, J = 7.7, 3.1 Hz, 1H), 8.06 (s, 1H), 7.59 (t, J = 7.6 Hz, 2H), 7.47 - 7.33 (m, 4H), 7.20 (d, J = 8.5 Hz, 1H), 7.03 (s, 1H), 6.97 (d, J = 6.7 Hz, 1H), 6.90 (d, J = 8.7 Hz, 1H), 4.81 (t, J = 11.6 Hz, 1H), 4.41 (s, 2H), 4.13 - 3.74 (m, 1H), 3.43 (s, 1H), 3.20 (d, J = 32.1 Hz, 3H), 2.86 (s, 1H), 2.68 - 2.53 (m, 10H), 2.15 (d, J = 14.4 Hz, 2H), 1.88 - 1.55 (m, 4H) | 703 |
| 381 | 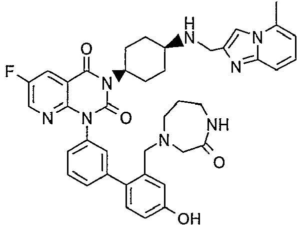 | 6-fluoro-1-{4'-hydroxy-2'-[(3-oxo-1,4-diazepan-1-yl)methyl]biphenyl-3-yl}-3-(cis-4-{[(5-methylimidazo[1,2-a]pyridin-2-yl)methyl]amino}cyclohexyl)pyrido[ 2,3-d]pyrimidine-2,4(1H,3H)-dione | - | 717 |

### EXAMPLE A

### Human Phosphodiesterase B2 Alpha Screen Assay

The assay uses recombinant Human Phosphodiesterase B2 (PDE4B2) produced in house (PrAZL0133), stored at -20 °C. The substrate uses cAMP, part of the Alpha Screen cAMP kit (Perkin Elmer, Cat# 6760625M), stored at 4 °C. The Alpha Screen kit also includes biotinylated cAMP, acceptor and donor beads.

The assay additions were as follows: Test compounds and controls were added to white 384-well flat-bottom plates (Greiner, Cat# 781075), 0.2 µl in 100% DMSO, followed by 10 µl PDE4B2 in reaction buffer. The reaction buffer constitution was: 50 mM Tris (pH 7.5), 8.3 mM MgC12,1.7 mM EGTA and 0.01% (w/v) Brij^{®}35. The enzyme and the compounds were incubated at room temperature for 15 minutes. Then 10 µl cAMP in reaction buffer was added. The assay was stopped after 60 minutes incubation at room temperature by adding 10 µl acceptor beads in detection buffer with 40 mM EDTA. The detection buffer constitution was: 5 mM Tris (pH 7.5), 0.1% (w/v) BSA and 0.1 % (v/v) Tween 20. This addition followed by an addition of 10 µl donor beads in detection buffer, with biotinylated cAMP. The plates were then incubated, dark at room temperature, for 5 hours followed by measurement on a Fusion^{™} -α analyser. pIC₅₀ values (presented in Table 14) were determined using Xlfit curve fitting using model 205.

**Table 14**

| Ex | PDE4B2 pIC₅₀ |
|---|---|
| 7 | 9.2 |
| 8 | 7.3 |
| 9 | 8.5 |

### EXAMPLE B

### Human Phosphodiesterase B2 Radiometric Assay

The assay uses recombinant Human Phosphodiesterase B2 (PDE4B2) produced in house (PrAZL0163), stored at -20 °C. This assay is based on the observation that 5'AMP, the product of the reaction catalysed by PDE4, binds preferentially to yttrium silicate SPA beads (Amersham Biosciences, UK) compared to the substrate, cAMP. Compounds at the appropriate concentration were preincubated at 30C for 30 min with an assay buffer containing 50 mM HEPES (pH 7.5), 8.3 mM MgCl2, 1.7 mM EGTA, 0.01% (w/v) Brij^{®}35 and 0.1 µg/mL recombinant PDE4B2. The reaction was started by the addition of [3H]cyclic AMP to give a final concentration of 8 nM, and was stopped 20 minutes after the addition of the substrate by the addition of yttrium silicate SPA beads containing 18mM Zn SO4. Bound [3H]cyclic AMP was measured using a Topcount NXT (Packard Bioscience, UK). pIC₅₀ values (presented in Table 15) were determined using Xlfit3 curve fitting, using model 205.

**Table 15**

| Example Number | PDE4B2 pIC50 | | Example Number | PDE4B2 pIC50 |
|---|---|---|---|---|
| 1 | 10.6 | | 209 | 11.2 |
| 2 | 9.8 | | 210 | 10.7 |
| 3 | 10.5 | | 211 | 10.8 |
| 4 | 10.4 | | 212 | 10.9 |
| 5 | 7.4 | | 213 | 10.8 |
| 6 | 11.1 | | 214 | 10.6 |
| 12 | 10.0 | | 215 | 10.5 |
| 13 | 10.2 | | 216 | 10.9 |
| 14 | 9.7 | | 217 | 10.5 |
| 15 | 10.9 | | 218 | 11.2 |
| 16 | 9.6 | | 219 | 11.3 |
| 17 | 11.1 | | 220 | 11.1 |
| 18 | 10.2 | | 221 | 11.0 |
| 19 | 11.5 | | 222 | 11.2 |
| 20 | 10.7 | | 223 | 11.0 |
| 21 | 10.3 | | 224 | 11.0 |
| 22 | 10.6 | | 225 | 10.8 |
| 23 | 10.4 | | 226 | 10.3 |
| 24 | 11.1 | | 227 | 11.1 |
| 25 | 10.6 | | 228 | 10.6 |
| 26 | 10.3 | | 229 | 11.2 |
| 27 | 9.6 | | 230 | 10.5 |
| 28 | 10.9 | | 231 | 9.9 |
| 29 | 10.1 | | 232 | 9.6 |
| 30 | 10.8 | | 233 | 10.3 |
| 31 | 9.3 | | 234 | 10.0 |
| 32 | 9.8 | | 235 | 9.9 |
| 33 | 11.0 | | 236 | 9.9 |
| 34 | 9.9 | | 237 | 10.4 |
| 35 | 8.2 | | 238 | 9.7 |
| 36 | 8.2 | | 239 | 9.5 |
| 37 | 8.2 | | 240 | 9.3 |
| 38 | 8.5 | | 241 | 10.1 |
| 39 | 8.0 | | 242 | 11.2 |
| 40 | 50% at 0.32uM | | 243 | 10.4 |
| 41 | 6.8 | | 244 | 11.0 |
| 42 | 7.7 | | 245 | 10.7 |
| 43 | 9.0 | | 246 | 10.7 |
| 44 | 10.2 | | 247 | 9.7 |
| 45 | 9.5 | | 248 | 8.9 |
| 46 | 8.7 | | 249 | 8.2 |
| 47 | 8.7 | | 250 | 9.7 |
| 48 | 9.4 | | 251 | 10.0 |
| 49 | 7.9 | | 252 | 9.4 |
| 50 | 11.1 | | 253 | 10.8 |
| 51 | 10.4 | | 254 | 10.5 |
| 52 | 8.6 | | 255 | 9.9 |
| 53 | 10.8 | | 256 | 10.8 |
| 54 | 10.8 | | 257 | 9.1 |
| 55 | 10.0 | | 258 | 8.7 |
| 56 | 10.6 | | 259 | 9.2 |
| 57 | 10.0 | | 260 | 9.3 |
| 58 | 10.8 | | 261 | 9.2 |
| 59 | 9.8 | | 262 | 9.1 |
| 60 | 8.2 | | 263 | 8.8 |
| 61 | 10.7 | | 264 | 8.6 |
| 62 | 9.5 | | 265 | 8.7 |
| 63 | 10.1 | | 266 | 9.0 |
| 64 | 10.1 | | 267 | 8.8 |
| 65 | 9.4 | | 268 | 10.9 |
| 66 | 7.2 | | 269 | 10.7 |
| 70 | 8.7 | | 270 | 11.2 |
| 71 | 10.1 | | 271 | 11.1 |
| 72 | 10.3 | | 272 | 9.1 |
| 73 | 10.6 | | 273 | 11.4 |
| 74 | 9.1 | | 274 | 10.2 |
| 75 | 9.5 | | 275 | 8.4 |
| 76 | 9.5 | | 277 | 9.1 |
| 77 | 10.6 | | 278 | 9.1 |
| 78 | 10.4 | | 279 | 10.4 |
| 79 | 10.7 | | 280 | 9.1 |
| 80 | 10.1 | | 281 | 9 |
| 81 | 11.2 | | 282 | 9.1 |
| 82 | 9.7 | | 283 | 8.9 |
| 83 | 8.2 | | 284 | 10.1 |
| 84 | 9.7 | | 285 | 9.1 |
| 85 | 9.5 | | 286 | 8.7 |
| 86 | 7.5 | | 287 | 9.2 |
| 87 | 9.5 | | 288 | 10.3 |
| 88 | 7.3 | | 289 | 10.9 |
| 89 | 8.7 | | 290 | 11 |
| 90 | 8.9 | | 291 | 9.7 |
| 91 | 8.1 | | 292 | 10.5 |
| 92 | 9.2 | | 293 | 8.7 |
| 93 | 8.3 | | 294 | 10.8 |
| 94 | 9.5 | | 295 | 10 |
| 95 | 10.7 | | 296 | 11 |
| 96 | 10.9 | | 297 | 10.5 |
| 97 | 11.2 | | 298 | 10.5 |
| 98 | 9.9 | | 299 | 10.8 |
| 99 | 9.9 | | 300 | 10.5 |
| 100 | 11.1 | | 301 | 10.4 |
| 101 | 10.0 | | 302 | 10 |
| 102 | 11.1 | | 303 | 10.7 |
| 103 | 11.8 | | 304 | 10.3 |
| 104 | 11.5 | | 305 | 10.3 |
| 105 | 10.6 | | 306 | 9.6 |
| 106 | 8.5 | | 307 | 8.6 |
| 107 | 7.0 | | 308 | 10.2 |
| 108 | 11.1 | | 309 | 10 |
| 109 | 10.8 | | 310 | 9.9 |
| 110 | 11.1 | | 311 | 9 |
| 111 | 10.9 | | 312 | 9.7 |
| 112 | 10.3 | | 313 | 9.4 |
| 113 | 10.9 | | 314 | 9.4 |
| 114 | 11.1 | | 315 | 9.8 |
| 115 | 10.4 | | 316 | 10.5 |
| 116 | 10.3 | | 317 | 11 |
| 117 | 9.9 | | 318 | 8.7 |
| 118 | 10.6 | | 319 | 9.2 |
| 119 | 9.5 | | 320 | 9.7 |
| 120 | 10.5 | | 321 | 10 |
| 121 | 9.8 | | 322 | 9.6 |
| 122 | 10.3 | | 323 | 9.6 |
| 123 | 10.4 | | 324 | 9.3 |
| 124 | 11.2 | | 325 | 8.7 |
| 125 | 9.8 | | 326 | 9.4 |
| 126 | 10.2 | | 327 | 9.6 |
| 127 | 9.7 | | 328 | 11 |
| 128 | 9.8 | | 329 | 9.9 |
| 129 | 10.4 | | 330 | 8.6 |
| 130 | 10.6 | | 331 | 9.4 |
| 131 | 9.9 | | 332 | 10.3 |
| 132 | 10.6 | | 333 | 8.7 |
| 133 | 11.0 | | 334 | 10.2 |
| 134 | 11.1 | | 335 | 11.1 |
| 135 | 11.5 | | 336 | 10.5 |
| 136 | 10.5 | | 337 | 10 |
| 137 | 10.9 | | 338 | 9.6 |
| 138 | 11.5 | | 339 | 10.4 |
| 207 | 9.5 | | 340 | 8.8 |
| 208 | 10.9 | | 341 | 9.3 |
| 342 | 10.5 | | 343 | 8.7 |
| 344 | 9.4 | | 359 | 10.6 |
| 345 | 10.5 | | 360 | 10.4 |
| 346 | 9.9 | | 361 | 9.7 |
| 348 | 10.6 | | 362 | 10.3 |
| 349 | 10.7 | | 369 | 8.2 |
| 350 | 10.8 | | 370 | 9.3 |
| 351 | 10.5 | | 371 | 9.4 |
| 352 | 10.8 | | 372 | 9.2 |
| 353 | 10.5 | | 373 | 9.8 |
| 354 | 10.4 | | 374 | 10.2 |
| 355 | 10.3 | | 375 | 10.9 |
| 356 | 10.2 | | 376 | 10.4 |
| 357 | 10.5 | | 377 | 10.2 |
| 358 | 10.9 | | 378 | 9.5 |

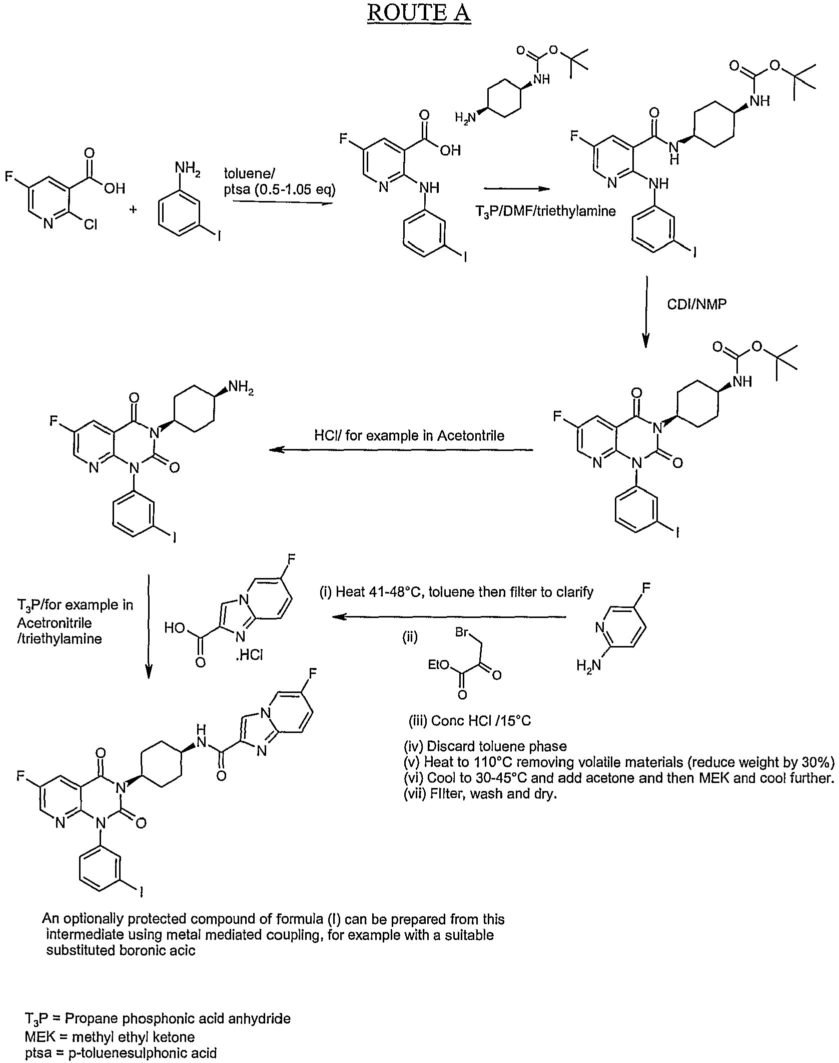

## Claims

1. A compound of formula (I): 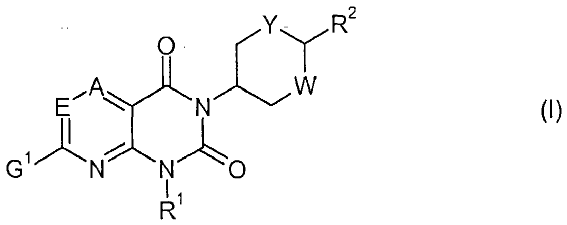 wherein:
A is N or CA¹;
E is N or CE¹;
W is (CH₂)ₙ;
Y is (CH₂)ₚ;
n and p are, independently 0 or 1;
R¹ is aryl or heteroaryl either of which is substituted by one or more of CO₂H, aryl, heteroaryl, (C₁₋₆ alkyl)NR³⁹R⁴⁰, C(O)NHaryl, C(O)N(C₁₋₆ alkyl)(aryl(C₁₋₆ alkyl)), C(O)NHheteroaryl, C(O)NHheterocyclyl, C(O)NH(CH₂)ᵥNH₂, C(O)NH(CH₂)ᵥNHCO₂(C₁₋₆ alkyl), C(O)NH((C₁₋₄ alkyl)aryl), C(O)N(C₁₋₄ alkyl)((C₁₋₄ alkyl)aryl), (C₁₋₆ alkyl)NHC(O)(C₁₋₆ alkoxy), heterocyclyl(C₁₋₄ alkoxy), CH=CH(aryl), C≡C(aryl), aryl(C₁₋₄ alkyl), heteroaryl(C₁₋₄ alkyl), aryloxy, heteroaryloxy, arylthio, heteroarylthio, CH=CH(heteroaryl) or C≡C(heteroaryl); and either of which may be additionally optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl) or CO₂(C₁₋₄ alkyl);
or R¹ is aryl(C₁₋₄ alkyl) or heteroaryl(C₁₋₄ alkyl) either of which is optionally substituted by halogen, cyano, hydroxy, C₁₋₄ alkyl, C₁₋₄ alkoxy, CF₃, OCF₃, C₁₋₄ alkylthio, S(O)(C₁₋₄ alkyl), S(O)₂(C₁₋₄ alkyl), CO₂H, CO₂(C₁₋₄ alkyl), C(O)NHaryl, C(O)NH(CH₂)ᵥH₂, C(O)NH(CH₂)ᵥNHCO₂(C₁₋₆ alkyl), aryl, heteroaryl, CH=CH(aryl), C≡C(aryl), CH=CH(heteroaryl) or C≡C(heteroaryl);
or R¹ is C₅₋₇ cycloalkyl optionally substituted by hydroxy, C₁₋₄ alkyl, CO₂H, CO₂(C₁₋₄ alkyl), aryl or heteroaryl;
or R¹ is C₁₋₁₀ alkyl;
or R¹ is C₁₋₆ alkyl substituted by NR⁴⁷R⁴³;
or R¹ is heterocyclyl optionally substituted by C₁₋₆ alkyl, aryl or heteroaryl; provided that R¹ is not: 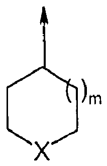
wherein:
X is S, S(O) or S(O)₂; and m is 0 or 1;
wherein the aryl or heteroaryl substituents of R¹ are optionally substituted by halogen, cyano, hydroxy, SH, C₁₋₄ alkyl, C₁₋₄ alkoxy, CF₃, OCF₃, C(O)H, C₁₋₆ alkylthio, S(O)(C₁₋₆ alkyl), S(O)₂(C₁₋₆ alkyl), CO₂H, CO₂(C₁₋₆ alkyl), NR⁴¹R⁴², C₁₋₆ alkyl (optionally substituted by halogen, OH, CO₂H, NR²⁹R³⁰, NHC(O)O(C₁₋₆ alkyl), OS(O)₂(C₁₋₆ alkyl) or heterocyclyl), C₁₋₆ alkoxy (optionally substituted by halogen, OH, CO₂H, NR³⁵R³⁶ or heterocyclyl), C₃₋₆ cycloalkyl (optionally substituted halogen, OH, CO₂H, NR³⁷R³⁸ or heterocyclyl) or heterocyclyl;
v is 1, 2, 3 or 4;
R² is NR⁵⁰C(O)R³ or NR⁴R⁵;
R³ is C₁₋₆ alkyl {optionally substituted by hydroxyl, C₁₋₆ alkoxy, NR⁷R⁸, heterocyclyl {optionally substituted by oxo, hydroxy, C₁₋₆ alkyl, CO₂(C₁₋₆ alkyl), aryl, heteroaryl, aryl(C₁₋₄ alkyl), heterocyclyl or C(O)(C₁₋₄ alkyl)phenyl}, aryl, heteroaryl, C₃₋₇ cycloalkyl, C₃₋₇ cycloalkyl(C₁₋₄ alkyl), CO₂H, CO₂(C₁₋₆ alkyl), aryl(C₁₋₄ alkoxy), aryl(C₁₋₄ alkylthio), S(O)₂(C₁₋₆ alkyl), NHC(O)heteroaryl or NHC(O)R⁶}, C₁₋₆ alkoxy, C₃₋₆ cycloalkyl {optionally substituted by hydroxyl, NR⁴³R⁴⁴ or C₁₋₆ alkyl}, heterocyclyl {optionally substituted by oxo, hydroxy, C₁₋₆ alkyl, amino, aryl, heteroaryl, aryl(C₁₋₄ alkyl), heteroaryl(C₁₋₄ alkyl), heterocyclyl or C(O)(C₁₋₄ alkyl)phenyl}, aryl(C₁₋₄ alkyl) {substituted by amino(C₁₋₄ alkyl)}, aryl or heteroaryl;
R⁴ is hydrogen, C₁₋₆ alkyl (optionally substituted by aryl or heteroaryl), aryl or heteroaryl;
R⁵ is hydrogen, C₁₋₆ alkyl (optionally substituted by hydroxyl, C₁₋₆ alkoxy, aryl, aryloxy, phenyl(C₁₋₆ alkoxy), heteroaryl, C₃₋₁₀ cycloalkyl, CO₂H, CO₂(C₁₋₆ alkyl), NHC(O)O(C₁₋₆ alkyl) or NHC(O)R⁶), C₁₋₆ alkoxy, C₃₋₆ cycloalkyl (optionally substituted by hydroxy, C₁₋₆ alkyl, phenyl, phenyl(C₁₋₆ alkyl), heteroaryl or heteroaryl(C₁₋₆ alkyl)), heterocyclyl (optionally substituted by C₁₋₆ alkyl, C(O)NH₂ or phenyl(C₁₋₆ alkyl)), aryl or heteroaryl;
R⁶ is C₁₋₆ alkyl or phenyl;
R⁷ and R⁸ are, independently, hydrogen, C₁₋₆ alkyl or phenyl(C₁₋₄ alkyl);
the foregoing phenyl, aryl and heteroaryl moieties of R³, R⁴, R⁵, R⁶, R⁷ and R⁸ are, independently, optionally substituted by: halogen, cyano, nitro, hydroxy, S(O)_{q}R⁹, OC(O)NR¹⁰R¹¹, NR¹²R¹³, NR¹⁴C(O)R¹⁵, NR¹⁶C(O)NR¹⁷R¹⁸, S(O)₂NR¹⁹R²⁰, NR²¹S(O)₂R²², C(O)NR²³R²⁴, C(O)R²⁵, CO₂R²⁶, NR²⁷CO₂R²⁸, OC(O)(C₁₋₆ alkyl), C₁₋₆ alkyl, C₁₋₆ hydroxyalkyl, C₁₋₆ haloalkyl, C₁₋₆ alkoxy(C₁₋₆)alkyl, amino(C₁₋₄ alkyl), di(C₁₋₆)alkylamino(C₁₋₆)alkyl, C₁₋₆ alkoxy, C₁₋₆ haloalkoxy, hydroxyl(C₁₋₆ alkoxy), heterocyclyl(C₁₋₆ alkoxy), C₁₋₆ alkoxy(C₁₋₆)alkoxy, amino(C₁₋₄ alkoxy), C₁₋₄ alkylamino(C₁₋₄ alkoxy) (itself optionally substituted by phenyl), di(C₁₋₄ alkyl)amino(C₁₋₄ alkoxy), C₁₋₆ alkylthio, C₂₋₆ alkenyl, C₂₋₆ alkynyl, C₃₋₁₀ cycloalkyl (itself optionally substituted by C₁₋₄ alkyl or oxo), methylenedioxy, difluoromethylenedioxy, heterocyclyl, heterocyclyl(C₁₋₄ alkyl), phenyl, phenyl(C₁₋₄)alkyl, phenoxy, phenylthio, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halogen, hydroxy, nitro, S(O)ᵣ(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃;
A¹, E¹ and G¹ are, independently, hydrogen, halogen, cyano, hydroxy, C₁₋₄ alkyl, C₁₋₄ alkoxy, CF₃ or OCF₃;
q and r are, independently, 0, 1 or 2;
unless otherwise stated heterocyclyl is optionally substituted by OH, C₁₋₆ alkyl, C₃₋₇ cycloalkyl, NR³¹R³², (C₁₋₆ alkyl)OH or (C₁₋₆ alkyl)NR³³R³⁴, NR⁴⁹CO₂(C₁₋₆ alkyl), CO₂(C₁₋₆ alkyl), C(O)(C₁₋₆ alkyl), C(O)heterocyclyl, heteroaryl, (C₁₋₆ alkyl)C(O)NR⁵³R⁵⁴, (C₁₋₆ alkyl)C(O)NR⁵⁵R⁵⁶, (C₁₋₆ alkyl)C(O)heterocyclyl or heterocyclyl;
R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷ and R²⁸ are, independently, C₁₋₆ alkyl {optionally substituted by halogen, hydroxy or C₁₋₆ alkoxy}, CH₂(C₂₋₆ alkenyl), phenyl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃} or heteroaryl {itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃};
R¹⁰, R¹¹, R¹², R¹³, R¹⁴, R¹⁵, R¹⁶, R¹⁷, R¹⁸, R¹⁹, R²⁰, R²¹, R²³, R²⁴, R²⁵, R²⁶ R²⁷ and R²⁸ can also be hydrogen;
R⁵⁰ is hydrogen or C₁₋₆ alkyl (optionally substituted by NR⁵¹R⁵²);
R³⁰, R³², R³⁴, R³⁶, R³⁸, R⁴⁰, R⁴², R⁴⁴ or R⁴⁸ are, independently, hydrogen, C₁₋₆ alkyl (optionally substituted by hydroxy, C₁₋₆ alkoxy, C₁₋₆ alkylthio, C₃₋₇ cycloalkyl (optionally substituted by hydroxy) or NR⁴⁵R⁴⁶), C₃₋₇ cycloalkyl (optionally substituted by hydroxy(C₁₋₆ alkyl)) or heterocyclyl (optionally substituted by C₁₋₆ alkyl);
R²⁹, R³¹, R³³, R³⁵, R³⁷, R³⁹, R⁴¹, R⁴³, R⁴⁵, R⁴⁶, R⁴⁷, R⁴⁹, R⁵¹, R⁵², R⁵³, R⁵⁴, R⁵⁵ and R⁵⁶ are independently, hydrogen or C₁₋₆ alkyl;
or a N-oxide thereof; or a pharmaceutically acceptable salt thereof.

2. A compound of formula (I) as claimed in claim 1 wherein E is CE¹ and E¹ is as defined in claim 1.

3. A compound of formula (I) as claimed in claim 1 or 2 wherein A is CH.

4. A compound of formula (I) as claimed in claim 1, 2 or 3 wherein G¹ is hydrogen or halogen.

5. A compound of formula (I) as claimed in claim 1, 2, 3 or 4 wherein n and p are both 1.

6. A compound of formula (I) as claimed in claim 1 wherein A is CA¹; E is CE¹; W and Y are both CH₂; and G¹, A¹ and E¹ arc, independently, hydrogen or halogen.

7. A compound of formula (I) as claimed in any one of the preceding claims wherein R¹ is phenyl substituted by phenyl {which is optionally substituted by halogen, hydroxy, CH(O), CO₂H, C₁₋₄ alkyl, C₁₋₄ alkyl(N(C₁₋₄ alkyl)₂), C₁₋₄ alkyl(NH₂), C₁₋₄ alkyl(NH(C₁₋₄ alkyl)), C₁₋₄ hydroxyalkyl, CF₃, C₁₋₄ alkylthio, C₁₋₄ alkyl(heterocyclyl) or C₁₋₄ alkylNHC(O)O(C₁₋₄ alkyl)} or heterocyclyl; and heterocyclyl is optionally substituted by C₁₋₆ alkyl.

8. A compound of formula (I) as claimed in any one of the preceding claims wherein R² is NHC(O)R³; and R³ is C₁₋₄ alkyl {substituted by NR⁷R⁸, heterocyclyl or heteroaryl}, C₃₋₇ cycloalkyl (optionally substituted by NR⁴³R⁴⁴) or heteroaryl; wherein R⁷, R⁸, R⁴³ and R⁴⁴ are as defined in claim 1; heteroaryl being optionally substituted by halo, V₁₋₄ alkyl, CF₃, C₁₋₄ alkoxy, OCF₃, heterocyclyl or amino(C₁₋₄ alkyl).

9. A compound of formula (IA) as claimed in claim 1: 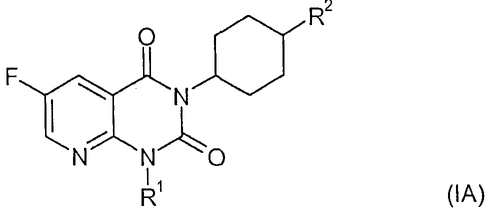 wherein:
R¹ is phenyl substituted by phenyl (substituted by CH₂(heterocyclyl)) and
optionally further substituted by hydroxyl; the heterocyclyl being optionally substituted by C₁₋₆ alkyl;
R² is NHC(O)R³;
R³ is Heteroaryl, Heterocyclyl, C₁₋₆ alkoxy or C₃₋₇ cycloalkyl; heteroaryl being optionally substituted by halo, C₁₋₄ alkyl or heterocyclyl; and, heterocyclyl being optionally substituted by C₁₋₄ alkyl;
and wherein heterocyclyl is a non-aromatic 5- or 6- membered ring optionally fused to one or more other non-aromatic rings and optionally fused to a benzene ring, comprising at least one heteroatom selected from the group comprising nitrogen, oxygen and sulphur;
and wherein heteroaryl is an aromatic 5- or 6- membered ring, optionally fused to one or more other rings (which may be carbocyclic or heterocyclic, and aromatic or non-aromatic), comprising at least one heteroatom selected from the group comprising nitrogen, oxygen and sulphur; or an N-oxide thereof, or an S-oxide or S-dioxide thereof;
or an N-oxide thereof; or a pharmaceutically acceptable salt thereof;

10. A compound of formula (IA) as claimed in claim 9, wherein the first phenyl of R¹ is substituted by the second phenyl of R¹ in the 3-position.

11. A compound of formula (I) as claimed in claim 1 which is selected from:
6-fluoro-*N*-{*cis*-4-[6-fluoro-2,4-dioxo-1-[4'-(piperazin-1-ylmethyl)biphenyl-3-yl]-1,4-dihydropyrido[2,3-*d*]pyrimidin-3(2*H*)-yl]cyclohexyl}imidazo[1,2-*a*]pyridine-2-carboxamide;
6-fluoro-*N*-{*cis*-4-[6-fluoro-2,4-dioxo-1-[4'-(2-pyrrolidin-1-ylethoxy)biphenyl-3-yl]-1,4-dihydropyrido[2,3-*d*]pyrimidin-3(2*H*)-yl]cyclohexyl} imidazo[1,2-*a*]pyridine-2-carboxamide;
6-fluoro-*N*-{*cis*-4-[6-fluoro-1-(4'-{3-[(3-hydroxypropyl)amino]propyl}biphenyl-3-yl)-2,4-dioxo-1,4-dihydropyrido[2,3-*d*]pyrimidin-3(2*H*)-yl]cyclohexyl}imidazo[1,2-*a*]pyridine-2-carboxamide;
6-Fluoro-3-(cis-4-{[(6-fluoroimidazo[1,2-*a*]pyridin-2-yl)methyl]amino}cyclohexyl)-1-[4'-hydroxy-2'-(morpholin-4-ylmethyl)biphenyl-3-yl]pyrido[2,3-*d*]pyrimidine-2,4(1*H*,3*H*)-dione; or,
*N*-{*cis*-4-[6-fluoro-1-{4'-[(4-methyl-1,4-diazepan-1-yl)methyl]biphenyl-3-yl}-2,4-dioxo-1,4-dihydropyrido[2,3-*d*]pyrimidin-3(2*H*)-yl]cyclohexyl}-6-methylpyridine-2-carboxamide;
or a pharmaceutically acceptable salt thereof.

12. A compound of formula (I) as claimed in claim 1 which is: 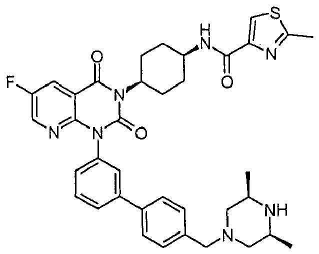 or a pharmaceutically acceptable salt thereof.

13. A compound of formula (I) as claimed in claim 1 which is: 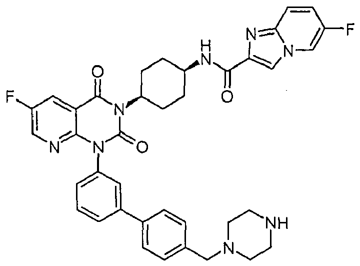 or a pharmaceutically acceptable salt thereof.

14. A process for the preparation of a compound of formula (I) as claimed in claim 1, the process comprising:
a) when R² is NHC(O)R³, removing the Boc protecting group from a compound of formula (II): 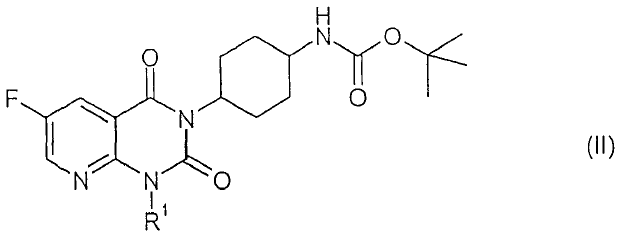 wherein R¹, G¹, E, A, Y and W arc as defined in claim 1, and reacting the product so formed with an acid or acid derivative of formula (III):
LG-R³ (III)
wherein R³ is as defined in formula (I), and LG is a leaving group; or,
b) conducting the ring closing reaction below: 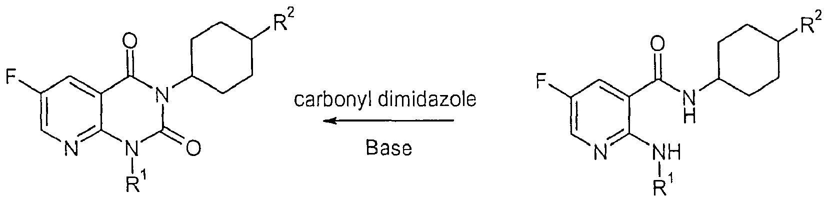 using a method know in the art.

15. A pharmaceutical composition which comprises a compound of the formula (I), or a pharmaceutically acceptable salt thereof, as claimed in any one of claims 1 to 13, and a pharmaceutically acceptable adjuvant, diluent or carrier.

16. A compound of the formula (I), or a pharmaceutically acceptable salt thereof, as claimed in any one of claims 1 to 13, for use in therapy.

17. Use of a compound of formula (I), or a pharmaceutically acceptable salt thereof, as claimed in any one of claims 1 to 13, in the manufacture of a medicament.

18. A compound of formula (I), or a pharmaceutically acceptable salt thereof, as claimed in any one of claims 1 to 13, for use in the treatment of asthma; chronic obstructive pulmonary disease (COPD); bronchitis; emphysema; bronchiectasis; cystic fibrosis; sarcoidosis; farmer's lung; hypersensitivity pneumonitis; lung fibrosis; complications of lung transplantation; vasculitic and thrombotic disorders of the lung vasculature, and pulmonary hypertension; antitussive activity; acute and chronic rhinitis; perennial and seasonal allergic rhinitis; nasal polyposis; acute viral infection; eosinophilic esophagitis; arthritides associated with or including osteoarthritis/osteoarthrosis; cervical and lumbar spondylitis, and low back and neck pain; osteoporosis; rheumatoid arthritis and Still's disease; seronegative spondyloarthropathies; septic arthritis and other infection-related arthopathies and bone disorders; acute and chronic crystal-induced synovitis; Behcet's disease; primary and secondary Sjogren's syndrome; systemic sclerosis and limited scleroderma; systemic lupus erythematosus, mixed connective tissue disease, and undifferentiated connective tissue disease; inflammatory myopathies; polymalgia rheumatica; juvenile arthritis; vasculitides; low back pain; Familial Mediterranean fever, Muckle-Wells syndrome, and Familial Hibernian Fever, Kikuchi disease; drug-induced arthalgias, tendonititides, and myopathies;
arthritides; intervertebral disc degeneration; temporomandibular joint degeneration; bone remodelling disease; polychondritits; scleroderma; mixed connective tissue disorder; spondyloarthropathies; periodontal disease;
psoriasis, atopic dermatitis, contact dermatitis; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis; cutaneous lymphomas, non-melanoma skin cancer; fixed drug eruptions;
blepharitis; conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral , fungal, and bacterial; glossitis, gingivitis, periodontitis; oesophagitis; eosinophilic gastro-enteritis, mastocytosis, Crohn's disease, colitis; proctitis, pruritis ani; coeliac disease, irritable bowel syndrome, food-related allergies which may have effects remote from the gut; hepatitis; fibrosis and cirrhosis of the liver; cholecystitis; pancreatitis;
nephritis; nephrotic syndrome; cystitis; acute and chronic urethritis, prostatitis, epididymitis, oophoritis and salpingitis; vulvo-vaginitis; Peyronie's disease; erectile dysfunction;
allograft rejection;
Alzheimer's disease and other dementing disorders; amyloidosis; multiple sclerosis and other demyelinating syndromes; cerebral atherosclerosis and vasculitis; temporal arteritis; myasthenia gravis; acute and chronic pain; neurosarcoidosis; central and peripheral nervous system complications of malignant, infectious or autoimmune processes;
Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;
acquired immune deficiency syndrome (AIDS), leprosy, Sezary syndrome; paraneoplastic syndromes;
atherosclerosis, affecting the coronary and peripheral circulation; pericarditis; myocarditis, inflammatory and auto-immune cardiomyopathies; ischaemic reperfusion injuries; endocarditis, valvulitis, and aortitis; vasculitides; phlebitis; thrombosis; prostate, breast, lung, ovarian, pancreatic, bowel and colon, stomach, skin and brain tumors and malignancies affecting the bone marrow and lymphoproliferative systems; irritable bowel disorder, or non-inflammatory diarrhea.

19. A pharmaceutical product comprising, in combination, a first active ingredient which is a compound of formula (I), or a pharmaceutically acceptable salt thereof, as claimed in any one of claims 1 to 13, and at least one further active ingredient selected from :-
• a β2. adrenoceptor agonist,
• a modulator of chemokine receptor function,
• an inhibitor of kinase function,
• a protease inhibitor,
• a steroidal glucocorticoid receptor agonist,
• an anticholinergic agent, or
• a non-steroidal glucocorticoid receptor agonist.

20. An intermediate compound of the following formula :

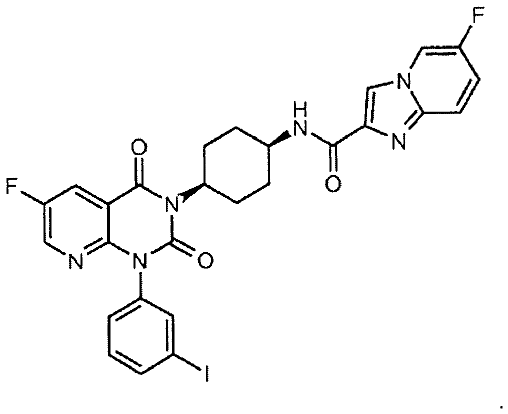

## Patentansprüche

1. Verbindung der Formel (I): 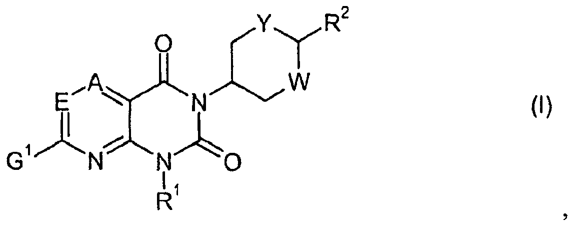 wobei:
A N oder CA¹ ist;
E N oder CE¹ ist;
W (CH₂)ₙ ist;
Y (CH₂)ₚ ist;
n und p unabhängig 0 oder 1 sind;
R¹ Aryl oder Heteroaryl ist, von denen eines substituiert ist durch ein oder mehrere von CO₂H, Aryl, Heteroaryl, (C₁₋₆-Alkyl)NR³⁹R⁴⁰, C(O)NH-Aryl, C(O)N(C₁₋₆-Alkyl)(Aryl(C₁₋₆-Alkyl)), C(O)NH-Heteroaryl, C(O)NH-Heterocyclyl, C(O)NH(CH₂)ᵥNH₂, C(O)NH(CH₂)ᵥNHCO₂(C₁₋₆-Alkyl), C(O)NH((C₁₋₄-Alkyl)Aryl), C(O)N(C₁₋₄-Al-kyl)((C₁₋₄-Alkyl)Aryl), (C₁₋₆-Alkyl)NHC(O)(C₁₋₆-Alₖoxy), Heterocyclyl(C₁₋₄-Alkoxy), CH=CH(Aryl), C≡C(Aryl), Aryl(C₁₋₄-Alkyl), Heteroaryl(C₁₋₄-Alkyl), Aryloxy, Heteroaryloxy, Arylthio, Heteroarylthio, CH=CH(Heteroaryl) oder C≡C(Heteroaryl); und von denen eines weiterhin gegebenenfalls substituiert sein kann durch Halogen, Cyano, Hydroxy, C₁₋₄-Alkyl, C₁₋₄-Alkoxy, CF₃, OCF₃, C₁₋₄-Alkylthio, S(O)(C₁₋₄-Alkyl), S(O)₂(C₁₋₄-Alkyl) oder CO₂(C₁₋₄-Alkyl);
oder R¹ Aryl(C₁₋₄-Alkyl) oder Heteroaryl(C₁₋₄-Alkyl) ist, von denen eines gegebenenfalls substituiert ist durch Halogen, Cyano, Hydroxy, C₁₋₄-Alkyl, C₁₋₄-Alkoxy, CF₃, OCF₃, C₁₋₄-Alkylthio, S(O)(C₁₋₄-Alkyl), S(O)₂(C₁₋₄-Alkyl), CO₂H, CO₂(C₁₋₄-Alkyl), C(O)NH-Aryl, C(O)NH(CH₂)ᵥNH₂, C(O)NH(CH₂)ᵥNHCO₂(C₁₋₆-Alkyl), Aryl, Heteroaryl, CH=CH(Aryl), C≡C(Aryl), CH=CH(Heteroaryl), C≡C(Heteroaryl);
oder R¹ C₅₋₇-Cycloalkyl ist, das gegebenenfalls substituiert ist durch Hydroxy, C₁₋₄-Alkyl, CO₂H, CO₂(C₁₋₄-Alkyl), Aryl oder Heteroaryl;
oder R¹ C₁₋₁₀-Alkyl ist;
oder R¹ C₁₋₆-Alkyl, substituiert durch NR⁴⁷R⁴⁸, ist;
oder R¹ Heterocyclyl ist, das gegebenenfalls substituiert ist durch C₁₋₆-Alkyl, Aryl oder Heteroaryl;
mit der Maßgabe, dass R¹ nicht: 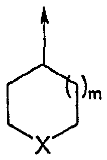
ist, wobei:
X S, S(O) oder S(O)₂ ist; und m 0 oder 1 ist;
wobei die Aryl- oder Heteroaryl-Substituenten von R¹ gegebenenfalls substituiert sind durch Halogen, Cyano, Hydroxy, SH, C₁₋₄-Alkyl, C₁₋₄-Alkoxy, CF₃, OCF₃, C(O)H, C₁₋₆-Alkylthio, S(O)(C₁₋₆-Alkyl), S(O)₂(C₁₋₆-Alkyl), CO₂H, CO₂(C₁₋₆-Alkyl), NR⁴¹R⁴², C₁₋₆-Alkyl (gegebenenfalls substituiert durch Halogen, OH, CO₂H, NR²⁹R³⁰, NHC(O)O(C₁₋₆-Alkyl), OS(O)₂(C₁₋₆-Alkyl) oder Heterocyclyl), C₁₋₆-Alkoxy (gegebenenfalls substituiert durch Halogen, OH, CO₂H, NR³⁵R³⁶ oder Heterocyclyl), C₃₋₆-Cycloalkyl (gegebenenfalls substituiert durch Halogen, OH, CO₂H, NR³⁷R³⁸ oder Heterocyclyl) oder Heterocyclyl;
v 1, 2, 3 oder 4 ist;
R² NR⁵⁰C(O)R³ oder NR⁴R⁵ ist;
R³ C₁₋₆-Alkyl {gegebenenfalls substituiert durch Hydroxyl, C₁₋₆-Alkoxy, NR⁷R⁸, Heterocyclyl {gegebenenfalls substituiert durch Oxo, Hydroxy, C₁₋₆-Alkyl, CO₂(C₁₋₆-Alkyl), Aryl, Heteroaryl, Aryl(C₁₋₄-Alkyl), Heterocyclyl oder C(O)(C₁₋₄-Alkyl)Phenyl}, Aryl, Heteroaryl, C₃₋₇-Cycloalkyl, C₃₋₇-Cycloalkyl(C₁₋₄-Alkyl), CO₂H, CO₂(C₁₋₆-Alkyl), Aryl(C₁₋₄-Alkoxy), Aryl(C₁₋₄-Alkylthio), S(O)₂(C₁₋₆-Alkyl), NHC(O)Heteroaryl oder NHC(O)R⁶}, C₁₋₆-Alk-oxy, C₃₋₆-Cycloalkyl {gegebenenfalls substituiert durch Hydroxyl, NR⁴³R⁴⁴ oder C₁₋₆-Al-kyl}, Heterocyclyl {gegebenenfalls substituiert durch Oxo, Hydroxy, C₁₋₆-Alkyl, Amino, Aryl, Heteroaryl, Aryl(C₁₋₄-Alkyl), Heteroaryl(C₁₋₄-Alkyl), Heterocyclyl oder C(O)(C₁₋₄-Alkyl)Phenyl}, Aryl(C₁₋₄-Alkyl) {substituiert durch Amino(C₁₋₄-Alkyl)}, Aryl oder Heteroaryl ist;
R⁴ Wasserstoff, C₁₋₆-Alkyl (gegebenenfalls substituiert durch Aryl oder Heteroaryl), Aryl oder Heteroaryl ist;
R⁵ Wasserstoff, C₁₋₆-Alkyl (gegebenenfalls substituiert durch Hydroxyl, C₁₋₆-Alkoxy, Aryl, Aryloxy, Phenyl(C₁₋₆-Alkoxy), Heteroaryl, C₃₋₁₀-Cycloalkyl, CO₂H, CO₂(C₁₋₆-Alkyl), NHC(O)O(C₁₋₆-Alkyl) oder NHC(O)R⁶), C₁₋₆-Alkoxy, C₃₋₆-Cycloalkyl (gegebenenfalls substituiert durch Hydroxy, C₁₋₆-Alkyl, Phenyl, Phenyl(C₁₋₆-Alkyl), Heteroaryl oder Heteroaryl(C₁₋₆-Alkyl)), Heterocyclyl (gegebenenfalls substituiert durch C₁₋₆-Alkyl, C(O)NH₂ oder Phenyl(C₁₋₆-Alkyl)), Aryl oder Heteroaryl ist;
R⁶ C₁₋₆-Alkyl oder Phenyl ist;
R⁷ und R⁸ unabhängig Wasserstoff, C₁₋₆-Alkyl oder Phenyl(C₁₋₄-Alkyl) sind;
die vorstehenden Phenyl-, Aryl- und Heteroaryl-Komponenten von R³, R⁴, R⁵, R⁶, R⁷ und R⁸ unabhängig gegebenenfalls substituiert sind durch: Halogen, Cyano, Nitro, Hydroxy, S(O)_{q}R⁹, OC(O)NR¹⁰R¹¹, NR¹²R¹³, NR¹⁴C(O)R¹⁵, NR¹⁶C(O)NR¹⁷R¹⁸, S(O)₂NR¹⁹R²⁰, NR²¹S(O)₂R²², C(O)NR²³R²⁴, C(O)R²⁵, CO₂R²⁶, NR²⁷COR²⁸, OC(O)(C₁₋₆-Alkyl), C₁₋₆-Alkyl, C₁₋₆-Hydroxyalkyl, C₁₋₆-Halogenalkyl, C₁₋₆-Alkoxy(C₁₋₆)Alkyl, Amino(C₁₋₄-Alkyl), Di(C₁₋₆)Alkylamino(C₁₋₆)Alkyl, C₁₋₆-Alkoxy, C₁₋₆-Halogenalkoxy, Hydroxyl(C₁₋₆-Alkoxy), Heterocyclyl(C₁₋₆-Alkoxy), C₁₋₆-Alkoxy(C₁₋₆)Alkoxy, Amino(C₁₋₄-Alkoxy), C₁₋₄-Alkylamino(C₁₋₄-Alkoxy) (selbst gegebenenfalls substituiert durch Phenyl), Di(C₁₋₄-Alkyl)Amino(C₁₋₄-Alkoxy), C₁₋₆-Alkylthio, C₂₋₆-Alkenyl, C₂₋₆-Alkinyl, C₃₋₁₀-Cycloalkyl (selbst gegebenenfalls substituiert durch C₁₋₄-Alkyl oder Oxo), Methylendioxy, Difluormethylendioxy, Heterocyclyl, Heterocyclyl(C₁₋₄-Alkyl), Phenyl, Phenyl(C₁₋₄)Alkyl, Phenoxy, Phenylthio, Phenyl(C₁₋₄)Alkoxy, Heteroaryl, Heteroaryl(C₁₋₄)Alkyl, Heteroaryloxy oder Heteroaryl(C₁₋₄)Alkoxy; wobei jede der unmittelbar vorstehenden Phenyl-und Heteroarylkomponenten gegebenenfalls substituiert ist mit Halogen, Hydroxy, Nitro, S(O)ᵣ(C₁₋₄-Alkyl), S(O)₂NH₂, S(O)₂NH(C₁₋₄-Alkyl), S(O)₂N(C₁₋₄-Alkyl)₂, Cyano, C₁₋₄-Alkyl, C₁₋₄-Alkoxy, C(O)NH₂, C(O)NH(C₁₋₄-Alkyl), C(O)N(C₁₋₄-Alkyl)₂, CO₂H, CO₂(C₁₋₄-Alkyl), NHC(O)(C₁₋₄-Alkyl), NHS(O)₂(C₁₋₄-Alkyl), C(O)(C₁₋₄-Alkyl), CF₃ oder OCF₃;
A¹, E¹ und G¹ unabhängig Wasserstoff, Halogen, Cyano, Hydroxy, C₁₋₄-Alkyl, C₁₋₄-Alkoxy, CF₃ oder OCF₃ sind;
q und r unabhängig 0, 1 oder 2 sind;
wenn nicht anders angegeben, Heterocyclyl gegebenenfalls substituiert ist durch OH, C₁₋₆-Alkyl, C₃₋₇-Cycloalkyl, NR³¹R³², (C₁₋₆-Alkyl)OH oder (C₁₋₆-Alkyl)NR³³R³⁴, NR⁴⁹CO₂(C₁₋₆-Alkyl), CO₂(C₁₋₆-Alkyl), C(O)(C₁₋₆-Alkyl), C(O)Heterocyclyl, Heteroaryl, (C₁₋₆-Alkyl)C(O)NR⁵³R⁵⁴, (C₁₋₆-Alkyl)C(O)NR⁵⁵R⁵⁶, (C₁₋₆-Alkyl)C(O)Heterocyclyl oder Heterocyclyl;
R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷ und R²⁸ unabhängig C₁₋₆-Alkyl {gegebenenfalls substituiert durch Halogen, Hydroxy oder C₁₋₆-Alkoxy}, CH₂(C₂₋₆-Alkenyl), Phenyl {selbst gegebenenfalls substituiert durch Halogen, Hydroxy, Nitro, NH₂, NH(C₁₋₄-Alkyl), N(C₁₋₄-Alkyl)₂, S(O)₂(C₁₋₄-Alkyl), S(O)₂NH₂, S(O)₂NH(C₁₋₄-Alkyl), S(O)₂N(C₁₋₄-Alkyl)₂, Cyano, C₁₋₄-Alkyl, C₁₋₄-Alkoxy, C(O)NH₂, C(O)NH(C₁₋₄-Alkyl), C(O)N(C₁₋₄-Alkyl)₂, CO₂H, CO₂(C₁₋₄-Alkyl), NHC(O)(C₁₋₄-Alkyl), NHS(O)₂(C₁₋₄-Alkyl), C(O)(C₁₋₄-Alkyl), CF₃ oder OCF₃} oder Heteroaryl {selbst gegebenenfalls substituiert durch Halogen, Hydroxy, Nitro, NH₂, NH(C₁₋₄-Alkyl), N(C₁₋₄-Alkyl)₂, S(O)₂(C₁₋₄-Alkyl), S(O)₂NH₂, S(O)₂NH(C₁₋₄-Alkyl), S(O)₂N(C₁₋₄-Alkyl)₂, Cyano, C₁₋₄-Alkyl, C₁₋₄-Alkoxy, C(O)NH₂, C(O)NH(C₁₋₄-Alkyl), C(O)N(C₁₋₄-Alkyl)₂, CO₂H, CO₂(C₁₋₄-Alkyl), NHC(O)(C₁₋₄-Alkyl), NHS(O)₂(C₁₋₄-Alkyl), C(O)(C₁₋₄-Alkyl), CF₃ oder OCF₃} sind;
R¹⁰, R¹¹, R¹², R¹³, R¹⁴, R¹⁵, R¹⁶, R¹⁷, R¹⁸, R¹⁹, R²⁰, R²¹, R²³, R²⁴, R²⁵, R²⁶, R²⁷ und R²⁸ ebenso Wasserstoff sein können;
R⁵⁰ Wasserstoff oder C₁₋₆-Alkyl (gegebenenfalls substituiert durch NR⁵¹R⁵²) ist;
R³⁰, R³², R³⁴, R³⁶, R³⁸, R⁴⁰, R⁴², R⁴⁴ oder R⁴⁸ unabhängig Wasserstoff, C₁₋₆-Alkyl (gegebenenfalls substituiert durch Hydroxy, C₁₋₆-Alkoxy, C₁₋₆-Alkylthio, C₃₋₇-Cycloalkyl (gegebenenfalls substituiert durch Hydroxy) oder NR⁴⁵R⁴⁶), C₃₋₇-Cycloalkyl (gegebenenfalls substituiert durch Hydroxy(C₁₋₆-Alkyl)) oder Heterocyclyl (gegebenenfalls substituiert durch C₁₋₆-Alkyl) sind;
R²⁹, R³¹, R³³, R³⁵, R³⁷, R³⁹, R⁴¹, R⁴³, R⁴⁵, R⁴⁶, R⁴⁷, R⁴⁹, R⁵¹, R⁵², R⁵³, R⁵⁴, R⁵⁵ und R⁵⁶ unabhängig Wasserstoff oder C₁₋₆-Alkyl sind;
oder ein N-Oxid davon oder ein pharmazeutisch akzeptables Salz davon.

2. Verbindung der Formel (I) nach Anspruch 1, wobei E CE¹ ist und E¹ wie in Anspruch 1 definiert ist.

3. Verbindung der Formel (I) nach Anspruch 1 oder 2, wobei A CH ist.

4. Verbindung der Formel (I) nach Anspruch 1, 2 oder 3, wobei G¹ Wasserstoff oder Halogen ist.

5. Verbindung der Formel (I) nach Anspruch 1, 2, 3 oder 4, wobei n und p beide 1 sind.

6. Verbindung der Formel (I) nach Anspruch 1, wobei A CA¹ ist; E CE¹ ist; W und Y beide CH₂ sind und G¹, A¹ und E¹ unabhängig Wasserstoff oder Halogen sind.

7. Verbindung der Formel (I) nach einem der vorhergehenden Ansprüche, wobei R¹ Phenyl, substituiert durch Phenyl {das gegebenenfalls substituiert ist durch Halogen, Hydroxy, CH(O), CO₂H, C₁₋₄-Alkyl, C₁₋₄-Alkyl(N(C₁₋₄-Alkyl)₂), C₁₋₄-Alkyl(NH₂), C₁₋₄-Alkyl(NH(C₁₋₄-Alkyl)), C₁₋₄-Hydroxyalkyl, CF₃, C₁₋₄-Alkylthio, C₁₋₄-Alkyl(Heterocyclyl) oder C₁₋₄-Alkyl-NHC(O)O(C₁₋₄-Alkyl)} oder Heterocyclyl, ist; und Heterocyclyl gegebenenfalls substituiert ist durch C₁₋₆-Alkyl.

8. Verbindung der Formel (I) nach einem der vorhergehenden Ansprüche, wobei R² NHC(O)R³ ist und R³ C₁₋₄-Alkyl {substituiert durch NR⁷R⁸, Heterocyclyl oder Heteroaryl}, C₃₋₇-Cycloalkyl (gegebenenfalls substituiert durch NR⁴³R⁴⁴) oder Heteroaryl ist; wobei R⁷, R⁸, R⁴³ und R⁴⁴ wie in Anspruch 1 definiert sind; wobei das Heteroaryl gegebenenfalls substituiert ist durch Halogen, C₁₋₄-Alkyl, CF₃, C₁₋₄-Alkoxy, OCF₃, Heterocyclyl oder Amino(C₁₋₄-Alkyl).

9. Verbindung der Formel (IA) nach Anspruch 1: 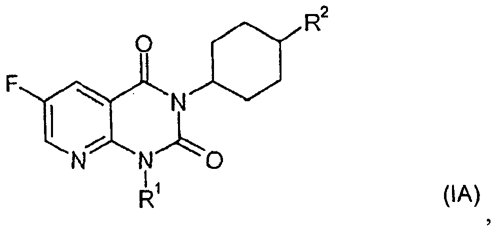 wobei:
R¹ Phenyl, substituiert durch Phenyl (substituiert durch CH₂(Heterocyclyl)) und gegebenenfalls ferner substituiert durch Hydroxyl, ist; wobei das Heterocyclyl gegebenenfalls substituiert ist durch C₁₋₆-Alkyl;
R² NHC(O)R³ ist;
R³ Heteroaryl, Heterocyclyl, C₁₋₆-Alkoxy oder C₃₋₇-Cycloalkyl ist; wobei das Heteroaryl gegebenenfalls substituiert ist durch Halogen, C₁₋₄-Alkyl oder Heterocyclyl; und das Heterocyclyl gegebenenfalls substituiert ist durch C₁₋₄-Alkyl;
und wobei das Heterocyclyl ein nicht-aromatischer 5- oder 6gliedriger Ring ist, der gegebenenfalls an einen oder mehrere andere nicht-aromatische Ringe anelliert und gegebenenfalls an einen Benzolring anelliert ist, umfassend mindestens ein Heteroatom, ausgewählt aus der Gruppe, umfassend Stickstoff, Sauerstoff und Schwefel;
und wobei das Heteroaryl ein aromatischer 5- oder 6gliedriger Ring ist, der gegebenenfalls an einen oder mehrere andere Ringe anelliert ist (die carbocyclisch oder heterocyclisch und aromatisch oder nicht-aromatisch sein können), umfassend mindestens ein Heteroatom, ausgewählt aus der Gruppe, umfassend Stickstoff, Sauerstoff und Schwefel; oder ein N-Oxid davon oder ein S-Oxid oder S-Dioxid davon;
oder ein N-Oxid davon oder ein pharmazeutisch akzeptables Salz davon.

10. Verbindung der Formel (IA) nach Anspruch 9, wobei das erste Phenyl von R¹ in der 3-Stellung durch das zweite Phenyl von R¹ substituiert ist.

11. Verbindung der Formel (I) nach Anspruch 1, ausgewählt aus:
6-Fluor-*N*-{*cis*-4-[6-fluor-2,4-dioxo-1-[4'-(piperazin-1-ylmethyl)biphenyl-3-yl]-1,4-dihydro-pyrido[2,3-*d*]pyrimidin-3(2*H*)-yl]cyclohexyl}imidazo[1,2-*a*]pyridin-2-carboxamid;
6-Fluor-*N*-{*cis*-4-[6-fluor-2,4-dioxo-1-[4'-(2-pyrrolidin-1-ylethoxy)biphenyl-3-yl]-1,4-di-hydropyrido[2,3-*d*]pyrimidin-3(2*H*)-yl]cyclohexyl} imidazo[1,2-*a*]pyridin-2-carboxamid;
6-Fluor-*N*-{*cis-*4-[6-fluor-1-(4'-{3-[(3-hydroxypropyl)amino]propyl}biphenyl-3-yl)-2,4-di-oxo-1,4-dihydropyrido[2,3-*d*]pyrimidin-3(2*H*)-yl]cyclohexyl}imidazo[1,2-*a*]pyridin-2-carboxamid;
6-Fluor-3-(*cis*-4- {[(6-fluorimidazo[1,2-*a*]pyridin-2-yl)methyl]amino} cyclohexyl)-1-[4'-hydroxy-2'-(morpholin-4-ylmethyl)biphenyl-3-yl]pyrido[2,3-*d*]pyrimidin-2,4(1*H*,3*H*)-dion oder
*N*-{*cis*-4-[6-Fluor-1-{4'-[(4-methyl-1,4-diazepan-1-yl)methyl]biphenyl-3-yl}-2,4-dioxo-1,4-dihydropyrido [2,3-*d*]pyrimidin-3 (2*H*)-yl]cyclohexyl}-6-methylpyridin-2-carboxamid;
oder ein pharmazeutisch akzeptables Salz davon.

12. Verbindung der Formel (I) nach Anspruch 1, die: 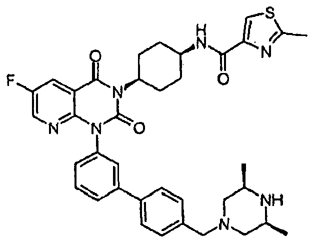 oder ein pharmazeutisch akzeptables Salz davon ist.

13. Verbindung der Formel (I) nach Anspruch 1, die: 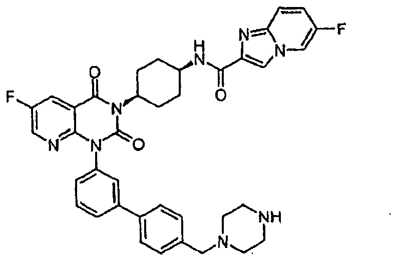 oder ein pharmazeutisch akzeptables Salz davon ist.

14. Verfahren zur Herstellung einer Verbindung der Formel (I) nach Anspruch 1, wobei das Verfahren umfasst:
a) wenn R² NHC(O)R³ ist, das Entfernen der Boc-Schutzgruppe aus einer Verbindung der Formel (II): 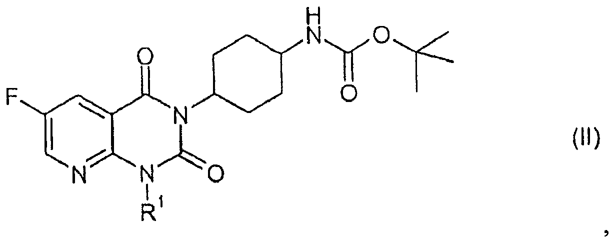 wobei R¹, G¹, E, A, Y und W wie in Anspruch 1 definiert sind, und das Umsetzen des so gebildeten Produktes mit einer Säure oder einem Säurederivat der Formel (III):
LG-R³ (III),
wobei R³ wie in Formel (I) definiert ist und LG eine Abgangsgruppe ist; oder
b) Durchführen der nachstehenden Ringschlussreaktion: 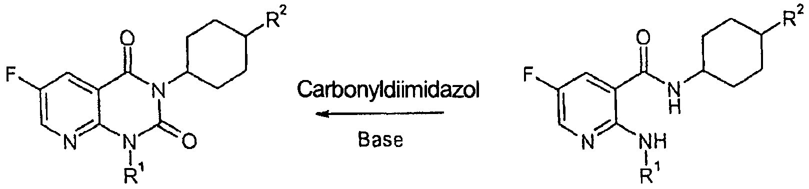 unter Anwendung eines in der Technik bekannten Verfahrens.

15. Pharmazeutische Zusammensetzung, die eine Verbindung der Formel (I) oder ein pharmazeutisch akzeptables Salz davon, nach einem der Ansprüche 1 bis 13, und ein(en) pharmazeutisch akzeptables/akzeptablen Adjuvans, Verdünnungsmittel oder Träger umfasst.

16. Verbindung der Formel (I) oder ein pharmazeutisch akzeptables Salz davon nach einem der Ansprüche 1 bis 13 zur Verwendung in der Therapie.

17. Verwendung einer Verbindung der Formel (I) oder eines pharmazeutisch akzeptablen Salzes davon nach einem der Ansprüche 1 bis 13 bei der Herstellung eines Medikaments.

18. Verbindung der Formel (I) oder ein pharmazeutisch akzeptables Salz davon nach einem der Ansprüche 1 bis 13 zur Verwendung bei der Behandlung von Asthma; der chronischobstruktiven Lungenerkrankung (COPD); Bronchitis; Emphysem; Bronchiektasie; cystischer Fibrose; Sarkoidose; Farmerlunge; exogene allergische Alveolitis; Lungenfibrose; Komplikationen bei der Lungentransplantation; Vaskulitis und thrombotischen Störungen der Lungengefäßversorgung und pulmonalem Hochdruck; antitussiver Aktivität; akuter und chronischer Rhinitis; ganzjähriger und saisonaler allergischer Rhinitis; nasaler Polyposis; einer akuten Virusinfektion; eosinophiler Ösophagitis; Arthritiden in Verbindung mit oder umfassend Osteoarthritis/Osteoarthrose; Zervikal- und Lumbalspondylose und Kreuz- und Nackenschmerzen; Osteoporose; Rheumatoidarthritis und der Still'schen Krankheit; seronegativen Spondyloarthropathien; septischer Arthritis und anderen infektionsbedingten Arthopathien und Knochenverletzungen; akuter und chronischer Kristall-induzierter Synovitis; der Behçet-Krankheit; des primären und sekundären Sjögren-Syndroms; systemischer Sklerose und eingeschränkter Sklerodermie; systemischem Lupus erythematodes, der gemischten Bindegewebskrankheit und der undifferenzierten Bindegewebskrankheit; entzündlichen Myopathien; rheumatischer Polymyalgie; juveniler Arthritis; Vaskulitiden; Kreuzschmerzen; des familiären Mittelmeerfiebers, des Muckle-Wells-Syndroms und des familiären hibernischen Fiebers, der Kikuchi-Krankheit; medikamenteninduzierten Arthalgien, Tendinitiden und Myopathien; Arthritiden; Zwischenwirbelscheibendegeneration; Degeneration des Articulatio temporomandibularis; der Knochen-Remodellierungskrankheit; Polychondritits; Sklerodermie; der gemischten Bindegewebskrankheit; Spondyloarthropathien; Parodontose;
Psoriasis, atopischer Dermatitis, Kontaktdermatitis; Phyto- und Photodermatitis; Dermatitis seborrhoica, Dermatitis herpetiformis, Lichen planus, Lichen sclerosus et atrophica, Pyoderma gangraenosum, Hautsarkoidose, Scheibenrose, Pemphigus, Pemphigoid, Epidermolysis bullosa, Urtikaria, des angioneurotischen Ödems, Vaskulitiden, toxischen Erythema, kutanen Eosinophilien, Alopecia areata, des männlichen Alopeziemusters, des Sweet-Syndroms, des Weber-Christian-Syndroms, Erythema multiforme; Zellulitis, sowohl infektiös als auch nicht infektiös; Pannikulitis; eines kutanen Lymphos, Hautkrebs ohne Melanom; fixierten Arzneimittelexanthemen;
Blepharitis; Konjunktivitis; Iritis; Uveitis anterior und posterior; Chorioiditis; der Autoimmunkrankheit; degenerativen oder Entzündungserkrankungen, die die Retina angreifen; Ophthalmitis, umfassend sympathische Ophthalmitis; Sarkoidose; Infektionen, einschließlich viral, Pilz und bakteriell; Glossitis, Gingivitis, Periodontitis; Ösophagitis; eosinophiler Gastroenteritis, Mastozytose, der Crohn-Krankheit, Colitis; Proktitis, Pruritis ani; Zöliakie, des Reizdarmsyndroms, nahrungsbedingen Allergien, die Auswirkungen außerhalb des Darms haben können; Hepatitis; Fibrose und Zirrhose der Leber; Cholezystitis; Pankreatitis; Nephritis; des nephrotischen Syndroms; Zystitis; akuter und chronischer Urethritis, Prostatitis, Epididymitis, Oophoritis und Salpingitis; Vulvovaginitis; der Peyronie-Krankheit; Erektionsstörungen;
Transplantatabstoßung;
der Alzheimer-Krankheit und anderen Demenzerkrankungen; Amyloidose; multipler Sklerose und anderen Entmarkungssyndromen; zerebraler Atherosklerose und Vaskulitis; Temporalarteriitis; Myasthenia gravis; akutem und chronischem Schmerz; Neurosarkoidose; bösartigen Komplikationen im zentralen und peripheren Nervensystem, Infektions- oder Autoimmunprozessen;
des Hashimoto-Syndroms, der Graves-Krankheit, der Addison-Krankheit, Diabetes mellitus, idiopathischer thrombozytopenischer Purpura, eosinophiler Fasciitis, des Hyper-IgE-Syndroms,
des Anti-Phospholipid-Syndroms;
des erworbenen Immunitätsmangelsyndroms (AIDS), Lepra, des Sezary-Syndroms; paraneoplastischen Syndromen;
Atherosklerose, die die koronare und periphere Zirkulation beeinträchtigt; Perikarditis; Myokarditis, entzündlichen und Autoimmunkardiomyopathien; ischämischen Reperfusionsverletzungen; Endocarditis, Valvulitis und Aortitis; Vaskulitiden; Phlebitis; Thrombose; Prostata-, Brust-, Lungen-, Eierstock-, Bauchspeicheldrüsen-, Darm- und Kolon-, Magen-, Haut- und Gehirntumoren und Malignitäten, die das Knochenmark und die lymphoproliferativen Systeme befallen; des Reizdarmsyndroms oder nicht entzündlicher Diarrhö.

19. Pharmazeutisches Produkt, umfassend in Kombination einen ersten Wirkstoff, der eine Verbindung der Formel (I) oder ein pharmazeutisch akzeptables Salz davon nach einem der Ansprüche 1 bis 13 ist, und mindestens einen weiteren Wirkstoff, ausgewählt aus:
- einem β2-Adrenozeptoragonisten,
- einem Modulator der Chemokinrezeptorfunktion,
- einem Inhibitor der Kinasefunktion,
- einem Proteaseinhibitor,
- einem steroidalen Glucocorticoidrezeptoragonisten,
- einem Anticholinergikum oder
- einem nicht steroidalen Glucocorticoidrezeptoragonisten.

20. Zwischenverbindung der folgenden Formel:

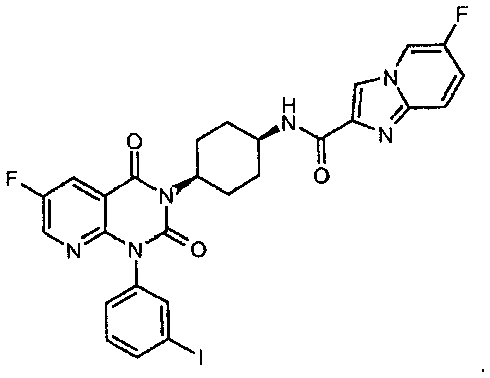

## Revendications

1. Composé de formule (I) : 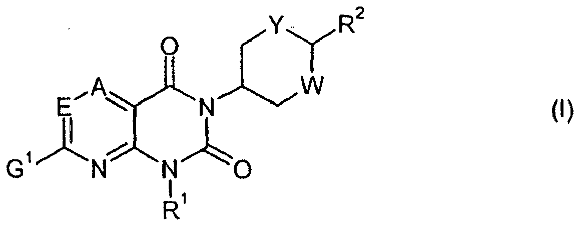 dans lequel :
A est N ou CA¹ ;
E est N ou CE¹ ;
W est (CH₂)ₙ ;
Y est (CH₂)ₚ ;
n et p sont, indépendamment, 0 ou 1 ;
R¹ est un aryle ou hétéroaryle, l'un ou l'autre est substitué par un ou plusieurs CO₂H, aryle, hétéroaryle, (C₁-₆ alkyle)NR³⁹R⁴⁰, C(O)NHaryle, C(O)N(C₁-₆ alkyle)(aryl(C₁-₆ alkyle)), C(O)NHhétéroaryle, C(O)NHhétérocyclyle, C(O)NH(CH₂)ᵥNH₂, C(O)NH(CH₂)ᵥNHCO₂(C₁-₆) alkyle), C(O)NH((C₁₋₄ alkyle)aryle), C(O)N(C₁₋₄ akyle)((C₁₋₄ alkyle)aryle), (C₁₋₆ alkyle)NHC(O)(C₁₋₆ alkoxy), hétérocyclyle(C₁₋₄ alkoxy), CH=CH(aryle), C≡C(aryle), aryl(C₁₋₄ alkyle), hétéroaryle(C1-4 alkyle), aryloxy, hétéroaryloxy, arylthio, hétéroarylthio, CH=CH(hétéroaryle) ou C≡C(hétéroaryle) ; et l'un ou l'autre peuvent être additionnellement substitués par un halogène, cyano, hydroxy, C₁₋₄ alkyle, C₁₋₄ alkoxy, CF₃, OCF₃, C₁₋₄ alkylthio, S(O)(C₁₋₄ alkyle), S(O)₂(C₁₋₄ alkyle) ou CO₂(C₁₋₄ alkyle) ;
ou R¹ est un aryle(C1-4 alkyle) ou un hétéroaryle(C1-4 alkyle), l'un ou l'autre est optionnellement substitué par un halogène, cyano, hydroxy, C₁₋₄ alkyle, C₁₋₄ alkoxy, CF₃, OCF₃, C₁₋₄ alkylethio, S(O)(C₁₋₄ alkyle), S(O)₂(C₁₋₄ alkyle), CO₂H, CO₂(C₁₋₄ alkyle), C(O)NHaryle, C(O)NH(CH₂)ᵥNH₂, C(O)NH(CH₂)ᵥNHCO₂(C₁₋₆ alkyle), aryle, hétéroaryle, CH=CH(aryle), C≡C(aryle), CH=CH(hétéroaryle), ou C≡C(hétéroaryle) ;
ou R¹ est C₅₋₇ cycloalkyle, optionnellement substitué par un hydroxy, C₁₋₄ alkyle, CO₂H, CO₂(C₁₋₄ alkyle), aryle, ou hétéroaryle ;
ou R¹ est C₁₋₁₀ alkyle ;
ou R¹ est C₁₋₆ alkyle substitué par NR⁴⁷R⁴⁸ ;
ou R¹ est un hétérocyclyle optionnellement substitué par C₁₋₆ alkyle, aryle ou hétéroaryle ;
à condition que R¹ n'est pas : 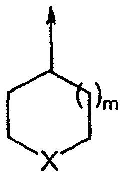
où
X est S , S(O) ou S(O)₂ ; et m est 0 ou 1 ;
où le substituant aryle ou hétéroaryle de R¹ sont optionnellement substitués par un halogène, cyano, hydroxy, SH, C₁₋₄ alkyle, C₁₋₄ alkoxy, CF₃, OCF₃, C(O)H, C₁₋₆ alkylthio, S(O)(C₁₋₆ alkyl), S(O)₂(C₁₋₆ alkyl), CO₂H, CO₂(C₁₋₆ alkyl), NR⁴¹R⁴², C₁₋₆ alkyle (optionnellement substitué par un halogène, OH, CO₂H, NR²⁹R³⁰, NHC(O)O(C₁₋₆ alkyl), OS(O)₂(C₁₋₆ alkyl) ou hétérocyclyle), C₁₋₆ alkoxy (optionnellement substitué par un halogène, OH, CO₂H, NR³⁵R³⁶ ou hétérocyclyle, C₃₋₆ cycloalkyle (optionnellement substitué par un halogène, OH, CO₂H, NR³⁷R³⁸ ou hétérocyclyle) ou hétérocyclyle ;
v est 1, 2, 3 ou 4 ;
R² est NR⁵⁰C(O)R³ ou NR⁴R⁵ ;
R³ est C₁₋₆ alkyle {optionnellement substitué par un hydroxyle, C₁₋₆ alkoxy, NR⁷R⁸, hétérocyclyle {optionnellement substitué par oxo, hydroxy, C₁₋₆ alkyle, CO₂(C₁₋₆ alkyle), aryle, hétéroaryle, aryle(C₁₋₄ alkyle), hétérocyclyle ou C(O)(C₁₋₄ alkyle)phényle}, aryle, hétéroaryle, C₃₋₇ cycloalkyle, C₃₋₇ cycloalkyle(C₁₋₄ alkyle), CO₂H, CO₂(C₁₋₆ alkyle), aryle(C₁₋₄ alkoxy), aryle(C₁₋₄ alkylthio), S(O)₂(C₁₋₆ alkyle), NHC(O)hétéroaryle ou NHC(O)R⁶} C₁₋₆ alkoxy, C₃₋₆ cycloalkyle, {optionnellement substitué par un hydroxyle, NR⁴³R⁴⁴ ou C₁₋₆ alkyle}, hétérocyclyle {optionnellement substitué par oxo, hydroxy, C₁₋₆ alkyle, amino, aryle, hétéroaryle, aryle(C₁₋₄ alkyle), hétéroaryle(C₁₋₄ alkyle), hétérocyclyle ou C(O)(C₁₋₄ alkyle)phényle}, aryle(C₁₋₄ alkyle) {substitué par amino(C₁₋₄ alkyle)}, aryle ou hétéroaryle ;
R⁴ est un hydrogène, C₁₋₆ alkyle (optionnellement substitué par un aryle ou hétéroaryle), aryle ou hétéroaryle ;
R⁵ est un hydrogène, C₁₋₆ alkyle (optionnellement substitué par un hydroxyle, C₁₋₆ alkoxy, aryle, aryloxy, phényle(C₁₋₆ alkoxy), hétéroaryle, C₃₋₁₀ cycloalkyle, CO₂H, CO₂(C₁₋₆ alkyle), NHC(O)O(C₁₋₆ alkyle), ou NHC(O)R⁶), C₁₋₆ alkoxy, C₃₋₆ cycloalkyle (optionnellement substitué par hydroxy, C₁₋₆ alkyle, phényle, phényle (C₁₋₆ alkyle), hétéroaryle ou hétéroaryle(C₁₋₆ alkyle)), hétérocyclyle (optionnellement substitué par C₁₋₆ alkyle, C(O)NH₂ ou phényle(C₁₋₆ alkyle)), aryle ou hétéroaryle ;
R⁶ est C₁₋₆ alkyle ou phényle,
R⁷ et R⁸ sont, indépendamment, un hydrogène, C₁₋₆ alkyle ou phényle(C₁₋₄ alkyle) ;
les groupements phényle, aryle et hétéroaryle de R³, R⁴, R⁵, R⁶, R⁷ et R⁸ précédents sont, indépendamment optionnellement substitués par : un halogène, cyano, nitro, hydroxy, S(O)_{q}R⁹, OC(O)NR¹⁰R¹¹, NR¹²R¹³, NR¹⁴C(O)R¹⁵, NR¹⁶C(O)NR¹⁷R¹⁸, S(O₂)NR¹⁹R²⁰, NR²¹S(O)₂R²², C(O)NR²³R²⁴, C(O)R²⁵, CO₂R²⁶, NR²⁷CO₂R²⁸, OC(O)(C₁₋₆ alkyle), C₁₋₆ alkyle, C₁₋₆ hydroxyalkyle, C₁₋₆-haloalkyle, C₁₋₆ alkoxy(C₁₋₆)alkyle, amino(C₁₋₄ alkyle), di(C₁₋₆)alkyleamino(C₁₋₆)alkyle, C₁₋₆ alkoxy, C₁₋₆ haloalkoxy, hydroxyl(C₁₋₆ alkoxy), hétérocyclyl(C₁₋₆ alkoxy), C₁₋₆ alkoxy(C₁₋₆)alkoxy, amino(C₁₋₄ alkoxy), C₁₋₄ alkyleamino(C₁₋₄ alkoxy) (lui-même optionnellement substitué par un phényle), di(C₁₋₄ alkyle)amino(C₁₋₄) alkoxy), C₁₋₆ alkylthio, C₂₋₆ alcényle, C₂₋₆ alcynyle, C₃₋₁₀ cycloalkyle (lui-même optionnellement substitué par C₁₋₄ alkyle ou oxo), méthylènedioxy, difluorométhylènedioxy, hétérocyclyle, hétérocyclyle(C₁₋₄ alkyle), phényle, phényle(C₁₋₄) alkyle, phénoxy, phénylethio, phényle(C₁₋₄)alkoxy, hétéroaryle, hétéroaryle(C₁₋₄)alkyle, hétéroaryloxy ou hétéroaryle(C₁₋₄)alkoxy ; dans lequel l'un quelconque des groupements phényle et hétéroaryle immédiatement précédents sont optionnellement substitués avec un halogène, hydroxy, nitro, S(O)ᵣ(C₁₋₄ alkyle), S(O)₂NH₂, S(O)₂NH(C₁₋₄ alkyle), S(O)₂N(C₁₋₄ alkyle)₂, cyano, C₁₋₄ alkyle, C₁₋₄ alkoxy, C(O)NH₂ , C(O)NH(C₁₋₄ alkyle), C(O)N(C₁₋₄ alkyle)₂, CO₂H, CO₂(C₁₋₄ alkyle), NHC(O)(C₁₋₄ alkyle), NHS(O)₂(C₁₋₄ alkyle), C(O)(C₁₋₄ alkyle), CF₃ ou OCF₃ ;
A¹, E¹ et G¹ sont, indépendamment, l'hydrogène, un halogène, cyano, hydroxy, C₁₋₄ alkyle, C₁₋₄ alkoxy, CF₃ ou OCF₃ ;
q et r sont, indépendamment, 0, 1 ou 2 ;
sauf indications contraires, l'hétérocyclyle est optionnellement substitué par OH, C₁₋₆ alkyle, C₃₋₇ cycloalkyle, NR³¹R³², (C₁₋₆ alkyle)OH ou (C₁₋₆ alkyle)NR³³R³⁴, NR⁴⁹CO₂(C₁₋₆ alkyle), CO₂(C₁₋₆ alkyle), C(O)(C₁₋₆ alkyle), C(O)hétérocyclyle, hétéroaryle, (C₁₋₆ alkyle)C(O)NR⁵³R⁵⁴, (C₁₋₆ alkyle)C(O)NR⁵⁵R⁵⁶, (C₁₋₆ alkyle)C(O)hétérocyclyle ou hétérocyclyle ;
R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷ et R²⁸ sont, indépendamment, C₁₋₆ alkyle {optionnellement substitué par un halogène, hydroxy ou C₁₋₆ alkoxy}, CH₂(C₂₋₆ alcényle), phényle {lui-même substitué par un halogène, hydroxy, nitro, NH₂, NH(C₁₋₄ alkyle), N(C₁₋₄ alkyle)₂, S(O)₂(C₁₋₄ alkyle), S(O)₂NH₂, S(O)₂NH(C₁₋₄ alkyle), S(O)₂N(C₁₋₄ alkyle)₂, cyano, C₁₋₄ alkyle, C₁₋₄ alkoxy, C(O)NH₂, C(O)NH(C₁₋₄ alkyle), C(O)N(C₁₋₄ alkyle)₂, CO₂H, CO₂(C₁₋₄ alkyle), NHC(O)C₁₋₄ alkyle), NHS(O)₂(C₁₋₄ alkyle), C(O)(C₁₋₄ alkyle), CF₃ ou OCF₃} ou hétéroaryle {lui-même optionnellement substitué par un halogène, hydroxy, nitro, NH₂, NH(C₁₋₄ alkyle), N(C₁₋₄ alkyle)₂, S(O)₂(C₁₋₄ alkyle), S(O)₂NH₂, S(O)₂NH(C₁₋₄ alkyle), S(O)₂N(C₁₋₄ alkyle)₂, cyano, C₁₋₄ alkyle, C₁₋₄ alkoxy, C(O)NH₂, C(O)NH(C₁₋₄ alkyle), C(O)N(C₁₋₄ alkyle)₂, CO₂H, CO₂(C₁₋₄ alkyle), NHC(O)(C₁₋₄ alkyle), NHS(O)₂(C₁₋₄ alkyle), C(O)(C₁₋₄ alkyle), CF₃ ou OCF₃} ;
R¹⁰, R¹¹, R¹², R¹³, R¹⁴, R¹⁵, R¹⁶, R¹⁷, R¹⁸, R¹⁹, R²⁰, R²¹, R²³, R²⁴, R²⁵, R²⁶, R²⁷ et R²⁸ peuvent aussi être l'hydrogène ;
R⁵⁰ est l'hydrogène ou C₁₋₆ alkyle (optionnellement substitué par NR⁵¹R⁵²) ;
R³⁰, R³², R³⁴, R³⁶, R³⁸, R⁴⁰, R⁴², R⁴⁴, ou R⁴⁸ sont, indépendamment, l'hydrogène, C₁₋₆ alkyle (optionnellement substitué par hydroxy, C₁₋₆ alkoxy, C₁₋₆ alkylthio, C₃₋₇ cycloalkyle (optionnellement substitué par hydroxy) ou NR⁴⁵R⁴⁶), C₃₋₇ cycloalkyle (optionnellement substitué par hydroxy(C₁₋₆ alkyle)) ou hétérocyclyle (optionnellement substitué par C₁₋₆ alkyle) ;
R²⁹ R³¹, R³³, R³⁵, R³⁷, R³⁹, R⁴¹, R⁴³, R⁴⁵, R⁴⁶, R⁴⁷, R⁴⁹, R⁵¹, R⁵², R⁵³, R⁵⁴, R⁵⁵ et R⁵⁶ sont, indépendamment, l'hydrogène ou C₁₋₆ alkyle ;
ou un N-oxyde de celui-ci ; ou un de ses sels pharmaceutiquement acceptables.

2. Composé de formule (I) tel que revendiqué dans la revendication 1 dans lequel E est CE¹ et E¹ est tel que défini dans la revendication 1.

3. Composé de formule (I) tel que revendiqué dans la revendication 1 ou 2 dans lequel A est CH.

4. Composé de formule (I) tel que revendiqué dans la revendication 1, 2 ou 3 dans lequel G¹ est l'hydrogène ou un halogène.

5. Composé de formule (I) tel que revendiqué dans la revendication 1, 2, 3 ou 4 dans lequel n et p sont tous les deux 1.

6. Composé de formule (I) tel que revendiqué dans la revendication 1 dans lequel A est CA¹ ; E est CE¹ ; W et Y sont tous les deux CH₂ ; et G¹, A¹ et E¹ sont, indépendamment, l'hydrogène ou un halogène.

7. Composé de formule (I) tel que revendiqué dans une quelconque des revendications précédentes dans lequel R¹ est un phényle substitué par un phényle {qui est substitué par un halogène, hydroxy, CH(O), CO₂H, C₁₋₄ alkyle, C₁₋₄ alkyle(N(C₁₋₄ alkyle)₂), C₁₋₄ alkyle(NH₂), C₁₋₄ alkyle(NH(C₁₋₄ alkyle)), C₁₋₄ hydroxyalkyle, CF₃, C₁₋₄ alkylthio, C₁₋₄ alkyle(hétérocyclyle) ou C₁₋₄ alkyle(NHC(O)O(C₁₋₄ alkyle)} ou hétérocyclyle ; et l'hétérocyclyle est optionnellement substitué par C₁₋₆ alkyle.

8. Composé de formule (I) tel que revendiqué dans une quelconque des revendications précédentes dans lequel R² est NHC(O)R³ ; et R³ est C₁₋₄ alkyle {substitué par NR⁷R⁸, hétérocyclyle ou hétéroaryle}, C₃₋₇ cycloalkyle (optionnellement substitué par NR⁴³R⁴⁴) ou hétéroaryle ; dans lequel R⁷, R⁸, R⁴³ et R⁴⁴ sont tels que définis dans la revendication 1 ; l'hétéroaryle étant optionnellement substitué par halo, C₁₋₄ alkyle, CF₃, C₁₋₄ alkoxy, OCF₃, hétérocyclyle ou amino(C₁₋₄ alkyle).

9. Composé de formule (IA) tel que revendiqué dans la revendication 1 : 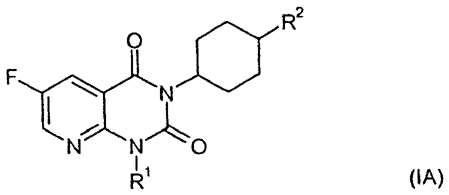 dans lequel :
R¹ est un phényle substitué par un phényle (substitué par CH₂(hétérocyclyle)) et en outre optionnellement substitué par un hydroxyle ; l'hétérocyclyle étant optionnellement substitué par C₁₋₆ alkyle ; R² est NHC(O)R³ ;
R³ est un hétéroaryle, hétérocyclyle, C₁₋₆ alkoxy ou C₃₋₇ cycloalkyle ; l'hétéroaryle étant optionnellement substitué par un halo, C₁₋₄ alkyle ou hétérocyclyle ; et, l'hétérocyclyle étant optionnellement substitué par C₁₋₄ alkyle ;
et où l'hétérocyclyle est un cycle non-aromatique à 5 ou 6 membres optionnellement fusionné à un ou plusieurs cycles non-aromatiques et optionnellement fusionnés à un cycle benzène, comprenant au moins un hétéroatome choisi parmi le groupe comprenant l'azote, l'oxygène et le soufre ;
et où l'hétéroaryle est un cycle aromatique à 5 ou 6 membres optionnellement fusionné à un ou plusieurs cycles (pouvant être carbocycliques ou hétérocycliques, et aromatiques ou non-aromatiques), comprenant au moins un hétéroatome choisi parmi le groupe comprenant l'azote, l'oxygène et le soufre ; ou un de ses N-oxyde, ou un de ses S-oxyde ou S-dioxyde ;
ou un de ses N-oxyde ; ou un de ses sels pharmaceutiquement acceptable.

10. Composé de formule (IA) tel que revendiqué dans la revendication 9, dans lequel le premier phényle de R¹ est substitué par le deuxième phényle de R¹ dans la position 3.

11. Composé de formule (I) tel que revendiqué dans la revendication 1, choisi parmi :
6-fluoro-N-{cis-4-[6-fluoro-2,4-dioxo-1-[4'-(piperazine-1-ylméthyle)biphényle-3-yle]-1,4-dihydropyrido[2,3-d]pyrimidin-3(2H)-yl]cyclohexyl}imidazo[1,2-a]pyridine-2-carboxamide ;
6-fluoro-N-{cis-4-[6-fluoro-2,4-dioxo-1-[4'-(2-pyrrolidin-1-yléthoxy)biphényle-3-yle]-1,4-dihydropyrido[2,3-d]pyrimidin-3(2H)-yl]cyclohexyl}imidazo[1,2-a]pyridine-2-carboxamide ;
6-fluoro-N-{cis-4-[6-fluoro-1-(4'-{3-[(3-hydroxypropyle)amino]propyle}biphényle-3-yle)-2,4-dioxo-1,4-dihydropyrido[2,3-d]pyrimidin-3(2H)-yl]cyclohexyl}imidazo[1,2-a]pyridine-2-carboxamide ;
6-fluoro-3-(cis-4-{[(6-fluoroimidazole[1,2-a]pyridin-2-yle)méthyle]amino}cyclohexyle)-1-[4'-hydroxy-2'-(morpholin-4-yleméthyle)biphényle-3-yle]pyrido[2,3-d]pyrimidine-2,4(1 H,3H)-dione ; ou,
N-{cis-4[6-fluoro-1-{4'-[(4-méthyle-1,4-diazepan-1-yle)méthyle]biphényle-3-yle}-2,4-dioxo-1,4-dihydropyrido[2,3-d]pyrimidin-3(2H)-yl]cyclohexyle}-6-méthylepyridine-2-carboxamide ;
ou un de ses sels pharmaceutiquement acceptable.

12. Composé de formule (I) tel que revendiqué dans la revendication 1 qui est : 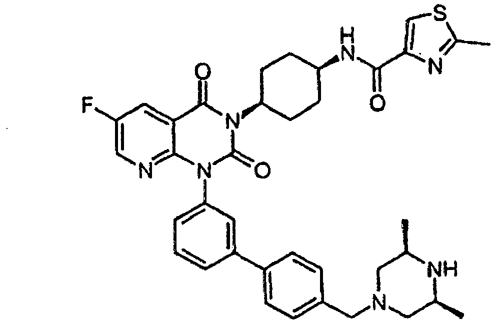 ou un de ses sels pharmaceutiquement acceptables.

13. Composé de formule (I) tel que revendiqué dans la revendication 1 qui est : 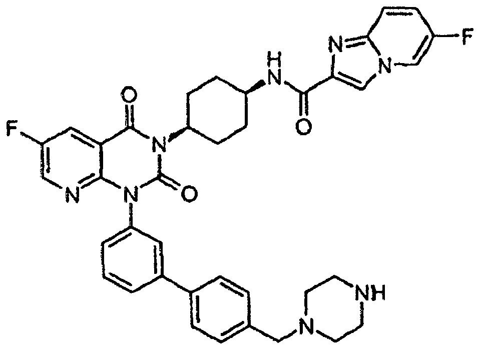 ou un de ses sels pharmaceutiquement acceptables.

14. Procédé pour la préparation d'un composé de formule (I) tel que revendiqué dans la revendication 1, le procédé comprenant :
a) lorsque R² est NHC(O)R³, éliminer le groupe protecteur Boc d'un composé de formule (II) : 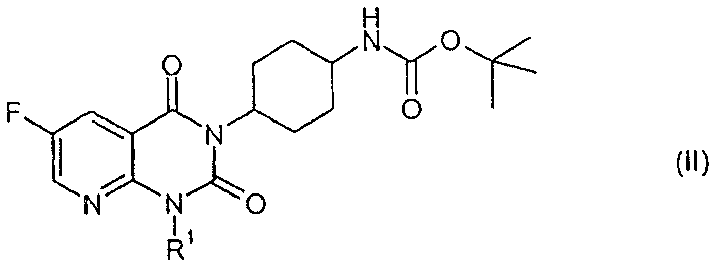 dans lequel R¹, G¹, E, A, Y et W sont tels que définis dans la revendication 1, et faire réagir le produit ainsi formé avec un acide ou dérivé acide de formule (III) :
LG-R³ (III)
dans lequel R³ est défini dans la formule (I), et LG est un groupe partant ; ou
b) mener la réaction de fermeture de cycle ci-dessous : 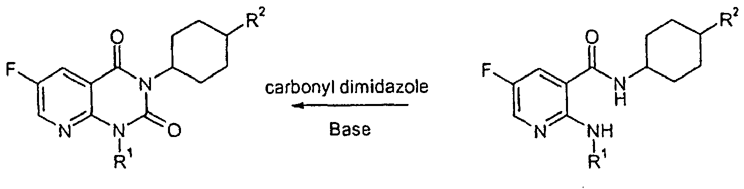 en utilisant une méthode connue dans l'état de la technique.

15. Composition pharmaceutique comprenant un composé de la formule (I), ou un de ses sels pharmaceutiquement acceptables, tel que revendiqué dans l'une quelconque des revendications 1 à 13, et un adjuvant, diluant ou support pharmaceutiquement acceptable.

16. Composé de formule (I), ou un de ses sels pharmaceutiquement acceptable, tel que revendiqué dans l'une quelconque des revendications 1 à 13, pour une utilisation thérapeutique.

17. Utilisation d'un composé de formule (I), ou d'un de ses sels pharmaceutiquement acceptable, tel que revendiqué dans l'une quelconque des revendications 1 à 13, dans la fabrication d'un médicament.

18. Composé de formule (I), ou un de ses sels pharmaceutiquement acceptables, tel que revendiqué dans l'une quelconque des revendications 1 à 13, utilisé dans le traitement de l'asthme ; la maladie pulmonaire obstructive chronique (MPOC) ; la bronchite ; l'emphysème ; la dilatation des bronches ; la fibrose kystique ; la sarcoïdose ; le poumon du fermier ; la pneumonie d'hypersensibilité ; la fibrose pulmonaire ; les complications de transplantation pulmonaire ; les troubles vasculaires et thrombotiques du système vasculaire pulmonaire, et l'hypertension pulmonaire ; l'activité antitussive ; la rhinite aiguë et chronique ; la rhinite allergique pérenne et saisonnière ; la polypose nasale ; l'infection virale aiguë ; l'oesophagite éosinophilique ; l'arthrite associée avec ou comprenant l'ostéoarthrite/ostéoarthrose ; la spondylarthrite ankylosante cervicale et lombaire, et les douleurs dans le bas du dos et le cou ; l'ostéoporose ; l'arthrite rhumatoïde et la maladie de Still's ; les spondyloarthropathies séronégatives ; l'arthrite septique et autres arthropathies et troubles osseux inflammatoires ; la synovite cristalline aiguë et chronique ; la maladie de Behcet ; le syndrome de Sjogren primaire et secondaire ; la sclérose systémique et sclérodermie limitée ; le lupus érythémateux disséminé, les maladies du collagène mixtes, et les maladies du collagène indifférencié ; les myopathies inflammatoires ; la polymyalgie rhumatismale ; l'arthrite juvénile ; les vascularites ; les douleurs au bas du dos ; la fièvre méditerranéenne familiale, le syndrome de Muckle-Wells, la fièvre hibernienne familiale ; la maladie de Kikuchi ; l'arthralgie d'origine médicamenteuse, les tendinites, et les myopathies ; l'arthrite ; les dégénérations du disque intervertébral ; la dégénération de l'articulation temporomandibulaire ; la maladie du remodelage osseux ; la polychondrite ; la sclérodermie ; la maladie du collagène mixte ; les spondyloarthropathies ; les maladies périodontales ; le psoriasis ; la dermatite atopique ; la dermatite de contact ; les phyto- et photodermatites ; la dermatite séborrhéique, la dermatite herpétiforme, le lichen plan, le lichen scléreux et atrophique, la pyodermie gangréneuse, la sarcoïdose de la peau, le lupus érythémateux discoïde, le pemphigus, la pemphigoïde, l'épidermolyse bulleuse, l'urticaire, l'oedème de Quincke, les vascularites, les érythèmes toxiques, les éosinophilies cutanées, l'alopécie en aires, la calvitie masculine, le syndrome de Sweet, le syndrome de Weber-Christian, l'érythème multiforme ; la cellulite, infectieuse ou non ; le panniculitis ; les lymphomes cutanés, le cancer de la peau sans présence de mélanome ; les éruptions pour une posologie médicamenteuse fixe ; la blépharite ; la conjonctivite ; l'iritite ; l'uvéite antérieure et postérieure ; la chloroïde ; les troubles auto-immunes, dégénératifs ou inflammatoires de la rétine ; l'ophtalmie, comprenant l'ophtalmie sympathique, les sarcoïdes ; les infections virales, fongiques et bactériennes ; la glossite ; la gingivite ; la parodontite ; l'oesophagite ; la gastro-entérite éosinophilique, la mastocytose, la maladie de Crohn, la colite ; la proctite, le prurit ; la maladie coeliaque, le syndrome de l'intestin irritable, les allergies alimentaires pouvant avoir des effets non-intestinaux ; l'hépatite ; la fibrose et la cirrhose du foie ; la cholécystite ; la pancréatite ; la néphrite ; le syndrome néphrotique ; la cystite ; l'urétrite aiguë et chronique, la prostatite, l'épididymite, l'oophorite et la salpingite ; la vulvo-vaginite ; la maladie de La Peyronie ; les troubles érectiles ; le rejet d'allogreffes ; la maladie d'Alzheimer et autres troubles démentiels ; l'amylose ; la sclérose en plaque et autres maladies démyélinisantes ; l'athérosclérose et vascularite cérébrale ; artérite temporale ; la myasthénie grave ; la douleur aiguë et chronique ; la neurosarcoïdose ; les complications du système nerveux central et périphérique dus à des procédés malins, infectieux ou auto-immunes ; la thyroïdite de Hashimoto, la maladie de Grave, la maladie d'Addison, le diabète sucré, le purpura thrombopénique idiopathique, la fasciite éosinophilique, le syndrome hyper-IgE, le syndrome des antiphospholipides ; le syndrome d'immunodéficience acquise (SIDA), la lèpre, le syndrome de Sezary ; les syndromes paranéoplastiques ; l'athérosclérose, affectant la circulation coronarienne et périphérique ; la péricardite ; la myocardite, les cardiomyopathies inflammatoires et auto-immunes ; les lésions d'ischémie-réperfusion ; l'endocardite, la valvulite, et l'aortite ; les vascularites, les phlébites, les thromboses ; les tumeurs et affectations malignes de la prostate, du sein, du poumon, des ovaires, du pancréas, de l'intestin et du colon, de l'estomac, de la peau et du cerveau affectant la moelle osseuse et les systèmes lymphoprolifératifs ; le syndrome de l'intestin irritable, ou la diarrhée non-inflammatoire.

19. Produit pharmaceutique comprenant, en combinaison, un premier ingrédient actif qui est un composé de formule (I), ou un de ses sels pharmaceutiquement acceptable, tel que revendiqué dans l'une quelconque des revendications 1 à 13, et au moins un autre ingrédient actif choisi parmi :
• un agoniste de récepteur adrénergique ß2,
• un modulateur de fonction récepteur de chimiokine,
• un inhibiteur de la fonction kinase,
• un inhibiteur de protéase,
• un agoniste de récepteur glucocorticoïde stéroïdien,
• un agent anticholinergique, ou
• un agoniste de récepteur glucocorticoïde non-stéroïdien.

20. Composé intermédiaire de formule suivante :
